# EUROPEAN PATENT SPECIFICATION

(11) **EP 4 329 749 B1**
(45) Date of publication and mention of the grant of the patent: **09.12.2026**
(21) Application number: 22796676.9
(22) Date of filing: 27.04.2022
(51) Int. Cl.: A61K 31/409, A61K 31/495, A61P 35/00, C07D 267/00, C07D 471/22, C07D 267/22, C07D 405/04, C07D 487/16, C07D 491/22, C07D 495/22, C07D 498/16, C07D 519/00

(54) **SMALL MOLECULE INHIBITORS OF KRAS G12C MUTANT**
KLEINMOLEKÜLIGE INHIBITOREN DES KRAS-G12C-MUTANTEN
INHIBITEURS À PETITES MOLÉCULES DE MUTANT DE KRAS G12C

(30) Priority: 27.04.2021 US 202163180430 P
(43) Date of publication of application: 06.03.2024
(73) Proprietor: Merck Sharp & Dohme LLC, Rahway, New Jersey 07065 (US)
(72) Inventor: SHIBATA, Kazuaki, Tsukuba-shi Ibaraki, 300--2611 (JP); ASAKURA, Hiroki, Tsukuba-shi Ibaraki, 300--2611 (JP); AKEMOTO, Kei, Tsukuba-shi Ibaraki, 300--2611 (JP); SAKAMOTO, Toshihiro, Tsukuba-shi Ibaraki, 300--2611 (JP); KONDO, Hitomi, Tsukuba-shi Ibaraki, 300--2611 (JP); YAMAMOTO, Tomohiro, Tsukuba-shi Ibaraki, 300--2611 (JP); MIURA, Risako, Tsukuba-shi Ibaraki, 300--2611 (JP); SCHÖPF, Patrick, Cambridge Science Park Cambridge CB4 0QA (GB); DEL POZO, Juan, Boston, Massachusetts 02115-5727 (US); GIAMBUSU, George Madalin, Boston, Massachusetts 02115-5727 (US); GRAHAM, Thomas H., Boston, Massachusetts 02115-5727 (US); HAN, Yongxin, Boston, Massachusetts 02115-5727 (US); HENNESSY, Elisabeth T., Boston, Massachusetts 02115-5727 (US); PALANI, Anandan, Boston, Massachusetts 02115-5727 (US); RYAN, Michael, Boston, Massachusetts 02115-5727 (US); SLOMAN, David L., Boston, Massachusetts 02115-5727 (US); HOWARD, Steven, Cambridge Science Park Cambridge CB4 0QA (GB)
(74) Representative: Kilburn & Strode LLP
(86) International application number: PCT/US2022/026599
(87) International publication number: WO 2022/232318

(56) References cited:
- WO-A1-2015/054572
- US-A1- 2018 334 454
- US-A1- 2020 140 437

## Description

### CROSS REFERENCE TO RELATED APPLICATION

The present application claims the benefit of U.S. Provisional Application No. 63/180,430, filed April 27, 2021.

### FIELD OF THE INVENTION

The present invention relates to cyclized aniline derivatives that inhibit the G12C mutant of Kirsten rat sarcoma (KRAS) protein and relates to a pharmaceutical composition comprising a compound of Formula (I) or (Ia) as well as methods of using such a compound for treatment of diseases, including cancers.

### BACKGROUND OF THE INVENTION

RAS, which is a small monomeric GTP-binding protein having a molecular weight of about 21 kDa, acts as a molecular on/off switch. RAS can bind to GTP by binding to proteins of a guanine nucleotide exchange factor (GEF) (e.g., SOS1), which forces the release of a bound nucleotide, and releasing GDP. When RAS binds to GTP, it becomes activated (turned on) and recruits and activates proteins necessary for the propagation of other receptors' signals, such as c-Raf and PI 3-kinase. RAS also possesses enzymatic activity with which it cleaves the terminal phosphate of the GTP nucleotide and converts it to GDP. The rate of conversion is usually slow, but can be dramatically sped up by a protein of the GTPase-activating protein (GAP) class, such as RasGAP. When GTP is converted into GDP, RAS is deactivated (turned off).

The commonly known members of the RAS subfamily include HRAS, KRAS, and NRAS. Of these, mutations of KRAS are observed in many malignant tumors: in 95% of pancreatic cancers, in 45% of colorectal cancers, and in 35% of lung cancers. The mutations often occur in the glycine residue at position 12; in pulmonary adenocarcinoma, in particular, the mutation in the glycine residue at position 12 occurs in about 90% of the total KRAS mutations Among such mutations, the most often occurring mutation (44%) has been reported to be a mutation into cysteine (Nature Reviews Drug Discovery, 13 (11), 828-51, 2014).

KRAS proteins having the G12C mutation havehistorically thought to exist in a constitutively active state (GTP-bound) in cancer cells. However, a recent study indicated that KRAS proteins having the G12C mutation havebasal GTPase activity. K-Ras has a pocket structure to which a therapeutic agent can bind. Part of the pocket contains Switch 1 (residue 30 to 40) and Switch 2 (residue 60 to 76). Switch 1 has threonine-35 and Switch 2 has glycine-60, and these amino acids respectively form a hydrogen bond with the γ-phosphoric acid of GTP, which keeps Switch 1 and Switch 2 in an active form. These two regions will be released by hydrolysis of GTP and liberate phosphoric acid to form an inactive GDP form. When GTP bound to K-Ras is replaced with GDP, the three-dimensional conformation of the switch region containing these switches is changed. The change may relate to a bond between K-Ras and a target protein, such as c-Raf.

Actually, it was reported that ARS-853 binds to the cysteine residue of the G12C mutant of inactive KRAS (GDP), thus preventing conversion of inactive KRAS (GDP) to active KRAS (GTP), inhibiting downstream signaling, and inducing apoptosis in cancer cells with the KRAS G12C mutation (WO 2014/152588; Cancer Discov., 6 (3), 316-29, 2016). It has also been reported that ARS-1620 with a quinazoline backbone exerts antitumor action in tumor-bearing mice expressing the KRAS G12C mutation by improving metabolic stability in mice (WO 2015/054572; Cell, 172 (3), 578-89, 2018).

However, because of its mode of action, there is a possibility that the inhibitors which bind to the inactive form of KRAS G12C are not able to exert sufficient effect to KRAS G12C-positive cancer patients in whom the active form of KRAS protein (GTP) tends to be increased by activation of a KRAS upstream pathway or deactivation of GTPase activity in clinical settings. In fact, it has been reported that the inhibition of KRAS activity and the antiproliferative effect of ARS-853 are attenuated by EGFR activation in a KRAS G12C mutated cell line (Cancer Discov., 6 (3), 316-29, 2016; Science, 351 (6273), 604-8, 2016).

### SUMMARY OF THE DISCLOSURE

The present disclosure provides cyclized aniline derivatives which modulate mutant KRAS, HRAS, and/or NRAS proteins and may be valuable pharmaceutically active compounds for the treatment of cancer. In some embodiments the disclosed compounds selectively inhibit the KRAS (G12C) protein. The compounds of Formula (I): 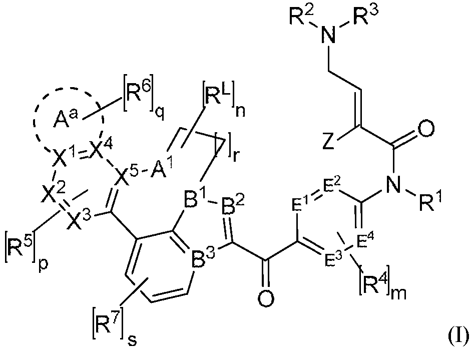 and their pharmaceutically acceptable salts, can modulate the activity of KRAS, HRAS and/or NRAS activity and thereby affect the signaling pathway which regulates cell growth, differentiation, and proliferation associated with oncological disorders. In certain embodiments, the compounds of Formula (I) can inhibit the KRAS (G12C) protein. The disclosure furthermore provides processes for preparing compounds of Formula (I), methods for using such compounds to treat oncological disorders, and pharmaceutical compositions which comprise compounds of Formula (I).

### DETAILED DESCRIPTION OF THE INVENTION

### Compounds of the Disclosure

In one embodiment, the present disclosure provides a compound having structural Formula (I), or a pharmaceutically acceptable salt thereof, as shown above, wherein:
R¹ is selected from the group consisting of H and C₁-C₆ alkyl;
R² and R³ are independently selected from the group consisting of:
   (i) H;
   (ii) C₁-C₆ alkyl, wherein the C₁-C₆ alkyl is unsubstituted or substituted by 1 to 2 R^{a} substituents independently selected from the group consisting of halo, hydroxy, C₁-C₃ alkoxy, C2-C6 alkenyl, C2-C6 alkynyl, C₁-C₃ alkylamino, C1-C3 dialkylamino, C1-C3 alkylsulfonyl, C2-C4 acyl, C1-C3 hydroxyalkyl, C₁-C₃ alkoxycarbonyl, C₁-C₃ hydroxycarbonyl, oxo (=O), C3-C8 cycloalkyl, and 4-to 10-membered mono- or bicyclic heterocycloalkyl; wherein the C3-C8 cycloalkyl or the 4-to 10-membered mono- or bicyclic heterocycloalkyl of R^{a} is unsubstituted or substituted by 1 to 2 substituents selected from the group consisting of halo, hydroxy, amino, C₁-C₃ alkyl, C₁-C₃ fluoroalkyl, and C₁-C₃ alkoxy; and
   (iii) a 3- to 8-membered monocyclic, bridged bicyclic, or spirocyclic saturated ring system containing 0 to 2 heteroatom groups selected from the group consisting of N, O, S, S(O), S(O)2, P, P(O), and P(O)2; wherein the 3- to 8-membered monocyclic, bridged bicyclic, or spirocyclic saturated ring system is unsubstituted or substituted by 1 to 4 substituents independently selected from the group consisting of halo, hydroxy, C₁-C₃ alkyl, C1-C3 fluoroalkyl, C1-C3 alkoxy, C2-C6 alkenyl, C2-C6 alkynyl, C1-C3 alkylamino, C1-C3 dialkylamino, C1-C3 alkylsulfonyl, C2-C4 acyl, C₁-C₃ hydroxyalkyl, C₁-C₃ alkoxy(C₁-C₃)alkyl, carboxy, and C1-C3 alkoxycarbonyl; or alternatively, R² and R³ together with the N atom to which they are attached form a 4- to 9-membered monocyclic, fused bicyclic, bridged bicyclic, or spirocyclic saturated ring system containing 0 to 2 additional heteroatoms selected from the group consisting of N, O, and S; wherein the 4- to 9-membered monocyclic, fused bicyclic, bridged bicyclic, or spirocyclic saturated ring system is unsubstituted or substituted by 1 to 4 substituents independently selected from the group consisting of halo, hydroxy, C₁-C₃ alkyl, C₁-C₃ fluoroalkyl, C₁-C₃ alkoxy, C₁-C₄ hydroxyalkyl, C3-C5 hydroxycycloalkyl, -(CH₃)₂N(C=O)CH₂-, CH₃(C=O)NHCH₂-, and C₁-C₃ alkoxy(C₁-C₃)alkyl; or alternatively, one of R² and R³ together with the N atom to which it is attached and an adjacent C atom form a 3- to 6-membered monocyclic saturated ring system containing 0 to 2 additional heteroatoms selected from the group consisting of N, O, and S; wherein the 3- to 6- membered monocyclic saturated ring system is unsubstituted or substituted by 1 to 4 substituents independently selected from the group consisting of halo, hydroxy, C₁-C₃ alkyl, C1-C3 fluoroalkyl, C1-C3 alkoxy, C1-C3 hydroxyalkyl, and C1-C3 alkoxy(C₁-C₃)alkyl;
at each occurrence R⁴ is independently selected from the group consisting of halo, cyano, C1-C3 alkyl, C1-C3 fluoroalkyl, C1-C3 alkoxy, and C1-C4 cyanoalkyl;
E¹, E², E³, and E⁴ are independently selected from the group consisting of N and C, with the proviso that no more than three of E¹, E², E³, and E⁴ are N;
one of B¹ and B³ is C and the other is N;
B² is N or C(R^{B}), wherein R^{B} is selected from the group consisting of H, C₁-C₆ alkyl, C2-C6 alkenyl, C2-C6 alkynyl and C3-C6 cycloalkyl;
at each occurrence R^{L} is independently selected from the group consisting of fluoro, hydroxy, C₁-C₆ alkyl, C₁-C₃ alkoxy, C₁-C₃ fluoroalkyl, oxo (=O), C2-C4 acyl, and methenyl (=CH₂), or
alternatively, two geminally substituted R^{L} substituents together with the carbon atom to which they are attached form a 3- to 6-membered spirocyclic ring containing 0 to 1 heteroatoms selecting from the group consisting of N, O, and S;
ring A^{a} is present or absent, and if present, is a 5- to 6-memberered heteroaryl containing 1 to 3 heteroatoms selected from the group consisting of N, O, and S;
X¹, X², X³, X⁴, and X⁵ are independently selected from the group consisting of N and C, with the proviso that no more than two of X¹, X², X³, X⁴, and X⁵ are N;
at each occurrence R⁵ is independently selected from the group consisting of C₁-C6 alkyl, C1-C6 fluoroalkyl, C1-C3 alkoxy, C1-C3 acyl, halo, hydroxy, amino, cyano, oxo (=O), C1-C4 cyanoalkyl, C1-C3 hydroxyalkyl, C1-C3 alkylamino, C1-C3 dialkylamino, C₁-C₃ alkoxy(C₁-C₃)alkyl, and a group of the formula 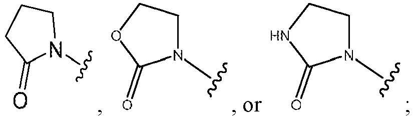
at each occurrence R⁶ is independently selected from the group consisting of C₁-C3 alkyl, C1-C3 fluoroalkyl, C1-C3 alkoxy, halo, hydroxy, oxo (=O), cyano, carbamoyl, C1-C4 cyanoalkyl, C1-C3 hydroxyalkyl, C1-C3 alkoxy(C1-C3)alkyl, , C3-C6 cycloalkyl, and C1-C4 carbamoylalkyl;
each occurrence of R⁷ is independently selected from the group consisting of halo, C1-C3 alkyl, C1-C3 fluoroalkyl, and C1-C3 alkoxy;
A¹ is selected from the group consisting of -CH₂-, -CH₂- N(R^{L})-, -C(=O)-N(R^{L})-, -CH₂-O-, -C(H)(R^{L})-, -N(H)-, -N(R^{L})-, -S-, -S(=O)-, and -O-;
Z is selected from the group consisting of H and halo;
subscript m is 0, 1, 2, or 3;
subscript n is 0, 1, 2, or 3;
subscript p is 0, 1, 2, 3, or 4;
subscript q is 0, 1, 2, or 3;
subscript r is 0, 1, 2, or 3; and
subscript s is 0, 1, 2, or 3.

In one embodiment, the C1-C6 alkyl is unsubstituted or substituted by 1 to 2 R^{a} substituents independently selected from the group consisting of halo, hydroxy, C₁-C₃ alkoxy, C2-C6 alkenyl, C2-C6 alkynyl, C₁-C₃ alkylamino, C₁-C₃ dialkylamino, C₁-C₃ alkylsulfonyl, C2-C4 acyl, C1-C3 hydroxyalkyl, C1-C3 alkoxycarbonyl, C1-C3 hydroxycarbonyl, C3-C8 cycloalkyl, and 4-to 10-membered mono- or bicyclic heterocycloalkyl; R² and R³ together with the N atom to which they are attached form the 4- to 8-membered saturated heterocycloalkyl having 0 to 2 additional heteroatoms selected from the group consisting of N, O, and S; wherein the 4- to 8-membered heterocycloalkyl is unsubstituted or substituted by 1 to 4 substituents independently selected from the group consisting of halo, hydroxy, C₁-C₃ alkyl, C₁-C₃ fluoroalkyl, C1-C3 alkoxy, C₁-C₃ hydroxyalkyl, and C₁-C₃ alkoxy(C₁-C₃)alkyl; at each occurrence R^{L} is independently selected from the group consisting of fluoro, hydroxy, C₁-C₆ alkyl, C₁-C3 alkoxy, C₁-C₃ fluoroalkyl, oxo (=O), and methenyl (=CH2); the ring A^{a} is present or absent, and if present, is the 5- to 6-memberered heteroaryl containing 1 to 2 heteroatoms selected from the group consisting of N, O, and S; at each occurrence R⁵ is independently selected from the group consisting of C₁-C₆ alkyl, C₁-C₆ fluoroalkyl, C₁-C₃ alkoxy, C1-C3 acyl, halo, hydroxy, amino, cyano, oxo (=O), C₁-C₄ cyanoalkyl, C₁-C₃ hydroxyalkyl, C₁-C₃ alkylamino, C₁-C₃ dialkylamino, C₁-C₃ alkoxy(C₁-C₃)alkyl, and the group of the formula 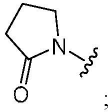 at each occurrence R⁶ is independently selected from the group consisting of C₁-C₃ alkyl, C1-C3 fluoroalkyl, C1-C3 alkoxy, halo, hydroxy, oxo (=O), cyano, carbamoyl, C1-C4 cyanoalkyl, C1-C3 hydroxyalkyl, C1-C3 alkoxy(C1-C3)alkyl, and C1-C4 carbamoylalkyl; A¹ is selected from the group consisting of -CH₂-, -C(H)(R^{L})-, -N(H)-, - N(R^{L})-, and -O-; Z is H; and subscript q is 0, 1, or 2.

In one embodiment, the present disclosure provides a compound having structural Formula (Ia), or a pharmaceutically acceptable salt therof, as shown below, 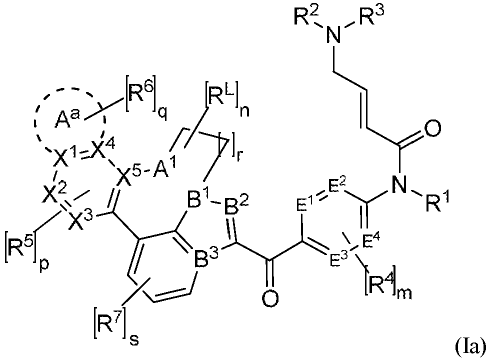 wherein:
R¹ is selected from the group consisting of H and C₁-C₆ alkyl;
R² and R³ are independently selected from the group consisting of:
   (i) H;
   (ii) C₁-C₆ alkyl, wherein the C₁-C₆ alkyl is unsubstituted or substituted by 1 to 2 R^{a} substituents independently selected from the group consisting of halo, hydroxy, C₁-C₃ alkoxy, C₂-C₆ alkenyl, C₂-C₆ alkynyl, C₁-C₃ alkylamino, C₁-C₃ dialkylamino, C₁-C₃ alkylsulfonyl, C₂-C₄ acyl, C₁-C₃ hydroxyalkyl, C₁-C₃ alkoxycarbonyl, C₁-C₃ hydroxycarbonyl, C₃-C₈ cycloalkyl, and 4-to 10-membered mono- or bicyclic heterocycloalkyl, wherein the C₃-C₈ cycloalkyl or the 4-to 10-membered mono- or bicyclic heterocycloalkyl of R^{a} is unsubstituted or substituted by 1 to 2 substituents selected from the group consisting of halo, hydroxy, amino, C₁-C₃ alkyl, C₁-C₃ fluoroalkyl, and C₁-C₃ alkoxy; and
   (iii) a 3- to 8-membered monocyclic, bridged bicyclic, or spirocyclic saturated ring system containing 0 to 2 heteroatom groups selected from the group consisting of N, O, S, S(O), S(O)₂, P, P(O), and P(O)₂, wherein the 3- to 8-membered monocyclic, bridged bicyclic, or spirocyclic saturated ring system is unsubstituted or substituted by 1 to 4 substituents independently selected from the group consisting of halo, hydroxy, C₁-C₃ alkyl, C₁-C₃ fluoroalkyl, C₁-C₃ alkoxy, C₂-C₆ alkenyl, C₂-C₆ alkynyl, C₁-C₃ alkylamino, C₁-C₃ dialkylamino, C₁-C₃ alkylsulfonyl, C₂-C₄ acyl, C₁-C₃ hydroxyalkyl, C₁-C₃ alkoxy(C₁-C₃)alkyl, carboxy and C₁-C₃ alkoxycarbonyl, or alternatively, R² and R³ together with the N atom to which they are attached form a 4- to 8-membered saturated heterocycloalkyl having 0 to 2 additional heteroatoms selected from the group consisting of N, O, and S, and wherein the 4- to 8-membered heterocycloalkyl is unsubstituted or substituted by 1 to 4 substituents independently selected from the group consisting of halo, hydroxy, C₁-C₃ alkyl, C₁-C₃ fluoroalkyl, C₁-C₃ alkoxy, C₁-C₃ hydroxyalkyl, and C₁-C₃ alkoxy(C₁-C₃)alkyl;
at each occurrence R⁴ is independently selected from the group consisting of halo, cyano, C₁-C₃ alkyl, C₁-C₃ fluoroalkyl, C₁-C₃ alkoxy and C₁-C₄ cyanoalkyl;
E¹, E², E³, and E⁴ are independently selected from the group consisting of N and C, with the proviso that no more than three of E¹, E², E³, and E⁴ are N;
one of B¹ and B³ is C and the other is N, and B² is N or C(R^{B}), wherein R^{B} is selected from the group consisting of H, C₁-C₆ alkyl, C₂-C₆ alkenyl, C₂-C₆ alkynyl and C₃-C₆ cycloalkyl;
at each occurrence R^{L} is independently selected from the group consisting of fluoro, hydroxy, C₁-C₆ alkyl, C₁-C₃ alkoxy, C₁-C₃ fluoroalkyl, oxo (=O), and methenyl (=CH₂), and alternatively, two geminally substituted R^{L} substituents together with the carbon atom to which they are attached form a 3- to 6-membered spirocyclic ring containing 0 to 1 heteroatoms selected from the group consisting of N, O, and S;
ring A^{a} is present or absent, and if present, is a 5- to 6-memberered heteroaryl containing 1 to 2 heteroatoms selected from the group consisting of N, O, and S;
X¹, X², X³, X⁴, and X⁵ are independently selected from the group consisting of N and C, with the proviso that no more than two of X¹, X², X³, X⁴, and X⁵ are N;
at each occurrence R⁵ is independently selected from the group consisting of C₁-C₆ alkyl, C₁-C₆ fluoroalkyl, C₁-C₃ alkoxy, C₁-C₃ acyl, halo, hydroxy, amino, cyano, oxo (=O), C₁-C₄ cyanoalkyl, C₁-C₃ hydroxyalkyl, C₁-C₃ alkylamino, C₁-C₃ dialkylamino, C₁-C₃ alkoxy(C₁-C₃)alkyl, and a group of the formula 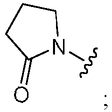 at each occurrence R⁶ is independently selected from the group consisting of C₁-C₃ alkyl, C₁-C₃ fluoroalkyl, C₁-C₃ alkoxy, halo, hydroxy, oxo (=O), cyano, carbamoyl, C₁-C₄ cyanoalkyl, C₁-C₃ hydroxyalkyl, C₁-C₃ alkoxy(C₁-C₃)alkyl, and C₁-C₄ carbamoylalkyl;
at each occurrence R⁷ is independently selected from the group consisting of halo, C₁-C₃ alkyl, C₁-C₃ fluoroalkyl, and C₁-C₃ alkoxy;
A¹ is selected from the group consisting of -CH₂-, -C(H)(R^{L})-, -N(H)-, -N(R^{L})-, and O;
subscript m is 0, 1, 2, or 3;
subscript n is 0, 1, 2, or 3;
subscript p is 0, 1, 2, 3, or 4;
subscript q is 0, 1, or 2;
subscript r is 0, 1, 2, or 3; and
subscript s is 0, 1, 2, or 3.

In another embodiment, the present disclosure provides a compound of Formula (I) or (Ia), wherein the group 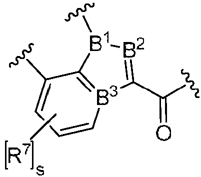 is 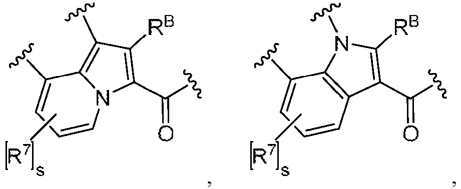 or

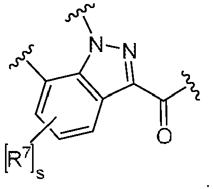

In another embodiment, the present disclosure provides a compound of Formula (I) or (Ia), wherein the group 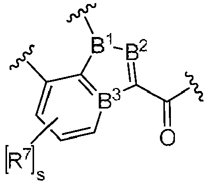 is

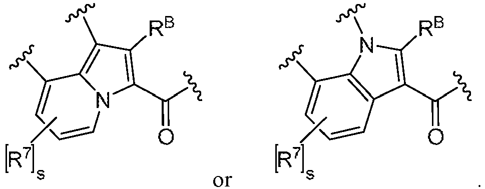

In another embodiment, the present disclosure provides a compound of Formula (I) or (Ia), wherein R^{B} is H or methyl, and the subscript s is 0.

In another embodiment, the present disclosure provides a compound of Formula (I) or (Ia), wherein X¹ is N or C; and X², X³, X⁴, and X⁵ are C.

In another embodiment, the present disclosure provides a compound of Formula (I) or (Ia), wherein the group 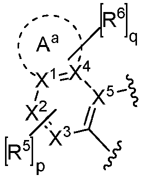 is

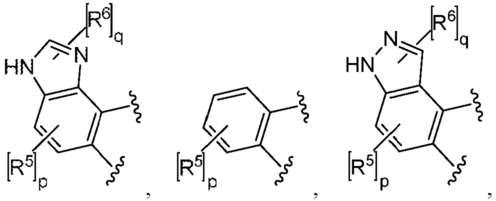

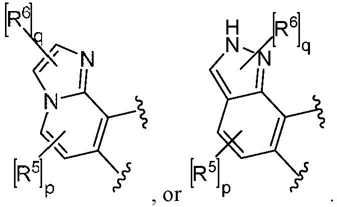

In another embodiment, the present disclosure provides a compound of Formula (I) or (Ia), wherein the group 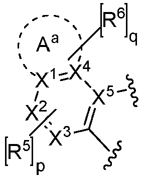 is

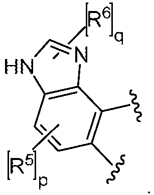

In another embodiment, the present disclosure provides a compound of Formula (I) or (Ia), wherein the group 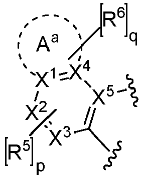 is 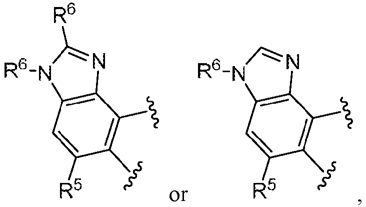 wherein
R⁵ is C₁-C₃ alkyl or C₁-C₃ fluoroalkyl; and R⁶ is C₁-C₃ alkyl, C₁-C₃ fluoroalkyl, or C1-C3 alkoxy(C1-C3)alkyl.

In another embodiment, the present disclosure provides a compound of Formula (I) or (Ia), wherein the group 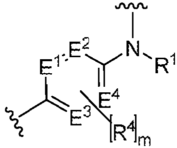 is

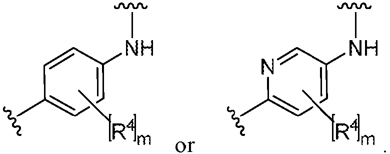

In another embodiment, the present disclosure provides a compound of Formula (I) or (Ia), wherein the group 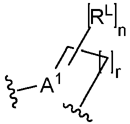 is

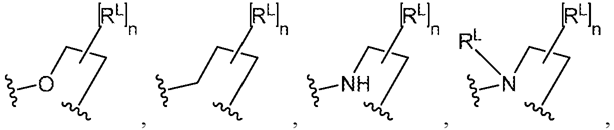

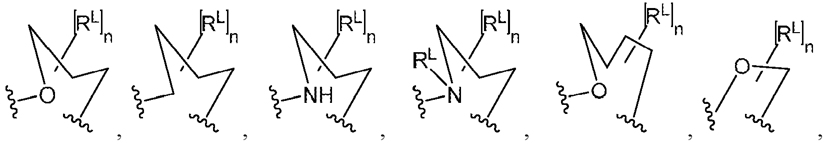

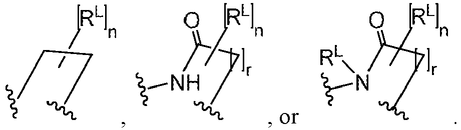

In another embodiment, the present disclosure provides a compound of Formula (I) or (Ia), wherein the group 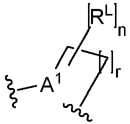 is

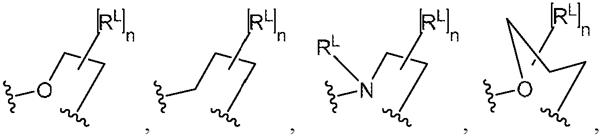

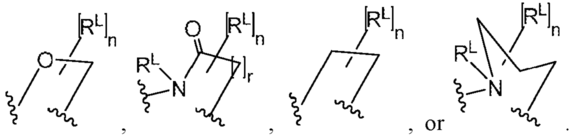

In another embodiment, the present disclosure provides a compound of Formula (I) or (Ia), wherein the group

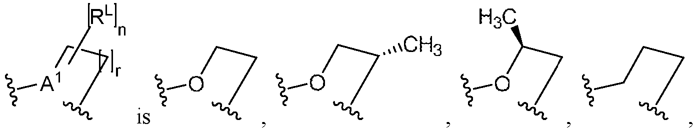

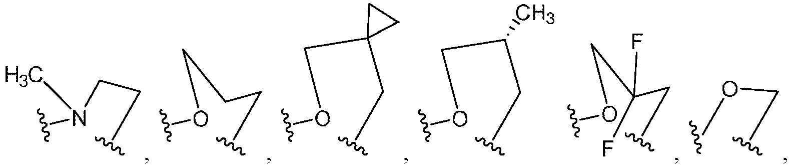

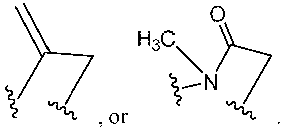

In another embodiment, the present disclosure provides a compound of Formula (I) or (Ia), wherein the group 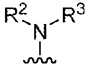 is

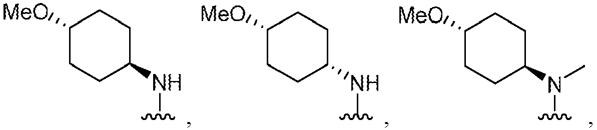

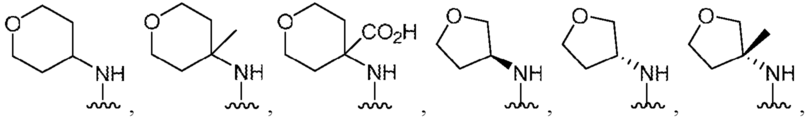

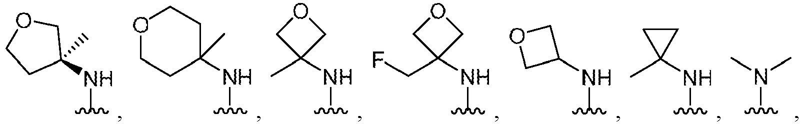

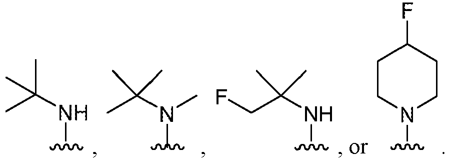

In another embodiment, the present disclosure provides a compound of Formula (I) or (Ia), wherein R² and R³ are independently H, C₁-C₆ alkyl, or C₁-C₆ fluoroalkyl.

In another embodiment, the present disclosure provides a compound of Formula (I) or (Ia), wherein the group 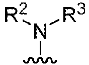 is

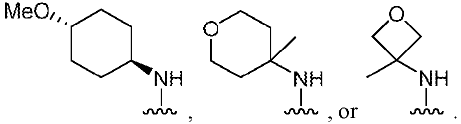

In some embodiments, the present disclosure provides a compound of Formula (I) or (Ia), wherein:
the group 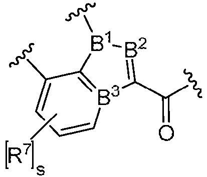 is 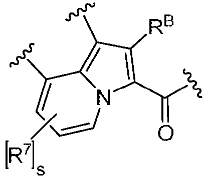 or 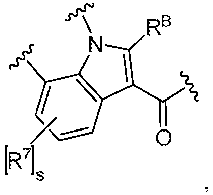 and R^{B} is H and the subscript s is 0;
the group 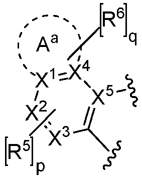 is 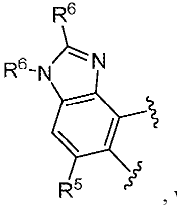 wherein R⁵ and R⁶ are independently C₁-C₃ alkyl or C₁-C₃ fluoroalkyl;
the group 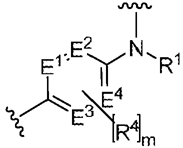 is 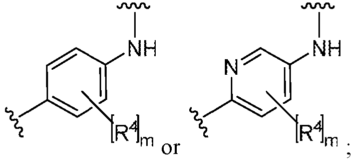
the group 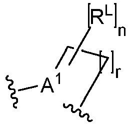 is 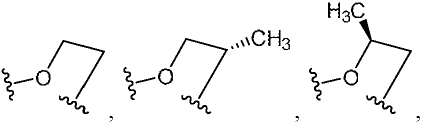 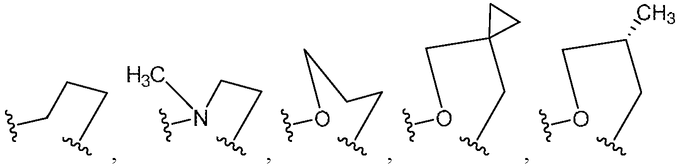 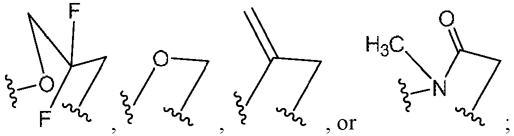 and
the group 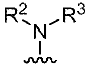 is

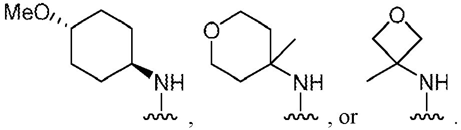

In specific embodiments, the present disclosure provides a compound or a pharmaceutically acceptable salt thereof as described in any one of Examples 1-300 as set forth below.

The present disclosure includes the pharmaceutically acceptable salts of the compounds defined herein, including the pharmaceutically acceptable salts of all structural formulas, embodiments and classes defined herein.

### Definitions

Unless defined otherwise, all technical and scientific terms used herein have the same meaning as is commonly understood by one of skill in the art to which this invention belongs.

As used throughout this disclosure, "a compound of Formula (I)" is to be understood to include "a compound of Formula (I) or a pharmaceutically acceptable salt thereof". Likewise "a compound of Formula (I)", "compound(s) disclosed herein", "compound(s) described herein", "compound(s) of the disclosure", etc., are used interchangeably and include both the compound, as well as a pharmaceutically acceptable salt thereof.

"Alkyl", as well as other groups having the prefix "alk", such as alkoxy, and the like, means carbon chains which may be linear or branched, or combinations thereof, containing the indicated number of carbon atoms. For instance, a C₁-C₆ alkyl means an alkyl group having one (i.e., methyl) up to 6 carbon atoms (i.e., hexyl). In particular embodiments, linear alkyl groups have 1-6 carbon atoms and branched alkyl groups have 3-7 carbon atoms. Examples of alkyl groups include methyl, ethyl, propyl, isopropyl, butyl, *sec-* and *tert-butyl,* pentyl, hexyl, heptyl, octyl, nonyl and the like.

"Alkoxy" and "alkyl-O-" are used interchangeably and refer to an alkyl group linked to oxygen.

"Alkoxyalkyl" means an alkoxy-alkyl group in which the alkoxy and alkyl groups are as previously defined. The bond to the parent moiety is through a carbon atom of the alkyl component. Non-limiting examples of suitable alkyoxyalkyl groups include methoxymethyl and methoxyethyl.

"Alkoxycarbonyl" means a carbonyl having an alkoxy group as previously defined, i.e., -C(O)-alkoxy. Non-limiting examples of suitable alkoxycarbonyl groups include (C₁-C₁₀ alkoxy)carbonyl, such as methoxycarbonyl, ethoxycarbonyl, propoxycarbonyl, isopropoxycarbonyl, butoxycarbonyl, isobutoxycarbonyl, tert-butoxycarbonyl, pentyloxycarbonyl, isopentyloxycarbonyl, hexyloxycarbonyl, and tert-butoxycarbonyl.

"Alkenyl" means an aliphatic hydrocarbon group containing at least one carbon-carbon double bond and which may be straight or branched. Branched means that one or more alkyl groups such as methyl, ethyl or propyl, are attached to a linear alkenyl chain. Non-limiting examples of alkenyl groups include ethenyl, propenyl, n-butenyl, 3-methylbut-2-enyl, and n-pentenyl.

"Alkynyl" means an aliphatic hydrocarbon group containing at least one carbon-carbon triple bond and which may be straight or branched. Non-limiting examples include ethynyl, propynyl, and butynyl.

"Arylcarbonyl" means a carbonyl having an aromatic hydrocarbon ring including phenyl, naphthyl, tetrahydronaphthyl or anthracenyl, i.e., aryl-C(O)-. Non-limiting examples of suitable arylcarbonyl groups include phenylcarbonyl, naphthylcarbonyl, fluorenylcarbonyl, anthrylcarbonyl, biphenylylcarbonyl, tetrahydronaphthylcarbonyl, chromanylcarbonyl, 2,3-dihydro-1,4-dioxanaphthalenylcarbonyl, indanylcarbonyl, and phenanthrylcarbonyl

"Acyl" means an alkylcarbonyl as previously defined or arylcarbonyl as previously defined.

"Aminoalkyl" means -alkyl-NH₂ group in which the alkyl is as previously defined. The bond to the parent moiety is through a carbon atom of the alkyl component. Non-limiting examples of suitable aminoalkyl groups include aminomethyl and aminoethyl. "Alkylamino" means -NH-alkyl group in which the alkyl is as previously defined. The bond to the parent moiety is through the nitrogen of the amino component.

"Alkylsulfonyl" means an alkyl-SO₂ group in which the alkyl group is previously defined, i.e., -S(O)₂-alkyl. The bond to the parent motiety is through the sulfur atom of the sulfonyl moiety. Non-limiting examples of suitable alkylsulfonyl groups include methylsulfonyl and ethylsulfonyl.

"Bicyclic ring system" refers to two joined rings. The rings may be fused, i.e., share two adjacent atoms, bridged, i.e., share more than two adjacent atoms, or "spirocyclic", i.e., share only a single atom.

"Carbamoyl" means a H₂N-C(O)- group, which is the univalent group formed by loss of -OH group of carbamic acid. The bond to the parent group is through the carbon atom of the carbonyl component.

"Carbamoylalkyl" means a carbamoyl-alkyl- group in which the carbamoyl and alkyl groups are previously defined. The bond to the parent motiety is through the alkyl group.

"Cyanoalkyl" means an -alkyl-CN group in which the alkyl is as previously defined. The bond to the parent moiety is through a carbon atom of the alkyl component. Non-limiting examples of suitable cyanoalkyl groups include cyanomethyl and 3-cyanopropyl.

"Cycloalkyl" means a saturated cyclic hydrocarbon radical. In particular embodiments, the cycloalkyl group has 3-12 carbon atoms, forming 1-3 carbocyclic rings that are fused. Examples of cycloalkyl include cyclopropyl, cyclobutyl, cyclopentyl, cyclohexyl, cycloheptyl, adamantyl, and the like.

"Dialkylamino" means an alkylamino as previously defined, wherein the amino atom is substituted by two alkyl substituents, which substitutions can be the same or different, e.g., -N(CH₃)₂ or -N(CH₃)(CH₂CH₃).

"Fluoroalkyl" includes mono-substituted as well as multiple fluoro-substituted alkyl groups, up to perfluoro substituted alkyl. For example, fluoromethyl, 1,1-difluoroethyl, trifluoromethyl or 1,1,1,2,2-pentafluorobutyl are included.

"Geminally" means two substituents, which may be the same or different, substituted on one carbon.

"Halogen" or "halo", unless otherwise indicated, includes fluorine (fluoro), chlorine (chloro), bromine (bromo) and iodine (iodo). In one embodiment, halo is fluoro (-F) or chloro (-Cl).

"Heterocycloalkyl" means a non-aromatic monocyclic, bicyclic or tricyclic ring system comprising about 3 to about 10 ring atoms, preferably about 5 to about 10 ring atoms, in which one or more of the atoms in the ring system is an element other than carbon, for example, nitrogen, oxygen, phosphorus or sulfur, alone or in combination. There are no adjacent oxygen and/or sulfur atoms present in the ring system. In some embodiments, heterocycloalkyls contain about 5 to about 6 ring atoms. The prefix aza, oxa, phospha or thia before the heterocyclyl root name means that at least a nitrogen, oxygen, phosphorus or sulfur atom respectively is present as a ring atom. In some embodiments, the nitrogen or sulfur atom of the heterocycloalkyl can be optionally oxidized to the corresponding N-oxide, S-oxide or S,S-dioxide. Non-limiting examples of suitable monocyclic heterocyclyl rings include piperidyl, pyrrolidinyl, piperazinyl, morpholinyl, thiomorpholinyl, thiazolidinyl, 1,4-dioxanyl, tetrahydrofuranyl, tetrahydrothiophenyl, phosphorinane, phosphinane, 1-oxophosphinan-1-ium and the like.

"Heteroaryl" refers to aromatic monocyclic, bicyclic and tricyclic ring structures in which one or more atoms in the ring, the heteroatom(s), is an element other than carbon. Heteroatoms are typically O, S, or N atoms. Examples of heteroaromatic groups include pyridinyl, pyrimidinyl, pyrrolyl, pyridazinyl, isoxazolyl, thiazolyl, oxazolyl, indolyl, benzoxazolyl, benzothiazolyl, and imidazolyl.

"Hydroxyalkyl" means a HO-alkyl- group in which alkyl is as previously defined. Preferred hydroxyalkyls contain lower alkyl. Non-limiting examples of suitable hydroxyalkyl groups include hydroxymethyl and 2-hydroxyethyl.

"Hydroxycarbonyl" means a HO-C(O)- group.

"Hydroxycycloalkyl" means a cycloalkyl substituted by one or more HO- groups.

"Oxo" means a O= group.

When any variable (e.g., R⁴) occurs more than one time in any constituent or in Formula (I) or (Ia) or other generic formulas herein, its definition on each occurrence is independent of its definition at every other occurrence. Combinations of substituents and/or variables are permissible only if such combinations result in stable compounds. In choosing compounds of the present disclosure, one of ordinary skill in the art will recognize that the various substituents, e.g., R⁴, are to be chosen in conformity with well-known principles of chemical structure connectivity and stability. Unless expressly stated to the contrary, substitution by a named substituent is permitted on any atom in a ring *(e.g.,* aryl, a heteroaryl ring, or a saturated heteroaryl ring) provided such ring substitution is chemically allowed and results in a stable compound. A "stable" compound is a compound which can be prepared and isolated and whose structure and properties remain or can be caused to remain essentially unchanged for a period of time sufficient to allow use of the compound for the purposes described herein (e.g., therapeutic or prophylactic administration to a subject).

The term "substituted" shall be deemed to include multiple degrees of substitution by a named substituent. Where multiple substituent moieties are disclosed or claimed, the substituted compound can be independently substituted by one or more of the disclosed or claimed substituent moieties, singly or plurally. By independently substituted, it is meant that the (two or more) substituents can be the same or different.

Unless expressly depicted or described otherwise, variables depicted in a structural formula with a "floating" bond, such as R⁴, are permitted on any available carbon atom in the ring to which the variable is attached. When a moiety is noted as being "optionally substituted" in Formula (I) or (Ia)or any embodiment thereof, it means that Formula (I) or (Ia)or the embodiment thereof encompasses compounds that contain the noted substituent (or substituents) on the moiety and also compounds that do not contain the noted substituent (or substituents) on the moiety.

The wavy line 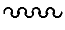 , as used herein, indicates a point of attachment to the rest of the compound.

The compounds of Formula (I) or (Ia) may contain a terminal amine group covalently bonded to the (E)-but-2-enamide moiety and additionally substituted by R² and R³.

In one embodiment, R² and R³ can independently be H. In one embodiment, R² and R³ can independently be a C₁-C₆ alkyl (e.g., Example 79). The C₁-C₆ alkyl is unsubstituted or substituted by 1 to 2 R^{a} substituents independently selected from halo, hydroxy, C₁-C₃ alkoxy, C₂-C₆ alkenyl, C₂-C₆ alkynyl, C₁-C₃ alkylamino, C₁-C₃ dialkylamino, C₁-C₃ alkylsulfonyl, C₂-C₄ acyl, C₁-C₃ hydroxyalkyl, C₁-C₃ alkoxycarbonyl, C₁-C₃ hydroxycarbonyl, oxo (=O), C₃-C₈ cycloalkyl, or 4-to 10-membered mono- or bicyclic heterocycloalkyl. The C₃-C₈ cycloalkyl or 4-to 10-membered mono- or bicyclic heterocycloalkyl of R^{a} is unsubstituted or substituted by 1 to 2 substituents selected from the group consisting of halo, hydroxy, amino, C₁-C₃ alkyl, C1-C3 fluoroalkyl, and C₁-C₃ alkoxy. In one embodiment, R² and R³ can independently be a 3- to 8-membered monocyclic (e.g., Example 51), bridged bicyclic (e.g., Example 73), or spirocyclic (e.g., Example 84) saturated ring system containing 0 to 2 heteroatom groups selected from the group consisting of N, O, S, S(O), S(O)2, P, P(O), and P(O)2. The 3- to 8-membered monocyclic, bridged bicyclic, or spirocyclic saturated ring system is unsubstituted or substituted by 1 to 4 substituents independently selected from the group consisting of halo, hydroxy, C₁-C₃ alkyl, C₁-C₃ fluoroalkyl, C₁-C₃ alkoxy, C₂-C₆ alkenyl, C₂-C₆ alkynyl, C₁-C₃ alkylamino, C₁-C₃ dialkylamino, C₁-C₃ alkylsulfonyl, C2-C4 acyl, C₁-C₃ hydroxyalkyl, C₁-C₃ alkoxy(C₁-C₃)alkyl, carboxy, and C₁-C₃ alkoxycarbonyl.

In one embodiment, R² and R³ together with the N atom to which they are attached form a 4- to 9-membered monocyclic (e.g., Example 114), fused bicyclic (e.g., Example 163), bridged bicyclic (e.g., Example 142), or spirocyclic (e.g., Example 253) saturated ring system containing 0 to 2 additional heteroatoms selected from the group consisting of N, O, and S. the 4- to 9-membered monocyclic, fused bicyclic, bridged bicyclic, or spirocyclic saturated ring system is unsubstituted or substituted by 1 to 4 substituents independently selected from the group consisting of halo, hydroxy, C₁-C₃ alkyl, C₁-C₃ fluoroalkyl, C₁-C₃ alkoxy, C₁-C₄ hydroxyalkyl, C₃-C₅ hydroxycycloalkyl, -(CH₃)₂N(C=O)CH₂-, CH3(C=O)NHCH2-, and C₁-C₃ alkoxy(C₁-C₃)alkyl.

In one embodiment, one of R² and R³ together with the N atom to which it is attached and an adjacent C atom form a 3- to 6-membered monocyclic saturated ring system containing 0 to 2 additional heteroatoms selected from the group consisting of N, O, and S (e.g., Example 250). The 3- to 6- membered monocyclic saturated ring system is unsubstituted or substituted by 1 to 4 substituents independently selected from the group consisting of halo, hydroxy, C₁-C₃ alkyl, C₁-C₃ fluoroalkyl, C₁-C₃ alkoxy, C₁-C₃ hydroxyalkyl, and C₁-C₃ alkoxy(C₁-C₃)alkyl.

The compounds of Formula (I) or (Ia) may contain one or more asymmetric centers and can thus occur as racemates and racemic mixtures, single enantiomers, diastereoisomeric mixtures and individual diastereoisomers. Centers of asymmetry that are present in the compounds of Formula (I) or (Ia) can all independently of one another have S configuration or R configuration. The compounds of Formula (I) or (Ia) include all possible enantiomers and diastereomers and mixtures of two or more stereoisomers, for example, mixtures of enantiomers and/or diastereomers, in all ratios. Thus, enantiomers are a subject of the disclosure in enantiomerically pure form, both as levorotatory and as dextrorotatory antipodes, in the form of racemates and in the form of mixtures of the two enantiomers in all ratios. In the case of a cis/trans isomerism, the disclosure includes both the cis form and the trans form as well as mixtures of these forms in all ratios. The present disclosure is meant to comprehend all such stereoisomeric forms of the compounds of Formula (I) or (Ia). Where a structural formula or chemical name specifies a particular configuration at a stereocenter, the enantiomer or stereoisomer of the compound resulting from that specified stereocenter is intended. Where a structural formula of the compounds of Formula (I) or (Ia) indicates a straight line at a chiral center, the structural formula includes both the S and R stereoisomers associated with the chiral center and mixtures thereof.

The compounds of Formula (I) or (Ia) may be separated into their individual diastereoisomers by, for example, fractional crystallization from a suitable solvent, for example, methanol or ethyl acetate or a mixture thereof, or via chiral chromatography using an optically active stationary phase. Absolute stereochemistry may be determined by X-ray crystallography of crystalline products or crystalline intermediates which are derivatized, if necessary, with a reagent containing an asymmetric center of known absolute configuration. Vibrational circular dichroism (VCD) may also be used to determine the absolute stereochemistry. Alternatively, any stereoisomer or isomers of the compounds of Formula (I) or (Ia) may be obtained by stereospecific synthesis using optically pure starting materials or reagents of known absolute configuration.

If desired, racemic mixtures of the compounds may be separated so that the individual enantiomers are isolated. The separation can be carried out by methods well known in the art, such as the coupling of a racemic mixture of compounds to an enantiomerically pure compound to form a diastereoisomeric mixture, followed by separation of the individual diastereoisomers by standard methods, such as fractional crystallization or chromatography. The coupling reaction is often the formation of salts using an enantiomerically pure acid or base. The diasteromeric derivatives may then be converted to the pure enantiomers by cleavage of the added chiral residue. The racemic mixture of the compounds can also be separated directly by chromatographic methods utilizing chiral stationary phases, which methods are well known in the art.

The compounds of Formula (I) or (Ia) which contain olefinic double bonds, unless specified otherwise, they are meant to include both E and Z geometric isomers.

Some of the compounds described herein may exist as tautomers which have different points of attachment of hydrogen accompanied by one or more double bond shifts. For example, a ketone and its enol form are keto-enol tautomers. The individual tautomers as well as mixtures thereof are encompassed by the compounds of Formula (I).

Some of the compounds of Formula (I) or (Ia) described herein may exist as atropisomers when the rotational energy barrier around a single bond is sufficiently high to prevent free rotation at a given temperature, thus allowing isolation of individual conformers with distinct properties. A typical example of stable atropisomers are exemplified by Examples 1-1 and 1-2 (described below), which can be resolved by chiral chromatography separation. The individual atropisomers as well as mixtures thereof are encompassed with compounds of Formula (I) or (Ia) of the present disclosure. When resolved, individual atropisomers can be designated by established conventions such as those specified by the International Union of Pure Applied Chemistry (IUPAC) 2013 Recommendations.

In the compounds of Formula (I), the atoms may exhibit their natural isotopic abundances, or one or more of the atoms may be artificially enriched in a particular isotope having the same atomic number, but an atomic mass or mass number different from the atomic mass or mass number predominantly found in nature. The present disclosure as described and claimed herein is meant to include all suitable isotopic variations of the compounds of Formula (I) or (Ia) and embodiments thereof. For example, different isotopic forms of hydrogen (H) include protium (¹H) and deuterium (²H, also denoted herein as D). Protium is the predominant hydrogen isotope found in nature. Enriching for deuterium may afford certain therapeutic advantages, such as increasing *in vivo* half-life or reducing dosage requirements or may provide a compound useful as a standard for characterization of biological samples. Isotopically-enriched compounds can be prepared without undue experimentation by conventional techniques well known to those skilled in the art or by processes analogous to those described in the Schemes and Examples herein using appropriate isotopically-enriched reagents and/or intermediates.

The term "pharmaceutically acceptable salts" refers to salts prepared from pharmaceutically acceptable non-toxic bases or acids. When a compound of Formula (I) or (Ia) is acidic, its corresponding salt can be conveniently prepared from pharmaceutically acceptable non-toxic bases, including inorganic bases and organic bases. Salts derived from such inorganic bases include aluminum, ammonium, calcium, copper (ic and ous), ferric, ferrous, lithium, magnesium, manganese (ic and ous), potassium, sodium, zinc and the like salts. Preferred are the ammonium, calcium, magnesium, potassium and sodium salts. Salts prepared from pharmaceutically acceptable organic non-toxic bases include salts of primary, secondary, and tertiary amines derived from both naturally occurring and synthetic sources. Pharmaceutically acceptable organic non-toxic bases from which salts can be formed include, for example, arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, dicyclohexylamine, lysine, methylglucamine, morpholine, piperazine, piperidine, polyamine resins, procaine, purines, theobromine, triethylamine, trimethylamine, tripropylamine, tromethamine and the like.

When a compound of Formula (I) or (Ia) is basic, its corresponding salt can be conveniently prepared from pharmaceutically acceptable non-toxic inorganic and organic acids. Such acids include, for example, acetic, benzene sulfonic, benzoic, camphorsulfonic, citric, ethanesulfonic, fumaric, gluconic, glutamic, hydrobromic, hydrochloric, isethionic, lactic, maleic, malic, mandelic, methane sulfonic, mucic, nitric, pamoic, pantothenic, phosphoric, succinic, sulfuric, tartaric, p-toluenesulfonic acid and the like. Preferred are citric, hydrobromic, hydrochloric, maleic, phosphoric, sulfuric, and tartaric acids. If a compound of Formula (I) or (Ia) simultaneously contain acidic and basic groups in the molecule, the disclosure also includes, in addition to the salt forms mentioned, inner salts or betaines (zwitterions). Salts can be obtained from the compounds of Formula (I) or (Ia) by customary methods which are known to the person skilled in the art, for example, by combination with an organic or inorganic acid or base in a solvent or dispersant, or by anion exchange or cation exchange from other salts. The present disclosure also includes all salts of the compounds of Formula (I) or (Ia) which, owing to low physiological compatibility, are not directly suitable for use in pharmaceuticals but which can be used, for example, as intermediates for chemical reactions or for the preparation of pharmaceutically acceptable salts.

Furthermore, the compounds of Formula (I) or (Ia) may exist in amorphous form and/or one or more crystalline forms, and as such all amorphous and crystalline forms and mixtures thereof of the compounds of Formula (I) or (Ia), including the Examples, are intended to be included within the scope of the present disclosure. In addition, some of the compounds of Formula (I) or (Ia) may form solvates with water *(i.e.,* a hydrate) or common organic solvents such as but not limited to ethyl acetate. Such solvates and hydrates, particularly the pharmaceutically acceptable solvates and hydrates, of the instant compounds are likewise encompassed within the scope of this disclosure, along with unsolvated and anhydrous forms.

Any pharmaceutically acceptable pro-drug modification of a compound of Formula (I) or (Ia) which results in conversion *in vivo* to a compound within the scope of this disclosure is also within the scope of this disclosure.

The terms "therapeutically effective (or efficacious ) amount" and similar descriptions such as "an amount efficacious for treatment" or "an effective dose" are intended to mean that amount of a compound of Formula (I) or (Ia) that will elicit the biological or medical response of a tissue, a system, animal or human that is being sought by a researcher, veterinarian, medical doctor or other clinician. In a preferred embodiment, the term "therapeutically effective amount" means an amount of a compound of Formula (I) or (Ia) that alleviates at least one clinical symptom in a human patient. The terms "prophylactically effective (or efficacious) amount" and similar descriptions such as "an amount efficacious for prevention" are intended to mean that amount of a compound of Formula (I) or (Ia) that will prevent or reduce the risk of occurrence of the biological or medical event that is sought to be prevented in a tissue, a system, animal or human by a researcher, veterinarian, medical doctor or other clinician.

### Dosages of the compounds of Formula (I) or (Ia)

The dosage regimen utilizing a compound of Formula (I) or (Ia) is selected in accordance with a variety of factors including type, species, age, weight, sex and medical condition of the patient; the severity of the condition to be treated; the potency of the compound chosen to be administered; the route of administration; and the renal and hepatic function of the patient. A consideration of these factors is well within the purview of the ordinarily skilled clinician for the purpose of determining the therapeutically effective or prophylactically effective dosage amount needed to prevent, counter, or arrest the progress of the condition. It is understood that a specific daily dosage amount can simultaneously be both a therapeutically effective amount, e.g., for treatment of an oncological condition, and a prophylactically effective amount, e.g., for prevention of an oncological condition.

While individual needs vary, determination of optimal ranges of effective amounts of the compounds of Formula (I) or (Ia) is within the skill of the art. For administration to a human in, for example, the curative or prophylactic treatment of the conditions and disorders identified herein, the typical dosages of the compounds of Formula (I) or (Ia) can be about 0.05 mg/kg/day to about 50 mg/kg/day, or at least 0.05 mg/kg, or at least 0.08 mg/kg, or at least 0.1 mg/kg, or at least 0.2 mg/kg, or at least 0.3 mg/kg, or at least 0.4 mg/kg, or at least 0.5 mg/kg, and any amount therebetween, to about 50 mg/kg or less, or about 40 mg/kg or less, or about 30 mg/kg or less, or about 20 mg/kg or less, or about 10 mg/kg or less and any amount therebetween, which can be, for example, about 2.5 mg/day (0.5 mg/kg x 5 kg) to about 5000 mg/day (50 mg/kg x 100 kg). For example, dosages of the compounds can be about 0.1 mg/kg/day to about 50 mg/kg/day, or about 0.05 mg/kg/day to about 10 mg/kg/day, or about 0.05 mg/kg/day to about 5 mg/kg/day, or about 0.05 mg/kg/day to about 3 mg/kg/day, or about 0.07 mg/kg/day to about 3 mg/kg/day, or about 0.09 mg/kg/day to about 3 mg/kg/day, or about 0.05 mg/kg/day to about 0.1 mg/kg/day, or about 0.1 mg/kg/day to about 1 mg/kg/day, or about 1 mg/kg/day to about 10 mg/kg/day, or about 1 mg/kg/day to about 5 mg/kg/day, or about 1 mg/kg/day to about 3 mg/kg/day, or about 3 mg/day to about 500 mg/day, or about 5 mg/day to about 250 mg/day, or about 10 mg/day to about 100 mg/day, or about 3 mg/day to about 10 mg/day, or about 100 mg/day to about 250 mg/day. Such doses may be administered in a single dose or may be divided into multiple doses.

### Pharmaceutical Compositions

The compounds of Formula (I) or (Ia) and their pharmaceutically acceptable salts can be administered to animals, preferably to mammals, and in particular to humans, as pharmaceuticals by themselves, in mixtures with one another or in the form of pharmaceutical compositions. The term "subject" or "patient" includes animals, preferably mammals and especially humans, who use the instant active agents for the prevention or treatment of a medical condition. Administering of the drug to the subject includes both self-administration and administration to the patient by another person. The subject may be in need of, or desire, treatment for an existing disease or medical condition, or may be in need of or desire prophylactic treatment to prevent or reduce the risk of occurrence of said disease or medical condition. As used herein, a subject "in need" of treatment of an existing condition or of prophylactic treatment encompasses both a determination of need by a medical professional as well as the desire of a patient for such treatment.

The present disclosure therefore also provides the compounds of Formula (I) or (Ia) and their pharmaceutically acceptable salts for use as pharmaceuticals, their use for modulating the activity of mutant KRAS, HRAS and/or NRAS proteins and in particular their use in the therapy and prophylaxis of the below-mentioned diseases or disorders as well as their use for preparing medicaments for these purposes. In certain embodiments, the compounds of Formula (I) or (Ia) and their pharmaceutically acceptable salts inhibit the KRAS G12C protein.

Furthermore, the present disclosure provides pharmaceutical compositions which comprise as active component an effective dose of at least one compound of Formula (I) or (Ia) and/or a pharmaceutically acceptable salt thereof and a customary pharmaceutically acceptable carrier, *i.e.,* one or more pharmaceutically acceptable carrier substances and/or additives.

Thus, the present disclosure provides, for example, said compound and its pharmaceutically acceptable salts for use as pharmaceutical compositions which comprise as active component an effective dose of at least one compound of Formula (I) or (Ia) and/or a pharmaceutically acceptable salt thereof and a customary pharmaceutically acceptable carrier, and the uses of said compound and/or a pharmaceutically acceptable salt thereof in the therapy or prophylaxis of the below-mentioned diseases or disorders, *e.g.,* cancer, as well as their use for preparing medicaments for these purposes.

The pharmaceutical compositions according to the disclosure can be administered orally, for example, in the form of pills, tablets, lacquered tablets, sugar-coated tablets, granules, hard and soft gelatin capsules, aqueous, alcoholic or oily solutions, syrups, emulsions or suspensions, or rectally, for example, in the form of suppositories. Administration can also be carried out parenterally, for example subcutaneously, intramuscularly or intravenously in the form of solutions for injection or infusion.

Other suitable administration forms are, for example, percutaneous or topical administration, for example, in the form of ointments, tinctures, sprays or transdermal therapeutic systems, or, for example, microcapsules, implants or rods. The preferred administration form depends, for example, on the disease to be treated and on its severity.

The amount of active compound of a compound described herein and/or its pharmaceutically acceptable salts in the pharmaceutical composition normally is from 0.01 to 200 mg, or from 0.1 to 200 mg, or from 1 to 200 mg, per dose, but depending on the type of the pharmaceutical composition, it can also be higher. In some embodiments, the amount of active compound of a compound of Formula (I) or (Ia) and/or its pharmaceutically acceptable salts in the pharmaceutical composition is from 0.01 to 10 mg per dose. The pharmaceutical compositions usually comprise 0.5 to 90 percent by weight of at least one compound of Formula (I) or (Ia) and/or its pharmaceutically acceptable salts. The preparation of the pharmaceutical compositions can be carried out in a manner known *per se.* For this purpose, one or more compounds of Formula (I) or (Ia) and/or their pharmaceutically acceptable salts, together with one or more solid or liquid pharmaceutical carrier substances and/or additives (or auxiliary substances) and, if desired, in combination with other pharmaceutically active compounds having therapeutic or prophylactic action, are brought into a suitable administration form or dosage form which can then be used as a pharmaceutical in human or veterinary medicine.

For the production of pills, tablets, sugar-coated tablets and hard gelatin capsules, it is possible to use, for example, lactose, starch, for example, maize starch, or starch derivatives, talc, stearic acid or its salts, etc. Carriers for soft gelatin capsules and suppositories are, for example, fats, waxes, semisolid and liquid polyols, natural or hardened oils, etc. Suitable carriers for the preparation of solutions, for example, of solutions for injection, or of emulsions or syrups are, for example, water, physiologically acceptable sodium chloride solution, alcohols such as ethanol, glycerol, polyols, sucrose, invert sugar, glucose, mannitol, vegetable oils, etc. It is also possible to lyophilize the compounds of Formula (I) or (Ia) and their pharmaceutically acceptable salts and to use the resulting lyophilisates, for example, for preparing preparations for injection or infusion. Suitable carriers for microcapsules, implants or rods are, for example, copolymers of glycolic acid and lactic acid.

Besides the active compounds and carriers, the pharmaceutical compositions can also contain customary additives, for example, fillers, disintegrants, binders, lubricants, wetting agents, stabilizers, emulsifiers, dispersants, preservatives, sweeteners, colorants, flavorings, aromatizers, thickeners, diluents, buffer substances, solvents, solubilizers, agents for achieving a depot effect, salts for altering the osmotic pressure, coating agents and/or antioxidants.

### Methods of Using the Compounds of Formula (I) or (Ia)

The present application provides a method of inhibiting RAS-mediated cell signaling comprising contacting a cell with a compound of Formula (I) or (Ia) or a pharmaceutically acceptable salt thereof. Inhibition of RAS-mediated signal transduction can be assessed and demonstrated by a wide variety of ways known in the art. Non-limiting examples include (a) a decrease in GTPase activity of RAS; (b) a decrease in GTP binding affinity or an increase in GDP binding affinity; (c) an increase in K_{off} of GTP or a decrease in K_{off} of GDP; (d) a decrease in the levels of signaling transduction molecules downstream in the RAS pathway, such as a decrease in pMEK, pERK, or pAKT levels; and/or (e) a decrease in binding of RAS complex to downstream signaling molecules including but not limited to Raf. Kits and commercially available assays can be utilized for determining one or more of the above.

The present application also provides methods of using the compounds of Formula (I) or (Ia) (or their pharmaceutically acceptable salts) or pharmaceutical compositions containing such compounds to treat disease conditions, including but not limited to, conditions implicated by mutant KRAS, HRAS and/or NRAS proteins (e.g., cancer), and in some embodiments the KRAS G12C mutant.

In some embodiments, a method for treatment of cancer is provided, the method comprising administering a therapeutically effective amount a compound of Formula (I) or (Ia) (or a pharmaceutically acceptable salt thereof) or any of the foregoing pharmaceutical compositions comprising such a compound to a subject in need of such treatment. In some embodiments, the cancer is mediated by a KRAS, HRAS or NRAS mutation, e.g., the KRAS G12C mutation. In various embodiments, the cancer is pancreatic cancer, colorectal cancer or lung cancer. In some embodiments, the cancer is gall bladder cancer, thyroid cancer, or bile duct cancer.

In some embodiments the present disclosure provides a method of treating a disorder in a subject in need thereof, wherein said method comprises determining if the subject has a KRAS, HRAS or NRAS mutation (e.g., KRAS G12C mutation) and if the subject is determined to have the KRAS, HRAS or NRAS mutation, then administering to the subject a therapeutically effective amount of a compound of Formula (I) or (Ia) or a pharmaceutically acceptable salt thereof.

The disclosed compounds inhibit anchorage-independent cell growth and therefore have the potential to inhibit tumor metastasis. Accordingly, another embodiment of the present disclosure provides a method for inhibiting tumor metastasis, the method comprising administering an effective amount a compound of Formula (I) or (Ia).

KRAS, HRAS or NRAS mutations have also been identified in hematological malignancies (e.g., cancers that affect blood, bone marrow and/or lymph nodes). Accordingly, certain embodiments are directed to administration of the compounds of Formula (I) or (Ia) *(e.g.,* in the form of a pharmaceutical composition) to a subject in need of treatment of a hematological malignancy. Such malignancies include, but are not limited to leukemias and lymphomas. For example, the presently disclosed compounds can be used for treatment of diseases such as acute lymphoblastic leukemia (ALL), acute myelogenous leukemia (AML), chronic lymphocytic leukemia (CLL), small lymphocytic lymphoma (SLL), chronic myelogenous leukemia (CML), acute monocytic leukemia (AMoL) and/ or other leukemias. In other embodiments, the compounds are useful for treatment of lymphomas such as Hodgkins lymphoma or non-Hodgkins lymphoma. In various embodiments, the compounds are useful for treatment of plasma cell malignancies such as multiple myeloma, mantle cell lymphoma, and Waldenstrom's macroglubunemia.

Determining whether a tumor or cancer comprises a KRAS, HRAS or NRAS mutation (e.g., the KRAS G12C mutation) can be undertaken by assessing the nucleotide sequence encoding the KRAS, HRAS or NRAS protein, by assessing the amino acid sequence of the KRAS, HRAS or NRAS protein, or by assessing the characteristics of a putative KRAS, HRAS or NRAS mutant protein. The sequences of wild-type human KRAS, HRAS or NRAS are known in the art.

Methods for detecting a mutation in a KRAS, HRAS or NRAS nucleotide sequence are also known by those of skill in the art. These methods include, but are not limited to, polymerase chain reaction-restriction fragment length polymorphism (PCR-RFLP) assays, polymerase chain reaction-single strand conformation polymorphism (PCR-SSCP) assays, real-time PCR assays, PCR sequencing, mutant allele-specific PCR amplification (MASA) assays, direct sequencing, primer extension reactions, electrophoresis, oligonucleotide ligation assays, hybridization assays, TaqMan assays, SNP genotyping assays, high resolution melting assays and microarray analyses. In some embodiments, samples are evaluated for KRAS, HRAS or NRAS mutations (e.g., the KRAS G12C mutation) by real-time PCR. In real-time PCR, fluorescent probes specific for the KRAS, HRAS or NRAS mutation are used. When a mutation is present, the probe binds and fluorescence is detected. In some embodiments, the KRAS, HRAS or NRAS mutation is identified using a direct sequencing method of specific regions (e.g., exon 2 and/or exon 3) in the KRAS, HRAS or NRAS gene.

Methods for detecting a mutation in a KRAS, HRAS or NRAS protein (e.g., the KRAS G12C mutation) are known by those of skill in the art. These methods include, but are not limited to, detection of a KRAS, HRAS or NRAS mutant using a binding agent *(e.g.,* an antibody) specific for the mutant protein, protein electrophoresis and Western blotting, and direct peptide sequencing.

A number of tissue samples can be assessed for determining whether a tumor or cancer comprises a KRAS, HRAS or NRAS mutation (e.g., the KRAS G12C mutation). In some embodiments, the sample is taken from a subject having a tumor or cancer. In some embodiments, the sample is a fresh tumor/cancer sample. In some embodiments, the sample is a frozen tumor/cancer sample. In some embodiments, the sample is a formalin-fixed paraffin-embedded sample. In some embodiments, the sample is a circulating tumor cell (CTC) sample. In some embodiments, the sample is processed to a cell lysate. In some embodiments, the sample is processed to DNA or RNA.

The present application also provides a method of treating a hyperproliferative disorder comprising administering a therapeutically effective amount of a compound of Formula (I) or (Ia), or a pharmaceutically acceptable salt thereof to a subject in need thereof. In some embodiments, said method relates to the treatment of a subject who suffers from a cancer such as acute myeloid leukemia, cancer in adolescents, adrenocortical carcinoma childhood, AIDS- related cancers (e.g., Lymphoma and Kaposi's Sarcoma), anal cancer, appendix cancer, astrocytomas, atypical teratoid, basal cell carcinoma, bile duct cancer, bladder cancer, bone cancer, brain stem glioma, brain tumor, breast cancer, bronchial tumors, Burkitt lymphoma, carcinoid tumor, atypical teratoid, embryonal tumors, germ cell tumor, primary lymphoma, cervical cancer, childhood cancers, chordoma, cardiac tumors, chronic lymphocytic leukemia (CLL), chronic myelogenous leukemia (CML), chronic myleoproliferative disorders, colon cancer, colorectal cancer, craniopharyngioma, cutaneous T-cell lymphoma, extrahepatic ductal carcinoma in situ (DCIS), embryonal tumors, CNS cancer, endometrial cancer, ependymoma, esophageal cancer, esthesioneuroblastoma, Ewing sarcoma, extracranial germ cell tumor, extragonadal germ cell tumor, eye cancer, fibrous histiocytoma of bone, gall bladder cancer, gastric cancer, gastrointestinal carcinoid tumor, gastrointestinal stromal tumors (GIST), germ cell tumor, gestational trophoblastic tumor, hairy cell leukemia, head and neck cancer, heart cancer, liver cancer, Hodgkin lymphoma, hypopharyngeal cancer, intraocular melanoma, islet cell tumors, pancreatic neuroendocrine tumors, kidney cancer, laryngeal cancer, lip and oral cavity cancer, liver cancer, lobular carcinoma in situ (LCIS), lung cancer, lymphoma, metastatic squamous neck cancer with occult primary, midline tract carcinoma, mouth cancer; multiple endocrine neoplasia syndromes, multiple myeloma/plasma cell neoplasm, mycosis fungoides, myelodysplasia syndromes, myelodysplastic/myeloproliferative neoplasms, multiple myeloma, Merkel cell carcinoma, malignant mesothelioma, malignant fibrous histiocytoma of bone and osteosarcoma, nasal cavity and paranasal sinus cancer, nasopharyngeal cancer, neuroblastoma, Non-Hodgkin lymphoma, non-small cell lung cancer (NSCLC), oral cancer, lip and oral cavity cancer, oropharyngeal cancer, ovarian cancer, pancreatic cancer, papillomatosis, paraganglioma, paranasal sinus and nasal cavity cancer, parathyroid cancer, penile cancer, pharyngeal cancer, pleuropulmonary blastoma, primary central nervous system (CNS) lymphoma, prostate cancer, rectal cancer, transitional cell cancer, retinoblastoma, rhabdomyosarcoma, salivary gland cancer, skin cancer, stomach (gastric) cancer, small cell lung cancer; small intestine cancer, soft tissue sarcoma, T-Cell lymphoma, testicular cancer, throat cancer, thymoma and thymic carcinoma, thyroid cancer, transitional cell cancer of the renal pelvis and ureter, trophoblastic tumor, unusual cancers of childhood, urethral cancer, uterine sarcoma, vaginal cancer, vulvar cancer, or viral-induced cancer. In some embodiments, said method relates to the treatment of a non-cancerous hyperproliferative disorder such as benign hyperplasia of the skin *(e.g.,* psoriasis), restenosis, or prostate *(e.g.,* benign prostatic hypertrophy (BPH)).

In some embodiments, the methods for treatment are directed to treating lung cancers, and the methods comprise administering a therapeutically effective amount of the compounds of Formula (I) or (Ia) (or pharmaceutical composition comprising such compounds) to a subject in need thereof. In certain embodiments, the lung cancer is a non-small cell lung carcinoma (NSCLC), for example, adenocarcinoma, squamous-cell lung carcinoma or large-cell lung carcinoma. In some embodiments, the lung cancer is a small cell lung carcinoma. Other lung cancers which the compounds of Formula (I) or (Ia) may provide therapeutic benefit for include, but are not limited to, glandular tumors, carcinoid tumors and undifferentiated carcinomas.

The present disclosure also provides methods of modulating a mutant KRAS, HRAS or NRAS protein activity (e.g., activity resulting from the KRAS G12C mutation) by contacting the protein with an effective amount of a compound of Formula (I) or (Ia). Modulation can be inhibiting or activating protein activity. In some embodiments, the present disclosure provides methods of inhibiting protein activity by contacting the mutant KRAS, HRAS or NRAS protein (e.g., KRAS G12C mutant) with an effective amount of a compound of Formula (I) or (Ia) in solution. In some embodiments, the present disclosure provides methods of inhibiting the mutant KRAS, HRAS or NRAS protein activity by contacting a cell, tissue, or organ that expresses the protein of interest. In some embodiments, the disclosure provides methods of inhibiting protein activity in subjects including, but not limited to, rodents and mammals (e.g., humans) by administering into the subjects an effective amount of a compound of Formula (I) or (Ia).

### Combination Therapies

One or more additional pharmacologically active agents may be administered in combination with a compound of Formula (I) or (Ia) (or a pharmaceutically acceptable salt thereof). An additional active agent (or agents) is intended to mean a pharmaceutically active agent (or agents) that is active in the body, including pro-drugs that convert to pharmaceutically active form after administration, which are different from the compound of Formula (I) or (Ia). The additional active agents also include free-acid, free-base and pharmaceutically acceptable salts of said additional active agents. Generally, any suitable additional active agent or agents, including chemotherapeutic agents or therapeutic antibodies, may be used in any combination with the compound of Formula (I) or (Ia) in a single dosage formulation (e.g., a fixed dose drug combination), or in one or more separate dosage formulations which allows for concurrent or sequential administration of the active agents (co-administration of the separate active agents) to subjects. In addition, the compounds of Formula (I) or (Ia) (or pharmaceutically acceptable salts thereof) can be administered in combination with radiation therapy, hormone therapy, surgery or immunotherapy.

The present application also provides methods for combination therapies in which the additional active agent is known to modulate other pathways, or other components of the same pathway, or even overlapping sets of target enzymes which are used in combination with a compound of Formula (I) or (Ia), or a pharmaceutically acceptable salt thereof. In one embodiment, such therapy includes, but is not limited to, the combination of one or more compounds of Formula (I) or (Ia) with chemotherapeutic agents, immunotherapeutic agents, hormonal and anti-hormonal agents, targeted therapy agents, and anti-angiogenesis agents, to provide a synergistic or additive therapeutic effect. In another embodiment, such therapy includes radiation treatment to provide a synergistic or additive therapeutic effect.

Examples of additional active agents (i.e., additional anti-cancer agents) include chemotherapeutic agents (e.g., cytotoxic agents), immunotherapeutic agents, hormonal and anti-hormonal agents, targeted therapy agents, and anti-angiogenesis agents. Many anti-cancer agents can be classified within one or more of these groups. While certain anti-cancer agents have been categorized within a specific group(s) or subgroup(s) herein, many of these agents can also be listed within one or more other group(s) or subgroup(s), as would be presently understood in the art. It is to be understood that the classification herein of a particular agent into a particular group is not intended to be limiting. Many anti-cancer agents are presently known in the art and can be used in combination with the compounds of the present disclosure.

Further, an agent can be an agonist, antagonist, allosteric modulator, toxin or, more generally, may act to inhibit or stimulate its target (e.g., receptor or enzyme activation or inhibition). For example, suitable for use are one or more agents (e.g., antibodies, antigen binding regions, or soluble receptors) that specifically bind and inhibit the activity of growth factors, such as antagonists of hepatocyte growth factor (HGF, also known as Scatter Factor), and antibodies or antigen binding regions that specifically bind its receptor "c-met".

In an embodiment, the additional anti-cancer agent is a chemotherapeutic agent, an immunotherapeutic agent, a hormonal agent, an anti-hormonal agent, a targeted therapy agent, or an anti-angiogenesis agent (or angiogenesis inhibitor). In an embodiment, the additional anti-cancer agent is selected from the group consisting of a chemotherapeutic agent, a mitotic inhibitor, a plant alkaloid, an alkylating agent, an antimetabolite, a platinum analog, an enzyme, a topoisomerase inhibitor, a retinoid, an aziridine, an antibiotic, a hormonal agent, an anti-hormonal agent, an anti-estrogen, an anti-androgen, an anti-adrenal, an androgen, a targeted therapy agent, an immunotherapeutic agent, a biological response modifier, a cytokine inhibitor, a tumor vaccine, a monoclonal antibody, an immune checkpoint inhibitor, an anti-PD-1 agent, an anti-PD-L1 agent, a colony-stimulating factor, an immunomodulator, an immunomodulatory imide (IMiD), an anti-CTLA4 agent, an anti-LAGl agent, an anti-OX40 agent, a GITR agonist, a CAR-T cell, a BiTE, a signal transduction inhibitor, a growth factor inhibitor, a tyrosine kinase inhibitor, an EGFR inhibitor, a histone deacetylase (HDAC) inhibitor, a proteasome inhibitor, a cell-cycle inhibitor, an anti-angiogenesis agent, a matrix-metalloproteinase (MMP) inhibitor, a hepatocyte growth factor inhibitor, a TOR inhibitor, a KDR inhibitor, a VEGF inhibitor, a HIF-1α inhibitor, a HIF-2α inhibitor, a fibroblast growth factor (FGF) inhibitor, a RAF inhibitor, a MEK inhibitor, an ERK inhibitor, a PI3K inhibitor, an AKT inhibitor, an MCL-1 inhibitor, a BCL-2 inhibitor, an SHP2 inhibitor, a HER-2 inhibitor, a BRAF-inhibitor, a gene expression modulator, an autophagy inhibitor, an apoptosis inducer, an antiproliferative agent, and a glycolysis inhibitor.

In one embodiment, the additional anti-cancer agent(s) is a chemotherapeutic agent. Non-limiting examples of chemotherapeutic agents include mitotic inhibitors and plant alkaloids, alkylating agents, anti-metabolites, platinum analogs, enzymes, topoisomerase inhibitors, retinoids, aziridines, and antibiotics.

Non-limiting examples of mitotic inhibitors and plant alkaloids include taxanes such as cabazitaxel, docetaxel, larotaxel, ortataxel, paclitaxel, and tesetaxel; demecolcine; epothilone; eribulin; etoposide (VP- 16); etoposide phosphate; navelbine; noscapine; teniposide; thaliblastine; vinblastine; vincristine; vindesine; vinflunine; and vinorelbine

Non-limiting examples of alkylating agents include nitrogen mustards such as chlorambucil, chlornaphazine, cholophosphamide, cytophosphane, estramustine, ifosfamide, mannomustine, mechlorethamine, mechlorethamine oxide hydrochloride, melphalan, novembichin, phenesterine, prednimustine, tris(2-chloroethyl)amine, trofosfamide, and uracil mustard; alkyl sulfonates such as busulfan, improsulfan, and piposulfan; nitrosoureas such as carmustine, chlorozotocin, fotemustine, lomustine, nimustine, ranimustine, streptozotocin, and TA-07; ethylenimines and methylamelamines such as altretamine, thiotepa, triethylenemelamine, triethylenethiophosphaoramide, trietylenephosphoramide, and trimethylolomelamine; ambamustine; bendamustine; dacarbazine; etoglucid; irofulven; mafosfamide; mitobronitol; mitolactol; pipobroman; procarbazine; temozolomide; treosulfan; and triaziquone.

Non-limiting examples of anti-metabolites include folic acid analogues such as aminopterin, denopterin, edatrexate, methotrexate, pteropterin, raltitrexed, and trimetrexate; purine analogs such as 6-mercaptopurine, 6-thioguanine, fludarabine, forodesine, thiamiprine, and thioguanine; pyrimidine analogs such as 5-fluorouracil (5-FU), 6-azauridine, ancitabine, azacytidine, capecitabine, carmofur, cytarabine, decitabine, dideoxyuridine, doxifiuridine, doxifluridine, enocitabine, floxuridine, galocitabine, gemcitabine, and sapacitabine; 3-aminopyridine-2-carboxaldehyde thiosemicarbazone; broxuridine; cladribine; cyclophosphamide; cytarabine; emitefur; hydroxyurea; mercaptopurine; nelarabine; pemetrexed; pentostatin; tegafur; and troxacitabine.

Non-limiting examples of platinum analogs include carboplatin, cisplatin, dicycloplatin, heptaplatin, lobaplatin, nedaplatin, oxaliplatin, satraplatin, and triplatin tetranitrate.

Non-limiting examples of enzymes include asparaginase and pegaspargase.

Non-limiting examples of topoisomerase inhibitors include acridine carboxamide, amonafide, amsacrine, belotecan, elliptinium acetate, exatecan, indolocarbazole, irinotecan, lurtotecan, mitoxantrone, razoxane, rubitecan, SN-38, sobuzoxane, and topotecan.

Non-limiting examples of retinoids include alitretinoin, bexarotene, fenretinide, isotretinoin, liarozole, RII retinamide, and tretinoin.

Non-limiting examples of aziridines include benzodopa, carboquone, meturedopa, and uredopa.

Non-limiting examples of antibiotics include intercalating antibiotics; anthracenediones; anthracycline antibiotics such as aclarubicin, amrubicin, daunomycin, daunorubicin, doxorubicin, epirubicin, idarubicin, menogaril, nogalamycin, pirarubicin, and valrubicin; 6-diazo-5-oxo- L-norleucine; aclacinomysins; actinomycin; authramycin; azaserine; bleomycins; cactinomycin; calicheamicin; carabicin; carminomycin; carzinophilin; chromomycins; dactinomycin; detorubicin; esorubicin; esperamicins; geldanamycin; marcellomycin; mitomycins; mitomycin C; mycophenolic acid; olivomycins; novantrone; peplomycin; porfiromycin; potfiromycin; puromycin; quelamycin; rebeccamycin; rodorubicin; streptonigrin; streptozocin; *tanespimycin;* tubercidin; ubenimex; zinostatin; zinostatin stimalamer; and zorubicin.

In one embodiment, the additional anti-cancer agent(s) is a hormonal and/or anti-hormonal agent (i.e., hormone therapy). Non-limiting examples of hormonal and anti-hormonal agents include anti-androgens such as abiraterone, apalutamide, bicalutamide, darolutamide, enzalutamide, flutamide, goserelin, leuprolide, and nilutamide; antiestrogens such as 4- hydroxy tamoxifen, aromatase inhibiting 4(5)-imidazoles, EM-800, fosfestrol, fulvestrant, keoxifene, LY 117018, onapristone, raloxifene, tamoxifen, toremifene, and trioxifene; anti-adrenals such as aminoglutethimide, dexaminoglutethimide, mitotane, and trilostane; androgens such as calusterone, dromostanolone propionate, epitiostanol, mepitiostane, and testolactone; abarelix; anastrozole; cetrorelix; deslorelin; exemestane; fadrozole; finasteride; formestane; histrelin (RL 0903); human chorionic gonadotropin; lanreotide; LDI 200 (Milkhaus); letrozole; leuprorelin; mifepristone; nafarelin; nafoxidine; osaterone; prednisone; thyrotropin alfa; and triptorelin.

In one embodiment, the additional anti-cancer agent(s) is an immunotherapeutic agent (i.e., immunotherapy). Non-limiting examples of immunotherapeutic agents include biological response modifiers, cytokine inhibitors, tumor vaccines, monoclonal antibodies, immune checkpoint inhibitors, colony-stimulating factors, and immunomodulators.

Non-limiting examples of biological response modifiers, including cytokine inhibitors (cytokines) such as interferons and interleukins, include interferon alfa/interferon alpha such as interferon alfa-2, interferon alfa-2a, interferon alfa-2b, interferon alfa-nl, interferon alfa-n3, interferon alfacon-1, peginterferon alfa-2a, peginterferon alfa-2b, and leukocyte alpha interferon; interferon beta such as interferon beta-1a, and interferon beta-1b; interferon gamma such as natural interferon gamma-1a, and interferon gamma-1b; aldesleukin; interleukin-1 beta; interleukin-2; oprelvekin; sonermin; tasonermin; and virulizin.

Non-limiting examples of tumor vaccines include APC 8015, AVICINE, bladder cancer vaccine, cancer vaccine (Biomira), gastrin 17 immunogen, Maruyama vaccine, melanoma lysate vaccine, melanoma oncolysate vaccine (New York Medical College), melanoma vaccine (New York University), melanoma vaccine (Sloan Kettering Institute), TICE^{®} BCG (Bacillus Calmette-Guerin), and viral melanoma cell lysates vaccine (Royal Newcastle Hospital).

Non-limiting examples of monoclonal antibodies include abagovomab, adecatumumab, aflibercept, alemtuzumab, blinatumomab, brentuximab vedotin, CA 125 MAb (Biomira), cancer MAb (Japan Pharmaceutical Development), daclizumab, daratumumab, denosumab, edrecolomab, gemtuzumab zogamicin, HER- 2 and Fc MAb (Medarex), ibritumomab tiuxetan, idiotypic 105AD7 MAb (CRC Technology), idiotypic CEA MAb (Trilex), ipilimumab, lintuzumab, LYM-1 -iodine 131 MAb (Techni clone), mitumomab, moxetumomab, ofatumumab, polymorphic epithelial mucin-yttrium 90 MAb (Antisoma), ranibizumab, rituximab, and trastuzumab.

Non-limiting examples of immune checkpoint inhibitors include anti-PD-1 agents or antibodies such as cemiplimab, nivolumab, and pembrolizumab; anti-PD-L1 agents or antibodies such as atezolizumab, avelumab, and durvalumab; anti-CTLA-4 agents or antibodies such as ipilumumab; anti-LAG1 agents; and anti-OX40 agents.

Non-limiting examples of colony-stimulating factors include darbepoetin alfa, epoetin alfa, epoetin beta, filgrastim, granulocyte macrophage colony stimulating factor, lenograstim, leridistim, mirimostim, molgramostim, nartograstim, pegfilgrastim, and sargramostim.

Non-limiting examples of additional immunotherapeutic agents include BiTEs, CAR-T cells, GITR agonists, imiquimod, immunomodulatory imides (IMiDs), mismatched double stranded RNA (Ampligen), resiquimod, SRL 172, and thymalfasin.

In one embodiment, the additional anti-cancer agent(s) is a targeted therapy agent (i.e., targeted therapy). Targeted therapy agents include, for example, monoclonal antibodies and small molecule drugs. Non-limiting examples of targeted therapy agents include signal transduction inhibitors, growth factor inhibitors, tyrosine kinase inhibitors, EGFR inhibitors, histone deacetylase (HDAC) inhibitors, proteasome inhibitors, cell-cycle inhibitors, angiogenesis inhibitors, matrix-metalloproteinase (MMP) inhibitors, hepatocyte growth factor inhibitors, TOR inhibitors, KDR inhibitors, VEGF inhibitors, fibroblast growth factors (FGF) inhibitors, MEK inhibitors, ERK inhibitors, PI3K inhibitors, AKT inhibitors, MCL-1 inhibitors, BCL-2 inhibitors, SHP2 inhibitors, HER-2 inhibitors, BRAF-inhibitors, gene expression modulators, autophagy inhibitors, apoptosis inducers, antiproliferative agents, and glycolysis inhibitors.

Non-limiting examples of signal transduction inhibitors include tyrosine kinase inhibitors, multiple-kinase inhibitors, anlotinib, avapritinib, axitinib, dasatinib, dovitinib, imatinib, lenvatinib, lonidamine, nilotinib, nintedanib, pazopanib, pegvisomant, ponatinib, vandetanib, and EGFR inhibitory agents.

Non-limiting examples of EGFR inhibitory agents include small molecule antagonists of EGFR such as afatinib, brigatinib, erlotinib, gefitinib, lapatinib, and osimertinib; and antibody-based EGFR inhibitors, including any anti-EGFR antibody or antibody fragment that can partially or completely block EGFR activation by its natural ligand. Antibody-based EGFR inhibitory agents may include, for example, those described in Modjtahedi, H., et al., 1993, Br. J. Cancer 67:247-253; Teramoto, T., et al., 1996, Cancer 77:639-645; Goldstein et al, 1995, Clin. Cancer Res. 1 : 1311-1318; Huang, S. M., et al., 1999, Cancer Res. 15:59(8): 1935-40; and Yang, X., et al., 1999, Cancer Res. 59: 1236-1243; monoclonal antibody Mab E7.6.3 (Yang, 1999 supra); Mab C225 (ATCC Accession No. HB-8508), or an antibody or antibody fragment having the binding specificity thereof; specific antisense nucleotide or siRNA; afatinib, cetuximab; matuzumab; necitumumab; nimotuzumab; panitumumab; and zalutumumab.

Non-limiting examples of histone deacetylase (HDAC) inhibitors include belinostat, panobinostat, romidepsin, and vorinostat.

Non-limiting examples of proteasome inhibitors include bortezomib, carfilzomib, ixazomib, marizomib (salinosporamide a), and oprozomib.

Non-limiting examples of cell-cycle inhibitors, including CDK inhibitors, include abemaciclib, alvocidib, palbociclib, and ribociclib.

In one embodiment, the additional anti-cancer agent(s) is an anti-angiogenic agent (or angiogenesis inhibitor) including, but not limited to, matrix-metalloproteinase (MMP) inhibitors; VEGF inhibitors; EGFR inhibitors; TOR inhibitors such as everolimus and temsirolimus; PDGFR kinase inhibitory agents such as crenolanib; HIF-lα inhibitors such as PX 478; HIF-2α inhibitors such as belzutifan and the HIF-2α inhibitors described in WO 2015/035223; fibroblast growth factor (FGF) or FGFR inhibitory agents such as B-FGF and RG 13577; hepatocyte growth factor inhibitors; KDR inhibitors; anti-Ang1 and anti-Ang2 agents; anti-Tie2 kinase inhibitory agents; Tek antagonists (US 2003/0162712; US 6,413,932); anti-TWEAK agents (US 6,727,225); ADAM distintegrin domain to antagonize the binding of integrin to its ligands (US 2002/0042368); anti-eph receptor and/or anti-ephrin antibodies or antigen binding regions (US 5,981,245; 5,728,813; 5,969,110; 6,596,852; 6,232,447; and 6,057,124); and anti-PDGF-BB antagonists as well as antibodies or antigen binding regions specifically binding to PDGF-BB ligands.

Non-limiting examples of matrix-metalloproteinase (MMP) inhibitors include MMP-2 (matrix-metalloproteinase 2) inhibitors, MMP-9 (matrix-metalloproteinase 9) inhibitors, prinomastat, RO 32-3555, and RS 13-0830. Examples of useful matrix metalloproteinase inhibitors are described, for example, in WO 96/33172, WO 96/27583, EP 1004578 , WO 98/07697, WO 98/03516, WO 98/34918, WO 98/34915, WO 98/33768, WO 98/30566, EP 0606046, EP 0931788, WO 90/05719, WO 99/52910, WO 99/52889, WO 99/29667, WO 1999/007675 , EP 1786785, EP 1181017, US 2009/0012085 , US 5,863,949, US 5,861,510, and EP 0780386. Preferred MMP-2 and MMP-9 inhibitors are those that have little or no activity inhibiting MMP-1. More preferred, are those that selectively inhibit MMP-2 and/or MMP-9 relative to the other matrix-metalloproteinases (i.e., MAP-1, MMP-3, MMP-4, MMP-5, MMP-6, MMP- 7, MMP- 8, MMP-10, MMP-11, MMP-12, and MMP-13).

Non-limiting examples of VEGF and VEGFR inhibitory agents include bevacizumab, cediranib, CEP 7055, CP 547632, KRN 633, orantinib, pazopanib, pegaptanib, pegaptanib octasodium, semaxanib, sorafenib, sunitinib, VEGF antagonist (Borean, Denmark), and VEGF-TRAP^{™}.

The additional anti-cancer agent(s) may also be another anti-angiogenic agent including, but not limited to, 2-methoxyestradiol, AE 941, alemtuzumab, alpha-D148 Mab (Amgen, US), alphastatin, anecortave acetate, angiocidin, angiogenesis inhibitors, (SUGEN, US), angiostatin, anti-Vn Mab (Crucell, Netherlands), atiprimod, axitinib, AZD 9935, BAY RES 2690 (Bayer, Germany, BC 1 (Genoa Institute of Cancer Research, Italy), beloranib, benefin (Lane Labs, US), cabozantinib, CDP 791 (Celltech Group, UK), chondroitinase AC, cilengitide, combretastatin A4 prodrug, CP 564959 (OSI, US), CV247, CYC 381 (Harvard University, US), E 7820, EHT 0101, endostatin, enzastaurin hydrochloride, ER-68203-00 (IVAX, US), fibrinogen-E fragment, Flk-1 (ImClone Systems, US), forms of FLT 1 (VEGFR 1), FR-111142, GCS-100, GW 2286 (GlaxoSmithKline, UK), IL-8, ilomastat, IM-862, irsogladine, KM-2550 (Kyowa Hakko, Japan), lenalidomide, lenvatinib, MAb alpha5beta3 integrin, second generation (Applied Molecular Evolution, USA and Medlmmune, US), MAb VEGF (Xenova, UK), marimastat, maspin (Sosei, Japan), metastatin, motuporamine C, M-PGA, ombrabulin, OXI4503, PI 88, platelet factor 4, PPI 2458, ramucirumab, rBPI 21 and BPI-derived antiangiogenic (XOMA, US), regorafenib, SC-236, SD-7784 (Pfizer, US), SDX 103 (University of California at San Diego, US), SG 292 (Telios, US), SU-0879 (Pfizer, US), TAN-1120, TBC-1635, tesevatinib, tetrathiomolybdate, thalidomide, thrombospondin 1 inhibitor, Tie-2 ligands (Regeneron, US), tissue factor pathway inhibitors (EntreMed, US), tumor necrosis factor-alpha inhibitors, tumstatin, TZ 93, urokinase plasminogen activator inhibitors, vadimezan, vandetanib, vasostatin, vatalanib, VE-cadherin-2 antagonists, xanthorrhizol, XL 784 (Exelixis, US), ziv-aflibercept, and ZD 6126.

In embodiments, the additional anti-cancer agent(s) is an additional active agent that disrupts or inhibits RAS-RAF-ERK or PI3K-AKT-TOR signaling pathways or is a PD-1 and/or PD-L1 antagonist. In embodiments, the additional anti-cancer agent(s) is a RAF inhibitor, EGFR inhibitor, MEK inhibitor, ERK inhibitor, PI3K inhibitor, AKT inhibitor, TOR inhibitor, MCL-1 inhibitor, BCL-2 inhibitor, SHP2 inhibitor, proteasome inhibitor, or immune therapy, including monoclonal antibodies, immunomodulatory imides (IMiDs), anti-PD-1, anti-PDL-1, anti-CTLA4, anti-LAGl, and anti-OX40 agents, GITR agonists, CAR-T cells, and BiTEs.

Non-limiting examples of RAF inhibitors include dabrafenib, encorafenib, regorafenib, sorafenib, and vemurafenib.

Non-limiting examples of MEK inhibitors include binimetinib, CI-1040, cobimetinib, PD318088, PD325901, PD334581, PD98059, refametinib, selumetinib, and trametinib.

Non-limiting examples of ERK inhibitors include LY3214996, LTT462, MK-8353, SCH772984, ravoxertinib, ulixertinib, and an ERKi as described in WO 2017/068412.

Non-limiting examples of PI3K inhibitors include 17-hydroxywortmannin analogs (e.g., WO 06/044453); AEZS-136; alpelisib; AS-252424; buparlisib; CAL263; copanlisib; CUDC-907; dactolisib (WO 06/122806); demethoxyviridin; duvelisib; GNE-477; GSK1059615; IC87114; idelalisib; INK1117; LY294002; Palomid 529; paxalisib; perifosine; PI-103; PI-103 hydrochloride; pictilisib (e.g., WO 09/036,082; WO 09/055,730); PIK 90; PWT33597; SF1126; sonolisib; TGI 00-115; TGX-221; XL147; XL-765; wortmannin; and ZSTK474.

Non-limiting examples of AKT inhibitors include Akt-1-1 (inhibits Aktl) (Barnett et al. (2005) Biochem. J., 385 (Pt. 2), 399-408); Akt-1-1,2 (Barnett et al. (2005) Biochem. J. 385 (Pt. 2), 399-408); API-59CJ-Ome (e.g., Jin et al. (2004) Br. J. Cancer 91, 1808-12); 1-H-imidazo[4,5-c]pyridinyl compounds (e.g., WO05011700); indole-3-carbinol and derivatives thereof *(e.g.,* U.S. Patent No. 6,656,963; Sarkar and Li (2004) J Nutr. 134(12 Suppl), 3493S-3498S); perifosine, Dasmahapatra et al. (2004) Clin. Cancer Res. 10(15), 5242-52, 2004); phosphatidylinositol ether lipid analogues (e.g., Gills and Dennis (2004) Expert. Opin. Investig. Drugs 13, 787-97); triciribine (Yang et al. (2004) Cancer Res. 64, 4394-9); imidazooxazone compounds including trans-3-amino-1-methyl-3-[4-(3-phenyl-5H-imidazo[1,2-c]pyrido[3,4-e][1,3]oxazin-2-yl)phenyl]-cyclobutanol hydrochloride (WO 2012/137870) ; afuresertib;; capivasertib; MK2206; patasertib, and those disclosed in WO 2011/082270 and WO 2012/177844.

Non-limiting examples of TOR inhibitors include deforolimus; ATP-competitive TORC1/TORC2 inhibitors, including PI-103, PP242, PP30, and Torin 1; TOR inhibitors in FKBP12 enhancer, rapamycins and derivatives thereof, including temsirolimus, everolimus, WO 9409010; rapalogs, e.g. as disclosed in WO 98/02441 and WO 01/14387, e.g. AP23573, AP23464, or AP23841; 40-(2-hydroxyethyl)rapamycin, 40-[3-hydroxy(hydroxymethyl)methylpropanoate]-rapamycin ; 40-epi-(tetrazolyl)-rapamycin (also called ABT578); 32-deoxorapamycin; 16-pentynyloxy-32(S)-dihydrorapanycin, and other derivatives disclosed in WO 05/005434; derivatives disclosed in US 5,258,389, WO 94/090101, WO 92/05179, US 5,118,677, US 5,118,678, US 5,100,883, US 5,151,413, US 5,120,842, WO 93/111130, WO 94/02136, WO 94/02485, WO 95/14023, WO 94/02136, WO 95/16691, WO 96/41807, WO 96/41807 and US 5,256,790; and phosphorus-containing rapamycin derivatives (e.g., WO 05/016252).

Non-limiting examples of MCL-1 inhibitors include AMG-176, MIK665, and S63845.

Non-limiting examples of SHP2 inhibitors include SHP2 inhibitors described in WO 2019/167000 and WO 2020/022323.

Additional non-limiting examples of anti-cancer agents that are suitable for use include 2-ethylhydrazide, 2,2',2"-trichlorotriethylamine, ABVD, aceglatone, acemannan, aldophosphamide glycoside, alpharadin, amifostine, aminolevulinic acid, anagrelide, ANCER, ancestim, anti-CD22 immunotoxins, antitumorigenic herbs, apaziquone, arglabin, arsenic trioxide, azathioprine, BAM 002 (Novelos), bcl-2 (Genta), bestrabucil, biricodar, bisantrene, bromocriptine, brostallicin, bryostatin, buthionine sulfoximine, calyculin, cell-cycle nonspecific antineoplastic agents, celmoleukin, clodronate, clotrimazole, cytarabine ocfosfate, DA 3030 (Dong-A), defofamine, denileukin diftitox, dexrazoxane, diaziquone, dichloroacetic acid, dilazep, discodermolide, docosanol, doxercalciferol, edelfosine, eflomithine, EL532 (Elan), elfomithine, elsamitrucin, eniluracil, etanidazole, exisulind, ferruginol, folic acid replenisher such as frolinic acid, gacytosine, gallium nitrate, gimeracil/oteracil/tegafur combination (S-1), glycopine, histamine dihydrochloride, HIT diclofenac, HLA-B7 gene therapy (Vical), human fetal alpha fetoprotein, ibandronate, ibandronic acid, ICE chemotherapy regimen, imexon, iobenguane, IT-101 (CRLX101), laniquidar, LC 9018 (Yakult), leflunomide, lentinan, levamisole + fluorouracil, lovastatin, lucanthone, masoprocol, melarsoprol, metoclopramide, miltefosine, miproxifene, mitoguazone, mitozolomide, mopidamol, motexafin gadolinium, MX6 (Galderma), naloxone + pentazocine, nitracrine, nolatrexed, NSC 631570 octreotide (Ukrain), olaparib, P-30 protein, PAC-1, palifermin, pamidronate, pamidronic acid, pentosan polysulfate sodium, phenamet, picibanil, pixantrone, platinum, podophyllinic acid, porfimer sodium, PSK (Polysaccharide-K), rabbit antithymocyte polyclonal antibody, rasburiembodiment, retinoic acid, rhenium Re 186 etidronate, romurtide, samarium (153 Sm) lexidronam, sizofiran, sodium phenylacetate, sparfosic acid, spirogermanium, strontium-89 chloride, suramin, swainsonine, talaporfin, tariquidar, tazarotene, tegafur-uracil, temoporfin, tenuazonic acid, tetrachlorodecaoxide, thrombopoietin, tin ethyl etiopurpurin, tirapazamine, TLC ELL-12, tositumomab-iodine 131, trifluridine and tipiracil combination, troponin I (Harvard University, US), urethan, valspodar, verteporfin, zoledronic acid, and zosuquidar.

The present disclosure further provides a method for using the compounds of Formula (I) or (Ia) or pharmaceutical compositions provided herein, in combination with radiation therapy to treat cancer. Techniques for administering radiation therapy are known in the art, and these techniques can be used in the combination therapy described herein. The administration of the compound of Formula (I) or (Ia) in this combination therapy can be determined as described herein.

Radiation therapy can be administered through one of several methods, or a combination of methods, including, without limitation, external-beam therapy, internal radiation therapy, implant radiation, stereotactic radiosurgery, systemic radiation therapy, radiotherapy and permanent or temporary interstitial brachy therapy. The term "brachytherapy," as used herein, refers to radiation therapy delivered by a spatially confined radioactive material inserted into the body at or near a tumor or other proliferative tissue disease site. The term is intended, without limitation, to include exposure to radioactive isotopes (e.g., At-211, I-131, I -125, Y-90, Re-186, Re-188, Sm-153, Bi-212, P-32, and radioactive isotopes of Lu). Suitable radiation sources for use as a cell conditioner of the present disclosure include both solids and liquids. By way of non-limiting example, the radiation source can be a radionuclide, such as I-125, I -131, Yb-169, Ir-192 as a solid source, I-125 as a solid source, or other radionuclides that emit photons, beta particles, gamma radiation, or other therapeutic rays. The radioactive material can also be a fluid made from any solution of radionuclide(s), e.g., a solution of I-125 or I-131, or a radioactive fluid can be produced using a slurry of a suitable fluid containing small particles of solid radionuclides, such as Au-198, Y-90. Moreover, the radionuclide(s) can be embodied in a gel or radioactive microspheres.

The present disclosure also provides methods for combination therapies in which the additional active agent is known to modulate other pathways, or other components of the same pathway, or even overlapping sets of target enzymes which are used in combination with a compound of Formula (I) or (Ia), or a pharmaceutically acceptable salt thereof. In one embodiment, such therapy includes, but is not limited to, the combination of one or more compounds of Formula (I) or (Ia) with chemotherapeutic agents, immunotherapeutic agents, hormonal therapy agents, therapeutic antibodies, targeted therapy agents, and radiation treatment, to provide a synergistic or additive therapeutic effect.

The compounds of the disclosure can be used in combination with the agents disclosed herein or other suitable agents, depending on the condition being treated. Hence, in some embodiments the one or more compounds of the disclosure will be co-administered with other agents as described above. When used in combination therapy, the compounds described herein are administered with the second agent simultaneously or separately. This administration in combination can include simultaneous administration of the two agents in the same dosage form, simultaneous administration in separate dosage forms, and separate administration. That is, a compound of Formula (I) or (Ia) and any of the agents described above can be formulated together in the same dosage form and administered simultaneously. Alternatively, a compound of Formula (I) or (Ia) and any of the agents described above can be simultaneously administered, wherein both the agents are present in separate formulations. In another alternative, a compound of Formula (I) or (Ia) can be administered just followed by and any of the agents described above, or vice versa. In some embodiments of the separate administration protocol, a compound of Formula (I) or (Ia) and any of the agents described above are administered a few minutes apart, or a few hours apart, or a few days apart.

As one aspect of the present disclosure contemplates the treatment of the disease/conditions with a combination of pharmaceutically active compounds that may be administered separately, the disclosure further relates to combining separate pharmaceutical compositions in kit form. The kit comprises two separate pharmaceutical compositions: a compound of Formula (I) or (Ia), and a second pharmaceutical compound. The kit comprises a container for containing the separate compositions such as a divided bottle or a divided foil packet. Additional examples of containers include syringes, boxes, and bags. In some embodiments, the kit comprises directions for the use of the separate components. The kit form is particularly advantageous when the separate components are preferably administered in different dosage forms *(e.g.,* oral and parenteral), are administered at different dosage intervals, or when titration of the individual components of the combination is desired by the prescribing health care professional.

The present disclosure also provides for the compound of Formula (I) or (Ia), or the pharmaceutically acceptable salt thereof, for use in therapy, or use of the compound of Formula (I) or (Ia), or the pharmaceutically acceptable salt thereof, in therapy. The present disclosure also provides for the compound of Formula (I) or (Ia), or the pharmaceutically acceptable salt thereof, for use in treating cancer, or use of a compound of Formula (I) or (Ia), or the pharmaceutically acceptable salt thereof, for treating cancer. The present disclosure also provides for the compound of Formula (I) or (Ia), or the pharmaceutically acceptable salt thereof, for the preparation of a medicament for the treatment of cancer, or use of the compound of Formula (I) or (Ia), or the pharmaceutically acceptable salt thereof, for the preparation of a medicament for the treatment of cancer. The present disclosure also provides for the compound of Formula (I) or (Ia), or the pharmaceutically acceptable salt thereof, and an additional anti-cancer agent, for use in the treatment of cancer, or use of the compound of Formula (I) or (Ia), or the pharmaceutically acceptable salt thereof, and the additional anti-cancer agent for treating cancer. The disclosure also provides the compound of Formula (I) or (Ia), or the pharmaceutically acceptable salt thereof, and an additional anti-cancer agent, for the preparation of a medicament for the treatment of cancer, or use of the compound of Formula (I) or (Ia), or the pharmaceutically acceptable salt thereof, and the additional anti-cancer agent, for the preparation of a medicament for the treatment of cancer. The present disclosure also provides for a pharmaceutical composition comprising the compound of Formula (I) or (Ia), or the pharmaceutically acceptable salt thereof, for use in the treatment of cancer, or use of the pharmaceutical composition comprising the compound of Formula (I) or (Ia), or the pharmaceutically acceptable salt thereof, for treating cancer. The present disclosure also provides for a pharmaceutical composition comprising the compound of Formula (I) or (Ia), or the pharmaceutically acceptable salt thereof, and an additional anti-cancer agent, for use in the treatment of cancer, or use of the pharmaceutical composition comprising the compound of Formula (I) or (Ia), or the pharmaceutically acceptable salt thereof, and the additional anti-cancer agent, for treating cancer.

### Methods of Preparing the Compounds of the Disclosure

The compounds described herein can be prepared according to the procedures of the following schemes and examples, using appropriate materials and are further exemplified by the following specific examples. The examples further illustrate details for the preparation of the compounds of the present disclosure. Those skilled in the art will readily understand that known variations of the conditions and processes of the following preparative procedures can be used to prepare these compounds. For instance, in some cases, the order of carrying out the steps of reaction schemes may be varied to facilitate the reaction or to avoid unwanted reaction products. These examples are provided for the purpose of further illustration only and are not intended to be limitations on the disclosure.

Disclosure of a compound likewise discloses a pharmaceutically acceptable salt of the compound, and disclosure of a pharmaceutically acceptable salt of a compound likewise discloses the compound, i.e., the non-salt or un-ionized form of the compound, or an alternative pharmaceutically acceptable salt of the compound, as would be readily understood by one of ordinary skill in the art.

Throughout the synthetic schemes and examples, abbreviations and acronyms may be used with the following meanings unless otherwise indicated: aq.: aqueous, c./con./conc.: concentrated, sat.: saturated, s: singlet, d: doublet, t: triplet, q: quartet, sep: septet, dd: double doublet, dt: double triplet, td: triple doublet, tt: triple triplet, ddd: double double doublet, ddt: double double triplet, dtd: double triple doublet, tdd: triple double doublet, m: multiplet, br: broad, brs: broad singlet, tert: tertiary, Ac: acetyl, AcOH: acetic acid, AIBN: azobisisobutyronitrile, Boc₂O: Di-tert-butyl decarbonate, Bu: butyl, CDCl₃: deuterated chloroform, CD₃OD: deuterated methanol, CELITE: diatomaceous earth, DCM: dichloromethane, DIAD: diisopropyl azodicarboxylate, DIEA: N,N-diisopropylethylamine, DIPEA: N,N-diisopropylethylamine, DMA: dimethylacetamide, DMB: 2,4-dimethoxybenzyl, DME: 1,2-dimethoxyethane, DMF: N,N-dimethylformamide, DMP: Dess-Martin periodinane, DMSO: dimethyl sulfoxide, DMSO-d₆: deuterated dimethyl sulfoxide, Et: ethyl, EtOAc: ethyl acetate, EtOH: ethanol, Et₃N: triethylamine, EtOvinyl-B: 2-[(E)-2-ethoxyvinyl]-4,4,5,5-tetramethyl-1,3,2-dioxaborolane, HATU: 1-[Bis(dimethylamino)methylene]-1H-1,2,3-triazolo[4,5-b]pyridinium 3-Oxide Hexafluorophosphate, HPLC: High pressure liquid chromatography, Indolizine-B: (8-(5,5-dimethyl-1,3,2-dioxaborinan-2-yl)indolizin-3-yl)(3,4,5-trifluorophenyl)methanone, IPE: diisopropyl ether, iPr: isopropyl, KOAc: potassium acetate, Me: methyl, MeOH: methanol, MTBE: methyl tert-butyl ether, NBS: N-bromosuccinimide = 1-bromopyrrolidine-2,5-dione, NIS: N-iodosuccinimide, NMP: N-methylpyrrolidone, Pd(dppf)Cl₂: [1,1'-Bis(diphenylphosphino)ferrocene]dichloropalladium(II), Ph: phenyl, RT: room temperature, SFC: supercritical fluid chromatography, SPhos Pd G3: (2-Dicyclohexylphosphino-2',6'-dimethoxybiphenyl) [2-(2'-amino-1,1'-biphenyl)]palladium(II) methanesulfonate, TEA: tridthylamine, TFA: trifluoroacetic acid, TFAA: trifluoroacetic anhydride, THF: tetrahydrofuran, TLC: thin layer chromatography, TMAD: N,N,N',N'-tetramethylazodicarboxamide, T3P: propanephosphonic acid anhydride.

The reagents used in the Examples are commercially available products unless indicated otherwise. Prepacked columns manufactured by Shoko Scientific Co., Ltd., or Biotage were used in silica gel column chromatography and basic silica gel column chromatography. AVANCE NEO 400 spectrometer (400 MHz; BRUKER) and AVANCE III HD 500 spectrometer (500 MHz; BRUKER) were used for NMR spectra. For a deuterated solvent containing tetramethylsilane, tetramethylsilane was used as the internal reference. For other cases, measurement was performed using an NMR solvent as the internal reference. All δ values are indicated in ppm. Microwave reaction was performed using an Initiator (trademark) manufactured by Biotage. XSelect CSH C18 OBD Prep Columns manufactured by Waters were used for preparative reversed-phase HPLC.

### Synthetic procedure A

### Example 1-1 and 1-2

### (E)-N-(4-(10-chloro-4,5-dihydrobenzo[7,8][1,4]oxazocino[6,5,4-hi]indole-1-carbonyl)-2,6-difluorophenyl)-4-(((1r,4r)-4-methoxycyclohexyl)amino)but-2-enamide

### Step 1:

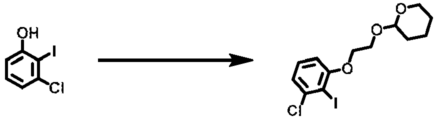

2-(2-bromoethoxy)tetrahydropyran (715 µL, 4.72 mmol) was added to a suspension of 3-chloro-2-iodo-phenol (1.00 g, 3.52 mmol) and K₂CO₃ (815 mg, 5.90 mmol) in DMF (7.9 mL). After stirring at 60°C for 4.5 h, the reaction mixture was diluted with EtOAc, washed with H₂O(2x) and brine then dried over Na₂SO₄ and evaporated. The residue was purified by column chromatography on silica gel (hexane/EtOAc), thereby obtaining 2-(2-(3-chloro-2-iodophenoxy)ethoxy)tetrahydro-2H-pyran (1.31 g, 87%).

### Step 2:

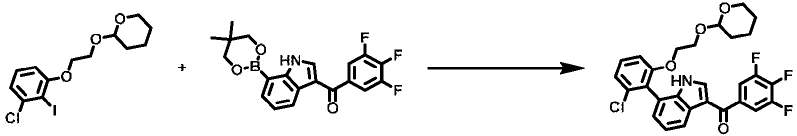

A solution of 2-(2-(3-chloro-2-iodophenoxy)ethoxy)tetrahydro-2H-pyran (360 mg, 0.941 mmol), (7-(5,5-dimethyl-1,3,2-dioxaborinan-2-yl)-1H-indol-3-yl)(3,4,5-trifluorophenyl)methanone (474 mg, 1.22 mmol), Pd(PPh₃)₄ (109 mg, 0.0941 mmol) in 1,4-Dioxane (4.9 mL) and 2M Na₂CO₃ aq. (917 µL, 1.83 mmol) was evacuated and N₂ back-filled before heating to 100 °C for 14.5 h. After cooling, the reaction mixture was diluted with CHCl₃, washed with H₂O and brine then dried over Na₂SO₄ and evaporated. The residue was purified by column chromatography on silica gel (hexane/EtOAc), thereby obtaining (7-(2-chloro-6-(2-((tetrahydro-2H-pyran-2-yl)oxy)ethoxy)phenyl)-1H-indol-3-yl)(3,4,5-trifluorophenyl)methanone (383 mg, 77%).

### Step 3:

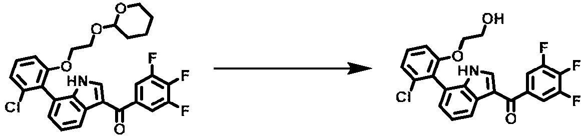

TFA (5.2 mL, 71 mmol) was added to a solution of (7-(2-chloro-6-(2-((tetrahydro-2H-pyran-2-yl)oxy)ethoxy)phenyl)-1H-indol-3-yl)(3,4,5-trifluorophenyl)methanone (374 mg, 0.706 mmol) in CHCl₃ (1.4 mL). After stirring at RT for 1 h, the reaction mixture was evaporated and purified by column chromatography on NH silica gel (hexane/EtOAc), thereby obtaining (7-(2-chloro-6-(2-hydroxyethoxy)phenyl)-1H-indol-3-yl)(3,4,5-trifluorophenyl)methanone (278 mg, 88%).

### Step 4:

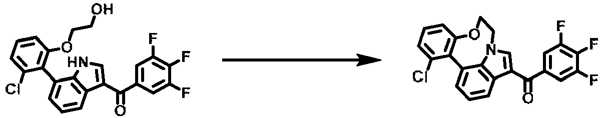

DIAD (543 µL, 2.76 mmol) was added to a solution of (7-(2-chloro-6-(2-hydroxyethoxy)phenyl)-1H-indol-3-yl)(3,4,5-trifluorophenyl)methanone (246 mg, 0.552 mmol) and PPh₃ (724 mg, 2.76 mmol) in DMF (28 mL) at 0°C. After stirring at RT for 20 min, the reaction mixture was diluted with EtOAc, washed with NaHCO₃ aq., H₂O(2x) and brine then dried over Na₂SO₄ and evaporated. A seed of diisopropyl hydrazine-1,2-dicarboxylate compound with triphenylphosphine oxide (1: 1) was added to the residue, and the resulting solid was suspended in MTBE (2.5 mL) and heptane (7.5 mL) at RT. The resulting suspension was filtered, rinsing with heptane, and the filtrate was evaporated and purified by column chromatography on silica gel (hexane/EtOAc), thereby obtaining (10-chloro-4,5-dihydrobenzo[7,8][1,4]oxazocino[6,5,4-hi]indol-1-yl)(3,4,5-trifluorophenyl)methanone (115 mg, 51%).

### Step 5:

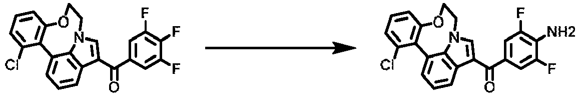

28% NH₃ aq. (11.5 mL, 82.7 mmol) was added to (10-chloro-4,5-dihydrobenzo[7,8][1,4]oxazocino[6,5,4-hi]indol-1-yl)(3,4,5-trifluorophenyl)methanone (118 mg, 0.276 mmol) in 1,4-Dioxane (1.2 mL). The reaction mixture was heated at 130 °C by microwave irradiation for 14 h. After cooling, the reaction mixture was diluted with EtOAc, washed with H₂O and brine then dried over Na₂SO₄ and evaporated. The residue was purified by column chromatography on silica gel (hexane/EtOAc), thereby obtaining (4-amino-3,5-difluorophenyl)(10-chloro-4,5-dihydrobenzo[7,8][1,4]oxazocino[6,5,4-hi]indol-1-yl)methanone (98.2 mg, 84%).

### Step 6:

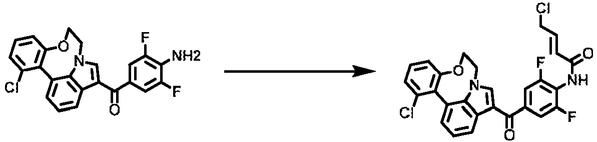

A solution of (4-amino-3,5-difluorophenyl)(10-chloro-4,5-dihydrobenzo[7,8][1,4]oxazocino[6,5,4-hi]indol-1-yl)methanone (98.2 mg, 0.231 mmol), 4-chlorocrotonic acid (36 mg, 0.30 mmol) and propylphosphonic anhydride solution (50 wt% in THF, 217 µL, 0.347 mmol) in DMF (770 µL) was stirred at RT for 15 min. Then Et3N (80.6 µL, 0.578 mmol) was added thereto, and the mixture was stirred at RT for 2.5 h. The reaction mixture was diluted with H₂O and sat. NaHCO₃ aq. then stirred at RT. The resulting solid was collected by filtration and vacuum-dried at RT, thereby obtaining crude (E)-4-chloro-N-(4-(10-chloro-4,5-dihydrobenzo[7,8][1,4]oxazocino[6,5,4-hi]indole-1-carbonyl)-2,6-difluorophenyl)but-2-enamide (99.8 mg, 82%).

### Step 7:

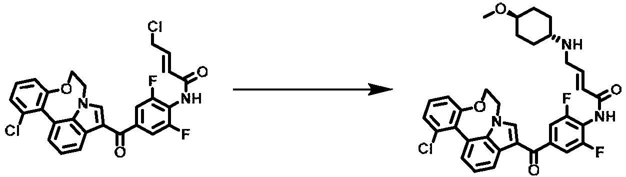

A solution of crude (E)-4-chloro-N-(4-(10-chloro-4,5-dihydrobenzo[7,8][1,4]oxazocino[6,5,4-hi]indole-1-carbonyl)-2,6-difluorophenyl)but-2-enamide (99.8 mg, 0.189 mmol), trans-4-methoxycyclohexanamine hydrochloride (94 mg, 0.57 mmol), KI (94 mg, 0.57 mmol) and K₃PO₄ (281 mg, 1.32 mmol) in DMF (630 µL) was stirred at RT for 2 h. The reaction mixture was diluted with H₂O then stirred at RT for 1 h. The resulting solid was collected by filtration and vacuum-dried at RT. The obtained solid was purified by column chromatography on silica gel (CHCl₃(1%Et₃N)/MeOH), thereby obtaining the racemic title compound (74.2 mg, 63%). The racemate (70 mg) was separated by CHIRALPAK IE (hexane-EtOH(0.1%Et₃N)) to give the title compound (Example 1-1, 27.6 mg, 40%; single isomer, 1st-eluting isomer and Example 1-2, 27.3 mg, 39%; single isomer, 2nd-eluting isomer). ¹H NMR: (500 MHz, CHLOROFORM-D) δ 8.48 (dd, J = 7.6, 1.5 Hz, 1H), 7.53 (s, 1H), 7.47-7.40 (m, 5H), 7.37 (t, J = 8.0 Hz, 1H), 7.20-7.07 (m, 3H), 6.26 (d, J = 15.6 Hz, 1H), 4.61-4.55 (m, 1H), 4.41-4.32 (m, 1H), 4.12 (dd, J = 11.6, 2.4 Hz, 1H), 3.91 (dd, J = 15.1, 2.3 Hz, 1H), 3.52 (dd, J = 5.2, 1.2 Hz, 2H), 3.35 (s, 3H), 3.18-3.12 (m, 1H), 2.58-2.51 (m, 1H), 2.09 (d, J = 10.1 Hz, 2H), 1.99 (d, J = 11.9 Hz, 2H), 1.33-1.11 (m, 4H); ESI [M+H]⁺: 620.5.

### Synthetic procedure B

### Example 12-1 and 12-2

### (E)-N-(4-(8,9-dimethyl-6-(trifluoromethyl)-12,13-dihydro-8H-imidazo[4",5":3',4']benzo[1',2':7,81[1,4]oxazocino[6,5,4-hi]indole-2-carbonyl)-2,6-difluorophenyl)-4-(((1r,4r)-4-methoxycyclohexyl)amino)but-2-enamide

### Step 1:

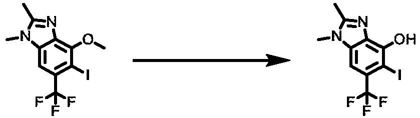

BBr₃ (1.0 M in CH₂Cl₂, 13.3 mL, 13.3 mmol) was added to a solution of 5-iodo-4-methoxy-1,2-dimethyl-6-(trifluoromethyl)-1H-benzo[d]imidazole (493 mg, 1.33 mmol) in CH₂Cl₂ (6.2 mL) at 0°C. After stirring at RT for 16 h, the reaction mixture was cooled to 0°C then CHCl₃/MeOH and sat.NaHCO₃ aq. were added to the reaction mixture slowly. The insoluble solid was filtered, washed with H₂O and CHCl₃, and vacuum-dried at 60 °C. The organic layer was dried over Na₂SO₄ and evaporated. The residue and filter cake were suspended in CHCl₃ (7 mL), stirred at RT. The solid was collected by filtration, washed with further CHCl₃, and vacuum-dried at 60°C, thereby obtaining 5-iodo-1,2-dimethyl-6-(trifluoromethyl)-1H-benzo[d]imidazol-4-ol (455 mg, 96%).

### Step 2:

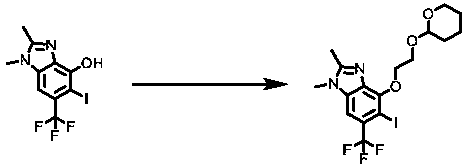

2-(2-bromoethoxy)tetrahydropyran (290 µL, 1.92 mmol) was added to a suspention of 5-iodo-1,2-dimethyl-6-(trifluoromethyl)-1H-benzo[d]imidazol-4-ol (455 mg, 1.28 mmol) and K₂CO₃ (265 mg, 1.92 mmol) in DMF (6.4 mL). After stirring at 60°C for 2.5 h, the reaction mixture was diluted with EtOAc, washed with H₂O (2x) and brine then dried over Na₂SO₄ and evaporated. The residue was purified by column chromatography on silica gel (CHCl₃/MeOH), thereby obtaining 5-iodo-1,2-dimethyl-4-(2-((tetrahydro-2H-pyran-2-yl)oxy)ethoxy)-6-(trifluoromethyl)-1H-benzo[d]imidazole (334 mg, 54%).

### Step 3:

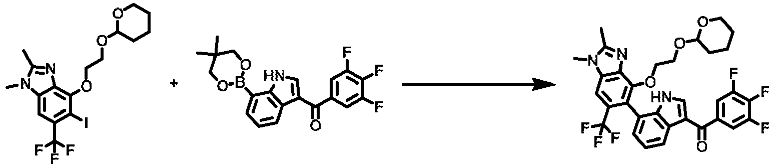

A solution of 5-iodo-1,2-dimethyl-4-(2-((tetrahydro-2H-pyran-2-yl)oxy)ethoxy)-6-(trifluoromethyl)-1H-benzo[d]imidazole (169 mg, 0.349 mmol), (7-(5,5-dimethyl-1,3,2-dioxaborinan-2-yl)-1H-indol-3-yl)(3,4,5-trifluorophenyl)methanone (270 mg, 0.698 mmol), Pd(PPh₃)₄ (40 mg, 0.035 mmol) in 1,4-Dioxane (3.5 mL) and 2M Na₂CO₃ aq. (523 µL, 1.05 mmol) was evacuated and N₂ back-filled before heating to 120 °C by microwave irradiation for 5 h. After cooling, the reaction mixture was diluted with EtOAc, washed with H₂O and brine then dried over Na₂SO₄ and evaporated. The residue was purified by column chromatography on silica gel (CHCl₃/MeOH), thereby obtaining (7-(1,2-dimethyl-4-(2-((tetrahydro-2H-pyran-2-yl)oxy)ethoxy)-6-(trifluoromethyl)-1H-benzo[d]imidazol-5-yl)-1H-indol-3-yl)(3,4,5-trifluorophenyl)methanone (121 mg, 55%).

### Step 4:

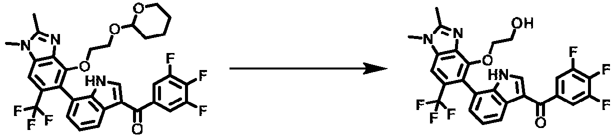

TFA (2.6 mL, 35 mmol) was added to a solution of (7-(1,2-dimethyl-4-(2-((tetrahydro-2H-pyran-2-yl)oxy)ethoxy)-6-(trifluoromethyl)-1H-benzo[d]imidazol-5-yl)-1H-indol-3-yl)(3,4,5-trifluorophenyl)methanone (217 mg, 0.344 mmol) in CHCl₃ (688 µL). After stirring at RT for 30 min, the reaction mixture was evaporated and purified by column chromatography on NH silica gel (CHCl₃/MeOH), thereby obtaining (7-(4-(2-hydroxyethoxy)-1,2-dimethyl-6-(trifluoromethyl)-1H-benzo[d]imidazol-5-yl)-1H-indol-3-yl)(3,4,5-trifluorophenyl)methanone (182 mg, 97%).

### Step 5:

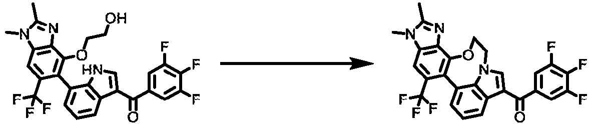

DIAD (467 µL, 2.37 mmol) was added to a solution of (7-(4-(2-hydroxyethoxy)-1,2-dimethyl-6-(trifluoromethyl)-1H-benzo[d]imidazol-5-yl)-1H-indol-3-yl)(3,4,5-trifluorophenyl)methanone (173 mg, 0.316 mmol) and PPh₃ (622 mg, 2.37 mmol) in DMF (16 mL) at 0°C. After stirring at RT for 10 min, the reaction mixture was diluted with EtOAc, washed with NaHCO₃ aq., H₂O and brine then dried over Na₂SO₄ and evaporated. The residue was suspended in isopropyl ether, stirred at RT. The resulting solid was removed by filtration, washing with further isopropyl ether, and the filtrate was evaporated. A seed of diisopropyl hydrazine-1,2-dicarboxylate compound with triphenylphosphine oxide (1:1) was added to a solution of the residue in isopropyl ether at RT. The resulting suspension was filtered, rinsing with isopropyl ether, and the filtrate was evaporated and purified by column chromatography on silica gel (CHCl₃/MeOH). The obtained gum was dissolved in isopropyl ether, and a seed of diisopropyl hydrazine-1,2-dicarboxylate compound with triphenylphosphine oxide (1:1) was added, stirred at RT. The resulting solid was removed by filtration, washing with further isopropyl ether, and the filtrate was evaporated, thereby obtaining crude (8,9-dimethyl-6-(trifluoromethyl)-12,13-dihydro-8H-imidazo[4",5":3',4']benzo[1',2':7,8][1,4]oxazocino[6,5,4-hi]indol-2-yl)(3,4,5-trifluorophenyl)methanone (330 mg, 197%).

### Step 6:

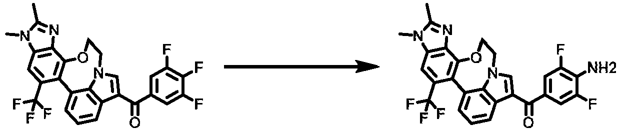

28% NH₃ aq. (13.2 mL, 94.8 mmol) was added to crude (8,9-dimethyl-6-(trifluoromethyl)-12,13-dihydro-8H-imidazo[4",5":3',4']benzo[1',2':7,8][1,4]oxazocino[6,5,4-hi]indol-2-yl)(3,4,5-trifluorophenyl)methanone (330 mg) in 1,4-Dioxane (1.7 mL). The reaction mixture was heated at 130 °C by microwave irradiation for 12 h. After cooling, the reaction mixture was diluted with EtOAc, washed with H₂O and brine then dried over Na₂SO₄ and evaporated. The residue was purified by column chromatography on silica gel (CHCl₃/MeOH), thereby obtaining crude (4-amino-3,5-difluorophenyl)(8,9-dimethyl-6-(trifluoromethyl)-12,13-dihydro-8H-imidazo[4",5":3',4']benzo[1',2':7,8][1,4]oxazocino[6,5,4-hi]indol-2-yl)methanone (118 mg, 71%).

### Step 7:

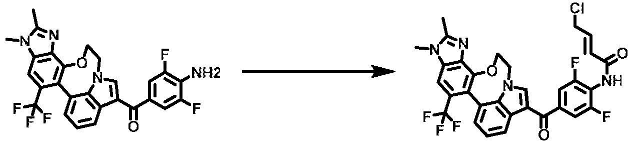

A solution of crude (4-amino-3,5-difluorophenyl)(8,9-dimethyl-6-(trifluoromethyl)-12,13-dihydro-8H-imidazo[4",5":3',4']benzo[1',2':7,8][1,4]oxazocino[6,5,4-hi]indol-2-yl)methanone (118 mg), 4-chlorocrotonic acid (47 mg, 0.39 mmol) and propylphosphonic anhydride solution (50 wt% in THF, 246 µL, 0.393 mmol) in DMF (1.1 mL) was stirred at RT for 25 min. Then Et₃N (110 µL, 0.786 mmol) was added thereto, and the mixture was stirred at RT for 2 h. Further 4-chlorocrotonic acid (47 mg, 0.39 mmol), propylphosphonic anhydride solution (50 wt% in THF, 246 µL, 0.393 mmol) and Et₃N (110 µL, 0.786 mmol) were added and stirring continued for 30 min. The reaction mixture was diluted with EtOAc, washed with NaHCO₃ aq., H₂O and brine then dried over Na₂SO₄ and evaporated. The residue was purified by column chromatography on silica gel (CHCl₃/MeOH), thereby obtaining crude (E)-4-chloro-N-(4-(8,9-dimethyl-6-(trifluoromethyl)-12,13-dihydro-8H-imidazo[4",5":3',4']benzo[1',2':7,8][1,4]oxazocino[6,5,4-hi]indole-2-carbonyl)-2,6-difluorophenyl)but-2-enamide (97.6 mg, 69%).

### Step 8:

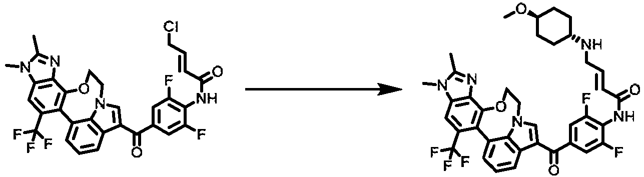

A solution of crude (E)-4-chloro-N-(4-(8,9-dimethyl-6-(trifluoromethyl)-12,13-dihydro-8H-imidazo[4",5":3',4']benzo[1',2':7,8][1,4]oxazocino[6,5,4-hi]indole-2-carbonyl)-2,6-difluorophenyl)but-2-enamide (97.6 mg), trans-4-methoxycyclohexanamine hydrochloride (77 mg, 0.47 mmol), KI (77 mg, 0.47 mmol) and K₃PO₄ (198 mg, 0.931 mmol) in DMF (775 µL) was stirred at RT for 1.5 h. The reaction mixture was diluted with EtOAc, washed with H₂O(2x) and brine then dried over Na₂SO₄ and evaporated. The residue was purified by preparative HPLC (water:acetonitrile (0.1% formic acid)). The purified fractions were washed with sat. NaHCO₃ aq., extracted with CHCl₃/MeOH then dried over Na₂SO₄ and evaporated, thereby obtaining the racemic title compound (37.0 mg, 33%). The racemate (28 mg) was separated by CHIRAL ART SB(YMC) (hexane-EtOH(0.1%Et₃N)) to give the title compound (Example 12-1, 8.60 mg, 31%; single isomer, 1st-eluting isomer and Example 12-2, 8.94 mg, 32%; single isomer, 2nd-eluting isomer). ¹H-NMR: (400 MHz, CHLOROFORM-D) δ 8.48 (dd, J = 8.0, 1.3 Hz, 1H), 7.69 (s, 1H), 7.54 (s, 1H), 7.45-7.35 (m, 3H), 7.31-7.25 (m, 1H), 7.11 (dt, J = 15.3, 5.3 Hz, 1H), 6.26 (dt, J = 15.3, 1.6 Hz, 1H), 4.59-4.38 (m, 3H), 3.93-3.82 (m, 4H), 3.50 (dd, J = 5.3, 1.8 Hz, 2H), 3.35 (s, 3H), 3.18-3.11 (m, 1H), 2.69 (s, 3H), 2.58-2.48 (m, 1H), 2.15-1.90 (m, 4H), 1.32-1.09 (m, 4H); ESI-MS [M+H]⁺: 722.6

### Synthetic procedure C

### Example 26

### (E)-N-(4-(13,13-difluoro-6,8,9-trimethyl-13,14-dihydro-8H,12H-imidazo[4",5":3',4']benzo[1',2':8,9][1,5]oxazonino[7,6,5-hi]indole-2-carbonyl)-2,6-difluorophenyl)-4-(((1r,4r)-4-methoxycyclohexyl)amino)but-2-enamide

### Step 1:

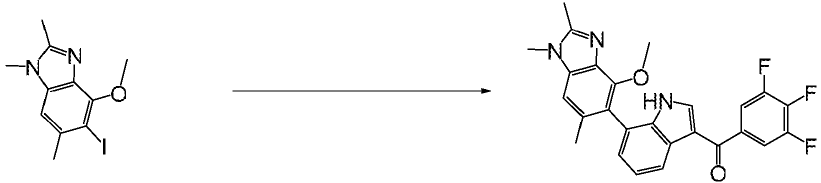

To a mixture of 5-iodo-4-methoxy-1,2,6-trimethyl-1H-benzo[d]imidazole (900 mg, 2.85 mmol), [7-(5,5-dimethyl-1,3,2-dioxaborinan-2-yl)-1H-indol-3-yl]-(3,4,5-trifluorophenyl)methanone (1.27 g, 3.27 mmol) and Pd(PPh₃)₄ (329 mg, 0.285 mmol) in 1,2-dimethoxyethane (18.2 mL) was added 2M aqueous sodium carbonate solution (5.69 mL, 11.4 mmol). After reacting by microwave apparatus at 120 °C for 2 hours, the mixture was diluted with EtOAc and water. The organic layer was washed successively with H₂O then brine, dried over sodium sulfate, and evaporated. The residue was purified by column chromatography on silica gel (acetone-hexane), thereby obtaining (7-(4-methoxy-1,2,6-trimethyl-1H-benzo[d]imidazol-5-yl)-1H-indol-3-yl)(3,4,5-trifluorophenyl)methanone (1.61 g, 3.47 mmol).

### Step 2:

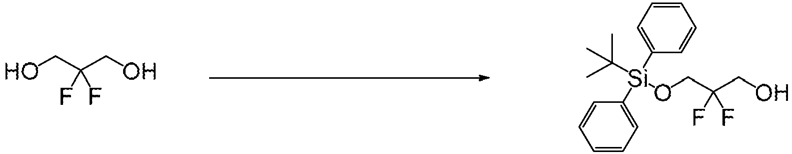

Sodium hydride (514 mg, 21.4 mmol) was added to a solution of 2,2-difluoropropane-1,3-diol (2.00 g, 17.8 mmol) in THF (60.0 mL) at 0 °C. After the reaction mixture was stirred at 0 °C for 15 minutes, tert-Butylchlorodiphenylsilane (4.80 mL, 18.7 mmol) was added and the mixture was stirred at ambient temperature for 1 hour. To the resulting mixture was added saturated aqueous NaHCO₃, and extracted with EtOAc, and washed successively with H₂O then brine, dried over sodium sulfate, and evaporated. The residue was purified by column chromatography on silica gel (EtOAc-hexane), thereby obtaining 3-((tert-butyldiphenylsilyl)oxy)-2,2-difluoropropan-1-ol (4.62 g, 13.2 mmol).

### Step 3:

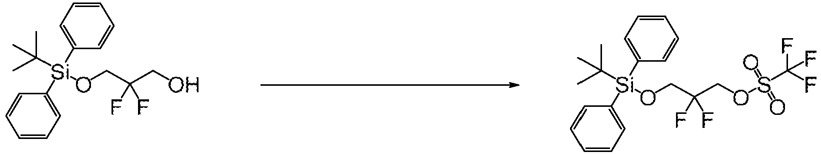

Trifluoromethanesulfonic anhydride (3.76 mL, 26.4 mmol) was added to a mixture of 3-((tert-butyldiphenylsilyl)oxy)-2,2-difluoropropan-1-ol (4.62 g, 13.2 mmol) and 2,6-lutidine (4.58 mL, 39.5 mmol) in CH₂Cl₂ (50.0 mL) at 0 °C. The reaction mixture was stirred at ambient temperature for 3 hours. To the resulting mixture was added CHCl₃ and 1M HCl, and extracted with CHCl₃, and washed successively with H₂O then brine, dried over sodium sulfate, and evaporated. The residue was purified by column chromatography on silica gel (EtOAc-hexane), thereby obtaining 3-((tert-butyldiphenylsilyl)oxy)-2,2-difluoropropyl trifluoromethanesulfonate (4.16 g, 8.62 mmol).

### Step 4:

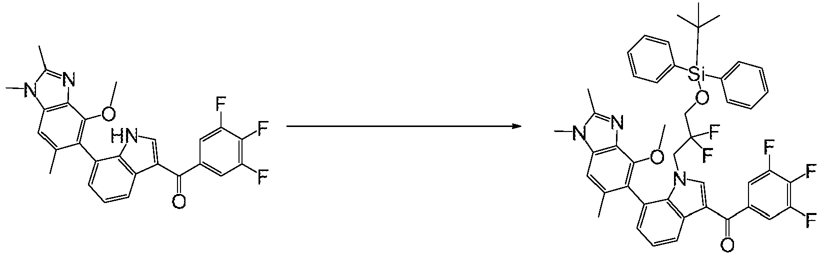

To a mixture of (7-(4-methoxy-1,2,6-trimethyl-1H-benzo[d]imidazol-5-yl)-1H-indol-3-yl)(3,4,5-trifluorophenyl)methanone (200 mg, 0.432 mmol) and potassium carbonate (179 mg, 1.29 mmol) in DMF (2.00 mL) was added 3-((tert-butyldiphenylsilyl)oxy)-2,2-difluoropropyl trifluoromethanesulfonate (400 µL, 0.863 mmol). After the mixture was stirred at 60 °C for 6 hours, the mixture was diluted with EtOAc and water. The organic layer was washed successively with H₂O then brine, dried over sodium sulfate, and evaporated. The residue was purified by column chromatography on silica gel (EtOAc-hexane), thereby obtaining (1-(3-((tert-butyldiphenylsilyl)oxy)-2,2-difluoropropyl)-7-(4-methoxy-1,2,6-trimethyl-1H-benzo[d]imidazol-5-yl)-1H-indol-3-yl)(3,4,5-trifluorophenyl)methanone (259 mg, 0.326 mmol).

### Step 5:

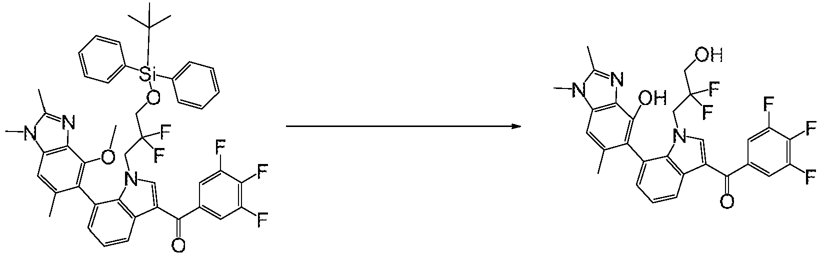

Boron tribromide (1.60 M in CH₂Cl₂, 2.75 mL, 4.39 mmol) was added to (1-(3-((tert-butyldiphenylsilyl)oxy)-2,2-difluoropropyl)-7-(4-methoxy-1,2,6-trimethyl-1H-benzo[d]imidazol-5-yl)-1H-indol-3-yl)(3,4,5-trifluorophenyl)methanone (259 mg, 0.325 mmol) in CH₂Cl₂ (2.20 mL). After the mixture was stirred at 35 °C for 2 hours, the mixture was cooled to ambient temperature. The mixture was diluted with CHCl₃ and quenched by NH₃, and extracted with CHCl₃, and washed successively with H₂O then brine, dried over sodium sulfate, and evaporated. The residue was used for the next reaction without further purification.

Tetra-n-butyl ammonium fluoride (1.00 M in THF, 975 µL, 0.975 mmol) was added to a solution of (1-(3-((tert-butyldiphenylsilyl)oxy)-2,2-difluoropropyl)-7-(4-hydroxy-1,2,6-trimethyl-1H-benzo[d]imidazol-5-yl)-1H-indol-3-yl)(3,4,5-trifluorophenyl)methanone (254 mg, 0.325 mmol) in THF (3.00 mL). After the mixture was stirred at ambient temperature for 30 minutes, the reaction mixture was evaporated. The residue was purified by column chromatography on silica gel (methanol-acetone-hexane), thereby obtaining (1-(2,2-difluoro-3-hydroxypropyl)-7-(4-hydroxy-1,2,6-trimethyl-1H-benzo[d]imidazol-5-yl)-1H-indol-3-yl)(3,4,5-trifluorophenyl)methanone (122 mg, 0.224 mmol).

### Step 6:

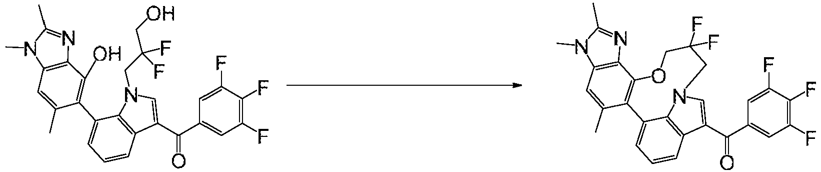

To a mixture of (1-(2,2-difluoro-3-hydroxypropyl)-7-(4-hydroxy-1,2,6-trimethyl-1H-benzo[d]imidazol-5-yl)-1H-indol-3-yl)(3,4,5-trifluorophenyl)methanone (122 mg, 0.224 mmol) and tri-n-butylphosphine (112 µL, 0.449 mmol) in THF (12.1 mL) was added *N,N,N',N*'-tetramethylazodicarboxamide (77.3 mg, 0.449 mmol). After the mixture was stirred at 40 °C for 2 hours, the reaction mixture was evaporated. The residue was purified by column chromatography on silica gel (acetone-hexane), thereby obtaining (13,13-difluoro-6,8,9-trimethyl-13,14-dihydro-8H,12H-imidazo[4",5":3',4']benzo[1',2':8,9][1,5]oxazonino[7,6,5-hi]indol-2-yl)(3,4,5-trifluorophenyl)methanone (84.4 mg, 0.161 mmol).

### Step 7:

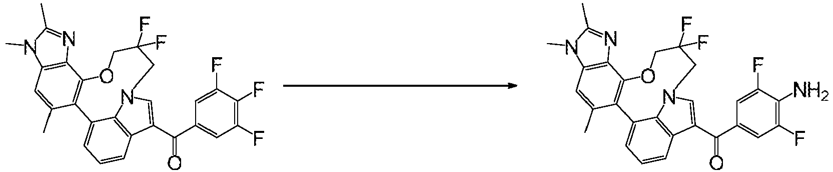

To a mixture of (13,13-difluoro-6,8,9-trimethyl-13,14-dihydro-8H,12H-imidazo[4",5":3',4']benzo[1',2':8,9][1,5]oxazonino[7,6,5-hi]indol-2-yl)(3,4,5-trifluorophenyl)methanone (84.0 mg, 0.160 mmol) in 1,4-dioxane (400 µL) was added ammonium hydroxide (3.40 mL, 29.0 mmol). After reacting by microwave apparatus at 130 °C for 14 hours, the mixture was diluted with EtOAc and water. The organic layer was washed successively with H₂O then brine, dried over sodium sulfate, and evaporated, thereby obtaining crude (4-amino-3,5-difluorophenyl)(13,13-difluoro-6,8,9-trimethyl-13,14-dihydro-8H,12H-imidazo[4",5":3',4']benzo[1',2':8,9][1,5]oxazonino[7,6,5-hi]indol-2-yl)methanone (113 mg, 0.220 mmol).

### Step 8:

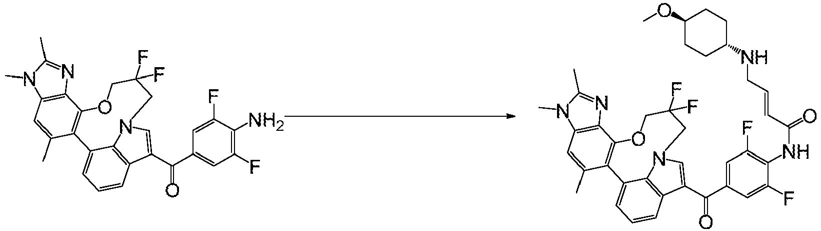

A solution of (4-amino-3,5-difluorophenyl)(13,13-difluoro-6,8,9-trimethyl-13,14-dihydro-8H,12H-imidazo[4",5":3',4']benzo[1',2':8,9][1,5]oxazonino[7,6,5-hi]indol-2-yl)methanone (69.0 mg, 0.132 mmol), 4-chlorocrotonic acid (23.9 mg, 0.198 mmol) and propylphosphonic anhydride solution (50 wt% in THF, 248 µL, 0.396 mmol) in DMF (1.00 mL) was stirred at ambient temperature for 20 minutes. Then triethylamine (110 µL, 0.792 mmol) was added thereto, and the mixture was stirred at 35 °C for 30 minutes. To the resulting mixture was added saturated aqueous NaHCO₃, and extracted with EtOAc, and washed successively with H₂O then brine, dried over sodium sulfate, and evaporated. The residue was used for the next reaction without further purification.

A solution of (E)-4-chloro-N-(4-(13,13-difluoro-6,8,9-trimethyl-13,14-dihydro-8H,12H-imidazo[4",5":3',4']benzo[1',2':8,9][1,5]oxazonino[7,6,5-hi]indole-2-carbonyl)-2,6-difluorophenyl)but-2-enamide (82.5 mg, 0.132 mmol), trans-4-methoxycyclohexanamine hydrochloride (43.7 mg, 0.264 mmol), KI (65.7 mg, 0.396 mmol) and K₃PO₄ (112 mg, 0.528 mmol) in DMF (1.00 mL) was stirred at at 35 °C for 1 hour. To the resulting mixture was added saturated aqueous NaHCO₃, and extracted with EtOAc, and washed successively with H₂O then brine, dried over sodium sulfate, and evaporated. The residue was purified by preparative HPLC (water:acetonitrile (0.1% formic acid)) to give the title compound (38.7 mg, 0.0539 mmol). ¹H-NMR: (400 MHz, CHLOROFORM-D) δ 8.46 (dd, J = 8.0, 1.3 Hz, 1H), 7.60 (d, J = 2.0 Hz, 1H), 7.52-7.47 (m, 2H), 7.41 (t, J = 7.8 Hz, 1H), 7.16-7.09 (m, 3H), 6.25 (d, J = 15.3 Hz, 1H), 5.81-5.70 (m, 1H), 4.81 (dd, J = 26.1, 14.6 Hz, 1H), 4.56 (td, J = 14.5, 9.9 Hz, 1H), 4.09-4.01 (m, 1H), 3.75 (s, 3H), 3.52 (dd, J = 5.3, 1.5 Hz, 2H), 3.35 (s, 3H), 3.19- 3.12 (m, 1H), 2.64 (s, 3H), 2.58-2.51 (m, 1H), 2.34 (s, 3H), 2.10-1.98 (m, 4H), 1.34-1.10 (m, 4H); ESI-MS [M+H]⁺: 718.4.

### Synthetic procedure D

### Example 27

### (E)-N-(2,6-difluoro-4-(6,8,9-trimethyl-8,11,12,13-tetrahydroimidazo[4",5":3',4']benzo[1',2':4,5]azocino[3,2,1-hi]indole-2-carbonyl)phenyl)-4-(((1r,4r)-4-methoxycyclohexyl)amino)but-2-enamide

### Step 1:

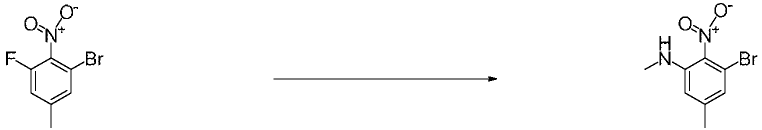

Methylamine (2M THF solution, 22.4 mL, 44.9 mmol) was added to a solution of 1-bromo-3-fluoro-5-methyl-2-nitrobenzene (3.50 g, 15.0 mmol) in DMSO (30.0 mL). After stirring at ambient temperature for 5 hours, EtOAc and saturated aqueous NaHCO₃ were added to the reaction mixture, then extracted with EtOAc, and washed successively with H₂O then brine, dried over sodium sulfate, and evaporated. Thereby obtaining crude 3-bromo-N,5-dimethyl-2-nitroaniline (3.75 g, 15.3 mmol).

### Step 2:

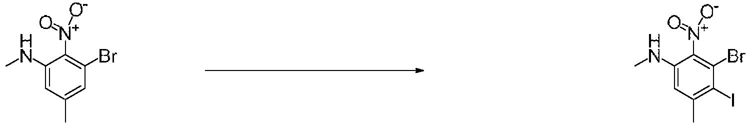

N-Iodosuccinimide (3.54 g, 15.7 mmol) was added to a solution of 3-bromo-N,5-dimethyl-2-nitroaniline (3.67 g, 15.0 mmol) in acetic acid (70.0 mL). After the mixture was stirred at 50 °C for 10 minutes, the mixture was cooled to ambient temperature and water (150 mL) was added to the reaction mixture. The resulting solid was collected by filtration, and vacuum-dried at 50 °C for 1 day, thereby obtaining crude 3-bromo-4-iodo-N,5-dimethyl-2-nitroaniline (5.14 g, 13.9 mmol)

### Step 3:

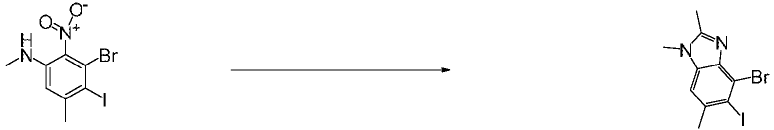

Acetic anhydride (6.23 mL, 64.7 mmol) was added to 3-bromo-4-iodo-N,5-dimethyl-2-nitroaniline (3.00 g, 8.09 mmol). After the mixture was stirred at 180 °C for 2 hours, the mixture was cooled to ambient temperature and water (10.0 mL) was added to the reaction mixture. The resulting solid was collected by filtration, and vacuum-dried at 50 °C for 1 day, thereby obtaining crude N-(3-bromo-4-iodo-5-methyl-2-nitrophenyl)-N-methylacetamide (2.69 g, 6.50 mmol).

Iron (2.53 g, 45.3 mmol) was added to a solution of N-(3-bromo-4-iodo-5-methyl-2-nitrophenyl)-N-methylacetamide (2.69 g, 6.50 mmol) in acetic acid (10.0 mL). After the mixture was stirred at 90 °C for 10 minutes, the mixture was cooled to ambient temperature. EtOAc was added to the reaction mixture and insoluble matter was filtered off through CELITE pad and the mixture was extracted with EtOAc and H₂O, and washed successively with H₂O then brine, dried over sodium sulfate, and evaporated. The residue was purified by column chromatography on silica gel (methanol-EtOAc-hexane), thereby obtaining 4-bromo-5-iodo-1,2,6-trimethyl-1H-benzo[d]imidazole (1.94 g, 5.31 mmol).

### Step 4:

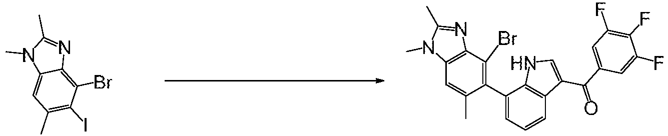

To a mixture of (7-(5,5-dimethyl-1,3,2-dioxaborinan-2-yl)-1H-indol-3-yl)(3,4,5-trifluorophenyl)methanone (223 mg, 0.58 mmol), 4-bromo-5-iodo-1,2,6-trimethyl-1H-benzo[d]imidazole (150 mg, 0.41 mmol) and Pd(PPh₃)₄ (47.5 mg, 0.041 mmol) in 1,2-dimethoxyethane (2.0 mL) was added 2M aqueous sodium carbonate solution (0.411 mL, 0.82 mmol). After reacting by microwave apparatus at 110 °C for 2 hrs, the mixture was diluted with EtOAc and water. The organic layer was washed with brine, dried over sodium sulfate and evaporated under reduced pressure. The residue was purified by column chromatography on silica gel (CHCl₃-methanol) to give (7-(4-bromo-1,2,6-trimethyl-1H-benzo[d]imidazol-5-yl)-1H-indol-3-yl)(3,4,5-trifluorophenyl)methanone (184 mg).

### Step 5:

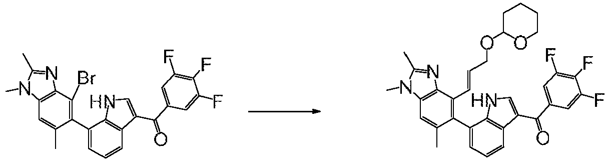

To a mixture of (7-(4-bromo-1,2,6-trimethyl-1H-benzo[d]imidazol-5-yl)-1H-indol-3-yl)(3,4,5-trifluorophenyl)methanone (158 mg), (E)-4,4,5,5-tetramethyl-2-(3-((tetrahydro-2H-pyran-2-yl)oxy)prop-1-en-1-yl)-1,3,2-dioxaborolane (250 mg, 0.93 mmol) and Pd(PPh₃)₄ (35.6 mg, 0.031 mmol) in 1,2-dimethoxyethane (4.0 mL) was added 2M aqueous sodium carbonate solution (0.463 mL, 0.93 mmol). After reacting by microwave apparatus at 120 °C for 6 hrs, the mixture was diluted with EtOAc and water. The organic layer was washed with brine, dried over sodium sulfate and evaporated under reduced pressure. The residue was purified by column chromatography on silica gel (CHCl₃-methanol) to give crude (E)-(3,4,5-trifluorophenyl)(7-(1,2,6-trimethyl-4-(3-((tetrahydro-2H-pyran-2-yl)oxy)prop-1-en-1-yl)-1H-benzo[d]imidazol-5-yl)-1H-indol-3-yl)methanone (116 mg).

### Step 6:

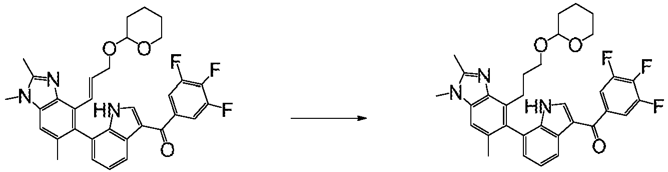

To a mixture of crude (E)-(3,4,5-trifluorophenyl)(7-(1,2,6-trimethyl-4-(3-((tetrahydro-2H-pyran-2-yl)oxy)prop-1-en-1-yl)-1H-benzo[d]imidazol-5-yl)-1H-indol-3-yl)methanone (116 mg) in MeOH (3.0 mL) and THF (0.3 mL) was added Palladium on carbon (10 wt.%, 100 mg). The mixture was stirred at RT overnight under hydrogen atmosphere. The reaction mixture was filtered through CELITE, rinsing with CHCl₃ and MeOH, and the filtrate was evaporated. The residue was purified by column chromatography on silica gel (CHCl₃-methanol) to give (3,4,5-trifluorophenyl)(7-(1,2,6-trimethyl-4-(3-((tetrahydro-2H-pyran-2-yl)oxy)propyl)-1H-benzo[d]imidazol-5-yl)-1H-indol-3-yl)methanone (43 mg).

### Step 7:

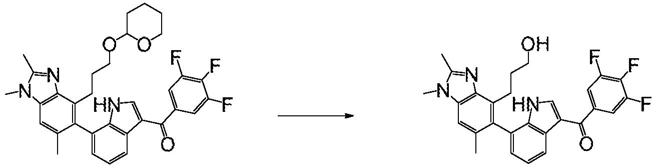

To a mixture of (3,4,5-trifluorophenyl)(7-(1,2,6-trimethyl-4-(3-((tetrahydro-2H-pyran-2-yl)oxy)propyl)-1H-benzo[d]imidazol-5-yl)-1H-indol-3-yl)methanone (43 mg) in CHCl₃ (1.0 mL) was added TFA (0.56 mL). The mixture was stirred at RT for 1 hr and evaporated. The residue was purified by column chromatography on amino silica gel (EtOAc-Hexane) to give (7-(4-(3-hydroxypropyl)-1,2,6-trimethyl-1H-benzo[d]imidazol-5-yl)-1H-indol-3-yl)(3,4,5-trifluorophenyl)methanone (26 mg).

### Step 8:

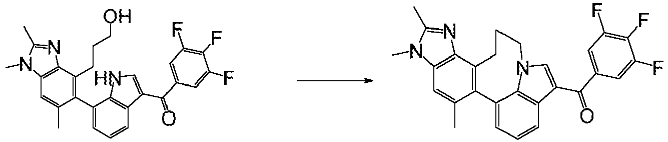

To a mixture of (7-(4-(3-hydroxypropyl)-1,2,6-trimethyl-1H-benzo[d]imidazol-5-yl)-1H-indol-3-yl)(3,4,5-trifluorophenyl)methanone (25 mg) and P(n-Bu3) (0.038 mL, 0.15 mmol) in THF (2.5 mL) was added TMAD (26.3 mg, 0.15 mmol) at 0 °C. The mixture was stirred at RT for 1 hr, then P(n-Bu3) (0.038 mL, 0.15 mmol) was added at RT, and TMAD (26.3 mg, 0.15 mmol) was added at 0 °C. The mixture was stirred at RT for 15 minutes, then additional P(n-Bu3) (0.038 mL, 0.15 mmol) was added at RT, and ]additional TMAD (26.3 mg, 0.15 mmol) was added at 0 °C. The mixture was stirred at RT for 1 hr. The mixture was diluted with EtOAc and water, and extracted with EtOAc. The organic layer was washed with H₂O, sat. NaHCO₃ aq., and brine, dried over sodium sulfate and evaporated under reduced pressure. The residue was purified by column chromatography on silica gel (EtOAc-Hexane) to give crude (3,4,5-trifluorophenyl)(6,8,9-trimethyl-8,11,12,13-tetrahydroimidazo[4",5":3',4']benzo[1',2':4,5]azocino[3,2,1-hi]indol-2-yl)methanone (27 mg).

### Step 9:

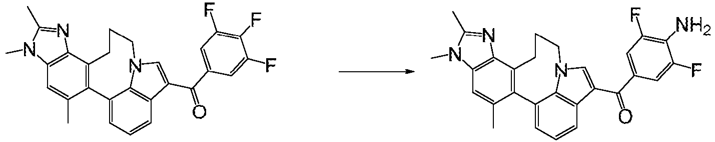

To a mixture of crude (3,4,5-trifluorophenyl)(6,8,9-trimethyl-8,11,12,13-tetrahydroimidazo[4",5":3',4']benzo[1',2':4,5]azocino[3,2,1-hi]indol-2-yl)methanone (27 mg) in 1,4-Dioxane (1 mL) was added 28% NH₃ aq. (1.63 mL) at RT. After reacting by microwave apparatus at 130 °C for 12 hrs, the mixture was diluted with EtOAc and water. The organic layer was washed with H₂O and brine, dried over sodium sulfate and evaporated under reduced pressure. The residue was purified by column chromatography on silica gel (EtOAc-Hexane, CHCl₃-MeOH, and Acetone-Hexane) to give (4-amino-3,5-difluorophenyl)(6,8,9-trimethyl-8,11,12,13-tetrahydroimidazo[4",5":3',4']benzo[1',2':4,5]azocino[3,2,1-hi]indol-2-yl)methanone (20 mg).

### Step 10:

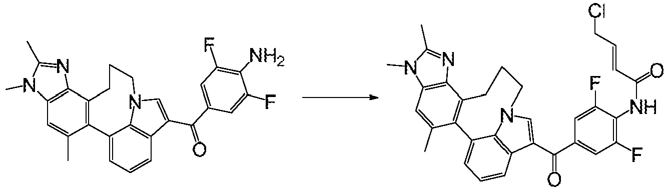

A solution of (4-amino-3,5-difluorophenyl)(6,8,9-trimethyl-8,11,12,13-tetrahydroimidazo[4",5":3',4']benzo[1',2':4,5]azocino[3,2,1-hi]indol-2-yl)methanone (20 mg), 4-chlorocrotonic acid (6.1 mg, 0.051 mmol) and propylphosphonic anhydride solution (50 wt% in THF, 0.061 mL, 0.097 mmol) in DMF (0.5 mL) was stirred at RT for 50 min. Then triethylamine (0.022 mL, 0.16 mmol) was added thereto, and the mixture was stirred at RT for 3 hrs. To the resulting mixture was added saturated aqueous NaHCO₃ and water, and extracted with EtOAc, and the organic layer was washed successively with H₂O, saturated aqueous NaHCO₃ then brine, dried over sodium sulfate, and evaporated to give crude (E)-4-chloro-N-(2,6-difluoro-4-(6,8,9-trimethyl-8,11,12,13-tetrahydroimidazo[4",5":3',4']benzo[1',2':4,5]azocino[3,2,1-hi]indole-2-carbonyl)phenyl)but-2-enamide (22 mg).

### Step 11:

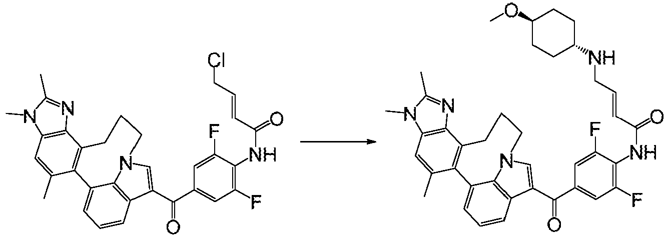

A solution of crude (E)-4-chloro-N-(2,6-difluoro-4-(6,8,9-trimethyl-8,11,12,13-tetrahydroimidazo[4",5":3',4']benzo[1',2':4,5]azocino[3,2,1-hi]indole-2-carbonyl)phenyl)but-2-enamide (22 mg), trans-4-methoxycyclohexanamine hydrochloride (16 mg, 0.097 mmol), KI (19 mg, 0.12 mmol) and K₃PO₄ (41 mg, 0.19 mmol) in DMF (0.5 mL) was stirred at 30 °C for 3 hrs. To the resulting mixture was added H₂O, and extracted with EtOAc, and the organic layer was washed successively with H₂O then brine, dried over sodium sulfate, and evaporated. The residue was purified by column chromatography on silica gel (CHCl₃-MeOH) to give the title compound (18 mg). ¹H-NMR: (400 MHz, CHLOROFORM-D) δ 8.44 (dd, J = 7.9, 1.0 Hz, 1H), 8.00-7.75 (brs, 1H), 7.51 (s, 1H), 7.40 (d, J = 7.8 Hz, 2H), 7.35 (d, J = 7.7 Hz, 1H), 7.18 (s, 1H), 7.14-7.08 (m, 2H), 6.33 (d, J = 15.4 Hz, 1H), 3.98-3.83 (m, 2H), 3.75 (s, 3H), 3.71-3.64 (m, 2H), 3.52-3.51 (m, 2H), 3.34 (s, 3H), 3.18-3.11 (m, 1H), 2.63 (s, 3H), 2.60-2.52 (m, 2H), 2.41 (s, 3H), 2.18-1.90 (m, 6H), 1.23-1.12 (m, 4H); ESI-MS [M+H]⁺: 666.6.

### Synthetic procedure E

### Example 30-1 and 30-2

### (E)-N-(4-(9,10-dimethyl-7-(trifluoromethyl)-13,14-dihydro-9H-imidazo[4",5":5',6']benzo[1',2':2,3]oxocino[4,5,6-hi]indolizine-2-carbonyl)-2,6-difluorophenyl)-4-(((1ry4r)-4-methoxycyclohexyl)amino)but-2-enamide

### Step 1:

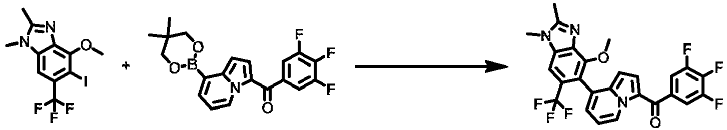

A solution of 5-iodo-4-methoxy-1,2-dimethyl-6-(trifluoromethyl)-1H-benzo[d]imidazole (4.62 g, 12.5 mmol), (8-(5,5-dimethyl-1,3,2-dioxaborinan-2-yl)indolizin-3-yl)(3,4,5-trifluorophenyl)methanone (6.28 g, 16.2 mmol), Pd(PPh₃)₄ (1.44 g, 1.25 mmol) in 1,4-Dioxane (42 mL) and 2M Na₂CO₃ aq. (12.2 mL, 24.3 mmol) was evacuated and N₂ back-filled before heating to 100 °C for 12 h. After cooling, the reaction mixture was diluted with EtOAc. The resulting suspension was filtered, rinsing with EtOAc, and the filtrate was washed with H₂O and brine then dried over Na₂SO₄ and evaporated. The residue was purified by column chromatography on silica gel (hexane/acetone), thereby obtaining (8-(4-methoxy-1,2-dimethyl-6-(trifluoromethyl)-1H-benzo[d]imidazol-5-yl)indolizin-3-yl)(3,4,5-trifluorophenyl)methanone (5.16 g, 80%).

### Step 2:

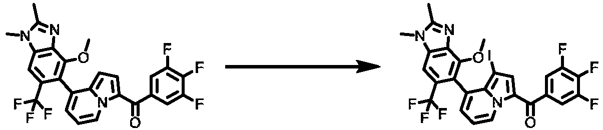

N-iodosuccinimide (2.12 g, 9.44 mmol) was added to a solution of (8-(4-methoxy-1,2-dimethyl-6-(trifluoromethyl)-1H-benzo[d]imidazol-5-yl)indolizin-3-yl)(3,4,5-trifluorophenyl)methanone (5.16 g, 9.97 mmol) in THF (40 mL) and AcOH (6.7 mL). After stirring at RT for 30 min, the reaction mixture was diluted with EtOAc, washed with sat. NaHCO₃ aq., H₂O(2x) and brine then dried over Na₂SO₄ and evaporated. The residue was purified by column chromatography on silica gel (hexane/acetone), thereby obtaining (1-iodo-8-(4-methoxy-1,2-dimethyl-6-(trifluoromethyl)-1H-benzo[d]imidazol-5-yl)indolizin-3-yl)(3,4,5-trifluorophenyl)methanone (6.42 g, 100%).

### Step 3:

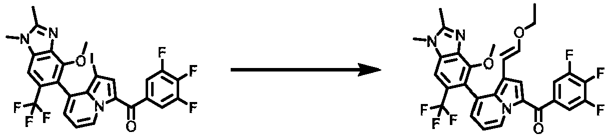

A solution of (1-iodo-8-(4-methoxy-1,2-dimethyl-6-(trifluoromethyl)-1H-benzo[d]imidazol-5-yl)indolizin-3-yl)(3,4,5-trifluorophenyl)methanone (5.00 g, 7.77 mmol), 2-[(E)-2-ethoxyvinyl]-4,4,5,5-tetramethyl-1,3,2-dioxaborolane (4.94 mL, 23.3 mmol), PdCl₂(dppf)·CH₂Cl₂ (635 mg, 0.777 mmol) in 1,4-Dioxane (39 mL) and 2M Na₂CO₃ aq. (17.5 mL, 35.0 mmol) was evacuated and N₂ back-filled before heating to 100 °C for 3 h. After cooling, the reaction mixture was diluted with EtOAc, washed with H₂O and brine then dried over Na₂SO₄ and evaporated. The residue was purified by column chromatography on silica gel (hexane/acetone), thereby obtaining (E)-(1-(2-ethoxyvinyl)-8-(4-methoxy-1,2-dimethyl-6-(trifluoromethyl)-1H-benzo[d]imidazol-5-yl)indolizin-3-yl)(3,4,5-trifluorophenyl)methanone (3.91 g, 86%).

### Step 4:

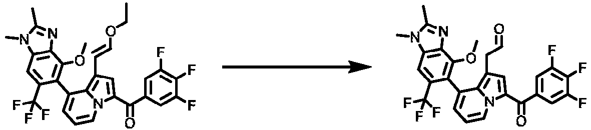

6M HCl aq. (11.1 mL, 66.6 mmol) was added to a solution of (E)-(1-(2-ethoxyvinyl)-8-(4-methoxy-1,2-dimethyl-6-(trifluoromethyl)-1H-benzo[d]imidazol-5-yl)indolizin-3-yl)(3,4,5-trifluorophenyl)methanone (3.91 g, 6.66 mmol) in THF (33 mL). After stirring at RT for 3 h, further 6M HCl aq. (2.8 mL, 16.7 mmol) was added and stirring continued for 1 h. The reaction mixture was diluted with EtOAc and quenched with sat.NaHCO₃ aq. at 0 °C. The organic layer was washed with brine then dried over Na₂SO₄ and evaporated. The residue was purified by column chromatography on silica gel (hexane/acetone), thereby obtaining 2-(8-(4-methoxy-1,2-dimethyl-6-(trifluoromethyl)- 1H-benzo[d]imidazol-5-yl)-3-(3,4,5-trifluorobenzoyl)indolizin-1-yl)acetaldehyde (2.94 g, 79%).

### Step 5:

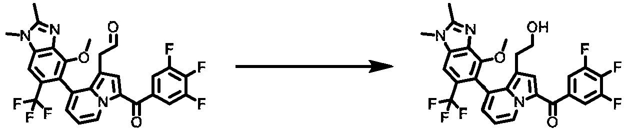

Sodium triacetoxyborohydride (1.63 g, 7.68 mmol) was added to a solution of 2-(8-(4-methoxy-1,2-dimethyl-6-(trifluoromethyl)-1H-benzo[d]imidazol-5-yl)-3-(3,4,5-trifluorobenzoyl)indolizin-1-yl)acetaldehyde (2.87 g, 5.12 mmol) and AcOH (440 µL, 7.68 mmol) in THF (26 mL). After stirring at RT for 1.5 h, further AcOH (147 µL, 2.56 mmol) and sodium triacetoxyborohydride (542 mg, 2.56 mmol) were added and stirring continued for 1 h. Further AcOH (147 µL, 2.56 mmol) and sodium triacetoxyborohydride (542 mg, 2.56 mmol) were added and stirring continued at 50 °C for 20 min. The organic layer was washed with brine then dried over Na₂SO₄ and evaporated. The residue was purified by column chromatography on silica gel (hexane/acetone), thereby obtaining (1-(2-hydroxyethyl)-8-(4-methoxy-1,2-dimethyl-6-(trifluoromethyl)-1H-benzo[d]imidazol-5-yl)indolizin-3-yl)(3,4,5-trifluorophenyl)methanone (2.20 g, 77%).

### Step 6:

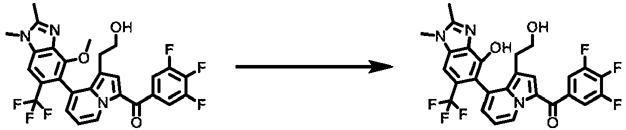

BBr₃ (1.0 M in CH₂Cl₂, 52.8 mL, 52.8 mmol) was added to a solution of (1-(2-hydroxyethyl)-8-(4-methoxy-1,2-dimethyl-6-(trifluoromethyl)-1H-benzo[d]imidazol-5-yl)indolizin-3-yl)(3,4,5-trifluorophenyl)methanone (1.97 g, 3.52 mmol) in CH₂Cl₂ (12 mL) at 0°C. After stirring at 30°C for 5 h, CHCl₃/MeOH(30%) and sat. NaHCO₃ aq. were added to the reaction mixture at 0 °C. The aqueous layer was extracted with CHCl₃/MeOH(10%) and combined organics were washed with brine then dried over Na₂SO₄ and evaporated. The residue was purified by column chromatography on silica gel (hexane/acetone), thereby obtaining (8-(4-hydroxy-1,2-dimethyl-6-(trifluoromethyl)-1H-benzo[d]imidazol-5-yl)-1-(2-hydroxyethyl)indolizin-3-yl)(3,4,5-trifluorophenyl)methanone (1.80 g, 94%).

### Step 7:

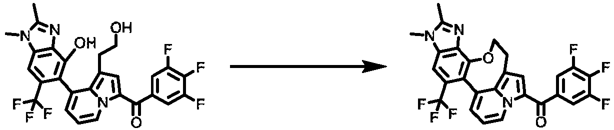

TMAD (849 mg, 4.93 mmol) was added to a solution of (8-(4-hydroxy-1,2-dimethyl-6-(trifluoromethyl)-1H-benzo[d]imidazol-5-yl)-1-(2-hydroxyethyl)indolizin-3-yl)(3,4,5-trifluorophenyl)methanone (1.80 g, 3.29 mmol) and P(n-Bu)3 (1.23 mL, 4.93 mmol) in THF (165 mL) at 0°C. After stirring at RT for 2 h, further P(n-Bu)3 (0.820 mL, 3.29 mmol) and TMAD (568 mg, 3.29 mmol) were added at 0°C and stirring continued for 1 h at RT. The resulting suspension was filtered, rinsing with EtOAc, and the filtrate was washed with NaHCO₃ aq., H₂O and brine then dried over Na₂SO₄ and evaporated. The residue was purified by column chromatography on silica gel (hexane/acetone) and suspended in MeOH, stirred at 50 °C. After cooling, the solid was collected by filtration, washing with further MeOH, and vacuum-dried, thereby obtaining (9,10-dimethyl-7-(trifluoromethyl)-13,14-dihydro-9H-imidazo[4",5":5',6']benzo[1',2':2,3]oxocino[4,5,6-hi]indolizin-2-yl)(3,4,5-trifluorophenyl)methanone (1.05 g, 60%).

### Step 8:

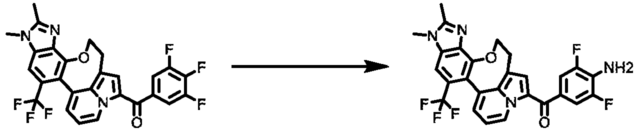

Ammonolysis reaction of (9,10-dimethyl-7-(trifluoromethyl)-13,14-dihydro-9H-imidazo[4",5":5',6']benzo[1',2':2,3]oxocino[4,5,6-hi]indolizin-2-yl)(3,4,5-trifluorophenyl)methanone (1.05 g, 1.98 mmol) was carried out in five batches. 28% NH₃ aq. (15.8 mL, 114 mmol) was added to starting material (200 mg, 0.377 mmol) in 1,4-Dioxane (2 mL). The reaction mixture was heated at 130 °C by microwave irradiation for 24 h. The other batches were carried out under similar conditions. After cooling, the mixture was diluted with EtOAc, washed with H₂O and brine then dried over Na₂SO₄ and evaporated. The residue was purified by column chromatography on silica gel (CHCl₃/MeOH), thereby obtaining (4-amino-3,5-difluorophenyl)(9,10-dimethyl-7-(trifluoromethyl)-13,14-dihydro-9H-imidazo[4",5":5',6']benzo[1',2':2,3]oxocino[4,5,6-hi]indolizin-2-yl)methanone (989 mg, 95%).

### Step 9:

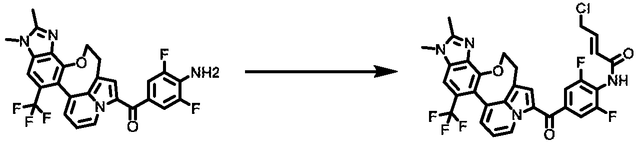

A solution of (4-amino-3,5-difluorophenyl)(9,10-dimethyl-7-(trifluoromethyl)-13,14-dihydro-9H-imidazo[4",5":5',6']benzo[1',2':2,3]oxocino[4,5,6-hi]indolizin-2-yl)methanone (748 mg, 1.42 mmol), 4-chlorocrotonic acid (318 mg, 2.64 mmol) and propylphosphonic anhydride solution (50 wt% in THF, 1.65 mL, 2.64 mmol) in DMF (7.1 mL) was stirred at RT for 15 min. Then Et₃N (594 µL, 4.26 mmol) was added thereto, and the mixture was stirred at RT for 1.5 h. The reaction mixture was diluted with EtOAc, washed with NaHCO₃ aq., H₂O and brine then dried over Na₂SO₄ and evaporated. The residue was suspended in isopropyl ether (20 mL), stirred at RT. The solid was collected by filtration, washing with further isopropyl ether, and vacuum-dried at RT, thereby obtaining (E)-4-chloro-N-(4-(9,10-dimethyl-7-(trifluoromethyl)-13,14-dihydro-9H-imidazo[4",5":5',6']benzo[1',2':2,3]oxocino[4,5,6-hi]indolizine-2-carbonyl)-2,6-difluorophenyl)but-2-enamide (794 mg, 89%).

### Step 10:

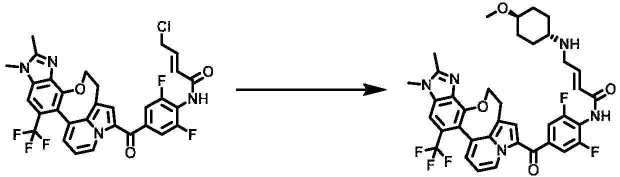

A solution of (E)-4-chloro-N-(4-(9,10-dimethyl-7-(trifluoromethyl)-13,14-dihydro-9H-imidazo[4",5":5',6']benzo[1',2':2,3]oxocino[4,5,6-hi]indolizine-2-carbonyl)-2,6-difluorophenyl)but-2-enamide (254 mg, 0.404 mmol), trans-4-methoxycyclohexanamine hydrochloride (201 mg, 1.21 mmol), KI (201 mg, 1.21 mmol) and K₃PO₄ (429 mg, 2.02 mmol) in DMF (1.3 mL) was stirred at 30 °C for 1 h. The reaction mixture was diluted with EtOAc, washed with H₂O(2x) and brine then dried over Na₂SO₄ and evaporated. The residue was purified by column chromatography on silica gel (CHCl₃(0.1%Et₃N)/MeOH), thereby obtaining the racemic title compound (250 mg, 86%). The racemate was separated by CHIRAL ART SB(YMC) (hexane-EtOH(0.1%Et₃N)) to give the title compound (Example 30-1, 96.2 mg, 38%; single isomer, 1st-eluting isomer and Example 30-2, 87.8 mg, 35%; single isomer, 2nd-eluting isomer). ¹H-NMR: (400 MHz, CHLOROFORM-D) δ 9.98 (dd, J = 7.0, 1.0 Hz, 1H), 7.64 (s, 1H), 7.44-7.39 (m, 2H), 7.17-7.08 (m, 4H), 7.02 (dd, J = 7.1, 7.1 Hz, 1H), 6.26 (dt, J = 15.3, 1.6 Hz, 1H), 4.73 (dd, J = 10.4, 4.4 Hz, 1H), 4.23-4.17 (m, 1H), 3.84 (s, 3H), 3.51 (dd, J = 5.1, 1.6 Hz, 2H), 3.35 (s, 3H), 3.18-3.11 (m, 1H), 2.88-2.80 (m, 1H), 2.70 (s, 3H), 2.61-2.50 (m, 2H), 2.13-1.97 (m, 4H), 1.33-1.09 (m, 4H); ESI-MS [M+H]⁺: 722.5

### Example 31-1 and 31-2

### (E)-N-(4-(9,10-dimethyl-7-(trifluoromethyl)-13,14-dihydro-9H-imidazo[4",5":5',6']benzo[1',2':2,3]oxocino[4,5,6-hi]indolizine-2-carbonyl)-2,6-difluorophenyl)-4-((3-methyloxetan-3-yl)amino)but-2-enamide

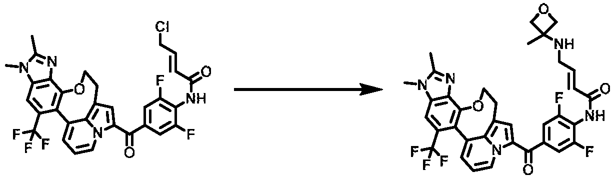

A solution of (E)-4-chloro-N-(4-(9,10-dimethyl-7-(trifluoromethyl)-13,14-dihydro-9H-imidazo[4",5":5',6']benzo[1',2':2,3]oxocino[4,5,6-hi]indolizine-2-carbonyl)-2,6-difluorophenyl)but-2-enamide (200 mg, 0.318 mmol), 3-methyloxetan-3-amine (141 µL, 1.59 mmol), KI (106 mg, 0.636 mmol) in DMSO (3.18 mL) was stirred at 30 °C for 6 h. The reaction mixture was diluted with EtOAc, washed with H₂O and brine then dried over Na₂SO₄ and evaporated. The residue was purified by column chromatography on silica gel (CHCl₃/MeOH), thereby obtaining the racemic title compound (210 mg). The racemate was separated by CHIRAL ART SB(YMC) (hexane-EtOH(0.1%Et₃N)) to give the title compound (Example 31-1, 80.1 mg, 37%; single isomer, 1st-eluting isomer and Example 31-2, 87.4 mg, 40%; single isomer, 2nd-eluting isomer). ¹H-NMR: (400 MHz, CHLOROFORM-D) δ 9.99 (dd, J = 7.0, 1.0 Hz, 1H), 7.65 (s, 1H), 7.45-7.39 (m, 2H), 7.19-7.10 (m, 4H), 7.02 (dd, J = 7.2, 7.2 Hz, 1H), 6.32 (dt, J = 15.3, 1.8 Hz, 1H), 4.73 (dd, J = 10.5, 4.3 Hz, 1H), 4.58 (d, J = 6.3 Hz, 2H), 4.43 (d, J = 6.5 Hz, 2H), 4.23-4.17 (m, 1H), 3.84 (s, 3H), 3.53 (dd, J = 5.0, 1.8 Hz, 2H), 2.91-2.80 (m, 1H), 2.70 (s, 3H), 2.57 (dd, J = 15.3, 2.3 Hz, 1H), 1.51 (s, 3H); ESI-MS [M+H]⁺: 680.4.

### Synthetic procedure F

### Example 42, 42-1, 42-2 and 43

### (E)-N-(2,6-difluoro-4-(9,10,13-trimethyl-7-(trifluoromethyl)-13,14-dihydro-9H-imidazo[4",5":5',6']benzo[1',2':2,3]oxocino[4,5,6-hi]indolizine-2-carbonyl)phenyl)-4-(((1r,4r)-4-methoxycyclohexyl)amino)but-2-enamide

### Step 1:

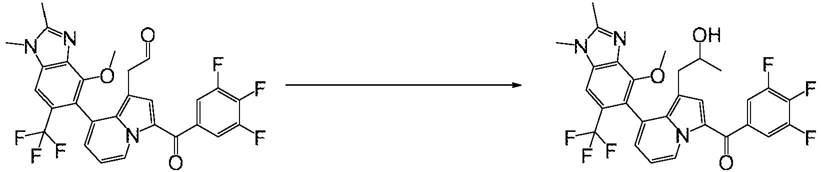

Methymagnesium bromide (3.40 M in THF, 196 µL, 0.665 mmol) was added to 2-(8-(4-methoxy-1,2-dimethyl-6-(trifluoromethyl)-1H-benzo[d]imidazol-5-yl)-3-(3,4,5-trifluorobenzoyl)indolizin-1-yl)acetaldehyde (186 mg, 0.332 mmol) in THF (250 µL) at - 78 °C. After the mixture was stirred at -78 °C for 5 minutes, the mixture was heated to ambient temperature, and was stirred for 30 minutes. The mixture was diluted with EtOAc and quenched by NH₄Cl, and extracted with EtOAc, and washed successively with H₂O then brine, dried over sodium sulfate, and evaporated. The residue was purified by column chromatography on silica gel (acetone-hexane), thereby obtaining (1-(2-hydroxypropyl)-8-(4-methoxy-1,2-dimethyl-6-(trifluoromethyl)-1H-benzo[d]imidazol-5-yl)indolizin-3-yl)(3,4,5-trifluorophenyl)methanone (182 mg, 0.316 mmol).

### Step 2:

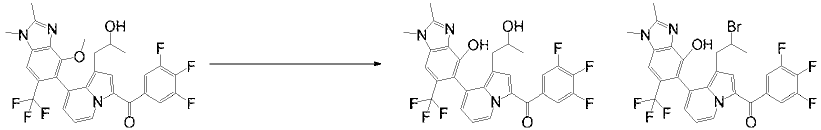

Boron tribromide (1.60 M in CH2Cl2, 4.74 mL, 4.74 mmol) was added to (1-(2-hydroxypropyl)-8-(4-methoxy-1,2-dimethyl-6-(trifluoromethyl)-1H-benzo[d]imidazol-5-yl)indolizin-3-yl)(3,4,5-trifluorophenyl)methanone (182 mg, 0.316 mmol) in CH₂Cl₂ (1.00 mL). After the mixture was stirred at 35 °C for 4 hours, the mixture was cooled to ambient temperature. The mixture was diluted with CHCl₃ and quenched by NH₃, and extracted with CHCl₃, and washed successively with H₂O then brine, dried over sodium sulfate, and evaporated. The residue was purified by column chromatography on silica gel (methanol-acetone-hexane), thereby obtaining (8-(4-hydroxy-1,2-dimethyl-6-(trifluoromethyl)-1H-benzo[d]imidazol-5-yl)-1-(2-hydroxypropyl)indolizin-3-yl)(3,4,5-trifluorophenyl)methanone (37.3 mg, 0.0664 mmol) and (1-(2-bromopropyl)-8-(4-hydroxy-1,2-dimethyl-6-(trifluoromethyl)-1H-benzo[d]imidazol-5-yl)indolizin-3-yl)(3,4,5-trifluorophenyl)methanone (116 mg, 0.185 mmol).

### Step 3:

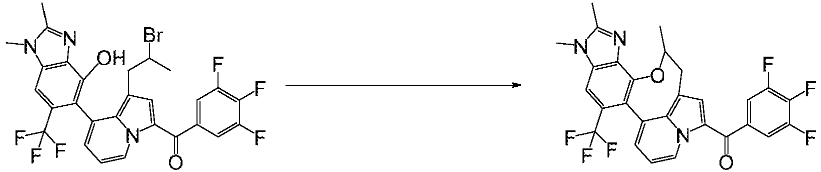

Potassium carbonate (103 mg, 0.186 mmol) was added to (1-(2-bromopropyl)-8-(4-hydroxy-1,2-dimethyl-6-(trifluoromethyl)-1H-benzo[d]imidazol-5-yl)indolizin-3-yl)(3,4,5-trifluorophenyl)methanone (116 mg, 0.186 mmol) in DMSO (15.0 mL). After the mixture was stirred at 40 °C for 1 hour, the mixture was cooled to ambient temperature. The mixture was diluted with EtOAc and water. The organic layer was washed successively with H₂O then brine, dried over sodium sulfate, and evaporated. The residue was purified by column chromatography on silica gel (acetone-hexane), thereby obtaining (3,4,5-trifluorophenyl)(9,10,13-trimethyl-7-(trifluoromethyl)-13,14-dihydro-9H-imidazo[4",5":5',6']benzo[1',2':2,3]oxocino[4,5,6-hi]indolizin-2-yl)methanone (89.8 mg, 0.165 mmol).

### Step 4:

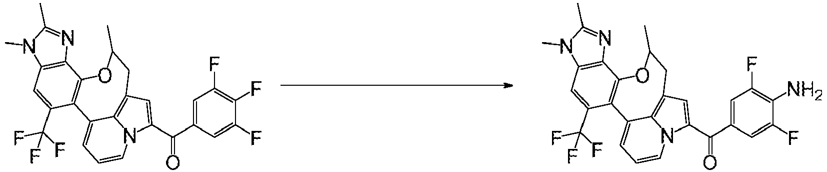

To a mixture of (3,4,5-trifluorophenyl)(9,10,13-trimethyl-7-(trifluoromethyl)-13,14-dihydro-9H-imidazo[4",5":5',6']benzo[1',2':2,3]oxocino[4,5,6-hi]indolizin-2-yl)methanone (89.0 mg, 0.160 mmol) in 1,4-dioxane (400 µL) was added ammonium hydroxide (3.40 mL, 29.0 mmol). After reacting by microwave apparatus at 130 °C for 14 hours, the mixture was diluted with EtOAc and water. The organic layer was washed successively with H₂O then brine, dried over sodium sulfate, and evaporated, thereby obtaining crude (4-amino-3,5-difluorophenyl)(9,10,13-trimethyl-7-(trifluoromethyl)-13,14-dihydro-9H-imidazo[4",5":5',6']benzo[1',2':2,3]oxocino[4,5,6-hi]indolizin-2-yl)methanone (79.0 mg, 0.150 mmol).

### Step 5:

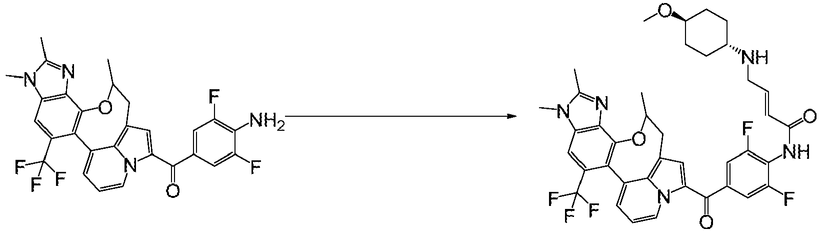

A solution of (4-amino-3,5-difluorophenyl)(9,10,13-trimethyl-7-(trifluoromethyl)-13,14-dihydro-9H-imidazo[4",5":5',6']benzo[1',2':2,3]oxocino[4,5,6-hi]indolizin-2-yl)methanone (79.0 mg, 0.146 mmol), 4-chlorocrotonic acid (26.5 mg, 0.220 mmol) and propylphosphonic anhydride solution (50 wt% in THF, 274 µL, 0.439 mmol) in DMF (1.00 mL) was stirred at ambient temperature for 20 minutes. Then triethylamine (122 µL, 0.878 mmol) was added thereto, and the mixture was stirred at 35 °C for 30 minutes. To the resulting mixture was added saturated aqueous NaHCO₃, and extracted with EtOAc, and washed successively with H₂O then brine, dried over sodium sulfate, and evaporated. The residue was used for the next reaction without further purification.

A solution of (E)-4-chloro-N-(2,6-difluoro-4-(9,10,13-trimethyl-7-(trifluoromethyl)-13,14-dihydro-9H-imidazo[4",5":5',6']benzo[1',2':2,3]oxocino[4,5,6-hi]indolizine-2-carbonyl)phenyl)but-2-enamide (94.0 mg, 0.146 mmol), trans-4-methoxycyclohexanamine hydrochloride (48.4 mg, 0.292 mmol), KI (72.8 g, 0.439 mmol) and K₃PO₄ (124 mg, 0.585 mmol) in DMF (1.00 mL) was stirred at at 35 °C for 1 hour. To the resulting mixture was added saturated aqueous NaHCO₃, and extracted with EtOAc, and washed successively with H₂O then brine, dried over sodium sulfate, and evaporated. The residue was purified by preparative HPLC (water:acetonitrile (0.1% formic acid)) to give Example 42 (29.6 mg, 0.0402 mmol) and crude Example 43 (4.76 mg). Example 42 was separated by CHIRALPAK ART SB(YMC) (hexane-ethanol(0.1%triethyl amine)) to give the title compound (2.64 mg, single isomer, 1^{st}-eluting isomer, Example 42-1) and (2.30 mg, single isomer, 2^{nd}-eluting isomer, Example 42-2). ¹H-NMR: (400 MHz, CHLOROFORM-D) δ 10.00 (d, J = 6.8 Hz, 1H), 7.63 (s, 1H), 7.44 (d, J = 8.0 Hz, 2H), 7.17-7.09 (m, 3H), 7.05-7.00 (m, 1H), 6.24 (d, J = 15.3 Hz, 1H), 4.97 (t, J = 5.6 Hz, 1H), 3.83 (s, 3H), 3.51 (d, J = 3.5 Hz, 2H), 3.36 (s, 3H), 3.18-3.14 (m, 1H), 2.97 (dd, J = 15.0, 5.0 Hz, 1H), 2.70 (s, 3H), 2.54 (t, J = 3.6 Hz, 1H), 2.33 (d, J = 15.0 Hz, 1H), 2.10-1.97 (m, 4H), 1.31-1.13 (m, 7H); ESI-MS [M+H]⁺: 736.5. The crude Example 43 was purified by column chromatography on silica gel (methanol-EtOAc-hexane), thereby obtaining Example 43 (1.36 mg, 0.00185 mmol). ¹H-NMR: (400 MHz, CHLOROFORM-D) δ 9.94 (d, J = 6.8 Hz, 1H), 7.63 (s, 1H), 7.44 (d, J = 8.0 Hz, 2H), 7.17-7.09 (m, 3H), 7.05-7.00 (m, 1H), 6.24 (d, J = 15.3 Hz, 1H), 4.53 (m, 1H), 3.82 (s, 3H), 3.51 (d, J = 3.5 Hz, 2H), 3.35 (s, 3H), 3.18-3.12 (m, 1H), 2.88 (dd, J = 15.0, 5.0 Hz, 1H), 2.67 (s, 3H), 2.59-2.52 (m, 1H), 2.33 (d, J = 15.0 Hz, 1H), 2.10-1.97 (m, 4H), 1.31-1.13 (m, 7H); ESI-MS [M+H]⁺: 736.5.

### Synthetic procedure G

### Example 44

### (E)-N-(2,6-difluoro-4-(6,8,9,11-tetramethyl-8,11,12,13-tetrahydroimidazo[4",5":5',6'lbenzo[1',2':5,6][1,4]diazocino[7,8,1-hi]indole-2-carbonyl)phenyl)-4-(((1r 4r)-4-methoxycyclohexyl)amino)but-2-enamide

### Step 1:

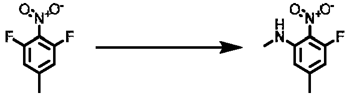

Methylamine (2M THF solution, 86 mL, 172 mmol) was added to a solution of 1,3-difluoro-5-methyl-2-nitrobenzene (11.9 g, 68.7 mmol) in DMSO (229 mL) under a water bath. After stirring at RT for 30 min, the reaction mixture was diluted with EtOAc and NaHCO₃ aq.. The resulting solid was removed by filtration, the organic layer was washed with H₂O(2x) and brine, the aqueous layer was extracted with EtOAc, washed with H₂O(2x) and brine. Combined organics were dried over Na₂SO₄ and evaporated, thereby obtaining crude 3-fluoro-N,5-dimethyl-2-nitroaniline (12.8 g, 101%).

### Step 2:

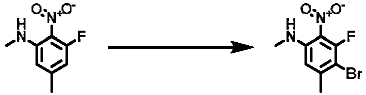

N-bromosuccinimide (13.8 g, 77.5 mmol) was added to a solution of 3-fluoro-N,5-dimethyl-2-nitroaniline (11.9 g, 64.6 mmol) in THF (215 mL). After stirring at RT for 2 h, the reaction mixture was diluted with EtOAc, washed with NaHCO₃ aq.(2x) and brine then dried over Na₂SO₄ and evaporated. The residue was suspended in isopropyl ether (238 mL), stirred at 50 °C. After cooling, the solid was collected by filtration, washing with further isopropyl ether, and vacuum-dried at 50 °C, thereby obtaining 4-bromo-3-fluoro-N,5-dimethyl-2-nitroaniline (14.0 g, 82%).

### Step 3:

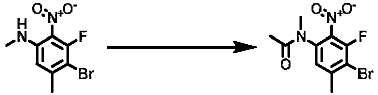

Acetylation of 4-bromo-3-fluoro-N,5-dimethyl-2-nitroaniline (13.0 g, 49.3 mmol) was carried out in two batches. Acetic anhydride (15.0 mL, 317 mmol) was added to the starting material (6.50 g, 24.7 mmol), then the reaction mixture was heated at 180 °C by microwave irradiation for 1 h. The other batch was carried out under the same conditions. After cooling, the reaction mixture was diluted with EtOAc, 5M NaOH aq. and NaHCO₃ aq. The organic layer was seperated and washed with NaHCO₃ aq. and brine then dried over Na₂SO₄ and evaporated. The residue was purified by column chromatography on silica gel (hexane/EtOAc), thereby obtaining N-(4-bromo-3-fluoro-5-methyl-2-nitrophenyl)-N-methylacetamide (14.6 g, 97%).

### Step 4:

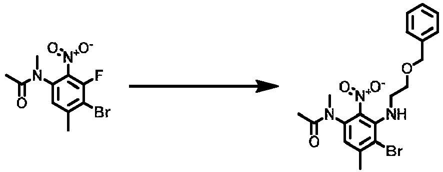

A solution of N-(4-bromo-3-fluoro-5-methyl-2-nitrophenyl)-N-methylacetamide (5.95 g, 19.5 mmol), 2-benzyloxyethanamine (4.38 mL, 29.3 mmol) and DIEA (6.68 mL, 39.0 mmol) in THF (65 mL) was stirred at 70 °C for 4 h. After cooling, the reaction mixture was diluted with EtOAc, washed with NH₄Cl aq.(3x) and brine then dried over Na₂SO₄ and evaporated. The residue was suspended in isopropyl ether (43 mL), stirred at 50 °C. After cooling, the solid was collected by filtration, washing with further isopropyl ether, and vacuum-dried at 40 °C, thereby obtaining N-(3-((2-(benzyloxy)ethyl)amino)-4-bromo-5-methyl-2-nitrophenyl)-N-methylacetamide (8.17 g, 96%).

### Step 5:

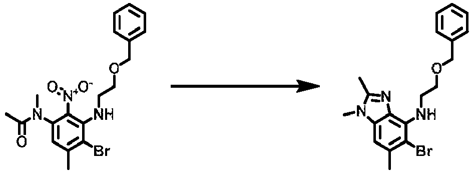

A suspension of N-(3-((2-(benzyloxy)ethyl)amino)-4-bromo-5-methyl-2-nitrophenyl)-N-methylacetamide (8.17 g, 18.7 mmol) and iron (10.5 g, 187 mmol) in AcOH (94 mL) was stirred at 60 °C for 3 h. After cooling, EtOAc was added to the reaction mixture then filtered through a CELITE pad, washing with EtOAc and CHCl₃/MeOH. The filtrate was evaporated, diluted with EtOAc, washed with NaHCO₃ aq. and brine then dried over Na₂SO₄ and evaporated. The residue was purified by column chromatography on silica gel (CHCl₃/MeOH), thereby obtaining N-(2-(benzyloxy)ethyl)-5-bromo-1,2,6-trimethyl-1H-benzo[d]imidazol-4-amine (6.69 g, 92%).

### Step 6:

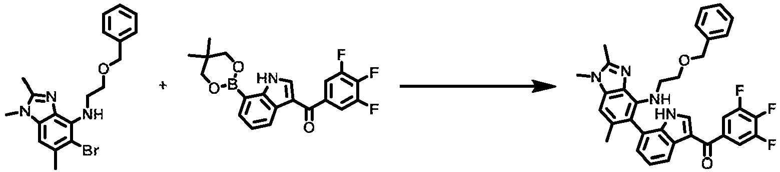

A suspension of N-(2-(benzyloxy)ethyl)-5-bromo-1,2,6-trimethyl-1H-benzo[d]imidazol-4-amine (769 mg, 1.98 mmol), (7-(5,5-dimethyl-1,3,2-dioxaborinan-2-yl)-1H-indol-3-yl)(3,4,5-trifluorophenyl)methanone (1.07 g, 2.77 mmol), SPhos Pd G3 (139 mg, 0.198 mmol) in THF (9.9 mL) and 2M K₃PO₄ aq. (2.08 mL, 4.16 mmol) was evacuated and N₂ back-filled before heating to 80 °C for 5 h. After cooling, the reaction mixture was diluted with CHCl₃, washed with H₂O and brine then dried over Na₂SO₄ and evaporated. The residue was purified by column chromatography on silica gel (hexane/acetone), thereby obtaining (7-(4-((2-(benzyloxy)ethyl)amino)-1,2,6-trimethyl-1H-benzo[d]imidazol-5-yl)-1H-indol-3-yl)(3,4,5-trifluorophenyl)methanone (1.03 g, 89%).

### Step 7:

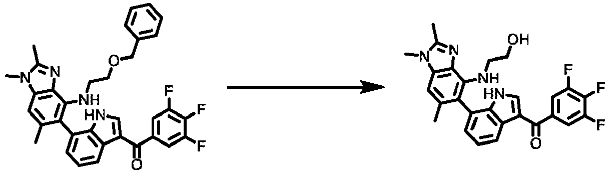

A suspension of (7-(4-((2-(benzyloxy)ethyl)amino)-1,2,6-trimethyl-1H-benzo[d]imidazol-5-yl)-1H-indol-3-yl)(3,4,5-trifluorophenyl)methanone (175 mg, 0.164 mmol) and Pd/C(10%) (175 mg) in EtOH (6.0 mL) was stirred at 40 °C under hydrogen (balloon) for 7 h. After cooling, the reaction mixture was filtered through a CELITE pad, washing with MeOH then evaporated. The residue was purified by column chromatography on silica gel (CHCl₃/MeOH), thereby obtaining (7-(4-((2-hydroxyethyl)amino)-1,2,6-trimethyl-1H-benzo[d]imidazol-5-yl)-1H-indol-3-yl)(3,4,5-trifluorophenyl)methanone (95.7 mg, 65%).

### Step 8:

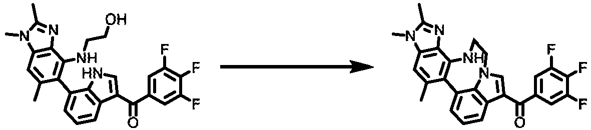

TMAD (78 mg, 0.45 mmol) was added to a solution of (7-(4-((2-hydroxyethyl)amino)-1,2,6-trimethyl-1H-benzo[d]imidazol-5-yl)-1H-indol-3-yl)(3,4,5-trifluorophenyl)methanone (89.3 mg, 0.181 mmol) and P(n-Bu)3 (113 µL, 0.453 mmol) in THF (9.1 mL) at 0°C. After stirring at 0°C for 3 h, the reaction mixture was diluted with EtOAc, washed with NaHCO₃ aq. and brine then dried over Na₂SO₄ and evaporated. The residue was purified by column chromatography on silica gel (CHCl₃/MeOH), thereby obtaining (3,4,5-trifluorophenyl)(6,8,9-trimethyl-8,11,12,13-tetrahydroimidazo[4",5":5',6']benzo[1',2':5,6][1,4]diazocino[7,8,1-hi]indol-2-yl)methanone (25.7 mg, 30%).

### Step 9:

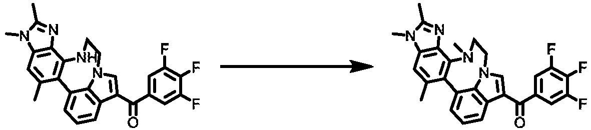

A solution of (3,4,5-trifluorophenyl)(6,8,9-trimethyl-8,11,12,13-tetrahydroimidazo[4",5":5',6']benzo[1',2':5,6][1,4]diazocino[7,8,1-hi]indol-2-yl)methanone (25.7 mg, 0.0542 mmol), 37% formaldehyde solution (4.52 µL, 0.0599 mmol) and TFA (4.02 µL, 0.0542 mmol) in THF (542 µL) was stirred at RT for 10 min. Then prepared reagent (mixture of sodium cyanoborohydride and zinc chloride (1:0.5) in MeOH (0.3 mol/L, 632 µL, 0.190 mmol)) was added thereto, and the mixture was stirred at RT for 1 h. The reaction mixture was diluted with CHCl₃, washed with H₂O and brine then dried over Na₂SO₄ and evaporated. The residue was purified by column chromatography on silica gel (hexane/acetone), thereby obtaining (6,8,9,11-tetramethyl-8,11,12,13-tetrahydroimidazo[4",5":5',6']benzo[1',2':5,6][1,4]diazocino[7,8,1-hi]indol-2-yl)(3,4,5-trifluorophenyl)methanone (17.7 mg, 67%).

### Step 10:

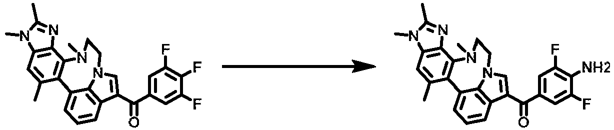

28% NH₃ aq. (1.75 mL, 12.6 mmol) was added to (6,8,9,11-tetramethyl-8,11,12,13-tetrahydroimidazo[4",5":5',6']benzo[1',2':5,6][1,4]diazocino[7,8,1-hi]indol-2-yl)(3,4,5-trifluorophenyl)methanone (17.7 mg, 0.0362 mmol) in 1,4-Dioxane (354 µL). The reaction mixture was heated at 130 °C by microwave irradiation for 12 h. After cooling, the reaction mixture was diluted with EtOAc, washed with H₂O and brine then dried over Na₂SO₄ and evaporated. The residue was purified by column chromatography on silica gel (CHCl₃/MeOH), thereby obtaining (4-amino-3,5-difluorophenyl)(6,8,9,11-tetramethyl-8,11,12,13-tetrahydroimidazo[4",5":5',6']benzo[1',2':5,6][1,4]diazocino[7,8,1-hi]indol-2-yl)methanone (14.3 mg, 81%).

### Step 11:

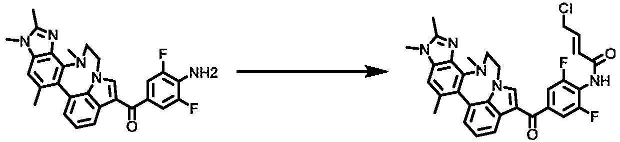

10%(w/v) 4-chlorocrotonic acid solution (35.6 µL, 0.0295 mmol) in propylphosphonic anhydride solution (50 wt% in THF, 0.0570 mmol) was added to a solution of (4-amino-3,5-difluorophenyl)(6,8,9,11-tetramethyl-8,11,12,13-tetrahydroimidazo[4",5":5',6']benzo[1',2':5,6][1,4]diazocino[7,8,1-hi]indol-2-yl)methanone (14.3 mg, 0.0295 mmol) and Et₃N (41.2 µL, 0.296 mmol) in DMF (295 µL). After stirring at RT for 20 min, further 10%(w/v) 4-chlorocrotonic acid solution (17.8 µL, 0.0148 mmol) in propylphosphonic anhydride solution (50 wt% in THF, 0.0285 mmol) was added and stirring continued for 20 min. The reaction mixture was diluted with EtOAc, washed with NaHCO₃ aq., H₂O and brine then dried over Na₂SO₄, passed through a small amount of NH silica gel (CHCl3/MeOH) and evaporated, thereby obtaining crude (E)-4-chloro-N-(2,6-difluoro-4-(6,8,9,11-tetramethyl-8,11,12,13-tetrahydroimidazo[4",5":5',6']benzo[1',2':5,6][1,4]diazocino[7,8,1-hi]indole-2-carbonyl)phenyl)but-2-enamide (16.2 mg, 94%).

### Step 12:

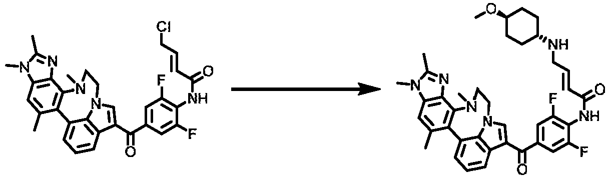

A solution of crude (E)-4-chloro-N-(2,6-difluoro-4-(6,8,9,11-tetramethyl-8,11,12,13-tetrahydroimidazo[4",5":5',6']benzo[1',2':5,6][1,4]diazocino[7,8,1-hi]indole-2-carbonyl)phenyl)but-2-enamide (16.2 mg), trans-4-methoxycyclohexanamine hydrochloride (14 mg, 0.083 mmol), KI (14 mg, 0.083 mmol) and K₃PO₄ (29 mg, 0.14 mmol) in DMF (275 µL) was stirred at 30 °C for 2 h. The reaction mixture was diluted with EtOAc, washed with H₂O(2x) and brine then dried over Na₂SO₄ and evaporated. The residue was purified by column chromatography on silica gel (CHCl₃(0.1%Et₃N)/MeOH) and preparative HPLC (water:acetonitrile (0.1% formic acid)). The purified fractions were washed with sat. NaHCO₃ aq., extracted with CHCl₃/MeOH then dried over Na₂SO₄ and evaporated, thereby obtaining the title compound (9.07 mg, 48%). ¹H-NMR: (400 MHz, CHLOROFORM-D) δ 8.40 (dd, J = 8.0, 1.0 Hz, 1H), 7.51 (s, 1H), 7.46-7.39 (m, 2H), 7.34 (dd, J = 7.6, 7.6 Hz, 1H), 7.16-7.08 (m, 3H), 6.36 (d, J = 15.3 Hz, 1H), 4.03-3.93 (m, 1H), 3.87 (dd, J = 14.5, 2.8 Hz, 1H), 3.73 (s, 3H), 3.55 (dd, J = 5.6, 1.4 Hz, 2H), 3.43 (d, J = 11.0 Hz, 1H), 3.34 (s, 3H), 3.17-3.10 (m, 2H), 3.08 (s, 3H), 2.69-2.58 (m, 4H), 2.40 (s, 3H), 2.16-2.00 (m, 4H), 1.33-1.19 (m, 4H); ESI-MS [M+H]⁺: 681.4.

### Synthetic procedure H

### Example 46

### (E)-N-(4-(8,9-dimethyl-6-(trifluoromethyl)-12,13-dihydro-8H-imidazo[4",5":3',4']benzo[1',2':7,8][1,4]oxazocino[6,5,4-hi]indazole-2-carbonyl)-2,6-difluorophenyl)-4-(((1r,4r)-4-methoxycyclohexyl)amino)but-2-enamide

### Step 1:

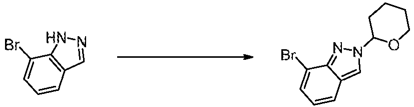

Pyridinium p-toluenesulfonate (255 mg) was added to 7-bromo-1H-indazole (2.00 g) and 3,4-dihydro-2H-pyran (1.70 g) in dichloromethane (25.0 mL), and the reaction mixture was stirred at 30°C for 17 hours. After dilution with chloroform, the mixture was washed with a saturated sodium bicarbonate solution and a saturated sodium chloride solution, followed by drying over sodium sulfate. After evaporation, the residue was purified by column chromatography (hexane:ethyl acetate), thereby obtaining 7-bromo-2-(tetrahydro-2H-pyran-2-yl)-2H-indazole (2.74 g).

### Step 2:

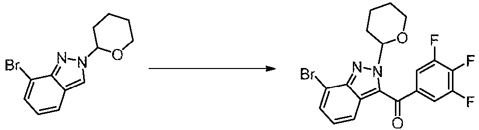

A solution of 7-bromo-2-(tetrahydro-2H-pyran-2-yl)-2H-indazole (2.74 g) obtained in step 1 in THF (30 mL) was cooled to -10°C. To the reaction mixture was added isopropylmagnesium chloride - lithium chloride complex (tetrahydrofuran solution (abt.14%), 14.6 mL). After stirring at -10°C for 1 hour, to the reaction mixture was added 3,4,5-trifluorobenzoyl chloride (2.84 g). A reaction was performed at -10°C for 1 hour. After warming up to room temparture, the reaction mixture was quenched with a saturated ammonium chloride solution. After the mixure was extracted with ethyl acetate, the organic layer was washed with a saturated sodium chloride solution, followed by drying over sodium sulfate. After evaporation, the obtained residue was purified by column chromatography (hexane:ethyl acetate), thereby obtaining (7-bromo-2-(tetrahydro-2H-pyran-2-yl)-2H-indazol-3-yl)(3,4,5-trifluorophenyl)methanone (2.70 g).

### Step 3:

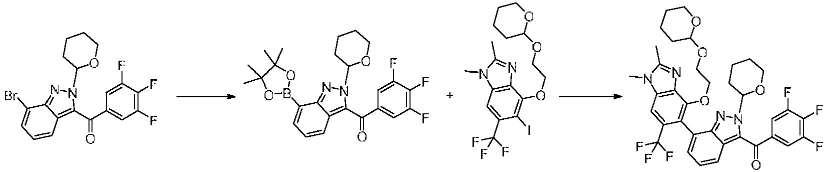

A solution of (7-bromo-2-(tetrahydro-2H-pyran-2-yl)-2H-indazol-3-yl)(3,4,5-trifluorophenyl)methanone (300 mg) obtained in step 2, 5,5,5',5'-tetramethyl-2,2'-bi(1,3,2-dioxaborinane) (260 mg), 1,1'-bis(diphenylphosphino)ferrocene]dichloropalladium(II) complex with dichloromethane (55.8 mg) and potassium acetate (201 mg) in 1,4-dioxane (4.0 mL) was stirred at 100°C for 3 hours. After dilution with ethyl acetate, the mixture was filtered through CELITE. The solvent was evaporated under reduced pressure, thereby obtaining (2-(tetrahydro-2H-pyran-2-yl)-7-(4,4,5,5-tetramethyl-1,3,2-dioxaborolan-2-yl)-2H-indazol-3-yl)(3,4,5-trifluorophenyl)methanone.

To the solution of crude (2-(tetrahydro-2H-pyran-2-yl)-7-(4,4,5,5-tetramethyl-1,3,2-dioxaborolan-2-yl)-2H-indazol-3-yl)(3,4,5-trifluorophenyl)methanone in tetrahydrofuran (4.0 mL), 5-iodo-1,2-dimethyl-4-(2-((tetrahydro-2H-pyran-2-yl)oxy)ethoxy)-6-(trifluoromethyl)-1H-benzo[d]imidazole (240 mg), (2-dicyclohexylphosphino-2',6'-dimethoxybiphenyl) [2-(2'-amino-1,1'-biphenyl)]palladium(II) methanesulfonate (43.4 mg) and 0.5 M tripotassium phosphate solution in water (0.93 mL) was added, followed by stirring at 80°C for 4 hours. After dilution with ethyl acetate, the mixture was washed with water and a saturated sodium chloride solution, followed by drying over sodium sulfate. The solvent was evaporated under reduced pressure, thereby obtaining crude (7-(1,2-dimethyl-4-(2-((tetrahydro-2H-pyran-2-yl)oxy)ethoxy)-6-(trifluoromethyl)-1H-benzo[d]imidazol-5-yl)-2-(tetrahydro-2H-pyran-2-yl)-2H-indazol-3-yl)(3,4,5-trifluorophenyl)methanone (200 mg).

### Step 4:

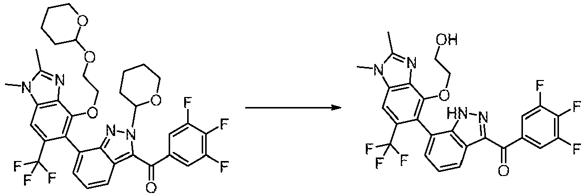

To the solution of crude (7-(1,2-dimethyl-4-(2-((tetrahydro-2H-pyran-2-yl)oxy)ethoxy)-6-(trifluoromethyl)-1H-benzo[d]imidazol-5-yl)-2-(tetrahydro-2H-pyran-2-yl)-2H-indazol-3-yl)(3,4,5-trifluorophenyl)methanone (200 mg) obtained in step 3 in THF (3 mL), 5M hydrochloric acid (0.10 mL) was added, followed by stirring at 30°C for 16 hours. After dilution with ethyl acetate, a saturated sodium bicarbonate solution was added to the mixture. After extraction with ethyl acetate, the mixture was washed with a saturated sodium chloride solution, followed by drying over sodium sulfate. After evaporation, the obtained residue was purified by column chromatography (hexane:acetone), thereby obtaining (7-(4-(2-hydroxyethoxy)-1,2-dimethyl-6-(trifluoromethyl)-1H-benzo[d]imidazol-5-yl)-1H-indazol-3-yl)(3,4,5-trifluorophenyl)methanone (25.0 mg).

### Step 5:

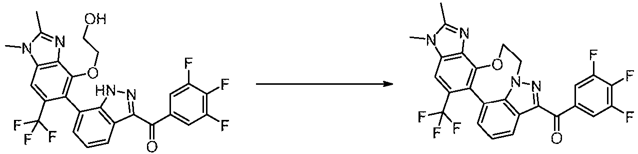

Tributylphosphine and N,N,N',N '-tetramethylazodicarboxamide (36.6 mg) were added to a solution of (7-(4-(2-hydroxyethoxy)-1,2-dimethyl-6-(trifluoromethyl)-1H-benzo[d]imidazol-5-yl)-1H-indazol-3-yl)(3,4,5-trifluorophenyl)methanone (25.0 mg) obtained in step 4 in tetrahydrofuran (5.0 mL) and toluene (5.0 mL), followed by stirring at 70°C for 1 hour. After tributylphosphine (23.2 µL) and N,N,N ',N '-tetramethylazodicarboxamide (12.3 mg) were added to the reaction mixture, a reaction was performed at 90 °C for 1 hour. The solvent was evaporated under reduced pressure, followed by purifing by column chromatography (hexane:acetone), thereby obtaining crude (8,9-dimethyl-6-(trifluoromethyl)-12,13-dihydro-8H-imidazo[4",5":3',4']benzo[1',2':7,8][1,4]oxazocino[6,5,4-hi]indazol-2-yl)(3,4,5-trifluorophenyl)methanone including tributylphosphine oxide.

### Step 6:

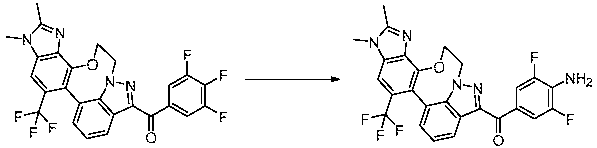

A solution of crude (8,9-dimethyl-6-(trifluoromethyl)-12,13-dihydro-8H-imidazo[4",5":3',4']benzo[1',2':7,8][1,4]oxazocino[6,5,4-hi]indazol-2-yl)(3,4,5-trifluorophenyl)methanone obtained in step 5 and 28% ammonia solution in water (1.3 mL) in 1,4-dioxane (1.3 mL) was stirred at 130°C for 12 hours using microwave irradiation. After dilution with ethyl acetate and water, the mixture was extracted with ethyl acetate. The combined organic layer was washed with water and a saturated sodium chloride solution, followed by drying over sodium sulfate. After evaporation, the obtained residue was purified by column chromatography (hexane:acetone), thereby obtaining (4-amino-3,5-difluorophenyl)(8,9-dimethyl-6-(trifluoromethyl)-12,13-dihydro-8H-imidazo[4",5":3',4']benzo[1',2':7,8][1,4]oxazocino[6,5,4-hi]indazol-2-yl)methanone (5.80 mg).

### Step 7:

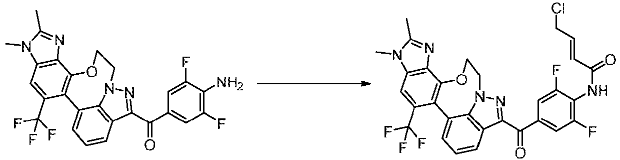

(E)-4-chlorobut-2-enoic acid (2.65 mg) and 1-propanephosphonic acid anhydride cyclic trimer (a 1.7M THF solution, 35 µL) were added to a solution of (4-amino-3,5-difluorophenyl)(8,9-dimethyl-6-(trifluoromethyl)-12,13-dihydro-8H-imidazo[4",5":3',4']benzo[1',2':7,8][1,4]oxazocino[6,5,4-hi]indazol-2-yl)methanone (5.80 mg) obtained in step 6 in DMF (500 µL), followed by stirring at room temperature for 20 minutes. Triethylamine (15 µL) was added to the reaction mixture. A reaction was performed at room temperature for 2 hours. After water was added to the reaction mixture, extraction was performed with ethyl acetate, followed by washing the organic layer with a saturated sodium chloride solution. The washed organic layer was dried over sodium sulfate. After evaporation, the obtained residue was purified by column chromatography (chloroform:ethanol), thereby obtaining (E)-4-chloro-N-(4-(8,9-dimethyl-6-(trifluoromethyl)-12,13-dihydro-8H-imidazo[4",5":3',4']benzo[1',2':7,8][1,4]oxazocino[6,5,4-hi]indazole-2-carbonyl)-2,6-difluorophenyl)but-2-enamide (6.02 mg).

### Step 8:

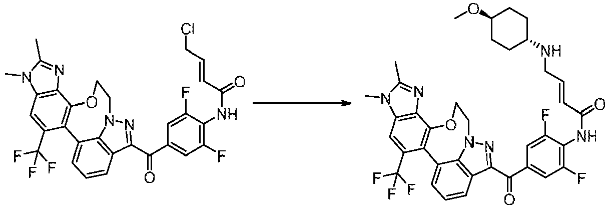

(1r,4r)-4-methoxycyclohexan-1-amine hydrochloride (3.17 mg), tripotassium phosphate (8.11 mg), and potassium iodide (4.76 mg) were added to a solution of (E)-4-chloro-N-(4-(8,9-dimethyl-6-(trifluoromethyl)-12,13-dihydro-8H-imidazo[4",5":3',4']benzo[1',2':7,8][1,4]oxazocino[6,5,4-hi]indazole-2-carbonyl)-2,6-difluorophenyl)but-2-enamide (6.02 mg) obtained in step 7 in DMF (500 µL). A reaction was performed at room temperature for 2 hours. After water was added to the reaction mixture, extraction was performed with ethyl acetate. The combined organic layer was washed with a saturated sodium chloride solution. After the washed layer was dried over sodium sulfate, the solvent was evaporated under reduced pressure. The obtained residue was purified by column chromatography (chloroform:ethanol), thereby obtaining the title compound (4.01 mg). ¹H-NMR: (400 MHz, CHLOROFORM-D) δ 8.51-8.47 (m, 1H), 8.08 (d, J = 8.8 Hz, 2H), 7.72 (s, 1H), 7.44-7.41 (m, 2H), 7.26 (s, 4H), 7.11 (td, J = 10.3, 5.1 Hz, 1H), 6.26 (d, J = 15.3 Hz, 1H), 5.39-5.33 (m, 2H), 4.68-4.46 (m, 4H), 3.86 (s, 3H), 3.51 (d, J = 5.0 Hz, 2H), 3.35 (s, 3H), 3.17-3.12 (m, 1H), 2.71 (s, 3H), 2.59-2.53 (m, 1H), 2.13-1.98 (m, 4H), 1.31-1.11 (m, 4H); ESI-MS [M+H]⁺: 723.5

### Synthetic procedure I

### Example 47

### (E)-N-(2,6-difluoro-4-(6,8,9,11-tetramethyl-12-oxo-8,11,12,13-tetrahydroimidazo[4",5":5',6']benzo[1',2':5,6][1,4]diazocino[7,8,1-hi]indole-2-carbonyl)phenyl)-4-(((1r,4r)-4-methoxycyclohexyl)amino)but-2-enamide

### Step 1:

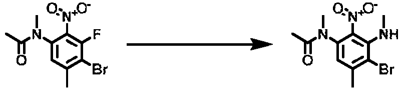

A solution of N-(4-bromo-3-fluoro-5-methyl-2-nitrophenyl)-N-methylacetamide (692 mg, 2.27 mmol), methylamine (2M THF solution, 3.40 mL, 6.80 mmol) in THF (7.6 mL) was stirred at 60 °C for 1.5 h. Further methylamine (2M THF solution, 1.70 mL, 3.40 mmol) was added and stirring continued for 1 h. After cooling, the reaction mixture was diluted with EtOAc, washed with NaHCO₃ aq. and brine then dried over Na₂SO₄ and evaporated. The residue was purified by column chromatography on silica gel (hexane/EtOAc), thereby obtaining N-(4-bromo-5-methyl-3-(methylamino)-2-nitrophenyl)-N-methylacetamide (694 mg, 97%).

### Step 2:

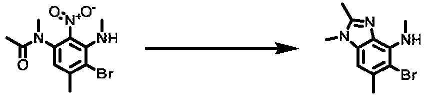

A suspension of N-(4-bromo-5-methyl-3-(methylamino)-2-nitrophenyl)-N-methylacetamide (694 mg, 2.20 mmol) and iron (1.23 g, 22.0 mmol) in AcOH (11 mL) was stirred at 60 °C for 3.5 h. After cooling, EtOAc was added to the reaction mixture then filtered through a CELITE pad, washing with EtOAc. The filtrate was evaporated, diluted with EtOAc, washed with NaHCO₃ aq. and brine then dried over Na₂SO₄ and evaporated. The residue was purified by column chromatography on silica gel (hexane/acetone), thereby obtaining 5-bromo-N,1,2,6-tetramethyl-1H-benzo[d]imidazol-4-amine (305 mg, 52%).

### Step 3:

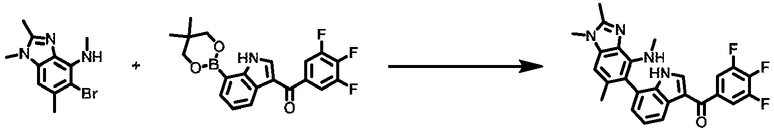

A suspension of 5-bromo-N,1,2,6-tetramethyl-1H-benzo[d]imidazol-4-amine (305 mg, 1.14 mmol), (7-(5,5-dimethyl-1,3,2-dioxaborinan-2-yl)-1H-indol-3-yl)(3,4,5-trifluorophenyl)methanone (660 mg, 1.70 mmol), SPhos Pd G3 (80 mg, 0.11 mmol) in THF (5.7 mL) and 2M K₃PO₄ aq. (1.28 mL, 2.56 mmol) was evacuated and N₂ back-filled before heating to 80 °C for 5 h. After cooling, the reaction mixture was diluted with CHCl₃, washed with H₂O and brine then dried over Na₂SO₄ and evaporated. The residue was purified by column chromatography on silica gel (CHCl₃/MeOH), thereby obtaining (3,4,5-trifluorophenyl)(7-(1,2,6-trimethyl-4-(methylamino)-1H-benzo[d]imidazol-5-yl)-1H-indol-3-yl)methanone (410 mg, 78%).

### Step 4:

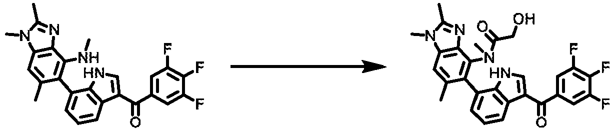

HATU (144 mg, 0.379 mmol) was added to a solution of (3,4,5-trifluorophenyl)(7-(1,2,6-trimethyl-4-(methylamino)-1H-benzo[d]imidazol-5-yl)-1H-indol-3-yl)methanone (70.1 mg, 0.152 mmol), 2-acetoxyacetic acid (35.8 mg, 0.303 mmol) and DIEA (104 µL, 0.606 mmol) in DMF (1.5 mL). After stirring at RT for 1 h, 2M NaOH aq. (758 µL, 1.52 mmol) was added and stirring continued for 10 min at RT. The reaction mixture was diluted with EtOAc, washed with H₂O and brine then dried over Na₂SO₄ and evaporated. The residue was purified by column chromatography on silica gel (CHCl₃/MeOH), thereby obtaining crude 2-hydroxy-N-methyl-N-(1,2,6-trimethyl-5-(3-(3,4,5-trifluorobenzoyl)-1H-indol-7-yl)-1H-benzo[d]imidazol-4-yl)acetamide (80.9 mg, 103%).

### Step 5:

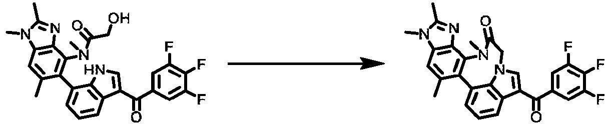

TMAD (67 mg, 0.39 mmol) was added to a solution of crude 2-hydroxy-N-methyl-N-(1,2,6-trimethyl-5-(3-(3,4,5-trifluorobenzoyl)-1H-indol-7-yl)-1H-benzo[d]imidazol-4-yl)acetamide (80.9 mg) and P(n-Bu)3 (97 µL, 0.39 mmol) in THF (7.8 mL) at 0°C. After stirring at RT for 2.5 h, the reaction mixture was diluted with CHCl₃, washed with H₂O and brine then dried over Na₂SO₄ and evaporated. The residue was purified by column chromatography on silica gel (CHCl₃/MeOH), thereby obtaining crude 6,8,9,11-tetramethyl-2-(3,4,5-trifluorobenzoyl)-8,11-dihydroimidazo[4",5":5',6']benzo[1',2':5,6][1,4]diazocino[7,8,1-hi]indol-12(13H)-one (56.6 mg, 73%).

### Step 6:

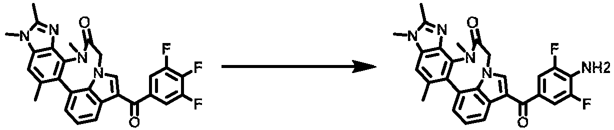

28% NH₃ aq. (4.7 mL, 34 mmol) was added to crude 6,8,9,11-tetramethyl-2-(3,4,5-trifluorobenzoyl)-8,11-dihydroimidazo[4",5":5',6']benzo[1',2':5,6][1,4]diazocino[7,8,1-hi]indol-12(13H)-one (56.6 mg) in 1,4-Dioxane (566 µL). The reaction mixture was heated at 130 °C by microwave irradiation for 12 h. After cooling, the reaction mixture was diluted with EtOAc, washed with H₂O and brine then dried over Na₂SO₄ and evaporated. The residue was purified by column chromatography on silica gel (CHCl₃/MeOH), thereby obtaining crude 2-(4-amino-3,5-difluorobenzoyl)-6,8,9,11-tetramethyl-8,11-dihydroimidazo[4",5":5',6']benzo[1',2':5,6][1,4]diazocino[7,8,1-hi]indol-12(13H)-one (9.1 mg, 16%).

### Step 7:

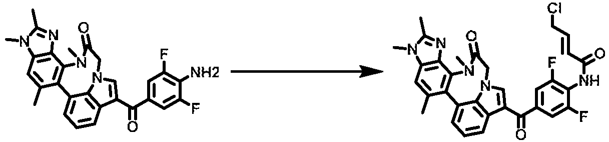

10%(w/v) 4-chlorocrotonic acid solution (27.4 µL, 0.0228 mmol) in propylphosphonic anhydride solution (50 wt% in THF, 0.0438 mmol) was added to a solution of crude 2-(4-amino-3,5-difluorobenzoyl)-6,8,9,11-tetramethyl-8,11-dihydroimidazo[4",5":5',6']benzo[1',2':5,6][1,4]diazocino[7,8,1-hi]indol-12(13H)-one (9.1 mg) and Et₃N (25.4 µL, 0.182 mmol) in DMF (182 µL). After stirring at RT for 1 h, further 10%(w/v) 4-chlorocrotonic acid solution (16.6 µL, 0.0138 mmol) in propylphosphonic anhydride solution (50 wt% in THF, 0.0266 mmol) was added and stirring continued for 20 min. The reaction mixture was diluted with EtOAc, washed with NaHCO₃ aq., H₂O and brine then dried over Na₂SO₄, passed through a small amount of NH silica gel (CHCl₃/MeOH) and evaporated, thereby obtaining crude (E)-4-chloro-N-(2,6-difluoro-4-(6,8,9,11-tetramethyl-12-oxo-8,11,12,13-tetrahydroimidazo[4",5":5',6']benzo[1',2':5,6][1,4]diazocino[7,8,1-hi]indole-2-carbonyl)phenyl)but-2-enamide (10.8 mg, 99%).

### Step 8:

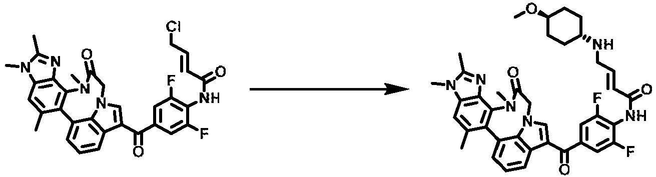

A solution of crude (E)-4-chloro-N-(2,6-difluoro-4-(6,8,9,11-tetramethyl-12-oxo-8,11,12,13-tetrahydroimidazo[4",5":5',6']benzo[1',2':5,6][1,4]diazocino[7,8,1-hi]indole-2-carbonyl)phenyl)but-2-enamide (10.8 mg), trans-4-methoxycyclohexanamine hydrochloride (9 mg, 0.05 mmol), KI (9 mg, 0.05 mmol) and K₃PO₄ (19 mg, 0.090 mmol) in DMSO (358 µL) was stirred at 30 °C for 1 h. The reaction mixture was diluted with DMSO then filtered and purified by preparative HPLC (water:acetonitrile (0.1% formic acid)). The purified fractions were washed with sat. NaHCO₃ aq., extracted with CHCl₃/MeOH then dried over Na₂SO₄ and evaporated, thereby obtaining the title compound (5.58 mg, 45%). ¹H-NMR: (400 MHz, CHLOROFORM-D) δ 8.45 (dd, J = 8.0, 1.0 Hz, 1H), 7.64 (s, 1H), 7.48-7.42 (m, 2H), 7.39 (dd, J = 8.4, 8.4 Hz, 1H), 7.34 (d, J = 0.8 Hz, 1H), 7.21-7.06 (m, 3H), 6.23 (dt, J = 15.3, 1.8 Hz, 1H), 4.98 (d, J = 12.8 Hz, 1H), 4.17 (d, J = 12.8 Hz, 1H), 3.78 (s, 3H), 3.49 (dd, J = 5.3, 1.8 Hz, 2H), 3.40 (s, 3H), 3.35 (s, 3H), 3.18-3.11 (m, 1H), 2.65 (s, 3H), 2.57-2.49 (m, 1H), 2.23 (s, 3H), 2.11-2.04 (m, 2H), 2.02-1.94 (m, 2H), 1.33-1.09 (m, 4H); ESI-MS [M+H]⁺: 695.6.

### Synthetic procedure J

### Example 48

### (E)-N-(2,6-difluoro-4-(2,3,5-trimethyl-13-methylene-12,13-dihydro-3H-imidazo[4",5":5',6']benzo[1',2':6,7]cyclohepta[1,2,3-hi]indolizine-10-carbonyl)phenyl)-4-(((1r,4r)-4-methoxycyclohexyl)amino)but-2-enamide

### Step 1:

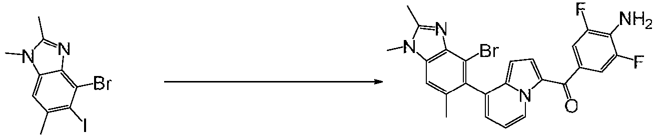

To a mixture of (4-amino-3,5-difluorophenyl)(8-(5,5-dimethyl-1,3,2-dioxaborinan-2-yl)indolizin-3-yl)methanone (380 mg, 0.98 mmol), 4-bromo-5-iodo-1,2,6-trimethyl-1H-benzo[d]imidazole (260 mg, 0.70 mmol) and Pd(PPh₃)₄ (81 mg, 0.070 mmol) in 1,2-dimethoxyethane (2.5 mL) was added 2M aqueous sodium carbonate solution (0.70 mL, 1.4 mmol). After reacting by microwave apparatus at 110 °C for 2 hrs, the mixture was diluted with EtOAc and water. Insoluble material was filtered off and the organic layer was washed with brine, dried over sodium sulfate and evaporated under reduced pressure. The residue was purified by column chromatography on silica gel (CHCl₃-methanol) to give (4-amino-3,5-difluorophenyl)(8-(4-bromo-1,2,6-trimethyl-1H-benzo[d]imidazol-5-yl)indolizin-3-yl)methanone (260 mg).

### Step 2:

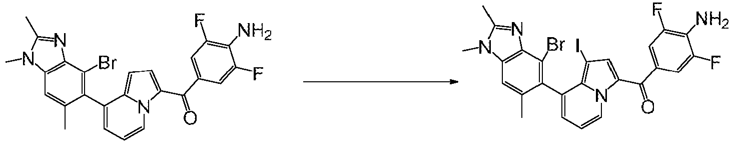

To a mixture of (4-amino-3,5-difluorophenyl)(8-(4-bromo-1,2,6-trimethyl-1H-benzo[d]imidazol-5-yl)indolizin-3-yl)methanone (250 mg) in THF (2.5 mL) was added NIS (110 mg, 0.49 mmol). The mixture was stirred at RT for 2 hrs and NIS (8.9 mg, 0.039 mmol) was added and the mixture was stirred at RT for 30 min. The mixture was diluted with EtOAc, water, sat. NaHCO₃ aq., and sat. NaCl aq., and the organic layer was washed with sat. NaHCO₃ aq. and brine, dried over sodium sulfate and evaporated under reduced pressure. The residue was purified by column chromatography on silica gel (hexane-acetone) to give crude (4-amino-3,5-difluorophenyl)(8-(4-bromo-1,2,6-trimethyl-1H-benzo[d]imidazol-5-yl)-1-iodoindolizin-3-yl)methanone (280 mg).

### Step 3:

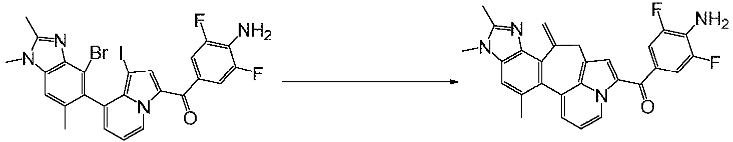

To a mixture of crude (4-amino-3,5-difluorophenyl)(8-(4-bromo-1,2,6-trimethyl-1H-benzo[d]imidazol-5-yl)-1-iodoindolizin-3-yl)methanone (180 mg), 2-allyl-4,4,5,5-tetramethyl-1,3,2-dioxaborolane (345 mg, 2.1 mmol) and Pd(PPh₃)₄ (30 mg, 0.026 mmol) in 1,2-dimethoxyethane (3.0 mL) and water (0.51 mL) was added sodium carbonate (220 mg, 2.1 mmol). After reacting by microwave apparatus at 100 °C for 12 hrs, the mixture was diluted with EtOAc and water and the organic layer was washed with brine, dried over sodium sulfate and evaporated under reduced pressure. The residue was purified by column chromatography on silica gel (hexane-acetone) to give (4-amino-3,5-difluorophenyl)(7,9,10-trimethyl-12-methylene-12,13-dihydro-9H-imidazo[4",5":5',6']benzo[1',2':6,7]cyclohepta[1,2,3-hi]indolizin-2-yl)methanone (17 mg).

### Step 4:

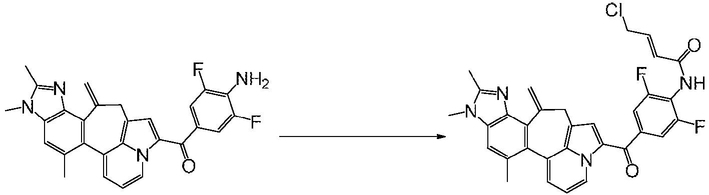

A solution of (4-amino-3,5-difluorophenyl)(7,9,10-trimethyl-12-methylene-12,13-dihydro-9H-imidazo[4",5":5',6'|benzo[1',2":6,7]cyclohepta[1,2,3-hi]indolizin-2-yl)methanone (17 mg), 4-chlorocrotonic acid (5.8 mg, 0.048 mmol) and propylphosphonic anhydride solution (50 wt% in THF, 0.057 mL, 0.092 mmol) in DMF (1.5 mL) was stirred at RT for 15 min. Then triethylamine (0.020 mL, 0.15 mmol) was added thereto, and the mixture was stirred at RT for 1 hr. 4-chlorocrotonic acid (5.8 mg, 0.048 mmol) and propylphosphonic anhydride solution (50 wt% in THF, 0.057 mL, 0.092 mmol) were added and the mixture was stirred at RT for 15 min. Then triethylamine (0.020 mL, 0.15 mmol) was added thereto, and the mixture was stirred at RT for 1 hr and at 30 °C for 1 hr. 4-chlorocrotonic acid (5.8 mg, 0.048 mmol) and propylphosphonic anhydride solution (50 wt% in THF, 0.057 mL, 0.092 mmol) were added and the mixture was stirred at RT for 15 min. Then triethylamine (0.020 mL, 0.15 mmol) was added thereto, and the mixture was stirred at 30 °C for 1 hr and at 50 °C overnight. To the resulting mixture was added saturated aqueous NaHCO₃ and water, and extracted with EtOAc, and the organic layer was washed successively with H₂O, saturated aqueous NaHCO₃ then brine, dried over sodium sulfate, and evaporated. The residue was purified by column chromatography on silica gel (CHCl₃-MeOH) to give (E)-4-chloro-N-(2,6-difluoro-4-(7,9,10-trimethyl-12-methylene-12,13-dihydro-9H-imidazo[4",5":5',6']benzo[1',2':6,7]cyclohepta[1,2,3-hi]indolizine-2-carbonyl)phenyl)but-2-enamide (12 mg).

### Step 5:

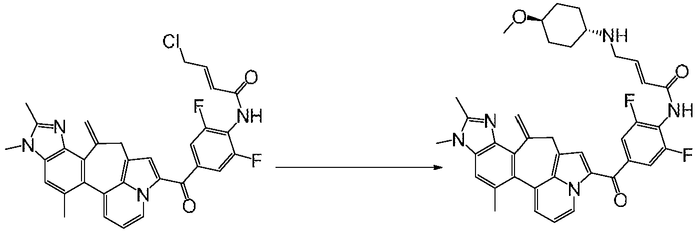

A solution of (E)-4-chloro-N-(2,6-difluoro-4-(7,9,10-trimethyl-12-methylene-12,13-dihydro-9H-imidazo[4",5":5',6']benzo[1',2':6,7]cyclohepta[1,2,3-hi]indolizine-2-carbonyl)phenyl)but-2-enamide (12 mg), trans-4-methoxycyclohexanamine hydrochloride (8.9 mg, 0.054 mmol), KI (11 mg, 0.065 mmol) and K₃PO₄ (23 mg, 0.11 mmol) in DMF (1.0 mL) was stirred at 30 °C for 30 min and at 40 °C for 3 hrs. To the resulting mixture was added H₂O and the organic layer was washed successively with H₂O then brine, dried over sodium sulfate, and evaporated. The residue was purified by column chromatography on silica gel (CHCl₃-MeOH) to give the title compound (9.6 mg). ¹H-NMR: (400 MHz, CHLOROFORM-D) δ 9.92 (dd, J = 5.5, 0.6 Hz, 1H), 7.72-7.68 (m, 0.5H), 7.54-7.50 (m, 0.5H), 7.43 (d, J = 8.0 Hz, 2H), 7.28- 7.26 (m, 1H), 7.22 (s, 1H), 7.17 (s, 1H), 7.12 (dt, J = 12.3, 4.2 Hz, 1H), 7.02 (t, J = 7.1 Hz, 1H), 6.28 (d, J = 15.3 Hz, 1H), 5.54 (brs, 1H), 5.39 (d, J = 1.5 Hz, 1H), 3.81 (d, J = 14.5 Hz, 1H), 3.74 (s, 3H), 3.65 (d, J = 14.3 Hz, 1H), 3.54-3.52 (m, 2H), 3.35 (s, 3H), 3.17-3.12 (m, 1H), 2.64 (s, 3H), 2.61 (s, 3H), 2.62-2.55 (m, 1H), 2.12-1.98 (m, 4H), 1.30-1.15 (m, 4H); MS-ESI [M+H]⁺: 664.5.

### Synthetic procedure K

### Example 49

### (E)-N-(2,6-difluoro-4-(8-oxo-11-(trifluoromethyl)-5,6-dihydro-4H,8H-pyrido[2',1':4,5][1,5]diazocino[3,2,1-hi]indole-1-carbonyl)phenyl)-4-(((1r,4r)-4-methoxycyclohexyl)amino)but-2-enamide

### Step 1:

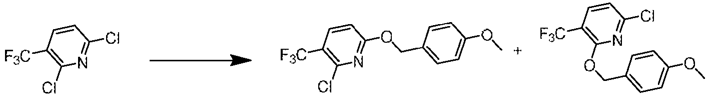

To a mixture of 2,6-dichloro-3-(trifluoromethyl)pyridine (2.15 g, 9.95 mmol) and 4-methoxybenzyl alcohol (1.36 mL, 10.9 mmol) in THF (30 mL) was added NaH (60 % in mineral oil, 0.796 g, 19.9 mmol). After stirried at room temperature for 2 hrs, 4-methoxybenzyl alcohol (0.4 mL) was added thereto. The mixture was stirred at room temperature for 30 min, and water and EtOAc were added thereto. The organic layer was washed with brine, dried over sodium sulfate and evaporated under reduced pressure. The residue was purified by column chromatography on silica gel (hexane-EtOAc) to give an approximately 3:1 mixture of 2-chloro-6-((4-methoxybenzyl)oxy)-3-(trifluoromethyl)pyridine and 6-chloro-2-((4-methoxybenzyl)oxy)-3-(trifluoromethyl)pyridine (3.42 g).

### Step 2:

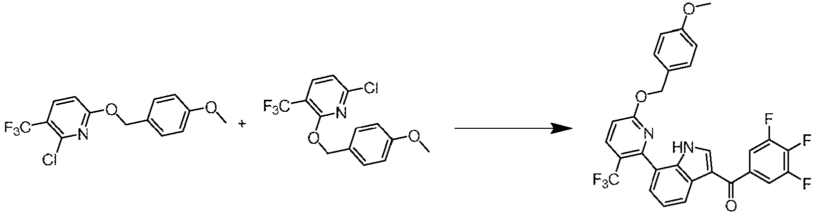

The mixture of the above mixture of 2-chloro-6-((4-methoxybenzyl)oxy)-3-(trifluoromethyl)pyridine and 6-chloro-2-((4-methoxybenzyl)oxy)-3-(trifluoromethyl)pyridine (317 mg), (7-(5,5-dimethyl-1,3,2-dioxaborinan-2-yl)-1H-indol-3-yl)(3,4,5-trifluorophenyl)methanone (386 mg, 0.998 mmol), S-Phos Pd G3 (35.1 mg, 0.0499 mmol), and aqueous potassium phosphate tribasic (1M, 2 mL, 2.00 mmol) in THF (8 mL) was strred at 80 °C for 1.5 hrs. The mixture was added EtOAc and water. The organic layer was separated, washed with brine, dried over sodium sulfate and evaporated under reduced pressure. The residue was purified by column chromatography on silica gel (hexane-EtOAc) to give (7-(6-((4-methoxybenzyl)oxy)-3-(trifluoromethyl)pyridin-2-yl)-1H-indol-3-yl)(3,4,5-trifluorophenyl)methanone (402 mg) and (7-(6-((4-methoxybenzyl)oxy)-5-(trifluoromethyl)pyridin-2-yl)-1H-indol-3-yl)(3,4,5-trifluorophenyl)methanone (123 mg).

### Step 3:

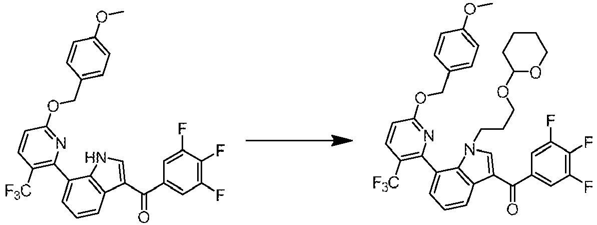

To a solution of (7-(6-((4-methoxybenzyl)oxy)-3-(trifluoromethyl)pyridin-2-yl)-1H-indol-3-yl)(3,4,5-trifluorophenyl)methanone (100 mg, 0.180 mmol) in DMSO (2 mL) was added potassium hydroxide (14 mg, 0.25 mmol) and 2-(3-bromopropoxy)tetrahydro-2H-pyran (0.0457 mL, 0.270 mmol). After stirred at room temperature for 3 hrs, water and EtOAc were added thereto. The organic layer was washed successively with water then brine, dried over sodium sulfate and evaporated under reduced pressure. The residue was purified by column chromatography on silica gel (hexane-EtOAc) to give (7-(6-((4-methoxybenzyl)oxy)-3-(trifluoromethyl)pyridin-2-yl)-1-(3-((tetrahydro-2H-pyran-2-yl)oxy)propyl)-1H-indol-3-yl)(3,4,5-trifluorophenyl)methanone (87.8 mg).

### Step 4:

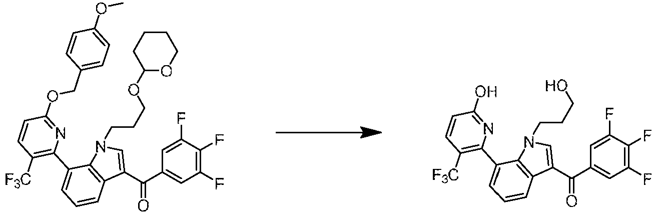

To a solution of (7-(6-((4-methoxybenzyl)oxy)-3-(trifluoromethyl)pyridin-2-yl)-1-(3-((tetrahydro-2H-pyran-2-yl)oxy)propyl)-1H-indol-3-yl)(3,4,5-trifluorophenyl)methanone (87.8 mg, 0.126 mmol) in THF (2 mL) was added 1M hydrochloric acid (1 mL, 1 mmol). After stirred at room temperature overnight, saturated aqueous NaHCO₃ was added to the reaction mixture, and extracted with EtOAc, and washed with brine, dried over sodium sulfate, and evaporated. The residue was purified by column chromatography on silica gel (CHCl₃-EtOH) to give (7-(6-hydroxy-3-(trifluoromethyl)pyridin-2-yl)-1-(3-hydroxypropyl)-1H-indol-3-yl)(3,4,5-trifluorophenyl)methanone (52.5 mg).

### Step 5:

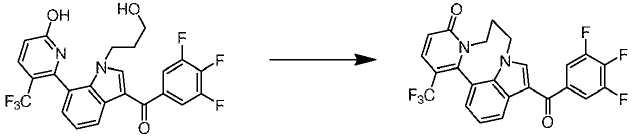

To the mixture of (7-(6-hydroxy-3-(trifluoromethyl)pyridin-2-yl)-1-(3-hydroxypropyl)-1H-indol-3-yl)(3,4,5-trifluorophenyl)methanone (52.5 mg, 0.106 mmol) and triphenylphosphine (83.6 mg, 0.319 mmol) in THF (3 mL) was added diisopropyl azodicarboxylate (0.0418 ml, 0.212 mmol), and the mixture was stirred at room temperature for 15 min. The resulting mixture was added methanol, and evaporated. The residue was purified by column chromatography on silica gel (CHCl₃-EtOH) to give crude 1-(3,4,5-trifluorobenzoyl)-11-(trifluoromethyl)-5,6-dihydro-4H,8H-pyrido[2',1':4,5][1,5]diazocino[3,2,1-hi]indol-8-one (63.9 mg).

### Step 6:

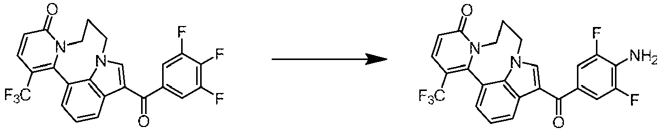

The mixture of the above 1-(3,4,5-trifluorobenzoyl)-11-(trifluoromethyl)-5,6-dihydro-4H,8H-pyrido[2',1':4,5][1,5]diazocino[3,2,1-hi]indol-8-one (63.9 mg), 28% aqueous ammonium hydroxide solution (2 mL) and 1,4-dioxane (2 mL) was heated by microwave apparatus at 120 °C for 12 hrs, then the mixture was diluted with EtOAc and brine. The organic layer was dried over sodium sulfate and evaporated under reduced pressure. The residue was purified by column chromatography on silica gel (hexane-EtOAc/ethanol(4/1)) to give 1-(4-amino-3,5-difluorobenzoyl)-11-(trifluoromethyl)-5,6-dihydro-4H,8H-pyrido[2',1':4,5][1,5]diazocino[3,2,1-hi]indol-8-one (44.0 mg)

### Step 7:

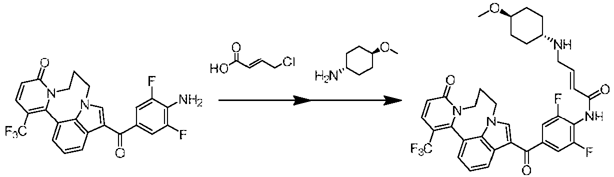

To the mixture of 1-(4-amino-3,5-difluorobenzoyl)-11-(trifluoromethyl)-5,6-dihydro-4H,8H-pyrido[2',1':4,5][1,5]diazocino[3,2,1-hi]indol-8-one (44.0 mg, 0.0929 mmol) and triethylamine (0.261 mL, 1.92 mmol) in DMF (2 mL) was added 10 % (w/v) (E)-4-chlorobut-2-enoic acid solution (0.12 mL) in propylphosphonic anhydride solution (50 wt% in THF). After stirred at 45 °C for 10 min, 10 % (w/v) (E)-4-chlorobut-2-enoic acid solution (0.06 mL) in propylphosphonic anhydride solution (50 wt% in THF) was added thereto twice. Saturated aqueous NaHCO₃ was added thereto, and the mixture was extracted with EtOAc, and washed successively with H₂O then brine, dried over sodium sulfate, and evaporated. To the residue was added DMSO (0.9 mL) and trans-4-methoxycyclohexan-1-amine (0.0639 mL). The mixture was heated at 45 °C for 1 hr. The resulting mixture was purified by preparative HPLC (water:acetonitrile (0.1% formic acid)) to give the title compound (31.3 mg). ¹H-NMR: (500 MHz, CHLOROFORM-D) δ 8.61 (dd, J = 8.1, 1.1 Hz, 1H), 7.72 (d, J = 9.8 Hz, 1H), 7.61 (s, 1H), 7.46 (d, J = 9.8 Hz, 2H), 7.42 (t, J = 7.8 Hz, 1H), 7.29 (d, J = 7.3 Hz, 1H), 7.13 (dt, J = 15.5, 5.3 Hz, 1H), 6.76 (d, J = 9.8 Hz, 1H), 6.28 (d, J = 15.6 Hz, 1H), 4.88 (dd, J = 13.9, 5.3 Hz, 1H), 4.21-4.18 (m, 1H), 4.09-4.03 (m, 1H), 3.55-3.49 (m, 3H), 3.36 (s, 3H), 3.18-3.11 (m, 1H), 2.62-2.53 (m, 1H), 2.41-2.31 (m, 1H), 2.10-2.06 (m, 2H), 2.01-1.95 (m, 3H), 1.30-1.13 (m, 4H); ESI-MS [M+H]⁺: 669.3.

Other Examples were prepared using the synthetic procedures A to K and purification notes as shown in the table below.

| Example | Compound name | Compound Structure | Synthetic procedure | Purification notes | 1H NMR | ES-MS [M+H]⁺ |
|---|---|---|---|---|---|---|
| 2-1, 2-2 | (E)-4-(tert-butylamino)-N-(4-(11-chloro-5,6-dihydro-4H-benzo[8,9] [1,5]oxaz onino[7,6,5-hi]indole-1-carbonyl)-2,6-difluorophenyl)but-2-enamide | 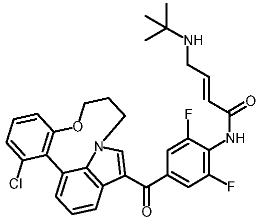 | A | Purified by preparative HPLC (CHIRALPAK IE (hexane-ethanol (0.1%triethyl amine))) to afford Example 2-1 (single isomer, 1st-eluting isomer) and Example 2-2 (single isomer, 2nd-eluting isomer) | 1H NMR (400 MHz, CHLOROFORM-D) δ 8.50 (dd, J = 8.0, 1.1 Hz, 1H), 7.57 (s, 1H), 7.46-7.39 (m, 3H), 7.36-7.27 (m, 4H), 7.20-7.11 (m, 1H), 7.10 (dd, J = 7.0, 2.4 Hz, 1H), 6.41 (br d, J = 15.3 Hz, 1H), 4.38-4.25 (m, 1H), 4.19-4.07 (m, 2H), 4.07-3.96 (m, 1H), 3.58-3.42 (m, 2H), 2.34-2.22 (m, 1H), 2.10-2.00 (m, 1H), 1.26-1.19 (m, 9H) | 578.5 |
| 3 | (E)-N-(4-(12-chloro-4,5,6,7-tetrahydrobenzo[2,3 ][1,6]oxazecino[4,5, 6-hi]indole-1-carbonyl)-2,6-difluorophenyl)-4-(((1r,4r)-4-methoxycyclohexyl) amino)but-2-enamide | 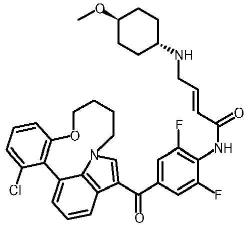 | A | | 1H-NMR (400 MHz, CHLOROFORM-D) δ 8.51 (dd, J = 8.0, 1.0 Hz, 1H), 7.59 (s, 1H), 7.44-7.38 (m, 3H), 7.34 (t, J = 8.4 Hz, 1H), 7.25-7.19 (m, 3H), 7.13 (dt, J = 15.3, 5.2 Hz, 1H), 6.93 (d, J = 8.0 Hz, 1H), 6.28 (d, J = 15.5 Hz, 1H), 4.41 (dd, J = 11.3, 2.3 Hz, 1H), 4.14 (t, J = 8.4 Hz, 1H), 4.09-4.01 (m, 1H), 3.97-3.90 (m, 1H), 3.52 (dd, J = 5.3, 1.5 Hz, 2H), 3.35 (s, 3H), 3.19-3.12 (m, 1H), 2.59-2.50 (m, 1H), 2.22-1.95 (m, 4H), 1.78-1.63 (m, 2H), 1.48-1.36 (m, 1H), 1.33-1.08 (m, 4H) | 648.6 |
| 4-1, 4-2 | (E)-N-(4-(11-chloro-5,6-dihydro-4H-benzo[8,9][1,5]oxaz onino[7,6,5-hi]indole-1-carbonyl)-2,6-difluorophenyl)-4-(((1r,4r)-4-methoxycyclohexyl) amino)but-2-enamide | 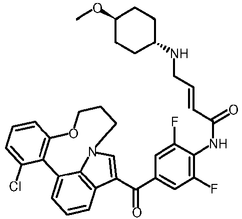 | A | Purified by preparative HPLC (CHIRALPAK IE (hexane-ethanol (0.1%triethyl amine))) to afford Example 4-1 (single isomer, 1st-eluting isomer) and Example 4-2 (single isomer, 2nd-eluting isomer) | 1H NMR (400 MHz, CHLOROFORM-D) δ 8.51 (d, J = 7.9 Hz, 1H), 7.54 (s, 1H), 7.47-7.37 (m, 3H), 7.37-7.27 (m, 4H), 7.18-7.04 (m, 2H), 6.29 (d, J = 15.4 Hz, 1H), 4.35-4.30 (m, 1H), 4.22-4.07 (m, 2H), 4.07-3.93 (m, 1H), 3.52 (d, J = 4.1 Hz, 2H), 3.35 (s, 3H), 3.25-3.06 (m, 1H), 2.60-2.45 (m, 1H), 2.37-2.22 (m, 1H), 2.16-1.93 (m, 4H), 1.36-1.10 (m, 4H) | 634.5 |
| 5-1, 5-2 | (E)-N-(2,6-difluoro-4-(8-(2-oxopyrrolidin-1-yl)-10-(trifluoromethyl)-4,5-dihydrobenzo [7,8] [1 ,4]oxazocino[6,5,4-hi]indole-1-carbonyl)phenyl)-4-(((1r,4r)-4-methoxycyclohexyl) amino)but-2-enamide | 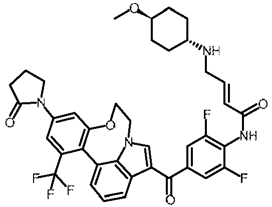 | A | Purified by preparative HPLC (CHIRALPAK IC (hexane-ethanol (0.1%triethyl amine))) to afford Example 5-1 (single isomer, 1st-eluting isomer) and Example 5-2 (single isomer, 2nd-eluting isomer) | 1H-NMR (400 MHz, CHLOROFORM-D) δ 8.46 (d, J = 8.0 Hz, 1H), 7.98 (s, 1H), 7.81 (d, J = 2.0 Hz, 1H), 7.57 (s, 1H), 7.41 (d, J = 8.3 Hz, 2H), 7.35 (t, J = 7.8 Hz, 1H), 7.18 (d, J = 7.3 Hz, 1H), 7.06 (dt, J = 15.3, 5.6 Hz, 1H), 6.39 (d, J = 15.3 Hz, 1H), 4.50-4.30 (m, 2H), 4.12 (d, J = 10.3 Hz, 1H), 3.97-3.92 (m, 3H), 3.58 (d, J = 5.3 Hz, 2H), 3.34 (s, 3H), 3.17-3.12 (m, 1H), 2.70-2.64 (m, J = 8.1 Hz, 3H), 2.28-2.21 (m, 2H), 2.11-2.02 (m, 4H), 1.31-1.21 (m, 4H) | 737.5 |
| 6 | (E)-N-(2,6-difluoro-4-(10-methyl-4,5-dihydrobenzo [7,8] [1 ,4]oxazocino[6,5,4-hi]indole-1-carbonyl)phenyl)-4-(((1r,4r)-4-methoxycyclohexyl) amino)but-2-enamide | 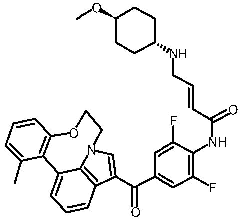 | A | | 1H-NMR (400 MHz, CHLOROFORM-D) δ 8.44 (d, J = 8.0 Hz, 1H), 7.53 (s, 1H), 7.44 (s, 1H), 7.42 (s, 1H), 7.40-7.32 (m, 2H), 7.23 (d, J = 7.8 Hz, 1H), 7.18-7.09 (m, 3H), 7.03 (d, J = 7.8 Hz, 1H), 6.27 (d, J = 15.3 Hz, 1H), 4.59-4.52 (m, 1H), 4.33 (td, J = 11.8, 2.3 Hz, 1H), 4.11 (dd, J = 11.4, 2.4 Hz, 1H), 3.86 (dd, J = 14.9, 1.9 Hz, 1H), 3.52 (d, J = 5.3 Hz, 2H), 3.36 (s, 3H), 3.19-3.12 (m, 1H), 2.58-2.51 (m, 1H), 2.40 (s, 3H), 2.10-2.07 (m, 2H), 2.00 (d, J = 12.0 Hz, 2H), 1.31-1.11 (m, 4H) | 600.3 |
| 7 | (E)-N-(2,6-difluoro-4-(10-fluoro-4,5-dihydrobenzo [7,8] [1 ,4]oxazocino[6,5,4-hi]indole-1-carbonyl)phenyl)-4-(((1r,4r)-4-methoxycyclohexyl) amino)but-2-enamide | 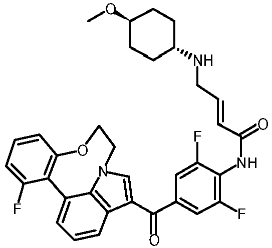 | A | | 1H-NMR (400 MHz, CHLOROFORM-D) δ 8.48 (dd, J = 6.5, 2.5 Hz, 1H), 7.54 (s, 1H), 7.43-7.37 (m, 5H), 7.19-7.09 (m, 3H), 7.02 (d, J = 8.0 Hz, 1H), 6.27 (d, J = 15.5 Hz, 1H), 4.68-4.61 (m, 1H), 4.40 (td, J = 11.9, 2.6 Hz, 1H), 4.16-4.11 (m, 1H), 3.95 (dd, J = 15.3, 2.3 Hz, 1H), 3.53-3.51 (m, 2H), 3.36 (s, 3H), 3.19-3.12 (m, 1H), 2.58-2.51 (m, 1H), 2.10-2.07 (m, 2H), 2.03-1.98 (m, 2H), 1.31-1.11 (m, 4H) | 604.2 |
| 8 | (E)-N-(4-(10-chloro-8-(2-oxopyrrolidin-1-yl)-4,5-dihydrobenzo [7,8] [1 ,4]oxazocino[6,5,4-hi]indole-1-carbonyl)-2,6-difluorophenyl)-4-(((1r,4r)-4-methoxycyclohexyl) amino)but-2-enamide | 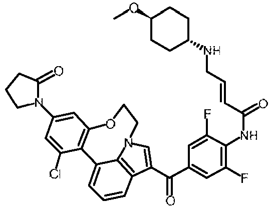 | A | | 1H-NMR (400 MHz, CHLOROFORM-D) δ 8.46 (dd, J = 6.9, 2.1 Hz, 1H), 7.71 (d, J = 2.3 Hz, 1H), 7.61 (d, J = 2.0 Hz, 1H), 7.53 (s, 1H), 7.43-7.38 (m, 4H), 7.23 (s, 1H), 7.12 (dt, J = 15.4, 5.2 Hz, 1H), 6.27 (d, J = 15.3 Hz, 1H), 4.63-4.56 (m, 1H), 4.35 (td, J = 11.9, 2.4 Hz, 1H), 4.19-4.11 (m, 1H), 3.94-3.88 (m, 3H), 3.52-3.51 (m, 2H), 3.35 (s, 3H), 3.18-3.12 (m, 1H), 2.67 (t, J = 8.1 Hz, 2H), 2.57-2.51 (m, 1H), 2.26-2.19 (m, 2H), 2.10-2.07 (m, 2H), 2.01-1.98 (m, 2H), 1.31-1.10 (m, 4H) | 703.3 |
| 9 | (E)-N-(4-(10-chloro-9-cyano-4,5-dihydrobenzo [7,8] [1 ,4]oxazocino[6,5,4-hi]indole-1-carbonyl)-2,6-difluorophenyl)-4-(((1r,4r)-4-methoxycyclohexyl) amino)but-2-enamide | 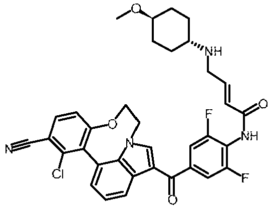 | A | | 1H-NMR (400 MHz, CHLOROFORM-D) δ 8.53 (d, J = 7.5 Hz, 1H), 7.78 (d, J = 8.5 Hz, 1H), 7.56 (s, 1H), 7.46-7.37 (m, 4H), 7.24 (d, J = 8.5 Hz, 1H), 7.12 (td, J = 10.3, 5.1 Hz, 1H), 6.28 (d, J = 15.5 Hz, 1H), 4.57-4.42 (m, 2H), 4.18 (d, J = 11.3 Hz, 1H), 3.99 (d, J = 14.0 Hz, 1H), 3.53-3.48 (m, 2H), 3.41-3.29 (m, 1H), 3.35 (s, 3H), 3.19-3.11 (m, 1H), 2.62-2.53 (m, 1H), 2.09 (d, J = 11.5 Hz, 2H), 2.03-1.94 (m, 2H), 1.31-1.19 (m, 4H) | 645.2 |
| 10 | (E)-N-(2,6-difluoro-4-(10-methoxy-4,5-dihydrobenzo [7,8] [1 ,4]oxazocino[6,5,4-hi]indole-1-carbonyl)phenyl)-4-(((1r,4r)-4-methoxycyclohexyl) amino)but-2-enamide | 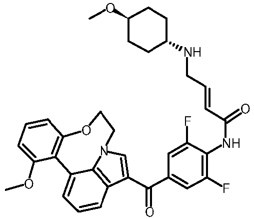 | A | | 1H-NMR (400 MHz, CHLOROFORM-D) δ 8.53 (d, J = 7.5 Hz, 1H), 7.78 (d, J = 8.5 Hz, 1H), 7.56 (s, 1H), 7.46-7.37 (m, 4H), 7.24 (d, J = 8.5 Hz, 1H), 7.12 (td, J = 10.3, 5.1 Hz, 1H), 6.28 (d, J = 15.5 Hz, 1H), 4.57-4.42 (m, 2H), 4.18 (d, J = 11.3 Hz, 1H), 3.99 (d, J = 14.0 Hz, 1H), 3.53-3.48 (m, 2H), 3.41-3.29 (m, 1H), 3.35 (s, 3H), 3.19-3.11 (m, 1H), 2.62-2.53 (m, 1H), 2.09 (d, J = 11.5 Hz, 2H), 2.03-1.94 (m, 2H), 1.31-1.19 (m, 4H) | 616.3 |
| 11-1, 11-2 | (E)-N-(2,6-difluoro-4-(10-(trifluoromethyl)-4,5-dihydrobenzo[7,8] [1 ,4]oxazocino[6,5,4-hi]indole-1-carbonyl)phenyl)-4-(((1r,4r)-4-methoxycyclohexyl) amino)but-2-enamide | 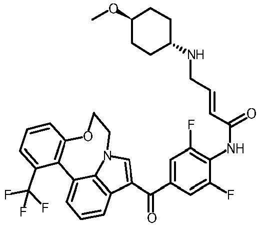 | A | Purified by preparative HPLC (CHIRALPAK IE (hexane-ethanol (0.1%triethyl amine))) to afford Example 11-1 (single isomer, 1st-eluting isomer) and Example 11-2 (single isomer, 2nd-eluting isomer) | 1H-NMR (400 MHz, CHLOROFORM-D) δ 8.50 (d, J = 8.0 Hz, 1H), 7.74 (d, J = 8.0 Hz, 1H), 7.59 (t, J = 7.9 Hz, 1H), 7.54 (s, 1H), 7.47 (s, 1H), 7.45 (s, 1H), 7.39 (t, J = 7.6 Hz, 2H), 7.22 (d, J = 7.5 Hz, 1H), 7.13 (td, J = 10.3, 5.0 Hz, 1H), 6.97 (s, 1H), 6.25 (d, J = 15.3 Hz, 1H), 4.54-4.47 (m, 1H), 4.37 (td, J = 11.9, 2.4 Hz, 1H), 4.15-4.10 (m, 1H), 3.92 (d, J = 14.5 Hz, 1H), 3.52 (dd, J = 5.0, 1.5 Hz, 2H), 3.36 (s, 3H), 3.19-3.12 (m, 1H), 2.62-2.50 (m, 1H), 2.10-2.07 (m, 2H), 1.99 (d, J = 12.3 Hz, 2H), 1.31-1.10 (m, 4H) | 654.3 |
| 13 | (E)-4-(tert-butylamino)-N-(2,6-difluoro-4-(6,8,9-trimethyl-12,13-dihydro-8H-imidazo[4",5":3',4']b enzo[1',2':7,8][1,4]o xazocino[6,5,4-hi]indole-2-carbonyl)phenyl)but -2-enamide | 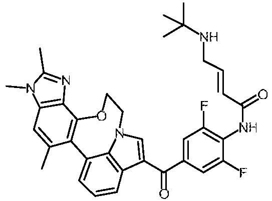 | B | | 1H-NMR (400 MHz, CHLOROFORM-D) δ 8.43 (dd, J = 8.0, 1.0 Hz, 1H), 7.53 (s, 1H), 7.46-7.40 (m, 2H), 7.37 (t, J = 7.8 Hz, 1H), 7.21-7.10 (m, 3H), 6.29 (dt, J = 15.4, 1.7 Hz, 1H), 4.64-4.46 (m, 2H), 4.45-4.34 (m, 1H), 3.84 (dd, J = 14.6, 1.9 Hz, 1H), 3.77 (s, 3H), 3.45 (dd, J = 5.0, 1.8 Hz, 2H), 2.63 (s, 3H), 2.49 (s, 3H), 1.10 (s, 9H) | 612.4 |
| 14-1, 14-2 | (E)-N-(2,6-difluoro-4-(6,8,9-trimethyl-12,13-dihydro-8H-imidazo[4",5":3',4']b enzo[1',2':7,8][1,4]o xazocino[6,5,4-hi]indole-2-carbonyl)phenyl)-4-(((1r,4r)-4-methoxycyclohexyl) amino)but-2-enamide | 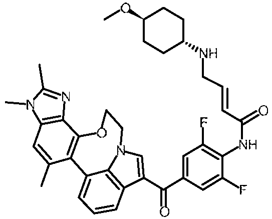 | B | Purified by preparative HPLC (CHIRAL ART SB(YMC) (hexane-ethanol (0.1 %triethyl amine))) to afford Example 14-1 (single isomer, 1st-eluting isomer) and Example 14-2 (single isomer, 2nd-eluting isomer) | 1H-NMR (400 MHz, CHLOROFORM-D) δ 8.43 (dd, J = 8.0, 1.0 Hz, 1H), 7.56 (s, 1H), 7.47-7.41 (m, 2H), 7.37 (dd, J = 7.6, 7.6 Hz, 1H), 7.21-7.07 (m, 4H), 6.26 (dt, J = 15.3, 1.6 Hz, 1H), 4.62-4.46 (m, 2H), 4.45-4.33 (m, 1H), 3.84 (dd, J = 14.4, 1.9 Hz, 1H), 3.75 (s, 3H), 3.51 (dd, J = 5.3, 1.8 Hz, 2H), 3.35 (s, 3H), 3.19-3.10 (m, 1H), 2.63 (s, 3H), 2.57-2.51 (m, 1H), 2.49 (s, 3H), 2.13-2.03 (m, 2H), 2.03-1.94 (m, 2H), 1.36-1.09 (m, 4H) | 668.5 |
| 15-1, 15-2 | (E)-N-(2,6-difluoro-4-(6,8,9-trimethyl-13,14-dihydro-8H,12H-imidazo[4",5":3',4']b enzo[1',2':8,9][1,5]o xazonino[7,6,5-hi]indole-2-carbonyl)phenyl)-4-(((1r,4r)-4-methoxycyclohexyl) amino)but-2-enamide | 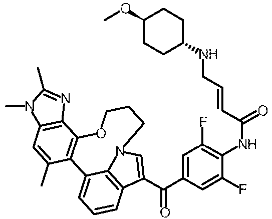 | B | Purified by preparative HPLC (CHIRALPAK IE (hexane-ethanol (0.1%triethyl amine))) to afford Example 15-1 (single isomer, 1st-eluting isomer) and Example 15-2 (single isomer, 2nd-eluting isomer) | 1H-NMR (400 MHz, CHLOROFORM-D) δ 8.45 (dd, J = 8.0, 1.3 Hz, 1H), 7.53 (s, 1H), 7.45-7.38 (m, 3H), 7.14-7.08 (m, 2H), 7.02 (d, J = 0.8 Hz, 1H), 6.29 (dt, J = 15.4, 1.6 Hz, 1H), 4.91-4.85 (m, 1H), 4.24-4.11 (m, 2H), 3.89 (td, J = 9.8, 5.0 Hz, 1H), 3.74 (s, 3H), 3.52 (dd, J = 5.3, 1.8 Hz, 2H), 3.35 (s, 3H), 3.18-3.11 (m, 1H), 2.63 (s, 3H), 2.59-2.52 (m, 1H), 2.26-2.17 (m, 4H), 2.12-1.92 (m, 4H), 1.30-1.12 (m, 4H) | 682.5 |
| 16 | (E)-N-(2,6-difluoro-4-(2,3,5-trimethyl-3H,12H-imidazo[4",5":3',4']b enzo[1',2':6,7][1,3]o xazepino[5,4,3-hi]indole-9-carbonyl)phenyl)-4-(((1r,4r)-4-methoxycyclohexyl) amino)but-2-enamide | 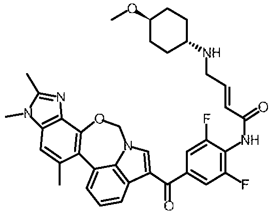 | B | | 1H-NMR (400 MHz, CHLOROFORM-D) δ 8.38 (d, J = 7.0 Hz, 1H), 7.63 (s, 1H), 7.51-7.39 (m, 4H), 7.15-7.09 (m, 2H), 6.26 (d, J = 15.3 Hz, 1H), 6.11-6.03 (m, 2H), 3.74 (s, 3H), 3.52 (d, J = 4.3 Hz, 2H), 3.35 (s, 3H), 3.17-3.12 (m, 1H), 2.75 (s, 3H), 2.64 (s, 3H), 2.56-2.48 (m , 1H), 2.10-1.98 (m, 4H), 1.30-1.15 (m, 4H). | 654.5 |
| 17 | (E)-N-(2,6-difluoro-4-((R)-6,8,9,13-tetramethyl-12,13-dihydro-8H-imidazo[4",5":3',4']b enzo[1',2':7,8][1,4]o xazocino[6,5,4-hi]indole-2-carbonyl)phenyl)-4-(((1r,4r)-4-methoxycyclohexyl) amino)but-2-enamide | 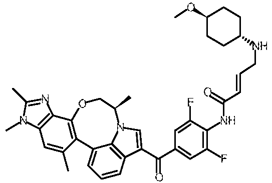 | B | | 1H-NMR (400 MHz, CHLOROFORM-D) δ 8.44 (dd, J = 7.9, 1.1 Hz, 1H), 7.52-7.45 (m, 3H), 7.38 (t, J = 7.8 Hz, 1H), 7.19 (dd, J = 7.4, 1.1 Hz, 1H), 7.16-7.10 (m, 2H), 6.27 (d, J = 14.8 Hz, 1H), 4.82 (dd, J = 6.3, 2.5 Hz, 1H), 4.61 (dd, J = 14.9, 2.9 Hz, 1H), 3.75 (s, 3H), 3.58-3.52 (m, 3H), 3.35 (s, 3H), 3.16-3.13 (m, 1H), 2.63 (s, 3H), 2.47 (s, 3H), 2.11-1.99 (m, 4H), 1.31-1.15 (m, 7H). | 682.5 |
| 18 | (E)-N-(2,6-difluoro-4-((S)-6,8,9,13-tetramethyl-12,13-dihydro-8H-imidazo[4",5":3',4']b enzo[1',2':7,8][1,4]o xazocino[6,5,4-hi]indole-2-carbonyl)phenyl)-4-(((1r,4r)-4-methoxycyclohexyl) amino)but-2-enamide | 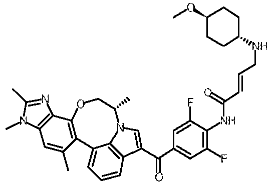 | B | | 1H-NMR (400 MHz, CHLOROFORM-D) δ 8.44 (dd, J = 8.0, 1.0 Hz, 1H), 7.52-7.45 (m, 3H), 7.38 (t, J = 7.8 Hz, 1H), 7.20 (dd, J = 7.5, 1.3 Hz, 1H), 7.14-7.09 (m, 2H), 6.26 (d, J = 15.3 Hz, 1H), 4.83-4.80 (m, 1H), 4.61 (dd, J = 14.8, 3.0 Hz, 1H), 3.75 (s, 3H), 3.58-3.52 (m, 3H), 3.36 (s, 3H), 3.19-3.12 (m, 1H), 2.62 (s, 3H), 2.47 (s, 3H), 2.10-1.98 (m, 4H), 1.31-1.14 (m, 7H). | 682.5 |
| 19 | (E)-N-(2,6-difluoro-4-((R)-6,8,9,12-tetramethyl-12,13-dihydro-8H-imidazo[4",5":3',4']b enzo[1',2':7,8][1,4]o xazocino[6,5,4-hi]indole-2-carbonyl)phenyl)-4-(((1r,4r)-4-methoxycyclohexyl) amino)but-2-enamide | 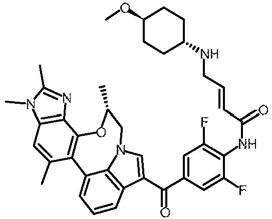 | B | | 1H-NMR (400 MHz, CHLOROFORM-D) δ 8.44 (dd, J = 8.0, 1.0 Hz, 1H), 7.52-7.43 (m, 3H), 7.38 (t, J = 7.8 Hz, 1H), 7.20 (dd, J = 7.5, 1.3 Hz, 1H), 7.16-7.06 (m, 2H), 6.26 (d, J = 15.3 Hz, 1H), 4.85-4.79 (m, 1H), 4.61 (dd, J = 14.8, 3.0 Hz, 1H), 3.75 (s, 3H), 3.58-3.51 (m, 3H), 3.35 (s, 3H), 3.19-3.04 (m, 1H), 2.62 (s, 3H), 2.47 (s, 3H), 2.10-1.98 (m, 4H), 1.32-1.11 (m, 7H). | 682.5 |
| 20 | (E)-N-(2,6-difluoro-4-((S)-6,8,9,12-tetramethyl-12,13-dihydro-8H-imidazo[4",5":3',4']b enzo[1',2':7,8][1,4]o xazocino[6,5,4-hi]indole-2-carbonyl)phenyl)-4-(((1r,4r)-4-methoxycyclohexyl) amino)but-2-enamide | 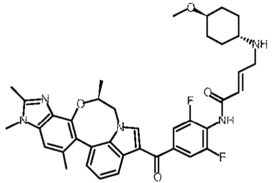 | B | | 1H-NMR (400 MHz, CHLOROFORM-D) δ 8.44 (dd, J = 8.0, 1.3 Hz, 1H), 7.51-7.44 (m, 2H), 7.38 (t, J = 7.8 Hz, 1H), 7.23-7.18 (m, 1H), 7.16-7.09 (m, 2H), 6.26 (d, J = 15.3 Hz, 1H), 4.85-4.79 (m, 1H), 4.61 (dd, J = 14.8, 3.0 Hz, 1H), 3.75 (s, 3H), 3.56-3.52 (m, 3H), 3.36 (s, 3H), 3.19-3.12 (m, 1H), 2.62 (s, 3H), 2.47 (s, 3H), 2.10-1.98 (m, 4H), 1.31-1.11 (m, 7H). | 682.5 |
| 21-1, 21-2 | (E)-N-(2,6-difluoro-4-(6',8',9'-trimethyl-8'H,12'H,14'H-spiro[cyclopropane-1,13'-imidazo[4",5":3',4']b enzo[1',2':8,9][1,5]o xazonino[7,6,5-hi]indole]-2'-carbonyl)phenyl)-4-(((1r,4r)-4-methoxycyclohexyl) amino)but-2-enamide | 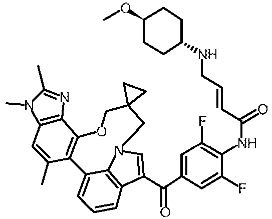 | B | Purified by preparative HPLC (CHIRAL ART SB(YMC) (hexane-ethanol (0.1%triethyl amine))) to afford Example 21-1 (single isomer, 1st-eluting isomer) and Example 21-2 (single isomer, 2nd-eluting isomer) | 1H-NMR (400 MHz, CHLOROFORM-D) δ 8.46 (dd, J = 8.0, 1.0 Hz, 1H), 7.52-7.39 (m, 4H), 7.16-7.09 (m, 2H), 7.03 (d, J = 0.8 Hz, 1H), 6.25 (d, J = 15.0 Hz, 1H), 4.54 (d, J = 14.5 Hz, 1H), 4.27 (d, J = 12.5 Hz, 1H), 4.09 (d, J = 12.3 Hz, 1H), 3.75 (s, 3H), 3.53-3.49 (m, 2H), 3.35 (s, 3H), 3.18-3.06 (m, 2H), 2.63 (s, 3H), 2.18 (s, 3H), 2.10-1.98 (m, 4H), 1.30-1.11 (m, 5H), 0.51-0.41 (m, 2H), 0.23-0.19 (m, 1H) | 708.5 |
| 22 | (E)-N-(2,6-difluoro-4-((S)-6,8,9,13-tetramethyl-13,14-dihydro-8H, 12H-imidazo[4",5":3',4']b enzo[1',2':8,9][1,5]o xazonino[7,6,5-hi]indole-2-carbonyl)phenyl)-4-(((1r,4r)-4-methoxycyclohexyl) amino)but-2-enamide | 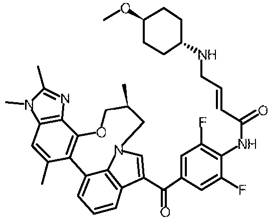 | B | | 1H-NMR (400 MHz, CHLOROFORM-D) δ 8.45 (dt, J = 8.0, 1.6 Hz, 1H), 7.53 (d, J = 5.0 Hz, 1H), 7.45 (dt, J = 13.0, 2.6 Hz, 2H), 7.41-7.36 (m, 1H), 7.16-7.09 (m, 1H), 7.05 (dd, J = 7.3, 1.3 Hz, 1H), 7.01-7.00 (m, 1H), 6.29 (d, J = 15.3 Hz, 1H), 4.58-4.52 (m, 1H), 4.47-4.37 (m, 1H), 3.75 (s, 3H), 3.68-3.61 (m, 1H), 3.53 (d, J = 5.3 Hz, 2H), 3.35 (s, 3H), 3.19-3.12 (m, 1H), 2.63-2.55 (m, 4H), 2.17-1.97 (m, 6H), 1.31-1.17 (m, 4H), 1.13 (d, J = 6.3 Hz, 3H). | 696.4 |
| 23 | (E)-N-(2,6-difluoro-4-((R)-6,8,9,13-tetramethyl-13,14-dihydro-8H,12H-imidazo[4",5":3',4']b enzo[1',2':8,9][1,5]o xazonino[7,6,5-hi]indole-2-carbonyl)phenyl)-4-(((1r,4r)-4-methoxycyclohexyl) amino)but-2-enamide | 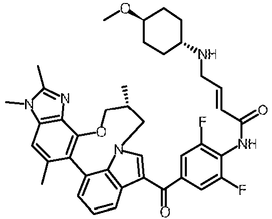 | B | Purified by preparative HPLC (CHIRALPAK SB(YMC) (hexane-ethanol (0.1%triethyl amine))) to afford Example 23 (single isomer, 1st-eluting isomer) and Example 24 (single isomer, 2nd-eluting isomer) | 1H-NMR (400 MHz, CHLOROFORM-D) δ 8.47-8.44 (m, 1H), 7.52 (d, J = 4.0 Hz, 1H), 7.46-7.43 (m, 2H), 7.39 (t, J = 7.6 Hz, 1H), 7.16-7.09 (m, 1H), 7.05 (dd, J = 7.4, 1.1 Hz, 1H), 7.00 (d, J = 3.3 Hz, 1H), 6.27 (d, J = 15.5 Hz, 1H), 4.58-4.52 (m, 1H), 4.47-4.37 (m, 1H), 3.75 (s, 3H), 3.68-3.61 (m, 1H), 3.54 (dd, J = 18.3, 5.0 Hz, 2H), 3.35 (s, 3H), 3.19-3.12 (m, 1H), 2.63-2.57 (m, 4H), 2.17-1.98 (m, 6H), 1.31-1.18 (m, 4H), 1.13 (d, J = 6.3 Hz, 3H). | 696.4 |
| 24 | | | | | 1H-NMR (400 MHz, CHLOROFORM-D) δ 8.47-8.44 (m, 1H), 7.52 (d, J = 4.0 Hz, 1H), 7.46-7.43 (m, 2H), 7.39 (t, J = 7.6 Hz, 1H), 7.16-7.09 (m, 1H), 7.05 (dd, J = 7.4, 1.1 Hz, 1H), 7.00 (d, J = 3.3 Hz, 1H), 6.27 (d, J = 15.5 Hz, 1H), 4.58-4.52 (m, 1H), 4.30-4.26 (m, 1H), 3.75 (s, 3H), 3.68-3.61 (m, 1H), 3.54 (dd, J = 18.3, 5.0 Hz, 2H), 3.35 (s, 3H), 3.19-3.12 (m, 1H), 2.63-2.57 (m, 4H), 2.17-1.98 (m, 6H), 1.31-1.18 (m, 4H), 1.13 (d, J = 6.3 Hz, 3H). | 696.4 |
| 25 | (E)-N-(2,6-difluoro-4-(1,8,9-trimethyl-6-(trifluoromethyl)-12,13-dihydro-8H-imidazo[4",5":3',4']b enzo[1',2':7,8][1,4]o xazocino[6,5,4-hi]indole-2-carbonyl)phenyl)-4-(((1r,4r)-4-methoxycyclohexyl) amino)but-2-enamide | 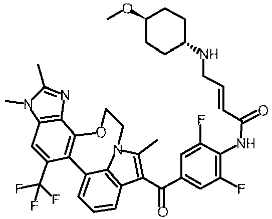 | B | | 1H-NMR (400 MHz, CHLOROFORM-D) δ 7.69 (s, 1H), 7.46-7.43 (m, 3H), 7.17-7.08 (m, 4H), 6.25 (d, J = 15.3 Hz, 1H), 4.45-4.30 (m, 3H), 4.02-3.94 (m, 1H), 3.85 (s, 3H), 3.50 (dd, J = 5.3, 1.8 Hz, 2H), 3.36 (s, 3H), 3.18-3.11 (m, 1H), 2.70 (s, 3H), 2.56-2.49 (m, 4H), 2.09-1.96 (m, 4H), 1.30-1.09 (m, 4H) | 736.3 |
| 28 | (E)-N-(4-(8,9-dimethyl-6-(trifluoromethyl)-8,11,12,13-tetrahydroimidazo [4' ',5":3',4']benzo[1',2': 4,5]azocino[3,2,1-hi]indole-2-carbonyl)-2,6-difluorophenyl)-4-(((1r,4r)-4-methoxycyclohexyl) amino)but-2-enamide | 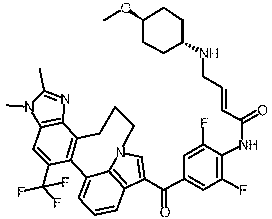 | D | | 1H-NMR (400 MHz, CHLOROFORM-D) δ 8.47 (dd, J = 8.0, 1.1 Hz, 1H), 7.72 (s, 1H), 7.52 (s, 1H), 7.45 (d, J = 7.8 Hz, 2H), 7.36 (t, J = 7.7 Hz, 1H), 7.17 (d, J = 7.4 Hz, 1H), 7.11 (dt, J = 15.4, 5.4 Hz, 1H), 6.31 (d, J = 15.4 Hz, 1H), 3.92-3.82 (m, 2H), 3.84 (s, 3H), 3.75-3.71 (m, 1H), 3.55-3.54 (m, 2H), 3.35 (s, 3H), 3.18-3.12 (m, 1H), 2.69 (s, 3H), 2.65-2.57 (m, 2H), 2.15-2.01 (m, 6H), 1.31-1.19 (m, 4H) | 720.4 |
| 29 | (E)-N-(4-(8,9-dimethyl-6-(trifluoromethyl)-11,12,13,14-tetrahydro-8H-imidazo[4",5":3',4']b enzo[1',2':4,5]azonin o[3,2,1-hi]indole-2-carbonyl)-2,6-difluorophenyl)-4-(((1r,4r)-4-methoxycyclohexyl)amino)but-2-enamide | 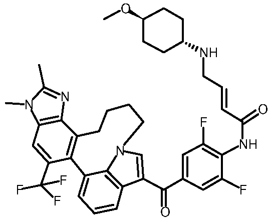 | D | | 1H-NMR (400 MHz, CHLOROFORM-D) δ 8.51 (dd, J = 8.0, 0.8 Hz, 1H), 7.67 (s, 1H), 7.50-7.43 (m, 3H), 7.40 (t, J = 7.7 Hz, 1H), 7.18-7.08 (m, 2H), 6.98-6.92 (brs, 1H), 6.24 (d, J = 15.3 Hz, 1H), 3.85 (s, 3H), 3.75-3.65 (m, 2H), 3.55-3.45 (m, 3H), 3.35 (s, 3H), 3.18-3.08 (m, 1H), 2.71 (s, 3H), 2.55-2.48 (m, 2H), 2.15-1.94 (m, 4H), 1.88-1.78 (m, 1H), 1.75-1.60 (m, 1H), 1.30-1.10 (m, 6H) | 734.4 |
| 32 | (E)-N-(2,6-difluoro-4-(7,9,10-trimethyl-13,14-dihydro-9H-imidazo[4", 5 ":5',6']b enzo[1',2':2,3]oxoci no[4,5,6-hi]indolizine-2-carbonyl)phenyl)-4-(((1r,4r)-4-methoxycyclohexyl) amino)but-2-enamide | 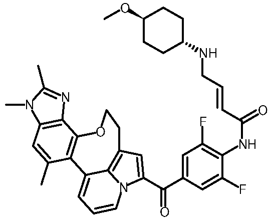 | E | | 1H-NMR (400 MHz, CHLOROFORM-D) δ 9.97 (dd, J = 7.0, 1.3 Hz, 1H), 7.45-7.39 (m, 2H), 7.17-7.00 (m, 6H), 6.24 (dt, J = 15.3, 1.7 Hz, 1H), 4.76 (dd, J = 10.4, 4.6 Hz, 1H), 4.20-4.14 (m, 1H), 3.74 (s, 3H), 3.69-3.56 (m, 1H), 3.51 (dd, J = 5.3, 1.8 Hz, 2H), 3.35 (s, 3H), 3.18-3.11 (m, 1H), 2.94-2.87 (m, 1H), 2.63 (s, 3H), 2.57-2.50 (m, 2H), 2.44 (s, 3H), 2.11-2.05 (m, 2H), 2.02-1.95 (m, 2H), 1.24-1.10 (m, 4H) | 668.4 |
| 33 | (E)-4-(tert-butylamino)-N-(4-(9,10-dimethyl-7-(trifluoromethyl)-13,14-dihydro-9H-imidazo[4", 5 ":5',6']b enzo[1',2':2,3]oxoci no[4,5,6-hi]indolizine-2-carbonyl)-2,6-difluorophenyl)but-2-enamide | 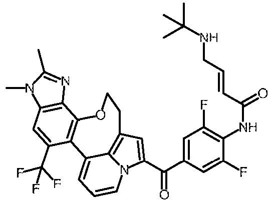 | E | | 1H-NMR (400 MHz, CHLOROFORM-D) δ 9.98 (dd, J = 7.0, 1.0 Hz, 1H), 7.64 (s, 1H), 7.46-7.36 (m, 2H), 7.18-7.11 (m, 3H), 7.02 (dd, J = 7.0, 7.0 Hz, 1H), 6.33 (d, J = 15.5 Hz, 1H), 4.73 (dd, J = 10.5, 4.5 Hz, 1H), 4.29-4.17 (m, 1H), 3.84 (s, 3H), 3.47 (dd, J = 5.3, 1.5 Hz, 2H), 2.88-2.80 (m, 1H), 2.70 (s, 3H), 2.57 (dd, J = 15.3, 2.3 Hz, 1H), 1.18 (s, 9H) | 666.4 |
| 34 | (E)-N-(4-(9,10-dimethyl-7-(trifluoromethyl)-13,14-dihydro-9H-imidazo[4", 5 ":5',6']b enzo[1',2':2,3]oxoci no[4,5,6-hi]indolizine-2-carbonyl)-2,6-difluorophenyl)-4-((tetrahydro-2H-pyran-4-yl)amino)but-2-enamide | 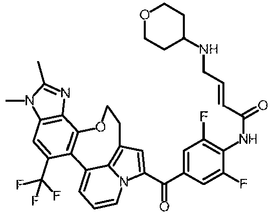 | E | | 1H-NMR (400 MHz, CHLOROFORM-D) δ 9.98 (dd, J = 7.0, 1.0 Hz, 1H), 7.65 (s, 1H), 7.47-7.38 (m, 2H), 7.17-7.00 (m, 5H), 6.27 (dt, J = 15.4, 1.7 Hz, 1H), 4.74 (dd, J = 10.4, 4.6 Hz, 1H), 4.23-4.17 (m, 1H), 4.04-3.95 (m, 2H), 3.84 (s, 3H), 3.54 (dd, J = 5.1, 1.6 Hz, 2H), 3.46-3.37 (m, 2H), 2.89-2.72 (m, 2H), 2.70 (s, 3H), 2.62-2.52 (m, 1H), 1.88-1.85 (m, 2H), 1.48-1.38 (m, 2H) | 694.4 |
| 35 | (E)-N-(4-(9,10-dimethyl-7-(trifluoromethyl)-13,14-dihydro-9H-imidazo[4",5":5',6']b enzo[1',2':2,3]oxoci no[4,5,6-hi]indolizine-2-carbonyl)-2,6-difluorophenyl)-4-((1-methylcyclopropyl)a mino)but-2-enamide | 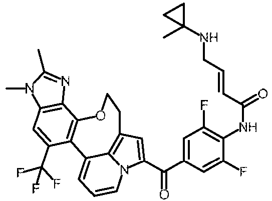 | E | | 1H-NMR (500 MHz, CHLOROFORM-D) δ 9.98 (dd, J = 7.0, 0.9 Hz, 1H), 7.64 (s, 1H), 7.44-7.39 (m, 2H), 7.18-7.09 (m, 3H), 7.06-6.99 (m, 2H), 6.22 (dt, J = 15.4, 2.0 Hz, 1H), 4.73 (dd, J = 10.4, 4.6 Hz, 1H), 4.23-4.17 (m, 1H), 3.84 (s, 3H), 3.55 (dd, J = 4.9, 1.8 Hz, 2H), 2.88-2.80 (m, 1H), 2.70 (s, 3H), 2.57 (dd, J = 15.3, 2.1 Hz, 1H), 1.29 (s, 3H), 0.60 (dd, J = 5.6, 4.7 Hz, 2H), 0.41 (dd, J = 6.3, 4.4 Hz, 2H) | 664.5 |
| 36 | (E)-N-(4-(9,10-dimethyl-7-(trifluoromethyl)-13,14-dihydro-9H-imidazo[4", 5 ":5',6']b enzo[1',2':2,3]oxoci no[4,5,6-hi]indolizine-2-carbonyl)-2,6-difluorophenyl)-4-((1-methyl-1-oxidophosphinan-4-yl)amino)but-2-enamide | 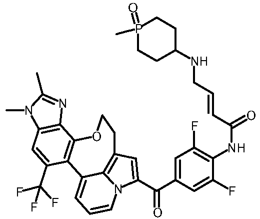 | E | | 1H-NMR (400 MHz, CHLOROFORM-D) δ 9.98 (dd, J = 7.0, 1.0 Hz, 1H), 8.01 (s, 1H), 7.64 (s, 1H), 7.46-7.39 (m, 2H), 7.19-7.08 (m, 3H), 7.01 (dd, J = 7.1, 7.1 Hz, 1H), 6.41 (dt, J = 15.3, 2.1 Hz, 1H), 4.73 (dd, J = 10.4, 4.6 Hz, 1H), 4.23-4.17 (m, 1H), 3.84 (s, 3H), 3.49 (dd, J = 4.8, 1.8 Hz, 2H), 2.88-2.80 (m, 1H), 2.76-2.70 (m, 4H), 2.57 (dd, J = 15.4, 2.1 Hz, 1H), 2.25-2.10 (m, 2H), 2.07-1.90 (m, 4H), 1.68-1.59 (m, 2H), 1.51 (d, J = 12.8 Hz, 3H) | 740.4 |
| 37 | (E)-N-(4-(9,10-dimethyl-7-(trifluoromethyl)-13,14-dihydro-9H-imidazo[4", 5 ":5',6']b enzo[1',2':2,3]oxoci no[4,5,6-hi]indolizine-2-carbonyl)phenyl)-4-(((1r,4r)-4-methoxycyclohexyl) amino)but-2-enamide | 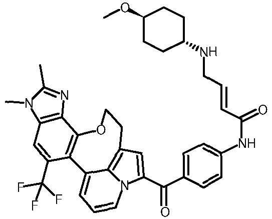 | E | | 1H-NMR (400 MHz, CHLOROFORM-D) δ 10.01-9.99 (m, 1H), 7.82 (d, J = 8.8 Hz, 2H), 7.69 (d, J = 8.5 Hz, 1H), 7.64 (s, 1H), 7.55-7.52 (m, 1H), 7.14 (s, 1H), 7.11-7.03 (m, 2H), 7.00-6.95 (m, 1H), 6.19 (d, J = 15.3 Hz, 1H), 4.72 (q, J = 5.1 Hz, 1H), 4.23-4.17 (m, 1H), 3.84 (s, 3H), 3.53-3.49 (m, 2H), 3.34 (s, 3H), 3.17-3.10 (m, 1H), 2.86-2.82 (m, 1H), 2.70 (s, 3H), 2.62-2.53 (m, 2H), 2.17-1.99 (m, 4H), 1.30-1.21 (m, 4H) | 686.4 |
| 38-1, 38-2 | (E)-N-(4-(9,10-dimethyl-7-(trifluoromethyl)-13,14-dihydro-9H-imidazo[4", 5 ":5',6']b enzo[1',2':2,3]oxoci no[4,5,6-hi]indolizine-2-carbonyl)-2,6-difluorophenyl)-4-(dimethylamino)but-2-enamide | 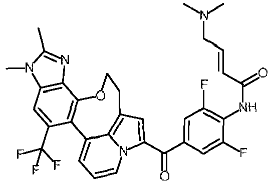 | E | Purified by preparative HPLC (CHIRALPAK ID (hexane-ethanol (0.1%triethyl amine))) to afford Example 38-1 (single isomer, 1st-eluting isomer) and Example 38-2 (single isomer, 2nd-eluting isomer) | 1H-NMR (400 MHz, CHLOROFORM-D) δ 9.99 (d, J = 6.8 Hz, 1H), 7.64 (s, 1H), 7.48-7.37 (m, 2H), 7.20-7.16 (m, 2H), 7.09-7.00 (m, 3H), 6.25 (d, J = 15.5 Hz, 1H), 4.74 (dd, J = 10.4, 4.6 Hz, 1H), 4.23-4.17 (m, 1H), 3.84 (s, 3H), 3.16 (d, J = 5.0 Hz, 2H), 2.89-2.80 (m, 1H), 2.70 (s, 3H), 2.57 (dd, J = 15.2, 1.6 Hz, 1H), 2.31 (s, 6H) | 638.4 |
| 39 | (E)-N-(4-(9,10-dimethyl-7-(trifluoromethyl)-13,14-dihydro-9H-imidazo[4", 5 ":5',6']b enzo[1',2':2,3]oxoci no[4,5,6-hi]indolizine-2-carbonyl)-2,6-difluorophenyl)-4-((4-methyltetrahydro-2H-pyran-4-yl)amino)but-2-enamide | 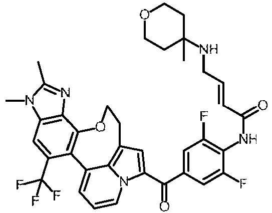 | E | | 1H-NMR (400 MHz, CHLOROFORM-D) δ 9.98 (d, J = 7.0 Hz, 1H), 7.65 (s, 1H), 7.43 (d, J = 8.0 Hz, 2H), 7.21-7.11 (m, 3H), 7.10-6.98 (m, 2H), 6.34 (d, J = 15.3 Hz, 1H), 4.74 (dd, J = 10.3, 4.5 Hz, 1H), 4.23-4.17 (m, 1H), 3.92-3.77 (m, 5H), 3.68-3.57 (m, 2H), 3.44 (d, J = 3.8 Hz, 2H), 2.89-2.81 (m, 1H), 2.70 (s, 3H), 2.63-2.52 (m, 1H), 1.58 (t, J = 5.0 Hz, 4H), 1.18 (s, 3H) | 708.4 |
| 40 | (E)-1-((4-((4-(9,10-dimethyl-7-(trifluoromethyl)-13,14-dihydro-9H-imidazo[4",5":5',6']b enzo[1',2':2,3]oxoci no[4,5,6-hi]indolizine-2-carbonyl)-2,6-difluorophenyl)amin o)-4-oxobut-2-en-1-yl)amino)cyclohexa ne-1-carboxylic acid | 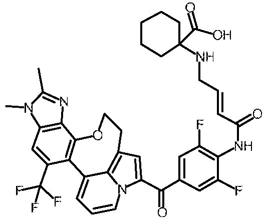 | E | | 1H-NMR (400 MHz, CHLOROFORM-D) δ 9.98 (d, J = 6.8 Hz, 1H), 7.65 (s, 1H), 7.53 (s, 1H), 7.41 (d, J = 8.0 Hz, 2H), 7.18-7.16 (m, 2H), 7.10-7.00 (m, 2H), 6.32 (d, J = 15.5 Hz, 1H), 4.85 (d, J = 3.3 Hz, 2H), 4.73 (dd, J = 10.3, 4.5 Hz, 1H), 4.20 (t, J = 10.5 Hz, 1H), 3.84 (s, 3H), 2.96-2.80 (m, 2H), 2.70 (s, 3H), 2.62-2.56 (m, 1H), 2.05-1.98 (m, 2H), 1.71-1.71 (m, 8H), 1.43-1.39 (m, 1H) | 736.2 |
| 41 | (E)-N-(4-(9,10-dimethyl-7-(trifluoromethyl)-13,14-dihydro-9H-imidazo[4",5":5',6']b enzo[1',2':2,3]oxoci no[4,5,6-hi]indolizine-2-carbonyl)-2,6-difluorophenyl)-4-((3-methyltetrahydrofur an-3-yl)amino)but-2-enamide | 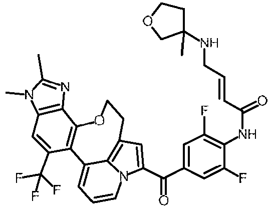 | E | | 1H-NMR (400 MHz, CHLOROFORM-D) δ 9.98 (dd, J = 7.0, 1.0 Hz, 1H), 7.65 (s, 1H), 7.46-7.43 (m, 2H), 7.15 (td, J = 10.0, 5.3 Hz, 3H), 7.04-7.00 (m, 1H), 6.31 (d, J = 15.3 Hz, 1H), 4.74 (dd, J = 10.5, 4.5 Hz, 1H), 4.24-4.17 (m, 1H), 4.03-3.97 (m, 1H), 3.92-3.87 (m, 1H), 3.84 (s, 3H), 3.72-3.61 (m, 2H), 3.49-3.47 (m, 3H), 2.88-2.82 (m, 1H), 2.70 (s, 3H), 2.62-2.55 (m, 1H), 2.07-1.95 (m, 1H), 1.86-1.79 (m, 1H), 1.32 (s, 3H) | 694.4 |
| 45 | (E)-N-(2,6-difluoro-4-(8,9,11-trimethyl-6-(trifluoromethyl)-8,11,12,13-tetrahydroimidazo [4' ',5":5',6']benzo[1',2': 5,6][1,4]diazocino[7 ,8,1-hi]indole-2-carbonyl)phenyl)-4-(((1r,4r)-4-methoxycyclohexyl) amino)but-2-enamide | 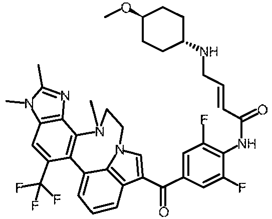 | G | | 1H-NMR (400 MHz, CHLOROFORM-D) δ 8.45 (dd, J = 8.0, 1.0 Hz, 1H), 7.70 (s, 1H), 7.49 (dd, J = 14.9, 7.9 Hz, 3H), 7.36-7.31 (m, 1H), 7.16-7.09 (m, 2H), 6.24 (d, J = 15.5 Hz, 1H), 3.98-3.91 (m, 1H), 3.83 (s, 3H), 3.53-3.49 (m, 2H), 3.41-3.37 (m, 2H), 3.36 (s, 3H), 3.19-3.11 (m, 2H), 3.09 (s, 3 H), 2.68 (s, 3H), 2.57-2.52 (m, 1H), 2.17-1.98 (m, 4H), 1.31-1.13 (m, 4H) | 735.4 |
| 50 | (E)-N-(4-(9,10-dimethyl-7-(trifluoromethyl)-13,14-dihydro-9H-imidazo[4", 5 ":5',6']b enzo[1',2':2,3]oxoci no[4,5,6-hi]indolizine-2-carbonyl)-2,6-difluorophenyl)-4-((3-(fluoromethyl)oxeta n-3-yl)amino)but-2-enamide | 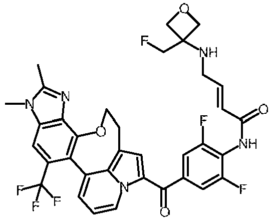 | E | | 1H-NMR (400 MHz, CHLOROFORM-D) δ 9.98 (d, J = 7.0 Hz, 1H), 7.65 (s, 1H), 7.44 (d, J = 7.9 Hz, 2H), 7.20-7.15 (m, 3H), 7.03 (t, J = 7.1 Hz, 1H), 6.95 (s, 1H), 6.34 (d, J = 15.2 Hz, 1H), 4.71 (d, J = 47.2 Hz, 2H), 4.75-4.70 (m, 1H), 4.68-4.62 (m, 2H), 4.47 (d, J = 6.9 Hz, 2H), 4.29-4.17 (m, 1H), 3.84 (s, 3H), 3.63-3.62 (m, 2H), 2.89-2.81 (m, 1H), 2.70 (s, 3H), 2.60-2.55 (m, 1H) | 698.4 |

### Synthesis of (7-(5,5-dimethyl-1,3,2-dioxaborinan-2-yl)-1H-indol-3-yl)(3,4,5-trifluorophenyl)methanone

### Step 1:

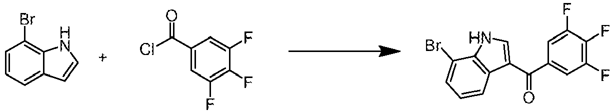

Dimethylaluminum chloride solution (1.0 M in hexane, 49 mL, 49 mmol) was added to an ice bath cooled solution of 7-bromo-1H-indole (4.80 g, 24.5 mmol) in DCM (100 mL) and stirred at ambient temperature for 30 min. 3,4,5-trifluorobenzoyl chloride (4.81 mL, 36.7 mmol) was added thereto, and the mixture was stirred at 0 °C for 20 min. 2N Hydrochloric acid was slowly added thereto and the mixture was diluted with the mixture of chloroform (80 mL) and ethanol (20 mL). The organic layer was separated and washed with aqueous NaHCO3 solution and brine, and then dried (Na₂SO₄) and evaporated. The residue was diluted with IPE and hexane, and the precipitate was collected via filtration to give (7-bromo-1H-indol-3-yl)(3,4,5-trifluorophenyl)methanone (4.54 g). The remained filtrate was evaporated and purified by column chromatography on silica gel (gradient elution, 10-50%, EtOAc-hexane) to give (7-bromo-1H-indol-3-yl)(3,4,5-trifluorophenyl)methanone (2.52 g). MS: [M+H]⁺ = 354, 356.

### Step 2:

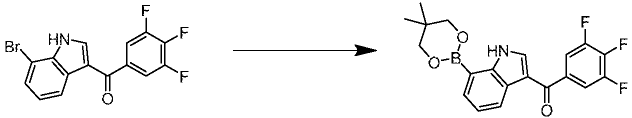

The mixture of (7-bromo-1H-indol-3-yl)(3,4,5-trifluorophenyl)methanone (7.89 g, 22.3 mmol), bis(neopentyl glycolato)diboron (10.06 g, 44.6 mmol), Pd(dppf)Cl₂-CH₂Cl₂ (910 mg, 1.11 mmol), potassium acetate (6.56 g, 66.8 mmol) in 1,4-dioxane (80 mL) was stirred at 90 °C for 12 hrs. After cooling, the reaction mixture was evaporated, and EtOAc and saturated aqueous NaHCO₃ were added thereto. The insolubles were removed by filtration, and the organic layer was separated, washed with brine, and then dried (Na₂SO₄) and evaporated. The residue was diluted with CHCl₃, and the precipitate was collected via filtration to give the title compound (4.90 g). The remained filtrate was evaporated and purified by column chromatography on silica gel (gradient elution, 10-50%, EtOAc/EtOH(4/1)-hexane) to give the title compound (3.08 g).

### Synthesis of 5-iodo-4-methoxy-1,2-dimethyl-6-(trifluoromethyl)-1H-benzo[d]imidazole

### Step 1:

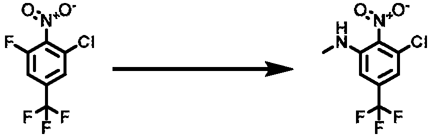

Methylamine (2M THF solution, 31.0 mL, 61.6 mmol) was added to a solution of 1-chloro-3-fluoro-2-nitro-5-(trifluoromethyl)benzene (5.00 g, 20.5 mmol) in THF (6.25 mL). After stirring at room temperature for 1 hour, the reaction mixture was concentrated under reduced pressure, thereby obtaining crude 3-chloro-N-methyl-2-nitro-5-(trifluoromethyl)aniline (5.23 g).

### Step 2:

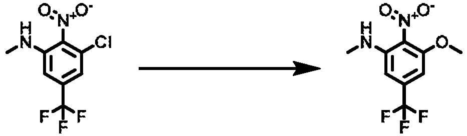

Sodium methoxide (17.4 g, 323 mmol) was added to a solution of 3-chloro-N-methyl-2-nitro-5-(trifluoromethyl)aniline (8.22 g, 32.3 mmol) in MeOH (108 mL). After stirring at 80 °C for 24 hours, the reaction mixture was diluted with EtOAc, washed with saturated aqueous NH₄Cl and brine then dried over Na₂SO₄ and evaporated. The residue was purified by column chromatography on silica gel (hexane/EtOAc), thereby obtaining 3-methoxy-N-methyl-2-nitro-5-(trifluoromethyl)aniline (7.08 g, 88%).

### Step 3:

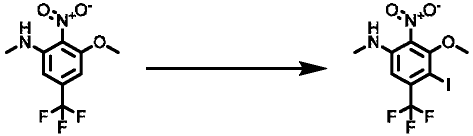

N-iodosuccinimide (7.00 g, 31.1 mmol) was added to a solution of 3-methoxy-N-methyl-2-nitro-5-(trifluoromethyl)aniline (7.08 g, 28.3 mmol) in AcOH (70.0 mL). After stirring at 40 °C for 14 hours, the mixture was cooled to room temperature, and H₂O was added to the reaction mixture. The resulting solid was collected by filtration, and vacuum-dried at 60 °C, thereby obtaining crude 4-iodo-3-methoxy-N-methyl-2-nitro-5-(trifluoromethyl)aniline (10.3 g, 97%).

### Step 4:

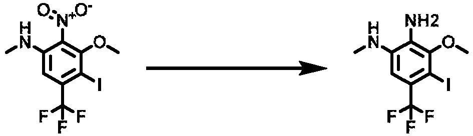

NH₄Cl (3.67 g, 68.5 mmol) and iron (7.65 g, 137 mmol) were added to a solution of 4-iodo-3-methoxy-N-methyl-2-nitro-5-(trifluoromethyl)aniline (10.3 g, 27.4 mmol) in MeOH (39.0 mL), THF (39.0 mL) and H₂O (19.5 mL). After stirring at 60 °C for 2 hours, the mixture was cooled to room temperature, and EtOAc was added to the reaction mixture then filtered through a CELITE pad. The filtrate was washed with H₂O and brine, then dried over Na₂SO₄ and evaporated. The residue was purified by column chromatography on silica gel (hexane/EtOAc), thereby obtaining 4-iodo-3-methoxy-N1-methyl-5-(trifluoromethyl)benzene-1,2-diamine (7.77 g, 82%).

### Step 5:

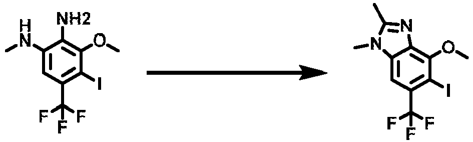

conc. HCl (187 µL, 2.25 mmol) and AcOH (1.29 mL, 22.5 mmol) were added to a solution of 4-iodo-3-methoxy-N1-methyl-5-(trifluoromethyl)benzene-1,2-diamine (7.77 g, 22.5 mmol) in trimethyl orthoacetate (40.0 mL, 314 mmol). After stirring at room temperature for 62 hours, the reaction was quenched with 5mol/L aqueous NaOH (5.45 mL, 27.3 mmol). The reaction mixture was diluted with EtOAc, washed with H₂O and brine then dried over Na₂SO₄ and evaporated. The residue was dissolved in EtOAc (8.0 mL) at 50 °C and heptane (32 mL) was added, then cooled to room temperature slowly to give the precipitaion. The precipitate was collected by filtration, washing with further heptane, and vacuum-dried at 60 °C, thereby obtaining the title compound (5.50 g, 66%). The filtrate was evaporated and purified by column chromatography on silica gel (hexane/EtOAc), thereby obtaining the title compound (1.56 g, 19%).

### Synthesis of 5-iodo-4-methoxy-1,2,6-trimethyl-1H-benzo[d]imidazole

### Step 1:

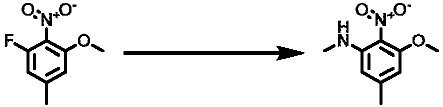

Methylamine (2M THF solution, 38.2 mL, 76.4 mmol) was added to a 1-fluoro-3-methoxy-5-methyl-2-nitrobenzene (4.04 g, 21.8 mmol). After the reaction mixture was heated at 70 °C by microwave irradiation for 12 hours, the mixture was cooled to room temperature. The mixture was diluted with EtOAc, washed with H₂O and brine then dried over Na₂SO₄ and evaporated, thereby obtaining crude 3-methoxy-N,5-dimethyl-2-nitroaniline (4.29 g, 100%).

### Step 2:

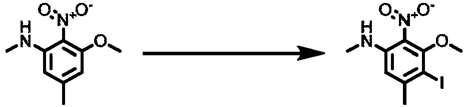

N-iodosuccinimide (6.72 g, 29.9 mmol) was added to a solution of 3-methoxy-N,5-dimethyl-2-nitroaniline (5.33 g, 27.2 mmol) in DMF (53.3 mL). After stirring at 45 °C for 2 hours, the mixture was cooled to room temperature, and H₂O and saturated aqueous NaHCO₃ were added to the mixture. The resulting solid was collected by filtration, and vacuum-dried at 40 °C, thereby obtaining crude 4-iodo-3-methoxy-N,5-dimethyl-2-nitroaniline (8.48 g, 97%).

### Step 3:

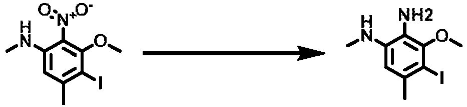

NH₄Cl (3.52 g, 65.8 mmol) and iron(7.35 g, 132 mmol) were added to a solution of 4-iodo-3-methoxy-N,5-dimethyl-2-nitroaniline (8.48 g, 26.3 mmol) in MeOH (43.8 mL), THF (43.8 mL) and H2O (21.9 mL). After stirring at 60 °C for 3 hours, the mixture was cooled to room temperature and diluted with EtOAc then filtered through a CELITE pad. The filtrate was washed with H₂O and brine then dried over Na₂SO₄ and evaporated. The residue was purified by column chromatography on silica gel (hexane/EtOAc), thereby obtaining 4-iodo-3-methoxy-N1,5-dimethylbenzene-1,2-diamine (6.68 g, 87%).

### Step 4:

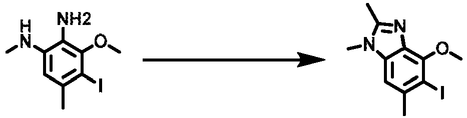

conc. HCl (95.4 µL, 1.15 mmol) and AcOH (656 µL, 11.5 mmol) were added to a solution of 4-iodo-3-methoxy-N1,5-dimethylbenzene-1,2-diamine (3.35 g, 11.5 mmol) in trimethyl orthoacetate (20.4 mL, 160 mmol). After stirring at room temperature for 14 hours, the reaction was quenched with 10mol/L aqueous NaOH (1.33 mL, 12.7 mmol). The reaction mixture was diluted with EtOAc, washed with H₂O and brine then dried over Na₂SO₄ and evaporated. The residue was purified by column chromatography on silica gel (hexane/EtOAc), thereby obtaining the title compound (2.09 g, 58%).

### Synthesis of (8-(5,5-dimethyl-1,3,2-dioxaborinan-2-yl)indolizin-3-yl)(3,4,5-trifluorophenyl)methanone

### Step 1:

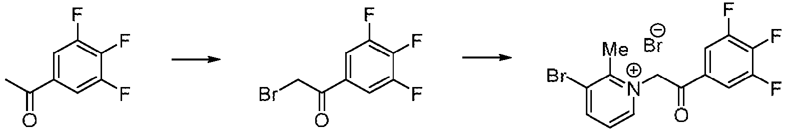

CuBr2 (4.23 g, 19.0 mmol) was added to a solution of 3',4',5'-trifluoroacetophenone (1.50 g, 8.61 mmol) in EtOAc (22.0 mL) and the reaction heated to 70 °C overnight. After cooling, the reaction was filtered through CELITE and the filtrate washed sequentially with sat. aq. NaHCO₃ (3x) and brine, then dried (MgSO₄) and evaporated to give intermediate 2-bromo-1-(3,4,5-trifluorophenyl)ethan-1-one.

3-bromo-2-methylpyridine (1.64 mL, 14.2 mmol) was added to a solution of 2-bromo-1-(3,4,5-trifluorophenyl)ethan-1-one (1.80 g, 7.11 mmol) in THF (10.0 mL) and the reaction heated to 80 °C for 4 h. Further 3-bromo-2-methylpyridine (0.820 mL, 7.11 mmol) was added and heating continued overnight. After cooling, heptane (15 mL) was added and the resultant precipitate collected via filtration, washing with further heptane, and dried in vacuo to give 3-bromo-2-methyl-1-[2-oxo-2-(3,4,5-trifluorophenyl)ethyl]pyridin-1-ium bromide. MS: [M]+ = 344.

### Step 2:

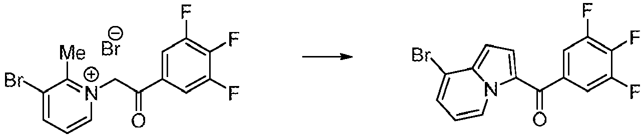

A mixture of DMF (4.08 mL, 52.7 mmol) and dimethyl sulfate (4.99 mL, 52.7 mmol) was heated to 80 °C for 2 h. After cooling, this solution was added to a separate solution of 3-bromo-2-methyl-1-[2-oxo-2-(3,4,5-trifluorophenyl)ethyl]pyridin-1-ium bromide (2.80 g, 6.59 mmol) in DMF (10 mL) and the reaction stirred at RT for 30 min. N,N-diisopropylethylamine (9.18 mL, 52.7 mmol) was added and the resulting suspension allowed to stand for 30 min. H₂O (~50 mL) was added and, after standing for a further 30 min, the precipitate was collected via filtration, washing with further water, and the solid dried in vacuo. This solid was re-crystallised from boiling EtOAc (~25 mL) to give (8-bromoindolizin-3-yl)(3,4,5-trifluorophenyl)methanone. MS: [M+H]+ = 354.

### Step 3:

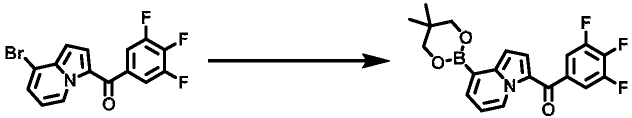

A solution of (8-bromoindolizin-3-yl)(3,4,5-trifluorophenyl)methanone (82.0 g, 232 mmol), 2-(5,5-dimethyl-1,3,2-dioxaborinan-2-yl)-5,5-dimethyl-1,3,2-dioxaborinane (63.0 g, 279 mmol), [1,1'-bis(diphenylphosphino)ferrocene]dichloropalladium(II) dichloromethane Adduct (4.70 g, 5.80 mmol), potassium acetate (39.0 g, 395 mmol) in THF (750 mL) was evacuated and N₂ back-filled before heating to reflux temperature (oil bath: 93 °C) for 1.5 hours. After cooling, the reaction mixture was filtered, rinsing with EtOAc, and the filtrate was washed with brine. The resulting suspension was filtered, rinsing with EtOAc, and the filtrate was washed with aqueous NaHCO₃ and brine then dried over Na₂SO₄ and evaporated. The residue was suspended in EtOAc (100 mL), stirred at room temperature for 3 hours. The solid was collected by filtration, washing with further hexane/EtOAc=3/1 (100 mL), and vacuum-dried, thereby obtaining the title compound (50.9 g, 57%). The filtrate was evaporated and purified by column chromatography on silica gel (hexane/EtOAc). The solid was suspended in EtOAc (7.0 mL) and hexane (14 mL) was slowly added. The suspension was stirred at room temperature for 2 hours and collected by filtration, washing with further hexane/EtOAc=5/1, and vacuum-dried, thereby obtaining the title compound (4.50 g, 5%).

### Synthesis of (4-amino-3,5-difluorophenyl)(8-(5,5-dimethyl-1,3,2-dioxaborinan-2-yl)indolizin-3-yl)methanone

### Step 1:

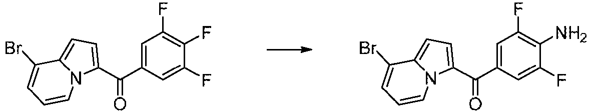

A 5 L pressure vessel was charged with (8-bromoindolizin-3-yl)(3,4,5-trifluorophenyl)methanone (208 g, 0.59 mol), THF (2 L), and 28% NH3 aq. (830 ml), and heated at 135 °C with stirring for 24 hr. The mixture was cooled to rt, poured into ice-water (1 L), and stirred for 2 hr. The precipitate was collected by filtration and rinsed with water (1 L) to give a crude solid. The solid was added EtOAc (1 L) and heated at 65 °C. To the mixture was slowly added hexane (2 L) then cooled down to rt. After stirring at rt for 20 hr, the solid was collected by filtration, rinsed with hexane/EtOAc (3/1), dried in vacuo to give 4-(8-Bromoindolizine-3-carbonyl)-2,6-difluoroaniline. MS: [M+H]+ = 351.

### Step 2:

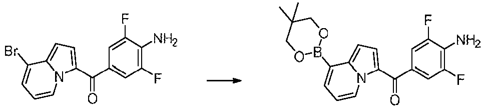

A 10 L 4-necked flask was charged with (4-amino-3,5-difluorophenyl)(8-bromoindolizin-3-yl)methanone (396 g, 1.13 mol), dry THF (3.8 L), and KOAc (188 g, 1.92 mol, 1.7 eq). The mixture was de-gassed (nitrogen gas was passed through the mixture at 50 °C for 40 min) and the mixture was added PdCl₂(dppf)-CH₂Cl₂ (28.6 g, 0.035 mol) and bis(neopentyl Glycolato) diboron (331 g, 1.47 mol). The resulting mixture was stirred at 70 °C for 10 hr. After cooled down to rt, the mixture was poured into ice-water (4 L), added EtOAc (4 L), and filtered (CELITE). The filtrate was separated and the aq. phase was re-extracted with EtOAc (2 L). Organic phases were combined and washed with water (3.5 L), sat NaCl aq. (3.5 L), dried (Na₂SO₄), filtered, and concentrated. The resulting crude material was purified by column chromatography (SiO₂ 10 kg, CH₂Cl₂/EtOAc= 10/1 to 3/1) and the resulting solid was further purified by washing with Hexane (500 ml) to give the title compound.

### Synthetic procedure F

### Example 130, 130-1 and 130-2

### (E)-N-(4-(9,13-dimethyl-7-(trifluoromethyl)-13,14-dihydro-9H-imidazo[4",5":5',6']benzo[1',2':2,3]oxocino[4,5,6-hi]indolizine-2-carbonyl)-2,6-difluorophenyl)-4-((3-methyloxetan-3-yl)amino)but-2-enamide

### Step 1:

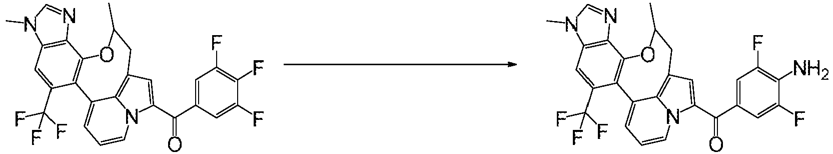

To a mixture of (9,13-dimethyl-7-(trifluoromethyl)-13,14-dihydro-9*H-*imidazo[4",5":5',6']benzo[1',2':2,3]oxocino[4,5,6-*hi*]indolizin-2-yl)(3,4,5-trifluorophenyl)methanone (129 mg, 0.244 mmol), obtained in the same manner as Example 42, in 1,4-dioxane (2.40 mL) was added 28-30% NH₃ aq. (8.20 mL). The reaction mixture was heated at 130 °C by microwave irradiation for 15 hours. After cooling, the mixture was diluted with EtOAc, washed with brine, dried over Na₂SO₄ and evaporated. The residue was purified by column chromatography on silica gel (hexane/acetone) to obtain (4-amino-3,5-difluorophenyl)(9,13-dimethyl-7-(trifluoromethyl)-13,14-dihydro-9*H*-imidazo[4",5":5',6']benzo[1',2':2,3]oxocino[4,5,6-*hi*]indolizin-2-yl)methanone (1^{st}-eluting diastereomer, 2^{nd}-eluting diastereomer).

### Step 2:

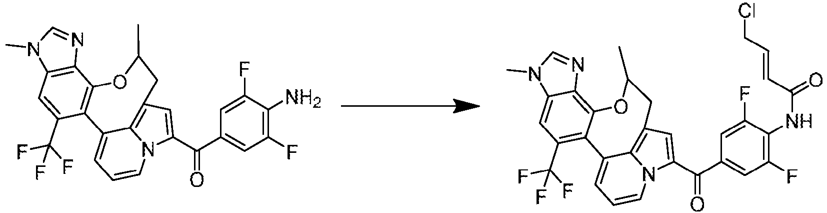

A solution of (4-amino-3,5-difluorophenyl)(9,13-dimethyl-7-(trifluoromethyl)-13,14-dihydro-9*H*-imidazo[4",5":5',6']benzo[1',2':2,3]oxocino[4,5,6-*hi*]indolizin-2-yl)methanone (2^{nd}-eluting diastereomer; 105 mg, 0.199 mmol), 4-chlorocrotonic acid (30.1 mg, 0.249 mmol) and propylphosphonic anhydride solution (50 wt% in THF, 0.312 mL, 0.499 mmol) in DMF (1.99 mL) was stirred at room temperature for 20 minutes. Then triethylamine (0.111 mL, 0.798 mmol) was added thereto, and the mixture was stirred at room temperature for 3 hours. 4-Chlorocrotonic acid (15.0 mg, 0.124 mmol) and propylphosphonic anhydride solution (50 wt% in THF, 0.156 mL, 0.250 mmol) were added and the reaction mixture was stirred at room temperature for 15 minutes. Then triethylamine (55.5 µL, 0.399 mmol) was added thereto, and the mixture was stirred at room temperature for 30 minutes. To the resulting mixture was added saturated aqueous NaHCO₃ and water, and the resulting mixture was extracted with EtOAc. The organic layer was washed successively with H₂O, saturated aqueous NaHCO₃, brine, dried over Na₂SO₄, and evaporated. The residue was purified by column chromatography on silica gel (hexane/acetone) to obtain (*E*)-4-chloro-*N*-(4-(9,13-dimethyl-7-(trifluoromethyl)-13,14-dihydro-9*H*-imidazo[4",5":5',6']benzo[1',2':2,3]oxocino[4,5,6-*hi*]indolizine-2-carbonyl)-2,6-difluorophenyl)but-2-enamide.

### Step 3:

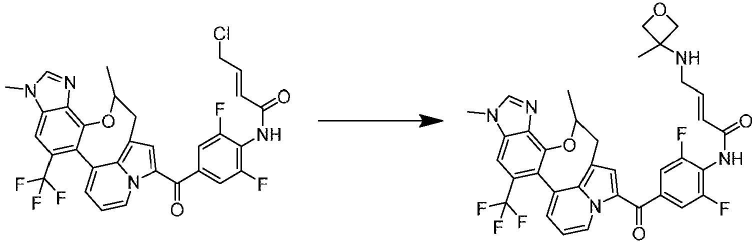

A mixture of (*E*)-4-chloro-*N*-(4-(9,13-dimethyl-7-(trifluoromethyl)-13,14-dihydro-9*H*-imidazo[4",5":5',6']benzo[1',2':2,3]oxocino[4,5,6-*hi*]indolizine-2-carbonyl)-2,6-difluorophenyl)but-2-enamide (125 mg, 0.199 mmol), KI (99.0 mg, 0.596 mmol) and 3-methyloxetan-3-amine (88.3 µL, 0.994 mmol) in DMSO (2.00 mL) was stirred at 40 ~ 50 °C for 4.5 hours. The mixture was diluted with water and extracted with EtOAc. The organic layer was washed with water, brine, dried over Na₂SO₄, and evaporated. The residue was purified by column chromatography on silica gel (CHCl₃/methanol) to obtain (*E*)-*N*-(4-(9,13-dimethyl-7-(trifluoromethyl)-13,14-dihydro-9*H-*imidazo[4",5":5',6']benzo[1',2':2,3]oxocino[4,5,6-*hi*]indolizine-2-carbonyl)-2,6-difluorophenyl)-4-((3-methyloxetan-3-yl)amino)but-2-enamide as Example 130.

### Step 4:

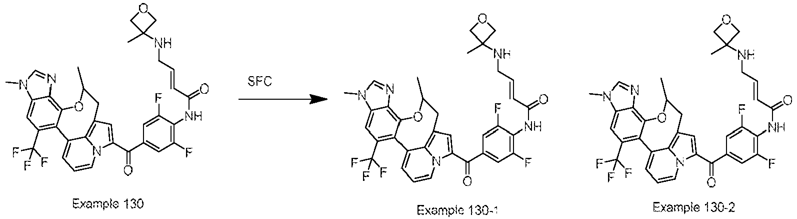

The racemate was separated by chiral SFC separation (Column: Daicel Chiralcel OD, 250 mm x 30 mm, 0.1% NH₄OH in EtOH) to give the title compound (1st-eluting isomer, Example 130-1) and (2nd-eluting isomer, Example 130-2). ¹H NMR (400 MHz, CDCl₃) δ 10.02 (dd, *J =* 7.00, 0.96 Hz, 1H), 8.04 (s, 1H), 7.76 (s, 1H), 7.43 (d, *J =* 7.83 Hz, 2H), 7.20-7.12 (m, 4H), 7.04 (t, *J=* 7.10 Hz, 1H), 6.34 (dt, *J=* 15.28, 1.68 Hz, 1H), 4.98 (m, 1H), 4.58 (d, *J =* 6.26 Hz, 2H), 4.43 (d, *J=* 6.50 Hz, 2H), 3.97 (s, 3H), 3.54-3.52 (m, 2H), 3.01-2.96 (m, 1H), 2.35 (d, *J=* 15.01 Hz, 1H), 1.52 (s, 3H), 1.23 (d, *J* = 6.18 Hz, 3H); ESI-MS [M+H]⁺: 680.

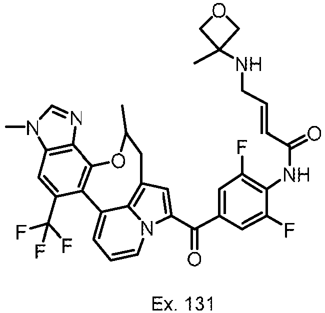

### Example 131

### (E)-N-(4-(9,13-dimethyl-7-(trifluoromethyl)-13,14-dihydro-9H-imidazo[4",5":5',6']benzo[1',2':2,3]oxocino[4,5,6-hi]indolizine-2-carbonyl)-2,6-difluorophenyl)-4-((3-methyloxetan-3-yl)amino)but-2-enamide

Example 131 was attained in the same manner as Example 130, using 1st-eluting diastereomer in Step 1, to yield the racemic mixture. ¹H NMR (400 MHz, CDCl₃) δ 9.96 (dd, *J=* 6.84, 1.12 Hz, 1H), 8.00 (s, 1H), 7.76 (s, 1H), 7.45 (d, *J =* 7.84 Hz, 2H), 7.17-7.11 (m, 2H), 7.06-6.95 (m, 3H), 6.33 (d, *J =* 15.32 Hz, 1H), 4.60 (d, *J =* 6.36 Hz, 2H), 4.59-4.51 (m, 1H), 4.44 (d, *J* = 6.49 Hz, 2H), 3.95 (s, 3H), 3.56-3.54 (m, 2H), 2.88-2.82 (m, 1H), 2.58-2.54 (m, 1H), 1.53 (s, 3H), 1.50 (d, *J=* 6.61 Hz, 3H); ESI-MS [M+H]⁺: 680.

### Synthetic procedure L

### Example 71

### (E)-N-(2,6-difluoro-4-(9,10,12-trimethyl-7-(trifluoromethyl)-9,12,13,14-tetrahydroimidazo[4",5":5',6']benzo[1',2':2,3]azocino[4,5,6-hi]indolizine-2-carbonyl)phenyl)-4-((3-methyloxetan-3-yl)amino)but-2-enamide

### Step 1:

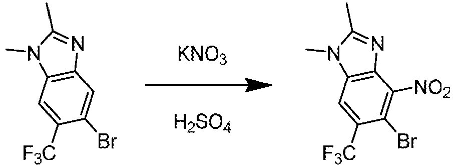

To a mixture of 5-bromo-1,2-dimethyl-6-(trifluoromethyl)-1*H*-benzo[*d*]imidazole (4.39 g, 15 mmol) in H₂SO₄ (15 mL) was added KNO₃ (1.80 g, 17.8 mmol) at 0 °C. The mixture was warmed to room temperature and stirred for 2 hours. The mixture was carefully poured into crushed ice and 30% NH₃ aq. was added until the mixture was neutralized. The resulting precipitate was collected by filtration, washed with water, and dried in vacuo at 50 °C to obtain 5-bromo-1,2-dimethyl-4-nitro-6-(trifluoromethyl)-1*H-*benzo[*d*]imidazole.

### Step 2:

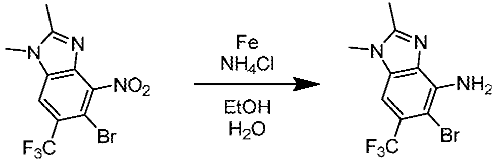

A solution of 5-bromo-1,2-dimethyl-4-nitro-6-(trifluoromethyl)-1*H-*benzo[*d*]imidazole (5.10 g, 15.0 mmol), iron powder (4.18 g, 74.8 mmol) and NH₄Cl (4.00 g, 74.8 mmol) in EtOH (100 mL) and H₂O (20 mL) was stirred at 70 °C for 30 minutes. The mixture was diluted with EtOAc (500 mL), and the mixture was filtered and concentrated. Water and CHCl₃ were added to the filtrate, and the organic layer was separated, washed with brine, dried over Na₂SO₄, and evaporated. The residue was diluted with diisopropyl ether, and the precipitate was collected by filtration to obtain 5-bromo-1,2-dimethyl-6-(trifluoromethyl)-1*H*-benzo[*d*]imidazol-4-amine.

### Step 3:

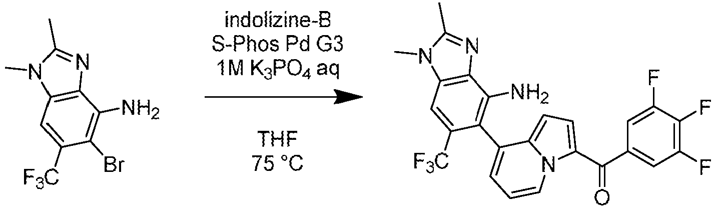

A solution of 5-bromo-1,2-dimethyl-6-(trifluoromethyl)-1*H*-benzo[*d*]imidazol-4-amine (1.23 g, 3.99 mmol), (8-(5,5-dimethyl-1,3,2-dioxaborinan-2-yl)indolizin-3-yl)(3,4,5-trifluorophenyl)methanone (2.32 g, 5.99 mmol), SPhos Pd G3 (281 mg, 0.399 mmol) in THF (40 mL) and 1 M K₃PO₄ aq. (5.99 mL, 5.99 mmol) was evacuated and N₂ back-filled before heating to 75 °C for 1.5 hours. After cooling, the reaction mixture was diluted with EtOAc. The mixture was filtered, rinsing with EtOAc, and the filtrate was washed with H₂O, brine, dried over Na₂SO₄, and evaporated. The residue was purified by column chromatography on silica gel (hexane-EtOAc/EtOH(4/1)). The residue was washed with diisopropyl ether, and the precipitate was collected by filtration to obtain (8-(4-amino-1,2-dimethyl-6-(trifluoromethyl)-1*H*-benzo[*d*]imidazol-5-yl)indolizin-3-yl)(3,4,5-trifluorophenyl)methanone.

### Step 4:

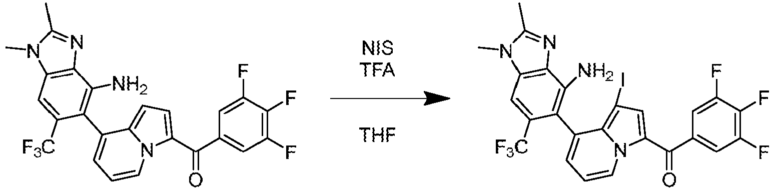

NIS (339 mg, 1.50 mmol) and TFA (0.25 mL, 3.37 mmol) were added to a solution of (8-(4-amino-1,2-dimethyl-6-(trifluoromethyl)-1*H*-benzo[*d*]imidazol-5-yl)indolizin-3-yl)(3,4,5-trifluorophenyl)methanone (720 mg, 1.43 mmol) in THF (10 mL). After stirring at room temperature for 70 minutes, additional NIS (50 mg, 0.222 mmol) was added. After stirring at room temperature for 2 hours, the mixture was diluted with EtOAc, sat. NaHCO₃ aq., and sat. Na₂S₂O₃ aq.. The organic layer was separated, washed with brine, dried over Na₂SO₄, and evaporated. The residue was triturated with diisopropyl ether and the precipitate was collected by filtration to obtain (8-(4-amino-1,2-dimethyl-6-(trifluoromethyl)-1*H*-benzo[*d*]imidazol-5-yl)-1-iodoindolizin-3-yl)(3,4,5-trifluorophenyl)methanone.

### Step 5:

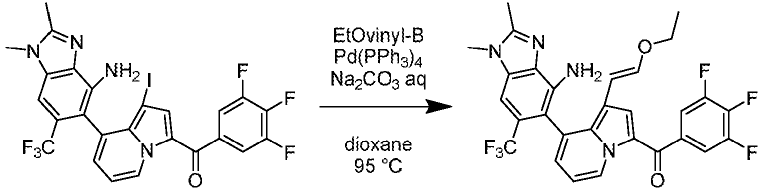

A mixture of (8-(4-amino-1,2-dimethyl-6-(trifluoromethyl)-1*H*-benzo[*d*]imidazol-5-yl)-1-iodoindolizin-3-yl)(3,4,5-trifluorophenyl)methanone (467 mg, 0.743 mmol), 2-[(*E*)-2-ethoxyvinyl]-4,4,5,5-tetramethyl-1,3,2-dioxaborolane (0.787 mL, 3.72 mmol) and Pd(PPh₃)₄ (85.9 mg, 0.0743 mmol) in 1,4-dioxane (7.0 mL) and 2 M Na₂CO₃ aq. (1.86 mL, 3.72 mmol) was evacuated and N₂ back-filled before heating to 95 °C for 4 hours. After cooling, the reaction mixture was diluted with EtOAc, washed with water and brine, dried over Na₂SO₄, and evaporated. The residue was triturated with diisopropyl ether and the precipitate were collected by filtration. The precipitate was purified by column chromatography on silica gel (hexane-EtOAc/EtOH(4/1)) to obtain (*E*)-(8-(4-amino-1,2-dimethyl-6-(trifluoromethyl)-1*H*-benzo[*d*]imidazol-5-yl)-1-(2-ethoxyvinyl)indolizin-3-yl)(3,4,5-trifluorophenyl)methanone.

### Step 6:

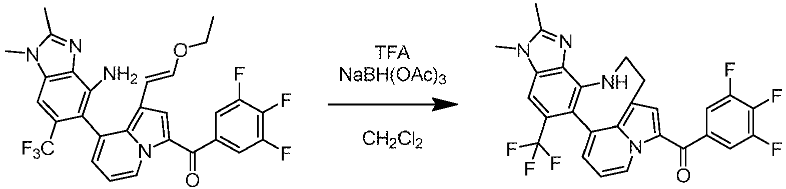

To a solution of (*E*)-(8-(4-amino-1,2-dimethyl-6-(trifluoromethyl)-1*H-*benzo[d]imidazol-5-yl)-1-(2-ethoxyvinyl)indolizin-3-yl)(3,4,5-trifluorophenyl)methanone (806 mg, 1.41 mmol) in CH₂Cl₂ (20.3 mL) was added sodium triacetoxyborohydride (597 mg, 2.82 mmol) and TFA (2.09 mL, 28.2 mmol) at room temperature. After stirring at room temperature for 3 minutes, the reaction was quenched with sat. NaHCO₃ aq. and 5 M NaOH aq., and the mixture was diluted with EtOAc. The organic layer was washed with brine, dried over Na₂SO₄, and evaporated. The residue was purified by column chromatography on silica gel (hexane-EtOAc/EtOH(4/1)) to obtain (9,10-dimethyl-7-(trifluoromethyl)-9,12,13,14-tetrahydroimidazo[4",5":5',6']benzo[1',2':2,3]azocino[4,5,6-*hi*]indolizin-2-yl)(3,4,5-trifluorophenyl)methanone.

### Step 7:

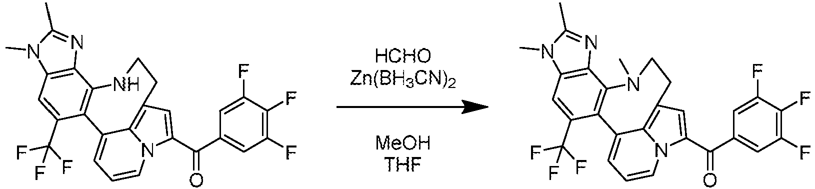

A mixture of (9,10-dimethyl-7-(trifluoromethyl)-9,12,13,14-tetrahydroimidazo[4",5":5',6']benzo[1',2':2,3]azocino[4,5,6-*hi*]indolizin-2-yl)(3,4,5-trifluorophenyl)methanone (364 mg, 0.689 mmol), 37% aqueous formaldehyde solution (0.104 mL, 1.38 mmol) and 0.3 M Zn(BH₃CN)₂ in MeOH (3.44 mL, 1.03 mmol; prepared from NaBH₃CN (1.03 mmol) and ZnCl₂ (0.515 mmol)) in THF (4.0 mL) was stirred at room temperature for 45 minutes. EtOAc and 30% ammonium solution were added. The layers were separated, and the organic layer was washed with brine, dried over Na₂SO₄, and evaporated. The residue was purified by column chromatography on silica gel (hexane-EtOAc/EtOH(4/1)) to obtain (3,4,5-trifluorophenyl)(9,10,12-trimethyl-7-(trifluoromethyl)-9,12,13,14-tetrahydroimidazo[4",5":5',6']benzo[1',2':2,3]azocino[4,5,6-*hi*]indolizin-2-yl)methanone.

### Step 8:

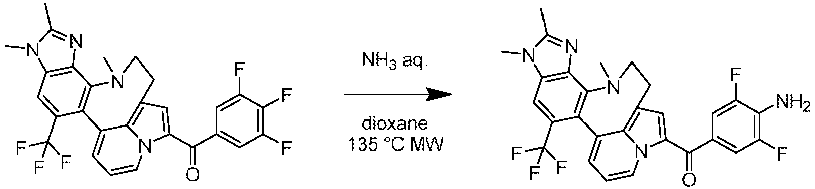

To the mixture of (3,4,5-trifluorophenyl)(9,10,12-trimethyl-7-(trifluoromethyl)-9,12,13,14-tetrahydroimidazo[4",5":5',6']benzo[1',2':2,3]azocino[4,5,6-*hi*]indolizin-2-yl)methanone (205 mg, 0.378 mmol) in 1,4-dioxane (1.5 mL) was added 28-30% NH₃ aq. (3.0 mL). The reaction mixture was heated to 135 °C by microwave irradiation for 12 hours. After cooling, the mixture was diluted with EtOAc, washed with brine, dried over Na₂SO₄, and evaporated. The residue was purified by column chromatography on silica gel (hexane-EtOAc/EtOH(4/1)) to obtain (4-amino-3,5-difluorophenyl)(9,10,12-trimethyl-7-(trifluoromethyl)-9,12,13,14-tetrahydroimidazo[4",5":5"6']benzo[1',2':2,3]azocino[4,5,6-*hi*]indolizin-2-yl)methanone.

### Step 9:

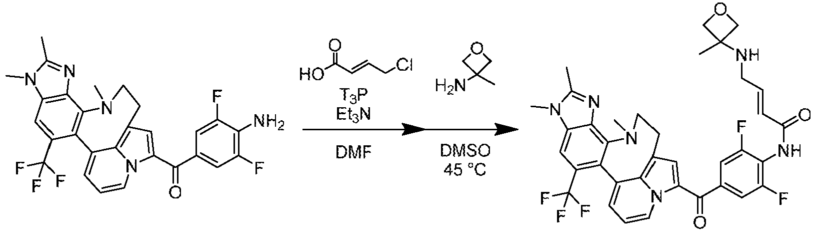

A solution of (4-amino-3,5-difluorophenyl)(9,10,12-trimethyl-7-(trifluoromethyl)-9,12,13,14-tetrahydroimidazo[4",5":5',6']benzo[1',2':2,3]azocino[4,5,6-*hi*]indolizin-2-yl)methanone (176 mg, 0.326 mmol) and triethylamine (1.28 mL, 9.38 mmol) in DMF (7.1 mL) was slowly added 0.4 mL of 4-chlorocrotonic acid T3P solution (prepared from (*E*)-4-chlorobut-2-enoic and T3P (50 wt% in THF) (1 mL/100 mg (4-chlorocrotonic acid))). Additional 4-chlorocrotonic acid T3P solution (0.3 mL) was added thereto. The resulting mixture was diluted with EtOAc, washed with NaHCO₃ aq., water, brine, dried over Na₂SO₄, and evaporated. To the residue was added DMSO (2 mL) and 3-methyloxetan-3-amine (140 µL), and the mixture was stirred at 45 °C for 3.5 hours. The mixture was purified by preparative reversed-phase HPLC (water:acetonitrile (0.1% formic acid)). Saturated NaHCO₃ aq. was added to the purified fractions containing the title compound, and the mixture was extracted with EtOAc. The combined organic layers were washed with brine, dried over Na₂SO₄, and the solvent was evaporated under reduced pressure to obtain (*E*)-*N*-(2,6-difluoro-4-(9,10,12-trimethyl-7-(trifluoromethyl)-9,12,13,14-tetrahydroimidazo[4",5":5',6']benzo[1',2':2,3]azocino[4,5,6-*hi*]indolizine-2-carbonyl)phenyl)-4-((3-methyloxetan-3-yl)amino)but-2-enamide.

The racemate was separated by CHIRALPAK-IA (hexane-EtOH(0.1% Et₃N):75/25), and the 2^{nd} elution was the title compound . ¹H NMR (400 MHz, CDCl₃) δ 9.97 (d, *J* = 6.8 Hz, 1H), 7.67 (s, 1H), 7.50 (s, 1H), 7.39 (d, *J* = 8.5 Hz, 2H), 7.16-7.06 (m, 3H), 7.00 (t, *J =* 6.8 Hz, 1H), 6.36 (d, *J=* 15.3 Hz, 1H), 4.59 (d, *J =* 6.0 Hz, 2H), 4.44 (d, *J=* 5.8 Hz, 2H), 3.82 (s, 3H), 3.53 (s, 2H), 3.44 (d, *J* = 9.5 Hz, 1H), 3.09 (s, 3H), 2.89-2.83 (m, 1H), 2.68 (s, 3H), 2.54 (d, *J =* 14.5 Hz, 1H), 2.27 (t, *J=* 13.4 Hz, 1H), 1.52 (s, 3H); ESI-MS [M+H]⁺: 693.

### Example 92

### (E)-N-(2,6-difluoro-4-(10-(methoxymethyl)-9,12-dimethyl-7-(trifluoromethyl)-9,12,13,14-tetrahydroimidazo[4",5":5',6']benzo[1',2':2,3]azocino[4,5,6-hi]indolizine-2-carbonyl)phenyl)-4-((3-methyloxetan-3-yl)amino)but-2-enamide

### Step 1:

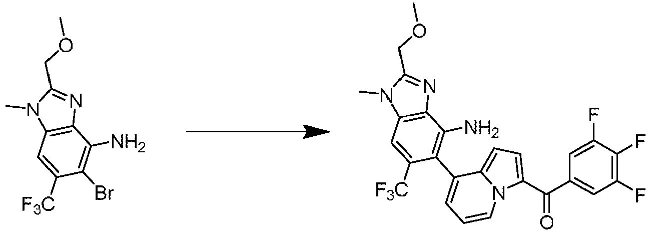

5-bromo-2-(methoxymethyl)-1-methyl-6-(trifluoromethyl)-1*H*-benzo[*d*]imidazol-4-amine (500 mg, 1.48 mmol), (8-(5,5-dimethyl-1,3,2-dioxaborinan-2-yl)indolizin-3-yl)(3,4,5-trifluorophenyl)methanone (1.15 g, 2.96 mmol), SPhos Pd G3 (281 mg, 0.148 mmol) in THF (15 mL) and 2 M K₃PO₄ aq. (1.85 mL, 3.70 mmol) was evacuated and N₂ back-filled before heating to 80 °C overnight. After cooling, the reaction mixture was diluted with EtOAc. The mixture was filtered, rinsing with EtOAc, and the filtrate was washed with water, brine, dried over Na₂SO₄, and evaporated. The residue was purified by column chromatography on silica gel twice (hexane-EtOAc/EtOH(4/1), then hexane-acetone). The residue was triturated with diisopropyl ether to obtain (8-(4-amino-2-(methoxymethyl)-1-methyl-6-(trifluoromethyl)-1*H*-benzo[*d*]imidazol-5-yl)indolizin-3-yl)(3,4,5-trifluorophenyl)methanone.

### Step 2:

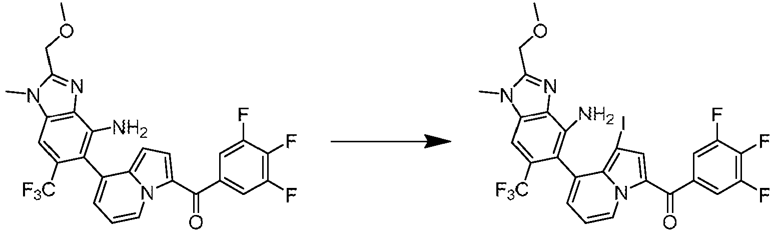

NIS (254 mg, 1.13 mmol) and TFA (0.210 mL, 2.26 mmol) were added to (8-(4-amino-2-(methoxymethyl)-1-methyl-6-(trifluoromethyl)-1*H*-benzo[*d*]imidazol-5-yl)indolizin-3-yl)(3,4,5-trifluorophenyl)methanone (651 mg, 1.13 mmol) in THF (5.5 mL). After stirring at room temperature for 4.5 hours, the mixture was diluted with EtOAc, sat. NaHCO₃ aq., and sat. Na₂S₂O₃ aq.. The organic layer was separated, washed with brine, dried over Na₂SO₄, and evaporated. The residue was purified by column chromatography on silica gel (hexane-acetone). The residue was triturated with diisopropyl ether and the precipitate was collected by filtration to obtain (8-(4-amino-2-(methoxymethyl)-1-methyl-6-(trifluoromethyl)-1H-benzo[d]imidazol-5-yl)-1-iodoindolizin-3-yl)(3,4,5-trifluorophenyl)methanone.

### Step 3:

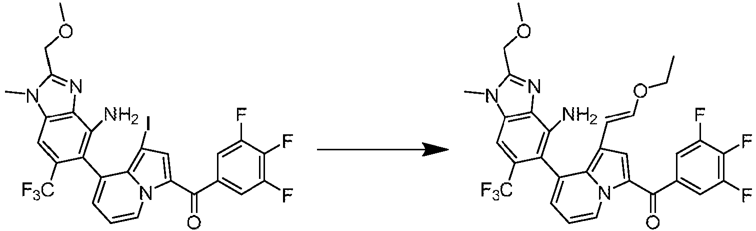

A mixture of (8-(4-amino-2-(methoxymethyl)-1-methyl-6-(trifluoromethyl)-1H-benzo[*d*]imidazol-5-yl)-1-iodoindolizin-3-yl)(3,4,5-trifluorophenyl)methanone (599 mg, 0.910 mmol), 2-[(E)-2-ethoxyvinyl]-4,4,5,5-tetramethyl-1,3,2-dioxaborolane (0.964 mL, 4.55 mmol), Pd(PPh₃)₄ (105 mg, 0.0910 mmol) in 1,4-dioxane (8.0 mL) and 2 M Na₂CO₃ aq. (2.05 mL, 3.72 mmol) was evacuated and N₂ back-filled before heating to 100 °C for 10 hours. After cooling, the reaction mixture was diluted with EtOAc, washed with water and brine, then dried over Na₂SO₄ and evaporated. The residue was purified by column chromatography on silica gel (hexane-acetone) to obtain (*E*)-(8-(4-amino-2-(methoxymethyl)-1-methyl-6-(trifluoromethyl)-1*H*-benzo[*d*]imidazol-5-yl)-1-(2-ethoxyvinyl)indolizin-3-yl)(3,4,5-trifluorophenyl)methanone.

### Step 4:

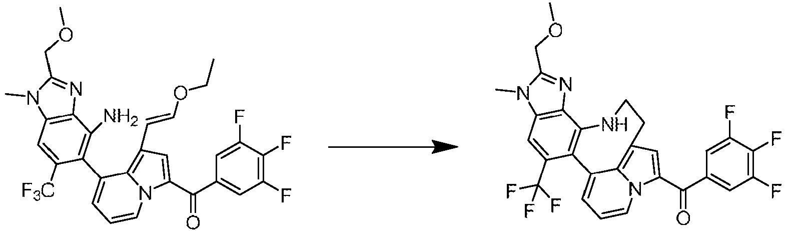

To a solution of (*E*)-(8-(4-amino-2-(methoxymethyl)-1-methyl-6-(trifluoromethyl)-1*H*-benzo[*d*]imidazol-5-yl)-1-(2-ethoxyvinyl)indolizin-3-yl)(3,4,5-trifluorophenyl)methanone (550 mg, 0.913 mmol) in CH₂Cl₂ (20.3 mL) was added sodium triacetoxyborohydride (580 mg, 2.74 mmol) and TFA (2.03 mL, 27.4 mmol) at room temperature. After stirring at room temperature for 3 minutes, the reaction was diluted with sat. NaHCO₃ aq. and 5 M NaOH aq., then the resulting mixture was diluted with EtOAc. The organic layer was washed with brine, dried over Na₂SO₄, and evaporated. The residue was purified by column chromatography on silica gel (hexane-EtOAc/EtOH(4/1)) to obtain (10-(methoxymethyl)-9-methyl-7-(trifluoromethyl)-9,12,13,14-tetrahydroimidazo[4",5":5',6']benzo[1',2':2,3]azocino[4,5,6-*hi*]indolizin-2-yl)(3,4,5-trifluorophenyl)methanone.

### Step 5:

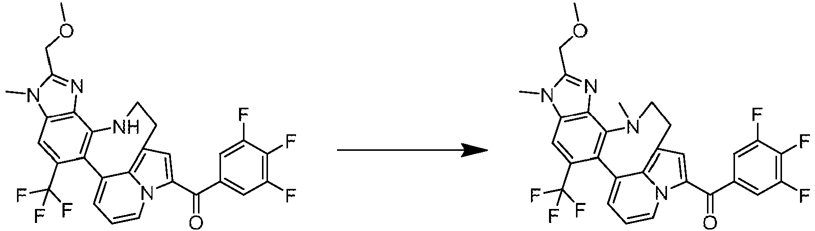

A mixture of (10-(methoxymethyl)-9-methyl-7-(trifluoromethyl)-9,12,13,14-tetrahydroimidazo[4",5":5',6']benzo[1',2':2,3]azocino[4,5,6-*hi*]indolizin-2-yl)(3,4,5-trifluorophenyl)methanone (510 mg, 0.913 mmol), aqueous 37% formaldehyde solution (0.228 mL, 2.74 mmol) and 0.3 M zinc cyanoborohydride solution in methanol (6.09 mL, 1.83 mmol; prepared from NaBH₃CN and 1/2 ZnCl₂) in THF (7.0 mL) was stirred at room temperature for 1 hour. The mixture was diluted with EtOAc and sat. NaHCO₃ aq., and the organic layer was washed with brine, dried over Na₂SO₄, and evaporated. The residue was purified by column chromatography on silica gel (hexane-EtOAc/EtOH(4/1)) to obtain (10-(methoxymethyl)-9,12-dimethyl-7-(trifluoromethyl)-9,12,13,14-tetrahydroimidazo[4",5":5',6']benzo[1',2':2,3]azocino[4,5,6-*hi*]indolizin-2-yl)(3,4,5-trifluorophenyl)methanone.

### Step 6:

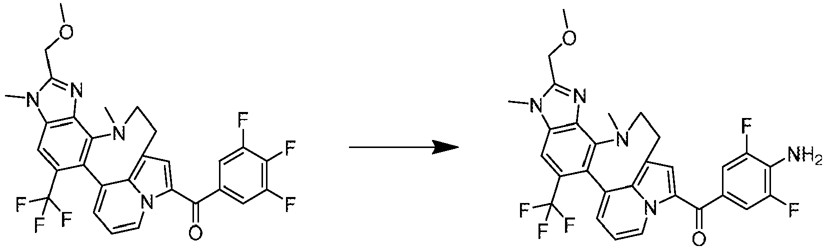

To a solution of (10-(methoxymethyl)-9,12-dimethyl-7-(trifluoromethyl)-9,12,13,14-tetrahydroimidazo[4",5":5',6']benzo[1',2':2,3]azocino[4,5,6-*hi*]indolizin-2-yl)(3,4,5-trifluorophenyl)methanone (450 mg, 0.786 mmol) in 1,4-dioxane (5.0 mL) was added 28-30% NH₃ aq. (10 mL). The reaction mixture was heated at 130 °C under microwave irradiation for 12 hours. After cooling, the mixture was diluted with EtOAc, washed with brine, dried over Na₂SO₄, and evaporated. The residue was purified by column chromatography on silica gel (hexane-EtOAc/EtOH(4/1)) to obtain (4-amino-3,5-difluorophenyl)(10-(methoxymethyl)-9,12-dimethyl-7-(trifluoromethyl)-9,12,13,14-tetrahydroimidazo[4",5":5',6']benzo[1',2':2,3]azocino[4,5,6-*hi*]indolizin-2-yl)methanone.

### Step 7:

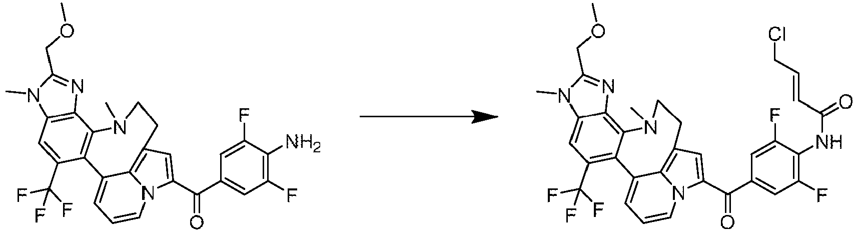

A solution of (4-amino-3,5-difluorophenyl)(10-(methoxymethyl)-9,12-dimethyl-7-(trifluoromethyl)-9,12,13,14-tetrahydroimidazo[4",5":5',6']benzo[1',2':2,3]azocino[4,5,6-*hi*]indolizin-2-yl)methanone (408 mg, 0.716 mmol) and triethylamine (2.00 mL, 1.43 mmol) in DMF (4.0 mL) was slowly added 0.895 mL of 4-chlorocrotonic acid T3P solution (prepared from (E)-4-chlorobut-2-enoic and T3P (50 wt% in THF) (100 mg 4-chlorocrotonic acid / mL)). Additional 4-chlorocrotonic acid T3P solution (0.895 mL) was added. The resulting mixture was diluted with EtOAc, washed with sat. NaHCO₃ aq., water, brine, dried over Na₂SO₄, and evaporated. The residue was purified by column chromatography on silica gel (hexane-EtOAc/EtOH(4/1)) to obtain (*E*)-4-chloro-*N*-(2,6-difluoro-4-(10-(methoxymethyl)-9,12-dimethyl-7-(trifluoromethyl)-9,12,13,14-tetrahydroimidazo[4",5":5',6']benzo[1',2':2,3]azocino[4,5,6-*hi*]indolizine-2-carbonyl)phenyl)but-2-enamide.

### Step 8:

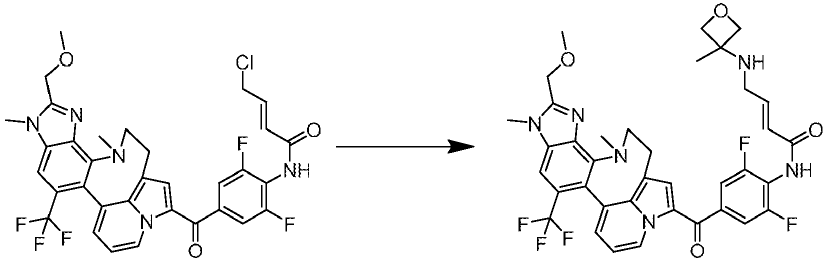

To a solution of (*E*)-4-chloro-*N*-(2,6-difluoro-4-(10-(methoxymethyl)-9,12-dimethyl-7-(trifluoromethyl)-9,12,13,14-tetrahydroimidazo[4",5":5',6']benzo[1',2':2,3]azocino[4,5,6-*hi*]indolizine-2-carbonyl)phenyl)but-2-enamide (150 mg, 0.223 mmol) in DMSO (0.50 mL) were added 3-methyloxetan-3-amine (98.3 µL, 1.12 mmol) and potassium iodide (55.8 mg, 0.336 mmol), and the mixture was stirred at 50 °C for 2 hours. The mixture was purified by column chromatography on silica gel (CHCl₃(0.5% Et₃N)-MeOH) to obtain (E)-N-(2,6-difluoro-4-(10-(methoxymethyl)-9,12-dimethyl-7-(trifluoromethyl)-9,12,13,14-tetrahydroimidazo[4",5":5',6']benzo[1',2':2,3]azocino[4,5,6-hi]indolizine-2-carbonyl)phenyl)-4-((3-methyloxetan-3-yl)amino)but-2-enamide.

The racemate was separated by CHIRALPAK-IE (DAICEL) (hexane-EtOH (0.1% Et₃N) (10/90)), and the 2^{nd} elution product was the title compound. ¹H NMR (400 MHz, CHLOROFORM-D) δ 9.97 (dd, J = 7.0, 1.0 Hz, 1H), 7.75 (s, 1H), 7.41 (d, J = 7.8 Hz, 2H), 7.31-7.24 (m, 1H), 7.18-6.98 (m, 4H), 6.34 (d, J = 15.3 Hz, 1H), 4.81 (d, J = 1.8 Hz, 2H), 4.59 (d, J = 6.3 Hz, 2H), 4.44 (d, J = 6.5 Hz, 2H), 3.94 (s, 3H), 3.53 (dd, J = 5.1, 1.6 Hz, 2H), 3.48-3.43 (m, 4H), 3.10 (s, 3H), 2.91-2.84 (m, 1H), 2.56-2.52 (m, 1H), 2.27 (td, J = 14.0, 4.3 Hz, 1H), 1.52 (s, 3H); ESI-MS [M+H]⁺: 723.

### Example 140

### (E)-N-(2,6-difluoro-4-(7-methoxy-9,10,12-trimethyl-9,12,13,14-tetrahydroimidazo[4",5":5',6']benzo[1',2':2,3]azocino[4,5,6-hi]indolizine-2-carbonyl)phenyl)-4-((1-methylcyclopropyl)amino)but-2-enamide

### Steps 1-3:

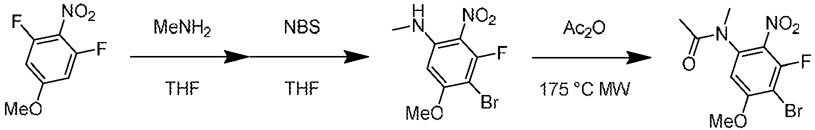

Methylamine (2 M THF solution, 30 mL) was added to a solution of 1,3-difluoro-5-methoxy-2-nitrobenzene (5.67 g, 30 mmol) in THF (21 mL). After stirring at room temperature for 15 minutes, water and EtOAc were added to the mixture. The organic layer was separated, washed with brine, dried over Na₂SO₄, and evaporated. The residue was diluted in THF (100 mL), cooled to 0 °C, and NBS (5.34 g, 30.0 mmol) was added. After stirring for 50 minutes, the precipitate was collected by filtration and washed with diisopropylether to obtain 4-bromo-3-fluoro-5-methoxy-N-methyl-2-nitroaniline (4.58 g). The above filtrate also contained 4-bromo-3-fluoro-5-methoxy-N-methyl-2-nitroaniline mainly and the filtrate was only used for the next reaction after evaporation. Acetic anhydride (40 mL) was added to the residue, then the reaction mixture was heated at 175 °C by microwave irradiation for 1 hour. After cooling, the mixture was diluted with MeOH and evaporated. The residue was diluted with EtOAc, washed with sat. NaHCO₃ aq., brine, dried over Na₂SO₄, and evaporated. The residue was purified by column chromatography on silica gel (hexane-EtOAc) to obtain *N*-(4-bromo-3-fluoro-5-methoxy-2-nitrophenyl)-*N*-methylacetamide.

### Step 4:

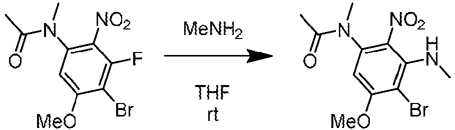

To the mixture of*N*-(4-bromo-3-fluoro-5-methoxy-2-nitrophenyl)-*N-*methylacetamide (4.66 g, 14.5 mmol) in THF (20 mL) was added methylamine (2 M THF solution, 21.8 mL). After stirring at room temperature for 30 minutes, water and EtOAc were added to the mixture. The organic layer was separated, washed with brine, and dried over Na₂SO₄. After evaporation, the residue was triturated with diisopropyl ether and collected by filtration to obtain *N*-(4-bromo-5-methoxy-3-(methylamino)-2-nitrophenyl)-N-methylacetamide.

### Step 5:

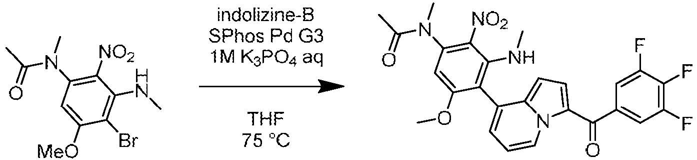

A mixture of *N*-(4-bromo-5-methoxy-3-(methylamino)-2-nitrophenyl)-*N-*methylacetamide (2.32 g, 6.98 mmol), (8-(5,5-dimethyl-1,3,2-dioxaborinan-2-yl)indolizin-3-yl)(3,4,5-trifluorophenyl)methanone (4.05 g, 10.5 mmol) and SPhos Pd G3 (252 mg, 0.323 mmol) in toluene (49 mL) and 1 M K₃PO₄ aq. (12.6 mL, 12.6 mmol) was evacuated and N₂ back-filled before heating to 100 °C for 1 hour. After cooling, the resulting mixture was diluted with EtOAc and the insoluble matters were removed by filtration. The filtrate was washed with water, brine, and dried over Na₂SO₄. After evaporation, the resulting residue was purified by column chromatography on silica gel (hexane-EtOAc(+10% CHCl₃)) to obtain N-(5-methoxy-3-(methylamino)-2-nitro-4-(3-(3,4,5-trifluorobenzoyl)indolizin-8-yl)phenyl)-*N*-methylacetamide.

### Step 6:

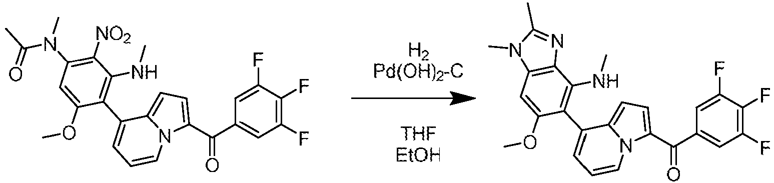

To a solution of *N*-(5-methoxy-3-(methylamino)-2-nitro-4-(3-(3,4,5-trifluorobenzoyl)indolizin-8-yl)phenyl)-*N*-methylacetamide (2.06 g, 3.91 mmol) in ethanol (20 mL) and THF (50 mL) was added palladium hydroxide on carbon (2.90 g, 20 wt. % loading (dry basis)). The mixture was stirred at room temperature overnight under hydrogen atmosphere. After nitrogen purging, the mixture was filtered and the filter cake was rinsed with EtOAc and MeOH. To the filtrate was added acetic acid (2 mL), and the mixture was concentrated. The residue was purified by column chromatography on silica gel (hexane-EtOAc/EtOH(4/1)) to give (8-(6-methoxy-1,2-dimethyl-4-(methylamino)-1*H*-benzo[*d*]imidazol-5-yl)indolizin-3-yl)(3,4,5-trifluorophenyl)methanone.

### Step 7:

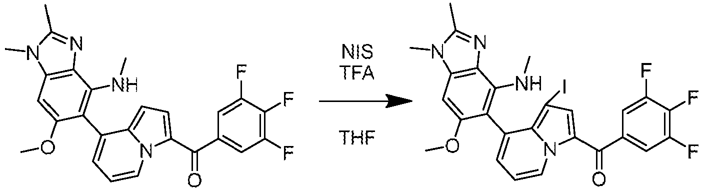

To a solution of (8-(6-methoxy-1,2-dimethyl-4-(methylamino)-1H-benzo[d]imidazol-5-yl)indolizin-3-yl)(3,4,5-trifluorophenyl)methanone (1.99 g, 4.16 mmol) in THF (50 mL) was added NIS (960 mg, 4.27 mmol) and TFA (1.60 mL, 21.5 mmol). After stirring at room temperature for 3 hours, the mixture was diluted with EtOAc, sat. NaHCO₃ aq., and sat. Na₂S₂O₃ aq.. The organic layer was separated, washed with brine, and dried over Na₂SO₄. After evaporation, the obtained residue was diluted with diisopropyl ether and the precipitate was collected by filtration to obtain (1-iodo-8-(6-methoxy-1,2-dimethyl-4-(methylamino)-1*H*-benzo[*d*]imidazol-5-yl)indolizin-3-yl)(3,4,5-trifluorophenyl)methanone.

### Step 8:

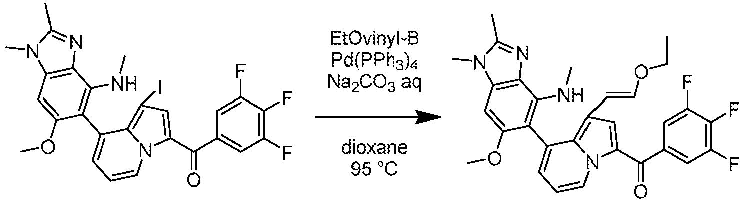

A mixture of (1-iodo-8-(6-methoxy-1,2-dimethyl-4-(methylamino)-1*H-*benzo[*d*]imidazol-5-yl)indolizin-3-yl)(3,4,5-trifluorophenyl)methanone (2.14 g, 3.54 mmol), 2-[(*E*)-2-ethoxyvinyl]-4,4,5,5-tetramethyl-1,3,2-dioxaborolane (3.75 mL, 17.7 mmol) and Pd(PPh₃)₄ (300 mg) in 1,4-dioxane (54 mL) and 2 M Na₂CO₃ aq. (8.85 mL, 17.7 mmol) was evacuated and N₂ back-filled before heating to 95 °C for 6 hours. After cooling, the reaction mixture was diluted with EtOAc, washed with water and brine, dried over Na₂SO₄, and evaporated. The residue was triturated by diisopropyl ether and the precipitates were collected by filtration to obtain (*E*)-(1-(2-ethoxyvinyl)-8-(6-methoxy-1,2-dimethyl-4-(methylamino)-1*H*-benzo[*d*]imidazol-5-yl)indolizin-3-yl)(3,4,5-trifluorophenyl)methanone.

### Step 9:

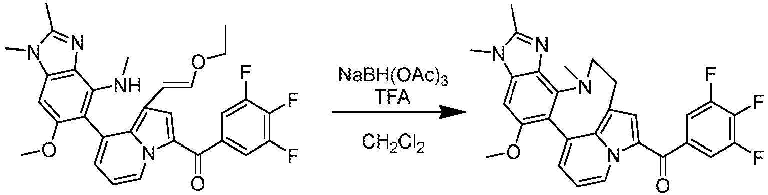

To a solution of (*E*)-(1-(2-ethoxyvinyl)-8-(6-methoxy-1,2-dimethyl-4-(methylamino)-1*H*-benzo[*d*]imidazol-5-yl)indolizin-3-yl)(3,4,5-trifluorophenyl)methanone (1.64 g, 3.00 mmol) in CH₂Cl₂ (25 mL) was added sodium triacetoxyborohydride (1.27 g, 5.99 mmol) and TFA (5.56 mL, 74.9 mmol) at room temperature. After stirring at room temperature for 30 minutes, the reaction was diluted with sat. NaHCO₃ aq., and the resulting mixture was diluted with EtOAc. The organic layer was separated, washed with brine, dried over Na₂SO₄, and evaporated. The residue was triturated by diisopropyl ether and the precipitates were collected by filtration to obtain (7-methoxy-9,10,12-trimethyl-9,12,13,14-tetrahydroimidazo[4",5":5"6']benzo[1',2':2,3]azocino[4,5,6-*hi*]indolizin-2-yl)(3,4,5-trifluorophenyl)methanone.

### Step 10:

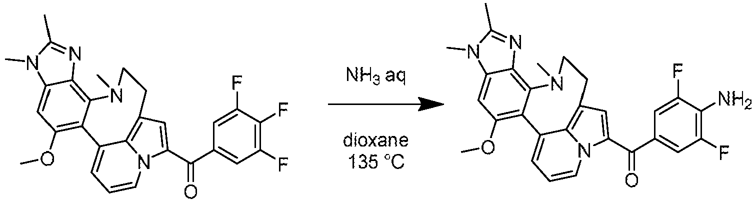

To the mixture of (7-methoxy-9,10,12-trimethyl-9,12,13,14-tetrahydroimidazo[4",5":5"6']benzo[1',2':2,3]azocino[4,5,6-*hi*]indolizin-2-yl)(3,4,5-trifluorophenyl)methanone (1.50 g, 2.97 mmol) in 1,4-dioxane (12 mL) was added 28-30% NH₃ aq. (24 mL). The reaction mixture was heated to 135 °C by microwave irradiation for 13 hours. After cooling, the mixture was diluted with EtOAc, washed with brine, dried over Na₂SO₄, and evaporated. The residue was triturated by EtOAc and diisopropyl ether, and the precipitate was collected by filtration to obtain (4-amino-3,5-difluorophenyl)(7-methoxy-9,10,12-trimethyl-9,12,13,14-tetrahydroimidazo[4",5":5',6']benzo[1',2':2,3]azocino[4,5,6-*hi*]indolizin-2-yl)methanone.

### Steps 11-12:

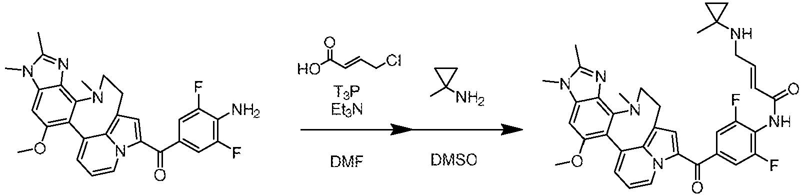

To the mixture of (4-amino-3,5-difluorophenyl)(7-methoxy-9,10,12-trimethyl-9,12,13,14-tetrahydroimidazo[4",5":5',6']benzo[1',2':2,3]azocino[4,5,6-*hi*]indolizin-2-yl)methanone (393 mg, 0.784 mmol), (*E*)-4-chlorobut-2-enoic acid (123 mg, 1.02 mmol) and T3P (50 wt% in THF, 1.47 mL) in THF (5 mL) was added triethylamine (0.641 mL, 4.70 mmol). After stirring at room temperature for 30 minutes, the resulting mixture was diluted with EtOAc, washed with sat. NaHCO₃ aq., water, brine, then dried over Na₂SO₄ and evaporated. To the residue was added DMSO (5 mL) and 1-methylcyclopropanamine (0.435 mL) and the mixture was stirred at room temperature for 5 hours. Additional methylcyclopropanamine (0.2 mL) was added, and the mixture was stirred at room temperature for 2 hours. Water was added and the mixture was extracted with EtOAc. The combined organic layers were washed with brine, dried over Na₂SO₄, and evaporated. The residue was suspended with ethanol and diisopropyl ether, and collected by filtration to obtain (*E*)-*N*-(2,6-difluoro-4-(7-methoxy-9,10,12-trimethyl-9,12,13,14-tetrahydroimidazo[4",5":5',6']benzo[1',2':2,3]azocino[4,5,6-*hi*]indolizine-2-carbonyl)phenyl)-4-((1-methylcyclopropyl)amino)but-2-enamide.

The racemate was separated by CHIRALPAK-IE (hexane-EtOH(0.1% Et₃N): 10/90) to obtain the title compound (as a 2^{nd} elution). ¹H NMR (400 MHz, CDCl₃) δ 9.99 (dd, *J* = 7.0, 1.0 Hz, 1H), 7.44-7.37 (m, 2H), 7.25 (dd, *J* = 7.1, 1.1 Hz, 1H), 7.16-7.08 (m, 3H), 7.04-7.00 (m, 1H), 6.78 (s, 1H), 6.25-6.20 (m, 1H), 3.79 (s, 3H), 3.73 (s, 3H), 3.69 (t, *J* = 2.6 Hz, 1H), 3.56-3.50 (m, 3H), 3.06 (s, 3H), 2.89-2.83 (m, 1H), 2.62 (s, 3H), 2.58-2.54 (m, 1H), 2.49-2.41 (m, 1H), 1.29 (s, 3H), 0.61 (dd, *J =* 5.9, 4.6 Hz, 2H), 0.41 (dd, *J* = 6.4, 4.4 Hz, 2H); ESI-MS [M+H]⁺: 639.

### Example 146

### (E)-N-(2,6-difluoro-4-(9,10,12-trimethyl-7-(trifluoromethyl)-10,12,13,14-tetrahydroindolizino[8',1':4,5,6]azocino[3,2-g]indazole-2-carbonyl)phenyl)-4-((3-methyloxetan-3-yl)amino)but-2-enamide

### Step 1:

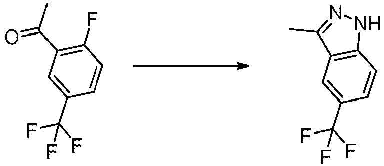

Hydrazine monohydrate (0.840 mL) was added to 1-(2-fluoro-5-(trifluoromethyl)phenyl)ethan-1-one (3.00 g, 14.6 mmol) in NMP (25.0 mL) and the mixture was stirred at 160 °C using microwave irradiation for 8 hours. After dilution with EtOAc, the mixture was washed with a saturated NaHCO₃ solution, brine, and dried over Na₂SO₄. After evaporation, the residue was purified by silica gel column chromatography (hexane:ethyl acetate) to obtain 3-methyl-5-(trifluoromethyl)-1*H*-indazole.

### Step 2:

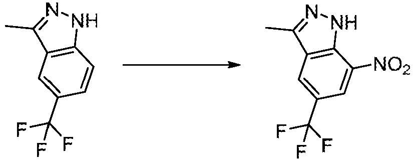

A solution of 3-methyl-5-(trifluoromethyl)-1*H*-indazole (280 mg, 1.40 mmol) in H₂SO₄ (0.68 mL) was cooled to 0 °C, and potassium nitrate (198 mg, 1.96 mmol) was added. After stirring at 0 °C for 15 minutes, the reaction mixture was poured into ice. The mixture was diluted with EtOAc, the organic layer was washed with diluted NH₄OH, brine, and dried over Na₂SO₄. After evaporation, the residue was purified by silica gel column chromatography (hexane:ethyl acetate) to afford 3-methyl-7-nitro-5-(trifluoromethyl)-1*H*-indazole.

### Step 3:

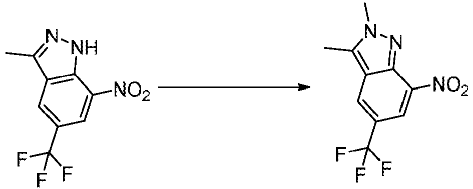

3-Methyl-7-nitro-5-(trifluoromethyl)-1*H*-indazole (330 mg, 1.35 mmol) and trimethyloxonium tetrafluoroborate (597 mg) in acetone (6 mL) were stirred at 30 °C for 1 hour. After evaporation, EtOAc and saturated NaHCO₃ solution were added to the mixture. The layers were separated and the aqueous later was extracted with EtOAc. The combined organic layers were washed with brine, dried over Na₂SO₄, and concentrated to afford 2,3-dimethyl-7-nitro-5-(trifluoromethyl)-2*H*-indazole.

### Step 4:

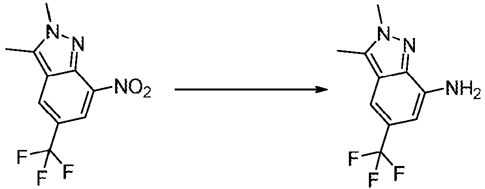

A solution of 2,3-dimethyl-7-nitro-5-(trifluoromethyl)-2*H*-indazole (2.27 g, 8.76 mmol), iron powder (2.5 g), and 2 M NH₄Cl solution (10 mL) in methanol (15 mL) and THF (15 mL) was stirred at 75 °C for 2 hours. The insoluble material was filtered and the filtrate was concentrated under reduced pressure. The residue was diluted with EtOAc, washed with water, brine, and dried over Na₂SO₄. After evaporation, residue was purified by silica gel column chromatography (hexane:ethyl acetate) to afford 2,3-dimethyl-5-(trifluoromethyl)-2*H*-indazol-7-amine.

### Step 5:

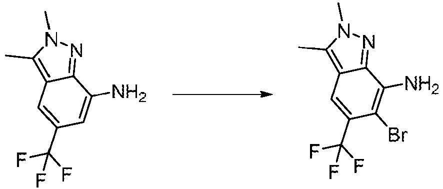

A solution of 2,3-dimethyl-5-(trifluoromethyl)-2H-indazol-7-amine (2.03 g) obtained in step 4 and N-bromosuccinimide (1.51 g) in DMF (30 mL) was stirred at room temperature for 30 minutes. The mixture was diluted with ethyl acetate and washed with a saturated sodium chloride solution, followed by drying over sodium sulfate. After evaporation, the residue was purified by column chromatography (hexane:ethyl acetate), thereby obtaining 6-bromo-2,3-dimethyl-5-(trifluoromethyl)-2H-indazol-7-amine.

### Step 6:

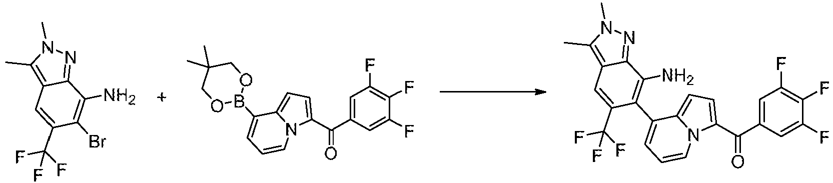

A solution of 6-bromo-2,3-dimethyl-5-(trifluoromethyl)-2H-indazol-7-amine (174 mg) obtained in step 5, (8-(5,5-dimethyl-1,3,2-dioxaborinan-2-yl)indolizin-3-yl)(3,4,5-trifluorophenyl)methanone (394 mg), SPhos Pd G3 (39.8 mg) in THF (5.00 mL) and 2M K₃PO₄ aq. (0.71 mL) was evacuated and N₂ back-filled before heating to 80 °C for 1 hour using microwave. After cooling, the reaction mixture was diluted with EtOAc. The resulting suspension was filtered, rinsing with EtOAc, and the filtrate was washed with H₂O and brine then dried over Na₂SO₄ and evaporated. The residue was purified by column chromatography on silica gel (hexane:acetone), thereby obtaining (8-(7-amino-2,3-dimethyl-5-(trifluoromethyl)-2H-indazol-6-yl)indolizin-3-yl)(3,4,5-trifluorophenyl)methanone.

### Step 7:

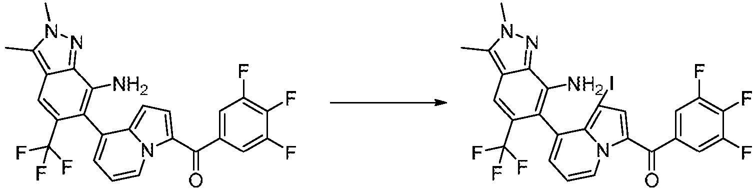

N-iodosuccinimide (160 mg) was added to a solution of (8-(7-amino-2,3-dimethyl-5-(trifluoromethyl)-2H-indazol-6-yl)indolizin-3-yl)(3,4,5-trifluorophenyl)methanone (330 mg) and TFA (0.22 mL) in THF (10 mL). After stirring at room temperature for 30 minutes, the reaction mixture was diluted with EtOAc, washed with sat. NaHCO₃ aq., H₂O (2x) and brine then dried over Na₂SO₄ and evaporated. The residue was purified by column chromatography on silica gel (hexane:ethyl acetate), thereby obtaining (8-(7-amino-2,3-dimethyl-5-(trifluoromethyl)-2H-indazol-6-yl)-1-iodoindolizin-3-yl)(3,4,5-trifluorophenyl)methanone.

### Step 8:

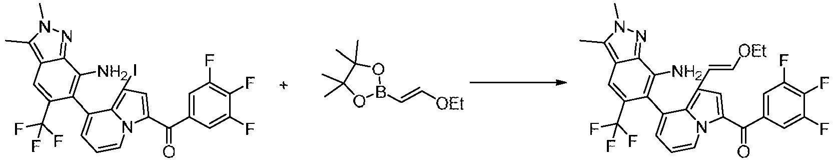

A solution of (8-(7-amino-2,3-dimethyl-5-(trifluoromethyl)-2H-indazol-6-yl)-1-iodoindolizin-3-yl)(3,4,5-trifluorophenyl)methanone (224 mg), 2-[(E)-2-ethoxyvinyl]-4,4,5,5-tetramethyl-1,3,2-dioxaborolane (0.38 mL), Pd(PPh₃)₄ (41.2 mg) and 2M Na₂CO₃ aq. (0.89 mL) in 1,4-dioxane (5.0 mL) was evacuated and N₂ back-filled before heating to 90 °C for 3 hours. After cooling, the reaction mixture was diluted with ethyl acetate, washed with H₂O and brine then dried over Na₂SO₄ and evaporated. The residue was purified by column chromatography on silica gel (hexane: ethyl acetate), thereby obtaining (E)-(8-(7-amino-2,3-dimethyl-5-(trifluoromethyl)-2H-indazol-6-yl)-1-(2-ethoxyvinyl)indolizin-3-yl)(3,4,5-trifluorophenyl)methanone.

### Step 9:

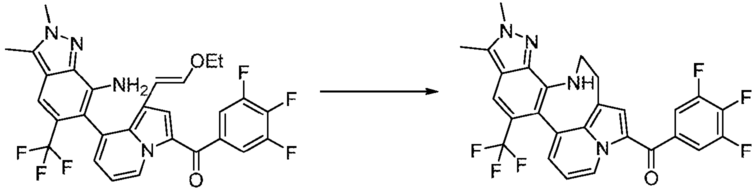

Sodium triacetoxyborohydride (160 mg) was added to a solution of (E)-(8-(7-amino-2,3-dimethyl-5-(trifluoromethyl)-2H-indazol-6-yl)-1-(2-ethoxyvinyl)indolizin-3-yl)(3,4,5-trifluorophenyl)methanone (140 mg) and TFA (0.22 mL) in dichloromethane (10 mL). After stirring at room temperature for 30 minutes, the reaction mixture was diluted with chloroform, washed with sat. NaHCO₃ aq., H₂O (2x) and brine then dried over Na₂SO₄ and evaporated. The residue was purified by column chromatography on silica gel (ethyl acetate: ethanol), thereby obtaining (9,10-dimethyl-7-(trifluoromethyl)-10,12,13,14-tetrahydroindolizino[8',1':4,5,6]azocino[3,2-g]indazol-2-yl)(3,4,5-trifluorophenyl)methanone.

### Step 10:

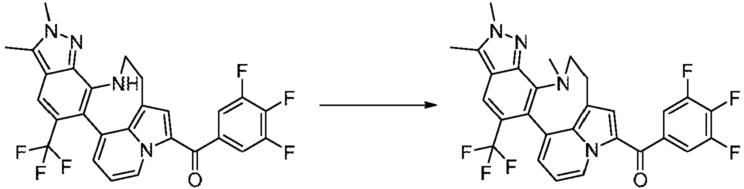

0.3M sodium cyanoborohydride-1/2 zinc chloride in methanol (0.66 mL) was added to a solution of (9,10-dimethyl-7-(trifluoromethyl)-10,12,13,14-tetrahydroindolizino[8',1':4,5,6]azocino[3,2-g]indazol-2-yl)(3,4,5-trifluorophenyl)methanone (52.0 mg) and 36% formaldehyde solution in water (25 µL) in THF (0.80 mL). After stirring at room temperature for 30 minutes, the reaction mixture was diluted with ethyl acetate, washed with sat. NaHCO₃ aq., H₂O (2x) and brine then dried over Na₂SO₄ and evaporated. The residue was purified by column chromatography on silica gel (hexane:ethyl acetate), thereby obtaining (3,4,5-trifluorophenyl)(9,10,12-trimethyl-7-(trifluoromethyl)-10,12,13,14-tetrahydroindolizino[8',1':4,5,6]azocino[3,2-g]indazol-2-yl)methanone.

### Step 11:

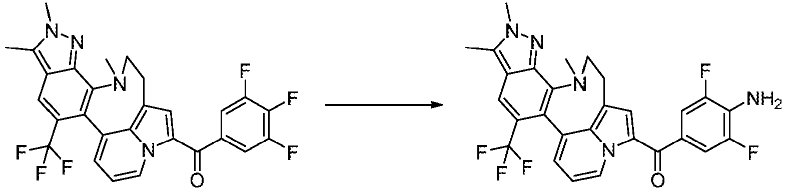

A solution of (3,4,5-trifluorophenyl)(9,10,12-trimethyl-7-(trifluoromethyl)-10,12,13,14-tetrahydroindolizino[8',1':4,5,6]azocino[3,2-g]indazol-2-yl)methanone (48.0 mg) obtained in step 10 and 28% ammonia solution in water (2.5 mL) in 1,4-dioxane (2.5 mL) was stirred at 140 °C for 12 hours using microwave irradiation. After dilution with ethyl acetate and water, the mixture was extracted with ethyl acetate. The combined organic layer was washed with water and a saturated sodium chloride solution, followed by drying over sodium sulfate. After evaporation, the obtained residue was purified by column chromatography (hexane:ethyl acetate), thereby obtaining (4-amino-3,5-difluorophenyl)(9,10,12-trimethyl-7-(trifluoromethyl)-10,12,13,14-tetrahydroindolizino[8',1':4,5,6]azocino[3,2-g]indazol-2-yl)methanone.

### Step 12:

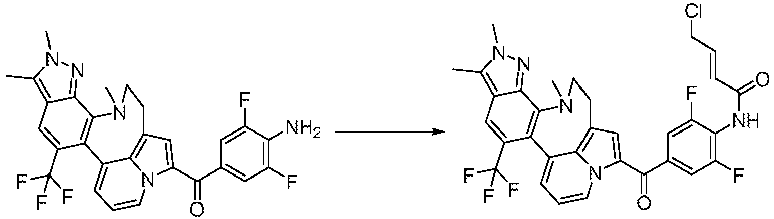

(E)-4-chlorobut-2-enoic acid (12.5 mg) and 1-propanephosphonic acid anhydride cyclic trimer (a 1.7M THF solution, 125 µL) were added to a solution of (4-amino-3,5-difluorophenyl)(9,10,12-trimethyl-7-(trifluoromethyl)-10,12,13,14-tetrahydroindolizino[8',1':4,5,6]azocino[3,2-g]indazol-2-yl)methanone (43.0 mg) in DMF (2.0 mL), followed by stirring at room temperature for 20 minutes. Triethylamine (45 µL) was added to the reaction mixture. A reaction was performed at room temperature for 2 hours. After water was added to the reaction mixture, extraction was performed with ethyl acetate, followed by washing the organic layer with a saturated sodium chloride solution. The washed organic layer was dried over sodium sulfate. After evaporation, the obtained residue was purified by column chromatography (chloroform:ethanol), thereby obtaining (E)-4-chloro-N-(2,6-difluoro-4-(9,10,12-trimethyl-7-(trifluoromethyl)-10,12,13,14-tetrahydroindolizino[8',1':4,5,6]azocino[3,2-g]indazole-2-carbonyl)phenyl)but-2-enamide.

### Step 13:

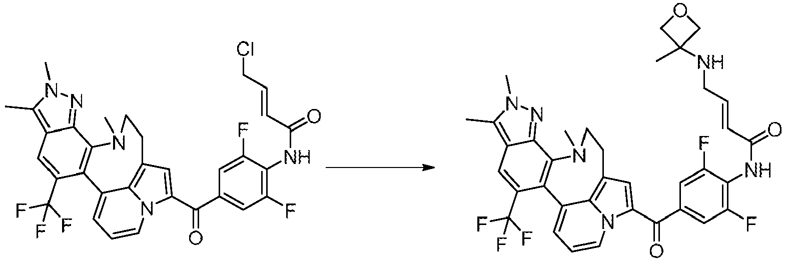

3-methyloxetan-3-amine (35 µL) and potassium iodide (19.8 mg) were added to a solution of (E)-4-chloro-N-(2,6-difluoro-4-(9,10,12-trimethyl-7-(trifluoromethyl)-10,12,13,14-tetrahydroindolizino[8',1':4,5,6]azocino[3,2-g]indazole-2-carbonyl)phenyl)but-2-enamide (51.0 mg) in DMSO (900 µL). A reaction was performed at room temperature for 2 hours. After water was added to the reaction mixture, extraction was performed with ethyl acetate. The combined organic layer was washed with a saturated sodium chloride solution. After the washed layer was dried over sodium sulfate, the solvent was evaporated under reduced pressure. The obtained residue was purified by column chromatography (chloroform:ethanol), thereby obtaining the racemic (E)-N-(2,6-difluoro-4-(9,10,12-trimethyl-7-(trifluoromethyl)-10,12,13,14-tetrahydroindolizino[8',1':4,5,6]azocino[3,2-g]indazole-2-carbonyl)phenyl)-4-((3-methyloxetan-3-yl)amino)but-2-enamide (35.0 mg). The racemate was separated by CHIRAL ART SB (YMC) (hexane:EtOH (0.1% Et₃N)) to give (E)-N-(2,6-difluoro-4-(9,10,12-trimethyl-7-(trifluoromethyl)-10,12,13,14-tetrahydroindolizino[8',1':4,5,6]azocino[3,2-g]indazole-2-carbonyl)phenyl)-4-((3-methyloxetan-3-yl)amino)but-2-enamide (single isomer, 1st-eluting isomer). ¹H-NMR: (400 MHz, CHLOROFORM-D) δ 9.97 (dd, J = 6.9, 0.9 Hz, 1H), 8.00 (s, 1H), 7.41 (dd, J = 10.5, 2.8 Hz, 2H), 7.29 (brs, 1H), 7.17-7.09 (m, 3H), 7.00 (t, J = 7.1 Hz, 1H), 6.34 (d, J = 15.5 Hz, 1H), 4.59 (d, J = 6.5 Hz, 2H), 4.44 (d, J = 6.8 Hz, 2H), 4.17 (s, 3H), 3.53 (dd, J = 5.3, 1.8 Hz, 2H), 3.46-3.43 (m, 1H), 3.02 (s, 3H), 2.86-2.79 (m, 1H), 2.69 (s, 3H), 2.56-2.52 (m, 1H), 2.36 (td, J = 13.8, 4.5 Hz, 1H), 2.05-2.03 (m, 1H), 1.52 (s, 3H); ESI-MS [M+H]⁺: 693.

### Example 174

### (E)-N-(2,6-difluoro-4-(7-methoxy-10-(methoxymethyl)-9,12-dimethyl-9,12,13,14-tetrahydroimidazo[4",5":5',6']benzo[1',2':2,3]azocino[4,5,6-hi]indolizine-2-carbonyl)phenyl)-4-((1-methylcyclopropyl)amino)but-2-enamide

### Steps 1-2:

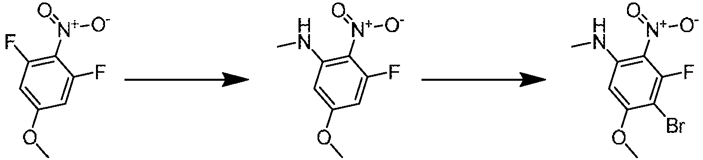

2M methaylamine in THF (50.2 mL) was slowly added to the mixture of 1,3-difluoro-5-methoxy-2-nitrobenzene (9.50 g, 50.2 mmol) in THF (35.2 mL) at 0 °C. After stirring at ambient temperature for 1 hour, water and EtOAc were added to the resulting slurry. The organic layer was separated, and washed with a saturated sodium chloride solution, followed by drying over sodium sulfate. After evaporation, to the obtained residue of 3-fluoro-5-methoxy-N-methyl-2-nitroaniline was added THF (200 mL), and NBS (8.94 g, 50.2 mmol) was added thereto at 0 °C. After stirring for 10 minutes, the precipitates were collected by filtration and washed with diisopropylether to obtain 4-bromo-3-fluoro-5-methoxy-N-methyl-2-nitroaniline (7.61 g, 27.3 mmol). The filtrates were diluted with EtOAc and washed with saturated NaHCO₃ aq. and brine, dried over Na₂SO₄ and evaporated. The residue was diluted with diisopropylether, and collected by filtration to obtain 4-bromo-3-fluoro-5-methoxy-N-methyl-2-nitroaniline.

### Step 3:

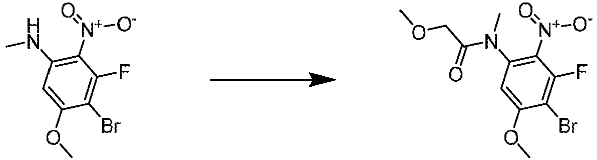

A solution of 4-bromo-3-fluoro-5-methoxy-N-methyl-2-nitroaniline (2.46 g, 8.82 mmol) in 2-methoxyacetic anhydride (15 mL) was stirred under microwave irradiation at 180 °C for 1 hour and then poured into MeOH. After the solution was evaporated to remove the solvent, the residue was diluted with EtOAc and saturated sodium bicarbonate solution. The organic layer was separated, and washed with a saturated sodium chloride solution, followed by drying over sodium sulfate and concentration. The residue was purified by column chromatography (hexane:ethyl acetate), thereby obtaining N-(4-bromo-3-fluoro-5-methoxy-2-nitrophenyl)-2-methoxy-N-methylacetamide.

### Step 4:

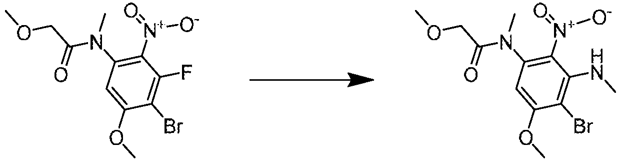

2M methaylamine in THF (7.7 mL, 15.3 mmol) was slowly added to the mixture of N-(4-bromo-3-fluoro-5-methoxy-2-nitrophenyl)-2-methoxy-N-methylacetamide (2.68 g, 50.2 mmol) in THF (35.2 mL) at 0 °C. After stirring at ambient temperature for 30 minutes, the water was added and the precipitate was collected by filtration to obtain N-(4-bromo-5-methoxy-3-(methylamino)-2-nitrophenyl)-2-methoxy-N-methylacetamide.

### Step 5:

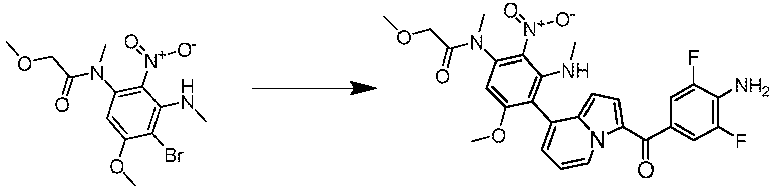

A solution of N-(4-bromo-5-methoxy-3-(methylamino)-2-nitrophenyl)-2-methoxy-N-methylacetamide (720 mg, 1.99 mmol), (4-amino-3,5-difluorophenyl)(8-(5,5-dimethyl-1,3,2-dioxaborinan-2-yl)indolizin-3-yl)methanone (1.07 g, 2.78 mmol) and SPhos Pd G3 (143 mg, 0.199 mmol) in THF (15 mL) and 2M K₃PO₄ aq. (1.99 mL, 3.98 mmol) was evacuated and N₂ back-filled before heating to 75 °C for 3 hours. After cooling, the reaction mixture was diluted with EtOAc/MeOH (ca. 10/1). The resulting suspension was filtered, rinsing with EtOAc, the filtrate was washed with H₂O and brine then dried over Na₂SO₄ and evaporated. The residue was suspended with diisopropyl ether and collected by filtration to obtain N-(4-(3-(4-amino-3,5-difluorobenzoyl)indolizin-8-yl)-5-methoxy-3-(methylamino)-2-nitrophenyl)-2-methoxy-N-methylacetamide.

### Step 6:

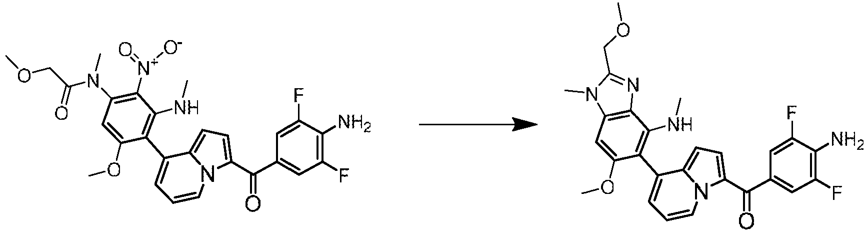

To a solution of N-(4-(3-(4-amino-3,5-difluorobenzoyl)indolizin-8-yl)-5-methoxy-3-(methylamino)-2-nitrophenyl)-2-methoxy-N-methylacetamide (1.14 g, 2.06 mmol) in EtOH (10 mL) and THF (25 mL), palladium hydroxide (1.00 g) was added, then evacuated and H₂ back-filled. After stirring at room temperature for 2 days, the mixture was diluted with EtOAc and MeOH, and the insoluble matter was filtered off. The filtrate was concentrated under reduced pressure and the residue was purified by columnn chromatography on silica gel (hexane-EtOAc/EtOH(4/1)), thereby obtaining (4-amino-3,5-difluorophenyl)(8-(6-methoxy-2-(methoxymethyl)-1-methyl-4-(methylamino)-1H-benzo[d]imidazol-5-yl)indolizin-3-yl)methanone.

### Step 7:

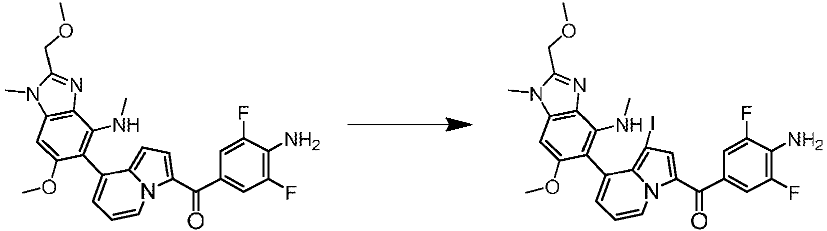

N-Iodosuccinimide (190 mg, 0.847 mmol) and TFA (0.126 mL) were added to a solution of (4-amino-3,5-difluorophenyl)(8-(6-methoxy-2-(methoxymethyl)-1-methyl-4-(methylamino)-1H-benzo[d]imidazol-5-yl)indolizin-3-yl)methanone (590 mg, 0.847 mmol) in THF (4.0 mL). After stirring at room temperature overnight, additional N-Iodosuccinimide (57.2 mg, 0.254 mmol) was added thereto. After stirring at room temperature for another 1 hour. The mixture was diluted with EtOAc, and sat. NaHCO₃ aq. and sat. Na₂S₂O₃ aq. were added. The organic layer was separated, and washed with brine, followed by drying over sodium sulfate. After evaporation, the residue was purified by columnn chromatography on silica gel (hexane-EtOAc/EtOH(4/1)), thereby obtaining (4-amino-3,5-difluorophenyl)(1-iodo-8-(6-methoxy-2-(methoxymethyl)-1-methyl-4-(methylamino)-1H-benzo[d]imidazol-5-yl)indolizin-3-yl)methanone.

### Step 8:

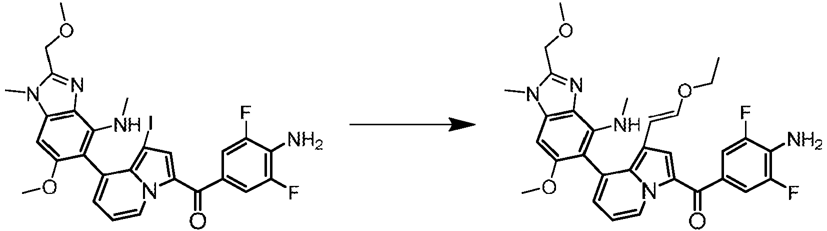

A mixture of (4-amino-3,5-difluorophenyl)(1-iodo-8-(6-methoxy-2-(methoxymethyl)-1-methyl-4-(methylamino)-1H-benzo[d]imidazol-5-yl)indolizin-3-yl)methanone (648 mg, 1.03 mmol), 2-[(E)-2-ethoxyvinyl]-4,4,5,5-tetramethyl-1,3,2-dioxaborolane (1.09 mL, 5.13 mmol), Pd(PPh₃)₄ (119 mg, 0.103 mmol) in 1,4-dioxane (15 mL) and 2M Na₂CO₃ aq. (2.3 mL, 4.62 mmol) was evacuated and N₂ back-filled before heating to 95 °C for 8 hours. After cooling, the reaction mixture was diluted with EtOAc, washed with H₂O and brine then dried over Na₂SO₄ and evaporated. The residue was purified by column chromatography on silica gel (hexane-EtOAc/EtOH(4/1)) to obtain (E)-(4-amino-3,5-difluorophenyl)(1-(2-ethoxyvinyl)-8-(6-methoxy-2-(methoxymethyl)-1-methyl-4-(methylamino)-1H-benzo[d]imidazol-5-yl)indolizin-3-yl)methanone.

### Step 9:

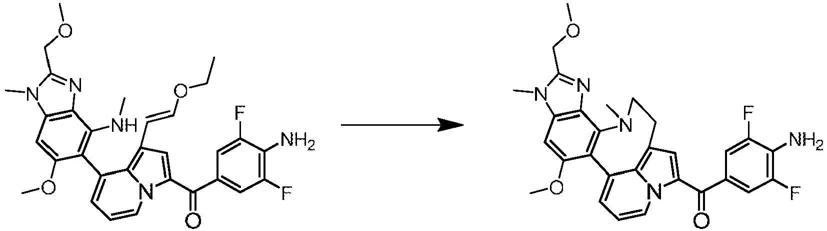

To a solution of (E)-(4-amino-3,5-difluorophenyl)(1-(2-ethoxyvinyl)-8-(6-methoxy-2-(methoxymethyl)-1-methyl-4-(methylamino)-1H-benzo[d]imidazol-5-yl)indolizin-3-yl)methanone (454 mg, 0.788 mmol) in CH₂Cl₂ (12 mL) was added sodium triacetoxyborohydride (334 mg, 1.57 mmol) and TFA (1.17 mL, 15.7 mmol) at room temperature. After stirring at room temperature for 15 minutes, additional sodium triacetoxyborohydride (334 mg, 1.57 mmol) and TFA (1.17 mL, 15.7 mmol) were added thereto. After stirring at room temperature for another 45 minutes, the reaction was quenched with sat. NaHCO₃ aq., then the resulting mixture was diluted with EtOAc. The organic layer was washed with brine, dried over Na₂SO₄ and evaporated. The residue was purified by column chromatography on silica gel (hexane-EtOAc/EtOH(4/1)) to obtain (4-amino-3,5-difluorophenyl)(7-methoxy-10-(methoxymethyl)-9,12-dimethyl-9,12,13,14-tetrahydroimidazo[4",5":5',6']benzo[1',2':2,3]azocino[4,5,6-*hi*]indolizin-2-yl)methanone.

### Step 10:

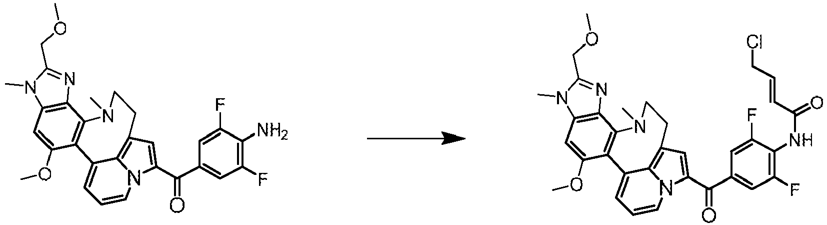

A solution of (4-amino-3,5-difluorophenyl)(7-methoxy-10-(methoxymethyl)-9,12-dimethyl-9,12,13,14-tetrahydroimidazo[4",5":5',6']benzo[1',2':2,3]azocino[4,5,6-hi]indolizin-2-yl)methanone (100 mg, 0.188 mmol) and triethylamine (0.524 mL, 3.76 mmol) in DMF (2.0 mL) was slowly added 0.470 mL of 4-chlorocrotonic acid T3P solution (prepared from (*E*)-4-chlorobut-2-enoic and T3P (50 wt% in THF)(100 mg 4-chlorocrotonic acid / mL)). After stirring at room temperature for 15 minutes, the resulting mixture was diluted with EtOAc, washed with sat. NaHCO₃ aq., H₂O and brine then dried over Na₂SO₄ and evaporated to obtain (E)-4-chloro-N-(2,6-difluoro-4-(7-methoxy-10-(methoxymethyl)-9,12-dimethyl-9,12,13,14-tetrahydroimidazo[4",5":5',6']benzo[1',2':2,3]azocino[4,5,6-hi]indolizine-2-carbonyl)phenyl)but-2-enamide.

### Step 11:

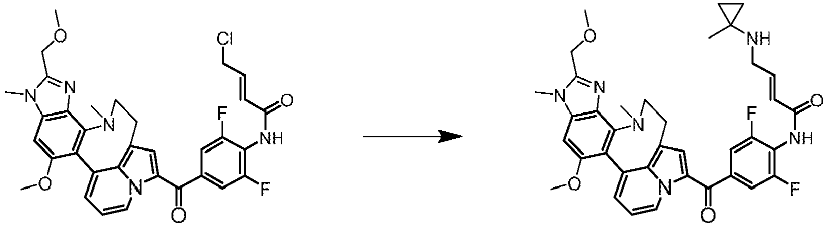

To a solution of (E)-4-chloro-N-(2,6-difluoro-4-(7-methoxy-10-(methoxymethyl)-9,12-dimethyl-9,12,13,14-tetrahydroimidazo[4",5":5',6']benzo[1',2':2,3]azocino[4,5,6-hi]indolizine-2-carbonyl)phenyl)but-2-enamide (118 mg, 0.186 mmol) in DMSO (0.50 mL) was added 1-methylcyclopropan-1-amine (73.7 µL, 0.930 mmol), and the mixture was stirred at 50 °C for 2 hours. The reaction mixture was diluted with EtOAc, washed with sat. NaHCO₃ aq., H₂O and brine then dried over Na₂SO₄ and evaporated to obtain (E)-N-(2,6-difluoro-4-(7-methoxy-10-(methoxymethyl)-9,12-dimethyl-9,12,13,14-tetrahydroimidazo[4",5":5',6']benzo[1',2':2,3]azocino[4,5,6-hi]indolizine-2-carbonyl)phenyl)-4-((1-methylcyclopropyl)amino)but-2-enamide (75.0 mg, 0.112 mmol).

The racemate was separated by CHIRALPAK-IE (DAICEL) (hexane-EtOH(0.1%Et₃N): 10/90), and the 2^{nd} elution was the title compound. ¹H-NMR: (400 MHz, CHLOROFORM-D) δ 9.99 (dd, J = 7.0, 1.0 Hz, 1H), 7.43-7.39 (m, 2H), 7.26-7.23 (m, 1H), 7.14-7.00 (m, 4H), 6.81 (s, 1H), 6.28 (d, J = 14.5 Hz, 1H), 4.79-4.72 (m, 2H), 3.96 (q, J = 7.7 Hz, 1H), 3.85-3.79 (m, 8H), 3.63 (dd, J = 9.0, 3.8 Hz, 1H), 3.54-3.43 (m, 7H), 3.07 (s, 3H), 2.91-2.84 (m, 1H), 2.58-2.54 (m, 1H), 2.49-2.41 (m, 1H), 2.18-2.10 (m, 1H), 1.80-1.72 (m, 1H); ESI-MS [M+H]⁺: 669.

### Example 187

### (E)-N-(4-(10,13-dimethyl-7-(trifluoromethyl)-14,15-dihydro-13H-imidazo[1",2":1',2']pyrido[3',4':2,3]azocino[4,5,6-hi]indolizine-2-carbonyl)-2,6-difluorophenyl)-4-((3-methyloxetan-3-yl)amino)but-2-enamide

### Step 1:

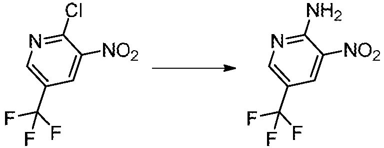

Ammonia (28% water solution, 10 mL) was added to 2-chloro-3-nitro-5-(trifluoromethyl)pyridine (2.10 g) in dioxane (8 mL), and the reaction mixture was stirred at 100 °C for 15 minutes using microwave. After dilution with ethyl acetate, the mixture was washed with water and a saturated sodium chloride solution, followed by drying over sodium sulfate. After evaporation, the residue was purified by column chromatography (hexane:ethyl acetate), thereby obtaining 3-nitro-5-(trifluoromethyl)pyridin-2-amine.

### Step 2:

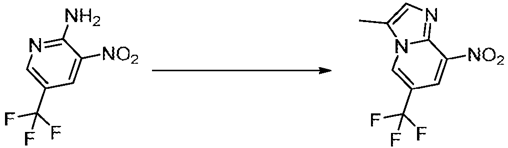

2M hydrochloric acid (3.7 mL) was added to a solution of 3-nitro-5-(trifluoromethyl)pyridin-2-amine (3.80 g) obtained in step 1 and 2-bromo-1,1-diethoxy-propane (4.1 g) in DMA (50 mL), followed by stirring at 100 °C for 1 hour and 120 °C for 15 hours. Water and ethyl acetate were added to the reaction mixture and the aqueous layer was extracted with ethyl acetate. The combined organic layer was washed with a saturated sodium chloride solution, followed by drying over sodium sulfate. After evaporation, the residue was purified by column chromatography (chloroform:ethanol), thereby obtaining 3-methyl-8-nitro-6-(trifluoromethyl)imidazo[1,2-a]pyridine.

### Step 3:

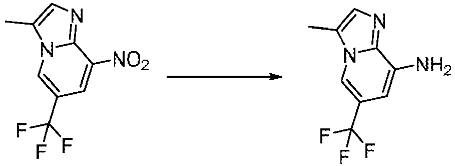

A solution of 3-methyl-8-nitro-6-(trifluoromethyl)imidazo[1,2-a]pyridine (1.8 g) obtained in step 2 and palladium on carbon (10wt%, 780 mg) in ethanol (30 mL) was stirred at 30 °C under H₂ atmosphere for 30 minutes. The insoluble matter was filtered off, followed by evaporating the solvent under reduced pressure. The obtained residue was purified by column chromatography (hexane:ethyl acetate), thereby obtaining 3-methyl-6-(trifluoromethyl)imidazo[1,2-a]pyridin-8-amine.

### Step 4:

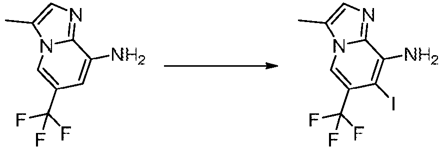

A solution of 3-methyl-6-(trifluoromethyl)imidazo[1,2-a]pyridin-8-amine (650 mg) obtained in step 3, N-iodosuccinimide (1.7 g) and TFA (0.67 mL) in THF (12 mL) was stirred at room temperature for 30 minutes. The mixture was diluted with ethyl acetate and washed with a saturated sodium chloride solution, followed by drying over sodium sulfate. After evaporation, the residue was purified by column chromatography (hexane:ethyl acetate), thereby obtaining 7-iodo-3-methyl-6-(trifluoromethyl)imidazo[1,2-a]pyridin-8-amine.

### Step 5:

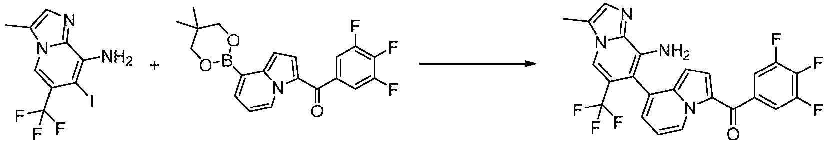

A solution of 7-iodo-3-methyl-6-(trifluoromethyl)imidazo[1,2-a]pyridin-8-amine (400 mg) obtained in step 4, (8-(5,5-dimethyl-1,3,2-dioxaborinan-2-yl)indolizin-3-yl)(3,4,5-trifluorophenyl)methanone (817 mg), SPhos Pd G3 (124 mg) in THF (3.00 mL) and 2M K₃PO₄ aq. (1.47 mL) was evacuated and N₂ back-filled before heating to 90 °C for 3 hours using microwave. After cooling, the reaction mixture was diluted with EtOAc. The resulting suspension was filtered, rinsing with EtOAc, and the filtrate was washed with H₂O and brine then dried over Na₂SO₄ and evaporated. The residue was purified by column chromatography on silica gel (hexane:acetone), thereby obtaining (8-(8-amino-3-methyl-6-(trifluoromethyl)imidazo[1,2-a]pyridin-7-yl)indolizin-3-yl)(3,4,5-trifluorophenyl)methanone.

### Step 6:

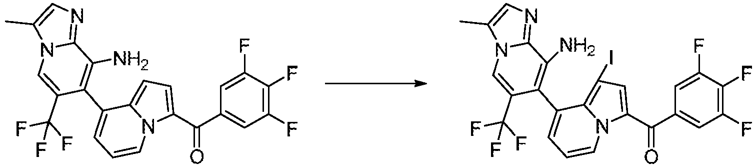

N-iodosuccinimide (1.26 g) was added to a solution of (8-(8-amino-3-methyl-6-(trifluoromethyl)imidazo[1,2-a]pyridin-7-yl)indolizin-3-yl)(3,4,5-trifluorophenyl)methanone (2.60 g) and TFA (1.38 mL) in THF (50 mL). After stirring at room temperature for 30 minutes, the reaction mixture was diluted with EtOAc, washed with sat. NaHCO₃ aq., H₂O (2x) and brine then dried over Na₂SO₄ and evaporated. The residue was purified by column chromatography on silica gel (hexane:ethyl acetate), thereby obtaining (8-(8-amino-3-methyl-6-(trifluoromethyl)imidazo[1,2-a]pyridin-7-yl)-1-iodoindolizin-3-yl)(3,4,5-trifluorophenyl)methanone.

### Step 7:

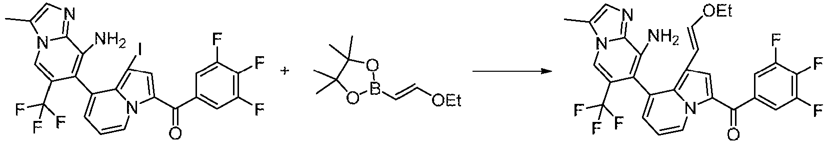

A solution of (8-(8-amino-3-methyl-6-(trifluoromethyl)imidazo[1,2-a]pyridin-7-yl)-1-iodoindolizin-3-yl)(3,4,5-trifluorophenyl)methanone (2.20 g), 2-[(E)-2-ethoxyvinyl]-4,4,5,5-tetramethyl-1,3,2-dioxaborolane (3.53 mL), SPhos Pd G3 (141 mg) and 2M K₃PO₄ aq. (1.47 mL) in 1,4-dioxane (40.0 mL) was evacuated and N₂ back-filled before heating to 95 °C for 3 hours. After cooling, the reaction mixture was diluted with ethyl acetate, washed with H₂O and brine then dried over Na₂SO₄ and evaporated. The residue was purified by column chromatography on silica gel (hexane:ethyl acetate), thereby obtaining (E)-(8-(8-amino-3-methyl-6-(trifluoromethyl)imidazo[1,2-a]pyridin-7-yl)-1-(2-ethoxyvinyl)indolizin-3-yl)(3,4,5-trifluorophenyl)methanone.

### Step 8:

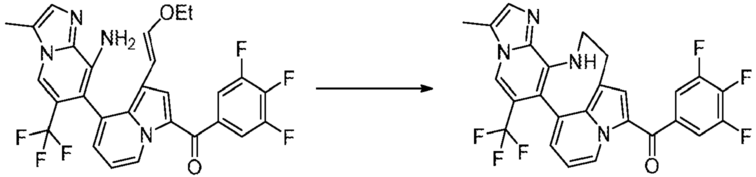

Sodium triacetoxyborohydride (1.29 g) was added to a solution of (E)-(8-(8-amino-3-methyl-6-(trifluoromethyl)imidazo[1,2-a]pyridin-7-yl)-1-(2-ethoxyvinyl)indolizin-3-yl)(3,4,5-trifluorophenyl)methanone (1.70 g) and TFA (2.26 mL) in dichloromethane (50 mL). After stirring at room temperature for 30 minutes, the reaction mixture was diluted with chloroform, washed with sat. NaHCO₃ aq., H₂O (2x) and brine then dried over Na₂SO₄ and evaporated. The residue was purified by column chromatography on silica gel (ethyl acetate:ethanol), thereby obtaining (10-methyl-7-(trifluoromethyl)-14,15-dihydro-13H-imidazo[1",2":1',2']pyrido[3',4':2,3]azocino[4,5,6-hi]indolizin-2-yl)(3,4,5-trifluorophenyl)methanone.

### Step 9:

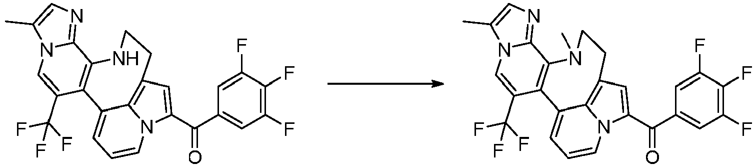

0.3M sodium cyanoborohydride-1/2 zinc chloride in methanol (12.2 mL) was added to a solution of (10-methyl-7-(trifluoromethyl)-14,15-dihydro-13H-imidazo[1",2":1',2']pyrido[3',4':2,3]azocino[4,5,6-hi]indolizin-2-yl)(3,4,5-trifluorophenyl)methanone (940 mg) and 36% formaldehyde solution in water (457 µL) in THF (20 mL). After stirring at room temperature for 30 minutes, the reaction mixture was diluted with ethyl acetate, washed with sat. NaHCO₃ aq., H₂O (2x) and brine then dried over Na₂SO₄ and evaporated. The residue was purified by column chromatography on silica gel (hexane:ethyl acetate), thereby obtaining (10,13-dimethyl-7-(trifluoromethyl)-14,15-dihydro-13H-imidazo[1",2":1',2']pyrido[3',4':2,3]azocino[4,5,6-hi]indolizin-2-yl)(3,4,5-trifluorophenyl)methanone.

### Step 10:

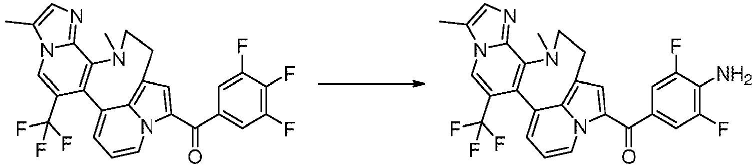

A solution of (10,13-dimethyl-7-(trifluoromethyl)-14,15-dihydro-13H-imidazo[1",2":1',2']pyrido[3',4':2,3]azocino[4,5,6-hi]indolizin-2-yl)(3,4,5-trifluorophenyl)methanone (850 mg) obtained in step 9 and 28% ammonia solution in water (10 mL) in 1,4-dioxane (10 mL) was stirred at 140 °C for 12 hours using microwave irradiation. After dilution with ethyl acetate and water, the mixture was extracted with ethyl acetate. The combined organic layer was washed with water and a saturated sodium chloride solution, followed by drying over sodium sulfate. After evaporation, the obtained residue was purified by column chromatography (hexane:ethyl acetate), thereby obtaining (4-amino-3,5-difluorophenyl)(10,13-dimethyl-7-(trifluoromethyl)-14,15-dihydro-13H-imidazo[1",2":1',2']pyrido[3',4':2,3]azocino[4,5,6-hi]indolizin-2-yl)methanone.

### Step 11:

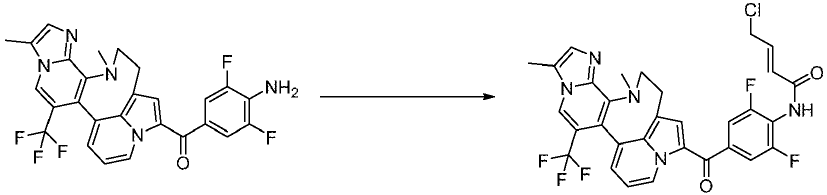

(E)-4-chlorobut-2-enoic acid (203 mg) and 1-propanephosphonic acid anhydride cyclic trimer (a 1.7M THF solution, 2.02 mL) were added to a solution of (4-amino-3,5-difluorophenyl)(10,13-dimethyl-7-(trifluoromethyl)-14,15-dihydro-13H-imidazo[1",2":1',2']pyrido[3',4':2,3]azocino[4,5,6-hi]indolizin-2-yl)methanone (685 mg) in DMF (8 mL), followed by stirring at room temperature for 20 minutes. Triethylamine (719 µL) was added to the reaction mixture. A reaction was performed at room temperature for 2 hours. After water was added to the reaction mixture, extraction was performed with ethyl acetate, followed by washing the organic layer with a saturated sodium chloride solution. The washed organic layer was dried over sodium sulfate. After evaporation, the obtained residue was purified by column chromatography (chloroform:ethanol), thereby obtaining (E)-4-chloro-N-(4-(10,13-dimethyl-7-(trifluoromethyl)-14,15-dihydro-13H-imidazo[1",2":1',2']pyrido[3',4':2,3]azocino[4,5,6-hi]indolizine-2-carbonyl)-2,6-difluorophenyl)but-2-enamide.

### Step 12:

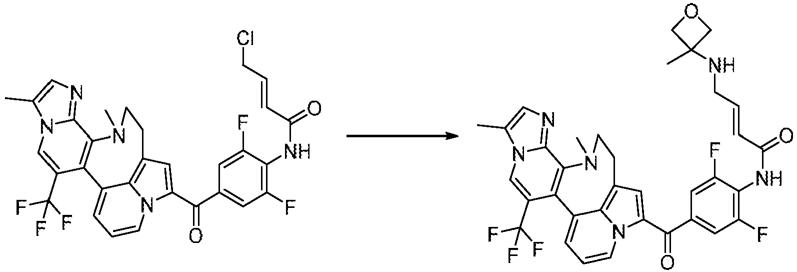

3-methyloxetan-3-amine (24 µL) and potassium iodide (13.1 mg) were added to a solution of (E)-4-chloro-N-(4-(10,13-dimethyl-7-(trifluoromethyl)-14,15-dihydro-13H-imidazo[1",2":1',2']pyrido[3',4':2,3]azocino[4,5,6-hi]indolizine-2-carbonyl)-2,6-difluorophenyl)but-2-enamide (33.0 mg) in DMSO (800 µL). A reaction was performed at room temperature for 2 hours. After water was added to the reaction mixture, extraction was performed with ethyl acetate. The combined organic layer was washed with a saturated sodium chloride solution. After the washed layer was dried over sodium sulfate, the solvent was evaporated under reduced pressure. The obtained residue was purified by column chromatography (chloroform:ethanol), thereby obtaining the racemic (E)-N-(4-(10,13-dimethyl-7-(trifluoromethyl)-14,15-dihydro-13H-imidazo[1",2":1',2']pyrido[3',4':2,3]azocino[4,5,6-hi]indolizine-2-carbonyl)-2,6-difluorophenyl)-4-((3-methyloxetan-3-yl)amino)but-2-enamide. The racemate was separated by CHIRALPAK IA (hexane:EtOH (0.1% Et₃N)) to give (E)-N-(4-(10,13-dimethyl-7-(trifluoromethyl)-14,15-dihydro-13H-imidazo[1",2":1',2']pyrido[3',4':2,3]azocino[4,5,6-hi]indolizine-2-carbonyl)-2,6-difluorophenyl)-4-((3-methyloxetan-3-yl)amino)but-2-enamide (2nd-eluting isomer). ¹H-NMR: (400 MHz, CHLOROFORM-D) δ 9.99 (dd, J = 7.0, 1.0 Hz, 1H), 8.36 (s, 1H), 7.57 (d, J = 0.8 Hz, 1H), 7.42 (d, J = 8.0 Hz, 2H), 7.18-7.09 (m, 3H), 7.02 (t, J = 7.1 Hz, 1H), 6.34 (d, J = 15.3 Hz, 1H), 4.59 (d, J = 6.3 Hz, 2H), 4.44 (d, J = 6.5 Hz, 2H), 3.56-3.53 (m, 2H), 3.06 (d, J = 10.5 Hz, 3H), 2.90-2.83 (m, 1H), 2.65-2.61 (m, 3H), 2.59-2.57 (m, 3H), 2.50-2.42 (m, 1H), 1.51 (s, 3H); ESI-MS [M+H]⁺: 679.

### Example 200

### (E)-N-(2,6-difluoro-4-(7-methoxy-9-(2-methoxyethyl)-10,12-dimethyl-9,12,13,14-tetrahydroimidazo[4",5":5',6']benzo[1',2':2,3]azocino[4,5,6-hi]indolizine-2-carbonyl)phenyl)-4-((1-methylcyclopropyl)amino)but-2-enamide

### Steps 1-3:

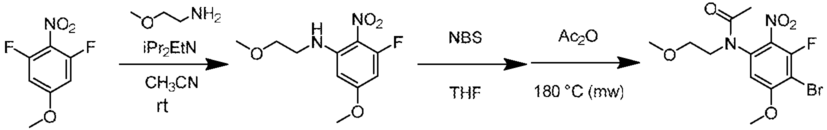

2-Methoxyethanamine (1.01 mL, 11.6 mmol) and N-ethyl-N-isopropyl-propan-2-amine (4.06 mL, 23.3 mmol) were added to a solution of 1,3-difluoro-5-methoxy-2-nitrobenzene (2.00 g, 10.6 mmol) in acetonitrile (53 mL). After stirring at room temperature for 3 hours, 2-methoxyethanamine (0.202 mL, 2.32 mmol) was added and the mixture was stirred at room temperature for 20 minutes. Water and EtOAc were added to the resulting mixture and the organic layer was separated, and washed with brine, followed by drying over Na₂SO₄. After evaporation, the obtained residue was purified by column chromatography on silica gel (hexane-acetone) to obtain 3-fluoro-5-methoxy-N-(2-methoxyethyl)-2-nitroaniline (1.95 g). 3-fluoro-5-methoxy-N-(2-methoxyethyl)-2-nitroaniline (1.95 g) was diluted in THF (40 mL), and NBS (1.42 g, 7.98 mmol) was added thereto at 0 °C. After stirring for 30 minutes, water, saturated aqueous NaHCO₃ and EtOAc were added to the resulting mixture and the organic layer was separated, and washed successively with H₂O, saturated aqueous NaHCO₃ then brine, followed by drying over Na₂SO₄, and evaporated. The residue was suspended in diisopropylether (20 mL) and the suspension was stirred at room temperature for 40 minutes. The precipitate was collected via filtration, washing with diisopropylether, and the solid dried *in vacuo* to give crude 4-bromo-3-fluoro-5-methoxy-N-(2-methoxyethyl)-2-nitroaniline (2.42 g). The mixure of crude 4-bromo-3-fluoro-5-methoxy-N-(2-methoxyethyl)-2-nitroaniline (700 mg) and acetic anhydride (3.92 mL) was heated at 180 °C by microwave irradiation for 12 hours. After cooling, the mixture was diluted with MeOH, and evaporated. Water, saturated aqueous NaHCO₃ and EtOAc were added to the residue and the organic layer was separated, and washed successively with H₂O, saturated aqueous NaHCO₃ then brine, followed by drying over Na₂SO₄, and evaporated. The residue was purified by column chromatography on silica gel (hexane-acetone) to obtain N-(4-bromo-3-fluoro-5-methoxy-2-nitrophenyl)-N-(2-methoxyethyl)acetamide.

### Step 4:

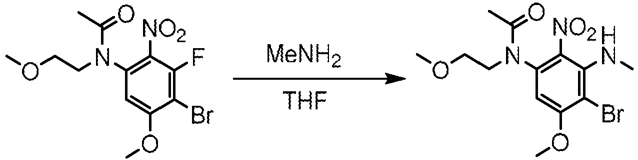

To the mixture of N-(4-bromo-3-fluoro-5-methoxy-2-nitrophenyl)-N-(2-methoxyethyl)acetamide (700 mg) in THF (3.8 mL) was added methylamine (2M THF solution, 2.88 mL). After stirring at room temperature for 20 minutes, water and EtOAc were added to the resulting mixture. The organic layer was separated, and washed with aqueous NaHCO₃ and brine, followed by drying over Na₂SO₄. After evaporation, the residue was purified by column chromatography on silica gel (hexane-acetone) to obtain N-(4-bromo-5-methoxy-3-(methylamino)-2-nitrophenyl)-N-(2-methoxyethyl)acetamide.

### Step 5:

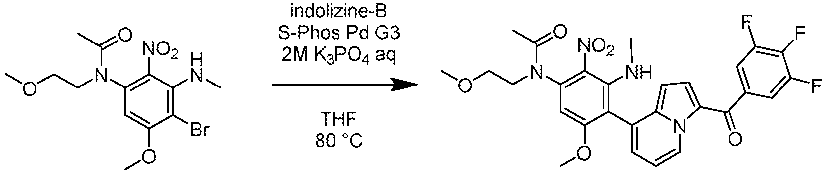

A mixture of N-(4-bromo-5-methoxy-3-(methylamino)-2-nitrophenyl)-N-(2-methoxyethyl)acetamide (652 mg, 1.73 mmol), (8-(5,5-dimethyl-1,3,2-dioxaborinan-2-yl)indolizin-3-yl)(3,4,5-trifluorophenyl)methanone (872 mg, 2.25 mmol) and S-Phos Pd G3 (122 mg) in THF (17 mL) and 2M K₃PO₄ aq. (2.60 mL, 5.20 mmol) was evacuated and N₂ back-filled before heating to 80 °C by microwave irradiation for 3 hours. After cooling, EtOAc, water and saturated aqueous NaCl were added and the insoluble matters were removed by filtration. The organic layer was dried over Na₂SO₄. After evaporation, the resulting residue was purified by column chromatography on silica gel (hexane-acetone) to obtain N-(5-methoxy-3-(methylamino)-2-nitro-4-(3-(3,4,5-trifluorobenzoyl)indolizin-8-yl)phenyl)-N-(2-methoxyethyl)acetamide.

### Step 6:

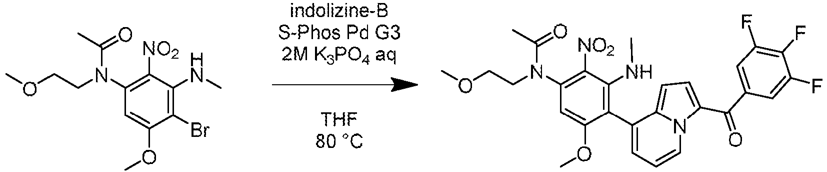

To a solution of N-(5-methoxy-3-(methylamino)-2-nitro-4-(3-(3,4,5-trifluorobenzoyl)indolizin-8-yl)phenyl)-N-(2-methoxyethyl)acetamide (987 mg, 1.73 mmol) in ethanol (17 mL) and THF (31 mL) was added palladium hydroxide on carbon (1.00 g, 20 wt. % loading (dry basis)). The mixture was stirred at room temperature for 24 hours and 40 °C for 46 hours under hydrogen atmosphere. After cooling, the mixture was filtered, rinsing with CHCl₃ and MeOH, and evaporated. The residue was purified by column chromatography on silica gel (hexane-acetone) to give (8-(6-methoxy-1-(2-methoxyethyl)-2-methyl-4-(methylamino)-1H-benzo[d]imidazol-5-yl)indolizin-3-yl)(3,4,5-trifluorophenyl)methanone.

### Step 7:

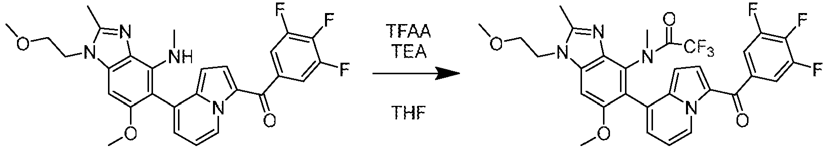

To a solution of (8-(6-methoxy-1-(2-methoxyethyl)-2-methyl-4-(methylamino)-1H-benzo[d]imidazol-5-yl)indolizin-3-yl)(3,4,5-trifluorophenyl)methanone (740 mg, 1.42 mmol) in THF (7.00 mL) was added triethylamine (0.773 mL, 5.66 mmol) at room temperature and trifluoroacetic anhydride (0.394 mL, 2.83 mmol) at 0 °C. After stirring at room temperature for 1 hour, to this solution was added triethylamine (0.193 mL, 1.42 mmol) at room temperature and trifluoroacetic anhydride (0.099 mL, 0.708 mmol) at 0 °C. After stirring at room temperature for 30 minutes, to this solution was added triethylamine (0.193 mL, 1.42 mmol) at room temperature and trifluoroacetic anhydride (0.099 mL, 0.708 mmol) at 0 °C additionally. After stirring at room temperature for 15 minutes, the mixture was diluted with EtOAc, and sat. NaHCO₃ aq. and water. The organic layer was separated, and washed with brine, followed by drying over Na₂SO₄. The solvent was evaporated to obtain crude 2,2,2-trifluoro-N-(6-methoxy-1-(2-methoxyethyl)-2-methyl-5-(3-(3,4,5-trifluorobenzoyl)indolizin-8-yl)-1H-benzo[d]imidazol-4-yl)-N-methylacetamide.

### Step 8:

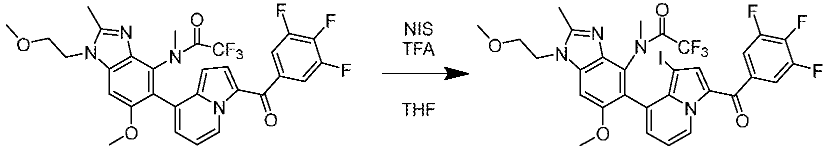

To a solution of crude 2,2,2-trifluoro-N-(6-methoxy-1-(2-methoxyethyl)-2-methyl-5-(3-(3,4,5-trifluorobenzoyl)indolizin-8-yl)-1H-benzo[d]imidazol-4-yl)-N-methylacetamide (849 mg) in THF (14 mL) was added N-iodosuccinimide (309 mg, 1.37 mmol) and TFA (0.255 mL, 3.43 mmol). After stirring at room temperature for 1 hour, the mixture was diluted with EtOAc and water. The organic layer was separated, and washed with water, 20% Na₂S₂O₃ aq., sat. NaHCO₃ aq., and brine, followed by drying over Na₂SO₄. The solvent was evaporated to obtain crude 2,2,2-trifluoro-N-(5-(1-iodo-3-(3,4,5-trifluorobenzoyl)indolizin-8-yl)-6-methoxy-1-(2-methoxyethyl)-2-methyl-1H-benzo[d]imidazol-4-yl)-N-methylacetamide.

### Step 9:

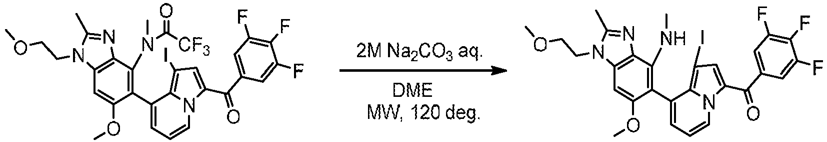

To the mixture of crude 2,2,2-trifluoro-N-(5-(1-iodo-3-(3,4,5-trifluorobenzoyl)indolizin-8-yl)-6-methoxy-1-(2-methoxyethyl)-2-methyl-1H-benzo[d]imidazol-4-yl)-N-methylacetamide (1.0 g) in 1,2-dimethoxyethane (14 mL) was added 2M Na₂CO₃ aq. (7.1 mL, 14 mmol). The reaction mixture was heated at 120 °C by microwave irradiation for 60 hours. After cooling, the mixture was diluted with EtOAc and water. The organic layer was separated, and washed with brine then dried over Na₂SO₄ and evaporated. The residue was purified by column chromatography on silica gel (hexane-acetone) to give (1-iodo-8-(6-methoxy-1-(2-methoxyethyl)-2-methyl-4-(methylamino)-1H-benzo[d]imidazol-5-yl)indolizin-3-yl)(3,4,5-trifluorophenyl)methanone.

### Steps 10-14:

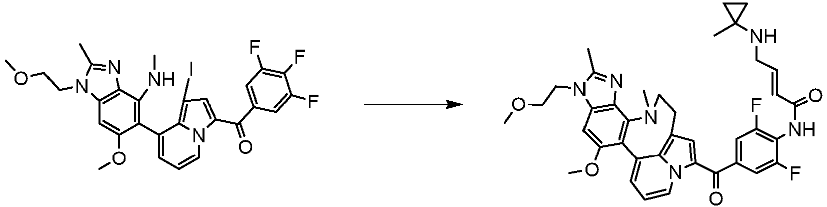

The procedures of following steps were performed in the same manner as Example 140 (Steps 8-12) using (1-iodo-8-(6-methoxy-1-(2-methoxyethyl)-2-methyl-4-(methylamino)-1H-benzo[d]imidazol-5-yl)indolizin-3-yl)(3,4,5-trifluorophenyl)methanone obtained in Step 9.

The racemate was separated by CHIRALPAK-IG (hexane-EtOH(0.1% v/v triethylamine)) to obtain (E)-N-(2,6-difluoro-4-(7-methoxy-9-(2-methoxyethyl)-10,12-dimethyl-9,12,13,14-tetrahydroimidazo[4",5":5',6']benzo[1',2':2,3]azocino[4,5,6-hi]indolizine-2-carbonyl)phenyl)-4-((1-methylcyclopropyl)amino)but-2-enamide as the second-eluting isomer. ¹H-NMR: (400 MHz, CHLOROFORM-D) δ 10.00 (dd, J = 6.96, 1.00 Hz, 1H), 7.36 (d, J = 7.92 Hz, 2H), 7.30-7.24 (m, 2H), 7.16-7.08 (m, 2H), 7.03 (t, J = 7.08 Hz, 1H), 6.81 (s, 1H), 6.25 (d, J = 15.38 Hz, 1H), 4.33-4.22 (m, 2H), 3.77 (s, 3H), 3.73 (t, J = 5.45 Hz, 2H), 3.56-3.51 (m, 3H), 3.35 (s, 3H), 3.06 (s, 3H), 2.90-2.83 (m, 1H), 2.63 (s, 3H), 2.58-2.43 (m, 2H), 1.30 (s, 3H), 0.63-0.60 (m, 2H), 0.42-0.40 (m, 2H); ESI-MS [M+H]⁺: 683.

### Example 217

### (S,E)-N-(4-(10,13-dimethyl-7-(trifluoromethyl)-14,15-dihydro-13H-[1,2,4]triazolo[4",3":1',2']pyrido[3',4':2,3]azocino[4,5,6-hi]indolizine-2-carbonyl)-2,6-difluorophenyl)-4-((tetrahydrofuran-3-yl)amino)but-2-enamide

### Step 1:

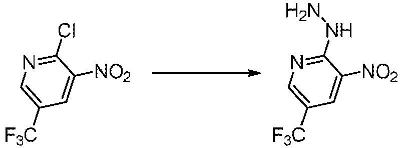

Hydrazine monohydrate (4.5 mL) was added to 2-chloro-3-nitro-5-(trifluoromethyl)pyridine (10 g) in EtOH (200 mL), and the reaction mixture was stirred at 30 °C for 2 hours. After evaporation, the mixture was diluted with ethyl acetate. The mixture was washed with water and a saturated sodium chloride solution, followed by drying over sodium sulfate. The solvent was evaporated under reduced pressure, thereby obtaining crude 2-hydrazineyl-3-nitro-5-(trifluoromethyl)pyridine.

### Step 2:

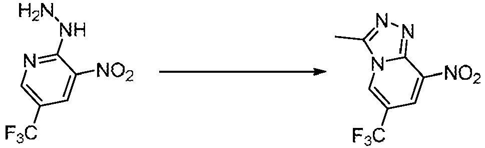

Trifluoroacetic acid was added to crude 2-hydrazineyl-3-nitro-5-(trifluoromethyl)pyridine (5.05 g) obtained in step 1 and 1,1,1-trimethoxyethane (13.5 mL) in ethanol (20.0 mL), and the reaction mixture was stirred at 100 °C for 2 hours. After dilution with ethyl acetate, the mixture was washed with a saturated sodium bicarbonate solution and a saturated sodium chloride solution, followed by drying over sodium sulfate. After evaporation, the residue was dissolved in EtOAc (10.0 mL) at 50 °C and heptane (10.0 mL) was added then cooled to ambient temperature slowly to give the precipitaion. The precipitate was collected by filtration, washing with further heptane, and vacuum-dried at 60 °C for 2 hours, thereby obtaining 3-methyl-8-nitro-6-(trifluoromethyl)-[1,2,4]triazolo[4,3-a]pyridine.

### Step 3:

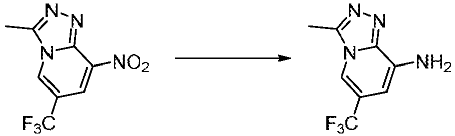

A solution of 3-methyl-8-nitro-6-(trifluoromethyl)-[1,2,4]triazolo[4,3-a]pyridine (3.90 g) obtained in step 2 and palladium hydroxide on carbon (20 wt%, 1.70 g) in ethanol (30.0 mL) was stirred at 30 °C under H₂ atmosphere for 30 minutes. The insoluble matter was filtered off, followed by evaporating the solvent under reduced pressure. The obtained residue was purified by column chromatography (hexane:ethyl acetate), thereby obtaining 3-methyl-6-(trifluoromethyl)-[1,2,4]triazolo[4,3-a]pyridin-8-amine.

### Step 4:

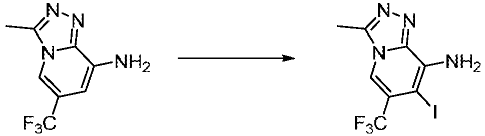

A solution of 3-methyl-6-(trifluoromethyl)-[1,2,4]triazolo[4,3-a]pyridin-8-amine (2.92 g) obtained in step 3, N-iodosuccinimide (4.47 g) and TFA (1.20 mL) in *N,N-*dimethylformamide (25 mL) was stirred at room temperature for 30 minutes. The mixture was diluted with ethyl acetate and washed with a saturated sodium chloride solution, followed by drying over sodium sulfate. After evaporation, the residue was purified by column chromatography (hexane:ethyl acetate), thereby obtaining 7-iodo-3-methyl-6-(trifluoromethyl)-[1,2,4]triazolo[4,3-a]pyridin-8-amine.

### Step 5:

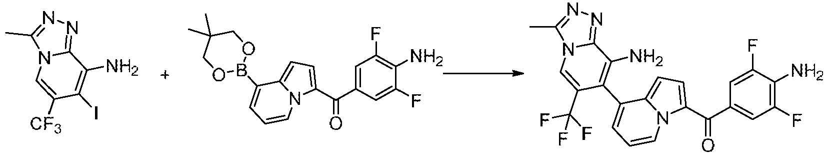

A solution of 7-iodo-3-methyl-6-(trifluoromethyl)-[1,2,4]triazolo[4,3-a]pyridin-8-amine (1.60 g) obtained in step 4, (4-amino-3,5-difluorophenyl)(8-(5,5-dimethyl-1,3,2-dioxaborinan-2-yl)indolizin-3-yl)methanone (2.88 g), SPhos Pd G3 (494 mg) and 2M K₃PO₄ aq. (5.84 mL) in THF (33.0 mL) was evacuated and N₂ back-filled before heating to 120 °C for 30 minutes using microwave. After cooling, the reaction mixture was diluted with EtOAc. The resulting suspension was filtered, rinsing with EtOAc, and the filtrate was washed with H₂O and brine then dried over Na₂SO₄ and evaporated. The residue was purified by column chromatography on silica gel (hexane:acetone), thereby obtaining (4-amino-3,5-difluorophenyl)(8-(8-amino-3-methyl-6-(trifluoromethyl)-[1,2,4]triazolo[4,3-a]pyridin-7-yl)indolizin-3-yl)methanone.

### Step 6:

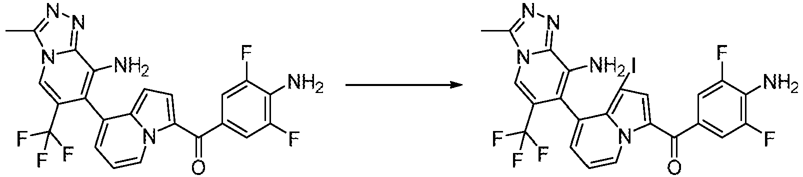

N-iodosuccinimide (2.60 g) was added to a solution of (4-amino-3,5-difluorophenyl)(8-(8-amino-3-methyl-6-(trifluoromethyl)-[1,2,4]triazolo[4,3-a]pyridin-7-yl)indolizin-3-yl)methanone (1.75 g) and TFA (1.55 mL) in THF (30 mL). After stirring at room temperature for 30 minutes, the reaction mixture was diluted with EtOAc, washed with sat. NaHCO₃ aq., H₂O (2x) and brine then dried over Na₂SO₄ and evaporated. The residue was purified by column chromatography on silica gel (hexane:ethyl acetate), thereby obtaining (4-amino-3,5-difluorophenyl)(8-(8-amino-3-methyl-6-(trifluoromethyl)-[1,2,4]triazolo[4,3-a]pyridin-7-yl)-1-iodoindolizin-3-yl)methanone.

### Step 7:

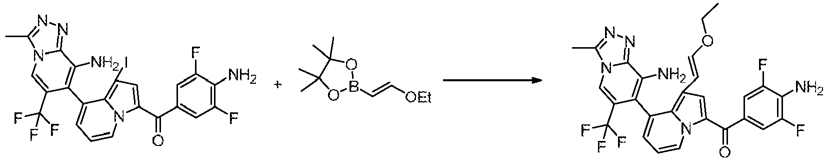

A solution of (4-amino-3,5-difluorophenyl)(8-(8-amino-3-methyl-6-(trifluoromethyl)-[1,2,4]triazolo[4,3-a]pyridin-7-yl)-1-iodoindolizin-3-yl)methanone (1.90 g), 2-[(E)-2-ethoxyvinyl]-4,4,5,5-tetramethyl-1,3,2-dioxaborolane (2.96 mL), Pd(PPh₃)₄ (323 mg) and 2M Na₂CO₃ aq. (6.98 mL) in 1,4-dioxane (35.0 mL) was evacuated and N₂ back-filled before heating to 95 °C for 3 hours. After cooling, the reaction mixture was diluted with ethyl acetate, washed with H₂O and brine then dried over Na₂SO₄ and evaporated. The residue was purified by column chromatography on silica gel (hexane:ethyl acetate), thereby obtaining (E)-(4-amino-3,5-difluorophenyl)(8-(8-amino-3-methyl-6-(trifluoromethyl)-[1,2,4]triazolo[4,3-a]pyridin-7-yl)-1-(2-ethoxyvinyl)indolizin-3-yl)methanone.

### Step 8:

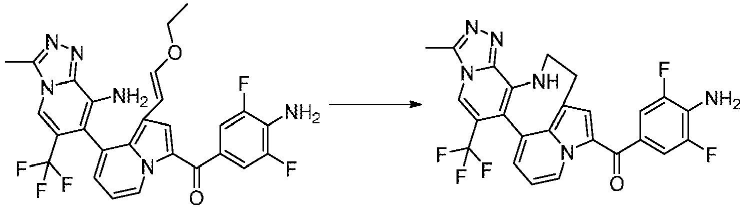

Sodium triacetoxyborohydride (983 mg) was added to a solution of (E)-(4-amino-3,5-difluorophenyl)(8-(8-amino-3-methyl-6-(trifluoromethyl)-[1,2,4]triazolo[4,3-a]pyridin-7-yl)-1-(2-ethoxyvinyl)indolizin-3-yl)methanone (1.29 g) and TFA (1.72 mL) in dichloromethane (50 mL). After stirring at room temperature for 30 minutes, the reaction mixture was diluted with chloroform, washed with sat. NaHCO₃ aq., H₂O (2x) and brine then dried over Na₂SO₄ and evaporated. The residue was purified by column chromatography on silica gel (ethyl acetate:ethanol), thereby obtaining (4-amino-3,5-difluorophenyl)(10-methyl-7-(trifluoromethyl)-14,15-dihydro-13H-[1,2,4]triazolo[4",3":1',2']pyrido[3',4':2,3]azocino[4,5,6-hi]indolizin-2-yl)methanone.

### Step 9:

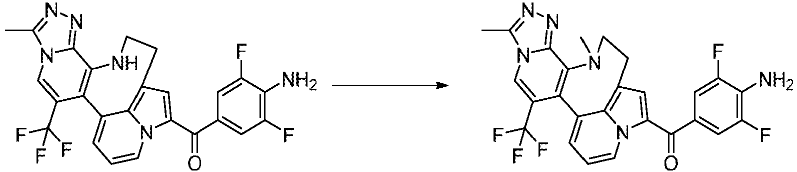

A solution containing sodium cyanoborohyride (55.0 mg) and zinc chloride (60.0 mg) in methanol (2.94 mL) was added to a solution of (4-amino-3,5-difluorophenyl)(10-methyl-7-(trifluoromethyl)-14,15-dihydro-13H-[1,2,4]triazolo[4",3": 1',2']pyrido[3',4':2,3]azocino[4,5,6-hi]indolizin-2-yl)methanone (211 mg), formaldehyde (36% w/v aq. solution, 110 µL) and TFA (65.5 µL) in THF (2.0 mL). After stirring at room temperature for 30 minutes, the reaction mixture was diluted with ethyl acetate, washed with sat. NaHCO₃ aq., H₂O (2x) and brine then dried over Na₂SO₄ and evaporated. The residue was purified by column chromatography on silica gel (hexane:ethyl acetate), thereby obtaining (4-amino-3,5-difluorophenyl)(10,13-dimethyl-7-(trifluoromethyl)-14,15-dihydro-13H-[1,2,4]triazolo[4",3":1',2']pyrido[3',4':2,3]azocino[4,5,6-hi]indolizin-2-yl)methanone.

### Step 10:

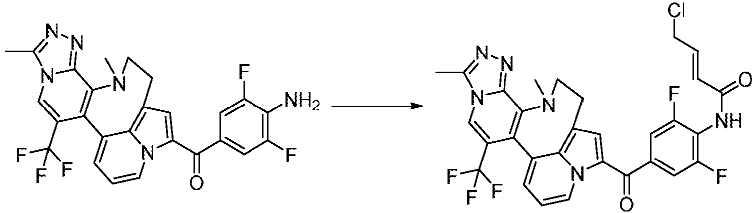

(E)-4-chlorobut-2-enoic acid (39 mg) and 1-propanephosphonic acid anhydride cyclic trimer (1.7 M solution in THF, 390 µL) were added to a solution of (4-amino-3,5-difluorophenyl)(10,13-dimethyl-7-(trifluoromethyl)-14,15-dihydro-13H-[1,2,4]triazolo[4",3":1',2']pyrido[3',4':2,3]azocino[4,5,6-hi]indolizin-2-yl)methanone (130 mg) in N,N-dimethylformamide (1.0 mL), followed by stirring at room temperature for 20 minutes. Triethylamine (137 µL) was added to the reaction mixture. After 2 h, water was added to the reaction mixture, extraction was performed with ethyl acetate, followed by washing the organic layer with a saturated sodium chloride solution. The washed organic layer was dried over sodium sulfate. After evaporation, the obtained residue was purified by column chromatography (chloroform:ethanol), thereby obtaining (E)-4-chloro-N-(4-(10,13-dimethyl-7-(trifluoromethyl)-14,15-dihydro-13H-[1,2,4]triazolo[4",3":1',2']pyrido[3',4':2,3]azocino[4,5,6-hi]indolizine-2-carbonyl)-2,6-difluorophenyl)but-2-enamide.

### Step 11:

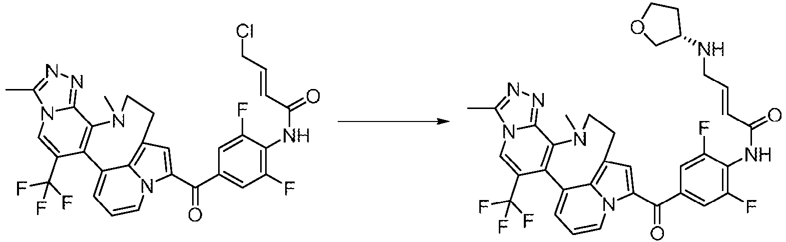

(S)-Tetrahydrofuran-3-amine (11.2 µL) and potassium iodide (6.33 mg) were added to a solution of (E)-4-chloro-N-(4-(10,13-dimethyl-7-(trifluoromethyl)-14,15-dihydro-13H-[1,2,4]triazolo[4",3":1',2']pyrido[3',4':2,3]azocino[4,5,6-hi]indolizine-2-carbonyl)-2,6-difluorophenyl)but-2-enamide (16.0 mg) in DMSO (500 µL). After 2 h, water was added to the reaction mixture, extraction was performed with ethyl acetate. The combined organic layer was washed with a saturated sodium chloride solution. After the washed layer was dried over sodium sulfate, the solvent was evaporated under reduced pressure. The obtained residue was purified by column chromatography (chloroform:ethanol), thereby obtaining the racemic (S,E)-N-(4-(10,13-dimethyl-7-(trifluoromethyl)-14,15-dihydro-13H-[1,2,4]triazolo[4",3":1',2']pyrido[3',4':2,3]azocino[4,5,6-hi]indolizine-2-carbonyl)-2,6-difluorophenyl)-4-((tetrahydrofuran-3-yl)amino)but-2-enamide (9.43 mg). The racemate was separated by CHIRALPAK IA (hexane:EtOH (0.1% v/v triethylamine)) to give (S,E)-N-(4-(10,13-dimethyl-7-(trifluoromethyl)-14,15-dihydro-13H-[1,2,4]triazolo[4",3":1',2']pyrido[3',4':2,3]azocino[4,5,6-hi]indolizine-2-carbonyl)-2,6-difluorophenyl)-4-((tetrahydrofuran-3-yl)amino)but-2-enamide as the second-eluting isomer. ¹H-NMR: (400 MHz, CHLOROFORM-D) δ 10.00 (dd, J = 6.9, 1.1 Hz, 1H), 8.95 (s, 1H), 7.44 (dd, J = 10.5, 2.8 Hz, 2H), 7.17-7.00 (m, 4H), 6.32-6.28 (m, 1H), 3.97 (dd, J = 14.9, 7.9 Hz, 1H), 3.82 (qd, J = 8.8, 5.8 Hz, 2H), 3.65 (dd, J = 9.1, 3.6 Hz, 1H), 3.51-3.42 (m, 4H), 3.01 (s, 3H), 2.91-2.85 (m, 1H), 2.67 (s, 3H), 2.65-2.64 (m, 1H), 2.46-2.38 (m, 1H), 2.19-2.10 (m, 1H), 1.81-1.74 (m, 1H); ESI-MS [M+H]⁺: 680.

### Example 224

(E)-N-(4-(7-(difluoromethyl)-10-(methoxymethyl)-9,12-dimethyl-9,12,13,14-tetrahydroimidazo[4",5":5',6']benzo[1',2':2,3]azocino[4,5,6-hi]indolizine-2-carbonyl)-2,6-difluorophenyl)-4-((3-methyloxetan-3-yl)amino)but-2-enamide

### Step 1:

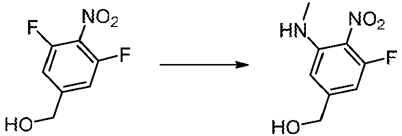

Methylamine (2 M solution in THF, 39.7 mL) was added to (3,5-difluoro-4-nitrophenyl)methanol (10.0 g) in THF (20.0 mL), and the reaction mixture was stirred at 30 °C for 2 hours. After evaporation, the mixture was diluted with ethyl acetate. The mixture was washed with water and a saturated sodium chloride solution, followed by drying over sodium sulfate. The solvent was evaporated under reduced pressure, thereby obtaining crude (3-fluoro-5-(methylamino)-4-nitrophenyl)methanol.

### Step 2:

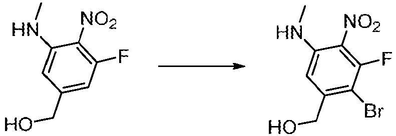

A solution of crude (3-fluoro-5-(methylamino)-4-nitrophenyl)methanol (10.3 g) obtained in step 1 and N-bromosuccinimide (9.62 g) in *N,N*-dimethylformamide (55.0 mL) was stirred at room temperature for 30 minutes. To the reaction mixture was added water (150 mL), and the reaction mixture was stirred at room temperature for 30 minutes. The precipitate was collected by filtration, washing with further water, and vacuum-dried at 60 °C for 16 hours, thereby obtaining (2-bromo-3-fluoro-5-(methylamino)-4-nitrophenyl)methanol.

### Step 3:

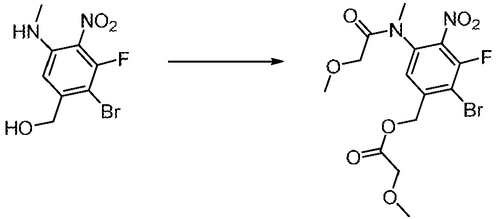

2-methoxyacetic anhydride (9.3 mL) was added to the (2-bromo-3-fluoro-5-(methylamino)-4-nitrophenyl)methanol (1.6 g), then the reaction mixture was heated at 100 °C by microwave irradiation for 3 hours. After cooling, the reaction mixture was diluted with EtOAc, 5M aq. NaOH, and NaHCO₃ aq. The organic layer was seperated and washed with NaHCO₃ aq. and brine then dried over Na₂SO₄ and evaporated. The residue was purified by column chromatography on silica gel (hexane:ethyl acetate), thereby obtaining 2-bromo-3-fluoro-5-(2-methoxy-N-methylacetamido)-4-nitrobenzyl 2-methoxyacetate.

### Step 4:

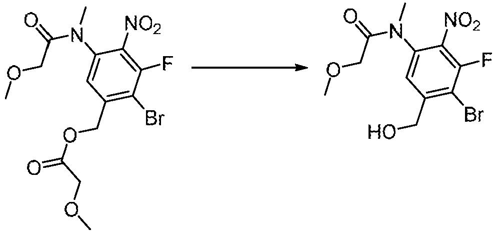

A solution of 2 M sodium hydroxide solution (10.0 mL) was added to a solution of 2-bromo-3-fluoro-5-(2-methoxy-N-methylacetamido)-4-nitrobenzyl 2-methoxyacetate (1.70 g) in ethanol (12.0 mL) and tetrahydrofuran (5.00 mL), followed by stirring at 50 °C for 2 hours. After cooling to room temperature, the reaction mixure was quenched with 5M aq. hydrochloric acid (4.00 mL). After dilution with ethyl acetate, the mixture was extracted with ethyl acetate. The combined organic layer was washed with water and a saturated sodium chloride solution, followed by drying over sodium sulfate. After evaporation, the residue was purified by column chromatography (chloroform:ethanol), thereby obtaining N-(4-bromo-3-fluoro-5-(hydroxymethyl)-2-nitrophenyl)-2-methoxy-N-methylacetamide.

### Step 5:

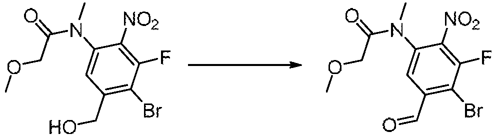

A solution of N-(4-bromo-3-fluoro-5-(hydroxymethyl)-2-nitrophenyl)-2-methoxy-N-methylacetamide (300 mg) obtained in step 4 and manganese dioxide (594 mg) in ethyl acetate (10.0 mL) was stirred at 60 °C for 14 hours. The insoluble matter was filtered off, followed by evaporating the solvent under reduced pressure. The obtained residue was purified by column chromatography (hexane:ethyl acetate), thereby obtaining N-(4-bromo-3-fluoro-5-formyl-2-nitrophenyl)-2-methoxy-N-methylacetamide.

### Step 6:

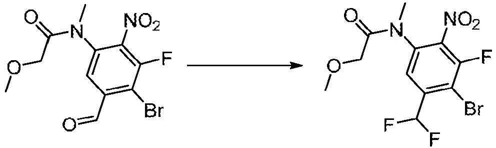

A solution of N-(4-bromo-3-fluoro-5-formyl-2-nitrophenyl)-2-methoxy-N-methylacetamide (600 mg) and bis(2-methoxyethyl)aminosulfur trifluoride (1.52 g) in CH₂Cl₂ (10.0 mL) was stirred at room temperature for 30 minutes. After dilution with chloroform, the mixture was washed with saturated NaHCO₃ aq. and a saturated sodium chloride solution, followed by drying over sodium sulfate. After evaporation, the obtained residue was purified by column chromatography (hexane:ethyl acetate), thereby obtaining N-(4-bromo-5-(difluoromethyl)-3-fluoro-2-nitrophenyl)-2-methoxy-N-methylacetamide.

### Step 7:

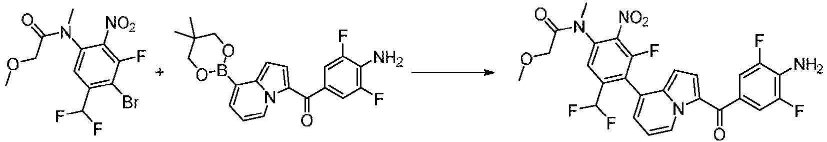

A solution of N-(4-bromo-5-(difluoromethyl)-3-fluoro-2-nitrophenyl)-2-methoxy-N-methylacetamide (520 mg) obtained in step 6, (8-(5,5-dimethyl-1,3,2-dioxaborinan-2-yl)indolizin-3-yl)(3,4,5-trifluorophenyl)methanone (700 mg), SPhos Pd G3 (98.7 mg) and 2M K₃PO₄ aq. (1.75 mL) in THF (10.0 mL) was evacuated and N₂ back-filled before heating to 70 °C for 1 hour using microwave. After cooling, the reaction mixture was diluted with EtOAc. The resulting suspension was filtered, rinsing with EtOAc, and the filtrate was washed with H₂O and brine then dried over Na₂SO₄ and evaporated. The residue was purified by column chromatography on silica gel (hexane:acetone), thereby obtaining N-(4-(3-(4-amino-3,5-difluorobenzoyl)indolizin-8-yl)-5-(difluoromethyl)-3-fluoro-2-nitrophenyl)-2-methoxy-N-methylacetamide.

### Step 8:

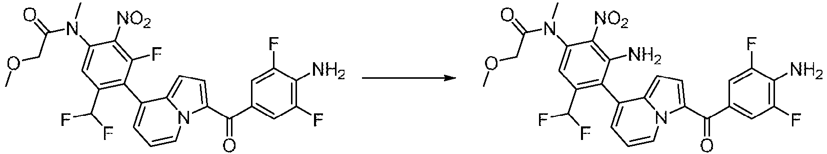

A solution of N-(4-(3-(4-amino-3,5-difluorobenzoyl)indolizin-8-yl)-5-(difluoromethyl)-3-fluoro-2-nitrophenyl)-2-methoxy-N-methylacetamide (550 mg) obtained in step 7, ammonia (28% w/v aq. solution, 5 mL) in 1,4-dioxane (10 mL) was stirred at 70 °C for 30 minutes using microwave irradiation. After dilution with ethyl acetate and water, the mixture was extracted with ethyl acetate. The combined organic layer was washed with water and a saturated sodium chloride solution, followed by drying over sodium sulfate. After evaporation, the obtained residue was purified by column chromatography (hexane:ethyl acetate), thereby obtaining N-(3-amino-4-(3-(4-amino-3,5-difluorobenzoyl)indolizin-8-yl)-5-(difluoromethyl)-2-nitrophenyl)-2-methoxy-N-methylacetamide.

### Step 9:

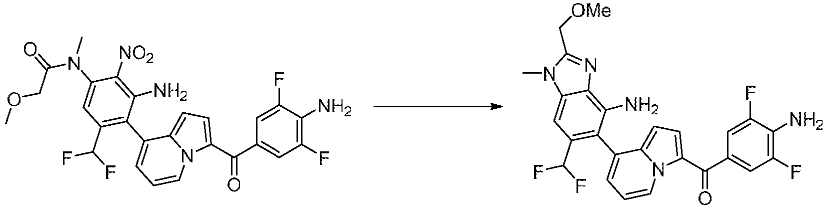

A solution of N-(3-amino-4-(3-(4-amino-3,5-difluorobenzoyl)indolizin-8-yl)-5-(difluoromethyl)-2-nitrophenyl)-2-methoxy-N-methylacetamide (360 mg) obtained in step 8 and palladium hydroxide on carbon (20 wt%, 300 mg) in ethanol (4.00 mL) was stirred at 30 °C under H₂ atmosphere for 15 hours. The insoluble matter was filtered off, followed by evaporating the solvent under reduced pressure. The obtained residue was purified by column chromatography (chloroform:ethanol), thereby obtaining (4-amino-3,5-difluorophenyl)(8-(4-amino-6-(difluoromethyl)-2-(methoxymethyl)-1-methyl-1H-benzo[d]imidazol-5-yl)indolizin-3-yl)methanone.

### Step 10:

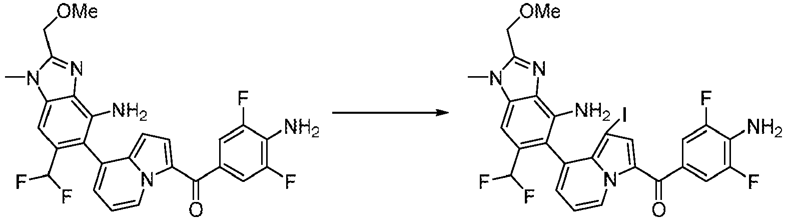

N-iodosuccinimide (87.1 mg) was added to a solution of (4-amino-3,5-difluorophenyl)(8-(4-amino-6-(difluoromethyl)-2-(methoxymethyl)-1-methyl-1H-benzo[d]imidazol-5-yl)indolizin-3-yl)methanone (180 mg) and TFA (1.31 µL) in THF (3.00 mL). After stirring at room temperature for 30 minutes, the reaction mixture was diluted with EtOAc, washed with sat. NaHCO₃ aq., H₂O (2x) and brine then dried over Na₂SO₄ and evaporated. The residue was purified by column chromatography on silica gel (hexane:ethyl acetate), thereby obtaining (4-amino-3,5-difluorophenyl)(8-(4-amino-6-(difluoromethyl)-2-(methoxymethyl)-1-methyl-1H-benzo[d]imidazol-5-yl)-1-iodoindolizin-3-yl)methanone.

### Step 11:

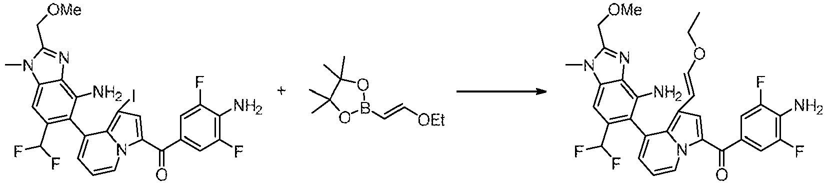

A solution of (4-amino-3,5-difluorophenyl)(8-(4-amino-6-(difluoromethyl)-2-(methoxymethyl)-1-methyl-1H-benzo[d]imidazol-5-yl)-1-iodoindolizin-3-yl)methanone (120 mg), 2-[(E)-2-ethoxyvinyl]-4,4,5,5-tetramethyl-1,3,2-dioxaborolane (185 µL), Pd(PPh₃)₄ (20.2 mg) and 2M aq. Na₂CO₃ (438 µL) in 1,4-dioxane (3.00 mL) was evacuated and N₂ back-filled before heating to 90 °C for 2 hours. After cooling, the reaction mixture was diluted with ethyl acetate, washed with H₂O and brine then dried over Na₂SO₄ and evaporated. The residue was purified by column chromatography on silica gel (hexane:ethyl acetate), thereby obtaining (E)-(4-amino-3,5-difluorophenyl)(8-(4-amino-6-(difluoromethyl)-2-(methoxymethyl)-1-methyl-1H-benzo[d]imidazol-5-yl)-1-(2-ethoxyvinyl)indolizin-3-yl)methanone.

### Step 12:

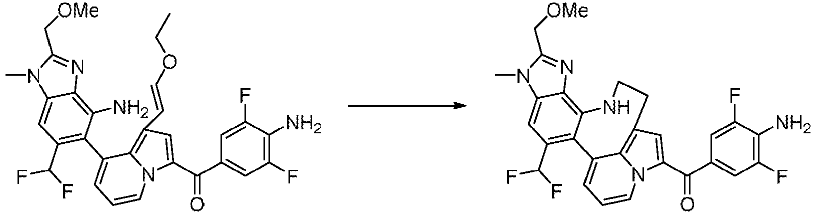

Sodium triacetoxyborohydride (58.3 mg) was added to a solution of (E)-(4-amino-3,5-difluorophenyl)(8-(4-amino-6-(difluoromethyl)-2-(methoxymethyl)-1-methyl-1H-benzo[d]imidazol-5-yl)-1-(2-ethoxyvinyl)indolizin-3-yl)methanone (79.6 mg) and TFA (102 µL) in dichloromethane (5.00 mL). After stirring at room temperature for 30 minutes, the reaction mixture was diluted with chloroform, washed with sat. NaHCO₃ aq., H₂O (2x) and brine then dried over Na₂SO₄ and evaporated. The residue was purified by column chromatography on silica gel (ethyl acetate:ethanol), thereby obtaining (4-amino-3,5-difluorophenyl)(7-(difluoromethyl)-10-(methoxymethyl)-9-methyl-9,12,13,14-tetrahydroimidazo[4",5":5',6']benzo[1',2':2,3]azocino[4,5,6-hi]indolizin-2-yl)methanone.

### Step 13:

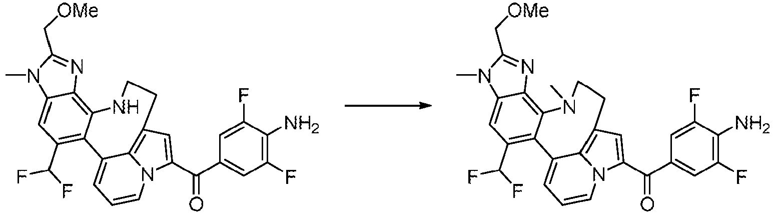

A solution containing sodium cyanoborohydride (16 mg) and zinc chloride (18 mg) in methanol (0.87 mL) was added to a solution of (4-amino-3,5-difluorophenyl)(7-(difluoromethyl)-10-(methoxymethyl)-9-methyl-9,12,13,14-tetrahydroimidazo[4",5::5',6']benzo[1',2':2,3]azocino[4,5,6-hi]indolizin-2-yl)methanone (65 mg) and formaldehyde (36% w/v aq. solution, 33 µL) in THF (2.0 mL). After stirring at room temperature for 30 minutes, the reaction mixture was diluted with ethyl acetate, washed with sat. NaHCO₃ aq., H₂O (2x) and brine then dried over Na₂SO₄ and evaporated. The residue was purified by column chromatography on silica gel (ethyl acetate: ethanol), thereby obtaining (4-amino-3,5-difluorophenyl)(7-(difluoromethyl)-10-(methoxymethyl)-9,12-dimethyl-9,12,13,14-tetrahydroimidazo[4",5":5',6']benzo[1',2':2,3]azocino[4,5,6-hi]indolizin-2-yl)methanone.

### Step 14:

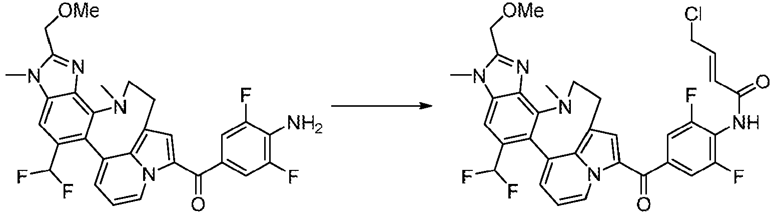

(E)-4-chlorobut-2-enoic acid (12.5 mg) and 1-propanephosphonic acid anhydride cyclic trimer (a 1.7 M THF solution, 125 µL) were added to a solution of (4-amino-3,5-difluorophenyl)(7-(difluoromethyl)-10-(methoxymethyl)-9,12-dimethyl-9,12,13,14-tetrahydroimidazo[4",5":5',6']benzo[1',2':2,3]azocino[4,5,6-hi]indolizin-2-yl)methanone (42.1 mg) in *N*,*N-*dimethylformamide (2.00 mL), followed by stirring at room temperature for 20 minutes. Triethylamine (45 µL) was added to the reaction mixture. A reaction was performed at room temperature for 2 hours. After water was added to the reaction mixture, extraction was performed with ethyl acetate, followed by washing the organic layer with a saturated sodium chloride solution. The washed organic layer was dried over sodium sulfate. After evaporation, the obtained residue was purified by column chromatography (chloroform: ethanol), thereby obtaining (E)-4-chloro-N-(4-(7-(difluoromethyl)-10-(methoxymethyl)-9,12-dimethyl-9,12,13,14-tetrahydroimidazo[4",5":5',6']benzo[1',2':2,3]azocino[4,5,6-hi]indolizine-2-carbonyl)-2,6-difluorophenyl)but-2-enamide.

### Step 15:

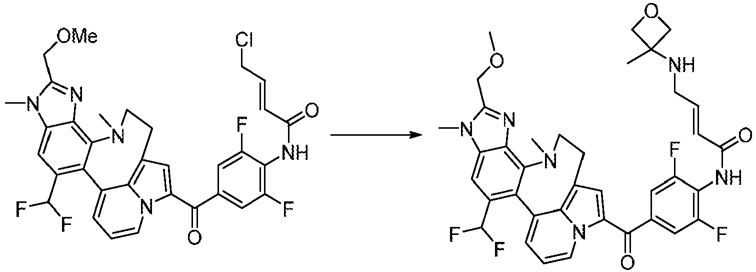

3-methyloxetan-3-amine (35 µL) and potassium iodide (20 mg) were added to a solution of (E)-4-chloro-N-(4-(7-(difluoromethyl)-10-(methoxymethyl)-9,12-dimethyl-9,12,13,14-tetrahydroimidazo[4",5":5',6']benzo[1',2':2,3]azocino[4,5,6-hi]indolizine-2-carbonyl)-2,6-difluorophenyl)but-2-enamide (48 mg) in DMSO (900 µL). A reaction was performed at room temperature for 2 hours. After water was added to the reaction mixture, extraction was performed with ethyl acetate. The combined organic layer was washed with a saturated sodium chloride solution. After the washed layer was dried over sodium sulfate, the solvent was evaporated under reduced pressure. The obtained residue was purified by column chromatography (chloroform:ethanol), thereby obtaining racemic (E)-N-(4-(7-(difluoromethyl)-10-(methoxymethyl)-9,12-dimethyl-9,12,13,14-tetrahydroimidazo[4",5":5',6']benzo[1',2':2,3]azocino[4,5,6-hi]indolizine-2-carbonyl)-2,6-difluorophenyl)-4-((3-methyloxetan-3-yl)amino)but-2-enamide (41.4 mg). The racemate was separated by CHIRALPAK IG (hexane:EtOH (0.1% v/v triethylamine)) to give (E)-N-(4-(7-(difluoromethyl)-10-(methoxymethyl)-9,12-dimethyl-9,12,13,14-tetrahydroimidazo[4",5":5',6']benzo[1',2':2,3]azocino[4,5,6-hi]indolizine-2-carbonyl)-2,6-difluorophenyl)-4-((3-methyloxetan-3-yl)amino)but-2-enamide as the second-eluting isomer. ¹H-NMR: (400 MHz, CHLOROFORM-D) δ 10.00 (dd, J = 7.0, 1.3 Hz, 1H), 7.78 (d, J = 1.5 Hz, 1H), 7.43-7.40 (m, 2H), 7.30 (brs, 1H), 7.14 (dt, J = 15.1, 5.3 Hz, 2H), 7.05-6.98 (m, 2H), 6.51 (t, J = 55.0 Hz, 1H), 6.34 (dd, J = 13.5, 1.8 Hz, 1H), 4.80 (dd, J = 14.4, 12.6 Hz, 2H), 4.59 (d, J = 6.3 Hz, 2H), 4.44 (d, J = 6.5 Hz, 2H), 3.93 (s, 3H), 3.54-3.52 (m, 2H), 3.47 (s, 3H), 3.10 (s, 3H), 2.92-2.85 (m, 1H), 2.59-2.55 (m, 1H), 2.37-2.30 (m, 1H), 2.18 (d, J = 2.5 Hz, 1H), 1.52 (s, 3H); ESI-MS [M+H]⁺: 705.

### Synthetic procedure M

### Example 90

### (S,E)-N-(2,6-difluoro-4-(6,8,9,11-tetramethyl-8,11,12,13-tetrahydroimidazo[4",5":5',6']benzo[1',2':5,6][1,4]diazocino[7,8,1-hi]indole-2-carbonyl)phenyl)-4-((tetrahydrofuran-3-yl)amino)but-2-enamide

### Step 1:

Mixture of atropisomers of (4-amino-3,5-difluorophenyl)(6,8,9,11-tetramethyl-8,11,12,13-tetrahydroimidazo[4",5":5',6']benzo[1',2':5,6][1,4]diazocino[7,8,1-hi]indol-2-yl)methanone (970 mg) obtained in step 10 (Example 44) was separated by CHIRALPAK-IG (hexane-IPA) to give 1st-eluting isomer and 2nd-eluting isomer.

### Step 2:

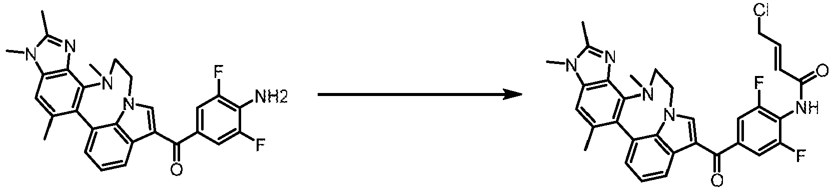

A solution of first-eluting isomer of (4-amino-3,5-difluorophenyl)(6,8,9,11-tetramethyl-8,11,12,13-tetrahydroimidazo[4",5":5',6']benzo[1',2':5,6][1,4]diazocino[7,8,1-hi]indol-2-yl)methanone (394 mg), 4-chlorocrotonic acid (147 mg, 1.22 mmol) and propylphosphonic anhydride solution (50% w/w in THF, 761 µL, 1.22 mmol) in *N,N-*dimethylformamide (2.70 mL) was stirred at room temperature for 15 min. Then triethylamine (339 µL, 2.43 mmol) was added, and the mixture was stirred at room temperature for 1 hour. Further 4-chlorocrotonic acid (49 mg, 0.41 mmol), propylphosphonic anhydride solution (50% w/w in THF, 254 µL, 0.407 mmol) and triethylamine (113 µL, 0.810 mmol) were added and stirring continued for 30 minutes. The reaction mixture was diluted with EtOAc, washed with NaHCO₃ aq., H₂O and brine then dried over Na₂SO₄ and evaporated. The residue was purified by column chromatography on silica gel (CHCl₃/MeOH), thereby obtaining (E)-4-chloro-N-(2,6-difluoro-4-(6,8,9,11-tetramethyl-8,11,12,13-tetrahydroimidazo[4",5":5',6']benzo[1',2':5,6][1,4]diazocino[7,8,1-hi]indole-2-carbonyl)phenyl)but-2-enamide.

### Step 3:

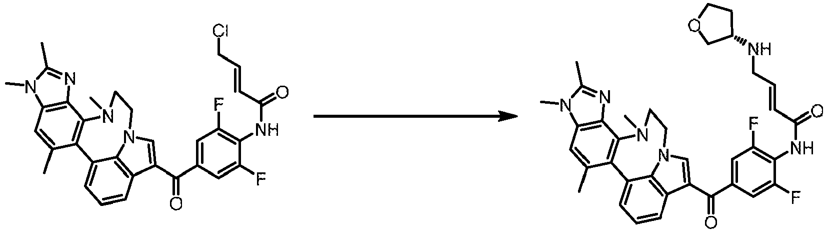

A solution of (E)-4-chloro-N-(2,6-difluoro-4-(6,8,9,11-tetramethyl-8,11,12,13-tetrahydroimidazo[4",5":5',6']benzo[1',2':5,6][1,4]diazocino[7,8,1-hi]indole-2-carbonyl)phenyl)but-2-enamide (42 mg, 0.072 mmol), KI (1 mg, 0.07 mmol) and (S)-tetrahydrofuran-3-amine (24.6 µL, 0.288 mmol) in DMSO (360 µL) was stirred at 40 °C for 1.5 hours. The reaction mixture was diluted with DMSO then filtered and purified by preparative HPLC (water:acetonitrile (0.1% v/v formic acid)). The purified fractions were washed with NaHCO₃ aq., extracted with CHCl₃/MeOH then dried over Na₂SO₄ and evaporated, thereby obtaining (S,E)-N-(2,6-difluoro-4-(6,8,9,11-tetramethyl-8,11,12,13-tetrahydroimidazo[4",5":5',6']benzo[1',2':5,6][1,4]diazocino[7,8,1-hi]indole-2-carbonyl)phenyl)-4-((tetrahydrofuran-3-yl)amino)but-2-enamide. (25.0 mg, 54%). ¹H-NMR: (400 MHz, CHLOROFORM-D) δ 8.41 (d, J = 7.5 Hz, 1H), 7.50 (s, 1H), 7.46-7.40 (m, 2H), 7.35 (dd, J = 7.6 Hz, 1H), 7.19-7.07 (m, 3H), 6.32 (d, J = 15.5 Hz, 1H), 4.05-3.77 (m, 5H), 3.73 (s, 3H), 3.64 (dd, J = 9.0, 3.5 Hz, 1H), 3.56-3.40 (m, 4H), 3.21-3.04 (m, 4H), 2.62 (s, 3H), 2.40 (s, 3H), 2.20-2.08 (m, 1H), 1.81-1.72 (m, 1H); ESI-MS [M+H]⁺: 639.5.

### Synthetic procedure N

### Example 120

### (S,E)-N-(2,6-difluoro-4-(9-methyl-7-(trifluoromethyl)-13,14-dihydro-9H-imidazo[4",5":5',6']benzo[1',2':2,3]oxocino[4,5,6-hi]indolizine-2-carbonyl)phenyl)-4-((tetrahydrofuran-3-yl)amino)but-2-enamide

### Step 1:

Mixture of atropisomers of (4-amino-3,5-difluorophenyl)(9-methyl-7-(trifluoromethyl)-13,14-dihydro-9H-imidazo[4",5":5',6']benzo[1',2':2,3]oxocino[4,5,6-hi]indolizin-2-yl)methanone (728 mg) was obtained in the same manner as Example 30-1 and 30-2. The racemate was separated by CHIRALPAK-IG (hexane-isopropanol) to give 1st-eluting isomer (286 mg) and 2nd-eluting isomer (266 mg).

### Step 2:

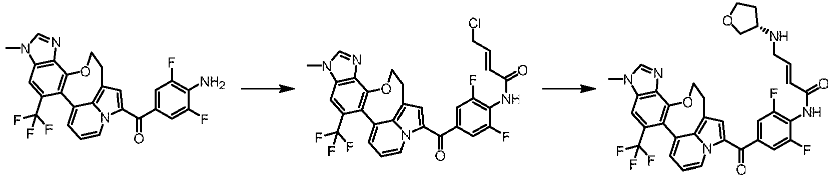

The procedure was performed in the same manner as Example 90 (Steps 2 and 3) using 1st-eluting isomer obtained in Step 1, thereby obtaining (S,E)-N-(2,6-difluoro-4-(9-methyl-7-(trifluoromethyl)-13,14-dihydro-9H-imidazo[4",5":5',6']benzo[1',2':2,3]oxocino[4,5,6-hi]indolizine-2-carbonyl)phenyl)-4-((tetrahydrofuran-3-yl)amino)but-2-enamide. ¹H-NMR: (400 MHz, CHLOROFORM-D) δ 9.99 (dd, J = 7.0, 1.0 Hz, 1H), 8.04 (s, 1H), 7.77 (s, 1H), 7.47-7.41 (m, 2H), 7.19-7.15 (m, 2H), 7.11 (dt, J = 15.4, 5.1 Hz, 1H), 7.03 (dd, J = 7.0, 7.0 Hz, 1H), 7.00 (brs, 1H), 6.28 (dt, J = 15.3, 1.8 Hz, 1H), 4.76 (dd, J = 10.4, 4.4 Hz, 1H), 4.26-4.19 (m, 1H), 4.00-3.92 (m, 4H), 3.85-3.77 (m, 2H), 3.63 (dd, J = 9.1, 3.6 Hz, 1H), 3.54-3.44 (m, 3H), 2.89-2.80 (m, 1H), 2.58 (dd, J = 15.3, 2.3 Hz, 1H), 2.18-2.09 (m, 1H), 1.80-1.72 (m, 1H); ESI-MS [M+H]⁺: 666.

### Synthetic procedure O

### Example 148

### (S,E)-N-(2,6-difluoro-4-(8,9,11-trimethyl-6-(trifluoromethyl)-8,11,12,13-tetrahydro-1,2a,8,10,11-pentaazacycloocta[1,2,3-cd:5,4-e']diindene-2-carbonyl)phenyl)-4-((tetrahydrofuran-3-yl)amino)but-2-enamide

### Step 1:

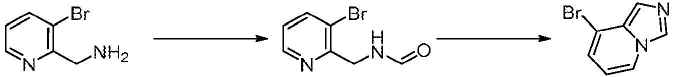

To a mixture of (3-bromopyridin-2-yl)methanamine (3.82 g, 20.4 mmol) and sodium hydrogen carbonate (858 mg, 10.2 mmol) was added ethyl formate (23.1 ml, 286 mmol). After stirring at 50 °C for 19 hours, the mixture was diluted with EtOAc then filtered through a CELITE pad. The filtrate was evaporated and the residue including N-((3-bromopyridin-2-yl)methyl)formamide was used in the next reaction.

Phosphorus oxychloride (3.81 mL, 40.8 mmol) was added to a solution of N-((3-bromopyridin-2-yl)methyl)formamide (4.39 g, 20.4 mmol) in 1,4-dioxane (66.0 mL). After stirring at 110 °C for 1 hour, saturated aqueous NaHCO₃ was added, and the resulting mixture was extracted with EtOAc. The organic extracts were washed successively with H₂O then brine, dried over sodium sulfate, and evaporated. The residue was purified by column chromatography on silica gel (EtOAc-hexane), thereby obtaining 8-bromoimidazo[1,5 -a]pyridine.

### Step 2:

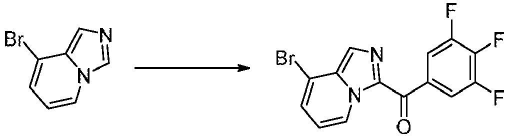

To a mixture of 8-bromoimidazo[1,5-a]pyridine (3.58 g, 18.2 mmol) and 3,4,5-trifluorobenzoyl chloride (4.67 mL, 36.3 mmol) in 1,2-dichloroethane (121 mL) was added triethylamine (7.59 mL, 54.5 mmol) at ambient temperature. After stirring at ambient temperature for 30 mintes, the reaction mixture was added H₂O and saturated aqueous NaHCO₃. The resulting solid was collected by filtration, and vacuum-dried at 50 °C, thereby obtaining (8-bromoimidazo[1,5-a]pyridin-3-yl)(3,4,5-trifluorophenyl)methanone.

### Step 3:

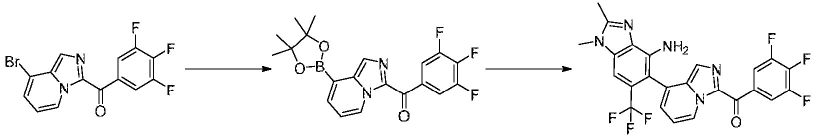

To a mixture of (8-bromoimidazo[1,5-a]pyridin-3-yl)(3,4,5-trifluorophenyl)methanone (800 mg, 2.25 mmol), 4,4,5,5-tetramethyl-2-(4,4,5,5-tetramethyl-1,3,2-dioxaborolan-2-yl)-1,3,2-dioxaborolane (686 mg, 2.70 mmol) and PdCl₂(dppf)CH₂Cl₂ (184 mg, 0.225 mmol) in 1,4-dioxane (11.3 mL) was added potassium acetate (663 mg, 6.76 mmol). After stirring at 90 °C for 90 minutes, the mixture was diluted with EtOAc and water. Extracted with EtOAc, and washed successively with H₂O then brine, dried over sodium sulfate, and evaporated. The residue including (8-(4,4,5,5-tetramethyl-1,3,2-dioxaborolan-2-yl)imidazo[1,5-a]pyridin-3-yl)(3,4,5-trifluorophenyl)methanone was used in the next reaction.

To a mixture of 5-bromo-1,2-dimethyl-6-(trifluoromethyl)-1H-benzo[d]imidazol-4-amine (649 mg, 1.69 mmol), (8-(4,4,5,5-tetramethyl-1,3,2-dioxaborolan-2-yl)imidazo[1,5-a]pyridin-3-yl)(3,4,5-trifluorophenyl)methanone (881 mg, 2.19 mmol) and SPhos Pd G3 (356 mg, 0.506 mmol) in THF (17.0 mL) was added 2M aqueous tripotassium phosphate solution (2.53 mL, 5.06 mmol). After stirring at 80 °C for 2 hours, the mixture was diluted with EtOAc and water. Extracted with EtOAc, and washed successively with H₂O then brine, dried over sodium sulfate, and evaporated. The residue was purified by column chromatography on silica gel (acetone-hexane), thereby obtaining (8-(4-amino-1,2-dimethyl-6-(trifluoromethyl)-1H-benzo[d]imidazol-5-yl)imidazo[1,5-a]pyridin-3-yl)(3,4,5-trifluorophenyl)methanone.

### Step 4:

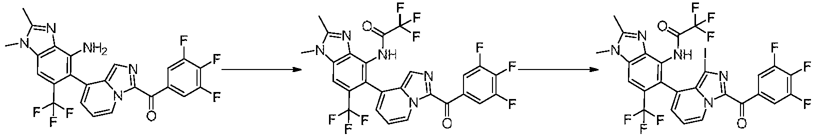

Trifluoroacetic anhydride (260 µL, 1.87 mmol) was added to a mixsolution of 2,2- (8-(4-amino-1,2-dimethyl-6-(trifluoromethyl)-1H-benzo[d]imidazol-5-yl)imidazo[1,5-a]pyridin-3-yl)(3,4,5-trifluorophenyl)methanone (470 mg, 0.934 mmol) and triethylamine (509 µL, 3.73 mmol) in THF (4.60 mL) at 0 °C. The reaction mixture was stirred at ambient temperature for 30 mintues. To the resulting mixture was added saturated aqueous NaHCO₃, and extracted with EtOAc, and washed successively with H₂O then brine, dried over sodium sulfate, and evaporated. The residue including N-(1,2-dimethyl-5-(3-(3,4,5-trifluorobenzoyl)imidazo[1,5-a]pyridin-8-yl)-6-(trifluoromethyl)-1H-benzo[d]imidazol-4-yl)-2,2,2-trifluoroacetamide was used in the next reaction.

To a mixture of N-(1,2-dimethyl-5-(3-(3,4,5-trifluorobenzoyl)imidazo[1,5-a]pyridin-8-yl)-6-(trifluoromethyl)-1H-benzo[d]imidazol-4-yl)-2,2,2-trifluoroacetamide (560 mg, 0.934 mmol) and TFA (346 µL, 4.68 mmol) in THF (4.60 mL) was added N-iodosuccinimide (462 mg, 2.06 mmol) at ambient temperature. After stirring at ambient temperature for 1 hour, the reaction mixture was added saturated aqueous NaHCO₃, Na₂S₂O₃, and extracted with EtOAc, and washed successively with H₂O then brine, dried over sodium sulfate, and evaporated. The residue was purified by column chromatography on silica gel (acetone-hexane), thereby obtaining 2,2,2-trifluoro-N-(5-(1-iodo-3-(3,4,5-trifluorobenzoyl)imidazo[1,5-a]pyridin-8-yl)-1,2-dimethyl-6-(trifluoromethyl)-1H-benzo[d]imidazol-4-yl)acetamide.

### Step 5:

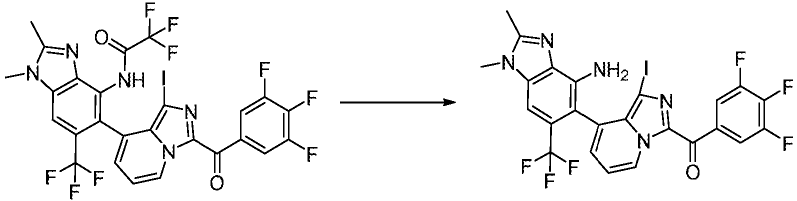

To a mixture of (2,2,2-trifluoro-N-(5-(1-iodo-3-(3,4,5-trifluorobenzoyl)imidazo[1,5-a]pyridin-8-yl)-1,2-dimethyl-6-(trifluoromethyl)-1H-benzo[d]imidazol-4-yl)acetamide (736 mg, 1.00 mmol) in 1,2-dimethoxyethane (15.0 mL) was added 2M aqueous sodium carbonate solution (5.00 mL, 10.0 mmol). After reacting by microwave apparatus at 120 °C for 30 minutes, the mixture was diluted with EtOAc and water. Extracted with EtOAc, and washed successively with H₂O then brine, dried over sodium sulfate, and evaporated thereby obtaining crude (8-(4-amino-1,2-dimethyl-6-(trifluoromethyl)-1H-benzo[d]imidazol-5-yl)-1-iodoimidazo[1,5-a]pyridin-3-yl)(3,4,5-trifluorophenyl)methanone.

### Step 6:

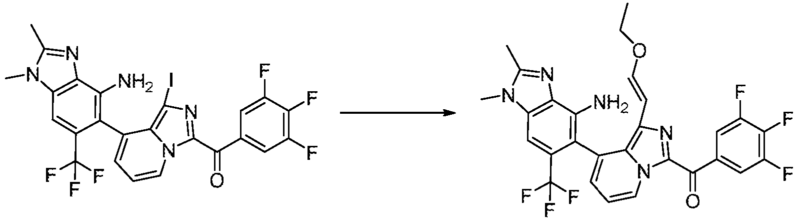

To a mixture of (8-(4-amino-1,2-dimethyl-6-(trifluoromethyl)-1H-benzo[d]imidazol-5-yl)-1-iodoimidazo[1,5-a]pyridin-3-yl)(3,4,5-trifluorophenyl)methanone (572 mg, 0.909 mmol), 2-[(E)-2-ethoxyvinyl]-4,4,5,5-tetramethyl-1,3,2-dioxaborolane (963 µL, 4.54 mmol) and Pd(PPh₃)₄ (105 mg, 0.0909 mmol) in 1,4-dioxane (6.00 mL) was added 2M aqueous sodium carbonate solution (2.27 mL, 4.54 mmol). After stirring at 90 °C for 40 minutes, the mixture was diluted with EtOAc and water. The layers were shaken and separated. The aqueous phase was extracted with EtOAc, and the combined organics were washed successively with H₂O then brine, dried over sodium sulfate, filtered, and evaporated. The residue was purified by column chromatography on silica gel (acetone-hexane), thereby obtaining (E)-(8-(4-amino-1,2-dimethyl-6-(trifluoromethyl)-1H-benzo[d]imidazol-5-yl)-1-(2-ethoxyvinyl)imidazo[1,5-a]pyridin-3-yl)(3,4,5-trifluorophenyl)methanone.

### Step 7:

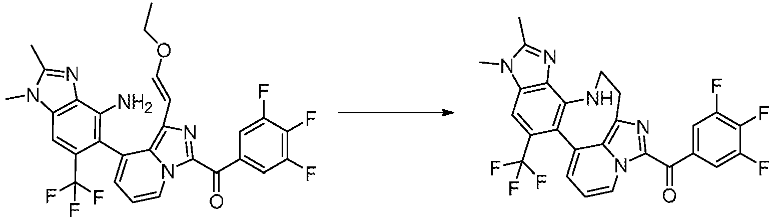

To a mixture of (E)-(8-(4-amino-1,2-dimethyl-6-(trifluoromethyl)-1H-benzo[d]imidazol-5-yl)-1-(2-ethoxyvinyl)imidazo[1,5-a]pyridin-3-yl)(3,4,5-trifluorophenyl)methanone (200 mg, 0.349 mmol) and sodium triacetoxyborohydride (222 mg, 1.05 mmol) in CH₂Cl₂ (3.89 mL) was added TFA (389 µL, 5.23 mmol) at ambient temperature. After stirring at ambient temperature for 90 minutes, the reaction mixture was added saturated aqueous NaHCO₃, and extracted with EtOAc, and washed successively with H₂O then brine, dried over sodium sulfate, and evaporated. The residue was purified by column chromatography on silica gel (acetone-hexane), thereby obtaining (8,9-dimethyl-6-(trifluoromethyl)-8,11,12,13-tetrahydro-1,2a, 8,10,11-pentaazacycloocta[1,2,3-cd:5,4-e']diinden-2-yl)(3,4,5-trifluorophenyl)methanone.

### Step 8:

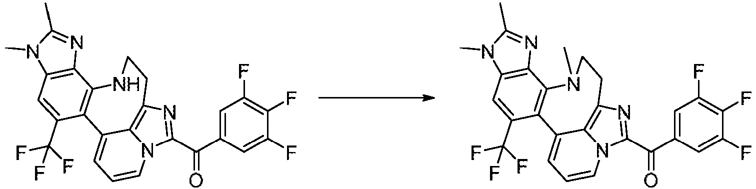

A solution of (8,9-dimethyl-6-(trifluoromethyl)-8, 11, 12, 13-tetrahydro-1,2a,8,10,11-pentaazacycloocta[1,2,3-cd:5,4-e']diinden-2-yl)(3,4,5-trifluorophenyl)methanone (51.1 mg, 0.0965 mmol), 37% formaldehyde solution (72.5 µL, 0.965 mmol) and TFA (14.3 µL, 0.193 mmol) in THF (500 µL) was stirred at ambient temperature for 5 minutes. Then a mixture of sodium cyanoborohydride and zinc chloride (1:0.5) in MeOH (0.3 mol/L, 1.13 mL, 0.338 mmol)) was added thereto, and the mixture was stirred at ambient temperature for 15 minutes. To the resulting mixture was added saturated aqueous NaHCO₃, and extracted with EtOAc, and washed successively with H₂O then brine, dried over sodium sulfate, filtered, and evaporated. The residue was purified by column chromatography on silica gel (acetone-hexane), thereby obtaining (3,4,5-trifluorophenyl)(8,9,11-trimethyl-6-(trifluoromethyl)-8,11,12,13-tetrahydro-1,2a,8,10,11-pentaazacycloocta[1,2,3-cd:5,4-e']diinden-2-yl)methanone.

### Step 9:

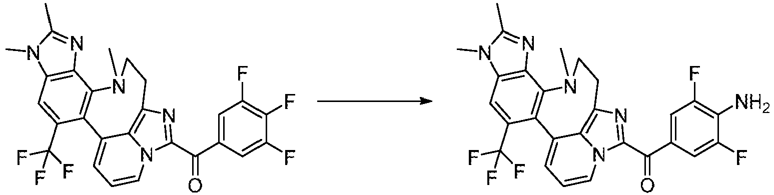

To a mixture of (3,4,5-trifluorophenyl)(8,9,11-trimethyl-6-(trifluoromethyl)-8,11,12,13-tetrahydro-1,2a,8,10,11-pentaazacycloocta[1,2,3-cd:5,4-e']diinden-2-yl)methanone (21.0 mg, 0.0390 mmol) in 1,4-dioxane (2.00 mL) was added ammonium hydroxide (2.10 mL, 18.0 mmol). After reacting by microwave apparatus at 140 °C for 4 hours, the mixture was diluted with EtOAc and water. The layers were shaken and separated. The aqueous phase was extracted with EtOAc, and the combined organics were washed successively with H₂O then brine, dried over sodium sulfate, filtered, and evaporated, thereby obtaining crude (4-amino-3,5-difluorophenyl)(8,9,11-trimethyl-6-(trifluoromethyl)-8,11,12,13-tetrahydro-1,2a,8,10,11-pentaazacycloocta[1,2,3-cd:5,4-e']diinden-2-yl)methanone.

### Step 10:

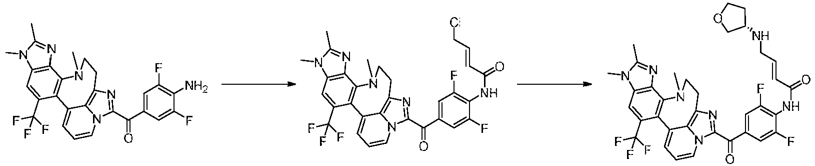

A solution of (4-amino-3,5-difluorophenyl)(8,9,11-trimethyl-6-(trifluoromethyl)-8,11,12,13-tetrahydro-1,2a,8,10,11-pentaazacycloocta[1,2,3-cd:5,4-e']diinden-2-yl)methanone (21.0 mg, 0.0389 mmol), 4-chlorocrotonic acid (5.85 mg, 0.0486 mmol) and propylphosphonic anhydride solution (50 wt% in THF, 60.7 µL, 0.0971 mmol) in DMF (500 µL) was stirred at ambient temperature for 10 minutes. Then triethylamine (21.6 µL, 0.155 mmol) was added thereto, and the mixture was stirred at ambient temperature for 30 minutes. To the resulting mixture was added saturated aqueous NaHCO₃, and extracted with EtOAc, and washed successively with H₂O then brine, dried over sodium sulfate, and evaporated. The residue including (E)-4-chloro-N-(2,6-difluoro-4-(8,9,11-trimethyl-6-(trifluoromethyl)-8,11,12,13-tetrahydro-1,2a,8,10,11-pentaazacycloocta[1,2,3-cd:5,4-e']diindene-2-carbonyl)phenyl)but-2-enamide was used in the next reaction.

A solution of (E)-4-chloro-N-(2,6-difluoro-4-(8,9,11-trimethyl-6-(trifluoromethyl)-8,11,12,13-tetrahydro-1,2a,8,10,11-pentaazacycloocta[1,2,3-cd:5,4-e']diindene-2-carbonyl)phenyl)but-2-enamide (25.0 mg, 0.0389 mmol), (3S)-tetrahydrofuran-3-amine (17.0 µL, 0.194 mmol) and KI (32.3 g, 0.194 mmol) in DMSO (1.00 mL) was stirred at 35 °C for 1 hour. The resulting mixture was purified by preparative HPLC (water:acetonitrile (0.1% formic acid)) to give the diastereomixture of the title compound (14.7 mg, 0.0212 mmol). The mixture was separated by CHIRALPAK IA (hexane-ethanol(0.1% triethylamine)) to give (S,E)-N-(2,6-difluoro-4-(8,9,11-trimethyl-6-(trifluoromethyl)-8,11,12,13-tetrahydro-1,2a,8,10,11-pentaazacycloocta[1,2,3-cd:5,4-e']diindene-2-carbonyl)phenyl)-4-((tetrahydrofuran-3-yl)amino)but-2-enamide (single isomer, 2nd-eluting isomer). ¹H-NMR: (400 MHz, CHLOROFORM-D) δ 9.86 (dd, J = 6.1, 1.9 Hz, 1H), 8.20 (d, J = 8.8 Hz, 2H), 7.70 (s, 1H), 7.14-7.07 (m, 3H), 6.26 (d, J = 15.3 Hz, 1H), 3.96 (dd, J = 14.9, 7.9 Hz, 1H), 3.85-3.78 (m, 5H), 3.68-3.56 (m, 3H), 3.49-3.44 (m, 3H), 3.06 (s, 3H), 3.02-2.94 (m, 2H), 2.70 (s, 3H), 2.51-2.44 (m, 1H), 2.18-2.09 (m, 1H), 1.77 (d, J = 4.3 Hz, 1H); ESI-MS [M+H]⁺: 695.

### Synthetic procedure P

### Example 152

### (E)-N-(2,6-difluoro-4-(10-(methoxymethyl)-9,13-dimethyl-12-oxo-7-(trifluoromethyl)-9,12,13,14-tetrahydroimidazo[4",5":3',4']benzo[1',2':6,7]azocino[5,4,3-hi]indolizine-2-carbonyl)phenyl)-4-((3-methyloxetan-3-yl)amino)but-2-enamide

### Step 1:

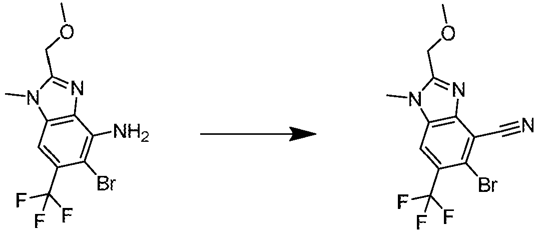

To a solution of 5-bromo-2-(methoxymethyl)-1-methyl-6-(trifluoromethyl)-1H-benzo[d]imidazol-4-amine (6.80 g, 20.1 mmol) in DMF (40 mL) was added tert-butyl nitrite (3.00 mL, 25.2 mmol) and copper(I) cyanide (3.60 g, 40.2 mmol), and the mixture was stirred at 50 °C for 1 hour. After cooling, EtOAc and 28-30% NH₃ aq. were added thereto. The organic layer was separated, washed with H₂O and brine, and dried over sodium sulfate. After evaporation, the resulting residue was purified by column chromatography on silica gel (hexane-EtOAc/EtOH(4/1)) to obtain 5-bromo-2-(methoxymethyl)-1-methyl-6-(trifluoromethyl)-1H-benzo[d]imidazole-4-carbonitrile.

### Step 2:

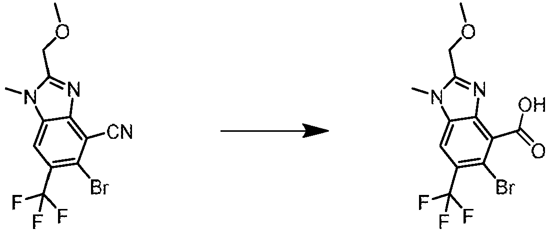

5-Bromo-2-(methoxymethyl)-1-methyl-6-(trifluoromethyl)-1H-benzo[d]imidazole-4-carbonitrile (1.00 g, 2.87 mmol) was diluted with 1,4-dioxane (3.0 mL) and 40% H₂SO₄ aq. (12 mL), and the reaction mixture was heated at 120 °C under microwave irradiation for 10 hours. After cooling, the mixture was poured into sat. NaHCO₃ aq. then sat. NaHCO₃ aq. and CHCl₃/MeOH (5/1) were added thereto. The resulting precipitate was collected by filtration and rinsed with water to obtain 5-bromo-2-(methoxymethyl)-1-methyl-6-(trifluoromethyl)- 1H-benzo[d]imidazole-4-carboxylic acid (462 mg, 1.26 mmol). The filtrate was washed with brine then dried over Na₂SO₄ and evaporated to obtain additional 5-bromo-2-(methoxymethyl)-1-methyl-6-(trifluoromethyl)-1H-benzo[d]imidazole-4-carboxylic acid.

### Step 3:

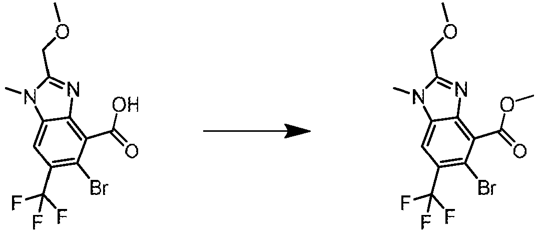

To a mixture of 5-bromo-2-(methoxymethyl)-1-methyl-6-(trifluoromethyl)-1H-benzo[d]imidazole-4-carboxylic acid (490 mg, 1.33 mmol) and potassium carbonate (368 mg, 2.66 mmol) in DMF (5.0 mL) and THF (5.0 mL) was added methyl iodide (0.249 mL, 4.00 mmol). After stirring at room temperature overnight, the reaction mixture was diluted with EtOAc and H₂O, mixed, and separated. The separated organic layer was washed with brine, dried over Na₂SO₄ and evaporated. The residue was purified by column chromatography on silica gel (hexane-EtOAc/EtOH(4/1)) to obtain methyl 5-bromo-2-(methoxymethyl)-1-methyl-6-(trifluoromethyl)-1H-benzo[d]imidazole-4-carboxylate.

### Step 4:

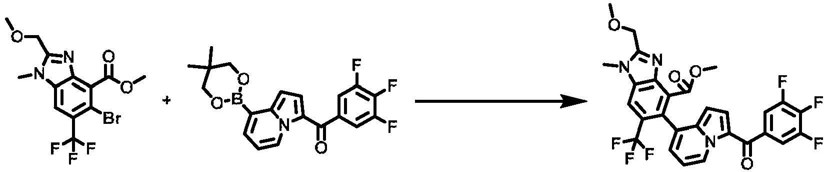

A solution of methyl 5-bromo-2-(methoxymethyl)-1-methyl-6-(trifluoromethyl)-1H-benzo[d]imidazole-4-carboxylate (561 mg, 1.47 mmol), (8-(5,5-dimethyl-1,3,2-dioxaborinan-2-yl)indolizin-3-yl)(3,4,5-trifluorophenyl)methanone (2.28 g, 5.89 mmol), SPhos Pd G3 (260 mg, 0.368 mmol) in Toluene (13.7 mL) and 2M K₃PO₄ aq. (4.41 mL, 8.83 mmol) was evacuated and N₂ back-filled before heating at 80 °C by microwave irradiation for 2 hours. After cooling, EtOAc was added to the reaction mixture then filtered through a CELITE pad, rinsing with EtOAc. The filtrate was washed with H₂O, NH₄Cl (aq.), and brine then dried over Na₂SO₄, filtered, and evaporated. The residue was purified by column chromatography on silica gel (hexane/acetone/CHCl₃), thereby obtaining methyl 2-(methoxymethyl)-1-methyl-5-(3-(3,4,5-trifluorobenzoyl)indolizin-8-yl)-6-(trifluoromethyl)-1H-benzo[d]imidazole-4-carboxylate.

### Step 5:

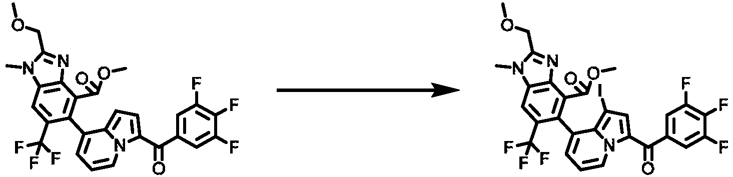

N-Iodosuccinimide (118 mg, 0.518 mmol) was added to a solution of methyl 2-(methoxymethyl)-1-methyl-5-(3-(3,4,5-trifluorobenzoyl)indolizin-8-yl)-6-(trifluoromethyl)-1H-benzo[d]imidazole-4-carboxylate (199 mg, 0.345 mmol) in THF (3.45 mL) and AcOH (148 µL, 2.59 mmol). The reaction mixture was stirred at room temperature for 2 hours and at 40 °C for 3.5 hours. After cooling, the mixture was diluted with EtOAc, washed with sat. NaHCO₃ (aq.), 20% Na₂S₂O₃ (aq.), and brine then dried over Na₂SO₄, filtered, and evaporated. The residue was purified by column chromatography on silica gel (hexane/acetone), thereby obtaining methyl 5-(1-iodo-3-(3,4,5-trifluorobenzoyl)indolizin-8-yl)-2-(methoxymethyl)-1-methyl-6-(trifluoromethyl)-1H-benzo[d]imidazole-4-carboxylate.

### Step 6:

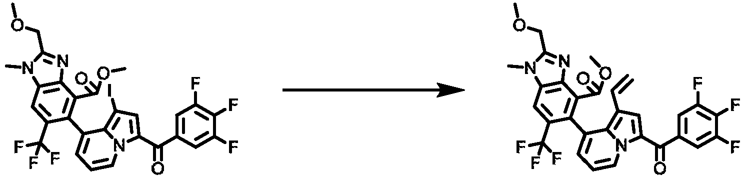

A mixture of methyl 5-(1-iodo-3-(3,4,5-trifluorobenzoyl)indolizin-8-yl)-2-(methoxymethyl)-1-methyl-6-(trifluoromethyl)-1H-benzo[d]imidazole-4-carboxylate (196 mg, 0.279 mmol), potassium vinyltrifluoroborate (112 mg, 0.838 mmol), PdCl₂(dppf) · CH₂Cl₂ (23 mg, 0.028 mmol) in 1,4-dioxane (2.79 mL) and 2M Na₂CO₃ aq. (629 µL, 1.26 mmol) was evacuated and N₂ back-filled before heating to 100 °C for 9 hours. After cooling, EtOAc was added to the reaction mixture then filtered, rinsing with EtOAc. The filtrate was washed with H₂O, NaHCO₃ (aq.), and brine then dried over Na₂SO₄, filtered, and evaporated. The residue was purified by column chromatography on silica gel (hexane/acetone) to obtain methyl 2-(methoxymethyl)-1-methyl-5-(3-(3,4,5-trifluorobenzoyl)-1-vinylindolizin-8-yl)-6-(trifluoromethyl)-1H-benzo[d]imidazole-4-carboxylate.

### Step 7:

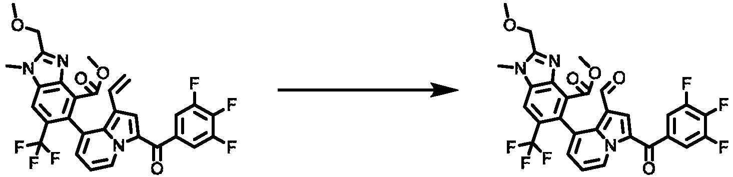

To a mixture of methyl 2-(methoxymethyl)-1-methyl-5-(3-(3,4,5-trifluorobenzoyl)-1-vinylindolizin-8-yl)-6-(trifluoromethyl)-1H-benzo[d]imidazole-4-carboxylate (112 mg, 0.186 mmol), 2,6-lutidine (43.4 µL, 0.372 mmol) and NaIO₄ (159 mg, 0.745 mmol) in 1,4-dioxane (1.86 mL) and H₂O (335 µL) was added 1% OsO₄ aq. (94.8 µL, 3.72 µmol). After stirring at room temperature overnight, the reaction mixture then filtered through a CELITE pad, rinsing with EtOAc. The filtrate was washed with H₂O and brine then dried over Na₂SO₄, filtered, and evaporated. The residue was purified by column chromatography on silica gel (hexane/acetone), thereby obtaining methyl 5-(1-formyl-3-(3,4,5-trifluorobenzoyl)indolizin-8-yl)-2-(methoxymethyl)-1-methyl-6-(trifluoromethyl)-1H-benzo[d]imidazole-4-carboxylate.

### Step 8:

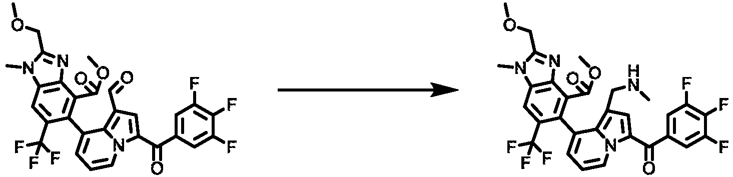

A solution of methyl 5-(1-formyl-3-(3,4,5-trifluorobenzoyl)indolizin-8-yl)-2-(methoxymethyl)-1-methyl-6-(trifluoromethyl)-1H-benzo[d]imidazole-4-carboxylate (69.1 mg, 0.115 mmol), methylamine (2M THF solution, 172 µL, 0.344 mmol) and TFA (8.52 µL, 0.115 mmol) in THF (575 µL) was stirred at room temperature for 1 hour. Then a mixture of sodium cyanoborohydride and zinc chloride (1:0.5) in MeOH (0.3 mol/L, 954 µL, 0.286 mmol)) was added thereto, and the mixture was stirred at room temperature for 5 hours. The reaction mixture was diluted with CHCl₃, washed with H₂O and brine then dried over Na₂SO₄, filtered, and evaporated. The residue was purified by column chromatography on silica gel (CHCl₃/MeOH), thereby obtaining methyl 2-(methoxymethyl)-1-methyl-5-(1-((methylamino)methyl)-3-(3,4,5-trifluorobenzoyl)indolizin-8-yl)-6-(trifluoromethyl)-1H-benzo[d]imidazole-4-carboxylate.

### Steps 9-10:

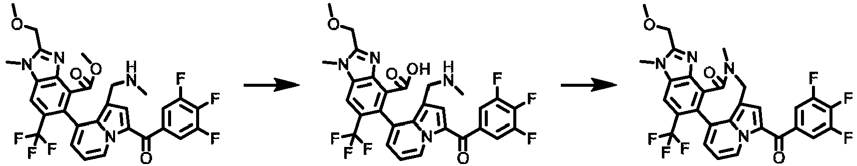

To a solution of methyl 2-(methoxymethyl)-1-methyl-5-(1-((methylamino)methyl)-3-(3,4,5-trifluorobenzoyl)indolizin-8-yl)-6-(trifluoromethyl)-1H-benzo[d]imidazole-4-carboxylate (45.6 mg, 73.7 µmol) in MeOH (737 µL) was added NaOH (aq.) (5M, 147 µL, 0.737 mmol), and the mixture was stirred at room temperature for 1 hour. Further NaOH (aq.) (5M, 147 µL, 0.737 mmol) was added and stirring continued for 45 minutes. To the reaction mixture was added HCl (aq.) (6M, 245 µL, 1.47 mmol), and the mixture was diluted with EtOAc, washed with H₂O and brine. To the aqueous layer was added sat. NaHCO₃ (aq.), and the solution was extracted with EtOAc. Combined organics were dried over Na₂SO₄, filtered, and evaporated, thereby obtaining crude 2-(methoxymethyl)-1-methyl-5-(1-((methylamino)methyl)-3-(3,4,5-trifluorobenzoyl)indolizin-8-yl)-6-(trifluoromethyl)-1H-benzo[d]imidazole-4-carboxylic acid.

HATU (42 mg, 0.11 mmol) was added to a solution of crude 2-(methoxymethyl)-1-methyl-5-(1-((methylamino)methyl)-3-(3,4,5-trifluorobenzoyl)indolizin-8-yl)-6-(trifluoromethyl)-1H-benzo[d]imidazole-4-carboxylic acid (73.7 µmol) and DIEA (37.8 µL, 0.221 mmol) in DMF (2.95 mL). After stirring at room temperature for 30 minutes, the reaction mixture was diluted with EtOAc/hexane, washed with NaHCO₃ (aq.), H₂O, and brine then dried over Na₂SO₄ and evaporated. The residue was purified by column chromatography on silica gel (hexane/acetone), thereby obtaining crude 10-(methoxymethyl)-9,13-dimethyl-2-(3,4,5-trifluorobenzoyl)-7-(trifluoromethyl)-13,14-dihydroimidazo[4",5":3',4']benzo[1',2':6,7]azocino[5,4,3-hi]indolizin-12(9H)-one.

### Step 11:

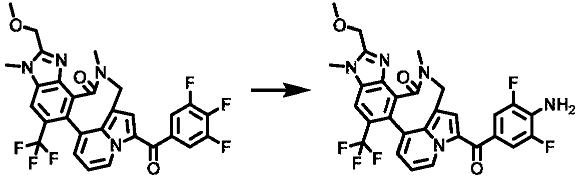

28% NH₃ aq. (3.0 mL, 22 mmol) was added to crude 10-(methoxymethyl)-9,13-dimethyl-2-(3,4,5-trifluorobenzoyl)-7-(trifluoromethyl)-13,14-dihydroimidazo[4",5":3',4']benzo[1',2':6,7]azocino[5,4,3-hi]indolizin-12(9H)-one (73.7 µmol) in 1,4-dioxane (666 µL). The reaction mixture was heated at 130 °C by microwave irradiation for 12 hours. After cooling, the reaction mixture was diluted with EtOAc, washed with H₂O and brine, then dried over Na₂SO₄, filtered, and evaporated. The residue was purified by column chromatography on silica gel (CHCl₃/MeOH), thereby obtaining 2-(4-amino-3,5-difluorobenzoyl)-10-(methoxymethyl)-9,13-dimethyl-7-(trifluoromethyl)-13,14-dihydroimidazo[4",5":3',4']benzo[1',2':6,7]azocino[5,4,3-hi]indolizin-12(9H)-one.

### Steps 12-13:

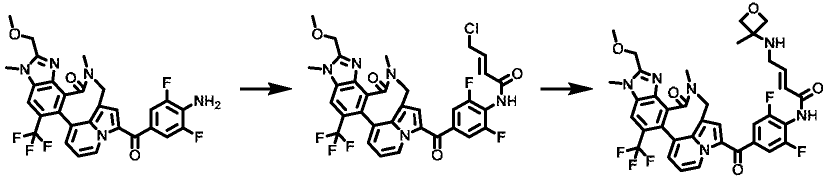

A solution of 2-(4-amino-3,5-difluorobenzoyl)-10-(methoxymethyl)-9,13-dimethyl-7-(trifluoromethyl)-13,14-dihydroimidazo[4",5":3',4']benzo[1',2':6,7]azocino[5,4,3-hi]indolizin-12(9H)-one (20.4 mg), 4-chlorocrotonic acid (9 mg, 0.07 mmol) and propylphosphonic anhydride solution (50 wt% in THF, 43.8 µL, 70.0 µmol) in DMF (700 µL) was stirred at room temperature for 15 minutes. Then Et₃N (19.5 µL, 0.140 mmol) was added thereto, and the mixture was stirred at room temperature for 1 hour. Further 4-chlorocrotonic acid (5 mg, 0.04 mmol), propylphosphonic anhydride solution (50 wt% in THF, 22.0 µL, 35.0 µmol) and Et₃N (9.76 µL, 70.0 µmol) were added and stirring continued for 1 hour. The reaction mixture was diluted with EtOAc, washed with NaHCO₃ (aq.), H₂O, and brine then dried over Na₂SO₄, filtered, and evaporated. The residue was passed through a small amount of NH silica gel (CHCl₃) and evaporated, thereby obtaining crude (E)-4-chloro-N-(2,6-difluoro-4-(10-(methoxymethyl)-9,13-dimethyl-12-oxo-7-(trifluoromethyl)-9,12,13,14-tetrahydroimidazo[4",5":3',4']benzo[1',2':6,7]azocino[5,4,3-hi]indolizine-2-carbonyl)phenyl)but-2-enamide.

A solution of crude (E)-4-chloro-N-(2,6-difluoro-4-(10-(methoxymethyl)-9,13-dimethyl-12-oxo-7-(trifluoromethyl)-9,12,13,14-tetrahydroimidazo[4",5":3',4']benzo[1',2':6,7]azocino[5,4,3-hi]indolizine-2-carbonyl)phenyl)but-2-enamide (21 mg, 35 µmol), KI (11 mg, 66 µmol), DIEA (10.5 µL, 61.2 µmol) and 3-methyloxetan-3-amine (13.6 µL, 0.153 mmol) in DMSO (306 µL) was stirred at 40 °C for 3 hours. The reaction mixture was diluted with DMSO then filtered and purified by preparative HPLC (water:acetonitrile (0.1% formic acid)). The purified fractions were washed with NaHCO₃ aq., extracted with CHCl₃/MeOH then dried over Na₂SO₄ and evaporated, thereby obtaining (E)-N-(2,6-difluoro-4-(10-(methoxymethyl)-9,13-dimethyl-12-oxo-7-(trifluoromethyl)-9,12,13,14-tetrahydroimidazo[4",5":3',4']benzo[1',2':6,7]azocino[5,4,3-hi]indolizine-2-carbonyl)phenyl)-4-((3-methyloxetan-3-yl)amino)but-2-enamide. ¹H-NMR: (400 MHz, CHLOROFORM-D) δ 9.99 (dd, J = 7.0, 1.0 Hz, 1H), 7.95 (s, 1H), 7.45-7.38 (m, 2H), 7.29 (s, 1H), 7.27-7.23 (m, 1H), 7.21-7.06 (m, 3H), 6.32 (dt, J = 15.3, 1.9 Hz, 1H), 4.88 (d, J = 13.0 Hz, 1H), 4.83 (d, J = 13.0 Hz, 1H), 4.77 (d, J = 15.5 Hz, 1H), 4.57 (d, J = 6.3 Hz, 2H), 4.43 (d, J = 6.5 Hz, 2H), 3.98 (s, 3H), 3.80 (d, J = 15.8 Hz, 1H), 3.53 (dd, J = 5.1, 1.9 Hz, 2H), 3.45 (s, 3H), 2.91 (s, 3H), 1.51 (s, 3H); ESI-MS [M+H]⁺: 738.

### Synthetic procedure Q

### Example 158

### (S,E)-N-(2,6-difluoro-4-(10-(methoxymethyl)-9,13-dimethyl-14-oxo-7-(trifluoromethyl)-9,12,13,14-tetrahydroimidazo[4",5":3',4']benzo[1',2':6,7]azocino[5,4,3-hi]indolizine-2-carbonyl)phenyl)-4-((tetrahydrofuran-3-yl)amino)but-2-enamide

### Step 1:

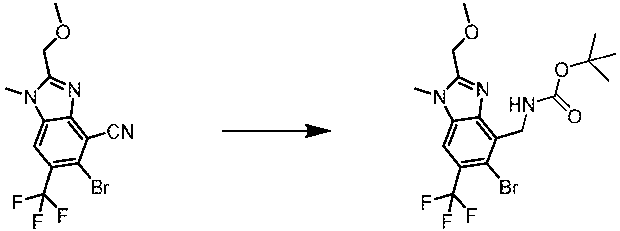

To a solution of 5-bromo-2-(methoxymethyl)-1-methyl-6-(trifluoromethyl)-1H-benzo[d]imidazole-4-carbonitrile (100 mg, 0.287 mmol) in dichloromethane (5.0 mL) was added diisobutylaluminium hydride in hexane (1.0 M, 1.15 mL, 1.15 mmol) at 0 °C, and the mixture was stirred at 0 °C for 30 minutes. 2M HCl was added to the mixture, and stirred at room temperature for 1 hour. The resulting mixture was diluted with EtOAc (5.0 mL), added sat. NaHCO₃ and Boc₂O (0.119 mL, 0.575 mmol). After stirring overnight, the mixture was filtered, rinsing with EtOAc, the filtrate was washed with H₂O and brine then dried over Na₂SO₄ and evaporated. The residue was purified by column chromatography on silica gel (hexane-EtOAc) to obtain *tert*-butyl ((5-bromo-2-(methoxymethyl)-1-methyl-6-(trifluoromethyl)-1H-benzo[d]imidazol-4-yl)methyl)carbamate.

### Step 2:

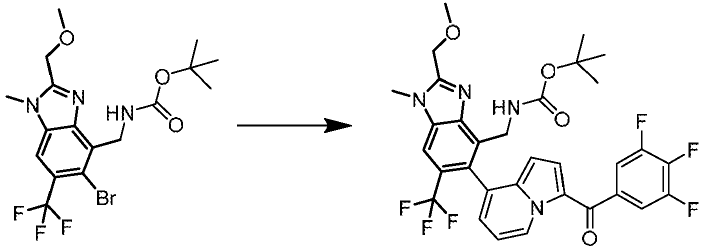

*Tert*-butyl ((5-bromo-2-(methoxymethyl)-1-methyl-6-(trifluoromethyl)-1H-benzo[d]imidazol-4-yl)methyl)carbamate (277 mg, 0.612 mmol), (8-(5,5-dimethyl-1,3,2-dioxaborinan-2-yl)indolizin-3-yl)(3,4,5-trifluorophenyl)methanone (388 mg, 1.00 mmol), S-Phos Pd G3 (30.2 mg, 0.0429 mmol) in THF (1.5 mL) and 2M K₃PO₄ aq. (0.612 mL, 1.22 mmol) was evacuated and N₂ back-filled before heating to 80 °C for 6 hours. After cooling, the reaction mixture was diluted with EtOAc. The resulting suspension was filtered, rinsing with EtOAc, the filtrate was washed with H₂O and brine then dried over Na₂SO₄, filtered, and evaporated. The residue was purified by column chromatography on silica gel (hexane- EtOAc/EtOH(4/1)), and collected by filtration, washed with diisopropyl ether to obtain *tert*-butyl ((2-(methoxymethyl)-1-methyl-5-(3-(3,4,5-trifluorobenzoyl)indolizin-8-yl)-6-(trifluoromethyl)-1H-benzo[d]imidazol-4-yl)methyl)carbamate.

### Step 3:

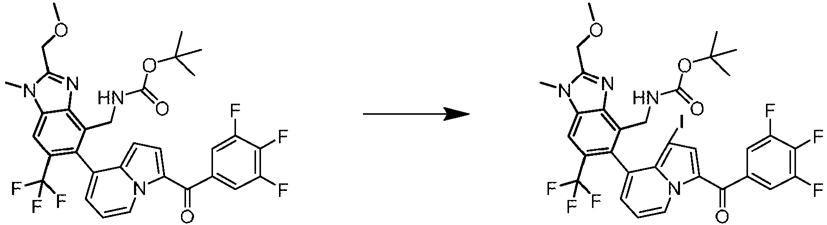

*N*-Iodosuccinimide (80.0 mg, 0.356 mmol) and TFA (0.0661 mL, 0.889 mmol) was added *tert*-butyl ((2-(methoxymethyl)-1-methyl-5-(3-(3,4,5-trifluorobenzoyl)indolizin-8-yl)-6-(trifluoromethyl)-1H-benzo[d]imidazol-4-yl)methyl)carbamate (230 mg, 0.356 mmol) in THF (4.0 mL). After stirring at room temperature for 1 hour, the mixture was diluted with EtOAc, and sat. NaHCO₃ aq. and sat. Na₂S₂O₃ aq. were added thereto. The organic layer was separated, and washed with a saturated sodium chloride solution, followed by drying over sodium sulfate. After evaporation, the obtained residue was purified by column chromatography on silica gel (hexane-EtOAc/EtOH(4/1)) to obtain *tert*-butyl ((5-(1-iodo-3-(3,4,5-trifluorobenzoyl)indolizin-8-yl)-2-(methoxymethyl)-1-methyl-6-(trifluoromethyl)-1H-benzo[d]imidazol-4-yl)methyl)carbamate.

### Step 4:

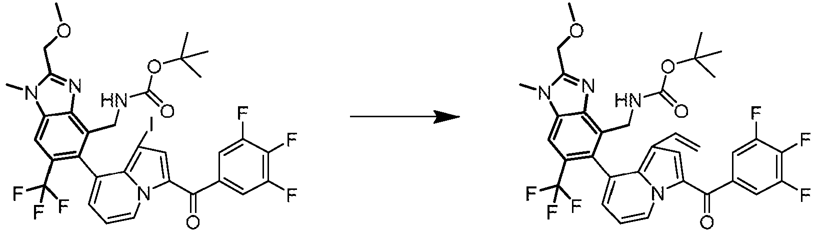

A mixture of *tert*-butyl ((5-(1-iodo-3-(3,4,5-trifluorobenzoyl)indolizin-8-yl)-2-(methoxymethyl)-1-methyl-6-(trifluoromethyl)-1H-benzo[d] imidazol-4-yl)methyl)carbamate (120 mg, 0.155 mmol), potassium vinyltrifluoroborate (52.0 mg, 0.388 mmol) and PdCl₂(dppf) ·CH₂Cl₂ (12.7 mg, 0.0155 mmol) in 1,4-dioxane (1.5 mL) and 2M Na₂CO₃ (aq.) (0.256 mL, 0.513 mmol) was evacuated and N₂ back-filled before heating to 100 °C for 8 hours. After cooling, the reaction mixture was diluted with EtOAc, washed with H₂O and brine, then dried over Na₂SO₄, filtered, and evaporated. The residue was purified by column chromatography on silica gel (hexane-acetone) to obtain *tert*-butyl ((2-(methoxymethyl)-1-methyl-5-(3-(3,4,5-trifluorobenzoyl)-1-vinylindolizin-8-yl)-6-(trifluoromethyl)-1H-benzo[d]imidazol-4-yl)methyl)carbamate.

### Step 5:

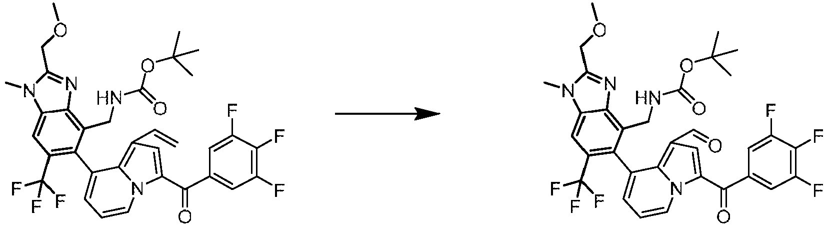

To a mixture of *tert-*butyl ((2-(methoxymethyl)-1-methyl-5-(3-(3,4,5-trifluorobenzoyl)-1-vinylindolizin-8-yl)-6-(trifluoromethyl)-1H-benzo[d]imidazol-4-yl)methyl)carbamate (82.4 mg, 0.123 mmol), 2,6-lutidine (0.0285 mL, 0.245 mmol) and NaIO₄ (105 mg, 0.490 mmol) in 1,4-dioxane (3.8 mL) and H₂O (0.221 mL) was added 1% OsO₄ (aq.) (0.0623 mL, 0.00245 mmol). After stirring at room temperature overnight, the reaction mixture was diluted with EtOAc. The resulting suspension was filtered, rinsing with EtOAc, the filtrate was washed with H₂O and brine then dried over Na₂SO₄, filtered, and evaporated. The residue was purified by column chromatography on silica gel (hexane-EtOAc/EtOH(4/1)) to obtain *tert*-butyl ((5-(1-formyl-3-(3,4,5-trifluorobenzoyl)indolizin-8-yl)-2-(methoxymethyl)-1-methyl-6-(trifluoromethyl)-1H-benzo[d]imidazol-4-yl)methyl)carbamate.

### Steps 6-8:

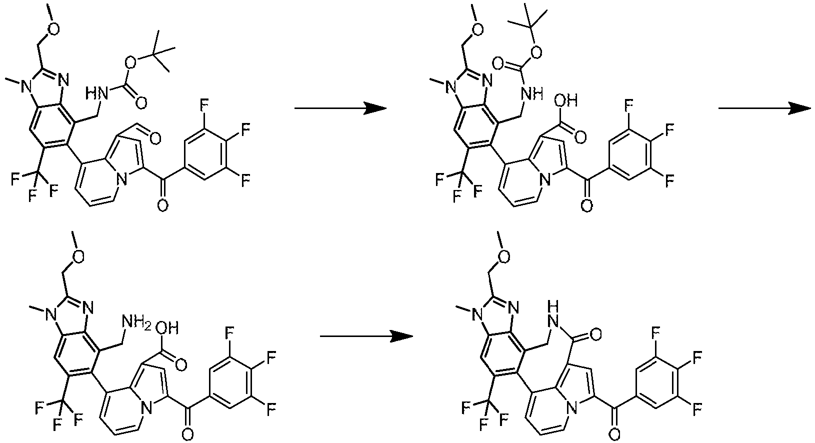

To a mixture of *tert*-butyl ((5-(1-formyl-3-(3,4,5-trifluorobenzoyl)indolizin-8-yl)-2-(methoxymethyl)-1-methyl-6-(trifluoromethyl)-1H-benzo[d]imidazol-4-yl)methyl)carbamate (53.0 mg, 0.0786 mmol), NaPH₂O₄ (aq.) (0.70M, 0.786 mL, 0.550 mmol), 2-methyl-2-butene (0.416 mL, 3.93 mmol) and sulfamic acid (76.3 mg, 0.786 mmol) in tert-butanol (0.40 mL) and THF (0.40 mL) was added sodium chlorite (42.6 mg, 0.471 mmol), and stirred at room temperature for 15 minutes. EtOAc and sat. NaHCO₃ aq. were added thereto, and the layers were mixed and separated. The separated organic layer was washed with brine, dried over Na₂SO₄,, filtered, and evaporated. The resulting crude product 8-(4-(((*tert*-butoxycarbonyl)amino)methyl)-2-(methoxymethyl)-1-methyl-6-(trifluoromethyl)-1H-benzo[d]imidazol-5-yl)-3-(3,4,5-trifluorobenzoyl)indolizine-1-carboxylic acid was diluted with MeOH (0.50 mL) and added HCl in dioxane (4.0M, 0.500 mL, 2.00 mmol). After stirring the mixture at room temperature for 30 minutes, EtOAc and sat. NaHCO₃ (aq.) were added thereto, and the layers were mixed and separated. The separated organic layer was washed with brine, dried over Na₂SO₄,, filtered, and evaporated. The resulting crude product 8-(4-(aminomethyl)-2-(methoxymethyl)-1-methyl-6-(trifluoromethyl)-1H-benzo[d]imidazol-5-yl)-3-(3,4,5-trifluorobenzoyl)indolizine-1-carboxylic acid was diluted with THF (4.6 mL) and added 1-(3-dimethylaminopropyl)-3-ethylcarbodiimide hydrochloride (30.1 mg, 0.157 mmol), 1-hydroxybenzotriazole hydrate (24.1 mg, 0.157 mmol), and triethylamine (0.219 mL, 0.157 mmol). After stirring at 50 °C for 1 hour, the mixture was diluted with EtOAc and sat. NaHCO₃ (aq.), and the layers were mixed and separated. The separated organic layer was washed with brine, dried over Na₂SO₄, filtered, and evaporated. The residue was purified by column chromatography on silica gel (hexane-EtOAc/EtOH(4/1)) to obtain 10-(methoxymethyl)-9-methyl-2-(3,4,5-trifluorobenzoyl)-7-(trifluoromethyl)-12,13-dihydroimidazo[4",5":3',4']benzo[1',2':6,7]azocino[5,4,3-hi]indolizin-14(9H)-one.

### Step 9:

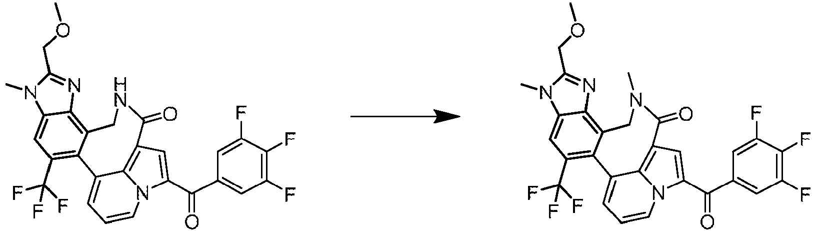

To a mixture of 10-(methoxymethyl)-9-methyl-2-(3,4,5-trifluorobenzoyl)-7-(trifluoromethyl)-12,13-dihydroimidazo[4",5":3',4']benzo[1',2':6,7]azocino[5,4,3-hi]indolizin-14(9H)-one (23.7 mg, 0.0414 mmol) and methyl iodide (7.75 µL, 0.124 mmol) in DMF (0.40 mL) was added sodium hydride (60 wt%, 3.31 mg, 0.0828 mmol). After stirring the mixture at room temperature for 1 hour, methyl iodide (7.75 µL, 0.124 mmol) and sodium hydride (60 wt%, 3.31 mg, 0.0828 mmol) were added thereto. The mixture was stirred at the same temperature for another 1 hour, and diluted with EtOAc and sat. NaHCO₃ (aq.). The layers were mixed and separated. The separated organic layer was washed with brine, dried over Na₂SO₄, filtered, and evaporated. The residue was purified by column chromatography on silica gel (hexane-EtOAc/EtOH(4/1)) to obtain 10-(methoxymethyl)-9,13-dimethyl-2-(3,4,5-trifluorobenzoyl)-7-(trifluoromethyl)-12,13-dihydroimidazo[4",5":3',4']benzo[1',2':6,7]azocino[5,4,3-hi]indolizin-14(9H)-one.

### Step 10:

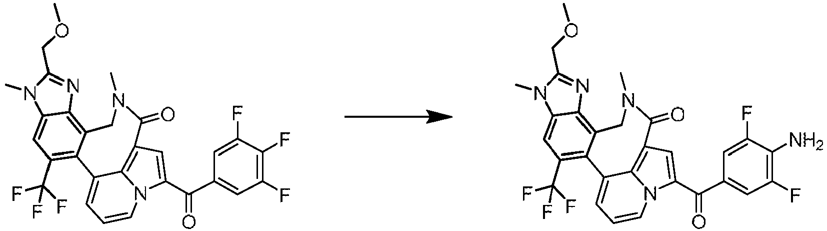

To a solution of 10-(methoxymethyl)-9,13-dimethyl-2-(3,4,5-trifluorobenzoyl)-7-(trifluoromethyl)-12,13-dihydroimidazo[4",5":3',4']benzo[1',2':6,7]azocino[5,4,3-hi]indolizin-14(9H)-one (26.5 mg, 0.0452 mmol) in 1,4-dioxane (2.0 mL) was added 28-30% NH₃ aq. (3.0 mL). The reaction mixture was heated at 130 °C under microwave irradiation for 12 hours. After cooling, the mixture was diluted with EtOAc, washed with brine then dried over Na₂SO₄ and evaporated. The residue was purified by column chromatography on silica gel (hexane-EtOAc/EtOH(4/1)) to obtain 2-(4-amino-3,5-difluorobenzoyl)-10-(methoxymethyl)-9,13-dimethyl-7-(trifluoromethyl)-12,13-dihydroimidazo[4",5":3',4']benzo[1',2':6,7]azocino[5,4,3-hi]indolizin-14(9H)-one.

### Step 11:

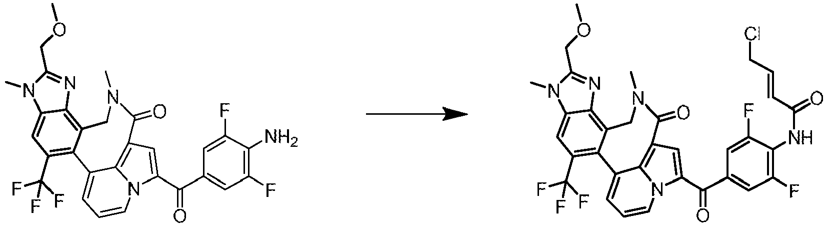

A solution of 2-(4-amino-3,5-difluorobenzoyl)-10-(methoxymethyl)-9,13-dimethyl-7-(trifluoromethyl)-12,13-dihydroimidazo[4",5":3',4']benzo[1',2':6,7]azocino[5,4,3-hi]indolizin-14(9H)-one (26.4 mg, 0.0452 mmol) and triethylamine (0.126 mL, 0.181 mmol) in DMF (1.0 mL) was slowly added 0.895 mL of 4-chlorocrotonic acid T3P solution (prepared from (E)-4-chlorobut-2-enoic and T3P (50 wt% in THF)(100 mg 4-chlorocrotonic acid / mL)). Additional 4-chlorocrotonic acid T3P solution (0.113 mL) was added thereto. The resulting mixture was diluted with EtOAc, washed with sat. NaHCO₃ (aq.), H₂O, and brine then dried over Na₂SO₄, filtered, and evaporated. The residue was purified by column chromatography on silica gel (hexane-EtOAc/EtOH(4/1)) to obtain (E)-4-chloro-N-(2,6-difluoro-4-(10-(methoxymethyl)-9,13-dimethyl-14-oxo-7-(trifluoromethyl)-9,12,13,14-tetrahydroimidazo[4",5":3',4']benzo[1',2':6,7]azocino[5,4,3-hi]indolizine-2-carbonyl)phenyl)but-2-enamide.

### Step 12:

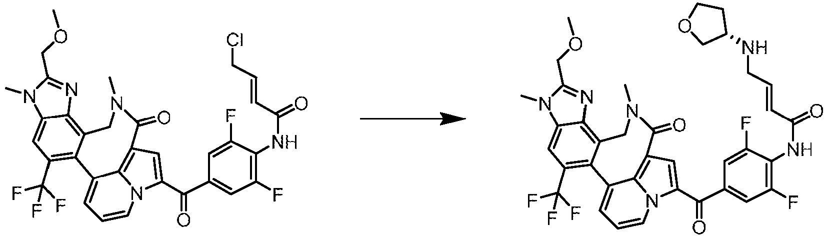

To a solution of (E)-4-chloro-N-(2,6-difluoro-4-(10-(methoxymethyl)-9,13-dimethyl-14-oxo-7-(trifluoromethyl)-9,12,13,14-tetrahydroimidazo[4",5":3',4']benzo[1',2':6,7]azocino[5,4,3-hi]indolizine-2-carbonyl)phenyl)but-2-enamide (28.9 mg, 0.0421 mmol) in DMSO (0.50 mL) was added (S)-tetrahydrofuran-3-amine (18.4 µL, 0.211 mmol), and the mixture was stirred at 40 °C for 2 hours. The mixture was purified by preparative reversed-phase HPLC (water:acetonitrile (0.1% formic acid)). Sat. NaHCO₃ (aq.) was added to the purified fractions containing the title compound, followed by extraction with ethyl acetate. The extract was washed with brine, dried over sodium sulfate, and filtered. The solvent was evaporated under reduced pressure, thereby obtaining (S,E)-N-(2,6-difluoro-4-(10-(methoxymethyl)-9,13-dimethyl-14-oxo-7-(trifluoromethyl)-9,12,13,14-tetrahydroimidazo[4",5":3',4']benzo[1',2':6,7]azocino[5,4,3-hi]indolizine-2-carbonyl)phenyl)-4-((tetrahydrofuran-3-yl)amino)but-2-enamide as an atropmixture.¹H-NMR: (400 MHz, CHLOROFORM-D) δ 10.01 (dd, J = 6.9, 1.1 Hz, 1H), 7.87 (s, 1H), 7.85 (s, 1H), 7.48 (s, 1H), 7.46 (s, 1H), 7.20 (d, J = 7.3 Hz, 1H), 7.13-7.07 (m, 2H), 6.29 (d, J = 15.3 Hz, 1H), 5.27 (d, J = 14.5 Hz, 1H), 5.06 (d, J = 14.5 Hz, 1H), 4.83 (s, 2H), 3.97-3.92 (m, 4H), 3.84-3.76 (m, 2H), 3.62 (dd, J = 9.1, 3.6 Hz, 1H), 3.48-3.45 (m, 6H), 3.15 (s, 3H), 2.17-2.08 (m, 1H), 1.79-1.74 (m, 1H); ESI-MS [M+H]⁺: 737.

### Synthetic procedure R

### Example 184

### (S,E)-N-(2,6-difluoro-4-(9-(methoxymethyl)-8,11-dimethyl-6-(trifluoromethyl)-8,11,12,13-tetrahydro-1,2a,8,10,11-pentaazacycloocta[1,2,3-cd:5,4-e']diindene-2-carbonyl)phenyl)-4-((tetrahydrofuran-3-yl)amino)but-2-enamide

### Step 1:

Mixture of atropisomers of (4-amino-3,5-difluorophenyl)(9-(methoxymethyl)-8,11-dimethyl-6-(trifluoromethyl)-8,11,12,13-tetrahydro-1,2a,8,10,11-pentaazacycloocta[1,2,3-cd:5,4-e']diinden-2-yl)methanone (26 mg) was obtained in the same manner as Example 148. The racemate was separated by CHIRALPAK-IG (hexane-IPA) to give 1st-eluting isomer (9.6 mg) and 2nd-eluting isomer.

### Step 2:

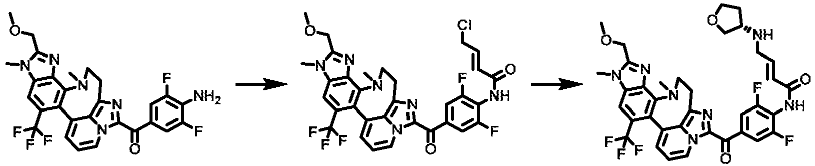

The procedure was performed in the same manner as Example 90 (Steps 2 and 3) using 1st-eluting isomer obtained in Step 1, thereby obtaining (S,E)-N-(2,6-difluoro-4-(9-(methoxymethyl)-8,11-dimethyl-6-(trifluoromethyl)-8,11,12,13-tetrahydro-1,2a,8,10,11-pentaazacycloocta[1,2,3-cd:5,4-e']diindene-2-carbonyl)phenyl)-4-((tetrahydrofuran-3-yl)amino)but-2-enamide. ¹H-NMR: (400 MHz, CHLOROFORM-D) δ 9.88-9.84 (m, 1H), 8.23-8.16 (m, 2H), 7.78 (s, 1H), 7.15-7.06 (m, 3H), 6.98 (brs, 1H), 6.25 (dt, J = 15.3, 1.8 Hz, 1H), 4.85-4.78 (m, 2H), 3.98-3.92 (m, 4H), 3.85-3.77 (m, 2H), 3.64-3.56 (m, 2H), 3.49-3.43 (m, 6H), 3.07 (s, 3H), 3.03-2.92 (m, 2H), 2.51-2.43 (m, 1H), 2.17-2.09 (m, 1H), 1.79-1.72 (m, 1H); ESI-MS [M+H]⁺: 724.

### Synthetic procedure S

### Example 199

### (S,E)-N-(4-(9,12-dimethyl-7-(trifluoromethyl)-9,12,13,14-tetrahydroimidazo[4",5":5',6']benzo[1',2':2,3]azocino[4,5,6-hi]indolizine-2-carbonyl)-2,6-difluorophenyl)-4-(2-(hydroxymethyl)azetidin-1-yl)but-2-enamide

### Step 1:

Mixture of atropisomers of (4-amino-3,5-difluorophenyl)(9,12-dimethyl-7-(trifluoromethyl)-9,12,13,14-tetrahydroimidazo[4",5":5',6']benzo[1',2':2,3]azocino[4,5,6-hi]indolizin-2-yl)methanone (885 mg) was obtained in the same manner as Example 71. The racemate was separated by CHIRALPAK-IE (hexane-IPA) to give 1st-eluting isomer (405 mg) and 2nd-eluting isomer.

### Step 2:

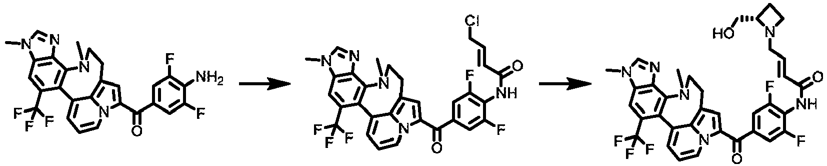

The procedure was performed in a similar manner as Example 90 (Steps 2 and 3) using 2nd-eluting isomer obtained in Step 1, thereby obtaining (S,E)-N-(4-(9,12-dimethyl-7-(trifluoromethyl)-9, 12, 13, 14-tetrahydroimidazo[4",5":5',6']benzo[1',2':2,3]azocino[4,5,6-hi]indolizine-2-carbonyl)-2,6-difluorophenyl)-4-(2-(hydroxymethyl)azetidin-1-yl)but-2-enamide. ¹H-NMR: (400 MHz, CHLOROFORM-D) δ 9.97 (dd, J = 6.9, 1.1 Hz, 1H), 8.03 (s, 1H), 7.80 (s, 1H), 7.45 (d, J = 8.0 Hz, 2H), 7.12 (s, 1H), 7.05 (d, J = 7.3 Hz, 1H), 7.02-6.93 (m, 2H), 6.22 (d, J = 15.3 Hz, 1H), 3.93 (s, 3H), 3.68-3.60 (m, 2H), 3.50-3.42 (m, 5H), 3.27-3.21 (m, 1H), 3.11 (s, 3H), 2.96-2.86 (m, 2H), 2.56 (d, J = 15.0 Hz, 1H), 2.32-2.22 (m, 2H), 2.03-1.96 (m, 1H); ESI-MS [M+H]⁺: 679.

### Synthetic procedure T

### Example 209

### (S,E)-N-(2,6-difluoro-4-(10-(methoxymethyl)-9-methyl-7-(trifluoromethyl)-9,12-dihydro-14H-imidazo[4",5":3',4']benzo[1',2':6,7]oxocino[5,4,3-hi]indolizine-2-carbonyl)phenyl)-4-((tetrahydrofuran-3-yl)amino)but-2-enamide

### Step 1:

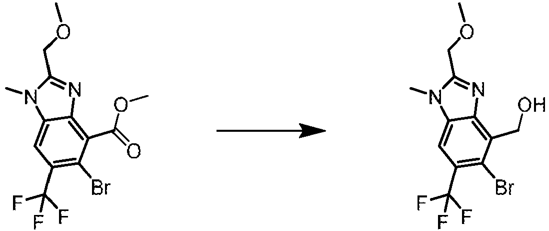

To a solution of methyl 5-bromo-2-(methoxymethyl)-1-methyl-6-(trifluoromethyl)-1*H*-benzo[d]imidazole-4-carboxylate (440 mg, 1.15 mmol) in dichloromethane (12 mL) was added diisobutylaluminium hydrdide in hexane (1.0M, 3.46 mL, 3.46 mmol) at 0 °C, and the mixture was stirred at 0 °C for 30 minutes. MeOH and 30% potassium sodium tartrate (aq.) were added to the reaction mixture and stirred at room temperature for 30 minutes. The resulting mixture was diluted with EtOAc and washed with brine, dried overn Na₂SO₄, evaporated to obtain (5-bromo-2-(methoxymethyl)-1-methyl-6-(trifluoromethyl)-1H-benzo[d]imidazol-4-yl)methanol.

### Step 2:

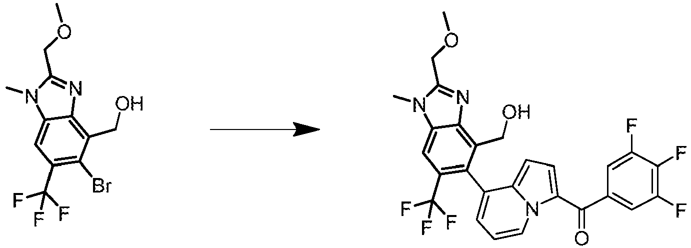

(5-bromo-2-(methoxymethyl)-1-methyl-6-(trifluoromethyl)-1*H-*benzo[d]imidazol-4-yl)methanol (383 mg, 1.08 mmol), (8-(5,5-dimethyl-1,3,2-dioxaborinan-2-yl)indolizin-3-yl)(3,4,5-trifluorophenyl)methanone (840 mg, 2.17 mmol), S-Phos Pd G3 (53.5 mg, 0.0759 mmol) in THF (6.0 mL) and 2M K₃PO₄ aq. (1.36 mL, 2.71 mmol) was evacuated and N₂ back-filled before heating to 80 °C for 4 hours. After cooling, the reaction mixture was diluted with EtOAc. The resulting suspension was filtered, rinsing with EtOAc, the filtrate was washed with H₂O and brine then dried over Na₂SO₄ and evaporated. The residue was purified by column chromatography on silica gel (hexane-EtOAc/EtOH(4/1)) to obtain (8-(4-(hydroxymethyl)-2-(methoxymethyl)-1-methyl-6-(trifluoromethyl)-1*H*-benzo[d]imidazol-5-yl)indolizin-3-yl)(3,4,5-trifluorophenyl)methanone.

### Step 3:

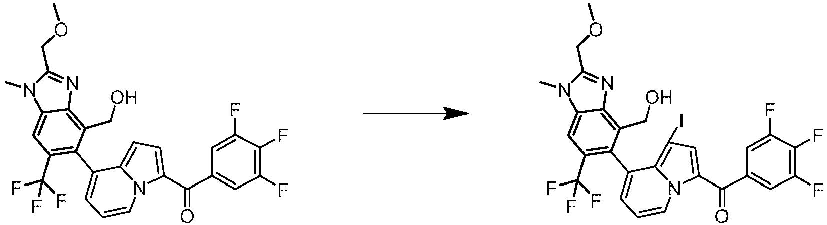

N-Iodosuccinimide (169 mg, 0.751 mmol) and TFA (0.139 mL, 1.88 mmol) were added (8-(4-(hydroxymethyl)-2-(methoxymethyl)-1-methyl-6-(trifluoromethyl)-1*H-*benzo[d]imidazol-5-yl)indolizin-3-yl)(3,4,5-trifluorophenyl)methanone (411 mg, 0.751 mmol) in THF (4.0 mL). After stirring at room temperature overnight, the mixture was diluted with EtOAc, and sat. NaHCO₃ aq. and sat. Na₂S₂O₃ aq. were added thereto. The organic layer was separated, and washed with a saturated sodium chloride solution, followed by drying over sodium sulfate. After evaporation, the obtained residue was purified by column chromatography on silica gel (hexane-EtOAc/EtOH(4/1)) to obtain (8-(4-(hydroxymethyl)-2-(methoxymethyl)-1-methyl-6-(trifluoromethyl)-1*H-*benzo[d]imidazol-5-yl)-1-iodoindolizin-3-yl)(3,4,5-trifluorophenyl)methanone.

### Step 4:

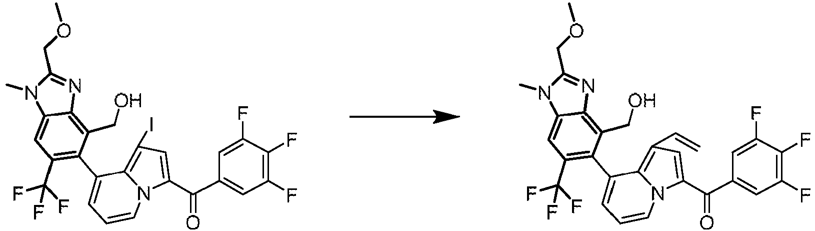

A mixture of (8-(4-(hydroxymethyl)-2-(methoxymethyl)-1-methyl-6-(trifluoromethyl)-1*H*-benzo[d]imidazol-5-yl)-1-iodoindolizin-3-yl)(3,4,5-trifluorophenyl)methanone (584 mg, 0.867 mmol), potassium vinyltrifluoroborate (290 mg, 2.17 mmol), PdCl₂(dppf) ·CH₂Cl₂ (70.8 mg, 0.0867 mmol) in 1,4-dioxane (12 mL) and 2M Na₂CO₃ aq. (1.43 mL, 2.86 mmol) was evacuated and N₂ back-filled before heating to 100 °C for 3 hours. After cooling, the reaction mixture was diluted with EtOAc, washed with H₂O and brine then dried over Na₂SO₄ and evaporated. The residue was purified by column chromatography on silica gel (hexane-acetone) to obtain (8-(4-(hydroxymethyl)-2-(methoxymethyl)-1-methyl-6-(trifluoromethyl)-1*H*-benzo[d]imidazol-5-yl)-1-vinylindolizin-3-yl)(3,4,5-trifluorophenyl)methanone.

### Step 5:

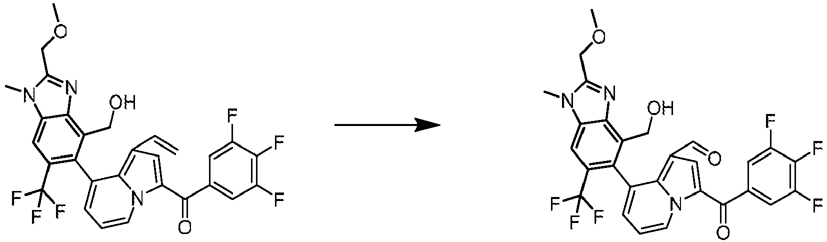

To a mixture of (8-(4-(hydroxymethyl)-2-(methoxymethyl)-1-methyl-6-(trifluoromethyl)-1*H*-benzo[d]imidazol-5-yl)-1-vinylindolizin-3-yl)(3,4,5-trifluorophenyl)methanone (461 mg, 0.804 mmol), lutidine (0.187 mL, 1.61 mmol) and NaIO₄ (688 mg, 3.22 mmol) in 1,4-dioxane (30 mL) and H₂O (1.45 mL) was added 1% O_{S}O₄ aq. (0.409 mL, 0.0161 mmol). After stirring at room temperature overnight, the reaction mixture was diluted with EtOAc. The resulting suspension was filtered, rinsing with EtOAc, the filtrate was washed with H₂O and brine then dried over Na₂SO₄ and evaporated. The residue was purified by column chromatography on silica gel (hexane-EtOAc/EtOH(4/1)) to obtain 8-(4-(hydroxymethyl)-2-(methoxymethyl)-1-methyl-6-(trifluoromethyl)-1*H*-benzo[d]imidazol-5-yl)-3-(3,4,5-trifluorobenzoyl)indolizine-1-carbaldehyde.

### Step 6:

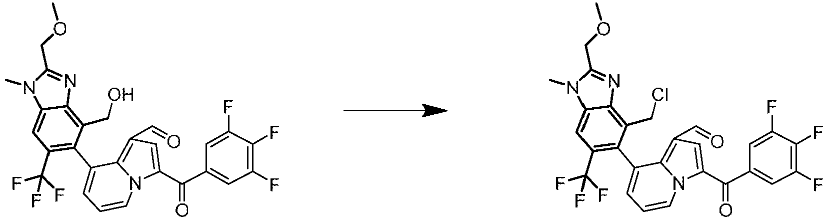

To a mixture of 8-(4-(hydroxymethyl)-2-(methoxymethyl)-1-methyl-6-(trifluoromethyl)-1*H*-benzo[d]imidazol-5-yl)-3-(3,4,5-trifluorobenzoyl)indolizine-1-carbaldehyde (197 mg, 0.342 mmol) and pyridine (0.111 mL, 1.37 mmol) in dichloromethane (4.0 mL) was added SOCl₂ (0.0499 mL, 0.685 mmol) at 0 °C, and stirred at the same temperature for 3.5 hours. Additional pyridine (0.333 mL, 4.11 mmol) and SOCl₂ (0.150 mL, 2.06 mmol) were added thereto and the reaction mixture was stirred at 0 °C for 30 minutes. Further pyridine (0.222 mL, 2.74 mmol) and SOCl₂ (0.100 mL, 1.37 mmol) were added to the reaction mixture two times and stirred at 0 °C to room temperature overnight. The resulting mixture was diluted with EtOAc and washed with brine, dried over Na₂SO₄, then evaporated. The residue was purified by column chromatography on silica gel (hexane-EtOAc/EtOH(4/1)) to obtain 8-(4-(chloromethyl)-2-(methoxymethyl)-1-methyl-6-(trifluoromethyl)-1H-benzo[d]imidazol-5-yl)-3-(3,4,5-trifluorobenzoyl)indolizine-1-carbaldehyde.

### Step 7:

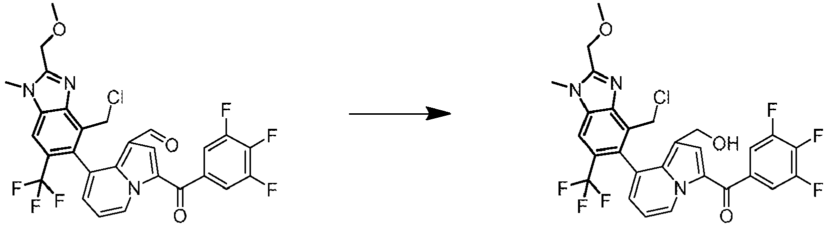

Sodium triacetoxyborohydride (164 mg, 0.775 mmol) and acetic acid (0.0443 mL, 0.775 mmol) were added to 8-(4-(chloromethyl)-2-(methoxymethyl)-1-methyl-6-(trifluoromethyl)-1*H*-benzo[d]imidazol-5-yl)-3-(3,4,5-trifluorobenzoyl)indolizine-1-carbaldehyde (92.0 mg, 0.155 mmol) in THF (3.0 mL). After stirring at room temperature overnight, the solvent was removed under reduced pressure. The residue was diluted with EtOAc and water, extracted with EtOAc. The separated organic phase was washed with brine and dried over sodium sulfate, filtered, evaporated to obtain (8-(4-(chloromethyl)-2-(methoxymethyl)-1-methyl-6-(trifluoromethyl)-1H-benzo[d]imidazol-5-yl)-1-(hydroxymethyl)indolizin-3-yl)(3,4,5-trifluorophenyl)methanone.

### Step 8:

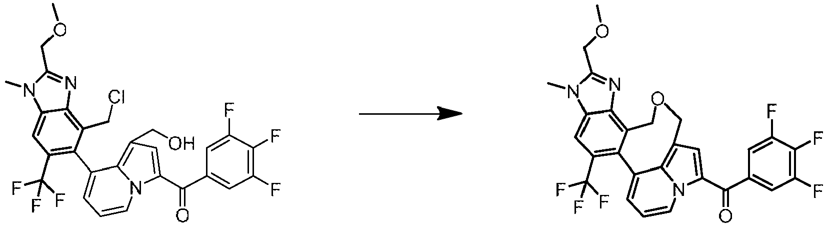

A mixture of (8-(4-(chloromethyl)-2-(methoxymethyl)-1-methyl-6-(trifluoromethyl)-1*H*-benzo[d]imidazol-5-yl)-1-(hydroxymethyl)indolizin-3-yl)(3,4,5-trifluorophenyl)methanone (92.4 mg, 0.155 mmol) and sodium hydride (ca.60 wt%, 12.4 mg, 0.310 mmol) in DMF (3.0 mL) was stirred at room temperature for 30 minutes. Then EtOAc and H₂O were added thereto, the separated orgaic layer was washed with brine, dried over Na₂SO₄, then evaporated. The residue was purified by column chromatography on silica gel (hexane-EtOAc/EtOH(4/1)) to obtain (10-(methoxymethyl)-9-methyl-7-(trifluoromethyl)-9,12-dihydro-14*H*-imidazo[4",5":3',4']benzo[1',2':6,7]oxocino[5,4,3-hi]indolizin-2-yl)(3,4,5-trifluorophenyl)methanone.

### Step 9:

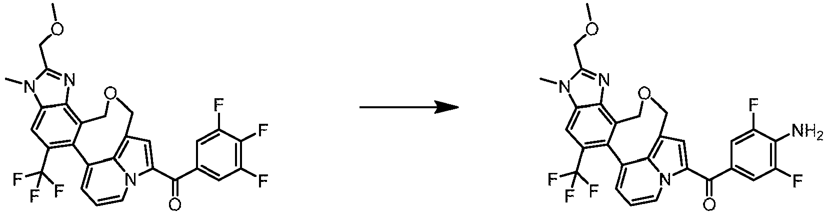

To a solution of (10-(methoxymethyl)-9-methyl-7-(trifluoromethyl)-9,12-dihydro-14*H*-imidazo[4",5":3',4']benzo[1',2':6,7]oxocino[5,4,3-hi]indolizin-2-yl)(3,4,5-trifluorophenyl)methanone (81.0 mg, 0.145 mmol) in 1,4-dioxane (2.5 mL) was added 28-30% NH₃ aq. (4.0 mL). The reaction mixture was heated at 130 °C under microwave irradiation for 12 hours. After cooling, the mixture was diluted with EtOAc, washed with brine then dried over Na₂SO₄ and evaporated. The residue was purified by column chromatography on silica gel (hexane-EtOAc/EtOH(4/1)) to obtain (4-amino-3,5-difluorophenyl)(10-(methoxymethyl)-9-methyl-7-(trifluoromethyl)-9,12-dihydro-14H-imidazo[4",5":3',4']benzo[1',2':6,7]oxocino[5,4,3-hi]indolizin-2-yl)methanone.

### Step 10:

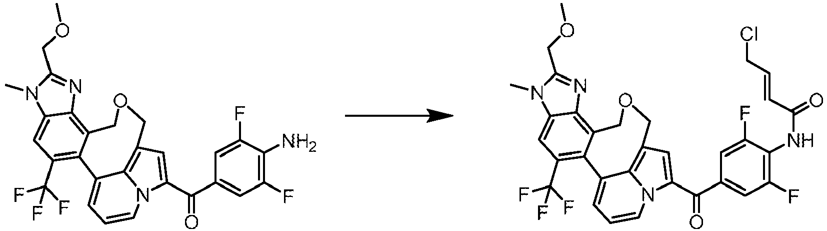

To a solution of (4-amino-3,5-difluorophenyl)(10-(methoxymethyl)-9-methyl-7-(trifluoromethyl)-9,12-dihydro-14*H*-imidazo[4",5":3',4']benzo[1',2':6,7]oxocino[5,4,3-hi]indolizin-2-yl)methanone (78.9 mg, 0.142 mmol), 4-chlorocrotonic acid (18.8 mg, 0.156 mmol) and T3P (50 wt% in THF, 0.133 mL, 0.213 mmol) in THF (1.0 mL) was added triethylamine (0.0593 mL, 0.425 mmol). After stirring at room temperature for 15 minutes, T3P (50 wt% in THF, 0.133 mL, 0.213 mmol) and triethylamine (0.0593 mL, 0.425 mmol) were added thereto. After stirring at room temperature for another 15 minutes, the resulting mixture was diluted with EtOAc, washed with sat. NaHCO₃ (aq.), H₂O and brine then dried over Na₂SO₄ and evaporated to obtain (E)-4-chloro-N-(2,6-difluoro-4-(10-(methoxymethyl)-9-methyl-7-(trifluoromethyl)-9,12-dihydro-14*H-*imidazo[4",5":3',4']benzo[1',2':6,7]oxocino[5,4,3-hi]indolizine-2-carbonyl)phenyl)but-2-enamide.

### Step 11:

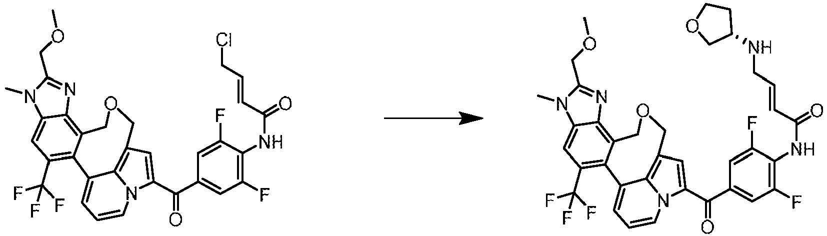

To a solution of (E)-4-chloro-N-(2,6-difluoro-4-(10-(methoxymethyl)-9-methyl-7-(trifluoromethyl)-9,12-dihydro-14*H*-imidazo[4",5":3',4']benzo[1',2':6,7]oxocino[5,4,3-hi]indolizine-2-carbonyl)phenyl)but-2-enamide (95.6 mg, 0.145 mmol) in DMSO (0.50 mL) was added (S)-tetrahydrofuran-3-amine (63.4 µL, 0.725 mmol), and the mixture was stirred at room temperature for 3 hours. The mixture was purified by preparative reversed-phase HPLC (water:acetonitrile (0.1% formic acid)). Sat. NaHCO₃ (aq.) was added to the purified fractions containing the title compound, followed by extraction with ethyl acetate. The extract was washed with brine and dried over sodium sulfate. The solvent was evaporated under reduced pressure, thereby obtaining (S,E)-N-(2,6-difluoro-4-(10-(methoxymethyl)-9-methyl-7-(trifluoromethyl)-9,12-dihydro-14H-imidazo[4",5":3',4']benzo[1',2':6,7]oxocino[5,4,3-hi]indolizine-2-carbonyl)phenyl)-4-((tetrahydrofuran-3-yl)amino)but-2-enamide. ¹H-NMR: (400 MHz, CHLOROFORM-D) δ 9.96 (dd, J = 7.0, 1.0 Hz, 1H), 7.94 (s, 1H), 7.47 (s, 1H), 7.45 (s, 1H), 7.33 (s, 1H), 7.19-7.04 (m, 4H), 6.28 (d, J = 15.3 Hz, 1H), 5.63 (d, J = 11.5 Hz, 1H), 4.86 (dd, J = 24.0, 12.8 Hz, 2H), 4.65 (d, J = 11.5 Hz, 1H), 4.43 (d, J = 13.3 Hz, 1H), 4.21 (d, J = 13.0 Hz, 1H), 3.99 (s, 3H), 3.97-3.93 (m, 1H), 3.85-3.77 (m, 2H), 3.63 (dd, J = 9.0, 3.8 Hz, 1H), 3.49-3.43 (m, 6H), 2.18-2.09 (m, 1H), 1.80-1.72 (m, 1H); ESI-MS [M+H]⁺: 710.

### Synthetic procedure U

### Example 215

### (E)-N-(2,6-difluoro-4-(9-(2-methoxyethyl)-12-methyl-7-(trifluoromethyl)-9,12,13,14-tetrahydroimidazo[4",5":5',6']benzo[1',2':2,3]azocino[4,5,6-hi]indolizine-2-carbonyl)phenyl)-4-((1-methylcyclopropyl)amino)but-2-enamide

### Step 1:

Mixture of atropisomers of (4-amino-3,5-difluorophenyl)(9-(2-methoxyethyl)-12-methyl-7-(trifluoromethyl)-9,12,13,14-tetrahydroimidazo[4",5":5',6']benzo[1',2':2,3]azocino[4,5,6-hi]indolizin-2-yl)methanone (1.46 g) was obtained in the same manner as Example 71. The racemate was separated by CHIRAL ART SB (hexane-IPA) to give 1st-eluting isomer and 2nd-eluting isomer.

### Step 2:

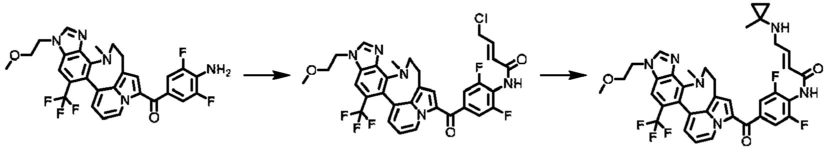

The procedure was performed in a similar manner as Example 90 (Steps 2 and 3) using 2nd-eluting isomer obtained in Step 1, thereby obtaining (E)-N-(2,6-difluoro-4-(9-(2-methoxyethyl)-12-methyl-7-(trifluoromethyl)-9,12,13,14-tetrahydroimidazo[4",5":5',6']benzo[1',2':2,3]azocino[4,5,6-hi]indolizine-2-carbonyl)phenyl)-4-((1-methylcyclopropyl)amino)but-2-enamide. ¹H-NMR: (400 MHz, CHLOROFORM-D) δ 9.97 (dd, J = 6.8, 1.0 Hz, 1H), 8.13 (s, 1H), 7.83 (s, 1H), 7.42 (d, J = 8.0 Hz, 2H), 7.16-7.10 (m, 2H), 7.06 (d, J = 7.3 Hz, 1H), 7.00 (t, J = 7.0 Hz, 1H), 6.28-6.24 (m, 1H), 4.41 (t, J = 5.0 Hz, 2H), 3.79 (t, J = 5.1 Hz, 2H), 3.56 (dd, J = 5.1, 1.9 Hz, 2H), 3.47-3.43 (m, 1H), 3.39 (s, 3H), 3.11 (s, 3H), 2.93-2.86 (m, 1H), 2.58-2.53 (m, 1H), 2.30 (td, J = 13.9, 4.3 Hz, 1H), 1.30 (s, 3H), 0.64 (t, J = 5.1 Hz, 2H), 0.42 (dd, J = 6.3, 4.5 Hz, 2H); ESI-MS [M+H]⁺: 707.

### Synthetic procedure V

### Example 240

### (E)-N-(2,6-difluoro-4-(7-methoxy-9-(2-methoxyethyl)-10-methyl-13,14-dihydro-9H-imidazo[4",5":5',6']benzo[1',2':2,3]thiocino[4,5,6-hi]indolizine-2-carbonyl)phenyl)-4-((1-methylcyclopropyl)amino)but-2-enamide

### Step 1:

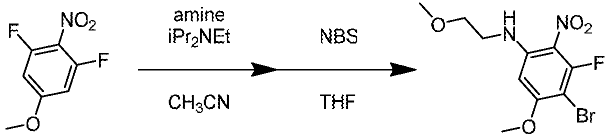

2-Methoxyethylamine (3.31 mL, 38 mmol) and *N,N*-diisopropylethylamine (13.3 mL, 76.1 mmol) was slowly added to a solution of 1,3-difluoro-5-methoxy-2-nitrobenzene (6.54 g, 34.6 mmol) in acetonitrile (150 mL) at room temperature. After stirring at room temperature overnight, water and EtOAc were added to the resulting mixture. The organic layer was separated, and washed with brine, followed by drying over sodium sulfate. After evaporation, to the obtained residue was added THF (100 mL), and NBS (6.16 g, 34.6 mmol) was added thereto at 0 °C. After stirring for 10 minutes, the precipitates were collected by filtration and washed with diisopropylether to obtain 4-bromo-3-fluoro-5-methoxy-N-(2-methoxyethyl)-2-nitroaniline (4.67 g, 42 %). The filtrates were diluted with EtOAc and washed with saturated NaHCO₃ (aq.) and brine, dried over Na₂SO₄ and evaporated. The residue was diluted with diisopropylether, and collected by filtration to obtain 4-bromo-3-fluoro-5-methoxy-N-(2-methoxyethyl)-2-nitroaniline.

### Step 2:

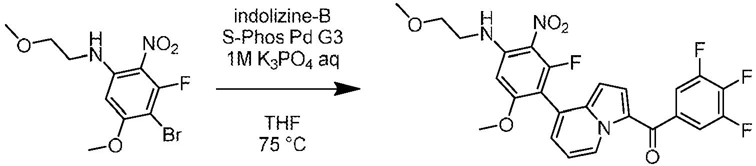

A mixture of 4-bromo-3-fluoro-5-methoxy-N-(2-methoxyethyl)-2-nitroaniline (4.49 g, 10 mmol, 72 wt%), (8-(5,5-dimethyl-1,3,2-dioxaborinan-2-yl)indolizin-3-yl)(3,4,5-trifluorophenyl)methanone, (5.00 g, 12.9 mmol), S-Phos Pd G3 (360 mg) in THF (70 mL) and 1M K₃PO₄ aq. (15 mL, 15.0 mmol) was evacuated and N₂ back-filled before heating to 75 °C for 1 hour. After cooling, the reaction mixture was diluted with EtOAc and brine. The organic layer was separated, and dried over sodium sulfate. After evaporation, the resulting residue was suspended with diisopropyl ether and collected by filtration to obtain (8-(2-fluoro-6-methoxy-4-((2-methoxyethyl)amino)-3-nitrophenyl)indolizin-3-yl)(3,4,5-trifluorophenyl)methanone.

### Step 3:

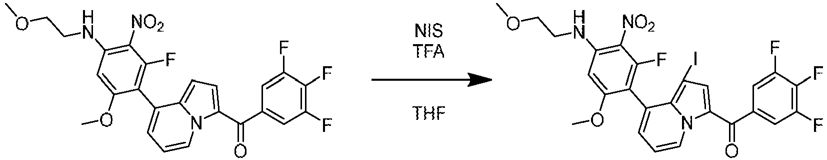

*N*-Iodosuccinimide (1.58 g, 7.02 mmol) and TFA (2.61 mL, 35.1 mmol) were added to a solution of (8-(2-fluoro-6-methoxy-4-((2-methoxyethyl)amino)-3-nitrophenyl)indolizin-3-yl)(3,4,5-trifluorophenyl)methanone (3.63 g, 7.02 mmol) in THF (100 mL). After stirring at room temperature for 2.5 hours, the mixture was diluted with EtOAc and saturated sodium bicarbonate solution. The organic layer was separated, and washed with brine, followed by drying over sodium sulfate. After evaporation, the precipitate was collected by filtration to obtain crude (8-(2-fluoro-6-methoxy-4-((2-methoxyethyl)amino)-3-nitrophenyl)-1-iodoindolizin-3-yl)(3,4,5-trifluorophenyl)methanone.

### Step 4:

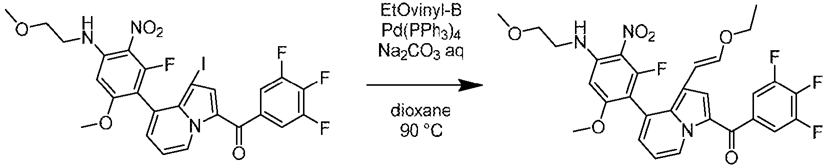

A mixture of crude (8-(2-fluoro-6-methoxy-4-((2-methoxyethyl)amino)-3-nitrophenyl)-1-iodoindolizin-3-yl)(3,4,5-trifluorophenyl)methanone (3.89 g), 2-[(E)-2-ethoxyvinyl]-4,4,5,5-tetramethyl-1,3,2-dioxaborolane (6.40 mL, 30.2 mmol), Pd(PPh₃)₄ (699 mg, 0.605 mmol) in 1,4-dioxane (78 mL) and 2M Na₂CO₃ aq. (15.1 mL, 30.2 mmol) was evacuated and N₂ back-filled before heating to 90 °C for 6 hours. After cooling, the reaction mixture was diluted with EtOAc, washed with H₂O and brine then dried over Na₂SO₄ and evaporated. The residue was purified by column chromatography on silica gel (hexane-CHCl₃) to obtain (£)-(1-(2-ethoxyvinyl)-8-(2-fluoro-6-methoxy-4-((2-methoxyethyl)amino)-3-nitrophenyl)indolizin-3-yl)(3,4,5-trifluorophenyl)methanone.

### Step 5:

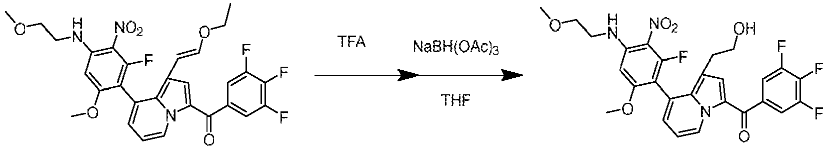

To (*E*)-(1-(2-ethoxyvinyl)-8-(2-fluoro-6-methoxy-4-((2-methoxyethyl)amino)-3-nitrophenyl)indolizin-3-yl)(3,4,5-trifluorophenyl)methanone (2.58 g, 4.39 mmol) was added TFA (16.0 mL) at room temperature. After stirring at room temperature for 5 minutes, water was added thereto. The resulting mixture was diluted with EtOAc. The organic layer was washed with sat. NaHCO₃ (aq.) and brine, dried over Na₂SO₄ and evaporated. To the residue was added THF (70 mL) and sodium triacetoxyborohydride (4.66 g, 22.0 mmol) at room temperature. After stirring at room temperature for 3 days, sat. NaHCO₃ aq. was slowly added thereto. The mixture was extracted with EtOAc, and the organic layer was washed with brine, dried over Na₂SO₄, and evaporated. The residue was purified by column chromatography on silica gel (hexane- CHCl₃-EtOH) to obtain (8-(2-fluoro-6-methoxy-4-((2-methoxyethyl)amino)-3-nitrophenyl)-1-(2-hydroxyethyl)indolizin-3-yl)(3,4,5-trifluorophenyl)methanone.

### Step 6:

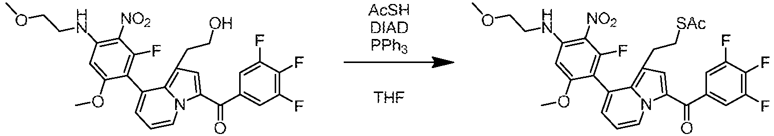

To a solution of triphenylphosphine (786 mg, 3.00 mmol) in THF (10 mL) was added diisopropyl azodicarboxylate (0.509 mL, 3.00 mmol). After the mixture turned to the suspension, the mixture of (8-(2-fluoro-6-methoxy-4-((2-methoxyethyl)amino)-3-nitrophenyl)-1-(2-hydroxyethyl)indolizin-3-yl)(3,4,5-trifluorophenyl)methanone (561 mg, 1.00 mmol) and thioacetic acid (0.127 mL, 1.80 mmol) in THF (7 mL) was added thereto. After stirring for 10 minutes, methanol was added thereto, and the mixture was evaporated. The residue was was purified by column chromatography on silica gel (hexane-CHCl₃) to obtain crude S-(2-(8-(2-fluoro-6-methoxy-4-((2-methoxyethyl)amino)-3-nitrophenyl)-3-(3,4,5-trifluorobenzoyl)indolizin-1-yl)ethyl) ethanethioate.

### Step 7:

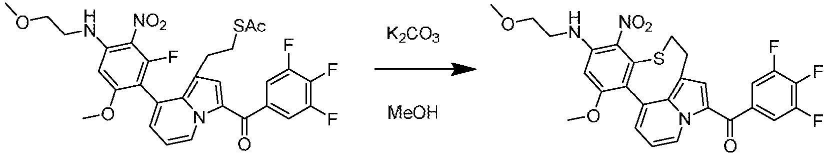

Potassium carbonate (3.655 g, 25.8 mmol) in methanol (250 mL) was evacuated and N₂ back-filled, and crude S-(2-(8-(2-fluoro-6-methoxy-4-((2-methoxyethyl)amino)-3-nitrophenyl)-3-(3,4,5-trifluorobenzoyl)indolizin-1-yl)ethyl) ethanethioate (799 mg) in THF (4 mL) was slowly added thereto. After stirred for 20 minutes, the mixture was neutralized with 2N HCl (aq.), and the methanolic solvent was evaporated. The residue was extracted with EtOAc, and the organic layer was dried over Na₂SO₄, and evaporated to obtain crude (9-methoxy-7-((2-methoxyethyl)amino)-6-nitro-3,4-dihydrobenzo[2,3]thiocino[4,5,6-hi]indolizin-1-yl)(3,4,5-trifluorophenyl)methanone.

### Step 8:

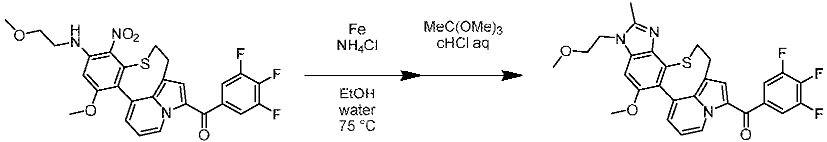

The mixture of crude (9-methoxy-7-((2-methoxyethyl)amino)-6-nitro-3,4-dihydrobenzo[2,3]thiocino[4,5,6-hi]indolizin-1-yl)(3,4,5-trifluorophenyl)methanone (719 mg), iron powder (720 mg, 12.9 mmol) and ammonium chloride (690 mg, 12.9 mmol) in ethanol (16 mL) and water (4 mL) was stirred at 75 °C for 1 hour. The resulting mixture was diluted with EtOAc, and the insoluble matters were removed by filtration. The filtrate was washed with saturated NaHCO₃ (aq.) and brine, dried over Na₂SO₄, and the solvent was removed by evaporation. To the residue was added trimethyl orthoacetate (4.68 mL) and concentrated HCl (100 uL). After stirred for 10 minutes, saturated sodium bicarbonate solution was added thereto. The mixture was extracted with EtOAc, washed with brine, followed by drying over sodium sulfate. After evaporation, the residue was purified by column chromatography on silica gel (CHCl₃-ethanol) to obtain (7-methoxy-9-(2-methoxyethyl)-10-methyl-13,14-dihydro-9H-imidazo[4",5":5',6']benzo[1',2':2,3]thiocino[4,5,6-hi]indolizin-2-yl)(3,4,5-trifluorophenyl)methanone.

### Step 9:

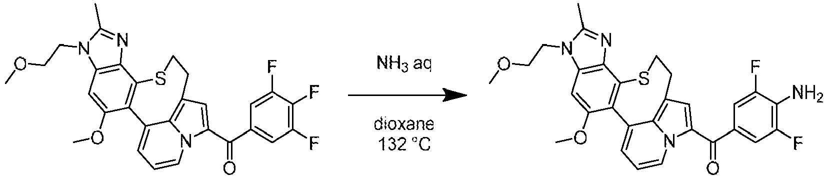

To the mixture of (7-methoxy-9-(2-methoxyethyl)-10-methyl-13,14-dihydro-9H-imidazo[4",5":5',6']benzo[1',2':2,3]thiocino[4,5,6-hi]indolizin-2-yl)(3,4,5-trifluorophenyl)methanone (130 mg, 0.236 mmol) in 1,4-dioxane (1.5 mL) was added 28-30% NH₃ (aq.) (3.0 mL). The reaction mixture was heated at 132 °C by microwave irradiation for 14 hours. After cooling, the mixture was diluted with EtOAc and THF, washed with brine then dried over Na₂SO₄ and evaporated to obtain (4-amino-3,5-difluorophenyl)(7-methoxy-9-(2-methoxyethyl)-10-methyl-13,14-dihydro-9H-imidazo[4",5":5',6']benzo[1',2':2,3]thiocino[4,5,6-hi]indolizin-2-yl)methanone.

### Step 10:

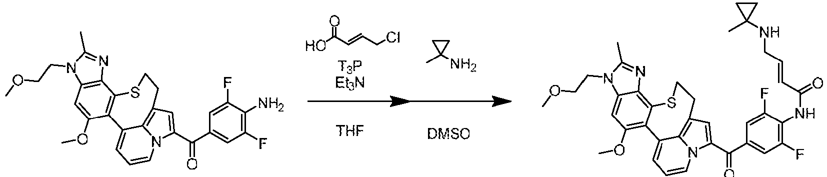

To the mixture of (4-amino-3,5-difluorophenyl)(7-methoxy-9-(2-methoxyethyl)-10-methyl-13,14-dihydro-9*H*-imidazo[4",5":5',6']benzo[1',2':2,3]thiocino[4,5,6-hi]indolizin-2-yl)methanone (129 mg, 0.235 mmol), (*E*)-4-chlorobut-2-enoic acid (36.8 mg, 0.306 mmol) and T3P (50 wt% in THF, 0.735 mL) in THF (3 mL) was added triethylamine (0.257 mL, 1.88 mmol). After stirred at room temperature for 1 hour, the resulting mixture was diluted with EtOAc, washed with NaHCO₃ (aq.), H₂O and brine then dried over Na₂SO₄ and evaporated. To the residue was added DMSO (0.9 mL) and 1-methylcyclopropanamine (0.186 mL) and the mixture was stirred at 40 °C for 2.5 hours. Water was added thereto, and the mixture was extracted with EtOAc. The extract was washed with brine, dried over sodium sulfate, and evaporated. The residue was purified by column chromatography on silica gel (CHCl₃-ethanol) to obtain *(E)-N-(2,6-*difluoro-4-(7-methoxy-9-(2-methoxyethyl)-10-methyl-13,14-dihydro-9H-imidazo[4",5":5',6']benzo[1',2':2,3]thiocino[4,5,6-hi]indolizine-2-carbonyl)phenyl)-4-((1-methylcyclopropyl)amino)but-2-enamide.

The racemate was separated by CHIRALPAK-ID (hexane-EtOH(0.1%Et₃N):30/70) to obtain (E)-N-(2,6-difluoro-4-(7-methoxy-9-(2-methoxyethyl)-10-methyl-13,14-dihydro-9H-imidazo[4",5":5',6'benzo[1',2":2,3]thiocino[4,5,6-hi]indolizine-2-carbonyl)phenyl)-4-((1-methylcyclopropyl)amino)but-2-enamide (2^{nd} elution). ¹H-NMR: (400 MHz, CHLOROFORM-D) δ 10.03 (dd, J = 7.0, 1.0 Hz, 1H), 7.78 (s, 1H), 7.35-7.32 (m, 2H), 7.19-7.09 (m, 3H), 7.05 (t, J = 7.0 Hz, 1H), 7.00 (s, 1H), 6.29 (dd, J = 13.6, 1.6 Hz, 1H), 4.30 (t, J = 5.4 Hz, 2H), 3.78 (s, 3H), 3.74 (t, J = 5.4 Hz, 2H), 3.54 (dd, J = 5.0, 1.8 Hz, 2H), 3.35 (s, 3H), 3.30-3.24 (m, 1H), 2.81-2.77 (m, 1H), 2.67 (s, 3H), 2.53 (td, J = 12.5, 4.3 Hz, 1H), 2.39-2.31 (m, 1H), 1.29 (s, 3H), 0.60 (dd, J = 5.8, 4.5 Hz, 2H), 0.40 (dd, J = 6.1, 4.4 Hz, 2H); ESI-MS [M+H]⁺: 686.

### Synthetic procedure W

### Example 246

### (E)-N-(2,6-difluoro-4-(7-methoxy-9-(2-methoxyethyl)-10-methyl-13,14-dihydro-9H-imidazo[4",5":5',6']benzo[1',2':2,3]oxocino[4,5,6-hi]indolizine-2-carbonyl)phenyl)-4-((1-methylcyclopentyl)amino)but-2-enamide

### Steps 1-2:

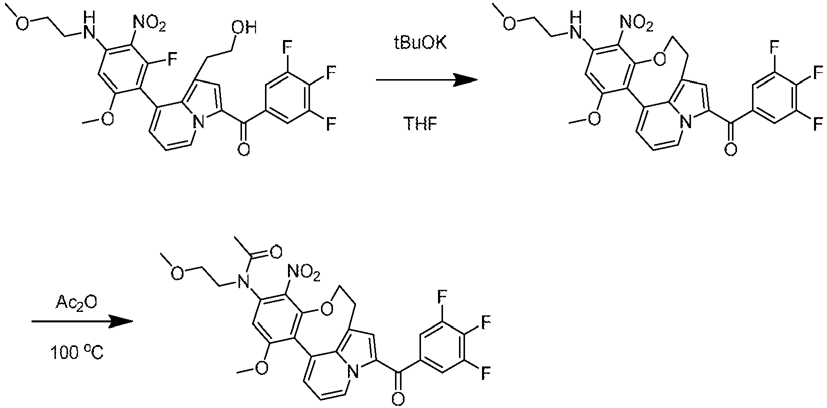

To a solution of (8-(2-fluoro-6-methoxy-4-((2-methoxyethyl)amino)-3-nitrophenyl)-1-(2-hydroxyethyl)indolizin-3-yl)(3,4,5-trifluorophenyl)methanone (112 mg, 0.200 mmol) obtained in step 5 (Example 240) in THF (3 mL) was added potassium tert-butoxide (1M in THF, 205 mmol). After stirred at room temperature for 15 minutes, water was added thereto. The resulting mixture was diluted with EtOAc. The organic layer was washed with sat. NaHCO₃ (aq.) and brine, dried over Na₂SO₄ and evaporated. To the residue was added acetic anhydride (2 mL), and the mixture was stirred at 100 °C for 2 hours. The resulting mixture was diluted with EtOAc. The organic layer was washed with sat. NaHCO₃ (aq.) and brine, dried over Na₂SO₄ and evaporated. The residue was purified by column chromatography on silica gel (hexane-EtOAc/EtOH(4/1)) to obtain N-(9-methoxy-6-nitro-1-(3,4,5-trifluorobenzoyl)-3,4-dihydrobenzo[2,3]oxocino[4,5,6-hi]indolizin-7-yl)-*N*-(2-methoxyethyl)acetamide.

### Steps 3-4:

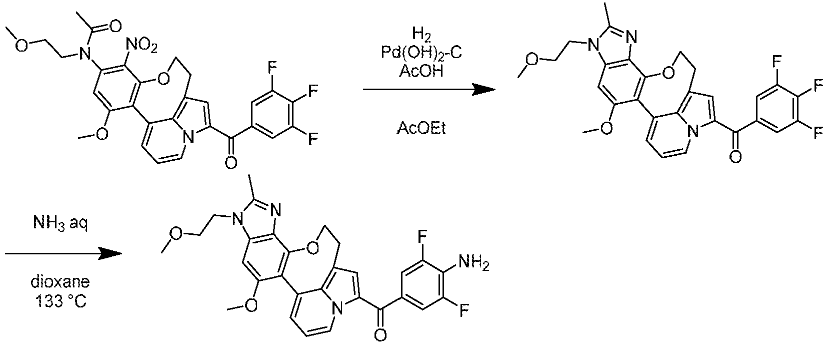

To a solution of*N*-(9-methoxy-6-nitro-1-(3,4,5-trifluorobenzoyl)-3,4-dihydrobenzo[2,3]oxocino[4,5,6-hi]indolizin-7-yl)-N-(2-methoxyethyl)acetamide (97.3 mg) in EtOAc (5 mL) was added acetic acid (0.05 mL) and palladium hydroxide on carbon (200 mg, 20 wt. % loading (dry basis)). The mixture was stirred at room temperature for 3 days under hydrogen atmosphere. After nitrogen purging, the mixture was filtered, rinsing with EtOAc and MeOH. The filtrate was evaporated, then to the residue was added 1,4-dioxane (1.5 mL) and 28-30% NH₃ (aq.) (3.0 mL). The reaction mixture was heated at 133 °C by microwave irradiation for 14 hours. After cooling, the mixture was diluted with EtOAc and THF, washed with brine then dried over Na₂SO₄ and evaporated to obtain (4-amino-3,5-difluorophenyl)(7-methoxy-9-(2-methoxyethyl)-10-methyl-13,14-dihydro-9*H*-imidazo[4",5":5',6']benzo[1',2':2,3]oxocino[4,5,6-hi]indolizin-2-yl)methanone.

### Step 5:

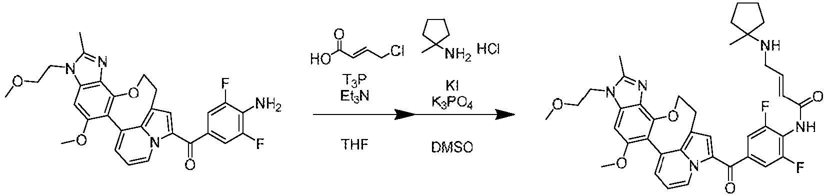

To the mixture of (4-amino-3,5-difluorophenyl)(7-methoxy-9-(2-methoxyethyl)-10-methyl-13,14-dihydro-9*H*-imidazo[4",5":5',6']benzo[1',2':2,3]oxocino[4,5,6-hi]indolizin-2-yl)methanone (86.2 mg, 0.161 mmol), (*E*)-4-chlorobut-2-enoic acid (25.2 mg, 0.209 mmol) and T3P (50 wt% in THF, 0.503 mL) in THF (5 mL) was added triethylamine (0.154 mL, 1.13 mmol). After stirred at room temperature for 1 hour, the resulting mixture was diluted with EtOAc, washed with NaHCO₃ (aq.), H₂O and brine then dried over Na₂SO₄ and evaporated. To the residue was added DMSO (5 mL), 1-methylcyclopentan-1-amine hydrochloride (0.186 mL, 0.805 mmol), KI (103 mg, 0.483 mmol) and K₃PO₄ (205 mg, 0.966 mmol) and the mixture was stirred at room temperature overnight. Water was added thereto, and the mixture was extracted with EtOAc. The extract was washed with brine, dried over sodium sulfate, and evaporated. The residue was purified by preparative HPLC (water:acetonitrile (0.1% formic acid)) to give (E)-N-(2,6-difluoro-4-(7-methoxy-9-(2-methoxyethyl)-10-methyl-13,14-dihydro-9*H-*imidazo[4",5":5',6']benzo[1',2':2,3]oxocino[4,5,6-hi]indolizine-2-carbonyl)phenyl)-4-((1-methylcyclopentyl)amino)but-2-enamide. ¹H-NMR: (400 MHz, CHLOROFORM-D) δ 9.98 (dd, J = 7.0, 1.0 Hz, 1H), 7.43-7.39 (m, 2H), 7.34 (dd, J = 7.3, 1.0 Hz, 1H), 7.19-7.12 (m, 2H), 7.02 (t, J = 7.1 Hz, 1H), 6.91 (s, 1H), 6.76 (s, 1H), 6.28 (d, J = 15.5 Hz, 1H), 4.80 (dd, J = 10.5, 4.8 Hz, 1H), 4.28 (t, J = 5.5 Hz, 2H), 4.22-4.16 (m, 1H), 3.83 (s, 3H), 3.75-3.73 (m, 2H), 3.45 (dd, J = 5.0, 1.5 Hz, 2H), 3.35 (s, 3H), 3.10-3.02 (m, 1H), 2.64 (s, 3H), 2.62-2.56 (m, 1H), 1.75-1.63 (m, 8H), 1.21 (s, 3H); ESI-MS [M+H]⁺: 698.

### Synthetic procedure X

### Synthesis of Int-252C:

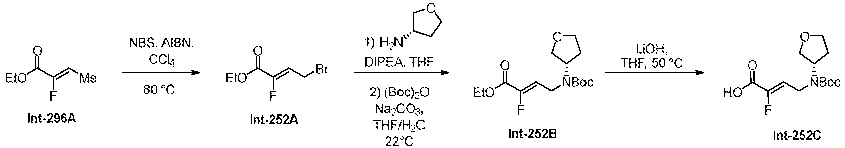

### Step A: ethyl (Z)-4-bromo-2-fluorobut-2-enoate (Int-252A)

A microwave vial was loaded with ethyl (Z)-2-fluorobut-2-enoate **(Int-296A)** (1.36 g, 10.29 mmol), N-bromosuccinimide (1.83 g, 10.29 mmol), Azobisisobutyronitrile (85 mg, 0.515 mmol) and 12 mL of CCl₄. The vial was sealed and heated to 80 °C for 16 hours. After this time, the mixture was filtered and the CELITE plug was washed with ether. The residue was purified through silica gel chromatography (40 g, Hex/10% EtOAc). Ethyl (Z)-4-bromo-2-fluorobut-2-enoate **(Int-252A)** was obtained. ¹H NMR (600 MHz, Chloroform-d) δ 6.39 (dt, *J=* 29.3, 8.6 Hz, 1H), 4.33 (q, *J =* 7.1 Hz, 2H), 4.08 (dd, *J* = 8.6*,* 1.9 Hz, 2H), 1.37 (t, *J=* 7.1 Hz, 3H).

### Step B: (S,Z)-4-((tert-butoxycarbonyl)(tetrahydrofuran-3-yl)amino)-2-fluorobut-2-enoate (Int-252B)

Ethyl (Z)-4-bromo-2-fluorobut-2-enoate **(Int-252A)** (1.55 g, 7.34 mmol) dissolved in 1 mL of THF was added to a solution of (S)-tetrahydrofuran-3-amine hydrochloride (1.815 g, 14.69 mmol) in THF (10 mL), followed by the addition of DIPEA (2.57 mL, 14.69 mmol). The reaction mixture was stirred at 22 °C for 12 h. The reaction mixture was concentrated under reduced pressure. The residue was diluted with DCM (25 mL). The organic layer was washed with brine (3 mL x 2), dried over Na₂SO₄ and filtered. The filtrate was concentrated under reduced pressure to give ethyl (*S*,*Z*)-2-fluoro-4-((tetrahydrofuran-3-yl)amino)but-2-enoate. The crude product was used in the next step without further purification, and was added to a vial containing sodium carbonate (1.142 mg, 10.77 mmol), THF (5 mL) and H₂O (5 mL). To this mixture, (Boc)₂O (2.475 mL, 10.77 mmol) was added in one portion. The reaction mixture was stirred at 22 °C for 20 hours. After this time, the reaction mixture was diluted with EtOAc (25 mL) and H₂O (5 mL). The aqueous layer was separated and extracted with EtOAc (25 mL x 3). The mixture was purified by silica gel chromatography, and the desired fraction was eluted with 35% EtOAc in hexanes to yield ethyl (*S*,*Z*)-4-((tert-butoxycarbonyl)(tetrahydrofuran-3-yl)amino)-2-fluorobut-2-enoate (Int-252B). ¹H NMR (499 MHz, Chloroform-d) δ 6.26 - 5.92 (m, 1H), 4.55 (s, 1H), 4.25 (q, *J =* 7.0 Hz, 2H), 4.14 - 3.88 (m, 3H), 3.78 - 3.70 (m, 1H), 3.69 - 3.59 (m, 2H), 2.26 - 2.09 (m, 1H), 1.89 - 1.76 (m, 1H), 1.42 (s, 9H), 1.29 (t, *J* = 7.1 Hz, 3H).

### Step C: (S,Z)-4-((tert-butoxycarbonyl)(tetrahydrofuran-3-yl)amino)-2-fluorobut-2-enoic acid (Int-252C)

Lithium hydroxide monohydrate was added (88 mg, 3.68mmol) to a solution of ethyl (*S*,*Z*)-4-((tert-butoxycarbonyl)(tetrahydrofuran-3-yl)amino)-2-fluorobut-2-enoate **(Int-252B)** (292 mg, 0.920 mmol) in THF (2 mL) and H₂O (2 mL), The reaction mixture was stirred at 50°C for 15 h. The reaction mixture was diluted with H₂O (1 mL) and EtOAc (1 mL). The mixture was acidified by citric acid until pH = 7. The reaction mixture was concentrated to yield (*S*,*Z*)-4-((tert-butoxycarbonyl)(tetrahydrofuran-3-yl)amino)-2-fluorobut-2-enoic acid **(Int-252C).** ¹H NMR (499 MHz, Chloroform-d) δ 10.08 (s, 1H), 6.19 (d, *J =* 32.2 Hz, 1H), 4.80 (s, 1H), 4.09 - 3.99 (m, 3H), 3.87 - 3.77 (m, 1H), 3.76 - 3.68 (m, 2H), 2.32 - 2.14 (m, 1H), 1.96 - 1.76 (m, 1H), 1.46 (s, 9H).

### SFC Separation to prepare (4-amino-3,5-difluorophenyl)(9,10-dimethyl-7-(trifluoromethyl)-13,14-dihydro-9H-imidazo[4",5":5',6']benzo[1',2':2,3]oxocino[4,5,6-hi]indolizin-2-yl)methanone (Int-AA)

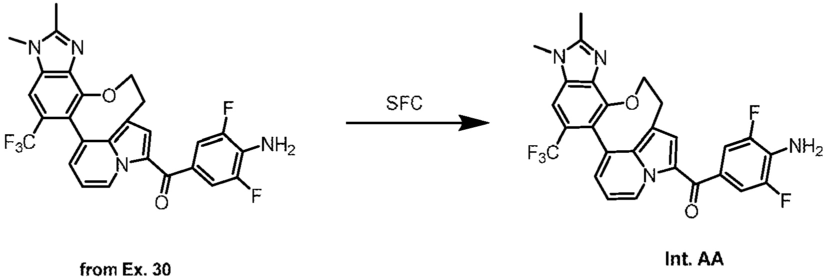

Atropmixture of (4-amino-3,5-difluorophenyl)(9,10-dimethyl-7-(trifluoromethyl)-13,14-dihydro-9*H*-imidazo[4",5":5',6']benzo[1',2':2,3]oxocino[4,5,6-hi]indolizin-2-yl)methanone (20.0 g, 38.0 mmol) was separated by SFC (Column: DAICEL CHIRALPAK AD(250mm*50mm,10um), Condition: 0.1% IPA in EtOH) to give (4-amino-3,5-difluorophenyl)(9,10-dimethyl-7-(trifluoromethyl)-13,14-dihydro-9H-imidazo[4",5":5',6']benzo[1',2':2,3]oxocino[4,5,6-hi]indolizin-2-yl)methanone **(Int. AA,** the first eluting isomer from SFC). MS (ESI): m/z (M+H) + 527.

### Example 252: (R,Z)-N-(4-(9,10-dimethyl-7-(trifluoromethyl)-13,14-dihydro-9H-imidazo[4",5":5',6']benzo[1',2':2,3]oxocino[4,5,6-hi]indolizine-2-carbonyl)-2,6-difluorophenyl)-2-fluoro-4-((tetrahydrofuran-3-yl)amino)but-2-enamide (Ex. 252)

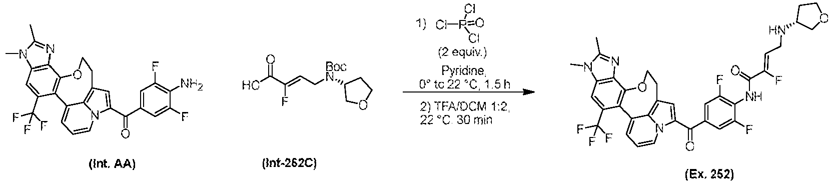

A vial was charged with (4-amino-3,5-difluorophenyl)(9,10-dimethyl-7-(trifluoromethyl)-13,14-dihydro-9H-imidazo[4",5":5',6']benzo[1',2':2,3]oxocino[4,5,6-hi]indolizin-2-yl)methanone **(Int. AA)** (81 mg, 0.154 mmol), *(R,Z)*-4-((*tert-*butoxycarbonyl)(tetrahydrofuran-3-yl)amino)-2-fluorobut-2-enoic acid (Int-252C) (26.1 mg, 0.096 mmol) and a stir bar. The vial was fitted with a septum and placed under nitrogen. Pyridine (0.5 mL) was added via syringe and the reaction was cooled to 0 °C. Phosphorus oxychloride (0.308 mL, 0.308 mmol) was added via syringe as a 1 M solution in pyridine, and the reaction was warmed to 22 °C for 1.5 hours and the solvent was evaporated under reduced pressure. The remaining residue was dissolved in 2 mL of DCM and 1 mL of TFA. The reaction was allowed to stir for 30 min at 22 °C. The volatiles were evaporated under reduced pressure and the crude was redissolved in DMSO, filtered and subjected to HPLC purification with a TFA modifier. (R,Z)-N-(4-(9,10-dimethyl-7-(trifluoromethyl)-13,14-dihydro-9H-imidazo[4",5":5',6']benzo[1',2':2,3]oxocino[4,5,6-hi]indolizine-2-carbonyl)-2,6-difluorophenyl)-2-fluoro-4-((tetrahydrofuran-3-yl)amino)but-2-enamide, 2TFA **(Ex. 252)** was obtained. ¹H NMR (499 MHz, DMSO-*d*₆) δ 10.70 (s, 1H), 9.92 (d, *J =* 6.7 Hz, 1H), 9.07 (s, 2H), 8.09 (s, 1H), 7.65 (s, 1H), 7.63 (s, 1H), 7.37 (s, 1H), 7.29 - 7.15 (m, 2H), 6.27 (dt, *J=* 33.7, 7.3 Hz, 1H), 4.63 - 4.51 (m, 2H), 4.13 - 4.01 (m, 1H), 4.00 - 3.95 (m, 4H), 3.90 (s, 3H) 3.82 - 3.73 (m, 2H), 3.72 - 3.62 (m, 1H), 2.80 - 2.72 (m, 1H), 2.70 - 2.65 (m, 1H), 2.60 (s, 3H), 2.27 (d, *J =* 13.8, 7.8 Hz, 1H), 2.02 (d, *J =* 3.9 Hz, 1H). MS (ESI): [M+H]⁺ *m*/*z:* 698.

### Synthetic procedure Y

### Synthesis of (Z)-4-bromo-2-fluorobut-2-enoic acid (Int-296C)

### Step A: ethyl (Z)-2-fluorobut-2-enoate (Int-296A)

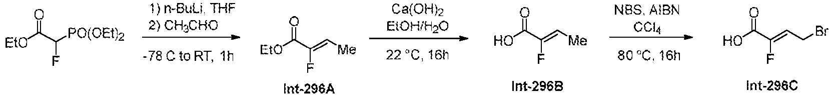

A dry 250 ml Schlenk-tube with a stir bar and a rubber septum was loaded with THF (60 ml) and ethyl 2-(diethoxyphosphoryl)-2-fluoroacetate (13.32 g, 55 mmol) under argon. The solution was cooled to -78 °C and n-BuLi (2.5 M in hexane, 22 ml, 55 mmol) was added dropwise over ca. 10 min. The yellow solution was stirred for 10 min at -78 °C and then acetaldehyde (3.1 ml, 2.4 g, 50 mmol) was added in one portion. The solution was stirred for 1 h at -78 °C, after which the cooling bath was removed, and the solution was stirred until it reached room temperature (ca. 45 min).

Next, a saturated aqueous solution of NH₄Cl (60 ml) was added and the heterogeneous solution was transferred to a separatory funnel along with 60 ml ether. The phases were separated and the organic phase was collected. The aqueous phase was extracted with ether (4 x 60 ml). The organic phases were combined and dried over anhydrous MgSO₄. The Et₂O was evaporated in the rotavapor (750 mbar, 40 °C) and the rest of the solvents were removed by distillation (oil bath 85 °C). After the solvents had been distilled, the temperature of the oil bath was increased to 130 °C to remove 2-hexanol (by-product from reaction of BuLi and acetaldehyde). After distilling a few drops, the oil bath was removed and the distillation apparatus was left to cool to room temperature. Next, the liquid residue was distilled under vacuum to give crude ethyl (Z)-2-fluorobut-2-enoate **(Int-296A)** (38 °C, 18 torr, 95 torr). The distillate was used in the next step without further purification. The product was obtained as 90/10 E/Z mixture. ¹H NMR (600 MHz, Chloroform-d) δ 6.00 (dq, *J=* 21.3, 7.8 Hz, 1H), 4.32 (q, *J* = 7.1 Hz, 2H), 2.03 (dd, *J* = 7.8, 2.9 Hz, 3H), 1.36 (t, *J* = 7.1 Hz, 3H).

### Step B: (Z)-2-fluorobut-2-enoic acid (Int-296B)

Ethyl (Z)-2-fluorobut-2-enoate **(Int-296A)** (1.3 g, 9.84 mmol) was dissolved in ethanol (5 mL) in a 30 mL vial. Then H₂O (10 mL) and calcium hydroxide (2.4 g, 32.5 mmol) were added. The mixture was stirred at room temperature for 16 hours. The reaction was neutralized by 1 N HCl. The ethanol was evaporated under vacuum and the aqueous solution was extracted by ethyl acetate (3 × 25 mL). The combined organic layers were dried over Na₂SO₄ and concentrated under vacuum to give (Z)-2-fluorobut-2-enoic acid. The crude compound was crystallized by dissolving the crude mixture in hexanes and allowing it to cool down to -20 °C for 1 hour, followed by filtration of the solid to obtain (Z)-2-fluorobut-2-enoic acid **(Int-296B).** ¹H NMR (600 MHz, Chloroform-d) δ 10.23 (bs, 1H), 6.19 (dq, *J=* 20.9, 7.8 Hz, 1H), 2.08 (dd, *J =* 7.8, 2.8 Hz, 3H).

### Step C: (Z)-4-bromo-2-fluorobut-2-enoic acid (Int-296C)

A microwave vial was loaded with ethyl (Z)-2-fluorobut-2-enoic acid **(Int-296B)** (530 mg, 5.09 mmol), N-bromosuccinimide (1.36 g, 7.64 mmol), azobisisobutyronitrile (84 mg, 0.509 mmol) and 10 mL of CCl₄. The vial was sealed and heated to 90 °C for 16 h. After this time, the solvent was evaporated, and the crude mixture was extracted with Et₂O and filtered and the CELITE plug was washed with Et₂O. The solvent was removed under reduced pressure. The residue was purified by silica gel chromatography. The compound is eluted with hexanes/30% EtOAc. (Z)-4-bromo-2-fluorobut-2-enoic acid **(Int-296C)** was obtained. ¹H NMR (600 MHz, Chloroform-d) δ 9.2 (bs, 1H), 6.54 (dt, *J* = 28.8, 8.6 Hz, 1H), 4.10 (dd, *J =* 8.6, 1.4 Hz, 2H).

### Example 296: (Z)-N-(4-(9,10-dimethyl-7-(trifluoromethyl)-13,14-dihydro-9H-imidazo[4",5":5',6']benzo[1',2':2,3]oxocino[4,5,6-hi]indolizine-2-carbonyl)-2,6-difluorophenyl)-2-fluoro-4-((4-methyltetrahydro-2H-pyran-4-yl)amino)but-2-enamide (Ex. 296)

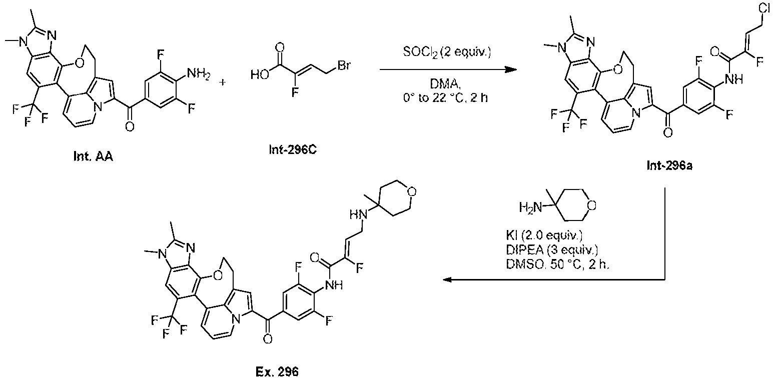

### Step A: (Z)-4-chloro-N-(4-(9,10-dimethyl-7-(trifluoromethyl)-13,14-dihydro-9H-imidazo[4",5":5',6']benzo[1',2':2,3]oxocino[4,5,6-hi]indolizine-2-carbonyl)-2,6-difluorophenyl)-2-fluorobut-2-enamide (Int. 296a)

A solution of anhydrous dimethylacetamide (0.6 mL) and (Z)-4-bromo-2-fluorobut-2-enoic acid **(Int-296C)** (61 mg, 0.167 mmol, 50% purity) under nitrogen was cooled to 0 °C. Thionyl chloride (22 µL, 0.304 mmol) was added dropwise (via syringe). After stirring for 10 min, (4-amino-3,5-difluorophenyl)(9,10-dimethyl-7-(trifluoromethyl)-13,14-dihydro-9H-imidazo[4",5":5',6']benzo[1',2':2,3]oxocino[4,5,6-hi]indolizin-2-yl)methanone **(Int. AA)** (80 mg, 0.152 mol) was added dropwise as a solution in DMA (1.0 mL). Upon complete addition, the mixture was warmed to 22 °C and stirred for to 2 h. The reaction was quenched with NaHCO₃ (aqueous), and extracted with EtOAc. The organic phase was combined, dried over Na₂SO₄ and evaporated under reduced pressure. The crude mixture was purified by silica gel chromatography, (ISCO, 12g, eluting with DCM/MeOH. The desired compound elutes with 20% MeOH in DCM). (Z)-4-chloro-N-(4-(9,10-dimethyl-7-(trifluoromethyl)-13,14-dihydro-9H-imidazo[4",5":5',6']benzo[1',2':2,3]oxocino[4,5,6-hi]indolizine-2-carbonyl)-2,6-difluorophenyl)-2-fluorobut-2-enamide **(Int. 296a)** was obtained. MS (ESI): m/z (M+H)⁺ 647.

### Step B: (Z)-N-(4-(9,10-dimethyl-7-(trifluoromethyl)-13,14-dihydro-9H-imidazo[4",5":5',6']benzo[1',2':2,3]oxocino[4,5,6-hi]indolizine-2-carbonyl)-2,6-difluorophenyl)-2-fluoro-4-((4-methyltetrahydro-2H-pyran-4-yl)amino)but-2-enamide

A vial was loaded with 4-methyltetrahydro-2H-pyran-4-amine (10.3 mg, 0.089 mmol) and DIPEA (16.1 µL, 0.178 mmol) and dissolved in 0.5 mL of DMSO. A solution of (Z)-4-chloro-N-(4-(9,10-dimethyl-7-(trifluoromethyl)-13,14-dihydro-9H-imidazo[4",5":5',6']benzo[1',2':2,3]oxocino[4,5,6-hi]indolizine-2-carbonyl)-2,6-difluorophenyl)-2-fluorobut-2-enamide **(Int. 296a)** (24 mg, 0.030 mmol) and KI (1.9 mg, 0.059 mmol) in 0.5 mL of DMSO was added via syringe. The mixture was heated to 50 °C for 2 hours. The mixture was filtered, washed with 0.3 mL of DMSO and submitted to HPLC purification using TFA modifier. (Z)-N-(4-(9,10-dimethyl-7-(trifluoromethyl)-13,14-dihydro-9H-imidazo[4",5":5',6']benzo[1',2':2,3]oxocino[4,5,6-hi]indolizine-2-carbonyl)-2,6-difluorophenyl)-2-fluoro-4-((4-methyltetrahydro-2H-pyran-4-yl)amino)but-2-enamide, 2TFA **(Ex. 296)** was obtained. ¹H NMR (600 MHz, DMSO) δ 10.73 (s, 1H), 9.92 (d, *J=* 7.0 Hz, 1H), 8.99 (s, 2H), 8.07 (s, 1H), 7.64 (d, *J=* 7.9 Hz, 2H), 7.37 (s, 1H), 7.26 - 7.17 (m, 3H), 7.15 (s, 1H), 7.07 (s, 1H), 6.29 (dt, *J =* 33.5, 7.4 Hz, 1H), 4.58 (dd, *J* = 10.2, 5.0 Hz, 1H), 4.14 - 3.98 (m, 1H), 3.89 (s, 3H), 3.87 - 3.66 (m, 3H), 3.49 (t, *J* = 10.9 Hz, 1H), 2.76 (d, *J=* 12.8 Hz, 1H), 2.65 (s, 3H), 2.63 - 2.54 (m, 1H), 1.86 - 1.77 (m, 2H), 1.77 - 1.71 (m, 2H), 1.46 (s, 3H). MS (ESI): *m*/*z* (M+H)⁺ 726.

### Synthetic procedure Z

### SFC Separation to prepare (4-amino-3,5-difluorophenyl)(9,10,12-trimethyl-7-(trifluoromethyl)-9,12,13,14-tetrahydroimidazo[4",5":5',6']benzo[1',2':2,3]azocino[4,5,6-hi]indolizin-2-yl)methanone (Int. BB)

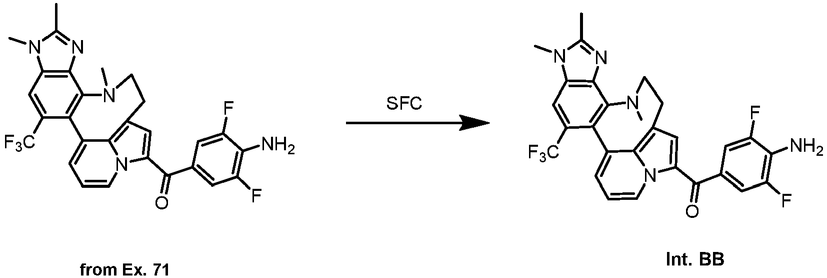

Atropmixture of (4-amino-3,5-difluorophenyl)(9,10,12-trimethyl-7-(trifluoromethyl)-9,12,13,14-tetrahydroimidazo[4",5":5',6']benzo[1',2':2,3]azocino[4,5,6-hi]indolizin-2-yl)methanone (1.95 g, 3.61 mmol) was separated by SFC (Column: DAICEL CHIRALPAK AD(250mm*50mm,10um), Condition: 0.1% NH₃H₂O/IPA, Mobile phase: A: CO2 B: IPA (0.1% NH₃H₂O)) to give (4-amino-3,5-difluorophenyl)(9,10,12-trimethyl-7-(trifluoromethyl)-9,12,13,14-tetrahydroimidazo[4",5":5"6']benzo[1',2':2,3]azocino[4,5,6-hi]indolizin-2-yl)methanone **(Int. BB,** the first eluting isomer from SFC). MS (ESI): m/z (M+H)⁺ 540.

### Example 285: (E)-N-(2,6-difluoro-4-(9,10,12-trimethyl-7-(trifluoromethyl)-9,12,13,14-tetrahydroimidazo[4",5":5',6']benzo[1',2':2,3]azocino[4,5,6-hi]indolizine-2-carbonyl)phenyl)-4-(3-fluoroazetidin-1-yl)but-2-enamide (Ex. 285)

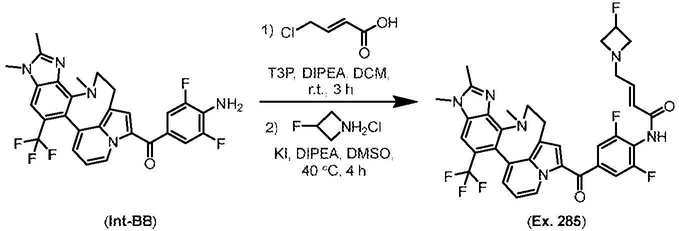

A vial with stir bar was charged with (4-amino-3,5-difluorophenyl)(9,10,12-trimethyl-7-(trifluoromethyl)-9,12,13,14-tetrahydroimidazo[4",5":5',6']benzo[1',2':2,3]azocino[4,5,6-hi]indolizin-2-yl)methanone **(Int. BB)** (54 mg, 0.100 mmol) and (*E*)-4-chlorobut-2-enoic acid (36.2 mg, 0.300 mmol) and placed under nitrogen. Dry DCM (2 mL) and 1-propanephosphonic anhydride solution (50 wt% in EtOAc, 298 µl, 0.500 mmol) were added via syringe. *N,N-*diisopropylethylamine (87 µl, 0.500 mmol) was added dropwise via syringe. After 3 hours, sat. aq. NaHCO₃ (3 mL) and DCM (3 mL) were added, and the contents were transferred to a separatory funnel. The funnel was shaken, and the layers were separated. The aqueous phase was washed with DCM (2 x 3 mL). The combined organics were washed with brine (3 mL), dried over MgSO4, filtered, and concentrated. The crude material including (*E*)-4-chloro-*N*-(2,6-difluoro-4-(9,10,12-trimethyl-7-(trifluoromethyl)-9,12,13,14-tetrahydroimidazo[4",5":5',6']benzo[1',2':2,3]azocino[4,5,6-hi]indolizine-2-carbonyl)phenyl)but-2-enamide was used in the next step without further purification.

A vial with stir bar was charged with (E)-4-chloro-N-(2,6-difluoro-4-(9,10,12-trimethyl-7-(trifluoromethyl)-9,12,13,14-tetrahydroimidazo[4",5":5',6']benzo[1',2':2,3]azocino[4,5,6-*hi*]indolizine-2-carbonyl)phenyl)but-2-enamide (24 mg, 0.037 mmol), 3-fluoroazetidine hydrochloride (20.85 mg, 0.187 mmol), and potassium iodide (31.0 mg, 0.187 mmol). DMSO (467 µl) and *N*,*N*-diisopropylethylamine (39.2 µl, 0.224 mmol) were added via syringe and the reaction was heated to 40 °C for 4 hours. After 4 hours, the reaction was diluted with water (4 mL) and EtOAc (4 mL) added, and the contents were transferred to a separatory funnel. The funnel was shaken, and the layers were separated. The aqueous phase was washed with EtOAc (2 x 4 mL). The combined organics were washed with brine (10 mL), dried over sodium sulfate, filtered, and concentrated. The crude material was subjected to HPLC purification with a TFA modifier. (E)-N-(2,6-difluoro-4-(9,10,12-trimethyl-7-(trifluoromethyl)-9,12,13,14-tetrahydroimidazo[4",5":5',6']benzo[1',2':2,3]azocino[4,5,6-hi]indolizine-2-carbonyl)phenyl)-4-(3-fluoroazetidin-1-yl)but-2-enamide, 2TFA **(Ex. 285)** was obtained. ¹H NMR (499 MHz, CD₃OD) δ 9.98 (d, *J =* 7.0 Hz, 1H), 8.20 (s, 1H), 7.52 (d, *J=* 8.0 Hz, 2H), 7.29 (s, 1H), 7.27 - 7.08 (m, 2H), 6.86 (dt, *J =* 13.8, 6.7 Hz, 1H), 6.62 (d, *J =* 15.3 Hz, 1H), 5.47 (d, *J=* 56.4 Hz, 1H), 4.69-4.56 (m, 2H), 4.54 - 4.33 (m, 2H), 4.20 (d, *J =* 6.6 Hz, 2H), 4.01 (s, 3H), 3.65 - 3.46 (m, 1H), 3.08 (d, *J =* 34.7 Hz, 4H), 2.83 (s, 3H), 2.73 (d, *J =* 14.4 Hz, 1H), 2.37 - 2.10 (m, 1H). [M+H]⁺ *m*/*z:* 681.

### Synthetic procedure AA

### SFC Separation to prepare (4-amino-3,5-difluorophenyl)(7-methoxy-10-(methoxymethyl)-9,12-dimethyl-9,12,13,14-tetrahydroimidazo[4",5":5',6']benzo[1',2':2,3]azocino[4,5,6-hi]indolizin-2-yl)methanone (Int. CC)

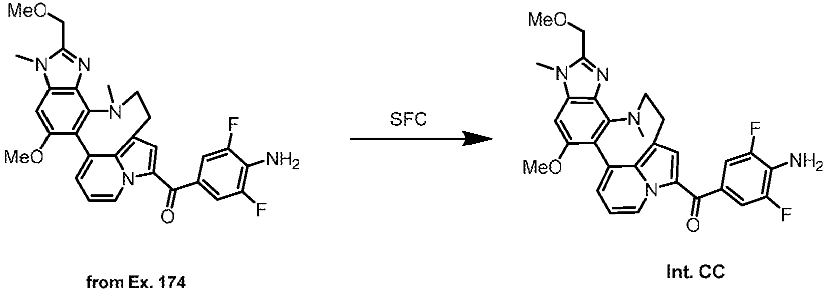

Atropmixture of (4-amino-3,5-difluorophenyl)(7-methoxy-10-(methoxymethyl)-9,12-dimethyl-9,12,13,14-tetrahydroimidazo[4",5":5',6']benzo[1',2':2,3]azocino[4,5,6-hi]indolizin-2-yl)methanone (788 mg, 1.48 mmol) was separated by SFC (Column: IZ (250mm*21mm, 5um), Condition: 40% MeOH w/ 0.1% NH₄OH) to give (4-amino-3,5-difluorophenyl)(7-methoxy-10-(methoxymethyl)-9,12-dimethyl-9,12,13,14-tetrahydroimidazo[4",5":5"6']benzo[1',2':2,3]azocino[4,5,6-hi]indolizin-2-yl)methanone (Int. CC, the second eluting isomer from SFC). MS (ESI): m/z (M+H)⁺ 532.

### Example 286: (Z)-N-(2,6-difluoro-4-(7-methoxy-10-(methoxymethyl)-9,12-dimethyl-9,12,13,14-tetrahydroimidazo[4",5":5',6']benzo[1',2':2,3]azocino[4,5,6-hi]indolizine-2-carbonyl)phenyl)-2-fluoro-4-((1-methylcyclopropyl)amino)but-2-enamide (Ex. 286)

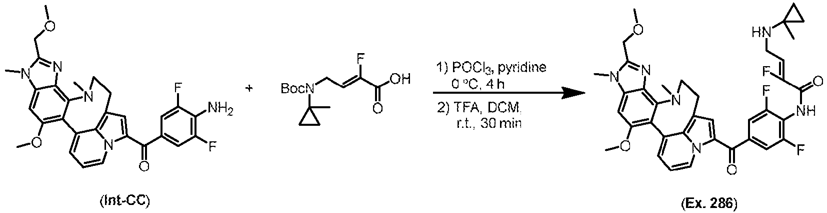

A vial with stir bar was charged with (4-amino-3,5-difluorophenyl)(7-methoxy-10-(methoxymethyl)-9,12-dimethyl-9,12,13,14-tetrahydroimidazo[4",5":5',6']benzo[1',2':2,3]azocino[4,5,6-hi]indolizin-2-yl)methanone **(Int. CC)** (25 mg, 0.047 mmol) and (Z)-4-((tert-butoxycarbonyl)(1-methylcyclopropyl)amino)-2-fluorobut-2-enoic acid (23.14 mg, 0.085 mmol) and placed under nitrogen. Dry pyridine (470 µl) and phosphorus oxychloride (8.77 µl, 0.094 mmol) were added via syringe. After 3 hours, sat. aq. NaHCO₃ (3 mL) and DCM (3 mL) were added, and the contents were transferred to a separatory funnel. The funnel was shaken, and the layers were separated. The aqueous phase was washed with DCM (2 x 3 mL). The combined organics were washed with brine (3 mL), dried over MgSO₄, filtered, and concentrated. The crude material including *Tert-*butyl (Z)-(4-((2,6-difluoro-4-(7-methoxy-10-(methoxymethyl)-9,12-dimethyl-9,12,13,14-tetrahydroimidazo[4",5":5',6']benzo[1',2':2,3]azocino[4,5,6-hi]indolizine-2-carbonyl)phenyl)amino)-3-fluoro-4-oxobut-2-en-1-yl)(1-methylcyclopropyl)carbamate was taken on in the next step without further purification.

*Tert-butyl* (Z)-(4-((2,6-difluoro-4-(7-methoxy-10-(methoxymethyl)-9,12-dimethyl-9,12,13,14-tetrahydroimidazo[4",5":5',6']benzo[1',2':2,3]azocino[4,5,6-hi]indolizine-2-carbonyl)phenyl)amino)-3-fluoro-4-oxobut-2-en-1-yl)(1-methylcyclopropyl)carbamate (37 mg, 0.047 mmol) was placed in a vial with stir bar and dissolved in DCM (313 µl). TFA (157 µl) was added dropwise and the reaction was allowed to stir for 30 min. After 30 min, the reaction was concentrated in vacuo. The crude material was subjected to HPLC purification with a TFA modifer. (Z)-N-(2,6-difluoro-4-(7-methoxy-10-(methoxymethyl)-9,12-dimethyl-9,12,13,14-tetrahydroimidazo[4",5":5',6']benzo[1',2':2,3]azocino[4,5,6-*hi*]indolizine-2-carbonyl)phenyl)-2-fluoro-4-((1-methylcyclopropyl)amino)but-2-enamide, 2TFA was obtained **(Ex. 286).** ¹H NMR (499 MHz, CD₃OD) δ 10.01 (d, J = 7.0 Hz, 1H), 7.58 - 7.46 (m, 4H), 7.33 (s, 1H), 7.22 (t, *J=* 7.1 Hz, 1H), 6.31 (dt, *J =* 32.0, 7.6 Hz, 1H), 4.86 (s, 2H), 4.07 (d, *J =* 7.5 Hz, 2H), 4.01 (s, 3H), 3.93 (s, 4H), 3.50 (d, *J* = 9.0 Hz, 7H), 2.88 (d, *J= 14.5* Hz, 1H), 2.54 - 2.35 (m, 1H), 1.55 (s, 3H), 1.11 (m 2H), 0.92 - 0.84 (m, 2H). [M+H]⁺ *m*/*z:* 687.

### Synthetic procedure BB

### Example 287: (E)-N-(2,6-difluoro-4-(7-methoxy-10-(methoxymethyl)-9,12-dimethyl-9,12,13,14-tetrahydroimidazo[4",5":5',6']benzo[1',2':2,3]azocino[4,5,6-hi]indolizine-2-carbonyl)phenyl)-4-(((1-fluorocyclopropyl)methyl)amino)but-2-enamide (Ex. 287)

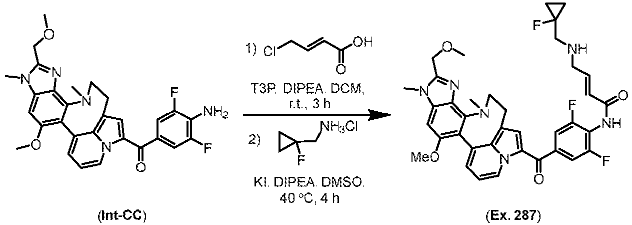

A vial with stir bar was charged with (4-amino-3,5-difluorophenyl)(7-methoxy-10-(methoxymethyl)-9,12-dimethyl-9,12,13,14-tetrahydroimidazo[4",5":5',6']benzo[1',2':2,3]azocino[4,5,6-*hi*]indolizin-2-yl)methanone (Int. CC) (70 mg, 0.132 mmol) and (*E*)-4-chlorobut-2-enoic acid (47.6 mg, 0.395 mmol) and placed under nitrogen. Dry DCM (2.6 mL) and 1-propanephosphonic anhydride solution (50 wt% in EtOAc, 392 µl, 0.658 mmol) were added via syringe. *N,N-*diisopropylethylamine (115 µl, 0.658 mmol) was added dropwise via syringe (turned an orange color). After 3 hours, sat. aq. sodium bicarbonate (3 mL) and DCM (3 mL) were added, the layers were shaken, and separated. The aqueous phase was washed with DCM (2 x 3 mL). The combined organics were washed with brine (3 mL), dried over MgSO4, filtered, and concentrated. The crude material including (E)-4-chloro-N-(2,6-difluoro-4-(7-methoxy-10-(methoxymethyl)-9,12-dimethyl-9,12,13,14-tetrahydroimidazo[4",5":5',6']benzo[1',2':2,3]azocino[4,5,6-*hi*]indolizine-2-carbonyl)phenyl)but-2-enamide was taken on in the next step without further purification.

A vial with stir bar was charged with (E)-4-chloro-N-(2,6-difluoro-4-(7-methoxy-10-(methoxymethyl)-9,12-dimethyl-9,12,13,14-tetrahydroimidazo[4",5":5',6']benzo[1',2':2,3]azocino[4,5,6-*hi*]indolizine-2-carbonyl)phenyl)but-2-enamide (28 mg, 0.044 mmol), (1-fluorocyclopropyl)methanamine, HCl (27.7 mg, 0.221 mmol) and potassium iodide (36.7 mg, 0.221 mmol). DMSO (552 µl) and *N*,*N*-diisopropylethylamine (38.6 µl, 0.221 mmol) were added via syringe and the reaction was heated to 40 °C for 4 hours. After 4 hours, the reaction was diluted with water (4 mL) and EtOAc (4 mL) added, and the contents were transferred to a separatory funnel. The funnel was shaken, and the layers were separated. The aqueous phase was washed with EtOAc (2 x 4 mL). The combined organics were washed with brine (10 mL), dried over sodium sulfate, filtered, and concentrated. The crude material was subjected to HPLC purification with a TFA modifier. *(E)-N-(2,6-difluoro-4-(7-methoxy-10-(methoxymethyl)-9,12-dimethyl-*9,12,13,14-tetrahydroimidazo[4",5":5',6']benzo[1',2':2,3]azocino[4,5,6-hi]indolizine-2-carbonyl)phenyl)-4-(((1-fluorocyclopropyl)methyl)amino)but-2-enamide, 2TFA **(Ex. 287)** was obtained. ¹H NMR (499 MHz, CD₃OD) δ 10.03 (d, *J* = 7.0 Hz, 1H), 7.59 - 7.45 (m, 4H), 7.36 (s, 1H), 7.24 (t, *J* = 7.1 Hz, 1H), 6.99 (dt, *J* = 14.2, 6.8 Hz, 1H), 6.64 (d, *J =* 15.4 Hz, 1H), 4.87 (s, 3H), 4.06-4.00 (m, 5H), 3.95 (s, 4H), 3.57 (d, *J=* 21.2 Hz, 2H), 3.52 (d, *J=* 8.8 Hz, 6H), 2.90 (d, *J =* 15.0 Hz, 1H), 2.55 - 2.42 (m, 1H), 1.28 (dd, *J =* 18.7, 7.1 Hz, 2H), 0.97 (q, *J =* 7.6 Hz, 2H). [M+H]⁺ *m*/*z:* 687.

### Synthetic procedure CC

### Synthesis of (E)-3-((2S,4R)-1-(tert-butoxycarbonyl)-4-fluoropyrrolidin-2-yl)acrylic acid (Int-293B)

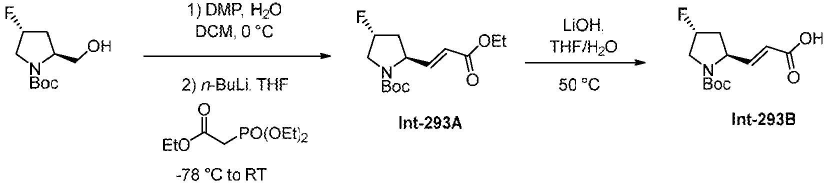

### Step A: tert-butyl (2S,4R)-2-((E)-3-ethoxy-3-oxoprop-1-en-1-yl)-4-fluoropyrrolidine-1-carboxylate (Int-293A)

A vial was loaded with (2S,4R)-tert-butyl 4-fluoro-2-(hydroxymethyl)pyrrolidine-1-carboxylate (320 mg, 1.459 mmol) and dissolved in 15 mL of DCM. The solution was cooled to 0 °C and Dess-Martin periodinane was added (929 mg, 2.19 mmol), followed by water (32 µL, 1.751 mmol). The reaction was stirred at 0 °C for three hours. After 3 hours at 0 °C, the reaction was allowed to stir for 1.5 hours at 22 °C. The mixture was diluted with ether, concentrated into a 2 mL of solvent by distillation under reduced pressure. The residue was redissolved in 30 mL of ether and then washed with 15 mL of a 1:1 mixture of a 10% solution of Na₂S₂O₃ and saturated aqueous NaHCO₃, followed by 10 mL of H₂O and 10 mL of brine. The aqueous layers were extracted with Et₂O (2 x20 mL), and the combined organic layers were washed with H₂O (10 mL) and brine (10 mL). The combined organic layers were dried with Na₂SO₄ and concentrated. The crude mixture including *tert-*butyl (2*S*,4*R*)-4-fluoro-2-formylpyrrolidine-1-carboxylate was used in the next step without further purification.

A dry 100 mL flask with a stir bar and a rubber septum was charged under nitrogen atmosphere with dry THF (5 ml) and ethyl 2-(diethoxyphosphoryl)acetate (327 mg, 1.457 mmol). The solution was cooled to -78 °C and n-BuLi (2.5 M in hexane, 583 µl, 1.457 mmol) was added dropwise over ca. 10 min. The solution was stirred for 10 min at -78 °C and then the crude *tert-*butyl (2*S*,4*R*)-4-fluoro-2-formylpyrrolidine-1-carboxylate (1.46 mmol) was added in one portion dissolved in 2 mL of THF. The solution was stirred for 1 h at -78 °C, after which the cooling bath was removed and the solution was stirred until it reached room temperature (ca. 45 min). Next, a saturated aqueous solution of NH₄Cl (5 ml) was added and the heterogeneous solution was transferred to a separatory funnel and diluted with 20 ml ether. The phases were separated and the organic phase collected. The aqueous phase was extracted with ether (3 x 20 ml). The organic phases were combined and dried over anhydrous MgSO₄. The crude mixture was purified by silica gel chromatography (the desired compound was eluted with 35% EtOAc) to yield *tert-*butyl (2S,4R)-2-((E)-3-ethoxy-3-oxoprop-1-en-1-yl)-4-fluoropyrrolidine-1-carboxylate **(Int-293A).** MS (ESI): m/z [M+H-Boc]⁺ = 188.

### Step B: (E)-3-((2S,4R)-1-(tert-butoxycarbonyl)-4-fluoropyrrolidin-2-yl)acrylic acid (Int-293B)

A vial was loaded with *tert-*butyl (2*S*,4*R*)-2-((*E*)-3-ethoxy-3-oxoprop-1-en-1-yl)-4-fluoropyrrolidine-1-carboxylate **(Int-293A)** (301.4 mg, 0.839 mmol) and LiOH (81 mg, 3.38 mmol). The mixture was dissolved in 2 mL of water and 2 mL of THF. The mixture was allowed to stir at room temperature for 20 minutes. After this time, the mixture was heated to 50 °C for 20 hours. Then, the reaction mixture was diluted with 15 mL of EtOAc, and the reaction was acidified to pH = 5-6 with an aquous HCl 1 M solution. The aqueous layer was extracted EtOAc (3x15 mL). The combined organic layers were dried over Na₂SO₄ and evaporated under reduced pressure. (*E*)-3-((2*S*,4*R*)-1-(*tert-*butoxycarbonyl)-4-fluoropyrrolidin-2-yl)acrylic acid **(Int-293B)** was obtained. MS (ESI): m/z [M+H]⁺ = 260.

### Example 293: (E)-N-(2,6-difluoro-4-(7-methoxy-10-(methoxymethyl)-9,12-dimethyl-9,12,13,14-tetrahydroimidazo[4",5":5',6']benzo[1',2':2,3]azocino[4,5,6-hi]indolizine-2-carbonyl)phenyl)-3-((2S,4R)-4-fluoropyrrolidin-2-yl)acrylamide (Ex. 293)

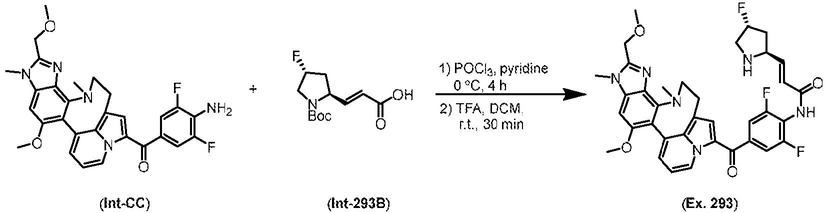

A vial with stir bar was charged with (4-amino-3,5-difluorophenyl)(7-methoxy-10-(methoxymethyl)-9,12-dimethyl-9,12,13,14-tetrahydroimidazo[4",5":5',6']benzo[1',2':2,3]azocino[4,5,6-hi]indolizin-2-yl)methanone **(Int. CC)** (30 mg, 0.056 mmol) and (E)-3-((2S,4R)-1-(tert-butoxycarbonyl)-4-fluoropyrrolidin-2-yl)acrylic acid **(Int-293B)** (26.3 mg, 0.102 mmol) and placed under nitrogen. Dry pyridine (564 µl) was added via syringe. Phosphorus oxychloride (10.52 µl, 0.113 mmol) was added dropwise via syringe. After 3 hours at 0 °C, sat. aq. NaHCO₃ (3 mL) and DCM (3 mL) were added, and the contents were transferred to a separatory funnel. The funnel was shaken, and the layers were separated. The aqueous phase was washed with DCM (2 x 3 mL). The combined organics were washed with brine (3 mL), dried over MgSO4, filtered, and concentrated. The crude material including *tert-*butyl (2S,4R)-2-((E)-3-((2,6-difluoro-4-(7-methoxy-10-(methoxymethyl)-9,12-dimethyl-9,12,13,14-tetrahydroimidazo[4",5":5',6']benzo[1',2':2,3]azocino[4,5,6-hi]indolizine-2-carbonyl)phenyl)amino)-3-oxoprop-1-en-1-yl)-4-fluoropyrrolidine-1-carboxylate was taken on in the next step without further purification.

*Tert-*butyl (2S,4R)-2-((E)-3-((2,6-difluoro-4-(7-methoxy-10-(methoxymethyl)-9,12-dimethyl-9,12,13,14-tetrahydroimidazo[4",5":5',6']benzo[1',2':2,3]azocino[4,5,6-hi]indolizine-2-carbonyl)phenyl)amino)-3-oxoprop-1-en-1-yl)-4-fluoropyrrolidine-1-carboxylate (43.6 mg, 0.056 mmol) was placed in a vial with stir bar and dissolved in DCM (376 µl). TFA (188 µl) was added dropwise and the reaction was allowed to stir for 30 min. After 30 min, LCMS analysis indicates the reaction is complete. The reaction was concentrated in vacuo (room temperature). The crude material was subjected to HPLC purification with a TFA modifer. (E)-N-(2,6-difluoro-4-(7-methoxy-10-(methoxymethyl)-9,12-dimethyl-9,12,13,14-tetrahydroimidazo[4",5":5',6']benzo[1',2':2,3]azocino[4,5,6-hi]indolizine-2-carbonyl)phenyl)-3-((2S,4R)-4-fluoropyrrolidin-2-yl)acrylamide, 2TFA **(Ex. 293)** was obtained. ¹H NMR (499 MHz, CD₃CN) δ 9.94 (d, *J =* 6.8 Hz, 1H), 9.82 (brs, 1H), 8.84 (s, 1H), 7.49 (d, *J* = 8.1 Hz, 2H), 7.43 (d, *J=* 7.2 Hz, 1H), 7.32 (d, *J=* 10.7 Hz, 2H), 7.15 (t, *J* = 7.0 Hz, 1H), 7.04 (dd, *J=* 15.5, 7.6 Hz, 1H), 6.60 (d, *J=* 15.7 Hz, 1H), 5.48 (d, *J=* 52.8 Hz, 1H), 4.77 (s, 2H), 4.69 - 4.55 (m, 1H), 3.91 (s, 3H), 3.89 (s, 3H) 3.80 (dd, *J=* 34.5, 9.9 Hz, 2H), 3.62 (dd, *J =* 24.6, 13.9 Hz, 2H), 3.52-3.35 (m, 6H), 2.82 (d, *J=* 14.2 Hz, 1H), 2.66 - 2.52 (m, 1H), 2.43 (t, *J* = 11.9 Hz, 1H), 2.26 (dt, *J=* 35.9, 11.5 Hz, 1H). [M+H]⁺ *m*/*z:* 673.

### Synthetic procedure DD

### Example 283: (E)-N-(4-(9,12-dimethyl-7-(trifluoromethyl)-9,12,13,14-tetrahydroindolizino[8',1':4,5,6]azocino[2,3-e]indazole-2-carbonyl)-2,6-difluorophenyl)-4-((3-methyloxetan-3-yl)amino)but-2-enamide (Ex. 283)

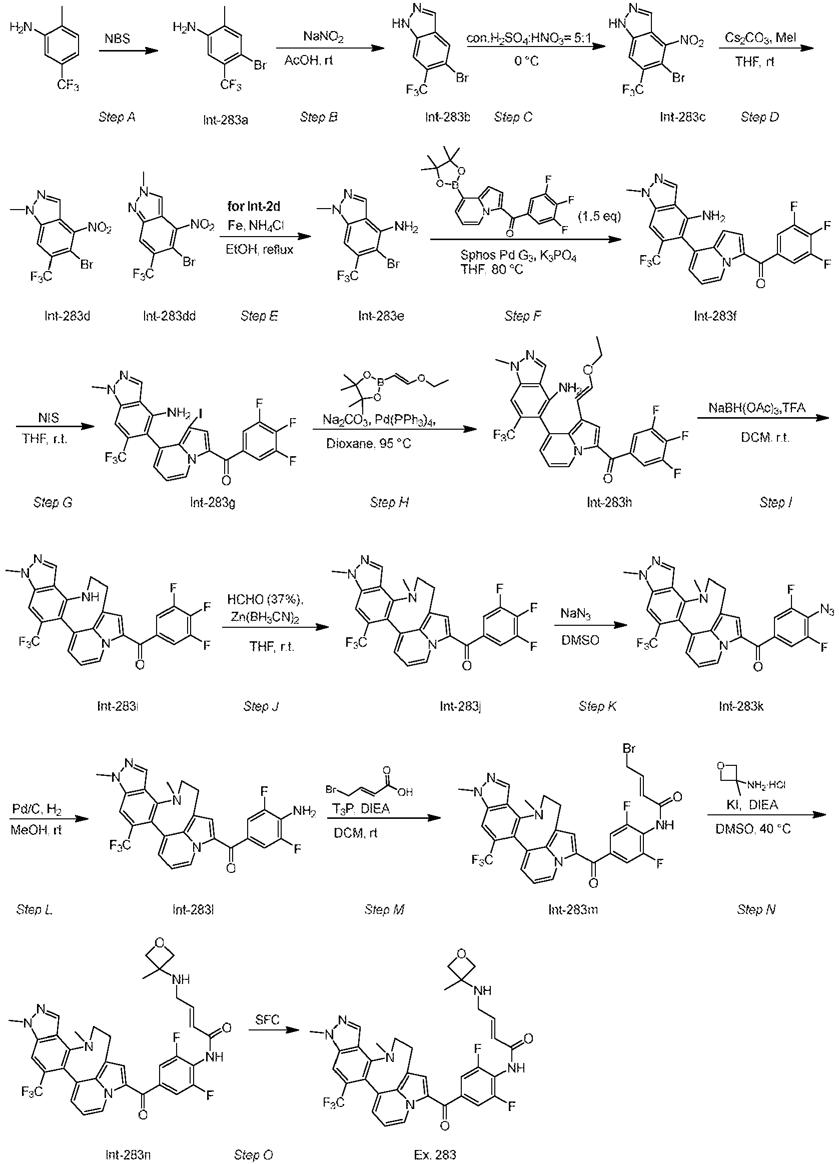

### Step A: 4-bromo-2-methyl-5-(trifluoromethyl)aniline (Int-283a)

To a solution of 2-methyl-5-(trifluoromethyl)aniline (20 g, 114 mmol) in acetonitrile (350 mL) was added NBS (22.3 g, 125 mmol) under 10 °C. After the addition, the mixture was stirred at 25 °C for 90 min. TLC (petroleum ether / ethyl acetate = 5:1) showed the new spot was formed. The mixture was quenched by addition of saturated Na₂S₂O₃ solution (50 mL), extracted with EtOAc (3 x 20 mL), dried over Na₂SO₄, filtered and the solvent was evaporated under reduced pressure to give the crude product. The crude product was purified by silica gel column chromatography using (petroleum ether /ethyl acetate 5 : 1 as eluent ) to give 4-bromo-2-methyl-5-(trifluoromethyl)aniline **(Int-283a).**

### Step B: 5-bromo-6-(trifluoromethyl)-1H-indazole (Int-283b)

To a solution of 4-bromo-2-methyl-5-(trifluoromethyl)aniline **(Int-283a)** (11.3 g, 44.4 mmol) in acetic acid (413 mL, 722 mmol) was added sodium nitrite (3.34 g, 48.4 mmol) at 0 °C slowly. After the addition, the mixture was stirred at 25 °C for 2 h. TLC (petroleum ether / ethyl acetate = 3:1) showed the new spot was formed. The reaction was quenched by addition of saturated NaHCO₃ solution until pH >7. The mixture was extracted with EtOAc (ca. 60 mL × 3). The combined organic layer was washed with brine, and then dried over Na₂SO₄, filtered and concentrated under reduced pressure to give crude product. The crude product was purified by silica gel column chromatography using (petroleum ether /ethyl acetate 3:1 as eluent) to give 5-bromo-6-(trifluoromethyl)-1*H*-indazole **(Int-283b).** MS (ESI) [M+H]⁺: *m*/*z* 264, 266.

### Step C: 5-bromo-4-nitro-6-(trifluoromethyl)-1H-indazole (Int-283c)

To a solution of 5-bromo-6-(trifluoromethyl)-1*H*-indazole **(Int-283b)** (8 g, 30.2 mmol) in conc. H₂SO₄ (160 mL, 2.94 mol) was added nitric acid fuming (8 mL, 30.2 mmol) at 0 °C slowly. After the addition, the mixture was stirred at 0 °C for 1 h. The reaction solution was poured into 500 mL of ice water, filtered and washed to collect the precipitation, and evaporated under reduced pressure to give 5-bromo-4-nitro-6-(trifluoromethyl)-1*H*-indazole **(Int-283c),** the crude product was used in the next step without purification. MS (ESI) [M+H]⁺: *m*/*z* 310, 312.

### Step D: 5-bromo-1-methyl-4-nitro-6-(trifluoromethyl)-1H-indazole (Int-283d) & 5-bromo-2-methyl-4-nitro-6-(trifluoromethyl)-2H-indazole (Int-283dd)

To a solution of 5-bromo-4-nitro-6-(trifluoromethyl)-1*H*-indazole **(Int-283c)** (6.5 g, 21.0 mmol) in THF (60 mL) was added Cs₂CO₃ (13.7 g, 41.9 mmol) at 0 °C. The mixture was stirred at 0 °C for 15 min. Then the reaction solution was added iodomethane (2.61 mL, 41.9 mmol) at 25 °C and the mixture was stirred for 15 h. The mixture was diluted with water (40 mL), extracted with EtOAc (3 x 40 mL), dried over Na₂SO₄, filtered and the solvent was evaporated under reduced pressure to give the crude product. The crude product was purified by silica gel column chromatography using (petroleum ether /ethyl acetate 2:1 as eluent ) to give 5-bromo-1-methyl-4-nitro-6-(trifluoromethyl)-1H-indazole **(Int-283d)** and 5-bromo-2-methyl-4-nitro-6-(trifluoromethyl)-2H-indazole **(Int-283dd).** MS (ESI) [M+H]⁺: *m*/*z* 324, 326. **Int-283d:** ¹H NMR (CDCl₃, 400 MHz) δ 8.02 (s, 1H), 7.94 (s, 1H), 4.14 (s, 3H) **Int-283dd:** ¹H NMR (CDCl₃, 400 MHz) δ 8.26 (s, 1H), 8.07 (s, 1H), 4.25 (s, 3H).

### Step E: 5-bromo-1-methyl-6-(trifluoromethyl)-1H-indazol-4-amine (Int-283e)

A mixture of 5-bromo-1-methyl-4-nitro-6-(trifluoromethyl)-1H-indazole **(Int-283d)** (2.5 g, 7.71 mmol), iron powder (1.11 g, 19.9 mmol), and ammonium chloride (1.07 g, 19.9 mmol) in EtOH (40 mL) and water (8 mL) was stirred at 70 °C for 1 h. The mixture was diluted with EtOAc (50 mL), and the insoluble precipitate was filtered off, and water (50 mL) was added. The organic layer was separated, washed with a saturated sodium chloride solution, and then dried over sodium sulfate. After filtration, the organic solvent was removed under reduced pressure and the obtained residue was diluted with diisopropyl ether (100 mL) and the precipitates were collected by filtration to obtain 5-bromo-1-methyl-6-(trifluoromethyl)-1H-indazol-4-amine **(Int-283e).** ¹H NMR (CD₃OD, 400 MHz) δ 8.34 (s, 1H), 7.34 (s, 1H), 3.33 (br, 3H).

### Step F: (8-(4-amino-1-methyl-6-(trifluoromethyl)-1H-indazol-5-yl)indolizin-3-yl)(3,4,5-trifluorophenyl)methanone (Int-283f)

To a solution of 5-bromo-1-methyl-6-(trifluoromethyl)-1H-indazol-4-amine (Int-**283e)** (1.7 g, 5.78 mmol), (8-(4,4,5,5-tetramethyl-1,3,2-dioxaborolan-2-yl)indolizin-3-yl)(3,4,5-trifluorophenyl)methanone (3.36 g, 8.67 mmol) in THF (20 mL) was added 1 M aqueous potassium phosphate (8.67 mL, 8.67 mmol) and SPhos Pd G3 (0.451 g, 0.578 mmol) at 25 °C under N₂ atmosphere. The mixture was stirred at 80 °C for 2 h. The mixture was cooled, diluted with water (20 mL), extracted with EtOAc (3 x 15 mL), dried over Na₂SO₄, filtered and the solvent was evaporated under reduced pressure to give the crude product. The reaction was quenched by addition of water (ca. 20 mL), extracted with EtOAc (ca. 15 mL × 3). The combined organic layer was washed with brine, and then dried over Na₂SO₄, filtered and concentrated under reduced pressure to give the crude product (8-(4-amino-1-methyl-6-(trifluoromethyl)-1H-indazol-5-yl)indolizin-3-yl)(3,4,5-trifluorophenyl)methanone **(Int-283f).** MS (ESI) [M+H]⁺: m/z 489.

### Step G: (8-(4-amino-1-methyl-6-(trifluoromethyl)-1H-indazol-5-yl)-1-iodoindolizin-3-yl)(3,4,5-trifluorophenyl)methanone (Int-283g)

To a solution of (8-(4-amino-1-methyl-6-(trifluoromethyl)-1H-indazol-5-yl)indolizin-3-yl)(3,4,5-trifluorophenyl)methanone **(Int-283f)** (1.5 g, 3.13 mmol) in THF (15 mL) was added TFA (0.542 mL, 7.04 mmol) and NIS (0.739 g, 3.29 mmol) at 25 °C. The mixture was stirred at 25 °C for 2 h. The mixture was quenched with sat. aq. NaHCO₃ (15 mL), extracted with EtOAC (3 x 15 mL), dried over Na₂SO₄, filtered and the solvent was evaporated under reduced pressure to give the crude product. The crude product was purified by flash silica gel chromatography (ISCO^{®}; 40 g SepaFlash^{®} Silica Flash Column, Eluent of 0~30% EA/PE gradient at 40 mL/min) to give (8-(4-amino-1-methyl-6-(trifluoromethyl)-1H-indazol-5-yl)-1-iodoindolizin-3-yl)(3,4,5-trifluorophenyl)methanone **(Int-283g).** MS (ESI) [M+H]⁺: *m*/*z* 615.

### Step H: (E)-(8-(4-amino-1-methyl-6-(trifluoromethyl)-1H-indazol-5-yl)-1-(2-ethoxyvinyl)indolizin-3-yl)(3,4,5-trifluorophenyl)methanone (Int-283h)

To a solution of (8-(4-amino-1-methyl-6-(trifluoromethyl)-1H-indazol-5-yl)-1-iodoindolizin-3-yl)(3,4,5-trifluorophenyl)methanone **(Int-283g)** (1.2 g, 1.95 mmol), (E)-2-(2-ethoxyvinyl)-4,4,5,5-tetramethyl-1,3,2-dioxaborolane (1.55 g, 7.81 mmol) in dioxane (12 mL) was added Na₂CO₃ (4.88 mL, 9.77 mmol) and Pd(Ph₃P)₄ (0.226 g, 0.195 mmol) at 25 °C under N₂ atmosphere. The mixture was stirred at 95 °C for 4 h. After cooled to room temperature, the reaction mixture was diluted with EtOAc (12 mL), washed with H₂O (12 mL) and brine (2 mL), dried over Na₂SO₄ and evaporated. The residue was triturated by diisopropyl ether (12 mL) and the precipitates were collected by filtration to obtain crude product. The crude product was purified by reversed MPLC to give (E)-(8-(4-amino-1-methyl-6-(trifluoromethyl)-1H-indazol-5-yl)-1-(2-ethoxyvinyl)indolizin-3-yl)(3,4,5-trifluorophenyl)methanone **(Int-283h).** MS (ESI) [M+H]⁺: *m*/*z* 559.

### Step I: (9-methyl-7-(trifluoromethyl)-9,12,13,14-tetrahydroindolizino[8',1':4,5,6]azocino[2,3-e]indazol-2-yl)(3,4,5-trifluorophenyl)methanone (Int-283i)

To a solution of (E)-(8-(4-amino-1-methyl-6-(trifluoromethyl)-1H-indazol-5-yl)-1-(2-ethoxyvinyl)indolizin-3-yl)(3,4,5-trifluorophenyl)methanone **(Int-283h)** (742 mg, 1.33 mmol) in DCM (8 mL) was added sodium triacetoxyborohydride (563 mg, 2.66 mmol) and TFA (2.047 mL, 26.6 mmol) at 25 °C. The mixture was stirred at 25 °C for 30 minutes. LCMS showed the reaction was completed. The reaction was quenched with aqueous sat. NaHCO₃ (8 mL) and 5 M aqueous NaOH (8 mL), then the resulting mixture was diluted with EtOAc (10 mL). The organic layer was washed with brine, dried over Na₂SO₄ and evaporated. The crude product was purified by flash silica gel chromatography (ISCO^{®}; 12 g SepaFlash^{®} Silica Flash Column, Eluent of 0~40% EA/PE gradient at 40 mL/min) to give (9-methyl-7-(trifluoromethyl)-9,12,13,14-tetrahydroindolizino[8',1':4,5,6]azocino[2,3-*e*]indazol-2-yl)(3,4,5-trifluorophenyl)methanone **(Int-283i).** MS (ESI) [M+H]⁺: *m*/*z* 515*.*

### Step J: (9,12-dimethyl-7-(trifluoromethyl)-9,12,13,14-tetrahydroindolizino[8',1':4,5,6]azocino[2,3-e]indazol-2-yl)(3,4,5-trifluorophenyl)methanone (Int-283j)

To a solution of (9-methyl-7-(trifluoromethyl)-9,12,13,14-tetrahydroindolizino[8',1':4,5,6]azocino[2,3-*e*]indazol-2-yl)(3,4,5-trifluorophenyl)methanone **(Int-283i)** (224 mg, 0.435 mmol) in THF (3 mL) was added aqueous formaldehyde (0.065 mL, 0.871 mmol, 37%) and Zn(BH₃CN)₂ (2.18 mL, 0.653 mmol) at 25 °C. The mixture was stirred at 25 °C for 1 h. EtOAc (4 mL) and 30% ammonium solution (3 mL) was added thereto, and the organic layer was washed with brine, dried over Na₂SO₄, and evaporated. The crude product was purified by flash silica gel chromatography (ISCO^{®}; 12 g SepaFlash^{®} Silica Flash Column, Eluent of 0~100% EtOAc/Pet. ether gradient @ 40 mL/min) to give (9,12-dimethyl-7-(trifluoromethyl)-9,12,13,14-tetrahydroindolizino[8',1':4,5,6]azocino[2,3-e]indazol-2-yl)(3,4,5-trifluorophenyl)methanone **(Int-283j).** MS (ESI) [M+H]⁺: *m*/*z* 529.

### Step K: (4-azido-3,5-difluorophenyl)(9,12-dimethyl-7-(trifluoromethyl)-9,12,13,14-tetrahydroindolizino[8',1':4,5,6]azocino[2,3-e]indazol-2-yl)methanone (Int-283k)

To a solution of (9,12-dimethyl-7-(trifluoromethyl)-9,12,13,14-tetrahydroindolizino[8',1':4,5,6]azocino[2,3-*e*]indazol-2-yl)(3,4,5-trifluorophenyl)methanone **(Int-283j)** (280 mg, 0.526 mmol) in DMSO (3 mL) was added sodium azide (100 mg, 1.538 mmol) at 25 °C. The mixture was stirred at 70 °C for 4 h. The mixture was cooled, diluted with water (3 mL), extracted with EtOAc (3 x 3 mL), dried over Na₂SO₄, filtered and the solvent was evaporated under reduced pressure to give (4-azido-3,5-difluorophenyl)(9,12-dimethyl-7-(trifluoromethyl)-9,12,13,14-tetrahydroindolizino[8',1':4,5,6]azocino[2,3-*e*]indazol-2-yl)methanone **(Int-283k),** used in the next step directly. MS (ESI) [M+H]⁺: *m*/*z 552.*

### Step L: (4-amino-3,5-difluorophenyl)(9,12-dimethyl-7-(trifluoromethyl)-9,12,13,14-tetrahydroindolizino[8',1':4,5,6]azocino[2,3-e]indazol-2-yl)methanone (Int-283l)

To a solution of (4-azido-3,5-difluorophenyl)(9,12-dimethyl-7-(trifluoromethyl)-9,12,13,14-tetrahydroindolizino[8',1':4,5,6]azocino[2,3-*e*]indazol-2-yl)methanone **(Int-283k)** (362 mg, 0.656 mmol) in MeOH (5 mL) was added wet Pd/C (6.99 mg, 0.066 mmol, 10%) at 25 °C under H₂ atmosphere (15 psi). The mixture was stirred at 25 °C for 30 min. The mixture was filtered and the solvent was evaporated under reduced pressure to give the crude product. The crude product was purified by flash silica gel chromatography (ISCO^{®}; 4 g SepaFlash^{®} Silica Flash Column, Eluent of 0~30% EtOAc/Pet. ether gradient at 40 mL/min) to give (4-amino-3,5-difluorophenyl)(9,12-dimethyl-7-(trifluoromethyl)-9,12,13,14-tetrahydroindolizino[8',1':4,5,6]azocino[2,3-e]indazol-2-yl)methanone (**Int-283l**). MS (ESI) [M+H]⁺: *m*/*z* 526*.*

### Step M: (E)-4-bromo-N-(4-(9,12-dimethyl-7-(trifluoromethyl)-9,12,13,14-tetrahydroindolizino[8',1':4,5,6]azocino[2,3-e]indazole-2-carbonyl)-2,6-difluorophenyl)but-2-enamide (Int-283m)

To a solution of (4-amino-3,5-difluorophenyl)(9,12-dimethyl-7-(trifluoromethyl)-9,12,13,14-tetrahydroindolizino[8',1':4,5,6]azocino[2,3-*e*]indazol-2-yl)methanone (Int-**283l**) (90 mg, 0.171 mmol), (E)-4-bromobut-2-enoic acid (48.0 mg, 0.291 mmol) in CH₂Cl₂ (1 mL) were added T3P (545 mg, 0.856 mmol, 50% in EA) and DIEA (0.179 mL, 1.028 mmol) at 25 °C . The mixture was stirred at 25 °C for 2 h. The mixture was diluted with sat.aq. NaHCO₃ (1 mL), extracted with DCM (3 x 2 mL), dried over Na₂SO₄, filtered and the solvent was evaporated under reduced pressure to give (E)-4-bromo-N-(4-(9,12-dimethyl-7-(trifluoromethyl)-9,12,13,14-tetrahydroindolizino[8',1':4,5,6]azocino[2,3-e]indazole-2-carbonyl)-2,6-difluorophenyl)but-2-enamide **(Int-283m),** used in the next step directly. MS (ESI) [M+H]⁺: *m*/*z* 672, 674.

### Step N: (E)-N-(4-(9,12-dimethyl-7-(trifluoromethyl)-9,12,13,14-tetrahydroindolizino[8',1':4,5,6]azocino[2,3-e]indazole-2-carbonyl)-2,6-difluorophenyl)-4-((3-methyloxetan-3-yl)amino)but-2-enamide (Int-283n)

To a solution of (E)-4-bromo-N-(4-(9,12-dimethyl-7-(trifluoromethyl)-9,12,13,14-tetrahydroindolizino[8',1':4,5,6]azocino[2,3-*e*]indazole-2-carbonyl)-2,6-difluorophenyl)but-2-enamide **(Int-283m)** (115 mg, 0.171 mmol) in DMSO (2 mL) was added DIEA (0.149 mL, 0.855 mmol), KI (142 mg, 0.855 mmol) and 3-methyloxetan-3-amine HCl (126 mg, 1.03 mmol) at 25 °C. The mixture was stirred at 40 °C for 4 h. The mixture was cooled, diluted with water (2 mL), extracted with EtOAc (3 x 2 mL), dried over Na₂SO₄, filtered and the solvent was evaporated under reduced pressure to give the crude product. The crude product was purified by flash silica gel chromatography (ISCO^{®}; 4 g SepaFlash^{®} Silica Flash Column, Eluent of 0~30% EtOAc/Pet. ether gradient at 40 mL/min) to give (E)-N-(4-(9,12-dimethyl-7-(trifluoromethyl)-9,12,13,14-tetrahydroindolizino[8',1':4,5,6]azocino[2,3-*e*]indazole-2-carbonyl)-2,6-difluorophenyl)-4-((3-methyloxetan-3-yl)amino)but-2-enamide **(Int-283n).** MS (ESI) [M+H]⁺: *m*/*z* 679.

### Step O: (E)-N-(4-(9,12-dimethyl-7-(trifluoromethyl)-9,12,13,14-tetrahydroindolizino[8',1':4,5,6]azocino[2,3-e]indazole-2-carbonyl)-2,6-difluorophenyl)-4-((3-methyloxetan-3-yl)amino)but-2-enamide (Ex. 283)

*(E)-N-(4-(9,12-dimethyl-7-(trifluoromethyl)-9,12,13,14-*tetrahydroindolizino[8',1':4,5,6]azocino[2,3-*e*]indazole-2-carbonyl)-2,6-difluorophenyl)-4-((3-methyloxetan-3-yl)amino)but-2-enamide **(Int-283n)** (50 mg, 0.074 mmol) was separated by SFC (Column: DAICEL CHIRALCEL IG (250 mm * 30 mm, 10 um), Condition: 0.1% NH₃ in EtOH, Flow rate: 80 mL/min.) to give (E)-N-(4-(9,12-dimethyl-7-(trifluoromethyl)-9,12,13,14-tetrahydroindolizino[8',1':4,5,6]azocino[2,3-*e*]indazole-2-carbonyl)-2,6-difluorophenyl)-4-((3-methyloxetan-3-yl)amino)but-2-enamide **(Ex. 283)** (the second eluting isomer from SFC). MS (ESI) [M+H]⁺: *m*/*z* 679. ¹H NMR (400 MHz, CD₃OD) δ 9.95 (dd, *J=* 6.2, 1.8 Hz, 1H), 8.43 (d, *J=* 0.6 Hz, 1H), 8.13 (s, 1H), 7.51 (d, *J* = 7.9 Hz, 2H), 7.25 (s, 1H), 7.16 - 7.09 (m, 2H), 6.98 (td, *J=* 15.3 Hz, 6.8, 1H), 6.67 (d, *J* = 15.3 Hz, 1H), 4.81 (d, *J* = 7.9 Hz, 2H), 4.61 (d, *J* = 7.8 Hz, 2H), 4.22 (s, 3H), 4.01 (dd, *J* = 6.8, 1.0 Hz, 2H), 3.31 - 3.27 (m, 1H), 3.04 - 2.97 (m, 4H), 2.67 (br dd, *J* = 15.0, 2.0, Hz, 1H), 2.30 - 2.18 (m, 1H), 1.76 (s, 3H).

### Synthetic procedure EE

### Example 300: (E)-N-(4-(7-(difluoromethoxy)-9,10,12-trimethyl-9,12,13,14-tetrahydroimidazo[4",5":5',6']benzo[1',2':2,3]azocino[4,5,6-hi]indolizine-2-carbonyl)-2,6-difluorophenyl)-4-((1-methylcyclopropyl)amino)but-2-enamide (Ex. 300)

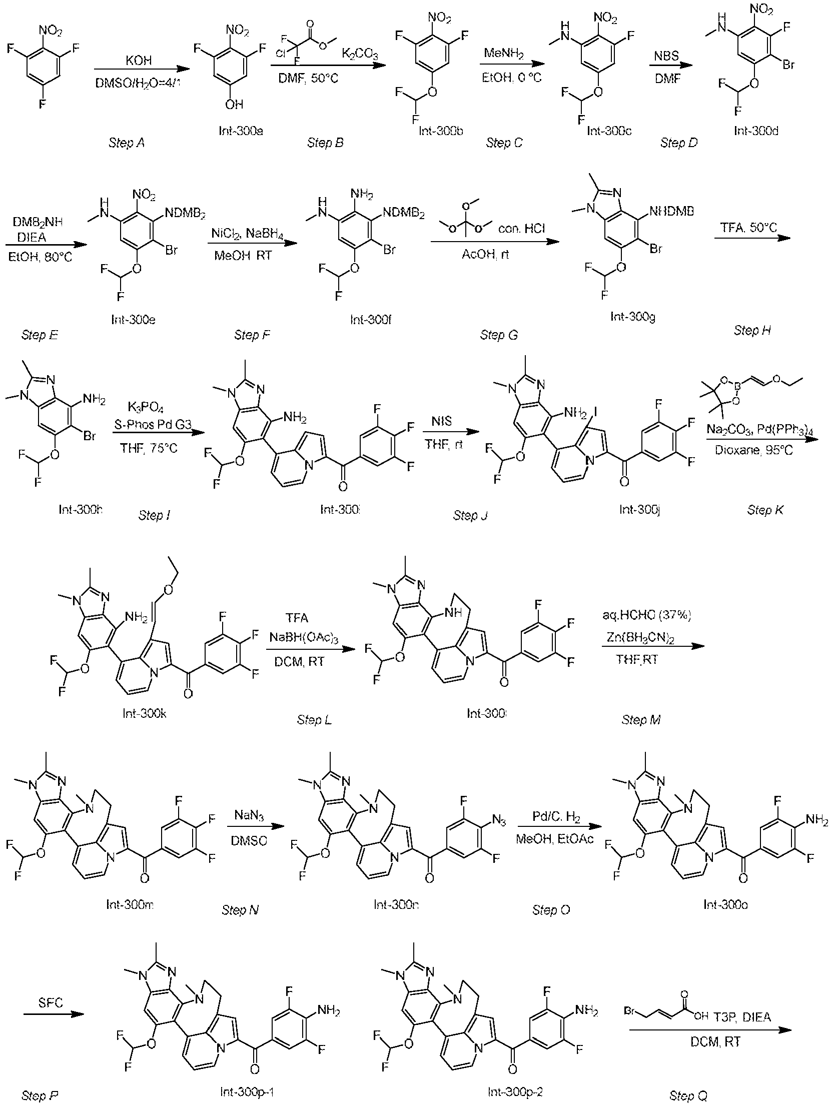

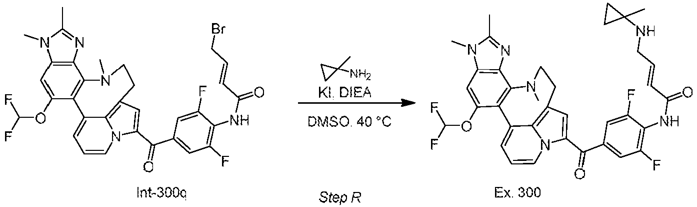

### Step A: 3,5-difluoro-4-nitrophenol (Int-300a)

To a solution of 1,3,5-trifluoro-2-nitrobenzene (20. g, 110 mmol) in DMSO (240 mL) and water (60 mL) was added potassium hydroxide (12.67 g, 226 mmol) at 25 °C. The mixture was stirred at 25 °C for 12 h. The mixture was acidified to pH 6 with aq. HCl (3 N), extracted with EtOAc (3 x 50 mL), dried over Na₂SO₄, filtered and the solvent was evaporated under reduced pressure to give the crude product. The crude product was purified by flash silica gel chromatography (Eluent of 0~10% EtOAc/Pet. ether gradient) to give 3,5-difluoro-4-nitrophenol **(Int-300a).** ¹H NMR (500MHz, CDCl₃) δ 7.89 (s, 1H), 6.51-6.56 (m, 2H).

### Step B: 5-(difluoromethoxy)-1,3-difluoro-2-nitrobenzene (Int-300b)

To a solution of 3,5-difluoro-4-nitrophenol **(Int-300a)** (3.6 g, 21 mmol) in DMF (50 mL) was added potassium carbonate (11.37 g, 82 mmol) and methyl 2-chloro-2,2-difluoroacetate (8.91 g, 61.7 mmol) at 25 °C. The mixture was stirred at 50 °C for 12 h. The mixture was cooled, diluted with water (30 mL), extracted with EtOAc (3 x 30 mL), dried over Na₂SO₄, filtered and the solvent was evaporated under reduced pressure to give the crude product. The crude product was purified by flash silica gel chromatography (Silica Flash Column, Eluent of 0~10% EtOAc/Pet. ether gradient) to give 5-(difluoromethoxy)-1,3-difluoro-2-nitrobenzene **(Int-300b).** ¹H NMR (500MHz, CDCl₃) δ 6.86-6.93 (m, 2H), 6.62 (t, J=71.5 Hz, 1H).

### Step C: 5-(difluoromethoxy)-3-fluoro-N-methyl-2-nitroaniline (Int-300c)

To a solution of 5-(difluoromethoxy)-1,3-difluoro-2-nitrobenzene **(Int-300b)** (2.4 g, 11 mmol) in EtOH (30 mL) was added methanamine (2.7 g, 29 mmol, 33% in EtOH) at 0 °C. The mixture was stirred at 0 °C for 1 h. The mixture was diluted with water (10 mL), extracted with EtOAc (3 x 10 mL), dried over Na₂SO₄, filtered and the solvent was evaporated under reduced pressure to give the crude product. The crude product was purified by flash silica gel chromatography (Silica Flash Column, Eluent of 0~30% EtOAc/Pet. ether gradient) to give 5-(difluoromethoxy)-3-fluoro-N-methyl-2-nitroaniline **(Int-300c).** MS (ESI) [M+H]⁺: *m*/*z 237.*

### Step D: 4-bromo-5-(difluoromethoxy)-3-fluoro-N-methyl-2-nitroaniline (Int-300d)

To a solution of 5-(difluoromethoxy)-3-fluoro-N-methyl-2-nitroaniline **(Int-300c)** (1.7 g, 7.20 mmol) in DMF (30 mL) was added 1-bromopyrrolidine-2,5-dione (1.345 g, 7.56 mmol) at 25 °C. The mixture was stirred at 25 °C for 1.5 h. The mixture was diluted with water (20 mL), extracted with EtOAc (3 x 10 mL), dried over Na₂SO₄, filtered and the solvent was evaporated under reduced pressure to give the crude product. The crude product was purified by flash silica gel chromatography (Silica Flash Column, Eluent of 0~30% EtOAc/Pet. ether gradient) to give 4-bromo-5-(difluoromethoxy)-3-fluoro-N-methyl-2-nitroaniline **(Int-300d).** MS (ESI) [M+H]⁺: *m*/*z* 315, 317.

### Step E: 4-bromo-5-(difluoromethoxy)-N3,N3-bis(2,4-dimethoxybenzyl)-N1-methyl-2-nitrobenzene-1,3-diamine (Int-300e)

To a solution of 4-bromo-5-(difluoromethoxy)-3-fluoro-N-methyl-2-nitroaniline **(Int-300d)** (1.1 g, 3.5 mmol) in EtOH (20 ml) was added N-ethyl-N-isopropylpropan-2-amine (1.829 mL, 10.47 mmol) and N-(2,4-dimethoxybenzyl)-1-(2,5-dimethoxyphenyl)methanamine (2.216 g, 6.98 mmol) at 25 °C. The mixture was stirred at 80 °C for 12 h. The mixture was cooled, diluted with water (20 mL), extracted with DCM (3 x15 mL), dried over Na₂SO₄, filtered and the solvent was evaporated under reduced pressure to give the crude product. The crude product was purified by flash silica gel chromatography (Silica Flash Column, Eluent of 0~10% EtOAc/Pet. ether gradient) to give 4-bromo-5-(difluoromethoxy)-N3,N3-bis(2,4-dimethoxybenzyl)-N1-methyl-2-nitrobenzene-1,3-diamine **(Int-300e).** MS (ESI) [M+H]⁺: *m*/*z* 612, 614.

### Step F: 4-bromo-5-(difluoromethoxy)-N3,N3-bis(2,4-dimethoxybenzyl)-N1-methylbenzene-1,2,3-triamine (Int-300f)

To a solution of 4-bromo-5-(difluoromethoxy)-N3,N3-bis(2,4-dimethoxybenzyl)-N1-methyl-2-nitrobenzene-1,3-diamine **(Int-300e)** (1.8 g, 2.9 mmol) in MeOH (30 mL) was added nickel(II) chloride (1.14 g, 8.82 mmol) and sodium borohydride (0.556 g, 14.70 mmol) at 25 °C under N₂ atmosphere. The mixture was stirred at 25 °C for 1 h. The mixture was quenched with water (20 mL), extracted with EtOAc (2 x 15 mL), dried over Na₂SO₄, filtered and the solvent was evaporated under reduced pressure to give the crude 4-bromo-5-(difluoromethoxy)-N3,N3-bis(2,4-dimethoxybenzyl)-N1-methylbenzene-1,2,3-triamine **(Int-300f).** MS (ESI) [M+H]⁺: *m*/*z* 582, 584.

### Step G: 5-bromo-6-(difluoromethoxy)-N-(2,4-dimethoxybenzyl)-1,2-dimethyl-1H-benzo[d]imidazol-4-amine (Int-300g)

To a mixture of 4-bromo-5-(difluoromethoxy)-N3,N3-bis(2,4-dimethoxybenzyl)-N1-methylbenzene-1,2,3-triamine **(Int-300f)** (1.7 g, 2.9 mmol) and 1,1,1-trimethoxyethane (5.26 g, 43.8 mmol) was added acetic acid (0.167 mL, 2.92 mmol) and conc. HCl (0.024 mL, 0.29 mmol) at 25 °C under N₂ atmosphere. The mixture was stirred at 25 °C for 12 h. The mixture was basified to pH 8 with aq. NaOH (2N), diluted with EtOAc (10 mL), washed with water (10 mL), dried over Na₂SO₄, filtered and the solvent was evaporated under reduced pressure to give the crude 5-bromo-6-(difluoromethoxy)-N-(2,4-dimethoxybenzyl)-1,2-dimethyl-1H-benzo[d]imidazol-4-amine **(Int-300g).** MS (ESI) [M+H]⁺: *m*/*z* 456 458.

### Step H: 5-bromo-6-(difluoromethoxy)-1,2-dimethyl-1H-benzo[d]imidazol-4-amine (Int-300h)

A mixture of 5-bromo-6-(difluoromethoxy)-N-(2,4-dimethoxybenzyl)-1,2-dimethyl-1H-benzo[d]imidazol-4-amine **(Int-300g)** (1.33 g, 2.91 mmol) and TFA (15 mL, 195 mmol) was stirred at 50 °C for 2 h. The mixture was evaporated under reduced pressure to give the crude product. The residue was purified by reverse preparative HPLC (Column: Boston Uni C18 150 * 40 mm * 5 um; Condition: water (0.01% TFA)-ACN; Begin B--End B: 14--44; Gradient Time (min): 10; 100% B Hold Time (min): 2; FlowRate (mL/min): 60) to give 5-bromo-6-(difluoromethoxy)-1,2-dimethyl-1H-benzo[d]imidazol-4-amine **(Int-300h).** MS (ESI) [M+H]⁺: *m*/*z* 306, 308.

### Step I: (8-(4-amino-6-(difluoromethoxy)-1,2-dimethyl-1H-benzo[d]imidazol-5-yl)indolizin-3-yl)(3,4,5-trifluorophenyl)methanone (Int-300i)

To a solution of 5-bromo-6-(difluoromethoxy)-1,2-dimethyl-1H-benzo[d]imidazol-4-amine **(Int-300h)** (350 mg, 1.14 mmol), (8-(5,5-dimethyl-1,3,2-dioxaborinan-2-yl)indolizin-3-yl)(3,4,5-trifluorophenyl)methanone (528 mg, 1.36 mmol) in THF (8 mL) was added potassium phosphate (1.136 ml, 1.704 mmol) and SPhos Pd G3 (89 mg, 0.11 mmol) at 25 °C under N₂ atmosphere. The mixture was stirred at 75 °C for 1.5 h. The mixture was cooled, diluted with water (5 mL), extracted with EtOAc (3 x 5 mL), dried over Na₂SO₄, filtered and the solvent was evaporated under reduced pressure to give the crude product. The crude product was purified by flash silica gel chromatography (Silica Flash Column, Eluent of 0~50% EA/PE gradient) to give (8-(4-amino-6-(difluoromethoxy)-1,2-dimethyl-1H-benzo[d]imidazol-5-yl)indolizin-3-yl)(3,4,5-trifluorophenyl)methanone **(Int-300i).** MS (ESI) [M+H]⁺: *m*/*z 501.*

### Step J: (8-(4-amino-6-(difluoromethoxy)-1,2-dimethyl-1H-benzo[d]imidazol-5-yl)-1-iodoindolizin-3-yl)(3,4,5-trifluorophenyl)methanone (Int-300j)

To a solution of (8-(4-amino-6-(difluoromethoxy)-1,2-dimethyl-1H-benzo[d]imidazol-5-yl)indolizin-3-yl)(3,4,5-trifluorophenyl)methanone **(Int-300i)** (550. mg, 1.10 mmol) in THF (10 mL) was added 1-iodopyrrolidine-2,5-dione (24.73 mg, 0.110 mmol) and 2,2,2-trifluoroacetic acid (0.191 mL, 2.47 mmol) at 25 °C. The mixture was stirred at 25 °C for 1 h. The mixture was quenched with sat. aq. NaHCO₃ (10 mL), extracted with EtOAc (3 x 10 mL), dried over Na₂SO₄, filtered and the solvent was evaporated under reduced pressure to give the crude product. The crude product was purified by flash silica gel chromatography (Silica Flash Column, Eluent of 0~50% EtOAc/Pet. ether gradient) to give (8-(4-amino-6-(difluoromethoxy)-1,2-dimethyl-1H-benzo[d]imidazol-5-yl)-1-iodoindolizin-3-yl)(3,4,5-trifluorophenyl)methanone (Int-300j). MS (ESI) [M+H]⁺: *m*/*z* 627.

### Step K: (E)-(8-(4-amino-6-(difluoromethoxy)-1,2-dimethyl-1H-benzo[d]imidazol-5-yl)-1-(2-ethoxyvinyl)indolizin-3-yl)(3,4,5-trifluorophenyl)methanone (Int-300k)

To a solution of (8-(4-amino-6-(difluoromethoxy)-1,2-dimethyl-1H-benzo[d]imidazol-5-yl)-1-iodoindolizin-3-yl)(3,4,5-trifluorophenyl)methanone **(Int-300j)** (550. mg, 0.878 mmol), (E)-2-(2-ethoxyvinyl)-4,4,5,5-tetramethyl-1,3,2-dioxaborolane (522 mg, 2.63 mmol) in dioxane (10 mL) was added sodium carbonate (2.195 mL, 4.39 mmol, 2M) and Pd(PPh₃)₄ (101 mg, 0.088 mmol) at 25 °C under N₂ atmosphere. The mixture was stirred at 95 °C for 4 h. After cooling, the reaction mixture was diluted with EtOAc (20 mL), washed with H₂O (15 mL) and brine (15 mL), dried over Na₂SO₄ and evaporated. The residue was purified by flash silica gel chromatography (Silica Flash Column, Eluent of 0~50% EtOAc/Pet. ether gradient) to give (E)-(8-(4-amino-6-(difluoromethoxy)-1,2-dimethyl-1H-benzo[d]imidazol-5-yl)-1-(2-ethoxyvinyl)indolizin-3-yl)(3,4,5-trifluorophenyl)methanone **(Int-300k).** MS (ESI) [M+H]⁺: *m*/*z* 571.

### Step L: (7-(difluoromethoxy)-9,10-dimethyl-9,12,13,14-tetrahydroimidazo[4",5":5',6']benzo[1',2':2,3]azocino[4,5,6-hi]indolizin-2-yl)(3,4,5-trifluorophenyl)methanone (Int-300l)

To a solution of (E)-(8-(4-amino-6-(difluoromethoxy)-1,2-dimethyl-1H-benzo[d]imidazol-5-yl)-1-(2-ethoxyvinyl)indolizin-3-yl)(3,4,5-trifluorophenyl)methanone **(Int-300k)** (450 mg, 0.789 mmol) in CH₂Cl₂ (8 mL) was added sodium triacetoxyhydroborate (334 mg, 1.58 mmol) and 2,2,2-trifluoroacetic acid (1.215 mL, 15.78 mmol) at 25 °C. The mixture was stirred at 25 °C for 2 h. The mixture was quenched with sat. aq. NaHCO₃ (20 mL), extracted with DCM (3 x 10 mL), dried over Na₂SO₄, filtered and the solvent was evaporated under reduced pressure to give the crude product. The crude product was purified by flash silica gel chromatography (Silica Flash Column, Eluent of 0~50% EtOAc/Pet. ether gradient) to give (7-(difluoromethoxy)-9,10-dimethyl-9,12,13,14-tetrahydroimidazo[4",5":5',6']benzo[1',2':2,3]azocino[4,5,6-hi]indolizin-2-yl)(3,4,5-trifluorophenyl)methanone (**Int-300l**). MS (ESI) [M+H]⁺: *m*/*z* 527.

### Step M: (7-(difluoromethoxy)-9,10,12-trimethyl-9,12,13,14-tetrahydroimidazo[4",5":5',6']benzo[1',2':2,3]azocino[4,5,6-hi]indolizin-2-yl)(3,4,5-trifluorophenyl)methanone (Int-300m)

To a solution of (7-(difluoromethoxy)-9,10-dimethyl-9,12,13,14-tetrahydroimidazo[4",5":5',6']benzo[1',2':2,3]azocino[4,5,6-hi]indolizin-2-yl)(3,4,5-trifluorophenyl)methanone (**Int-3001**) (370. mg, 0.700 mmol) in THF (6 mL) was added formaldehyde (0.104 mL, 1.40 mmol, 37% aqueous) and Zn(BH₃CN)₂ (3.50 mL, 1.05 mmol, 0.3 M) at 25 °C. The mixture was stirred at 25 °C for 1 h. The mixture was diluted with EtOAc (10 mL) and NH₃·H₂O (1 mL), washed with brine (10 mL), dried over Na₂SO₄, filtered and the solvent was evaporated under reduced pressure to give the crude product. The crude product was purified by flash silica gel chromatography (Silica Flash Column, Eluent of 0~50% EtOAc/Pet. ether gradient) to give (7-(difluoromethoxy)-9,10,12-trimethyl-9,12,13,14-tetrahydroimidazo[4",5":5',6']benzo[1',2':2,3]azocino[4,5,6-hi]indolizin-2-yl)(3,4,5-trifluorophenyl)methanone **(Int-300m).** MS (ESI) [M+H]⁺: *m*/*z* 541.

### Step N: (4-azido-3,5-difluorophenyl)(7-(difluoromethoxy)-9,10,12-trimethyl-9,12,13,14-tetrahydroimidazo[4",5":5',6']benzo[1',2':2,3]azocino[4,5,6-hi]indolizin-2-yl)methanone (Int-300n)

To a solution of (7-(difluoromethoxy)-9,10,12-trimethyl-9,12,13,14-tetrahydroimidazo[4",5":5',6']benzo[1',2':2,3]azocino[4,5,6-hi]indolizin-2-yl)(3,4,5-trifluorophenyl)methanone **(Int-300m)** (150. mg, 0.278 mmol) in DMSO (3 mL) was added sodium azide (200. mg, 3.08 mmol) at 25 °C, the reaction was sitrred at 70 °C for 5 h. After cooled to room temperature, the mixture was diluted with water (5 mL), extracted with EtOAc (3 x 5 mL), the organic layer was separated, washed with brine (5 mL), dried over Na₂SO₄, evaporated to give crude (4-azido-3,5-difluorophenyl)(7-(difluoromethoxy)-9,10,12-trimethyl-9,12,13,14-tetrahydroimidazo[4",5":5',6']benzo[1',2':2,3]azocino[4,5,6-hi]indolizin-2-yl)methanone **(Int-300n).** MS (ESI) [M+H]⁺: *m*/*z* 564*.*

### Step O: (4-amino-3,5-difluorophenyl)(7-(difluoromethoxy)-9,10,12-trimethyl-9,12,13,14-tetrahydroimidazo[4",5":5',6']benzo[1',2':2,3]azocino[4,5,6-hi]indolizin-2-yl)methanone (Int-300o)

A mixture of (4-azido-3,5-difluorophenyl)(7-(difluoromethoxy)-9,10,12-trimethyl-9,12,13,14-tetrahydroimidazo[4",5":5',6']benzo[1',2':2,3]azocino[4,5,6-hi]indolizin-2-yl)methanone (156 mg, 0.277 mmol) and wet Pd/C (58.9 mg, 0.055 mmol, 10%) in EtOAc (10 mL) and MeOH (2 mL) was stirred at 25 °C for 1 h under under 15 psi of H₂. The mixture was filtered and the solvent was evaporated under reduced pressure to give the crude product. The crude product was purified by flash silica gel chromatography (Silica Flash Column, Eluent of 0~50% EtOAc/Pet. ether gradient) to give (4-amino-3,5-difluorophenyl)(7-(difluoromethoxy)-9,10,12-trimethyl-9,12,13,14-tetrahydroimidazo[4",5":5',6']benzo[1',2':2,3]azocino[4,5,6-hi]indolizin-2-yl)methanone (**Int-300o**). MS (ESI) [M+H]⁺: *m*/*z* 538.

### Step P: (4-amino-3,5-difluorophenyl)(7-(difluoromethoxy)-9,10,12-trimethyl-9,12,13,14-tetrahydroimidazo[4",5":5',6']benzo[1',2':2,3]azocino[4,5,6-hi]indolizin-2-yl)methanone (Int-300p)

(4-amino-3,5-difluorophenyl)(7-(difluoromethoxy)-9,10,12-trimethyl-9,12,13,14-tetrahydroimidazo[4",5":5',6']benzo[1',2':2,3]azocino[4,5,6-hi]indolizin-2-yl)methanone **(Int-300o)** (120 mg, 0.223 mmol) was separated by SFC (Column: DAICEL CHIRALPAK IG (250 mm * 30 mm, 10 um), Condition: 0.1% NH₃H₂O/IPA, Mobile phase: A: CO₂ B: IPA (0.1% NH₃H₂O)) to give (4-amino-3,5-difluorophenyl)(7-(difluoromethoxy)-9,10,12-trimethyl-9,12,13,14-tetrahydroimidazo[4",5":5',6']benzo[1',2':2,3]azocino[4,5,6-hi]indolizin-2-yl)methanone **(Int-300p-1,** the first eluting isomer from SFC), (4-amino-3,5-difluorophenyl)(7-(difluoromethoxy)-9,10,12-trimethyl-9,12,13,14-tetrahydroimidazo[4",5":5',6']benzo[1',2':2,3]azocino[4,5,6-hi]indolizin-2-yl)methanone **(Int-300p-2,** the second eluting isomer from SFC). MS (ESI) [M+H]⁺: *m*/*z* 538*.*

### Step Q: (E)-4-bromo-N-(4-(7-(difluoromethoxy)-9,10,12-trimethyl-9,12,13,14-tetrahydroimidazo[4",5":5',6']benzo[1',2':2,3]azocino[4,5,6-hi]indolizine-2-carbonyl)-2,6-difluorophenyl)but-2-enamide (Int-300q)

To a solution of (4-amino-3,5-difluorophenyl)(7-(difluoromethoxy)-9,10,12-trimethyl-9,12,13,14-tetrahydroimidazo[4",5":5',6']benzo[1',2':2,3]azocino[4,5,6-hi]indolizin-2-yl)methanone (40 mg, 0.074 mmol) **(Int-300p-1)** and (E)-4-bromobut-2-enoic acid (18. mg, 0.11 mmol) in CH₂Cl₂ (2 mL) was added 2,4,6-tripropyl-1,3,5,2,4,6-trioxatriphosphinane 2,4,6-trioxide (237 mg, 0.372 mmol, 50% in EtOAc) and N-ethyl-N-isopropylpropan-2-amine (0.078 mL, 0.44 mmol) at 25 °C. The mixture was stirred at 25 °C for 1 h. The mixture was quenched with sat. aq. NaHCO₃ (2 mL), extracted with DCM (3 x 2 mL), dried over Na₂SO₄, filtered and the solvent was evaporated under reduced pressure to give (E)-4-bromo-N-(4-(7-(difluoromethoxy)-9,10,12-trimethyl-9,12,13,14-tetrahydroimidazo[4",5":5',6']benzo[1',2':2,3]azocino[4,5,6-hi]indolizine-2-carbonyl)-2,6-difluorophenyl)but-2-enamide **(Int-300q).** MS (ESI) [M+H]⁺: *m*/*z* 684.0, 686.0.

### Step R: (E)-N-(4-(7-(difluoromethoxy)-9,10,12-trimethyl-9,12,13,14-tetrahydroimidazo[4",5":5',6']benzo[1',2':2,3]azocino[4,5,6-hi]indolizine-2-carbonyl)-2,6-difluorophenyl)-4-((1-methylcyclopropyl)amino)but-2-enamide (Ex. 300)

To a solution of (E)-4-bromo-N-(4-(7-(difluoromethoxy)-9, 10, 12-trimethyl-9,12,13,14-tetrahydroimidazo[4",5":5',6']benzo[1',2':2,3]azocino[4,5,6-hi]indolizine-2-carbonyl)-2,6-difluorophenyl)but-2-enamide **(Int-300q)** (50. mg, 0.073 mmol), 1-methylcyclopropan-1-amine, HCl (24 mg, 0.22 mmol) in DMSO (1 mL) was added N-ethyl-N-isopropylpropan-2-amine (0.077 mL, 0.44 mmol) and potassium iodide (61 mg, 0.37 mmol) at 25 °C. The mixture was stirred at 40 °C for 1 h. The mixture was cooled, diluted with water (2 mL), extracted with DCM (3 x 2 mL), dried over Na₂SO₄, filtered and the solvent was evaporated under reduced pressure to give the crude product. The residue was purified by reverse preparative HPLC (Column: Welch Xtimate C18 150 * 25 mm * 5 um; Condition: water (0.01% TFA)-ACN; Begin B--End B: 22--52; Gradient Time (min): 11; 100% B Hold Time (min): 2; FlowRate (mL/min): 25) to give (E)-N-(4-(7-(difluoromethoxy)-9,10,12-trimethyl-9,12,13,14-tetrahydroimidazo[4",5":5',6']benzo[1',2':2,3]azocino[4,5,6-hi]indolizine-2-carbonyl)-2,6-difluorophenyl)-4-((1-methylcyclopropyl)amino)but-2-enamide **(Ex. 300).** MS (ESI) [M+H]⁺: *m*/*z* 675 ¹H NMR (400MHz, MeOD) δ 10.02 (d, J=6.8 Hz, 1H), 7.75 (s, 1H), 7.50-7.52 (m, 2H), 7.36-7.39 (m, 1H), 7.20 - 7.22 (m, 1H), 6.93 - 6.98 (m, 1H), 6.62 - 6.80 (m, 3H), 4.00 - 4.06 (m, 2H), 3.60 (s, 3H), 3.58 - 3.60 (m, 1H), 3.02 - 3.09 (m, 4H), 2.87 (s, 3H), 2.77-2.80 (m, 1H), 2.47-2.48 (m, 1H), 1.33 (s, 3H), 1.10 -1.14 (m, 2H), 0.87-0.91 (m, 2H).

### Synthesis of 5-bromo-2-(methoxymethyl)-1-methyl-6-(trifluoromethyl)-1H-benzo[d]imidazol-4-amine

### Step 1:

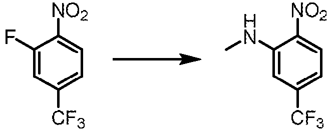

Methylamine (2M THF solution, 108 mL) was added to 2-fluoro-1-nitro-4-(trifluoromethyl)benzene (15.0 g) in THF (15 mL), and the reaction mixture was stirred at room temperature overnight. After dilution with ethyl acetate, the mixture was washed with water and a saturated sodium chloride solution and dried over sodium sulfate, filtered, concentrated, thereby obtaining N-methyl-2-nitro-5-(trifluoromethyl)aniline (15.6 g).

### Step 2:

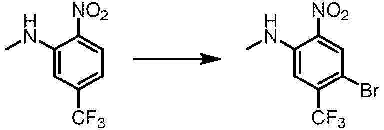

N-Bromosuccinimide (13.9 g) was added to N-methyl-2-nitro-5-(trifluoromethyl)aniline (15.6 g) obtained in step 1 in DMA (100 mL). After stirring at 50 °C for 1 hour, sodiumthiosulfate and water (150 mL) was added to the reaction mixture. The resulting precipitate was collected by filtration, thereby obtaining 4-bromo-N-methyl-2-nitro-5-(trifluoromethyl)aniline (20.6 g).

### Step 3:

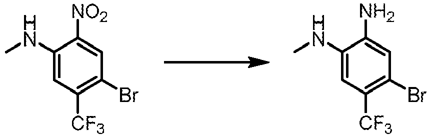

A solution of 4-bromo-N-methyl-2-nitro-5-(trifluoromethyl)aniline (7.0 g) obtained in step 2, iron powder (6.54 g), and ammonium chloride (6.26 g) in methanol (30 mL), THF (30 mL) and water (7 mL) was stirred at 60 °C for 2 hours. The insoluble matter was filtered off, followed by evaporating the solvent under reduced pressure. After dilution with ethyl acetate, the mixture was washed with water and a saturated sodium chloride solution, followed by drying over sodium sulfate. After evaporation, the obtained residue was purified by column chromatography on silica (hexane:acetone), thereby obtaining 4-bromo-N1-methyl-5-(trifluoromethyl)benzene-1,2-diamine.

### Step 4:

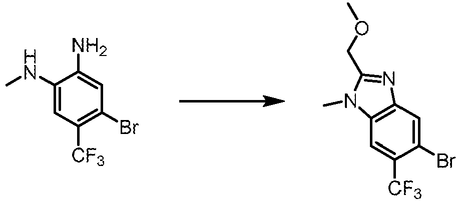

T3P in THF (1.6 M, 18 mL) was added to a solution of 4-bromo-N1-methyl-5-(trifluoromethyl)benzene-1,2-diamine (3.51 g) obtained in step 3 and 2-methoxyacetic acid (1.1 mL) in DMF (10 mL). After stirring at room temperature for 15 minutes, triethylamine (7.2 mL) was added to the reaction mixture, followed by stirring at room temperature for 2 hours. After dilution with ethyl acetate, the mixture was washed with saturated sodium bicarbonate, water and a saturated sodium chloride solution, followed by drying over sodium sulfate. After the solvent was evaporated under reduced pressure, acetic acid was added to the residue, followed by stirring at 50 °C for 2 hours. After dilution with ethyl acetate, the organic layer was washed with a saturated sodium bicarbonate solution and a saturated sodium chloride solution, followed by drying over sodium sulfate. After evaporation, the obtained residue was purified by column chromatography (hexane:acetone), thereby obtaining 5-bromo-2-(methoxymethyl)-1-methyl-6-(trifluoromethyl)-1H-benzo[d]imidazole.

### Step 5:

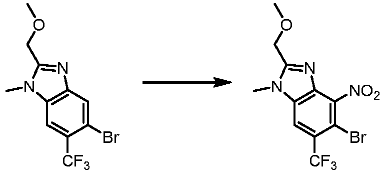

Potassium nitrate (1.31 g) was added to 5-bromo-2-(methoxymethyl)-1-methyl-6-(trifluoromethyl)-1H-benzo[d]imidazole (2.79 g) obtained in step 4 in sulfuric acid (10 mL) at 0 °C. After stirring at room temperature overnight, the reaction mixture was carefully poured into the crushed ice and 30% ammonia solution and saturated sodium bicarbonate was added. The resulting precipitate was collected by filtration, there by obtaining 5-bromo-2-(methoxymethyl)-1-methyl-4-nitro-6-(trifluoromethyl)-1H-benzo[d]imidazole.

### Step 6:

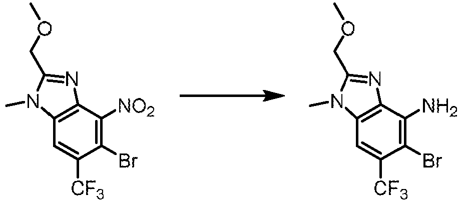

A solution of 5-bromo-2-(methoxymethyl)-1-methyl-4-nitro-6-(trifluoromethyl)-1H-benzo[d]imidazole (3.09 g) obtained in step 5, iron powder (2.34 g), and ammonium chloride (2.24 g) in ethanol (42 mL), THF (18 mL) and water (12 mL) was stirred at 60 °C for 2 hours. The insoluble matter was filtered off, followed by evaporating the solvent under reduced pressure. After dilution with ethyl acetate, the mixture was washed with water and a saturated sodium chloride solution, followed by drying over sodium sulfate. After evaporation, the obtained residue was purified by column chromatography (hexane:acetone), thereby obtaining 5-bromo-2-(methoxymethyl)-1-methyl-6-(trifluoromethyl)-1H-benzo[d]imidazol-4-amine (2.50 g).

Other Examples were prepared using the synthetic procedures A to EE and purification notes as shown in the table below. In some instances, the compounds presented in the Examples (including the tables) were isolated and are represented as salts, e.g., trifluoroacetate salts such as Ex. 260. One of ordinary skill in the art will appreciate that, depending on the isolation conditions, the un-ionized form of the compound could be isolated, or a salt form having a counterion other than trifluoroacetate could be isolated. Such un-ionized forms of the compound and alternative salt forms of the compound are within the scope of the present disclosure. Such un-ionized forms of the compound include, without limitation: 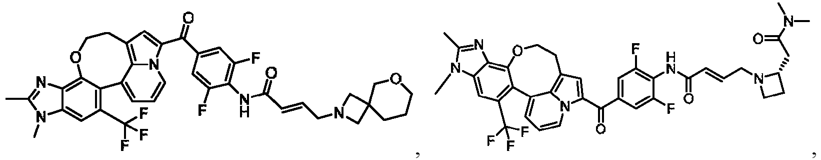 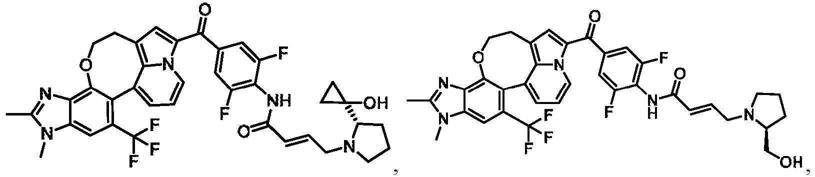 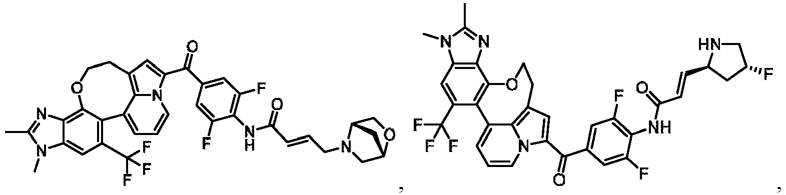 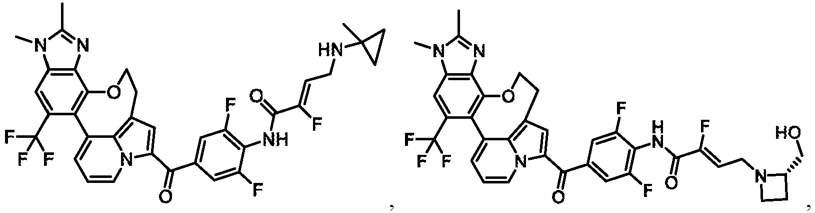 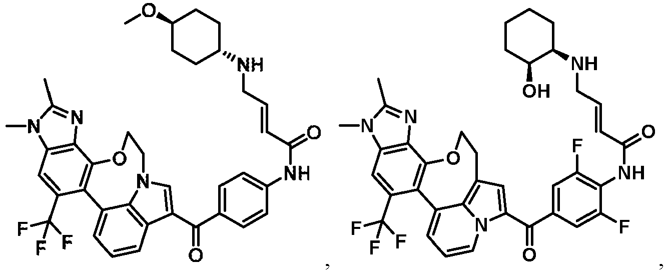 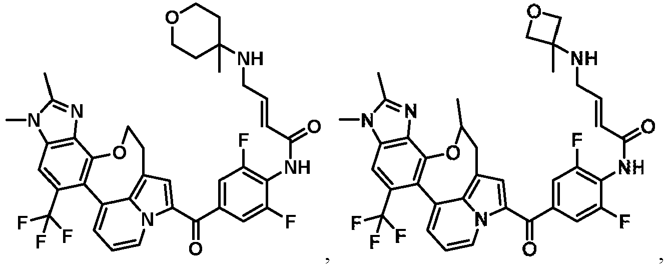 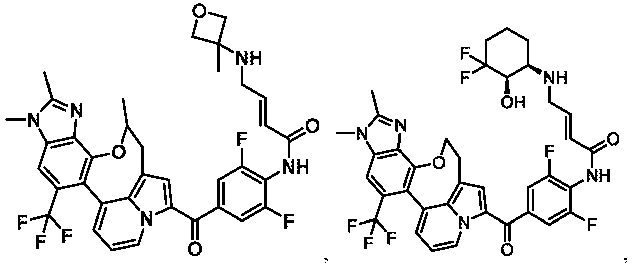 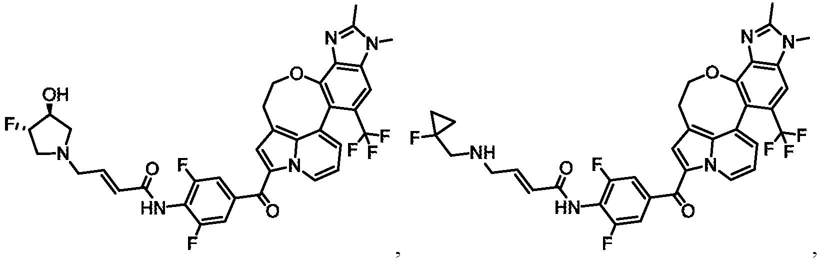 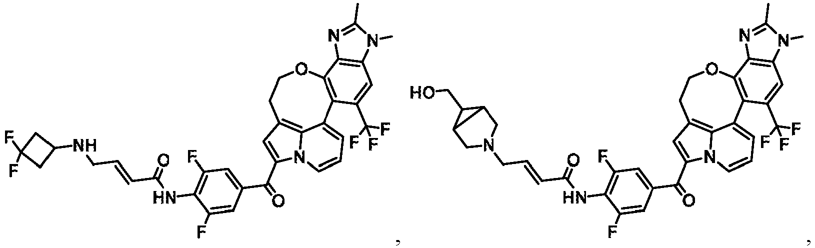 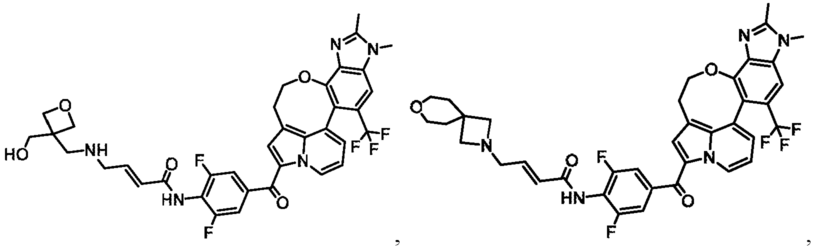 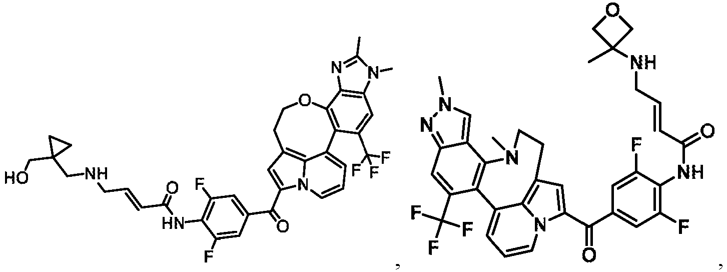 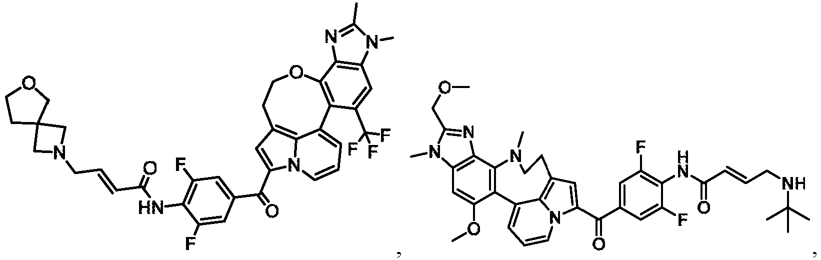 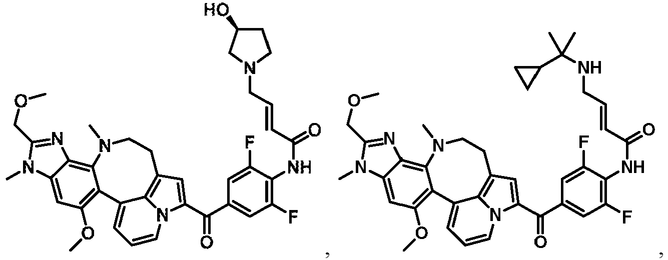 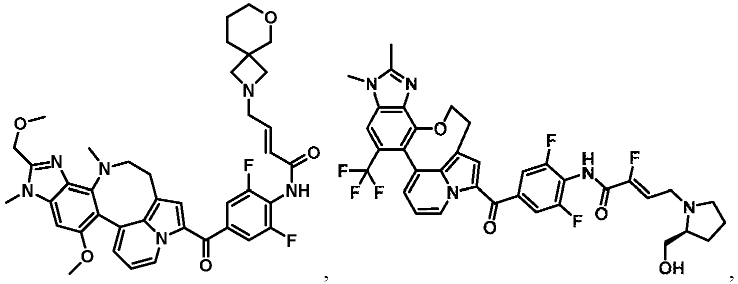 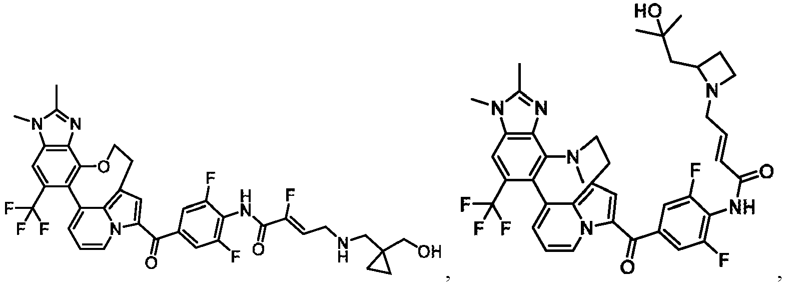 and

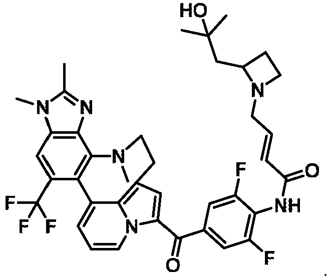

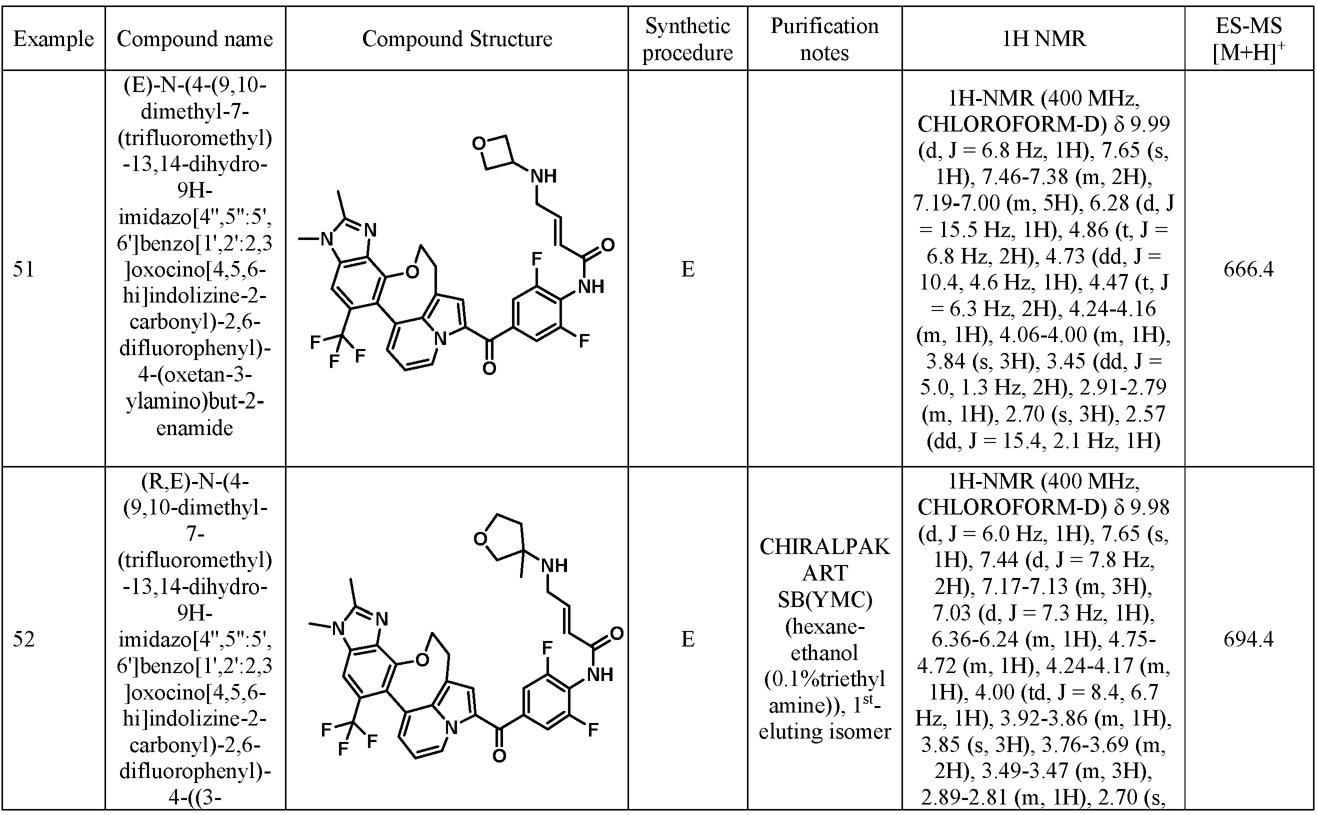

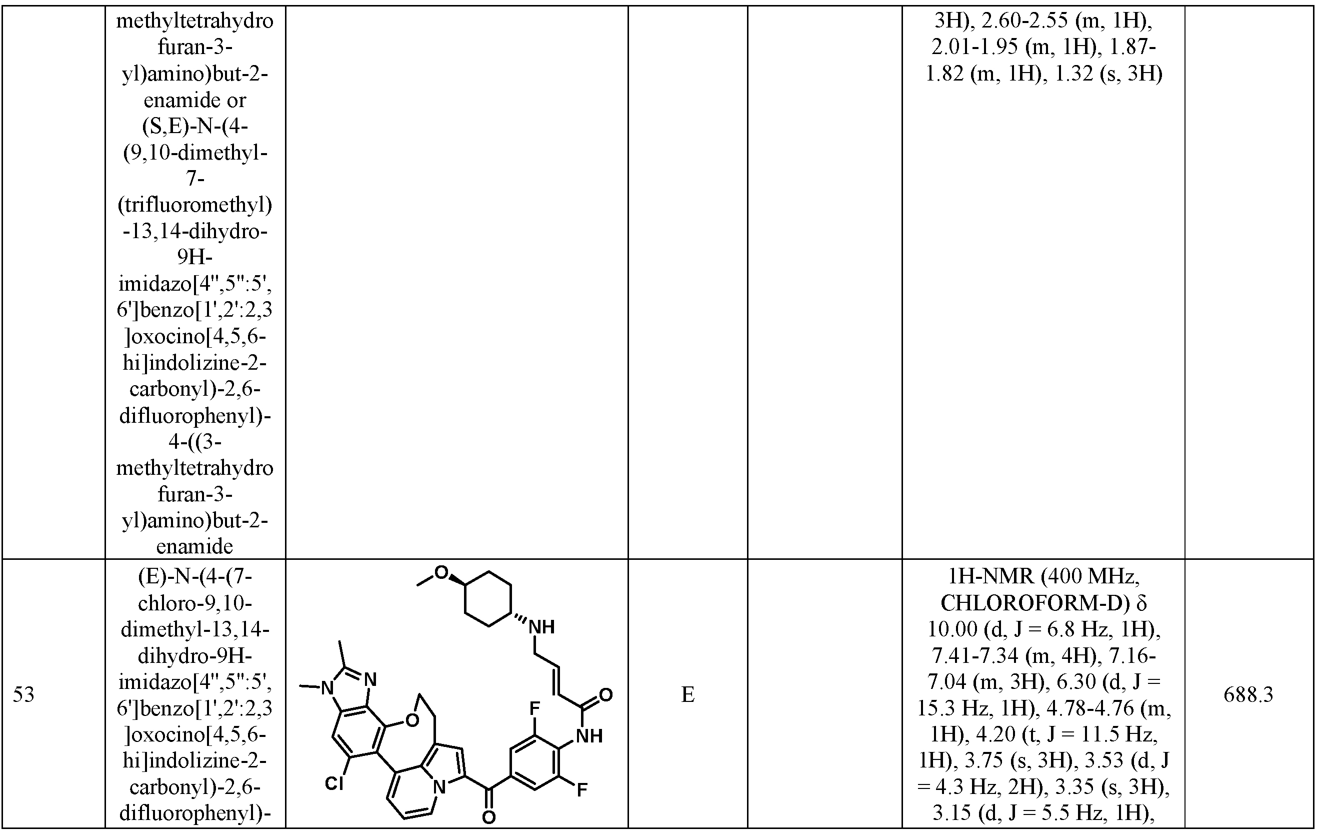

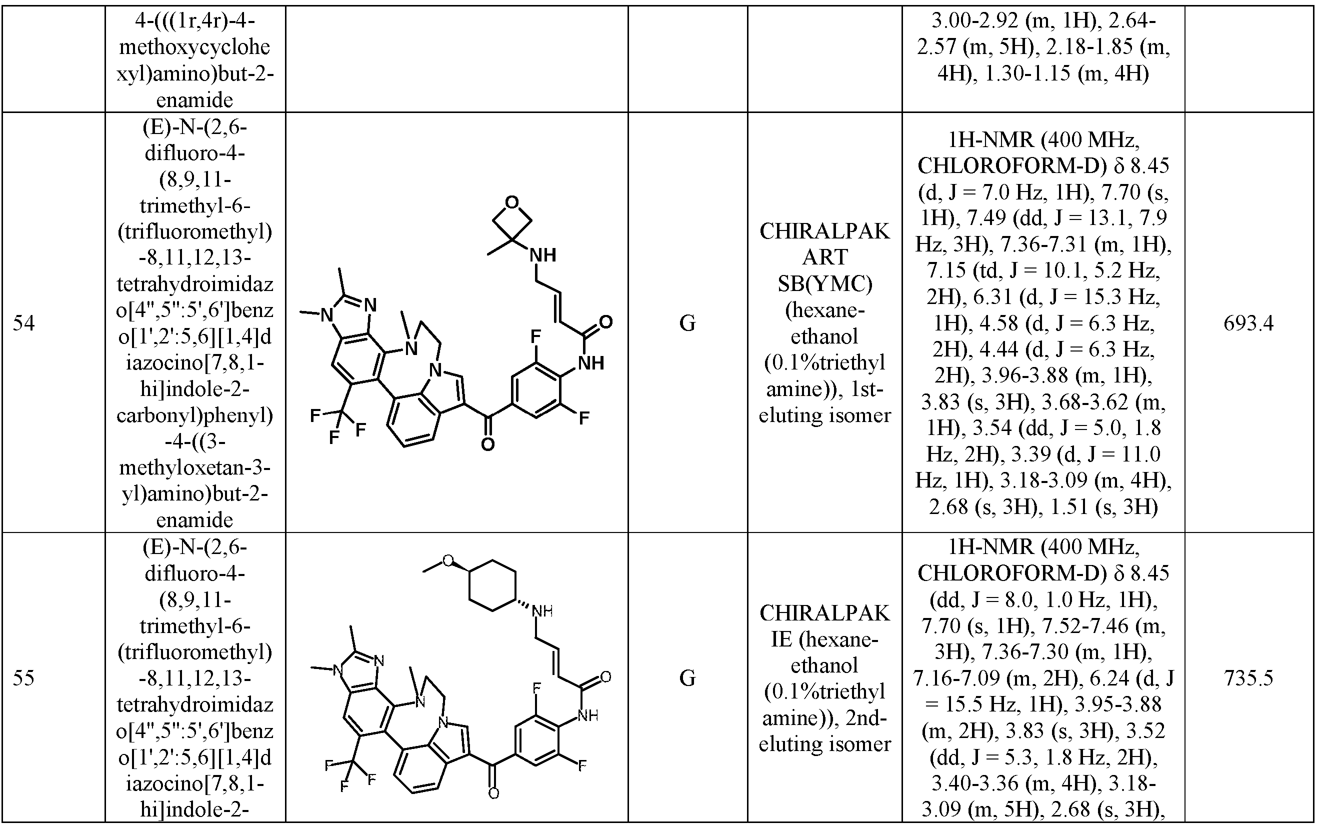

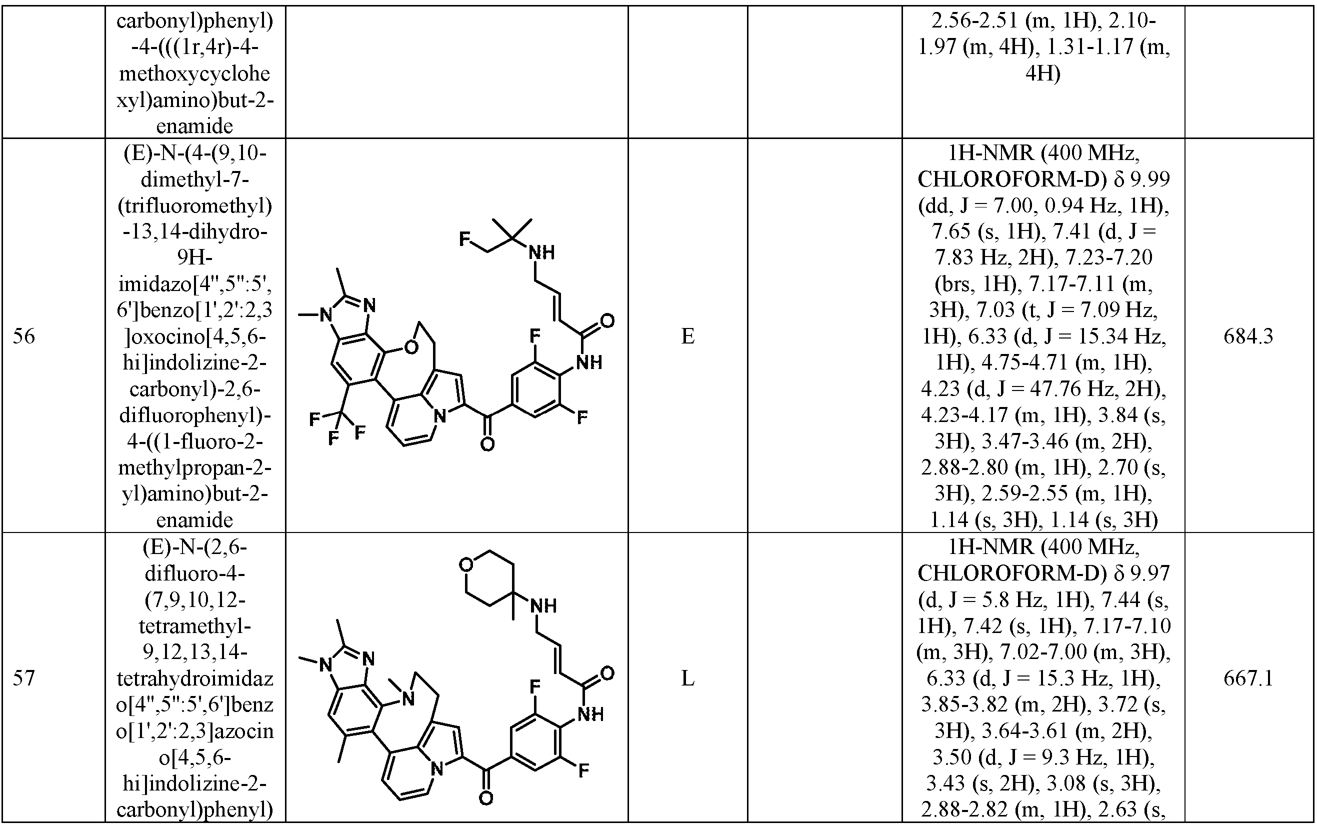

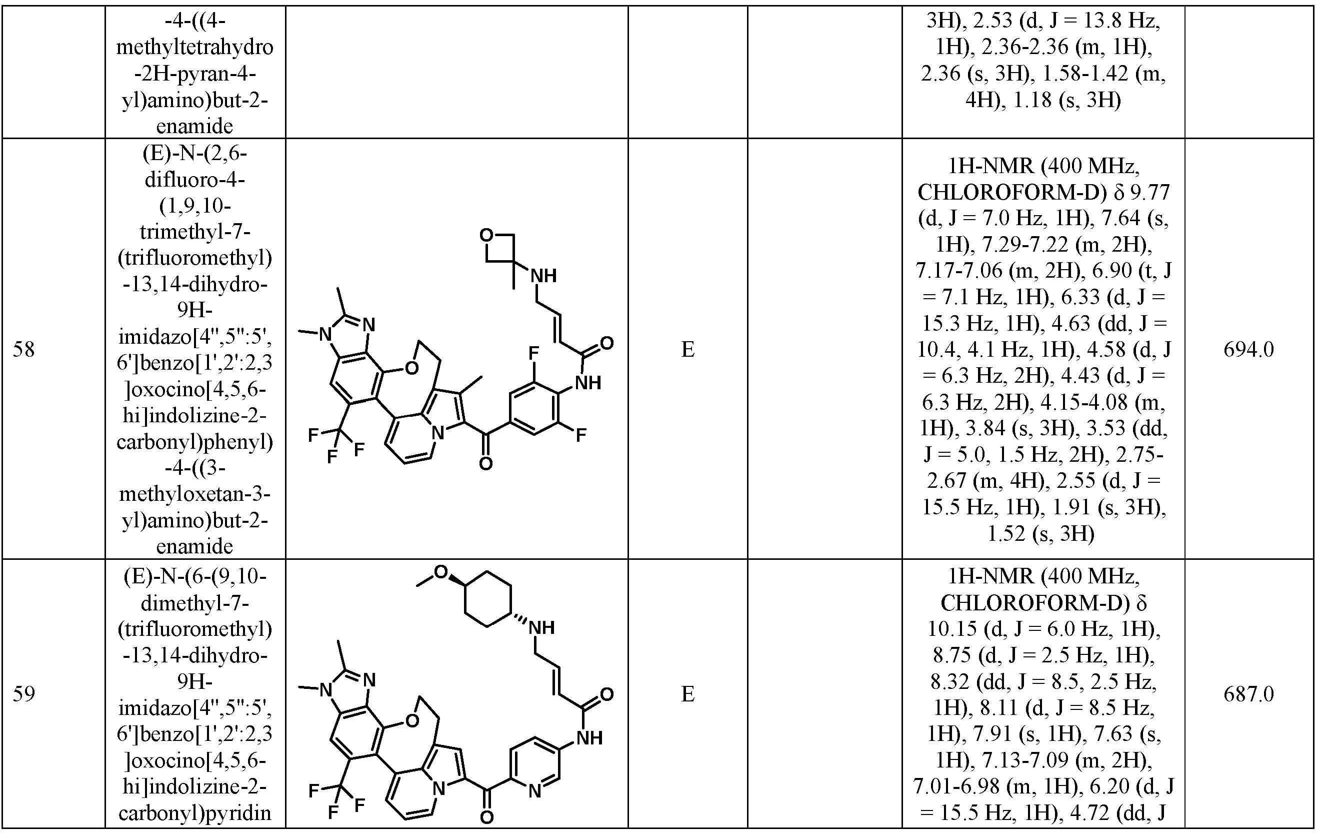

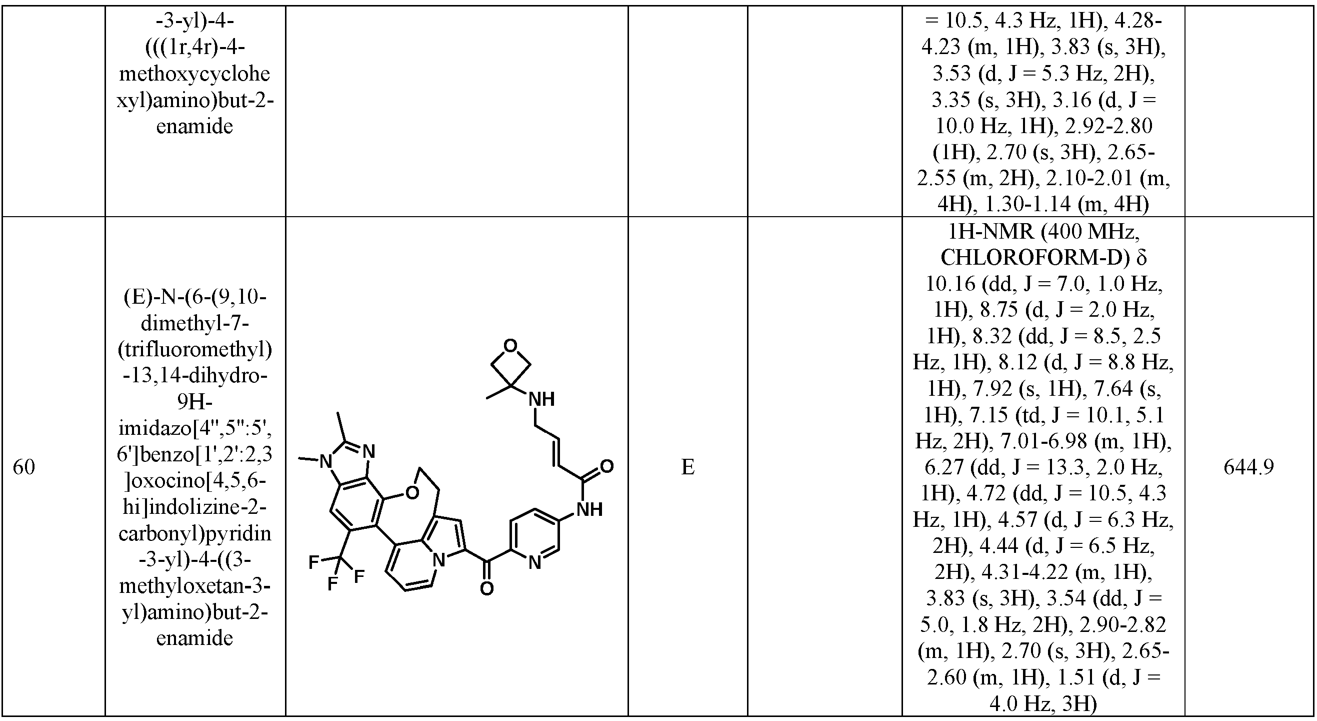

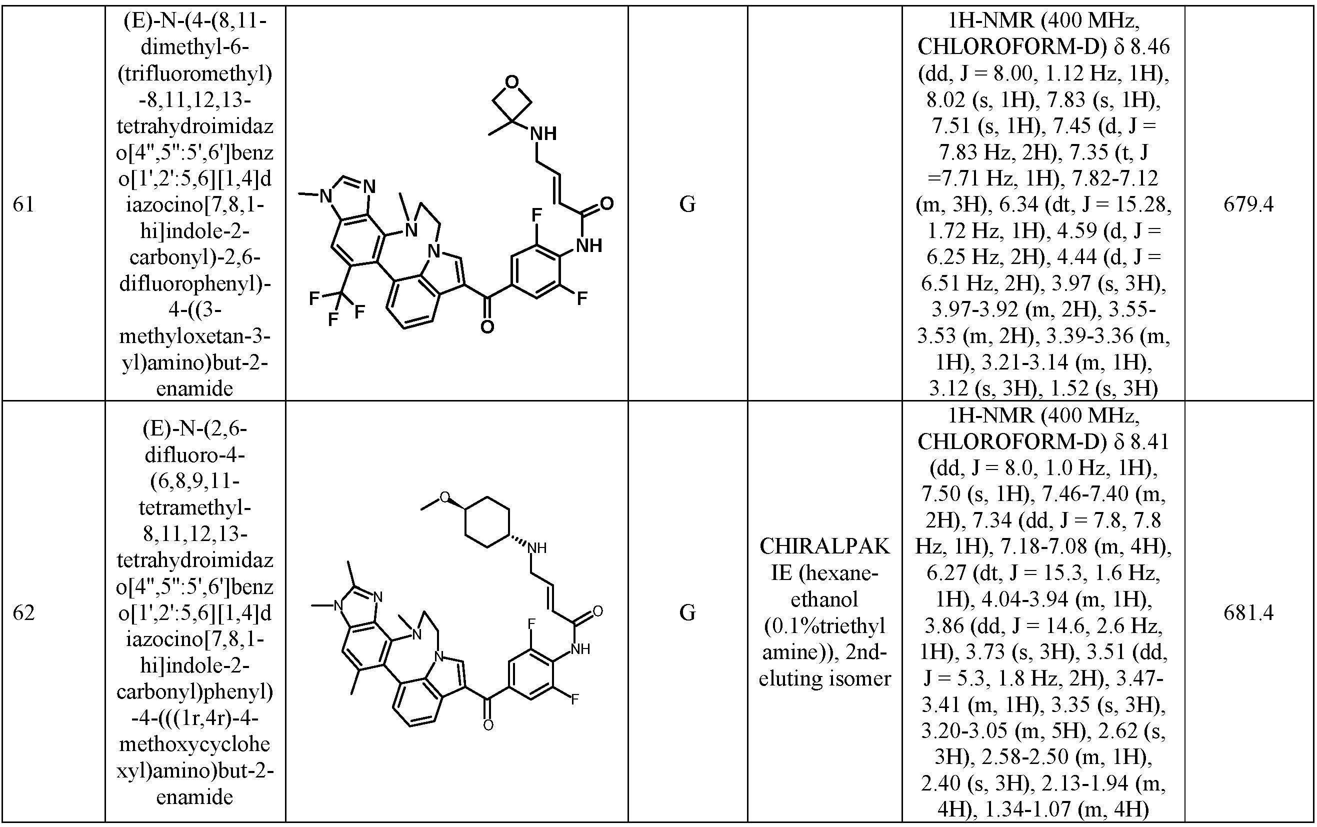

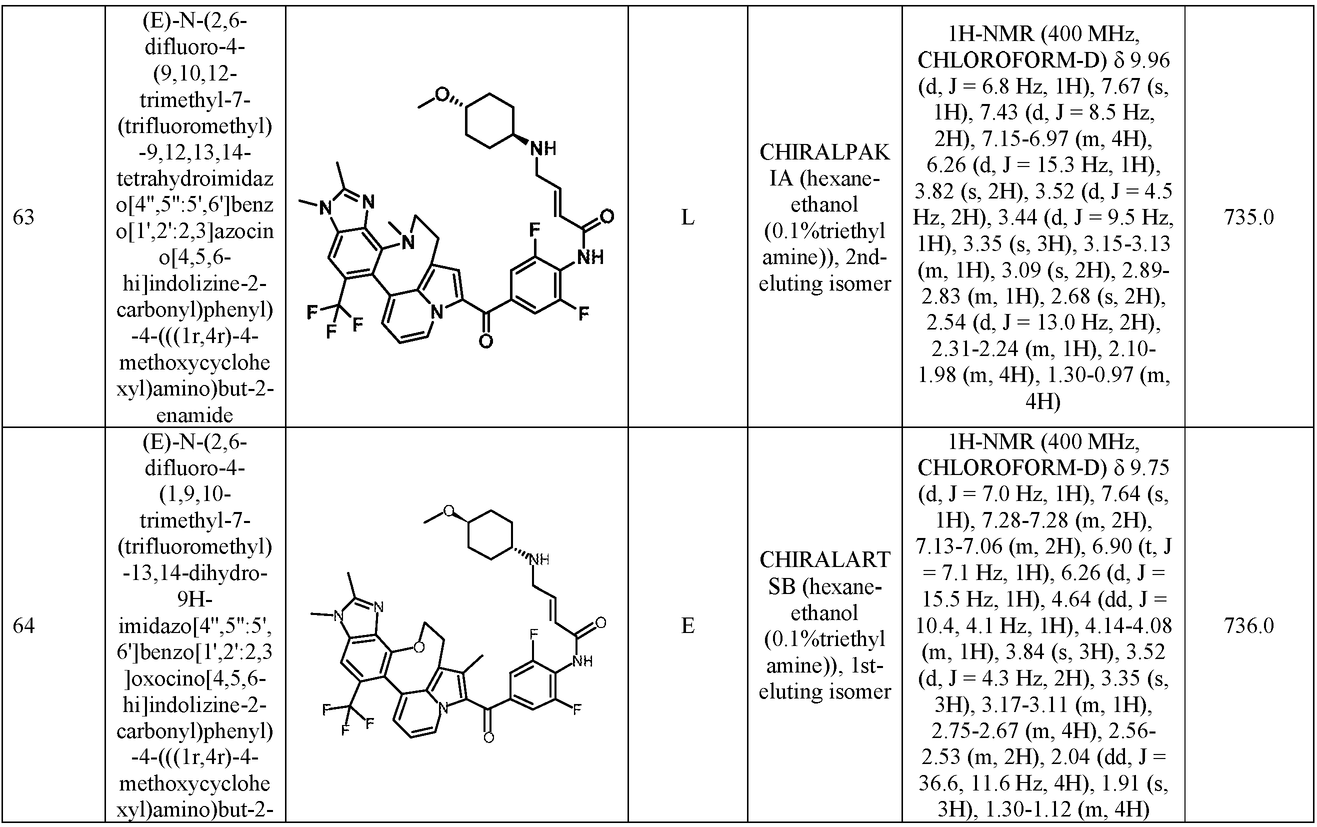

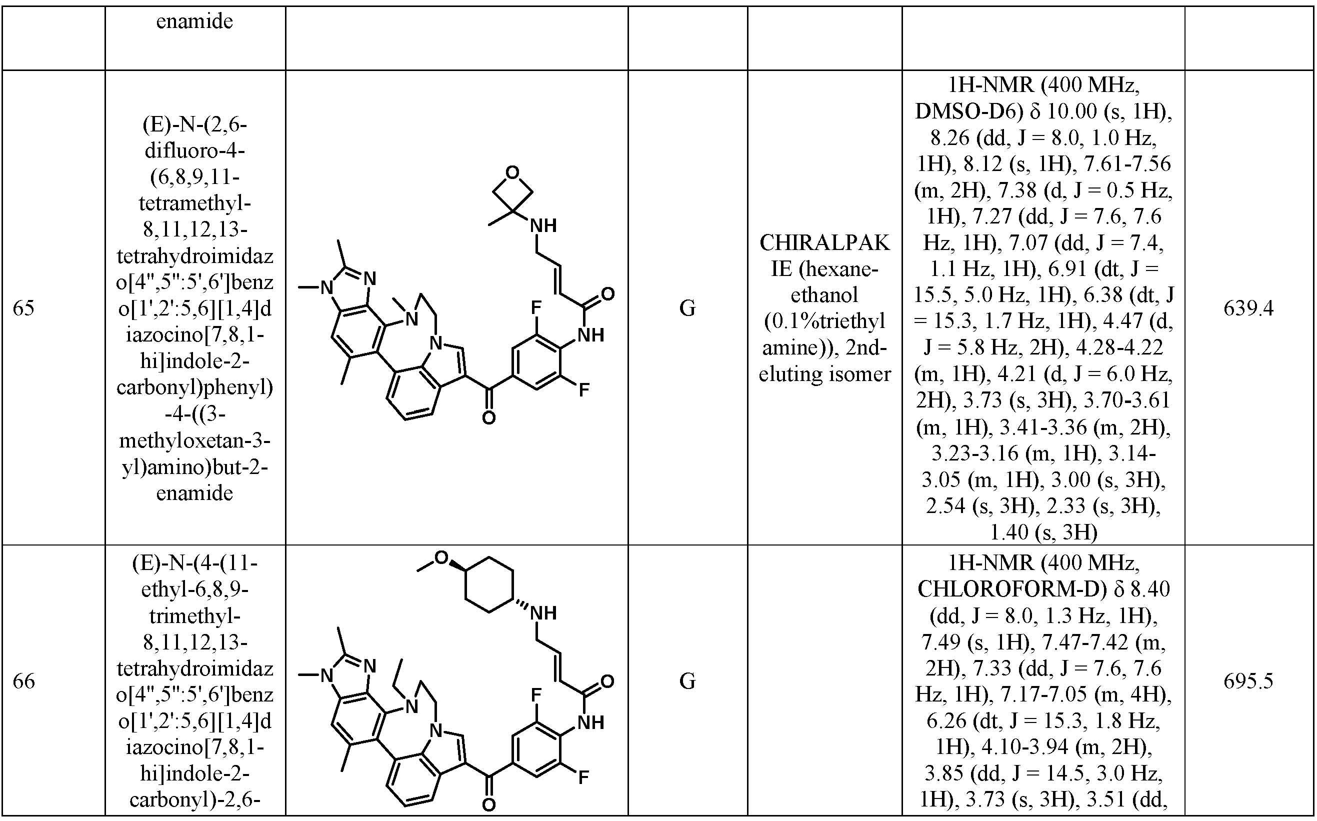

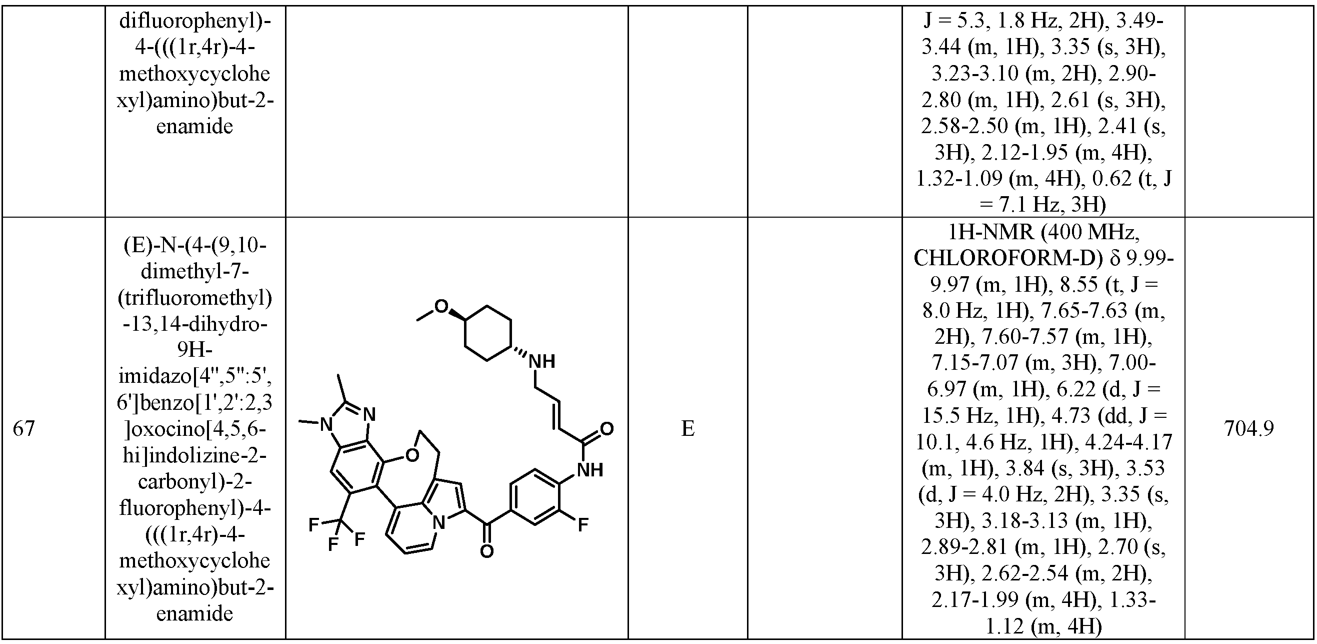

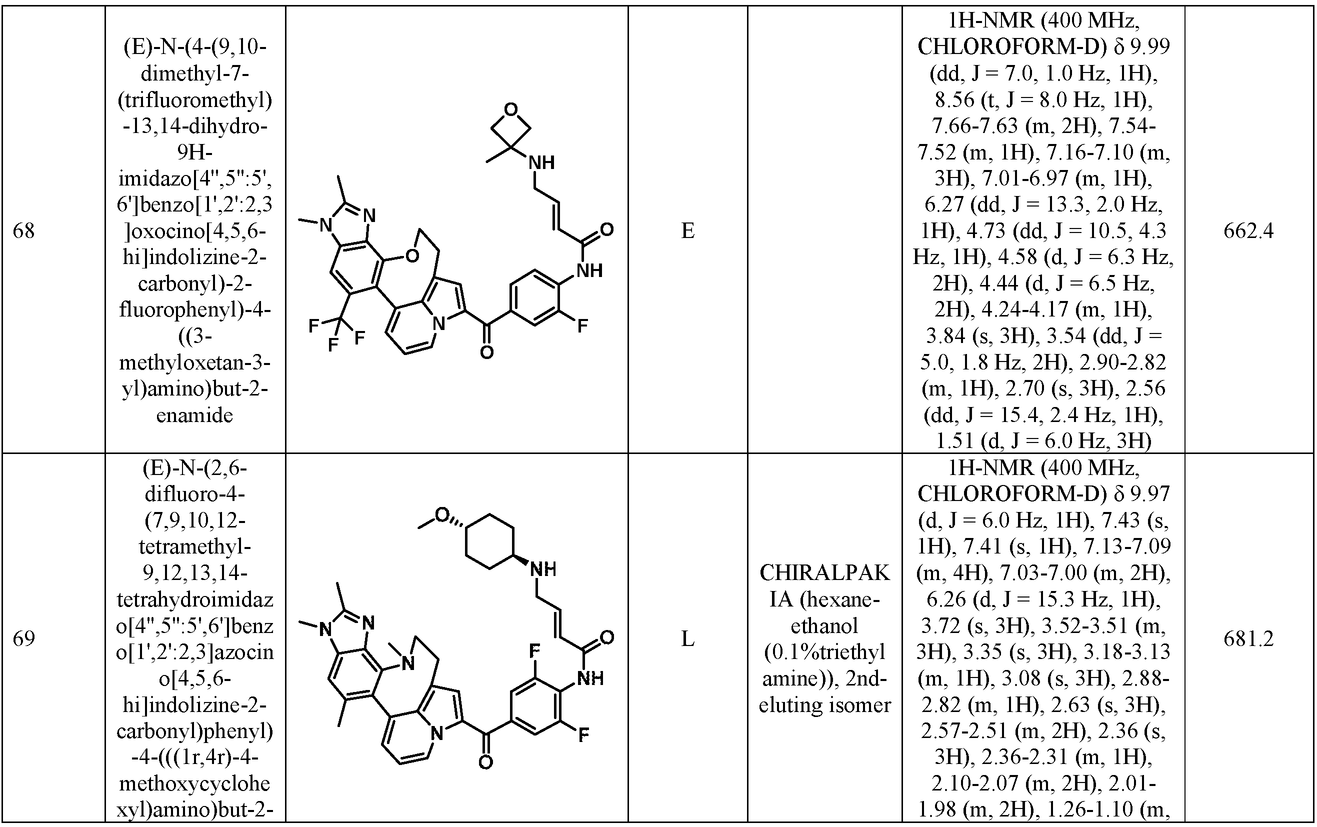

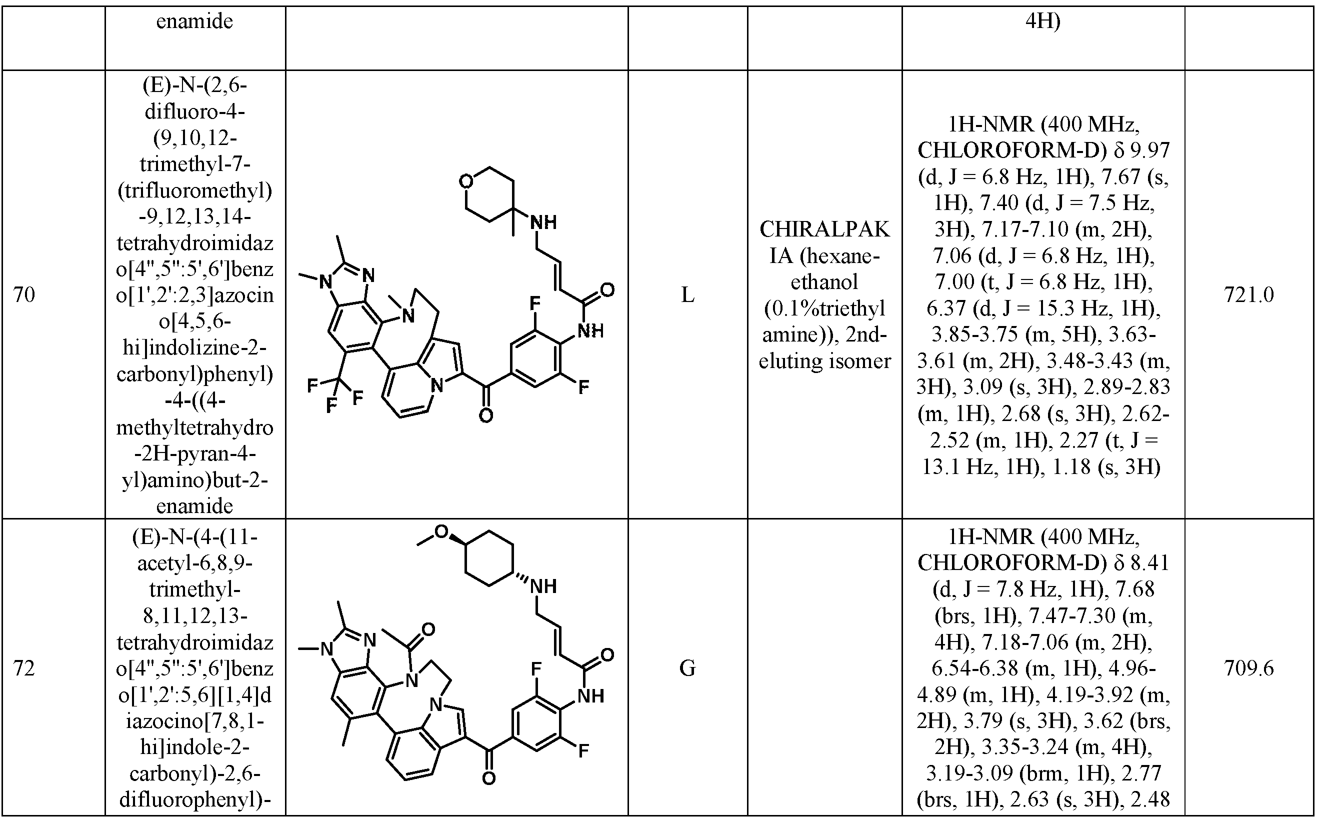

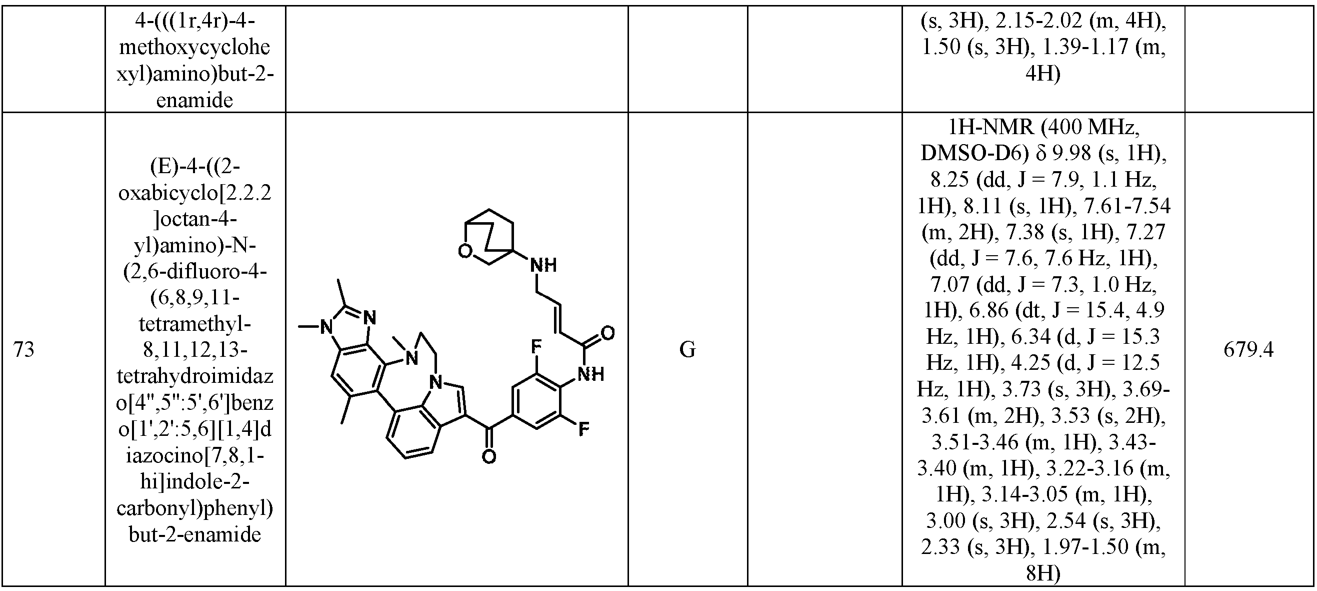

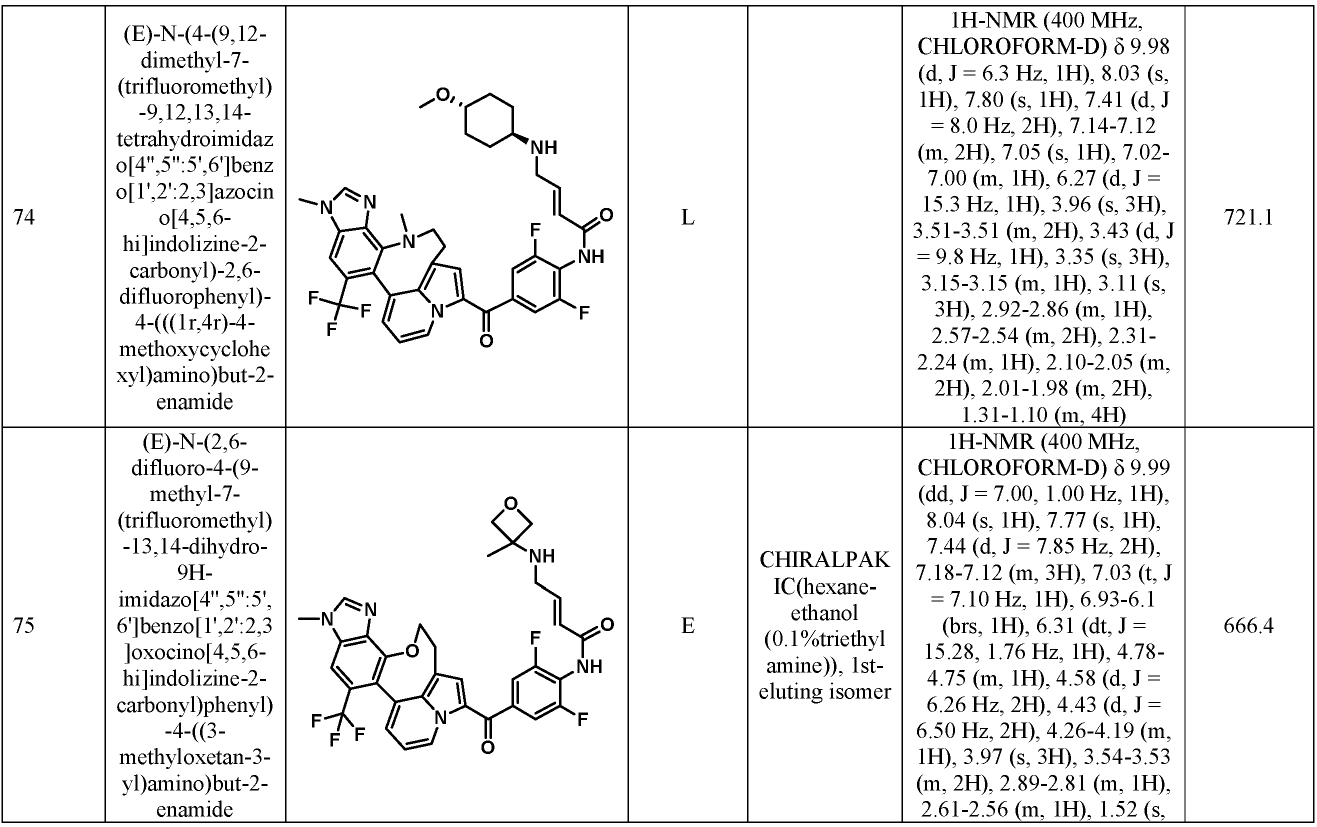

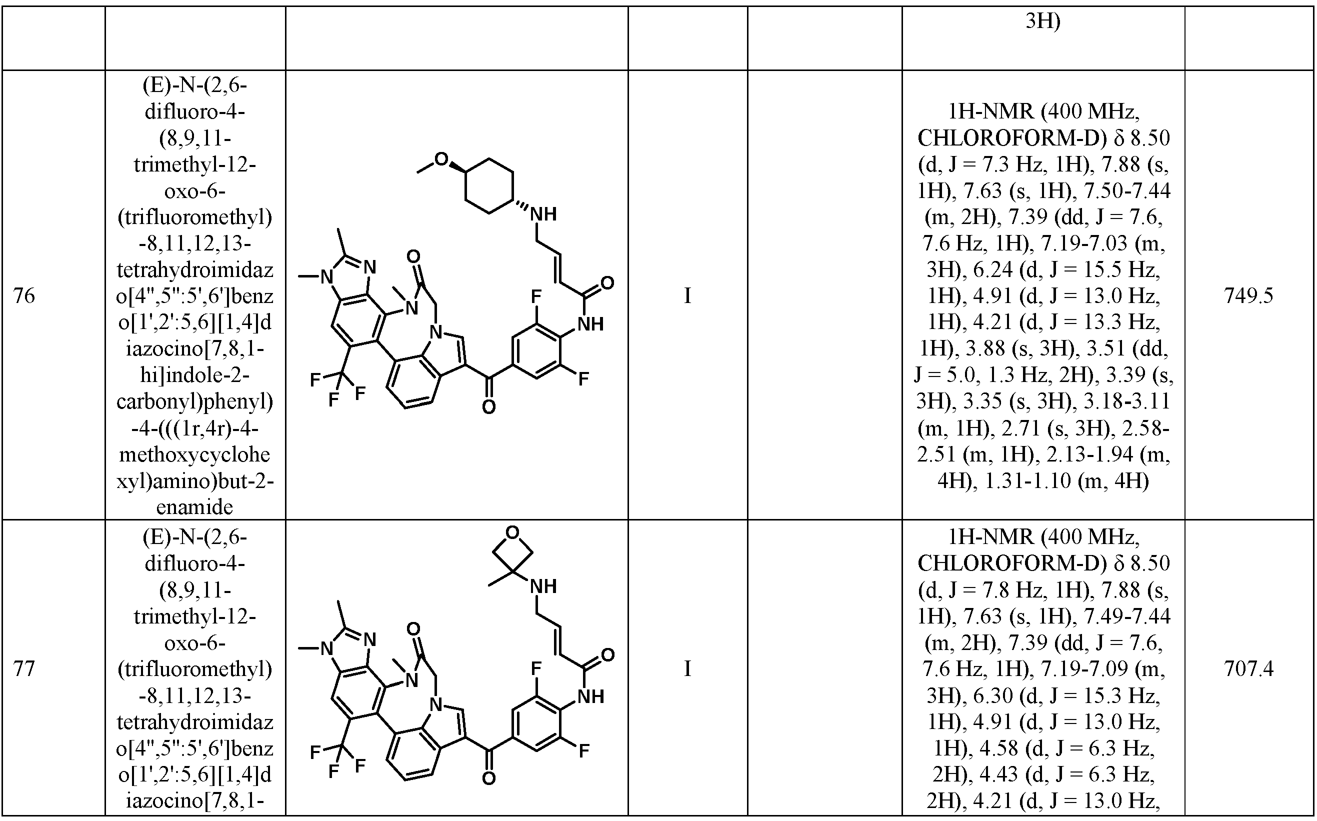

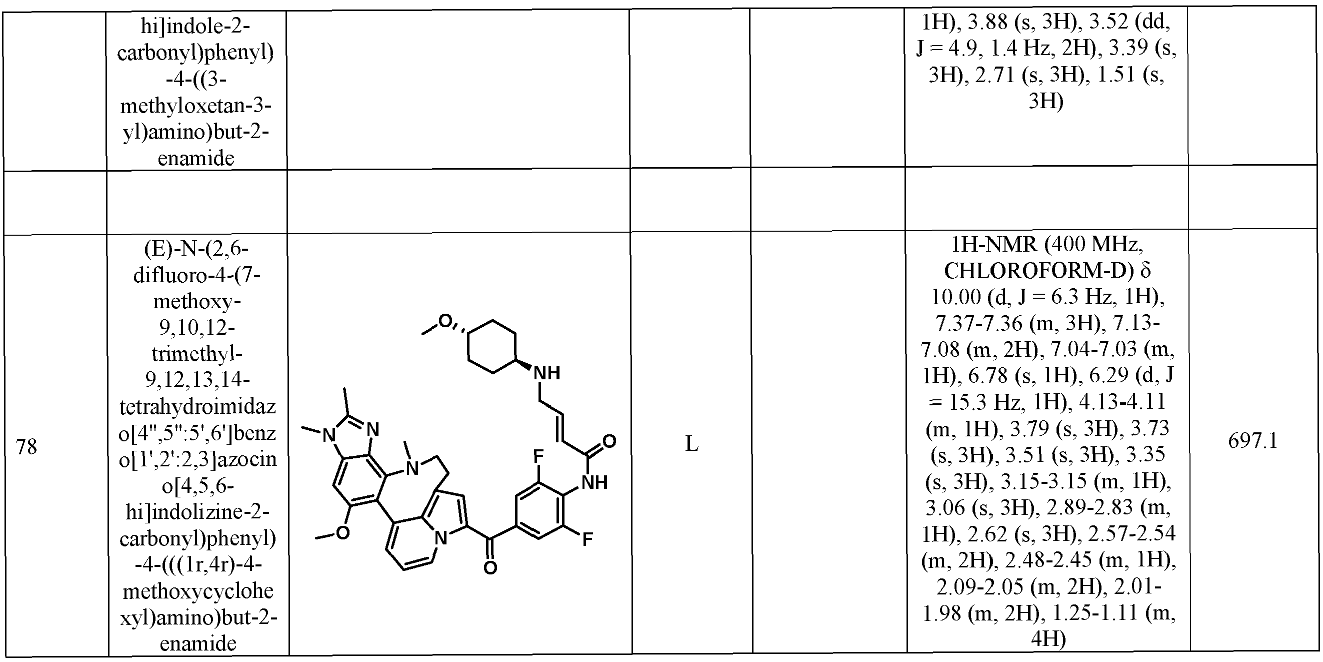

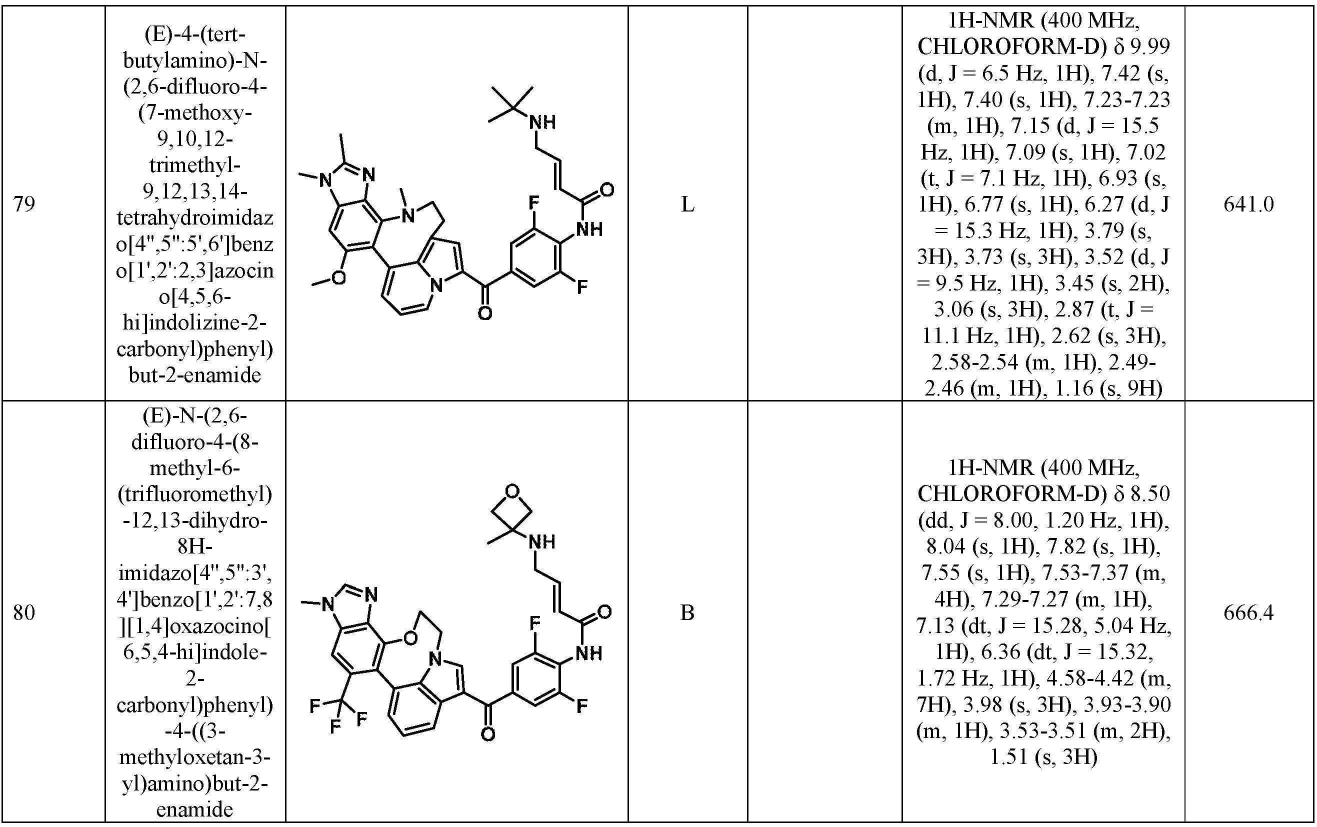

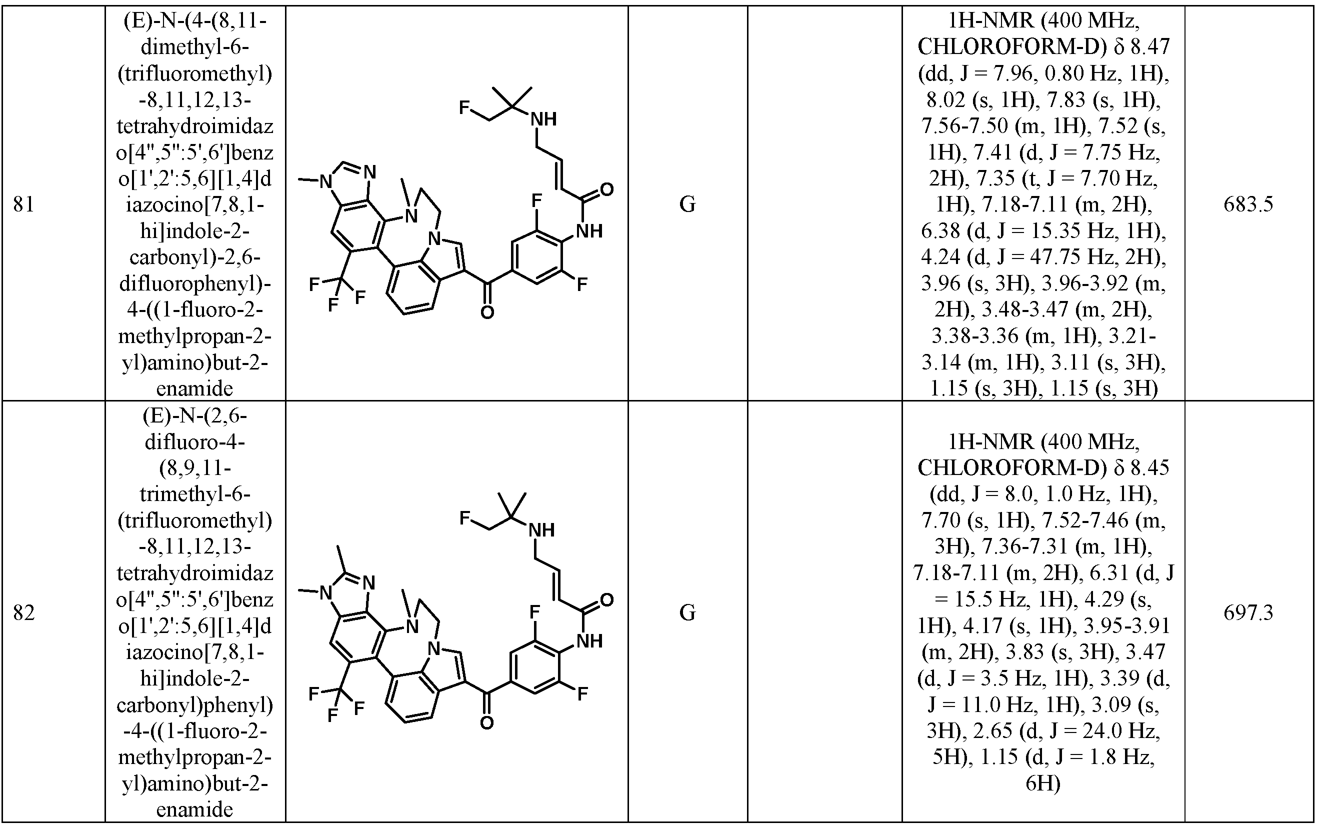

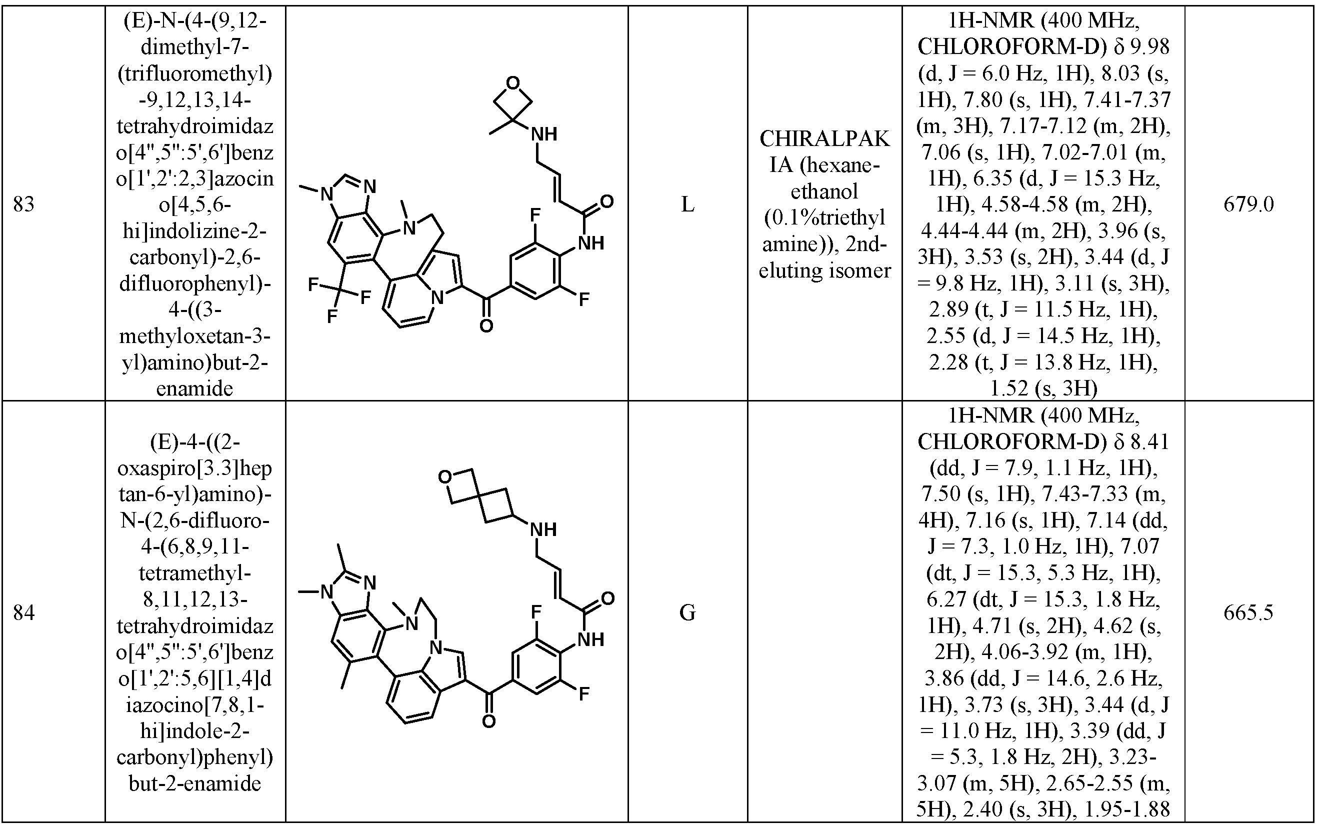

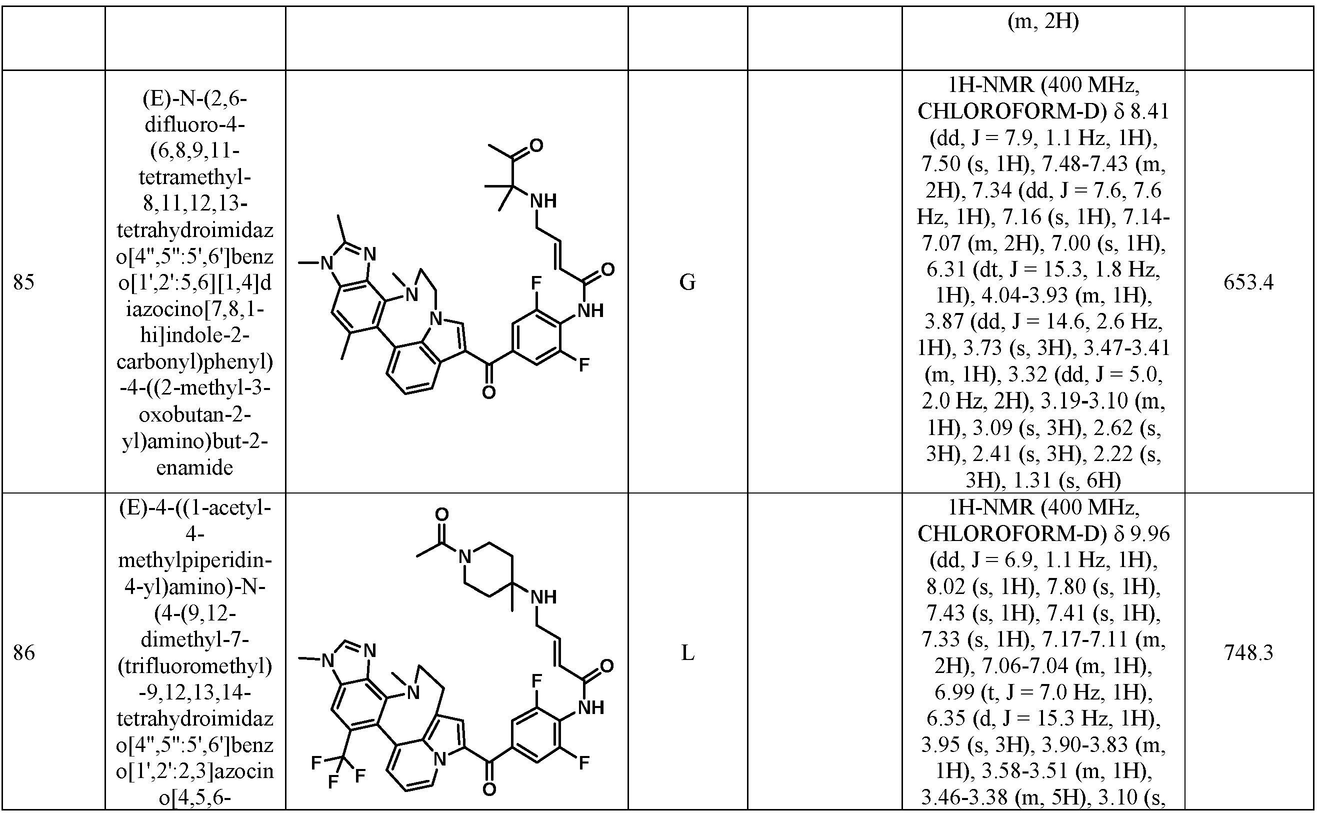

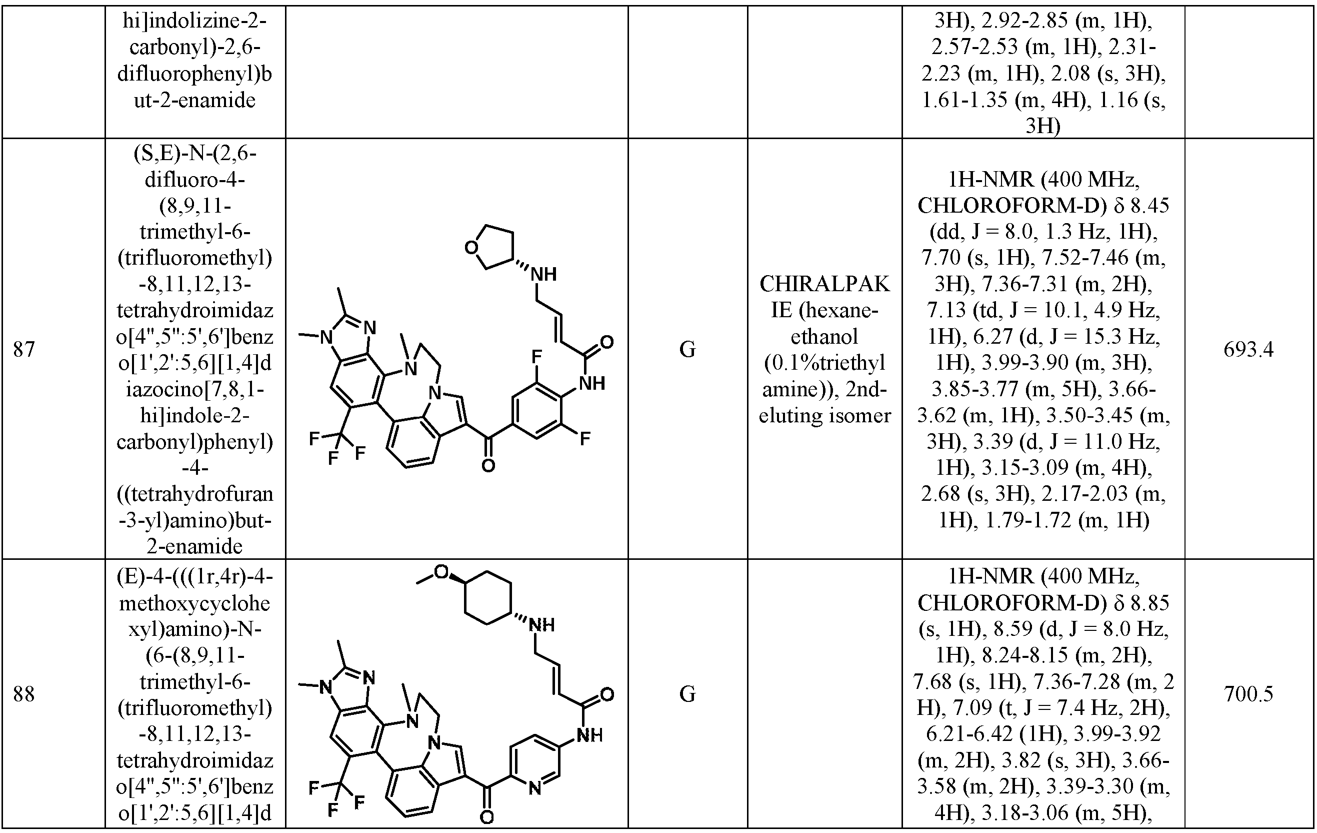

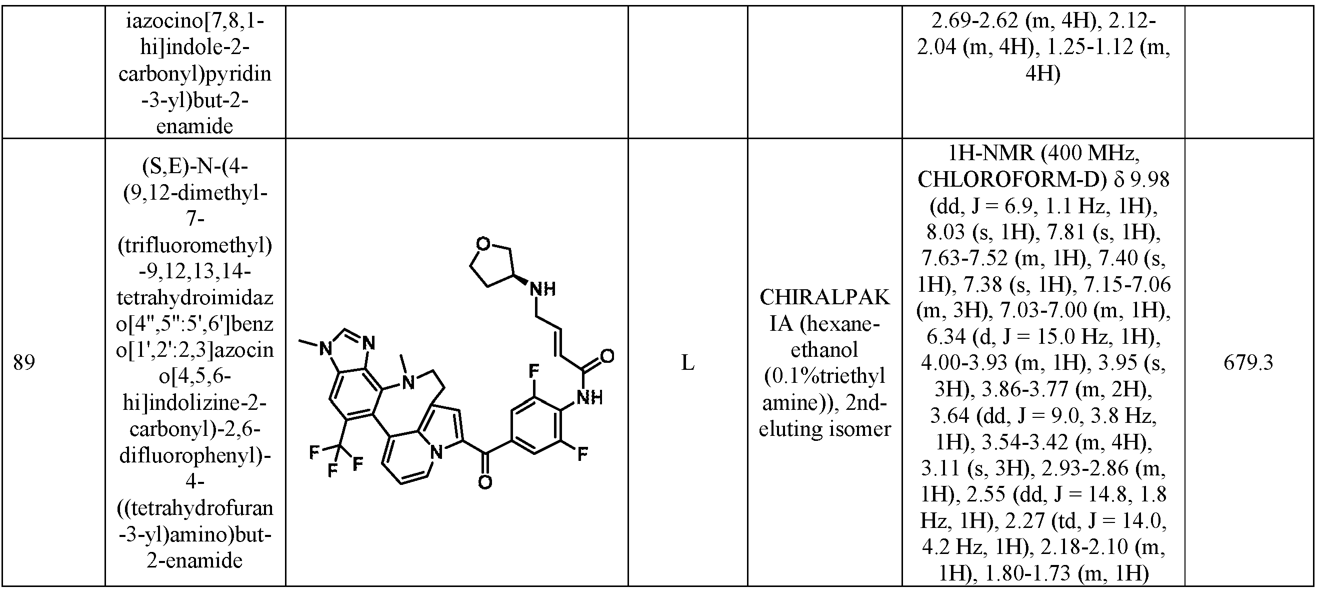

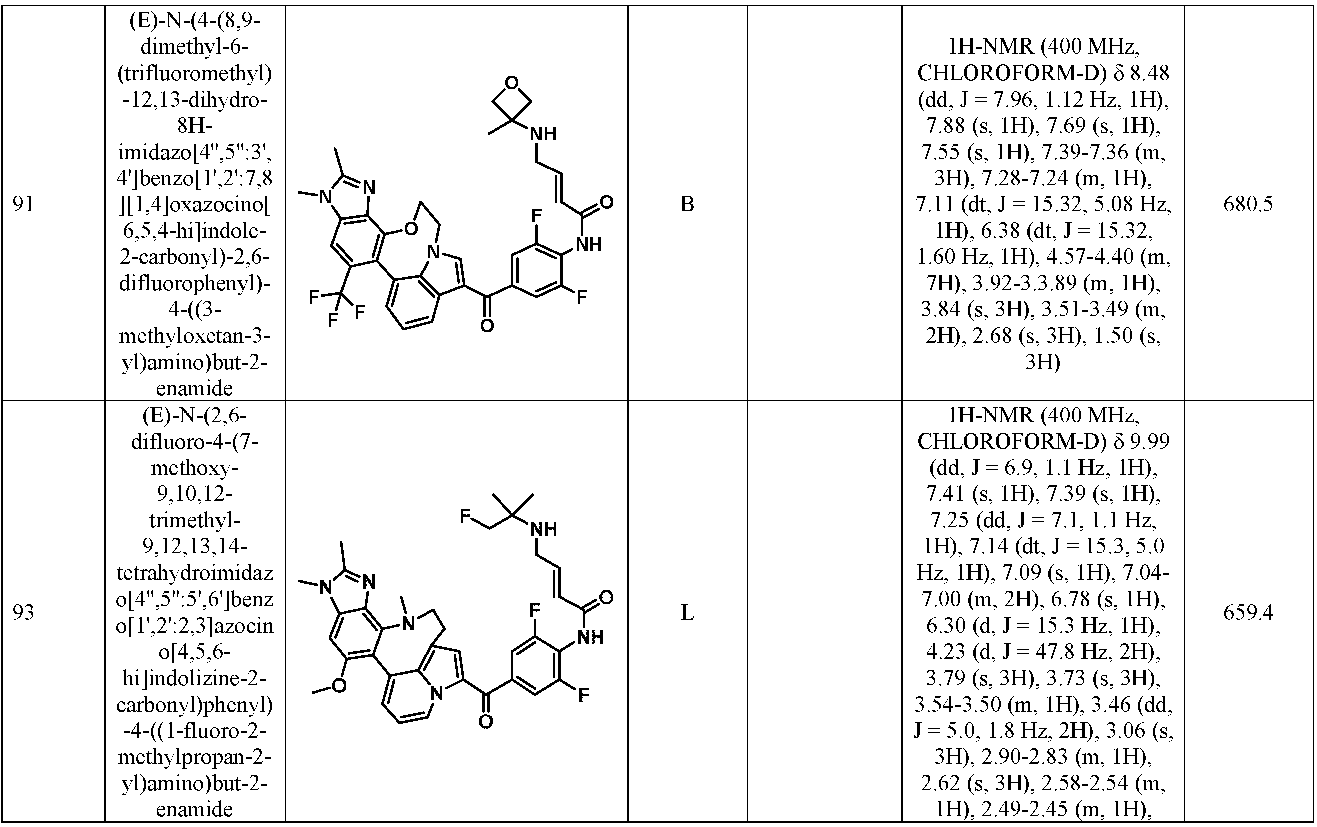

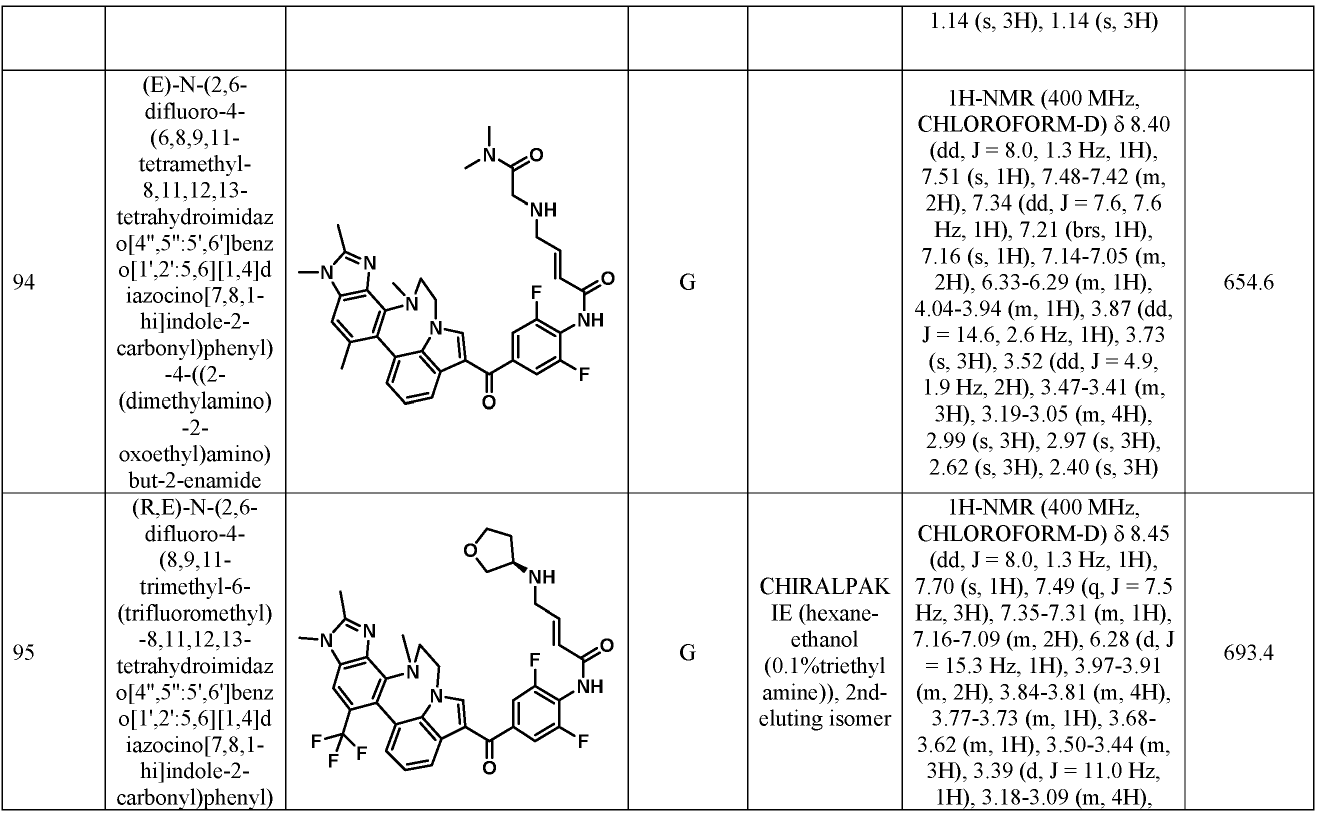

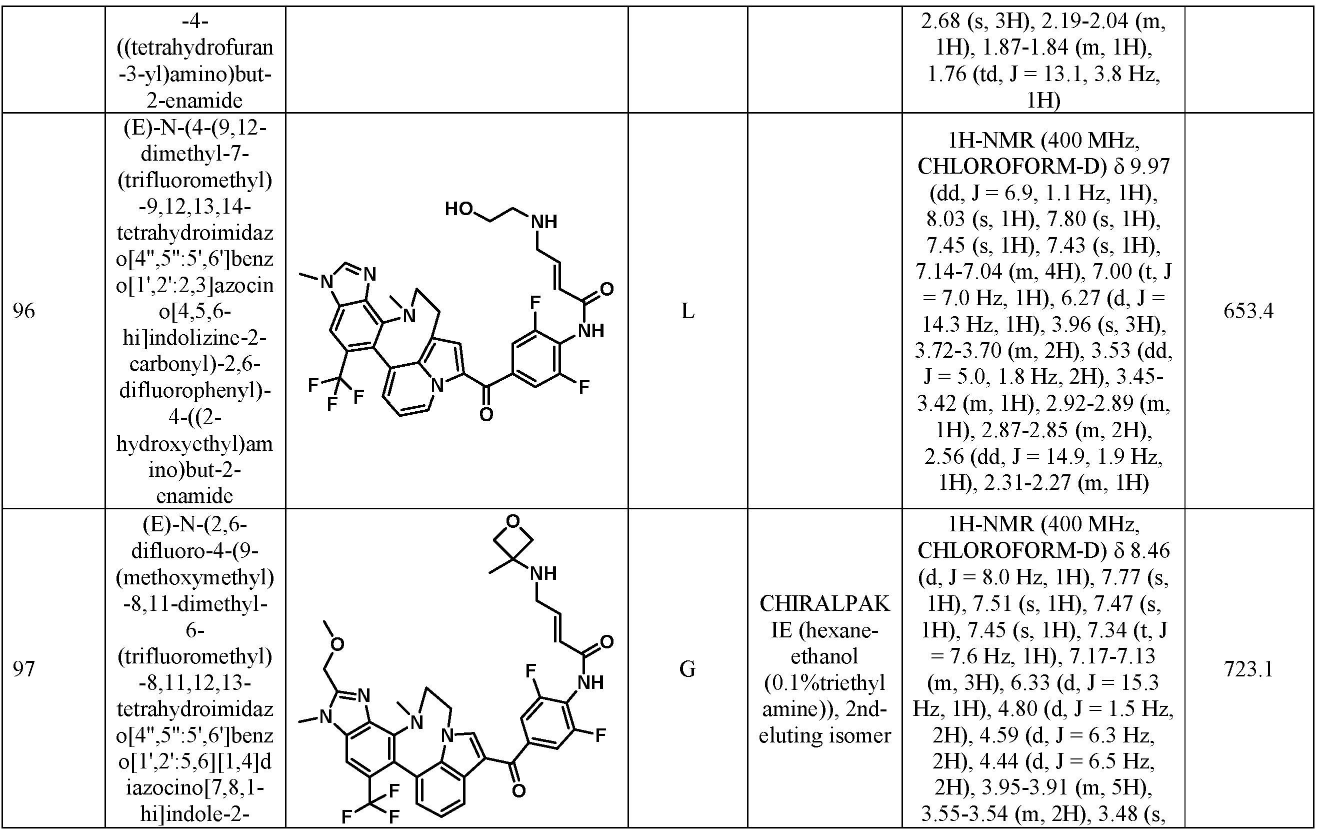

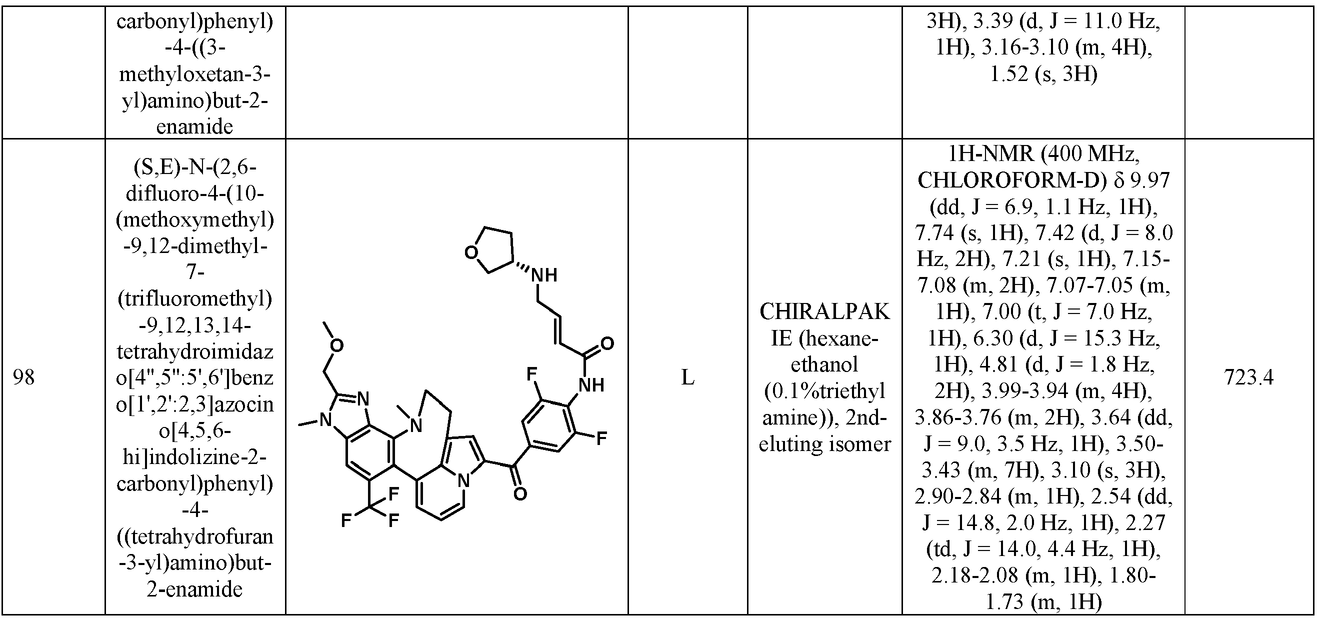

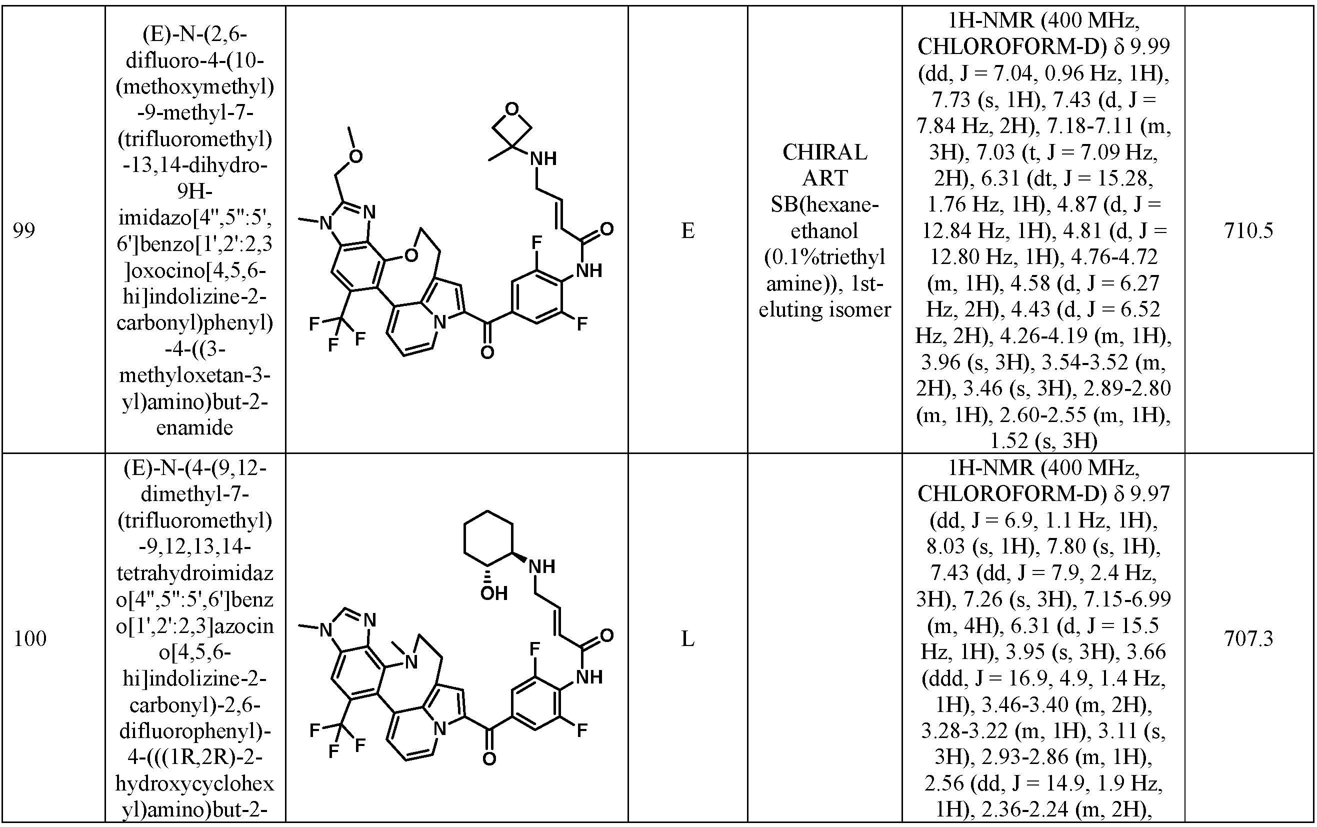

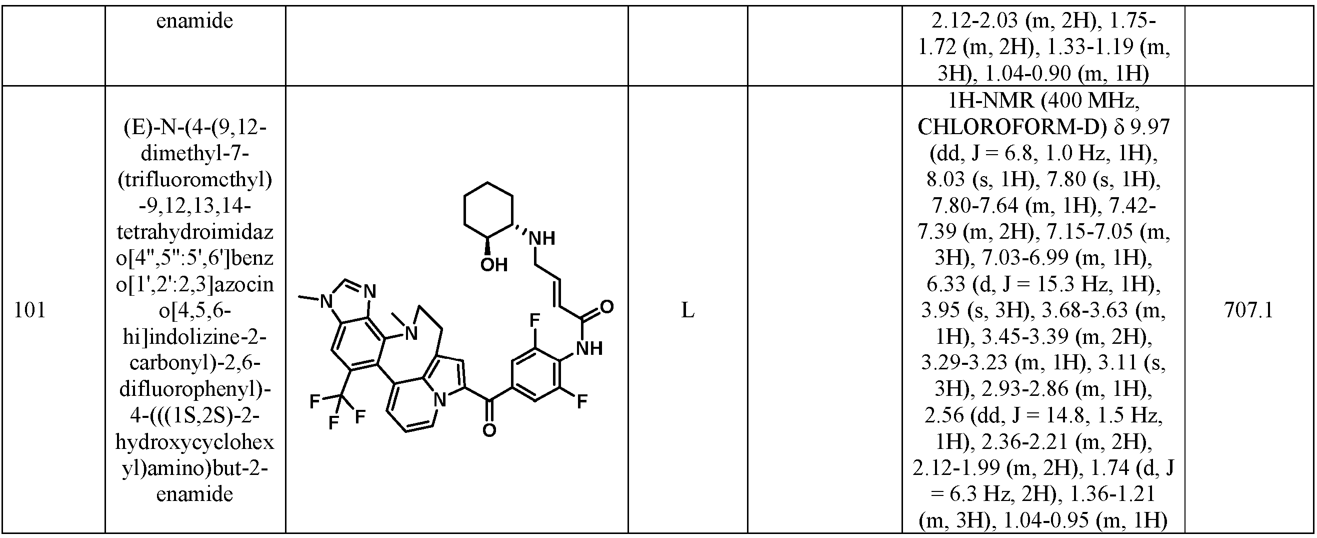

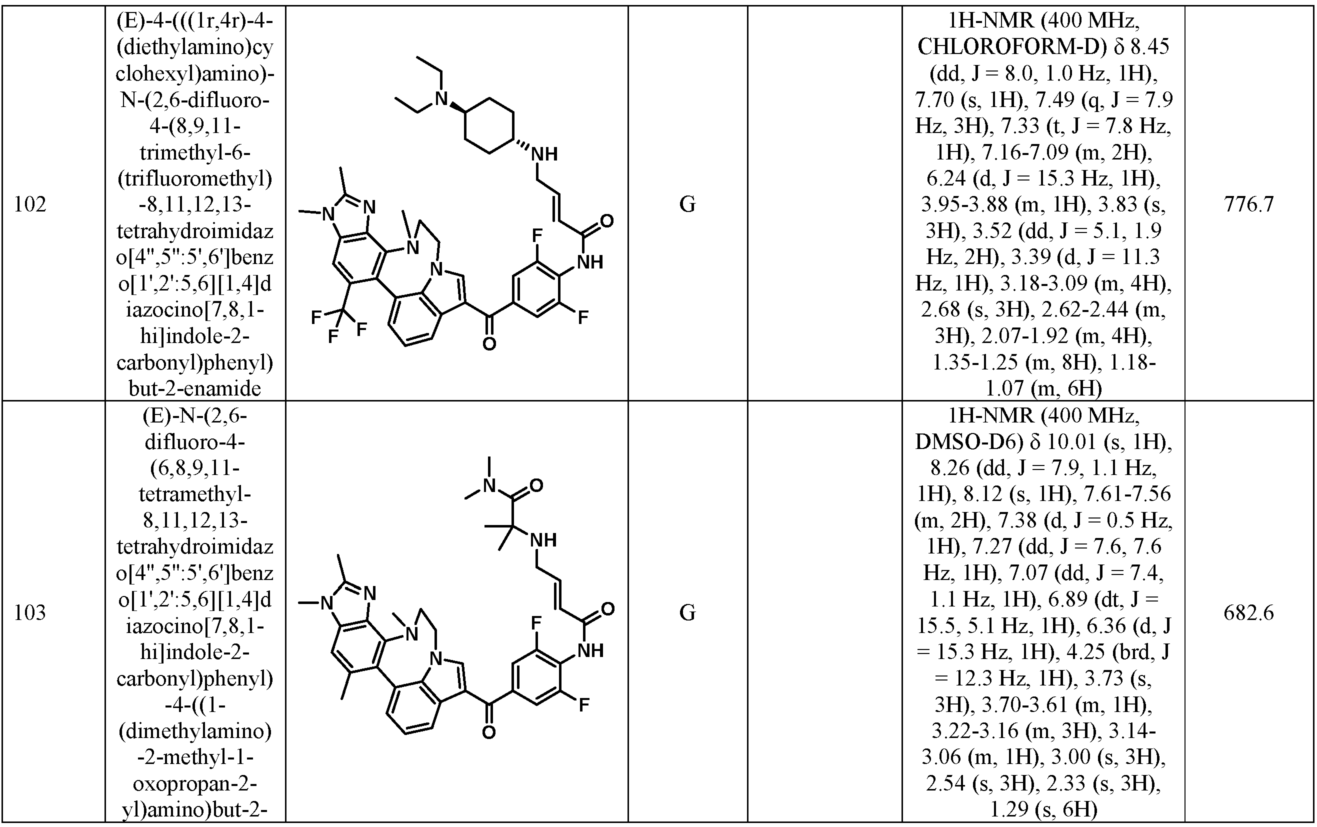

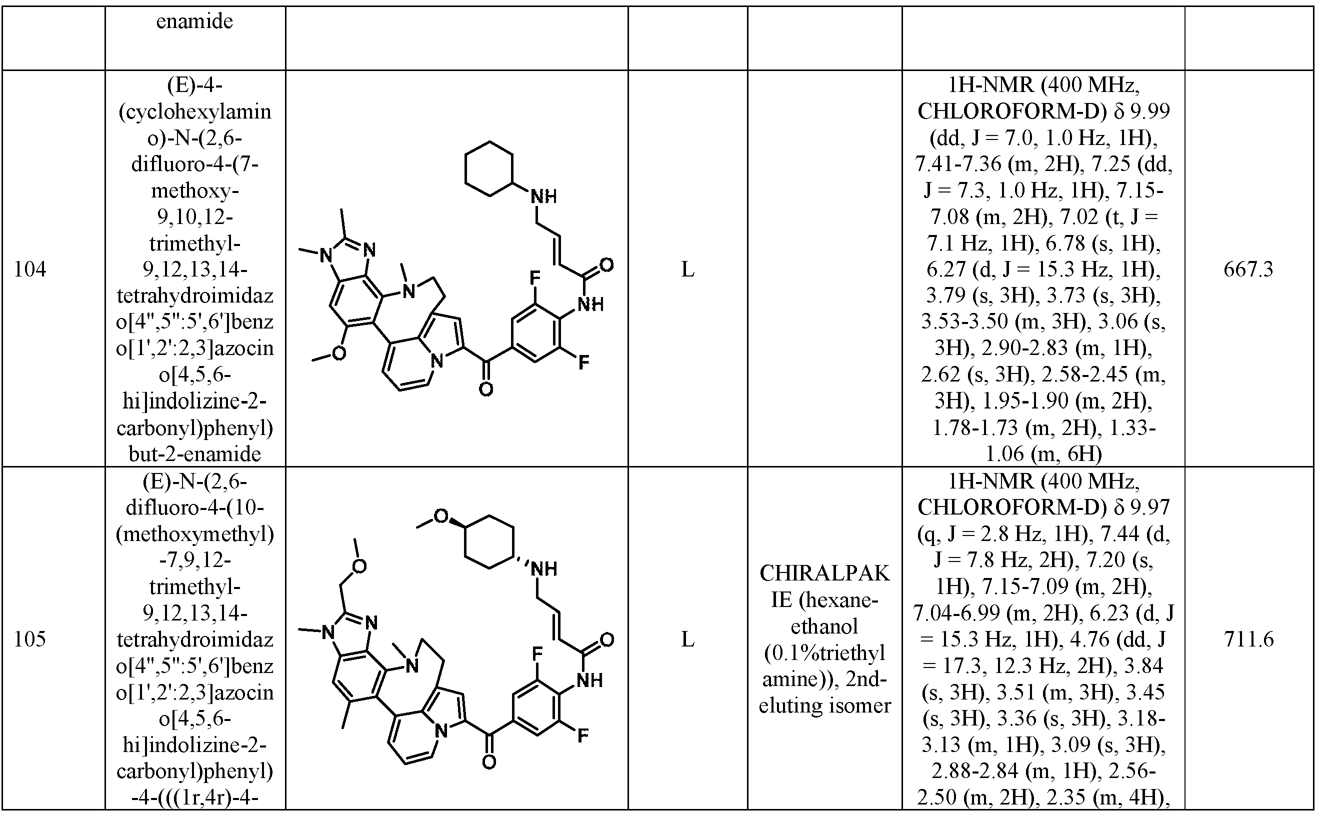

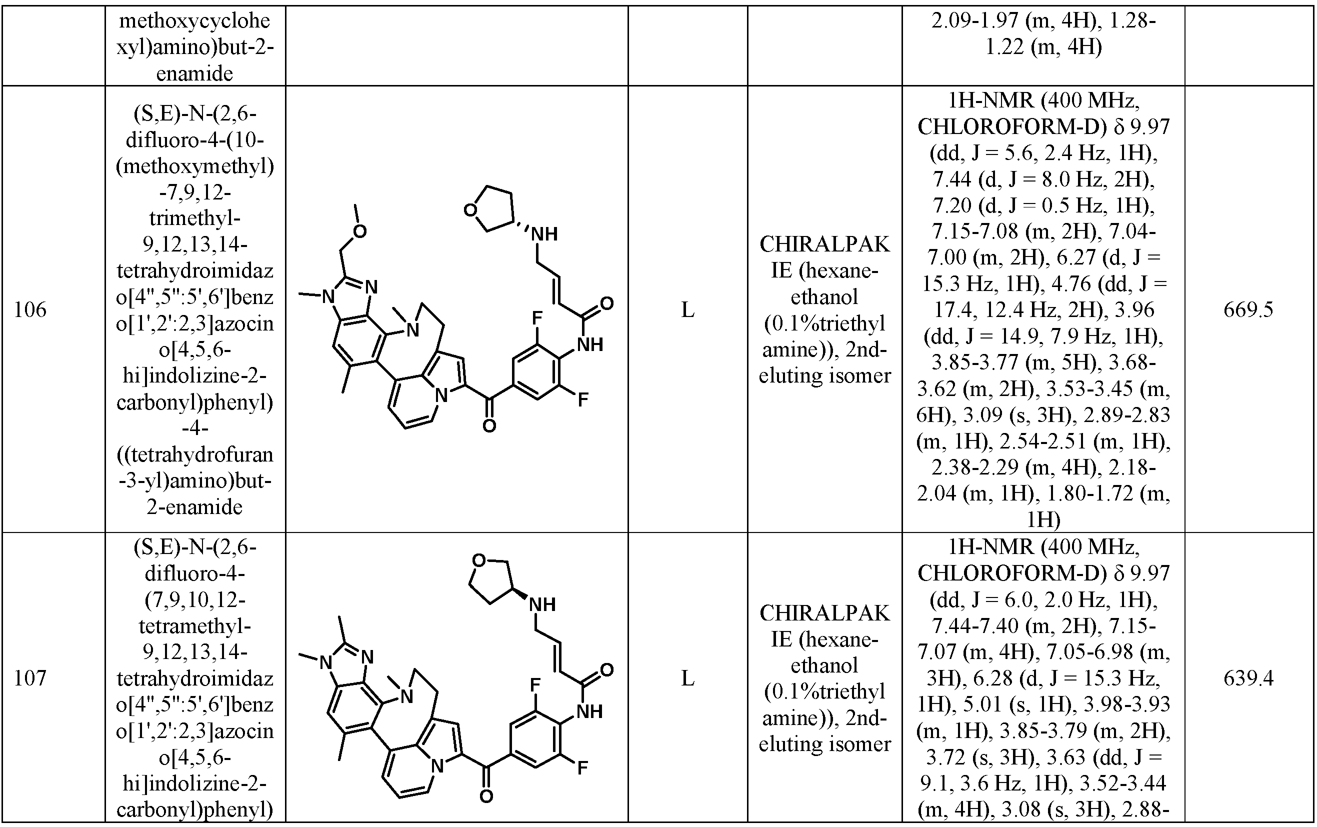

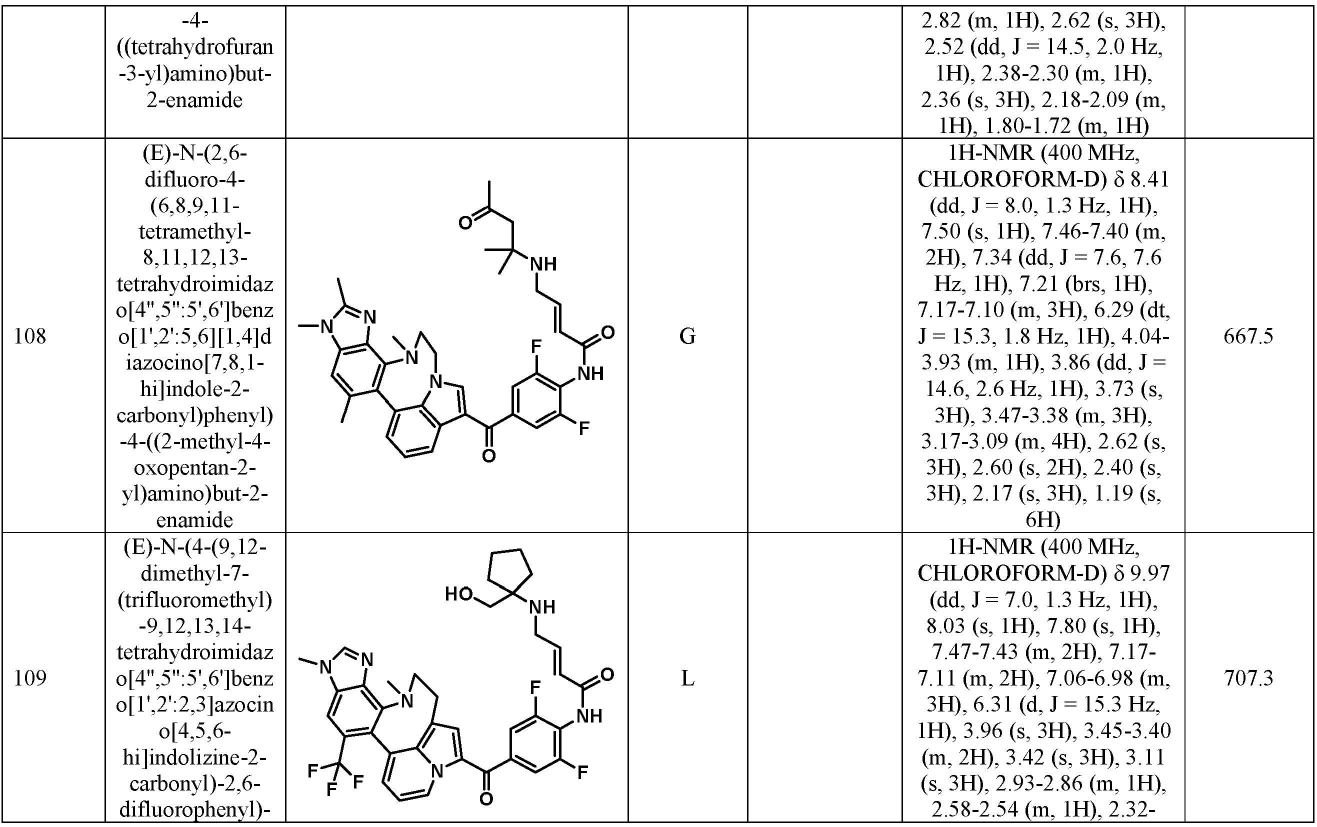

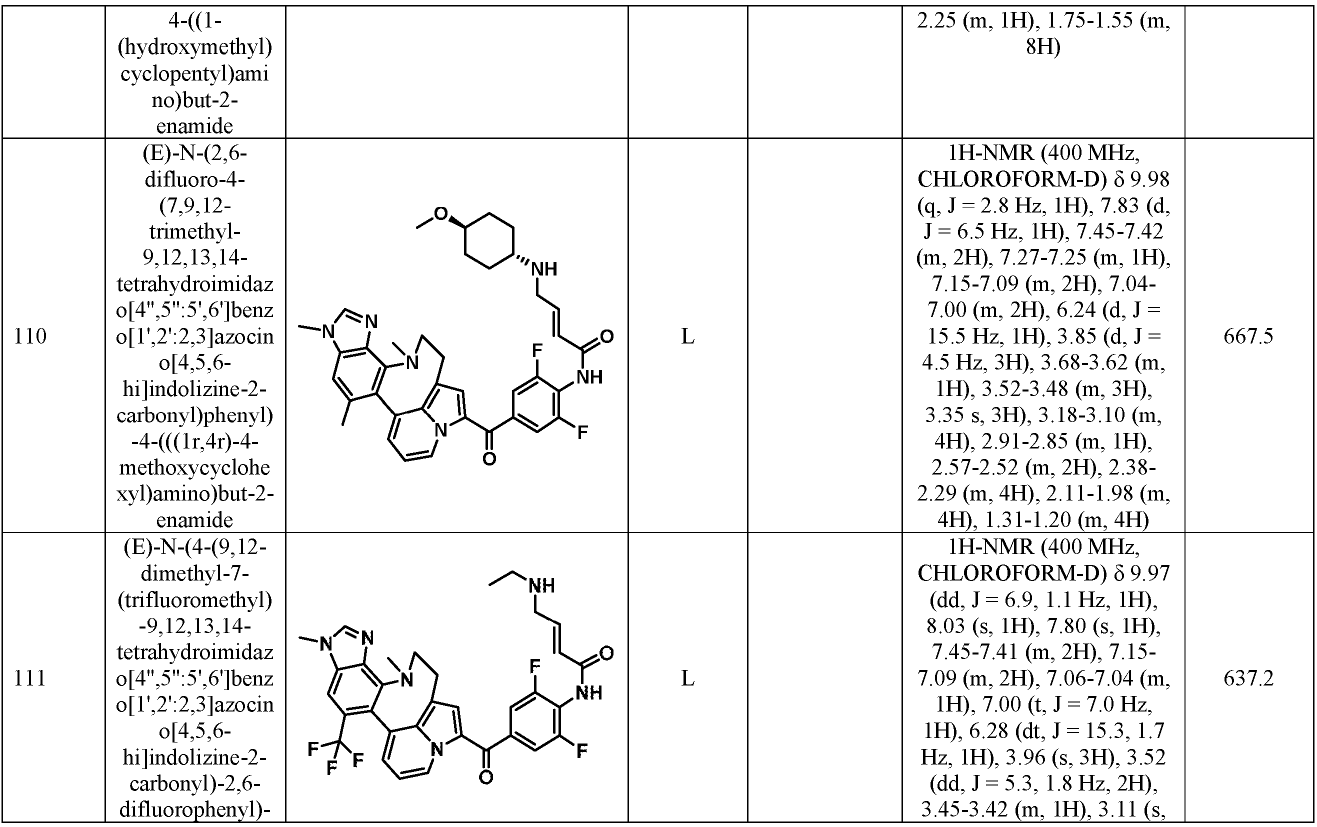

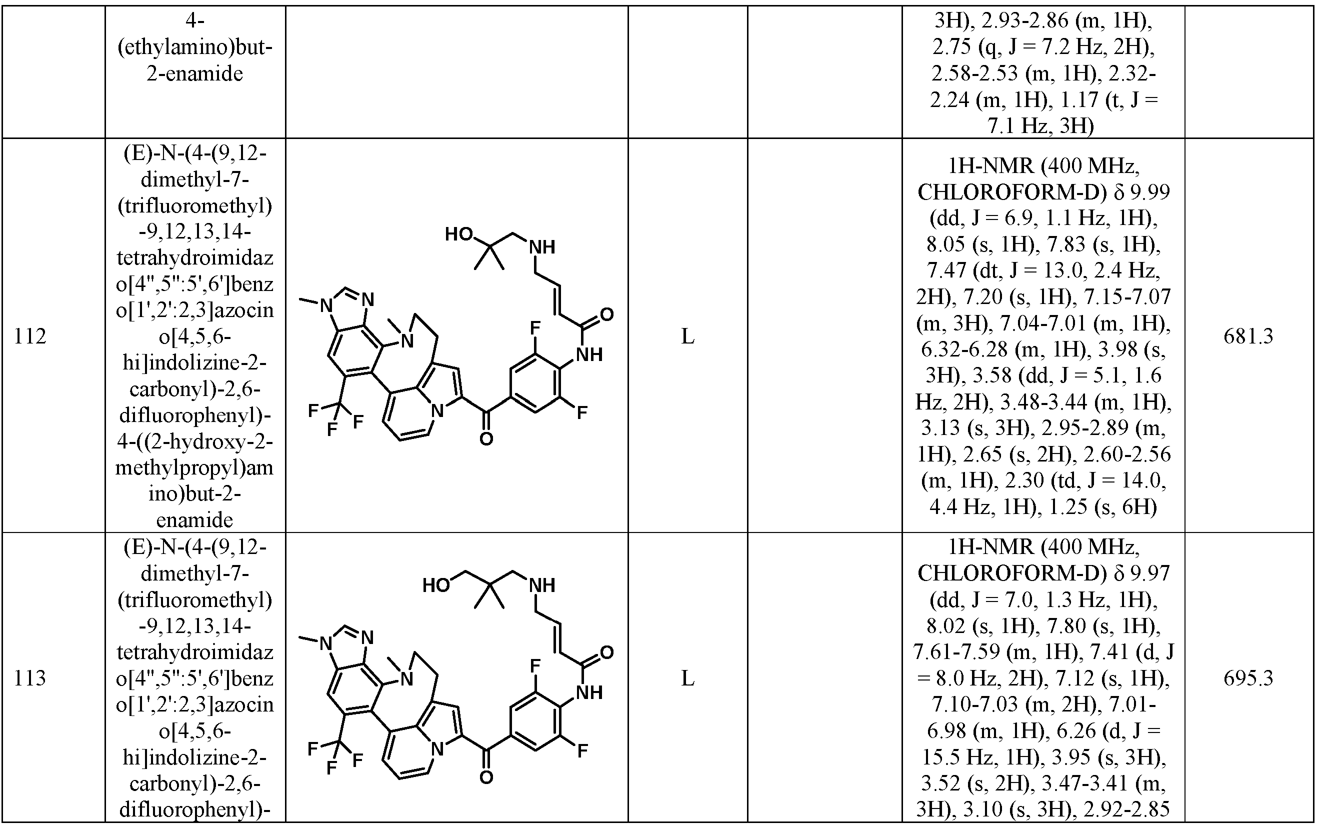

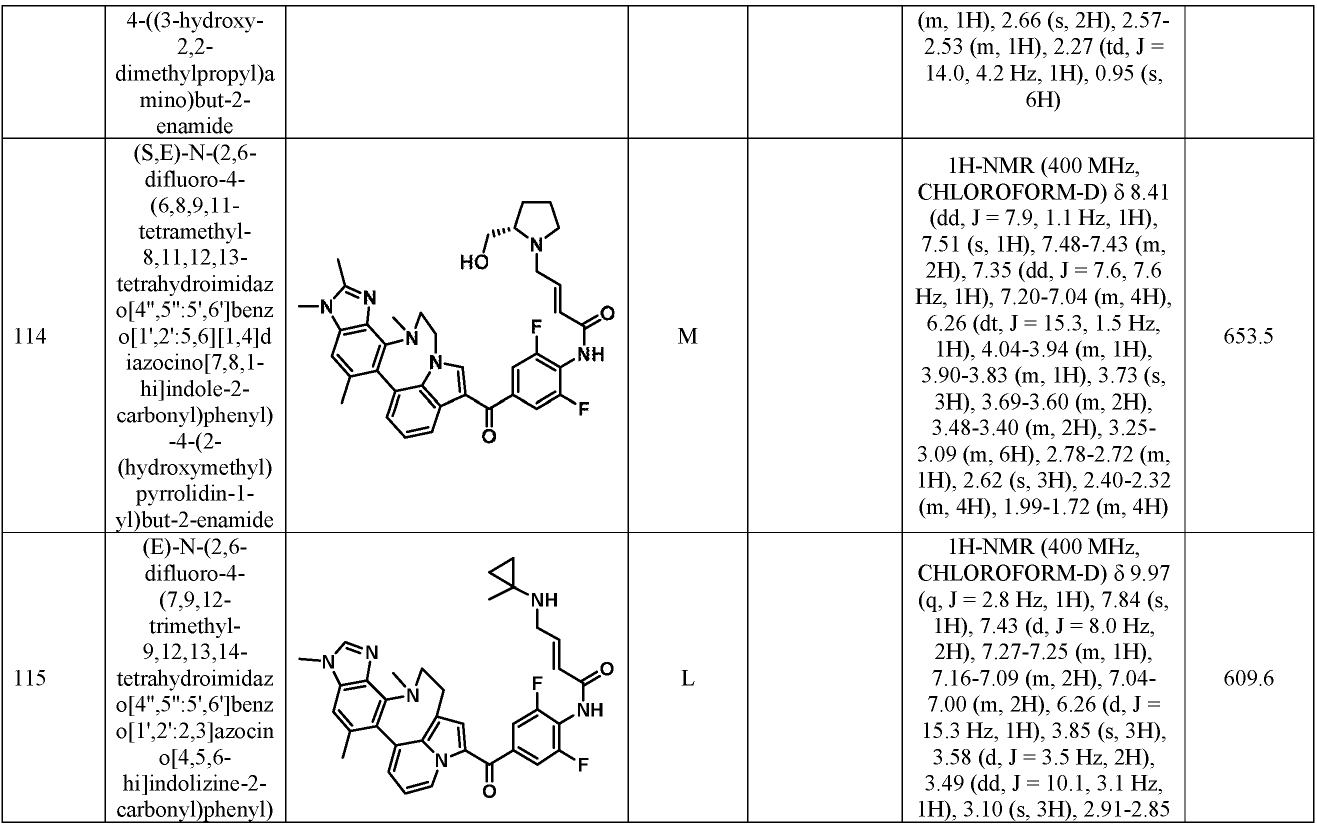

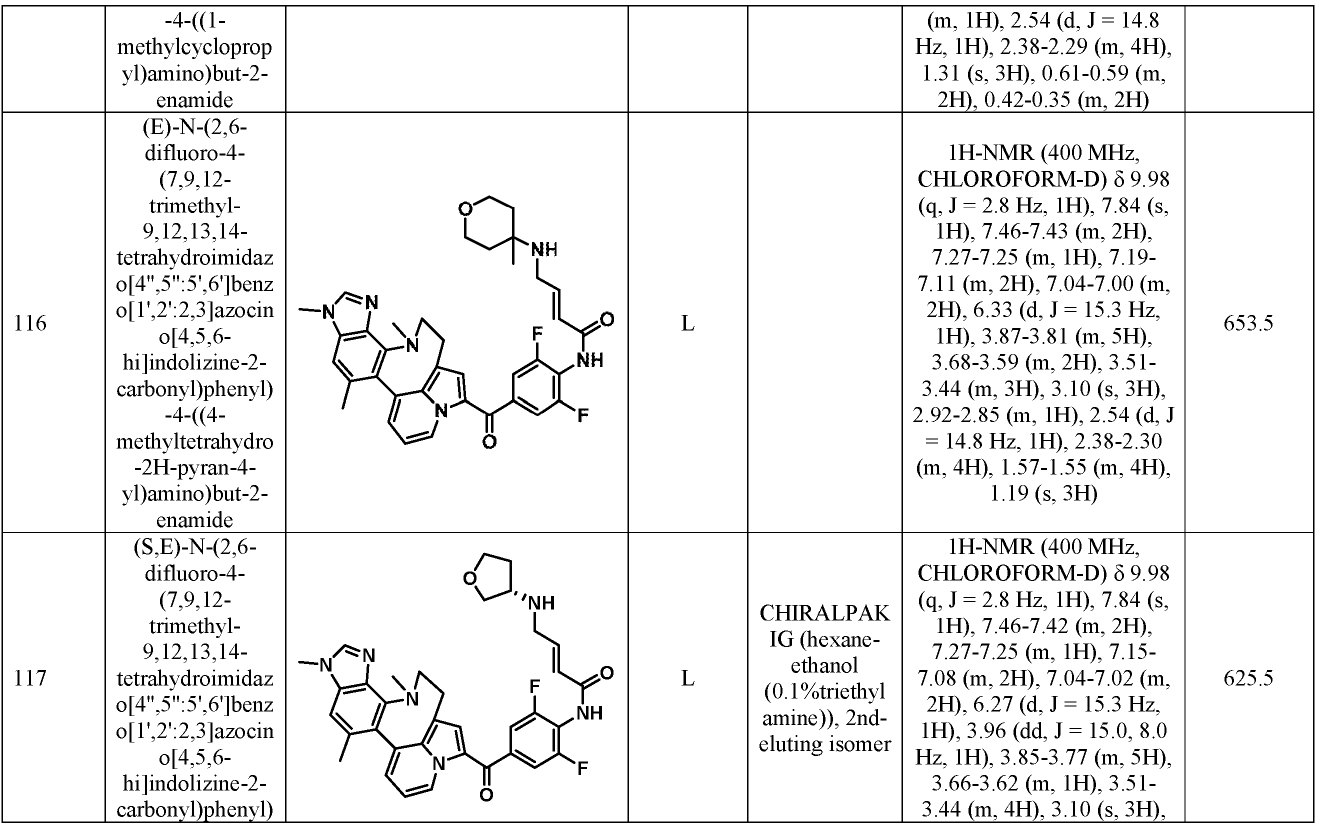

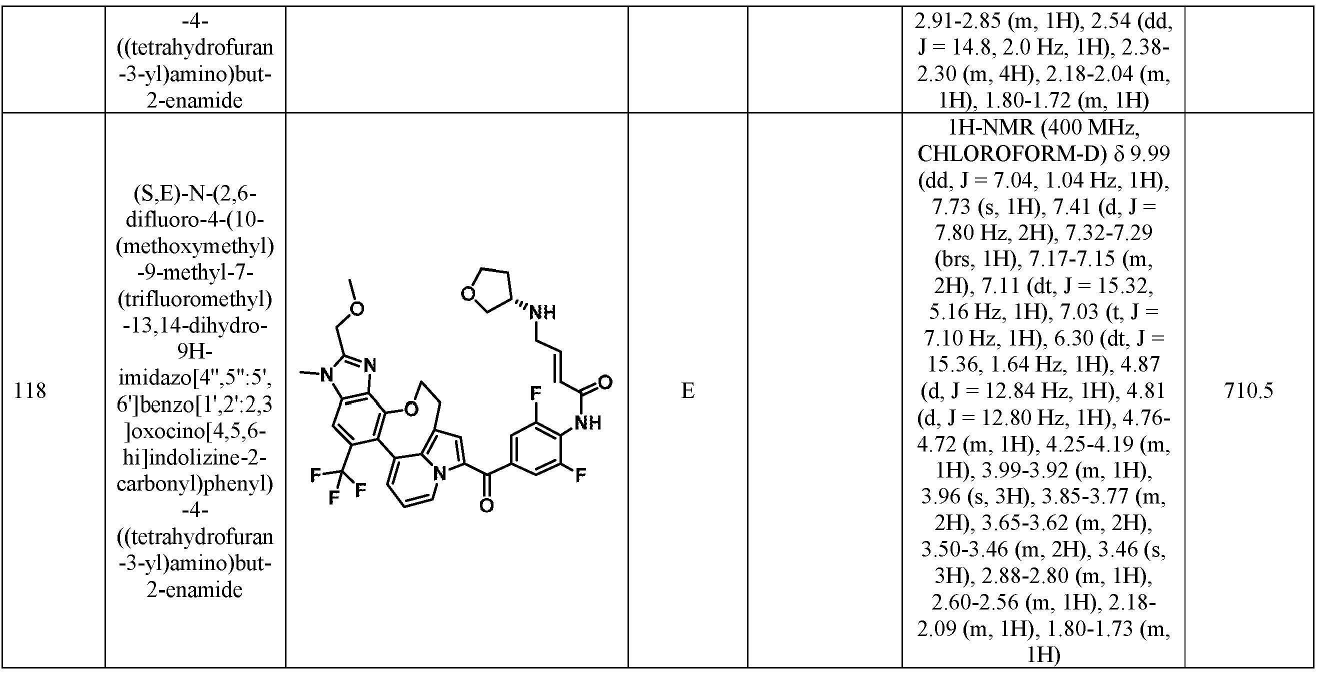

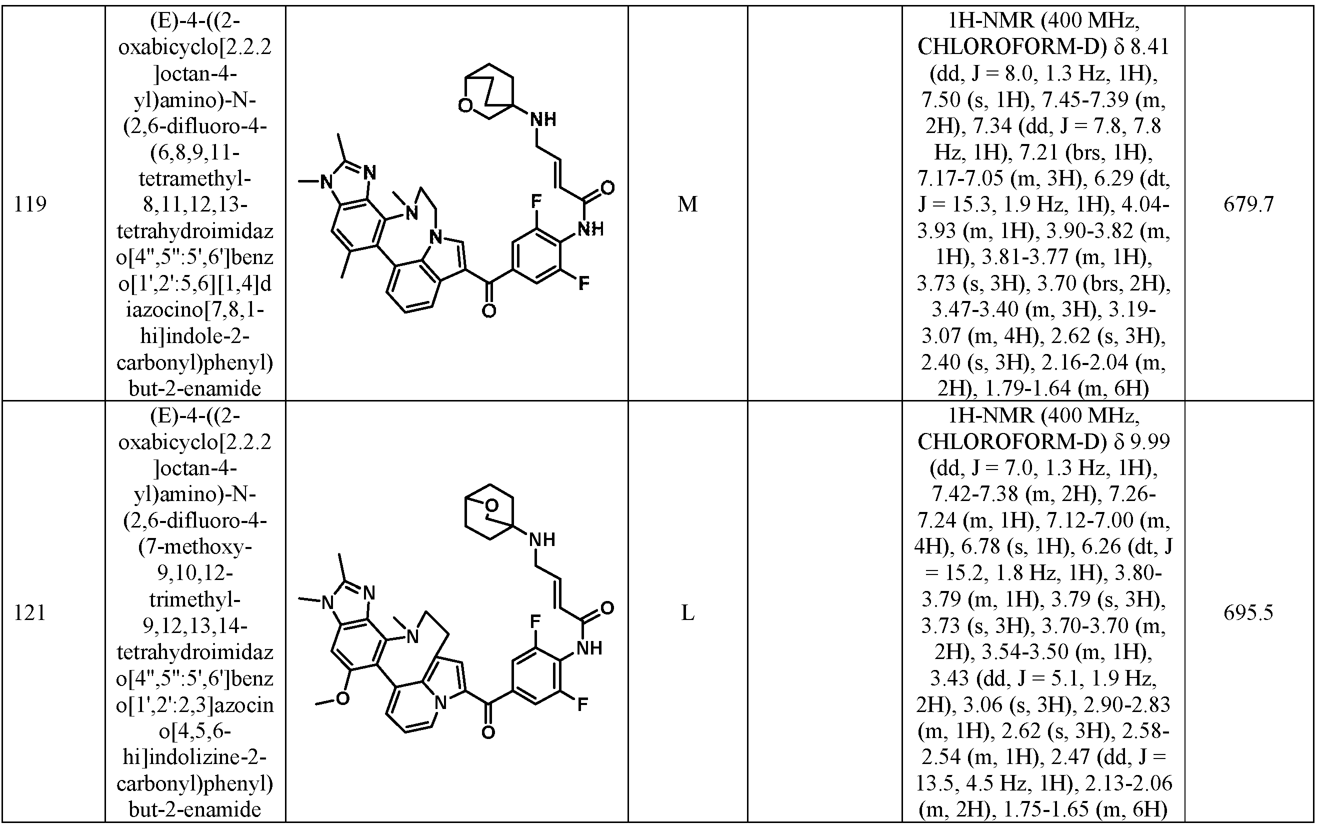

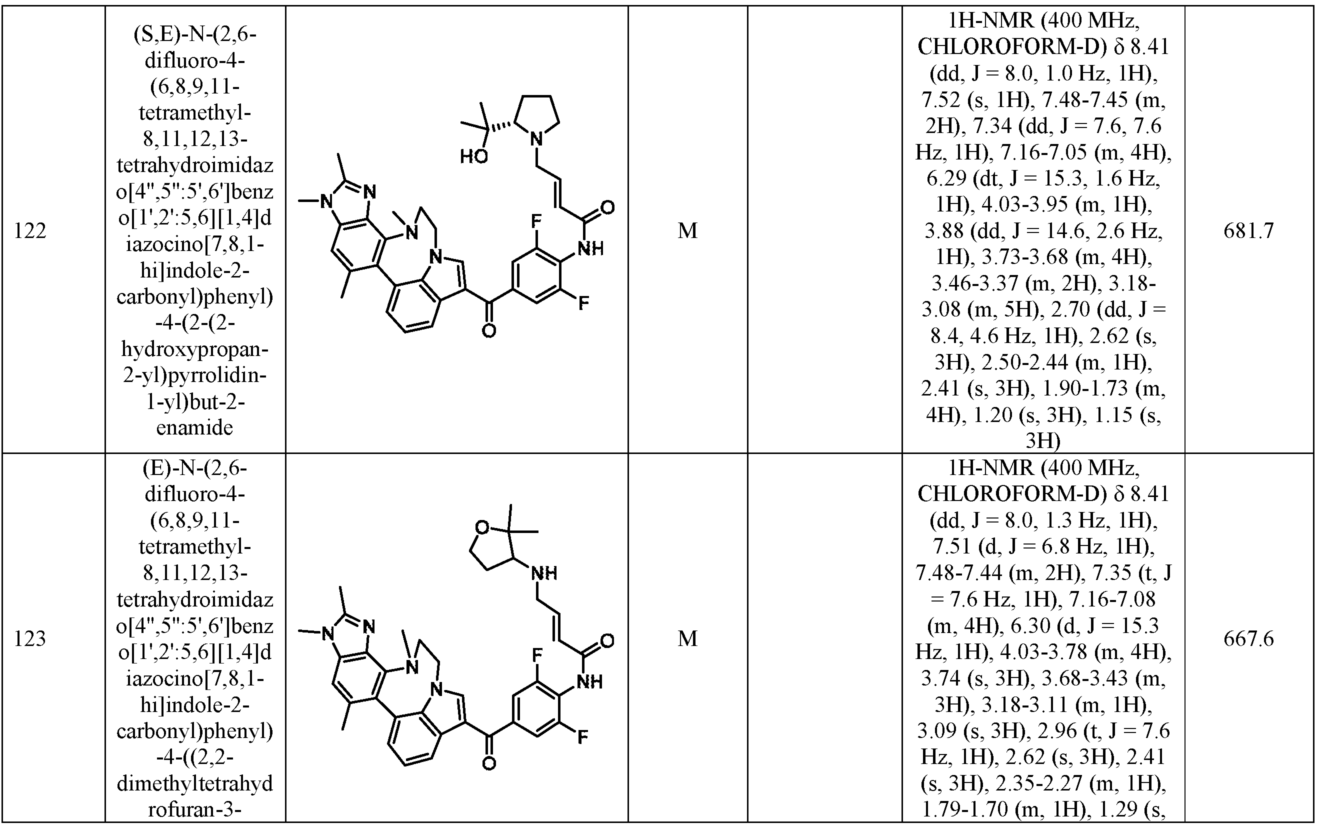

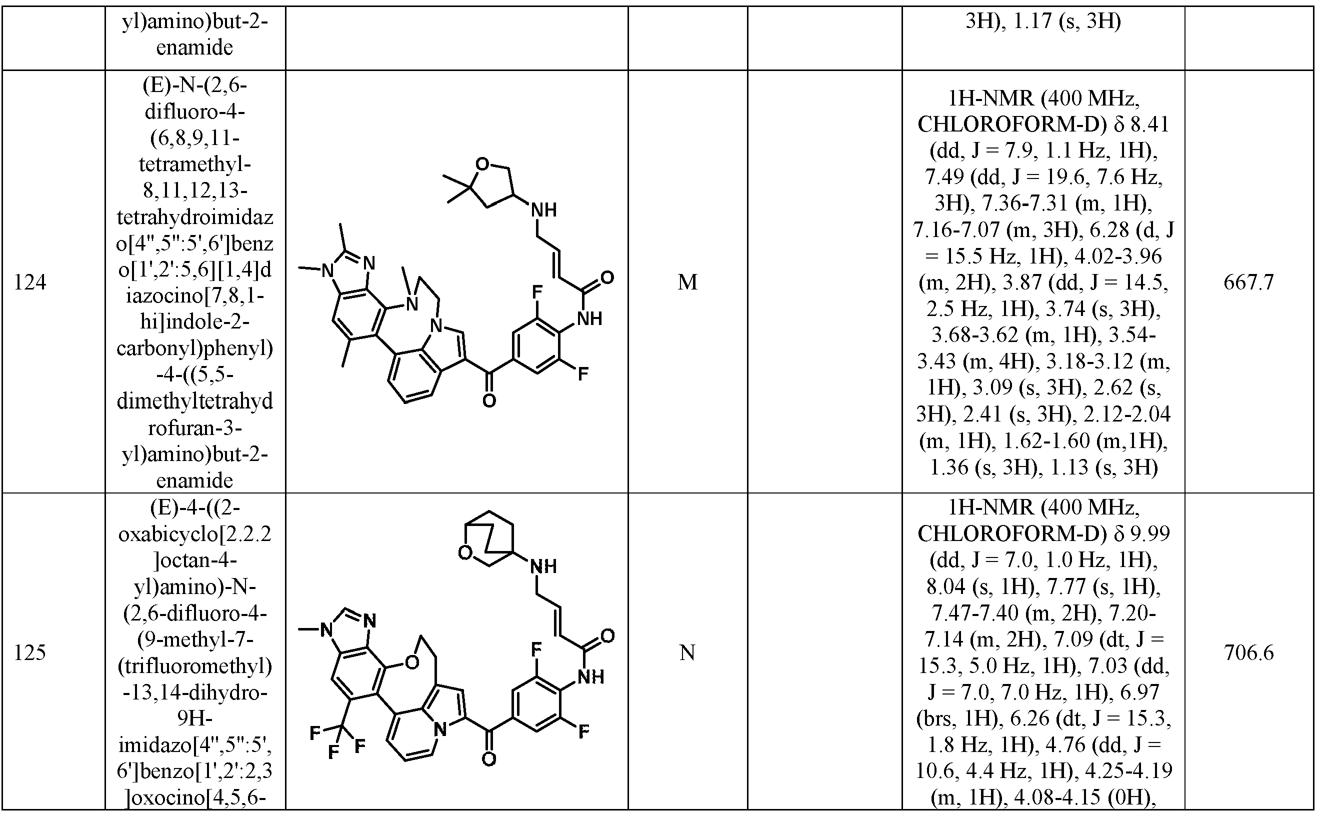

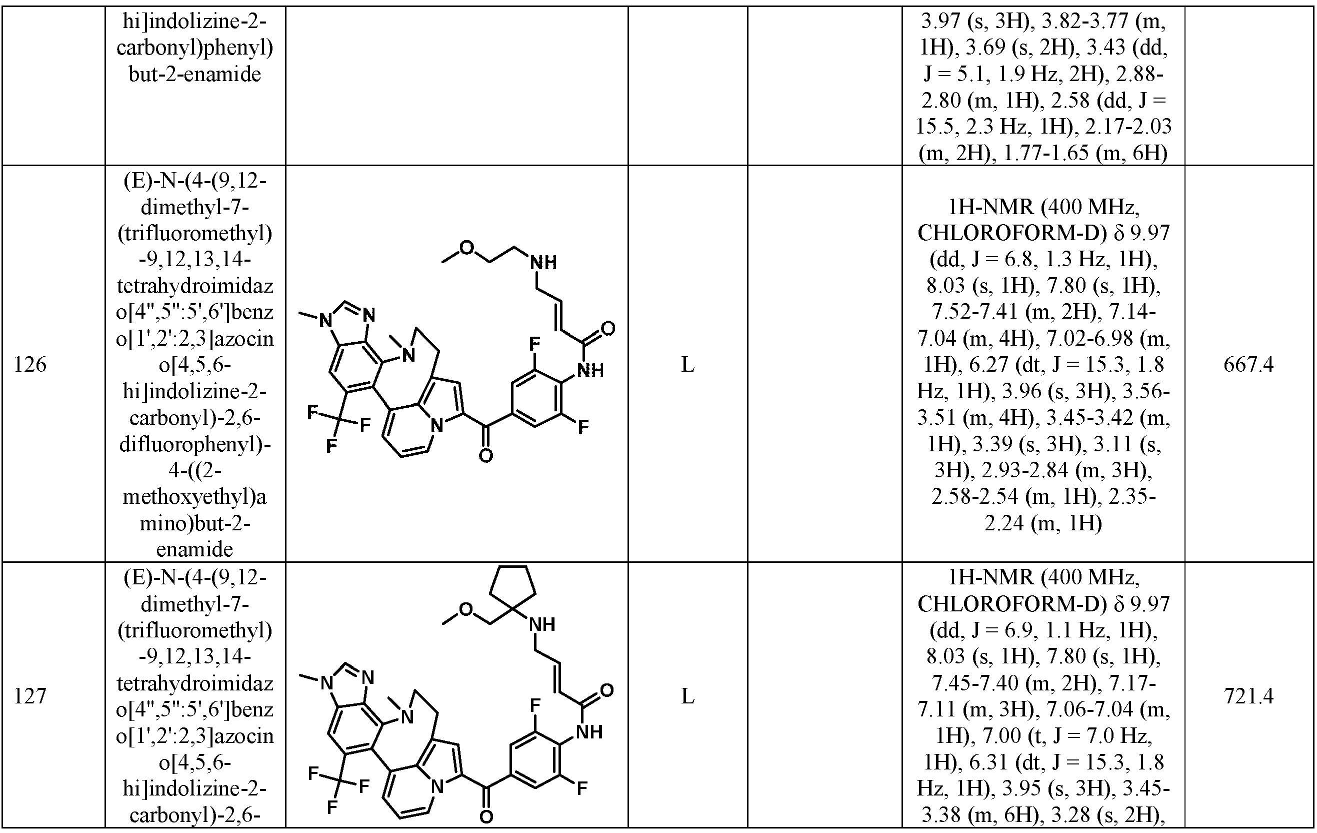

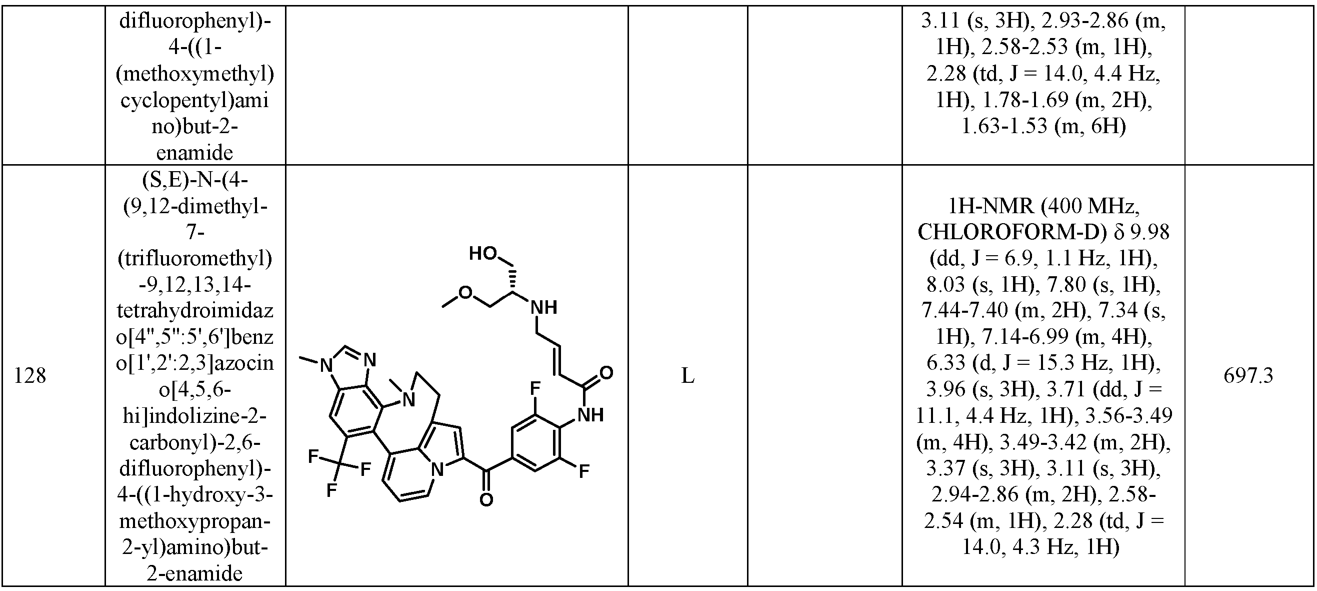

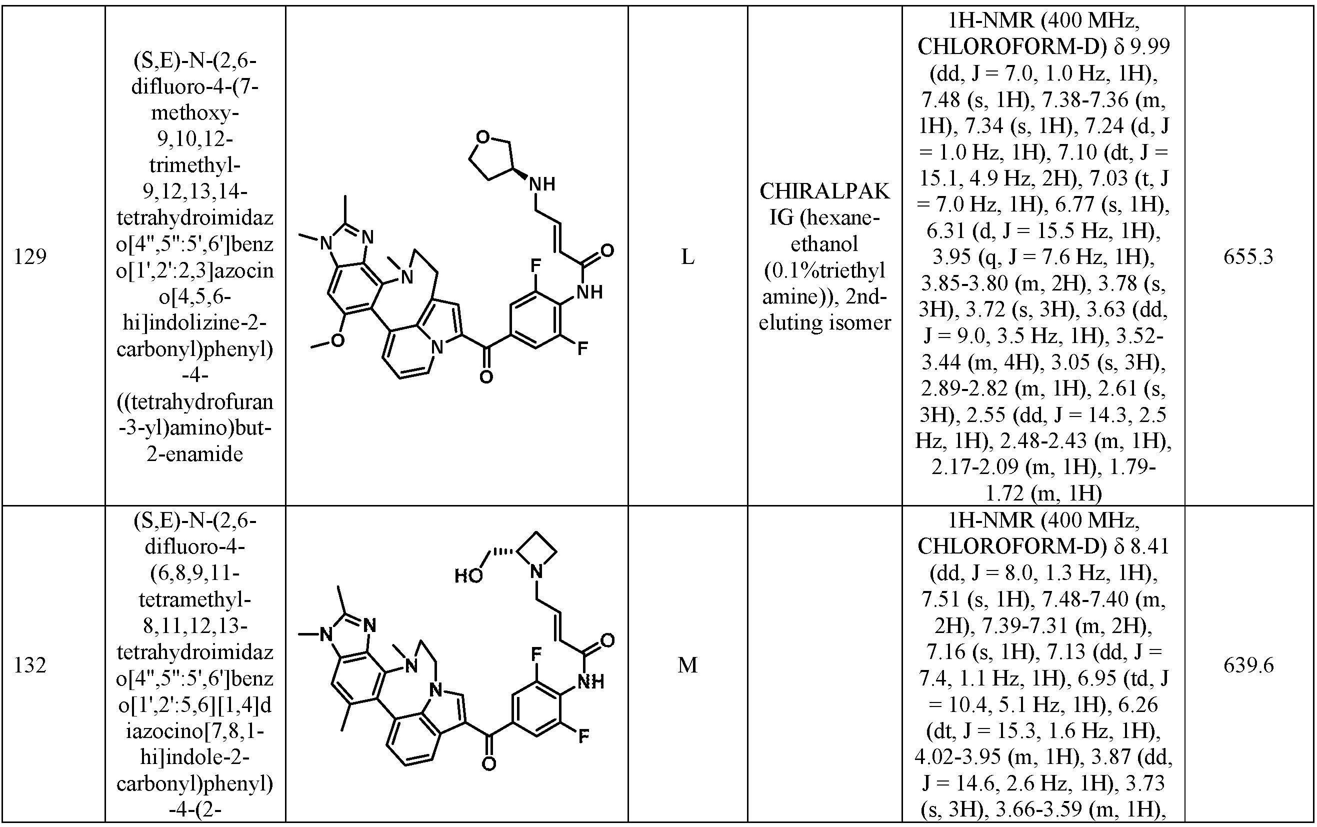

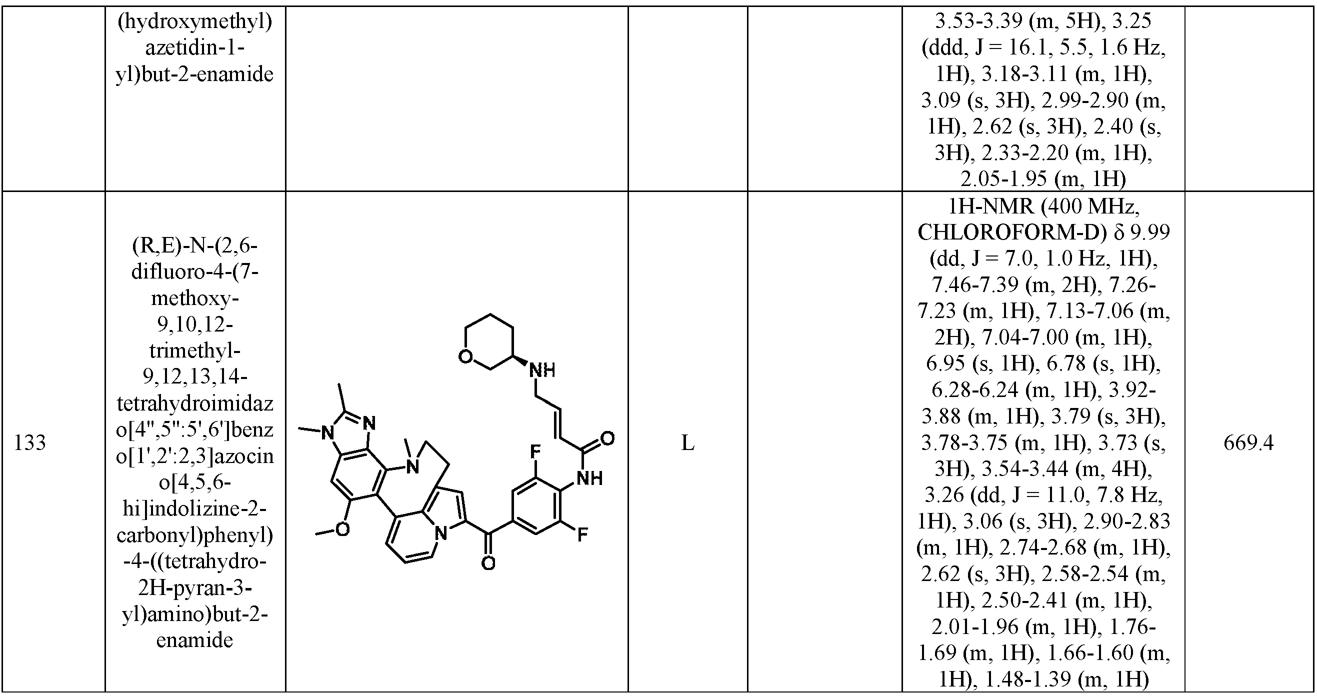

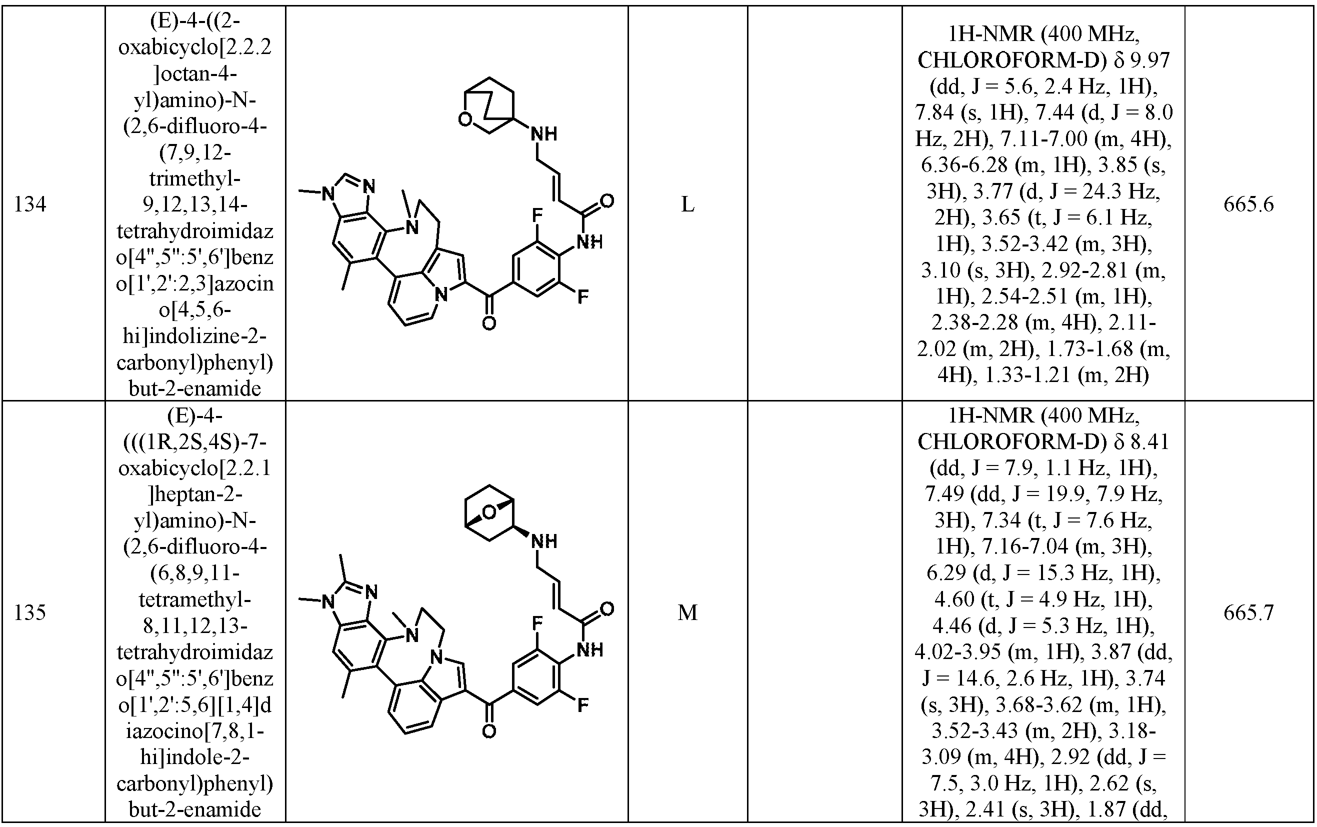

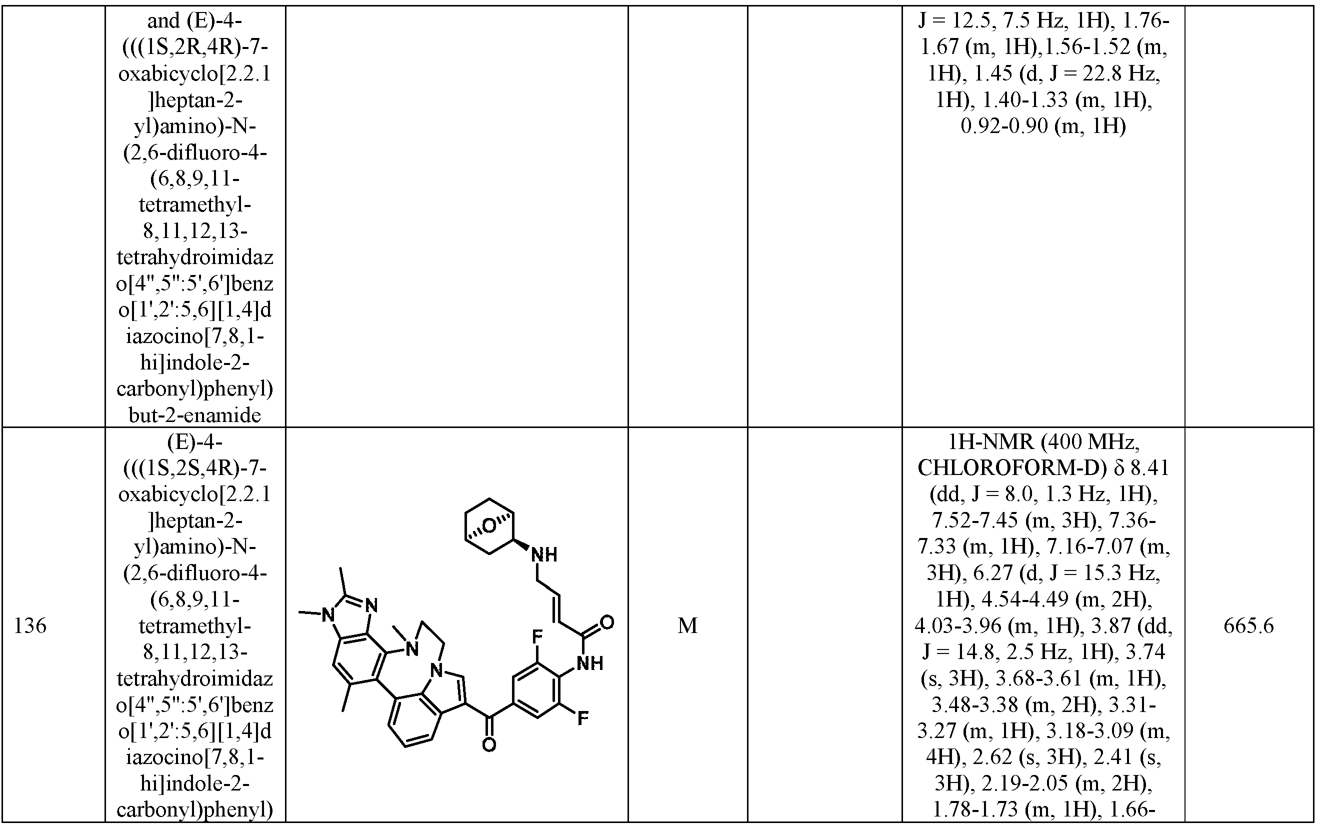

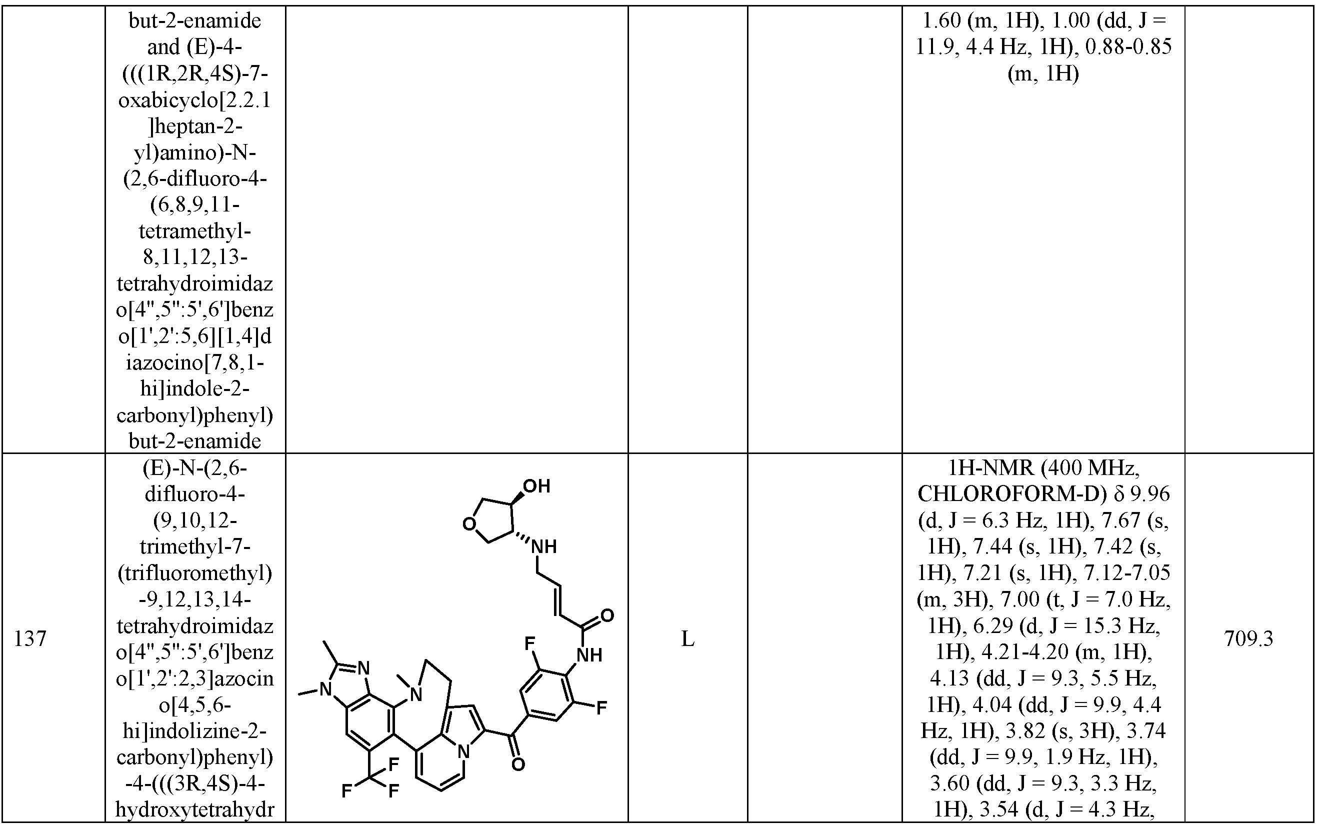

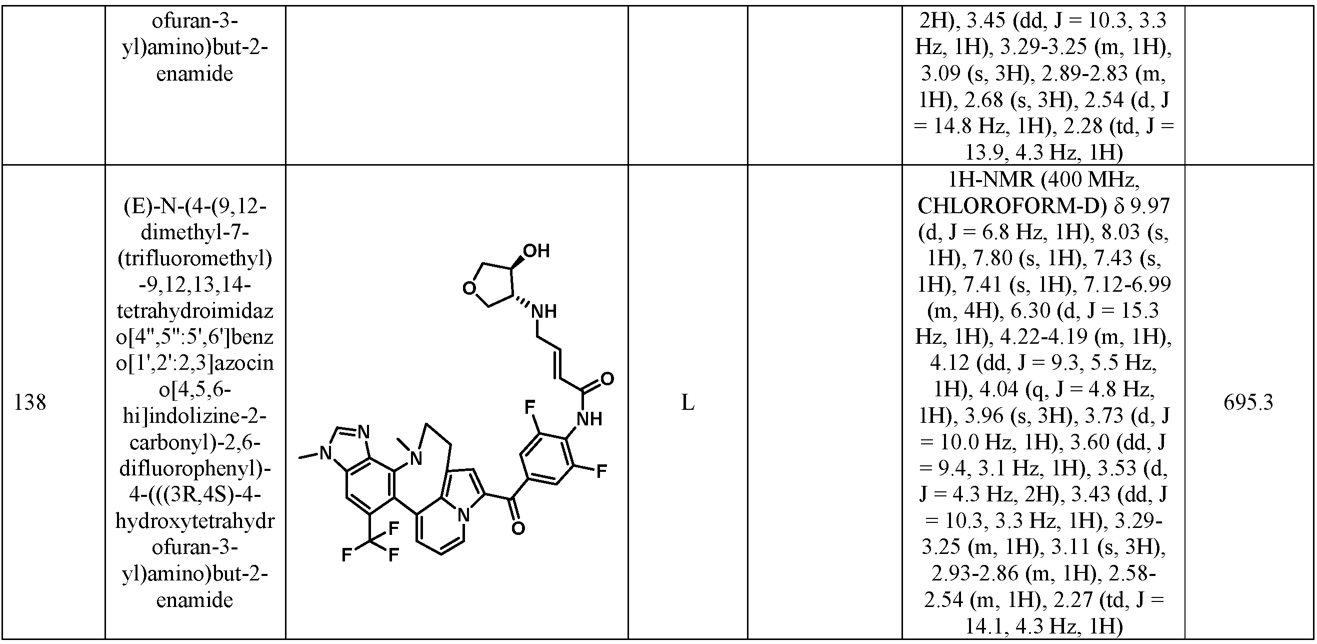

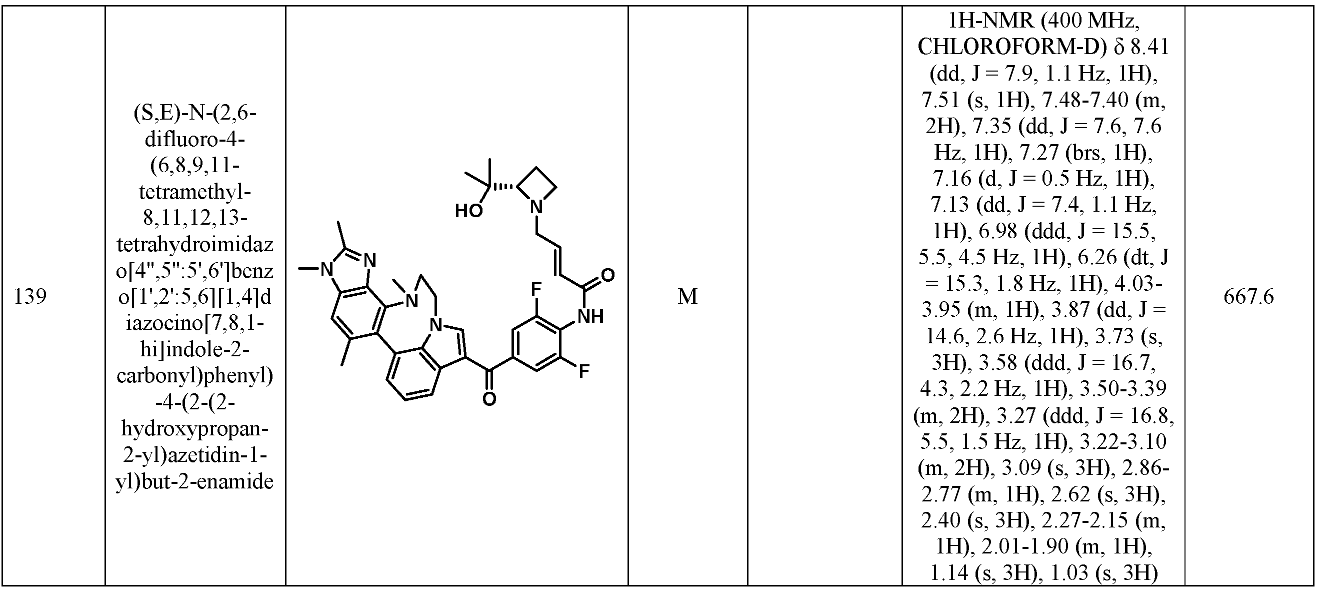

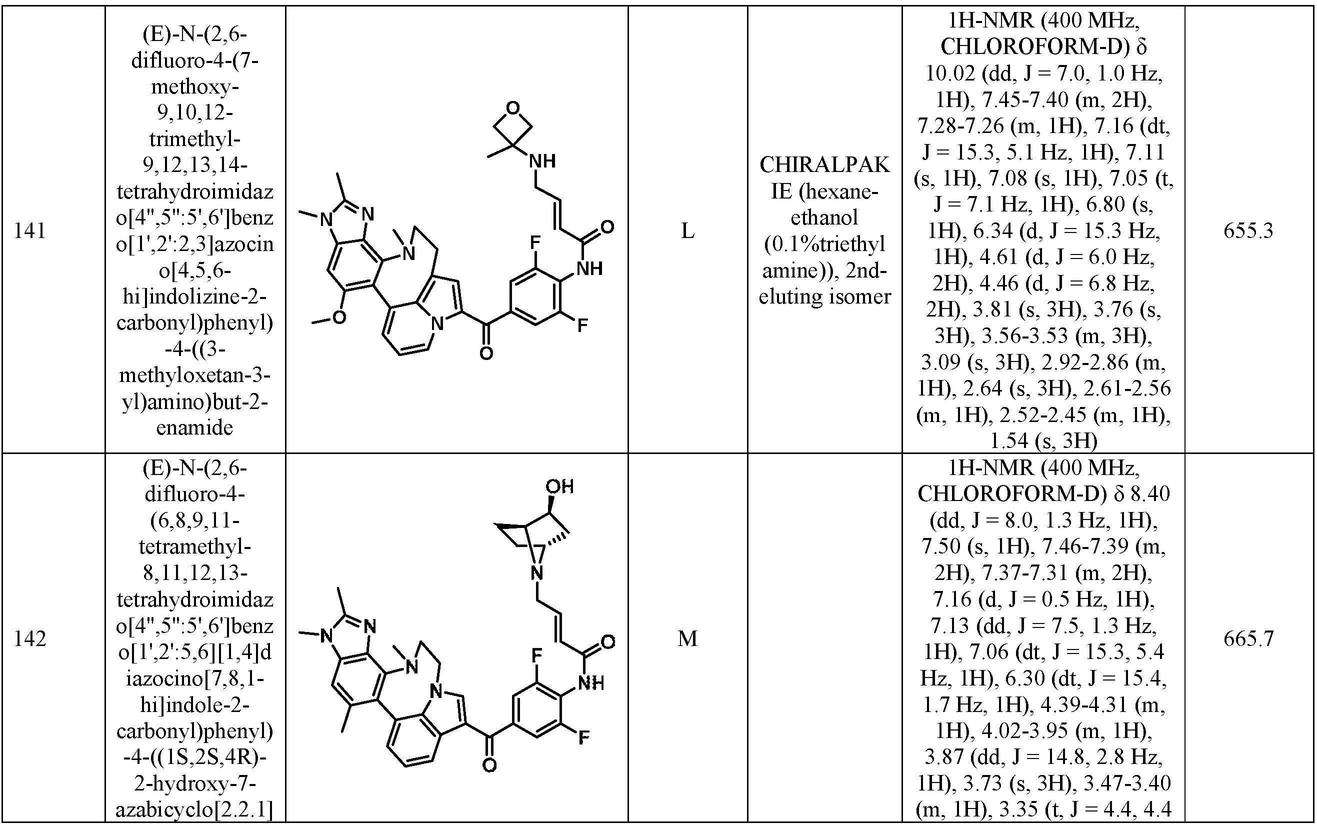

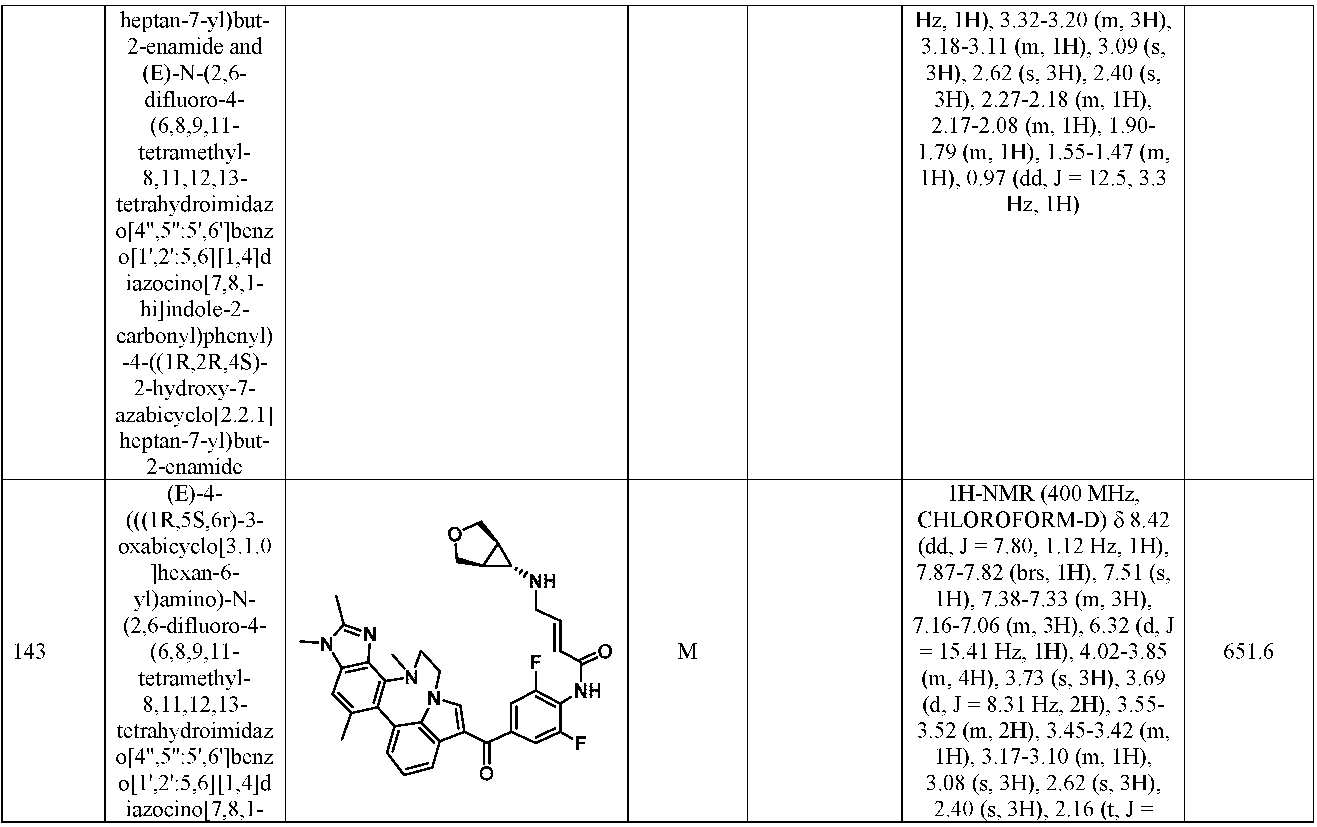

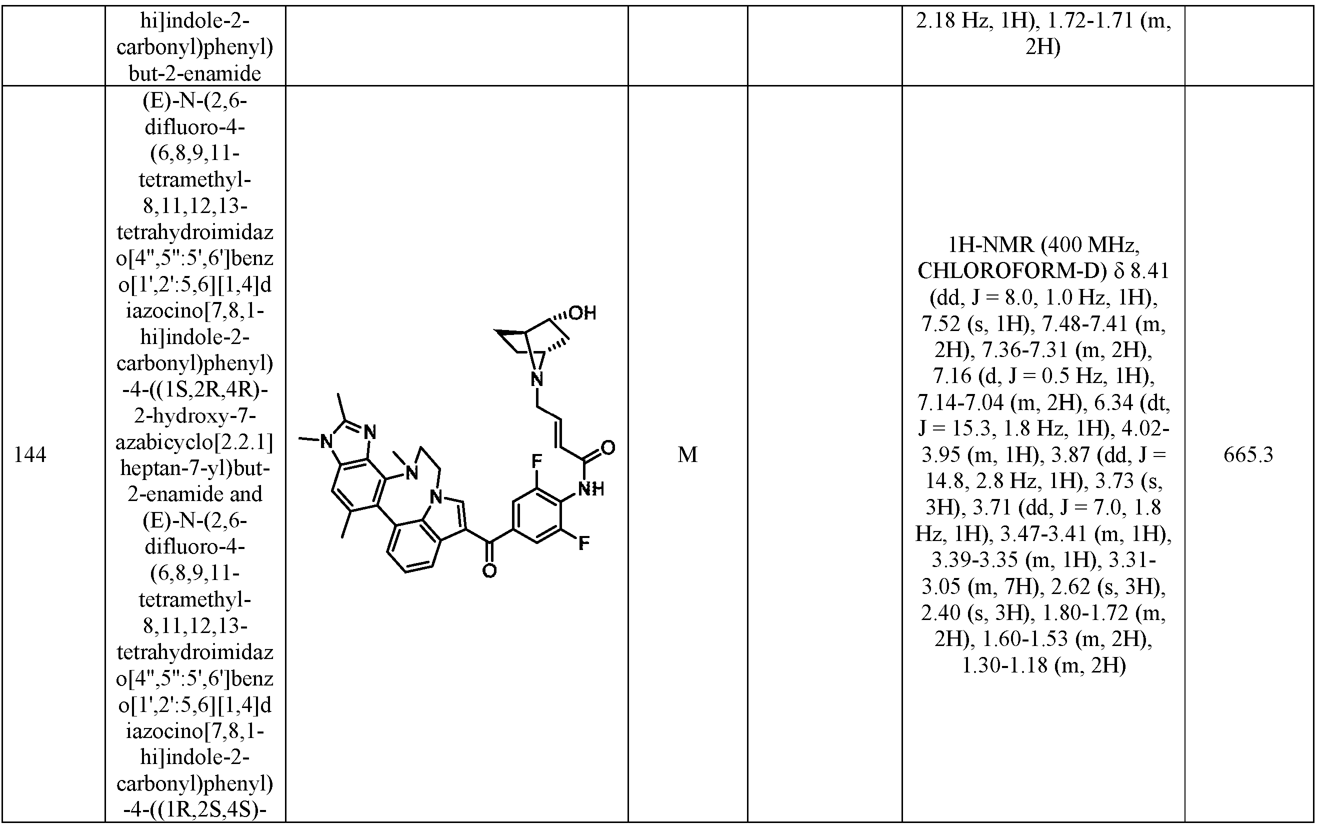

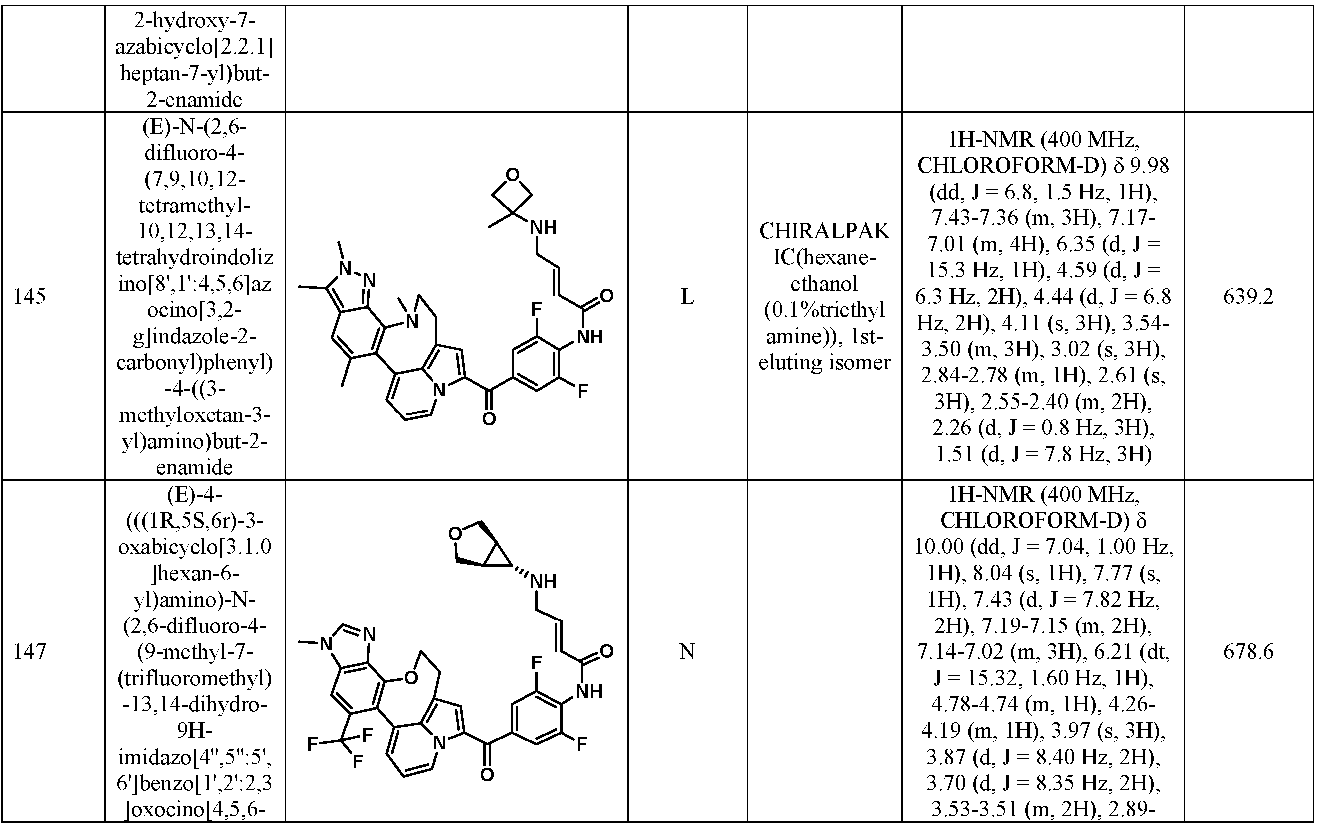

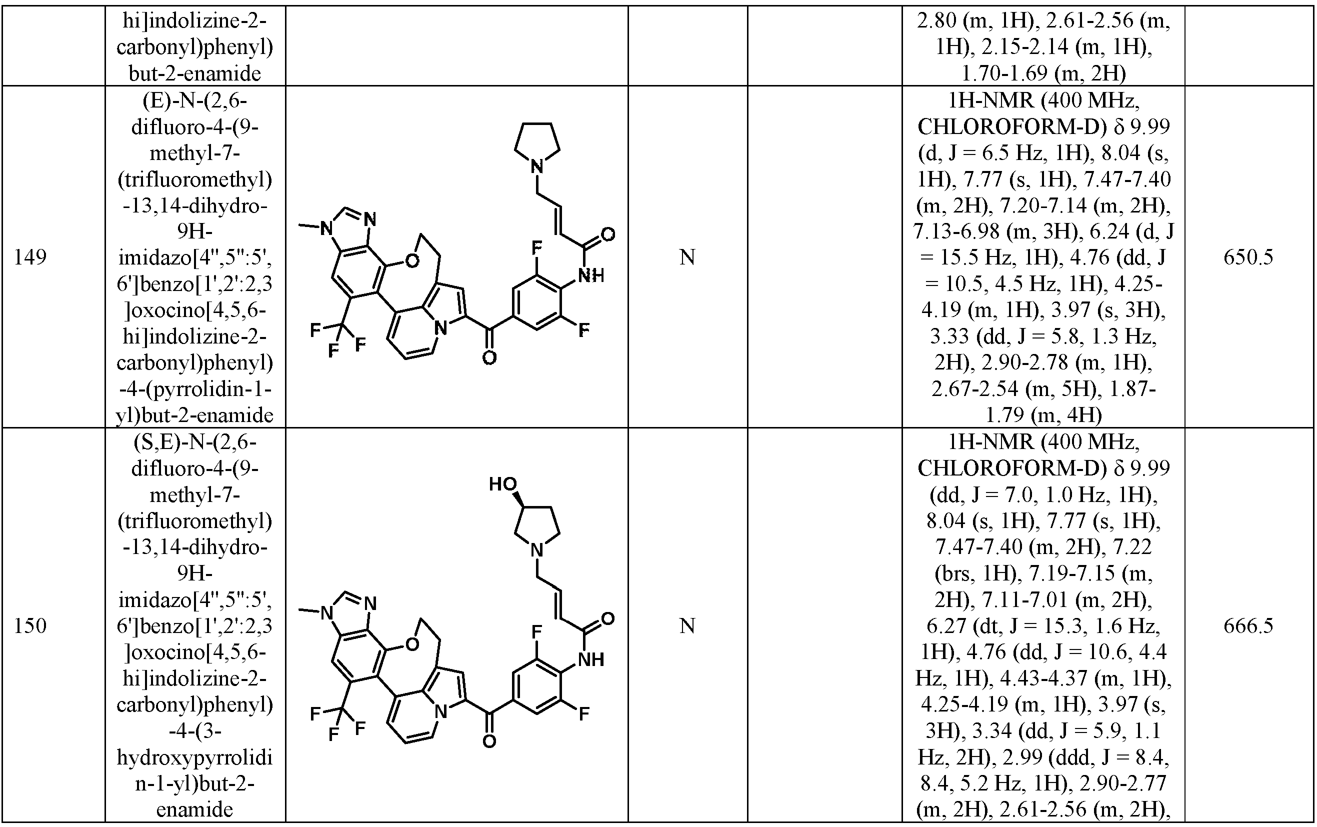

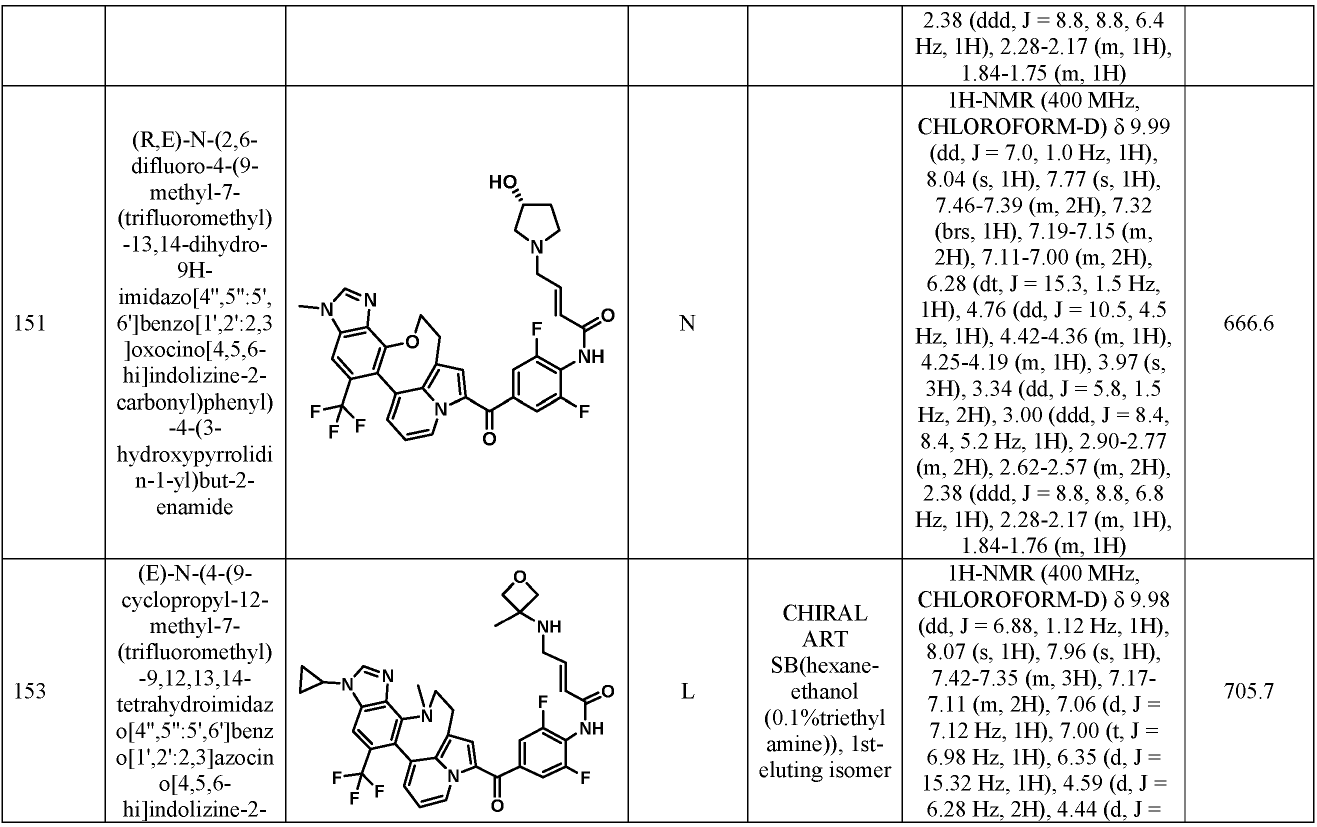

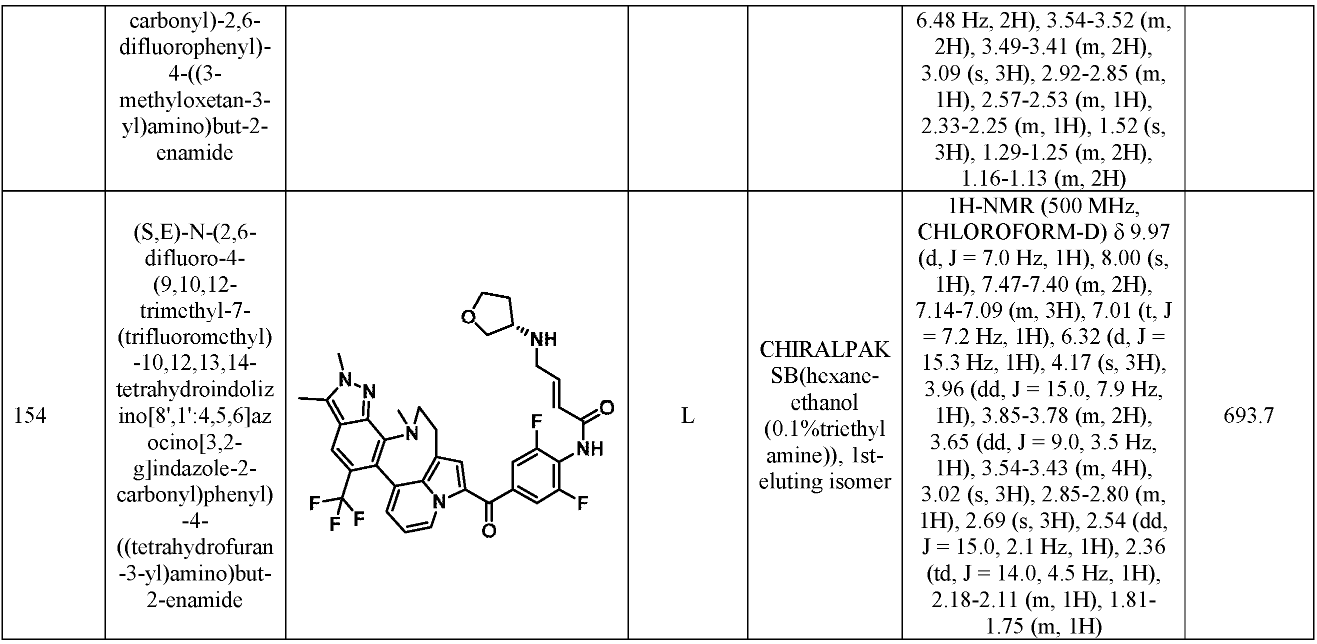

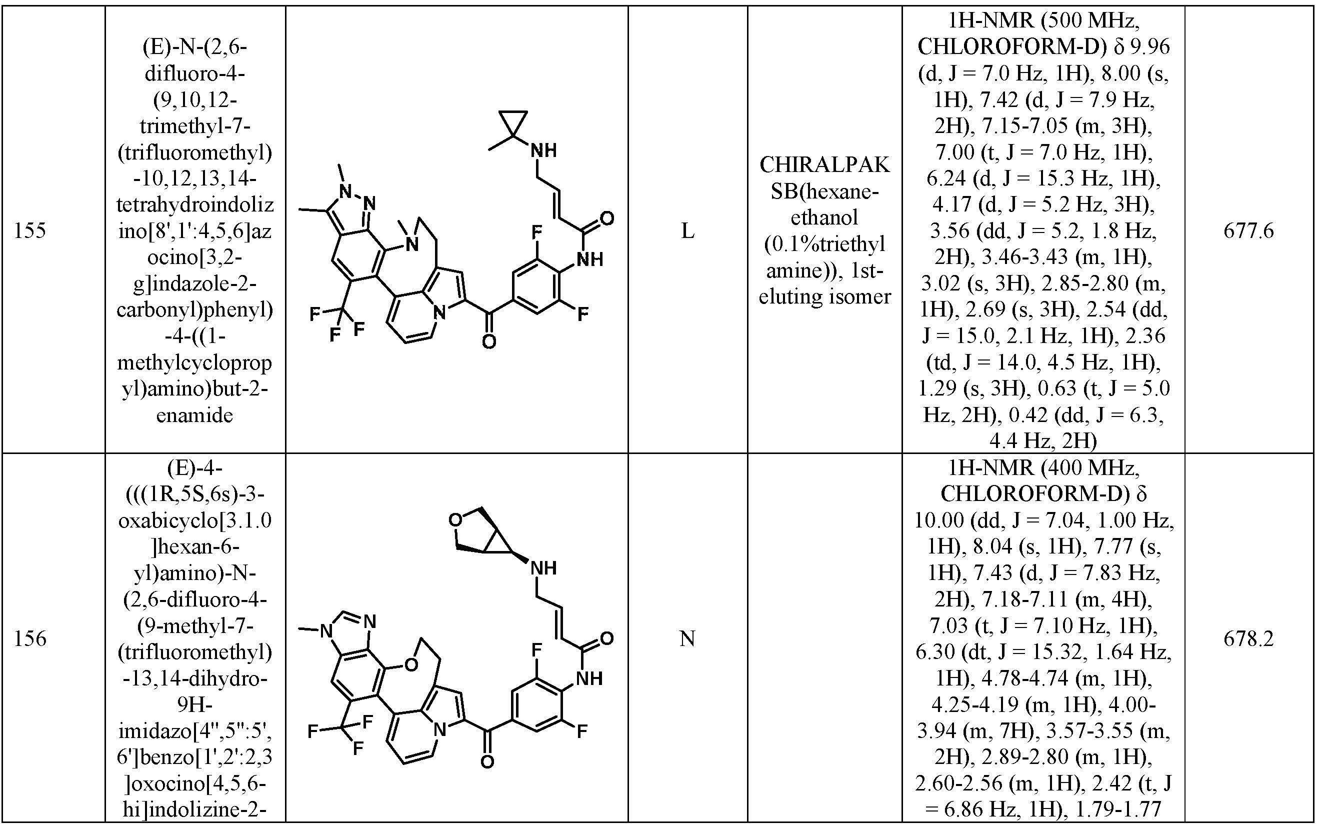

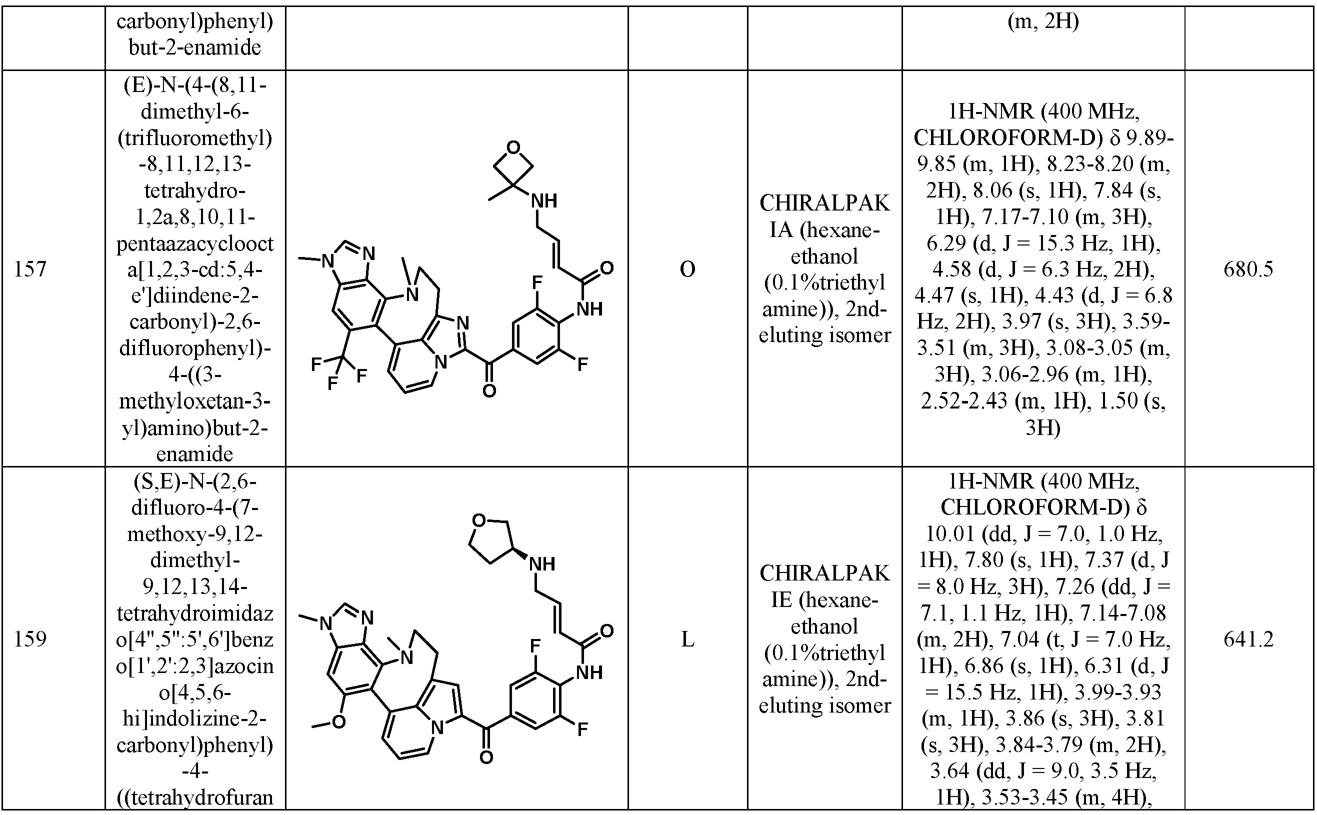

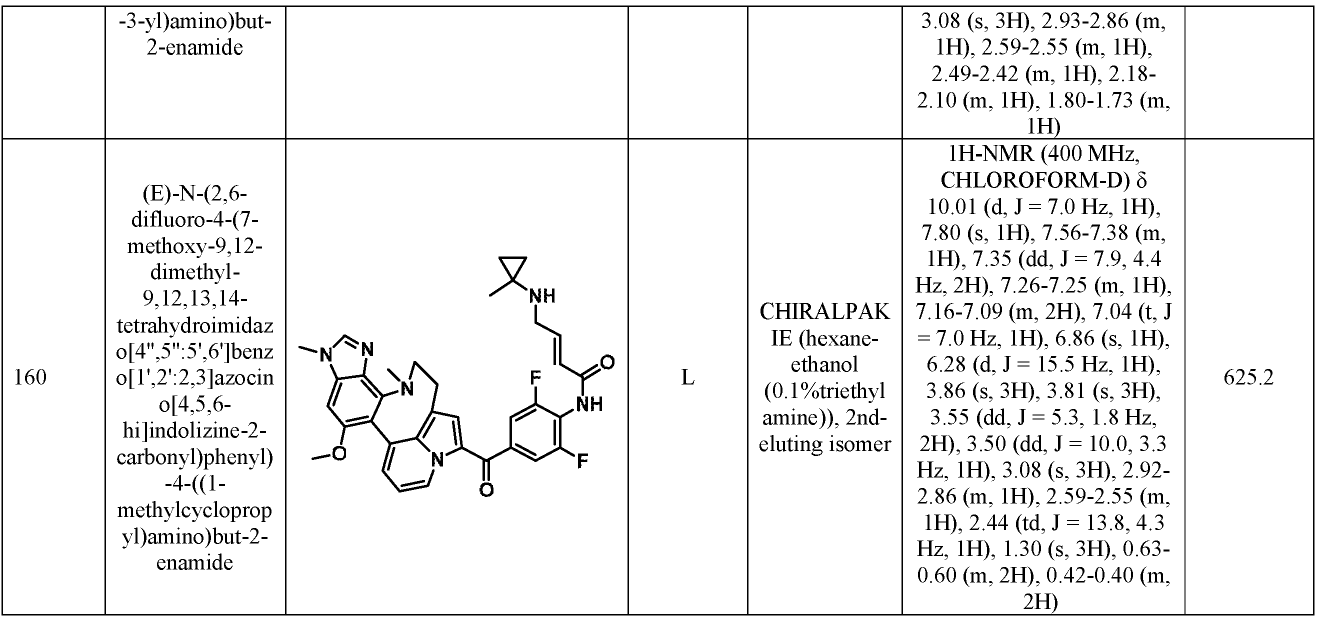

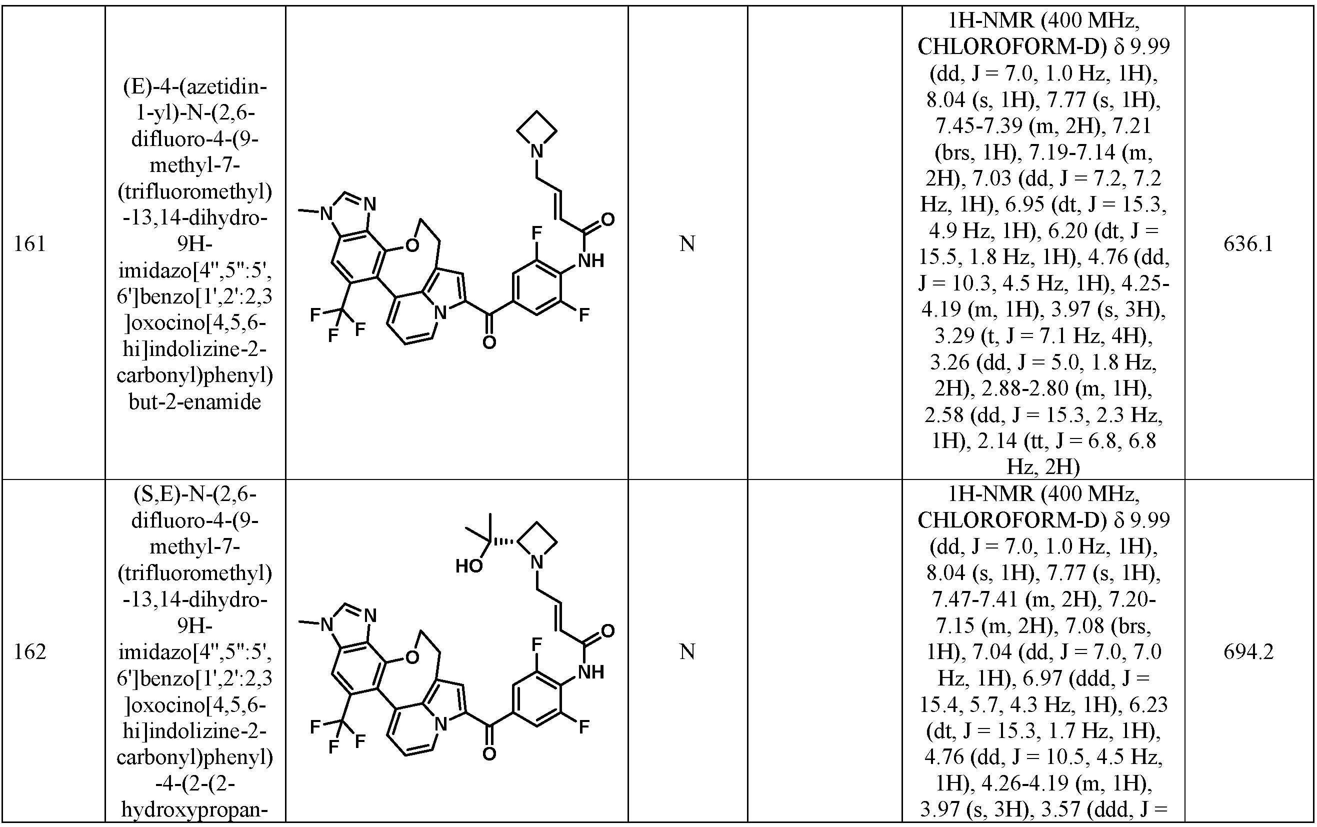

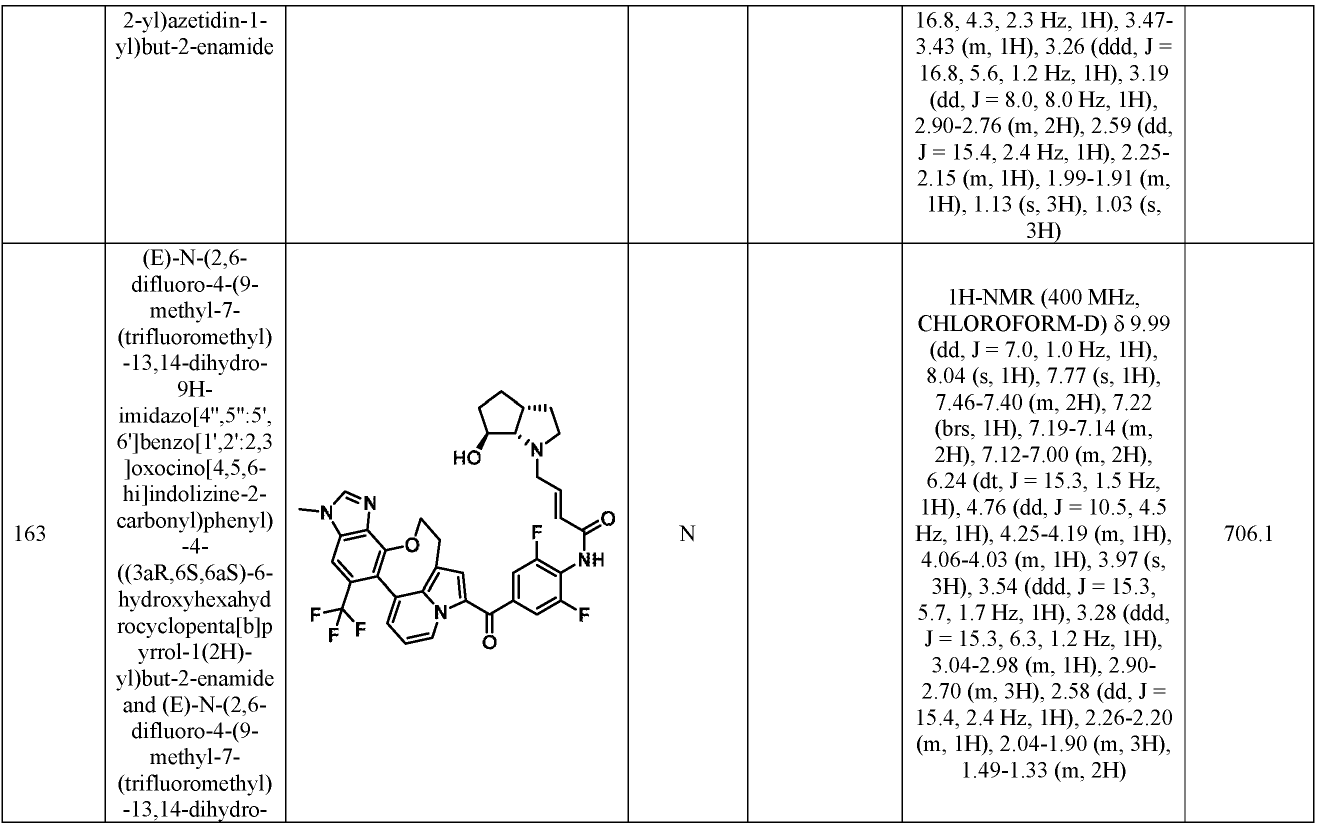

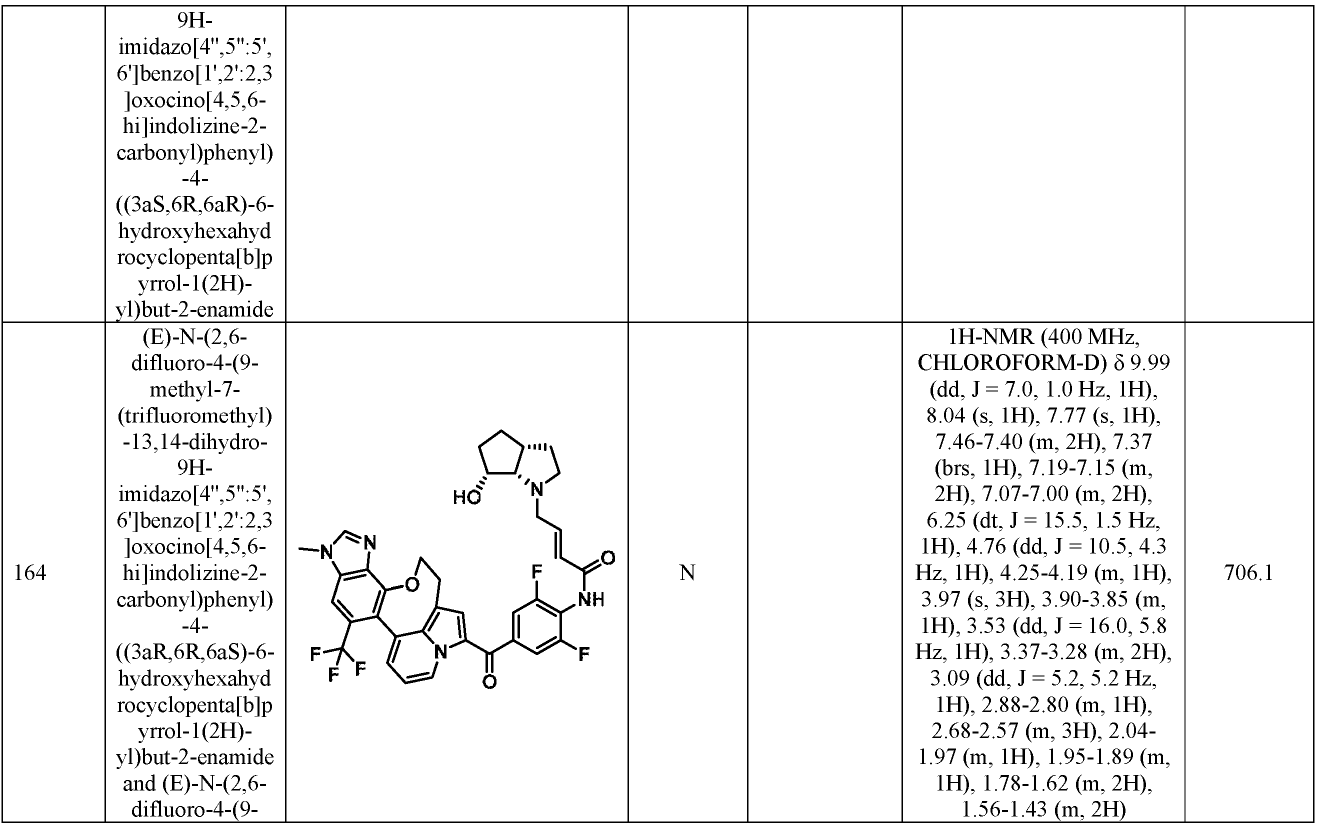

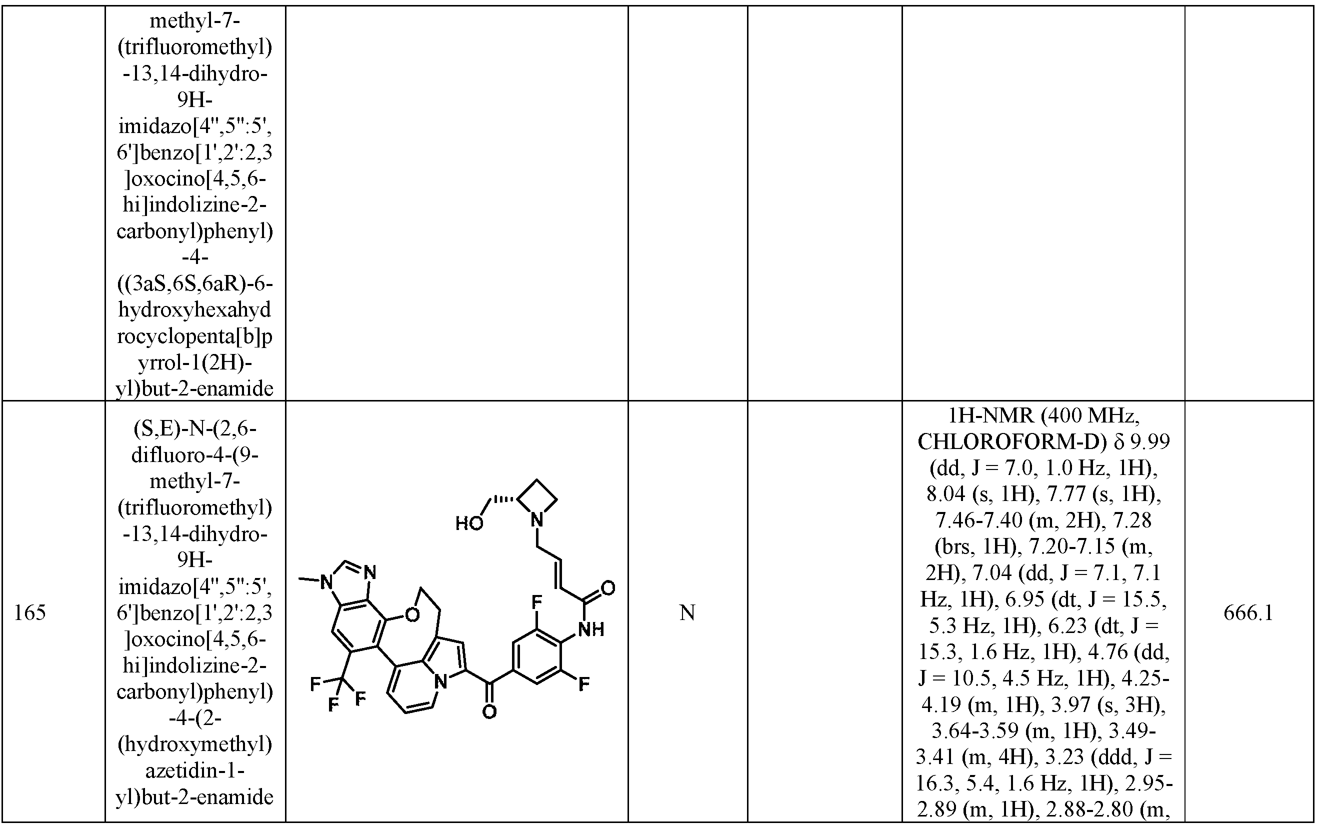

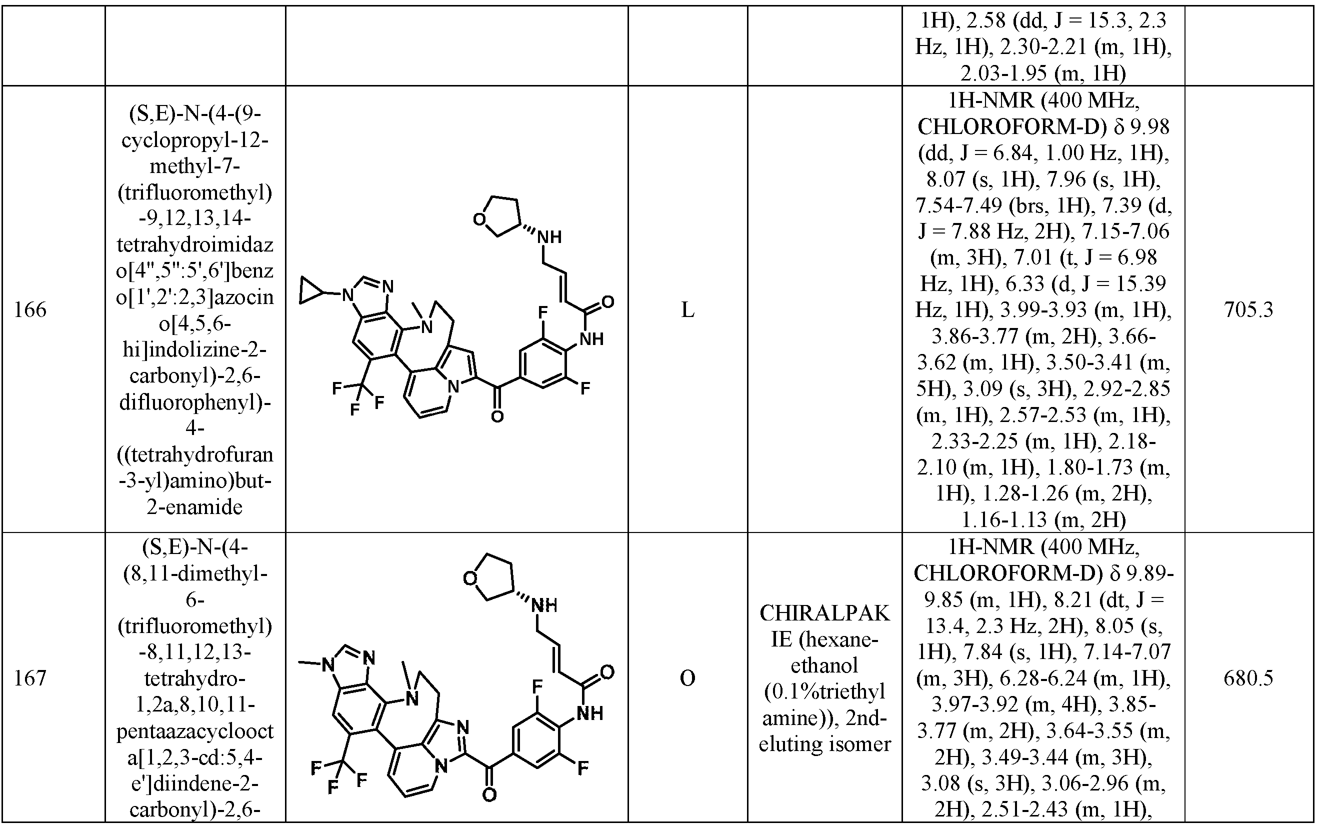

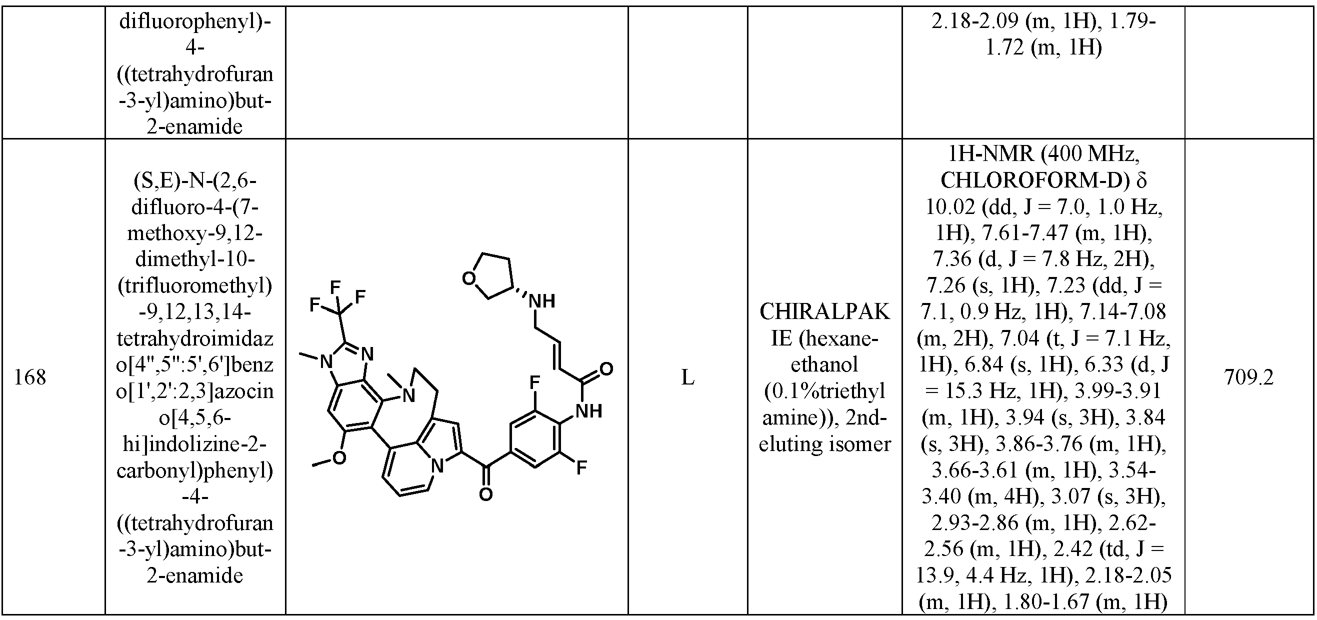

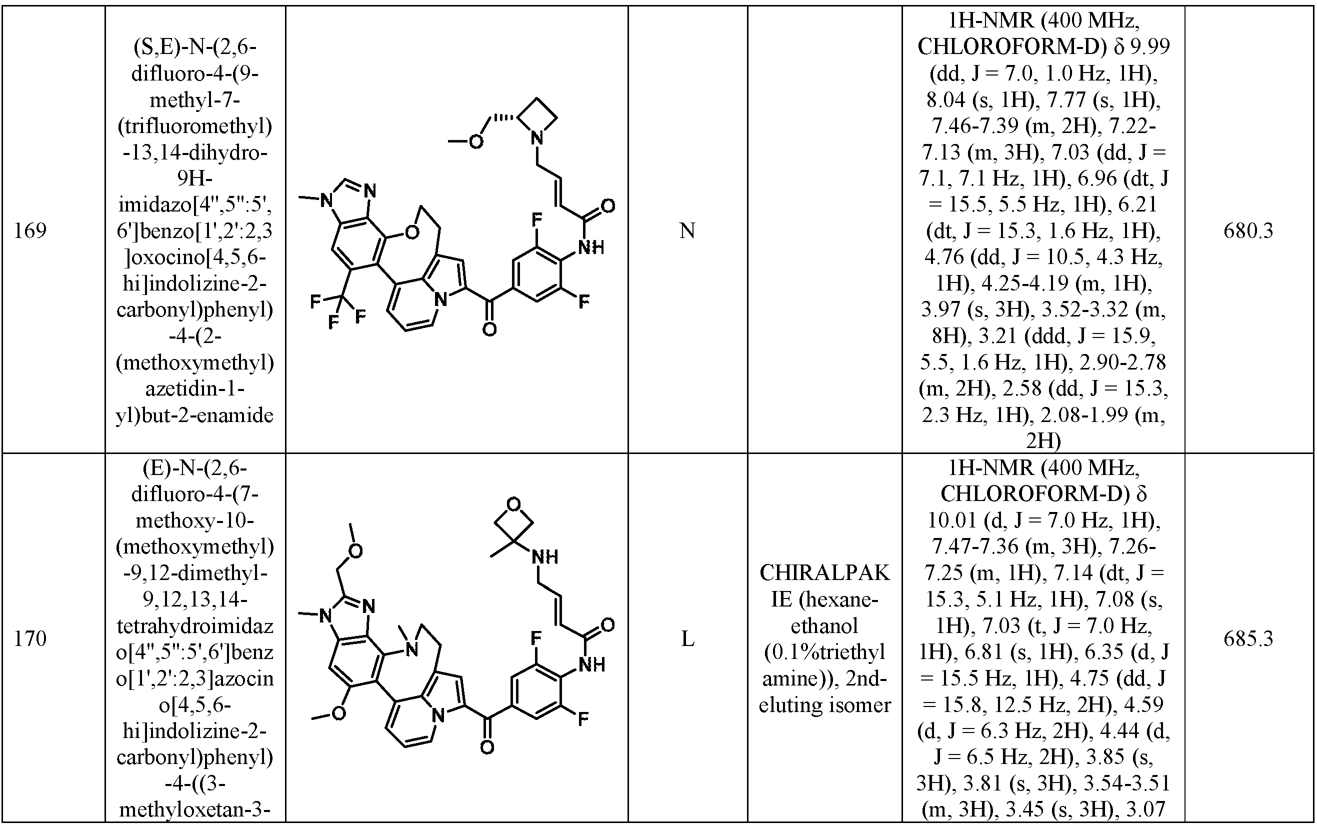

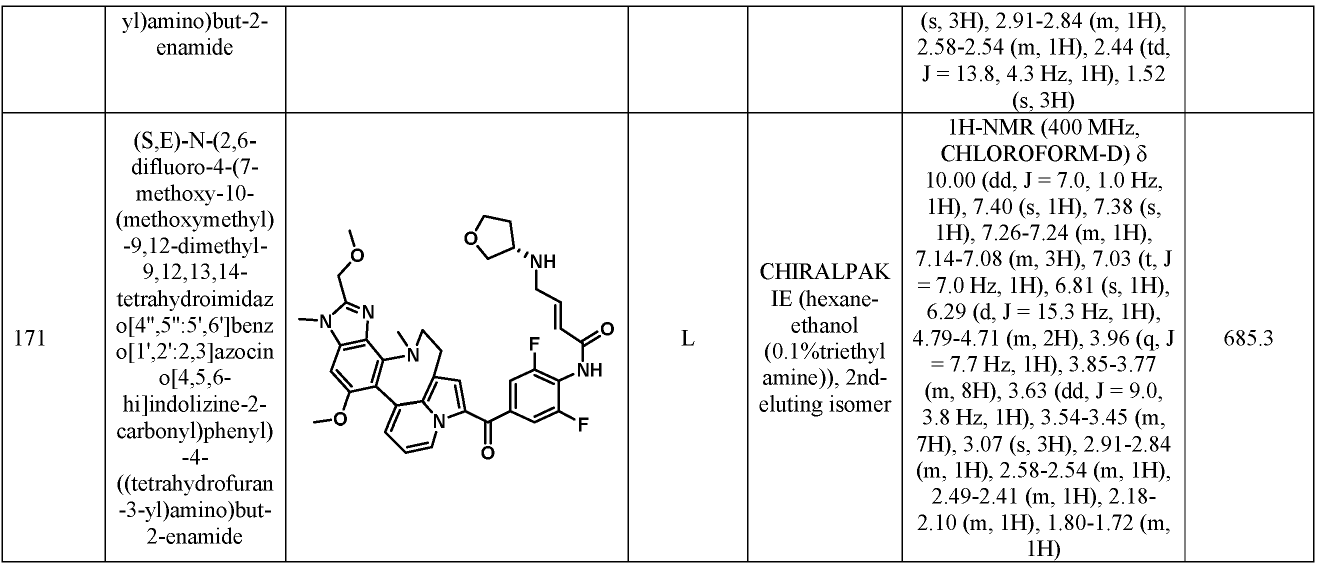

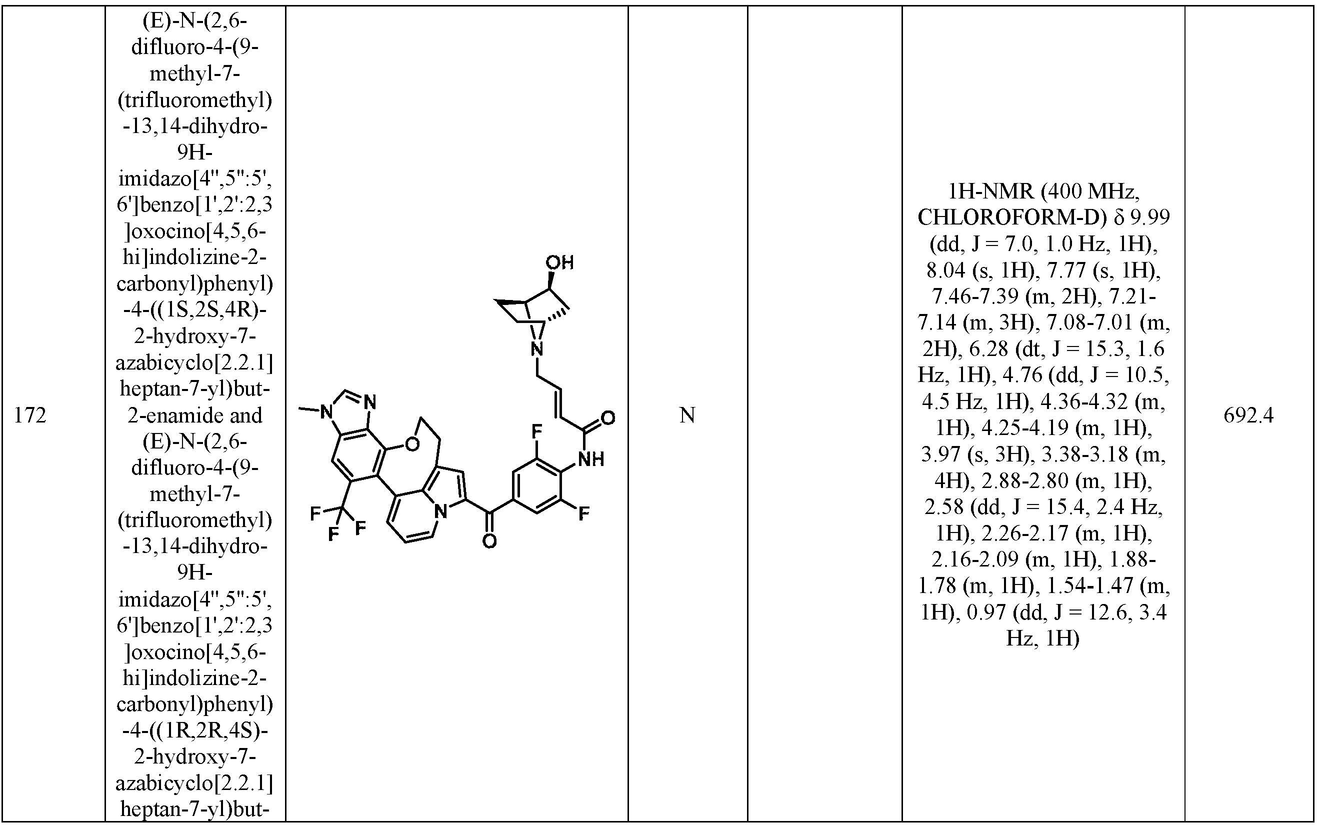

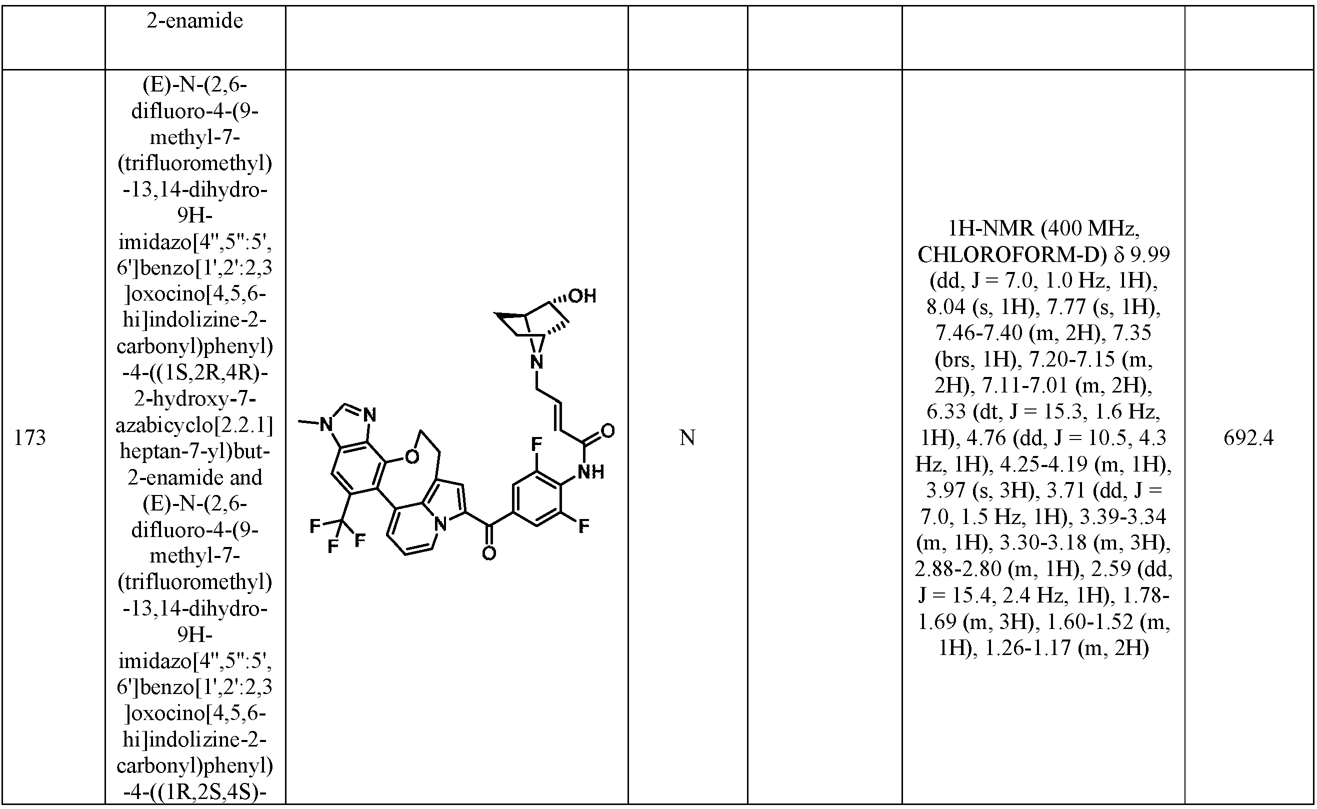

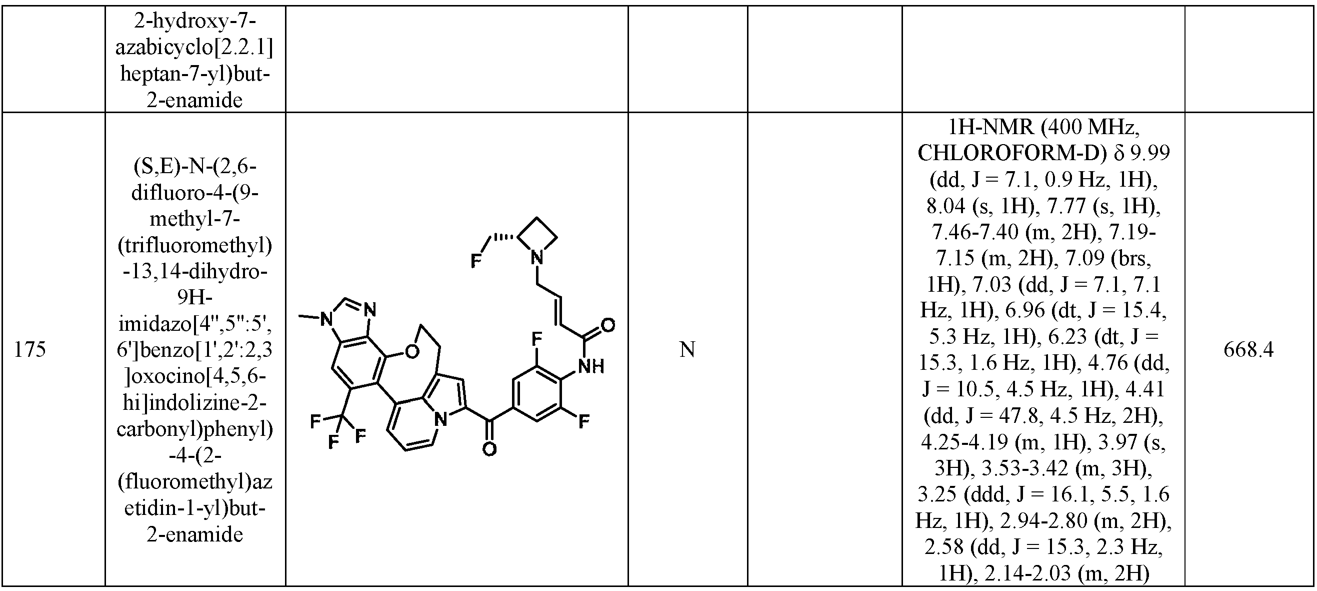

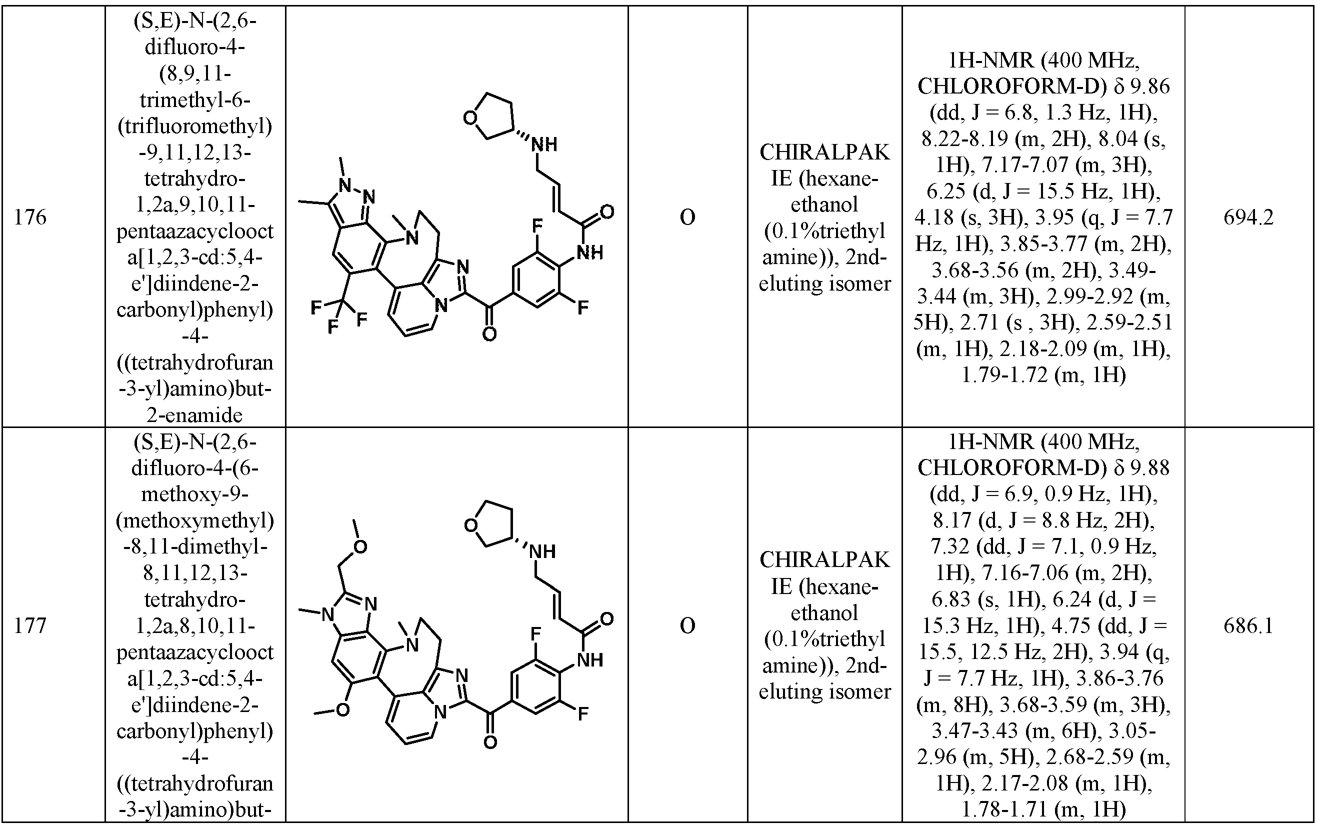

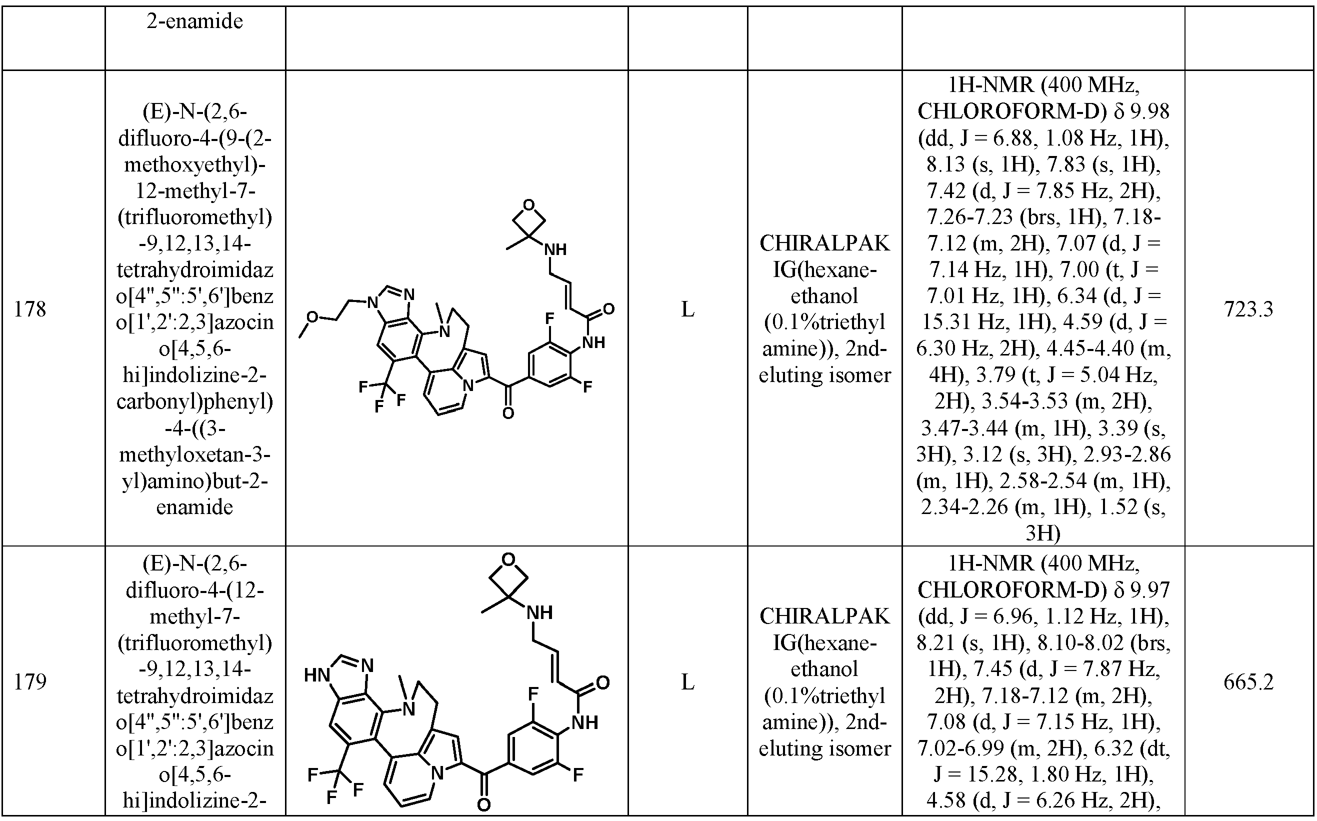

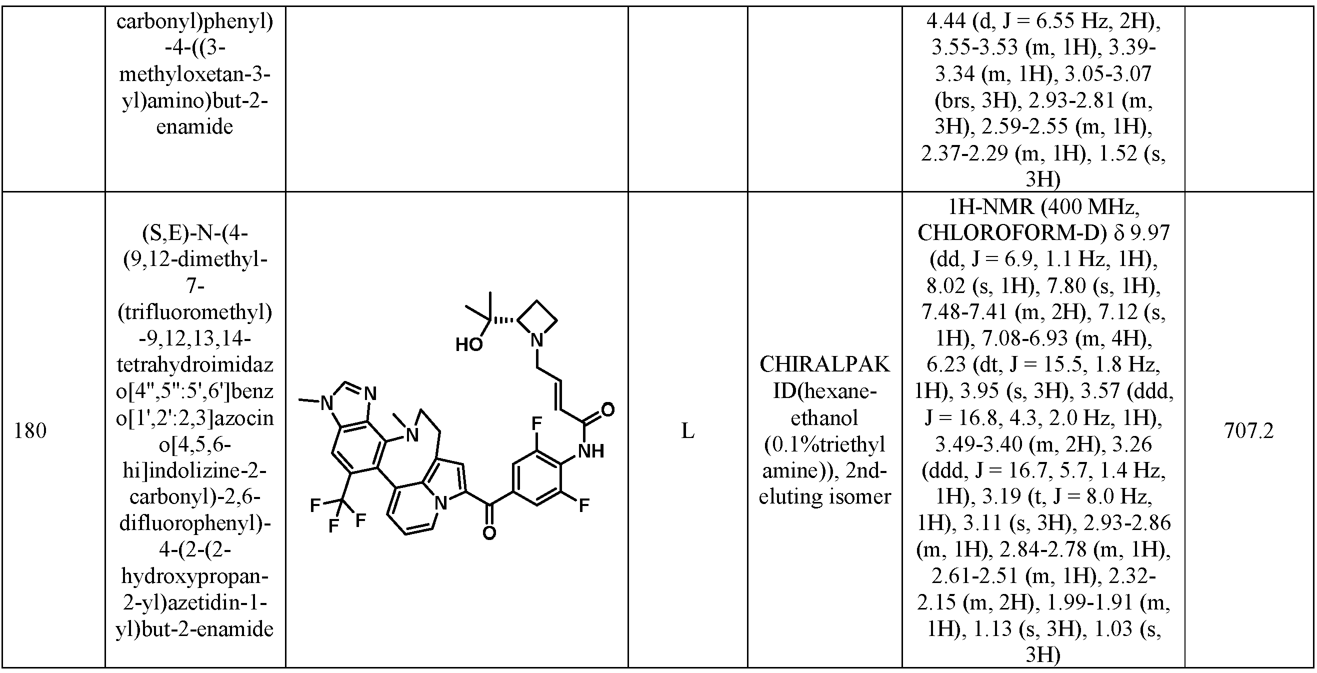

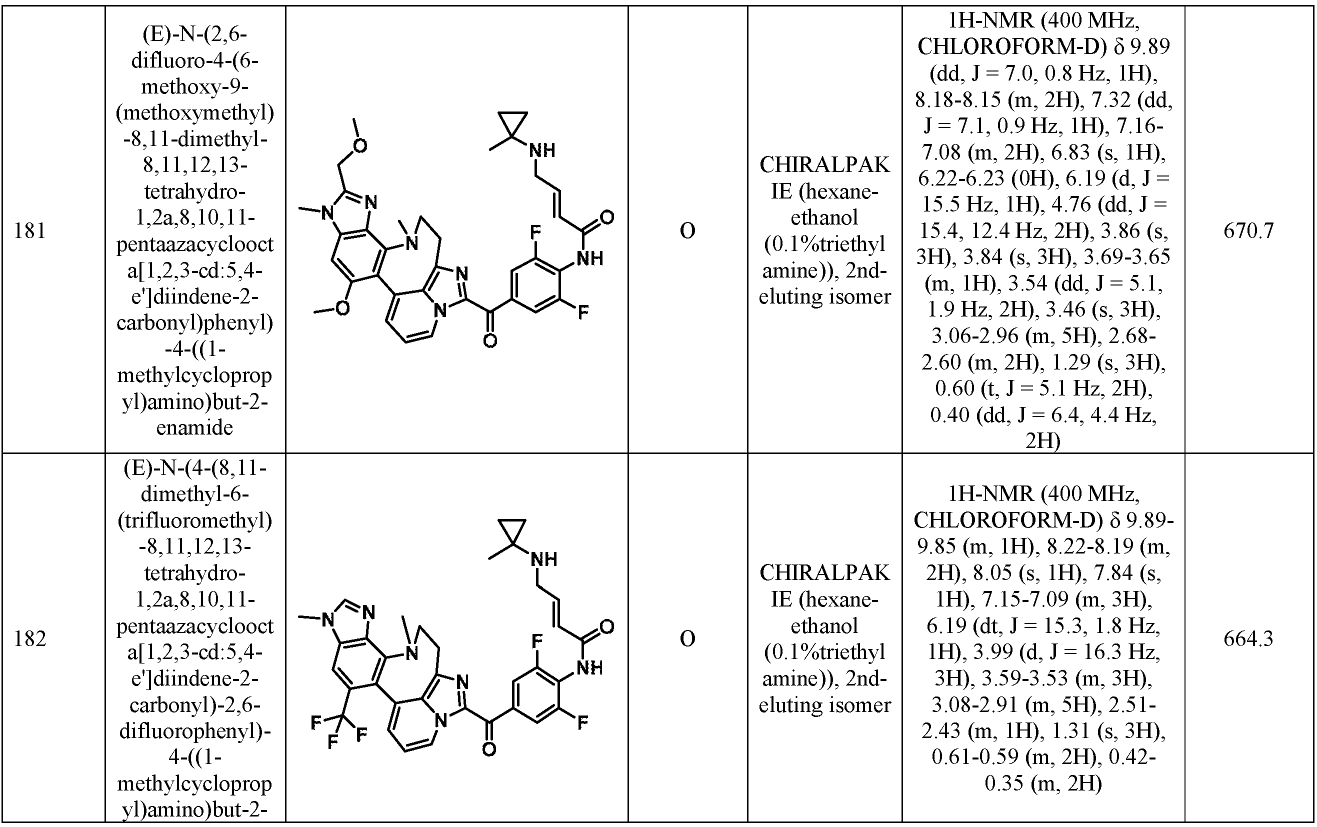

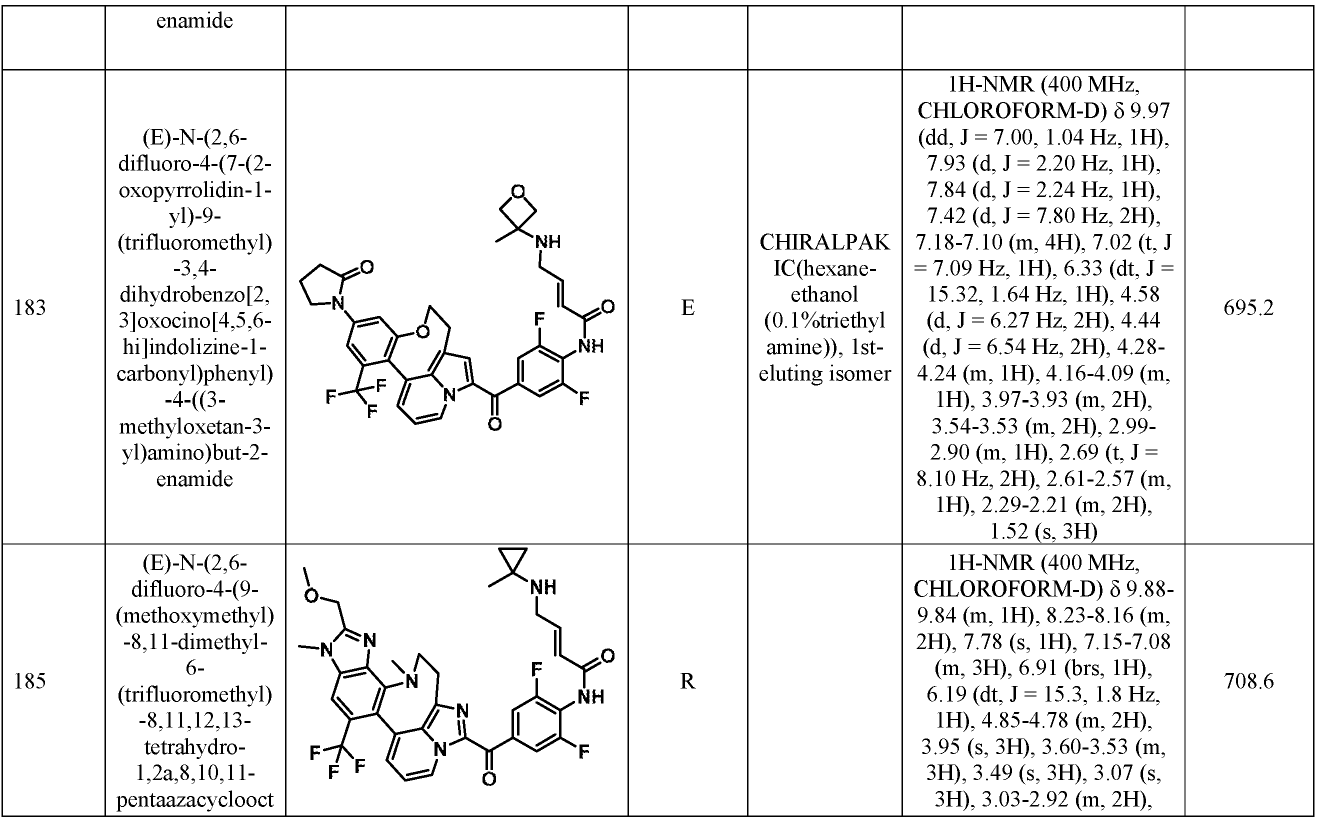

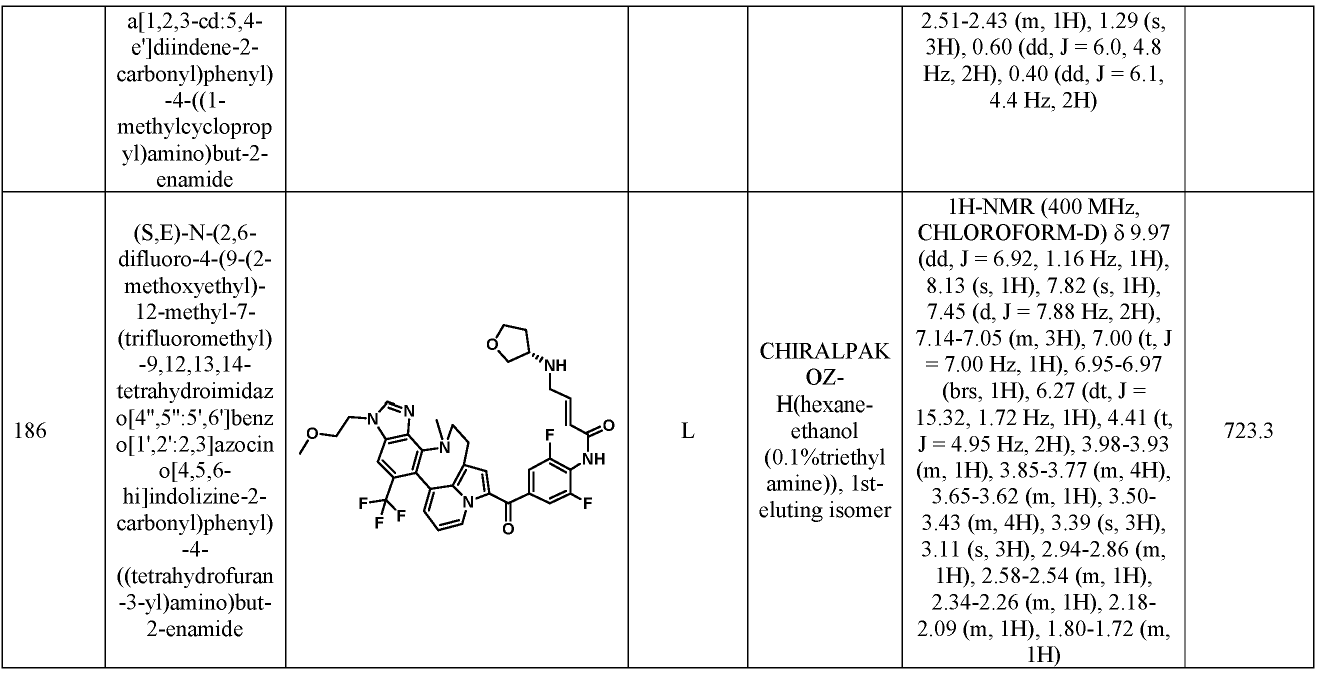

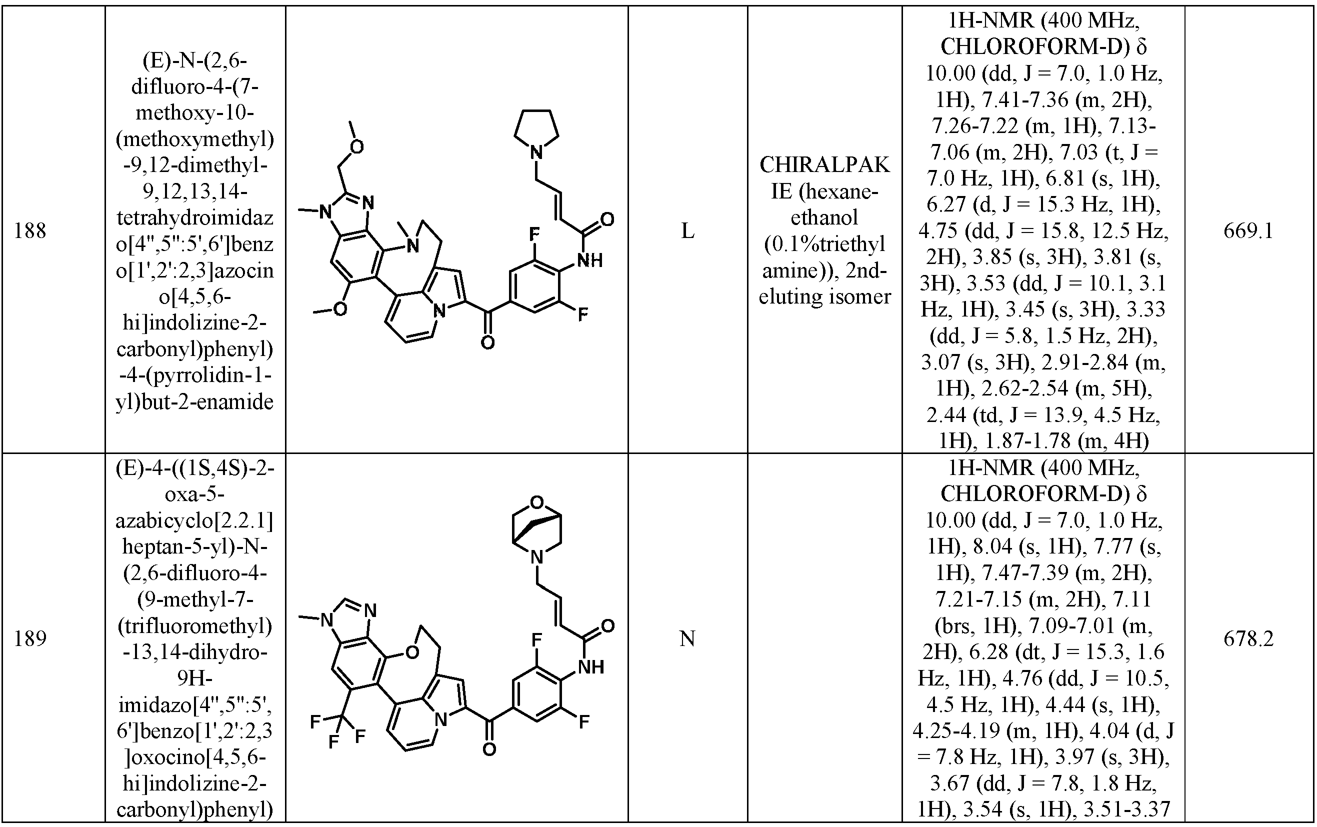

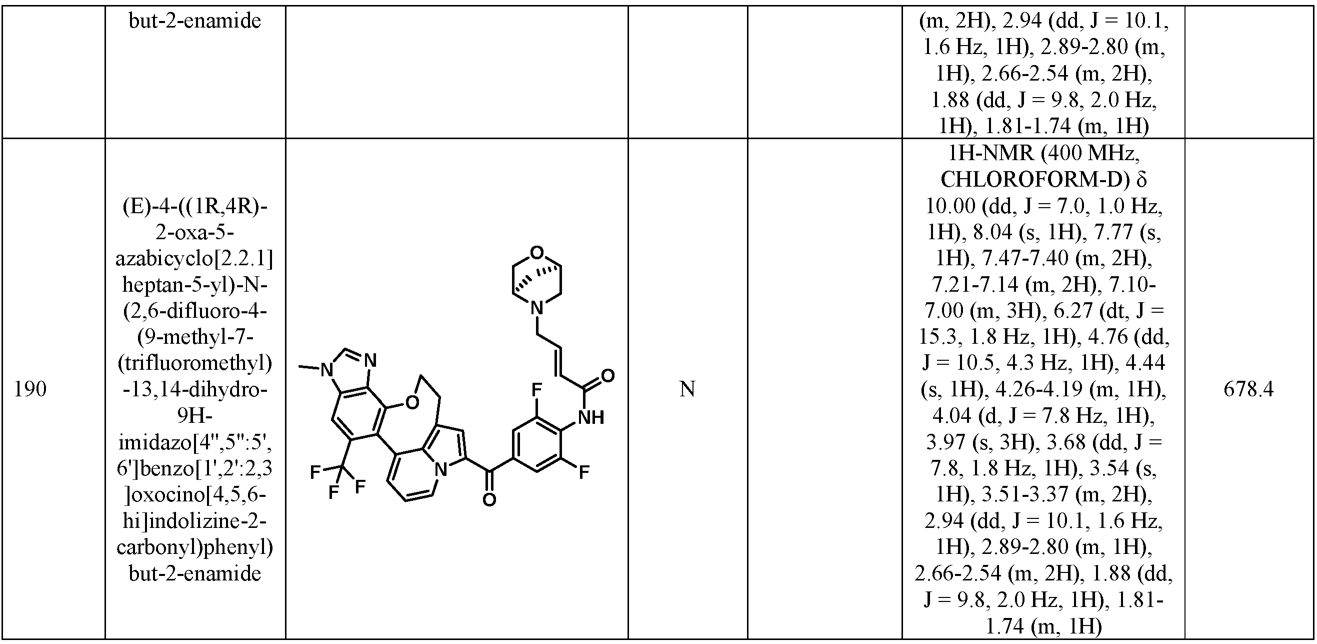

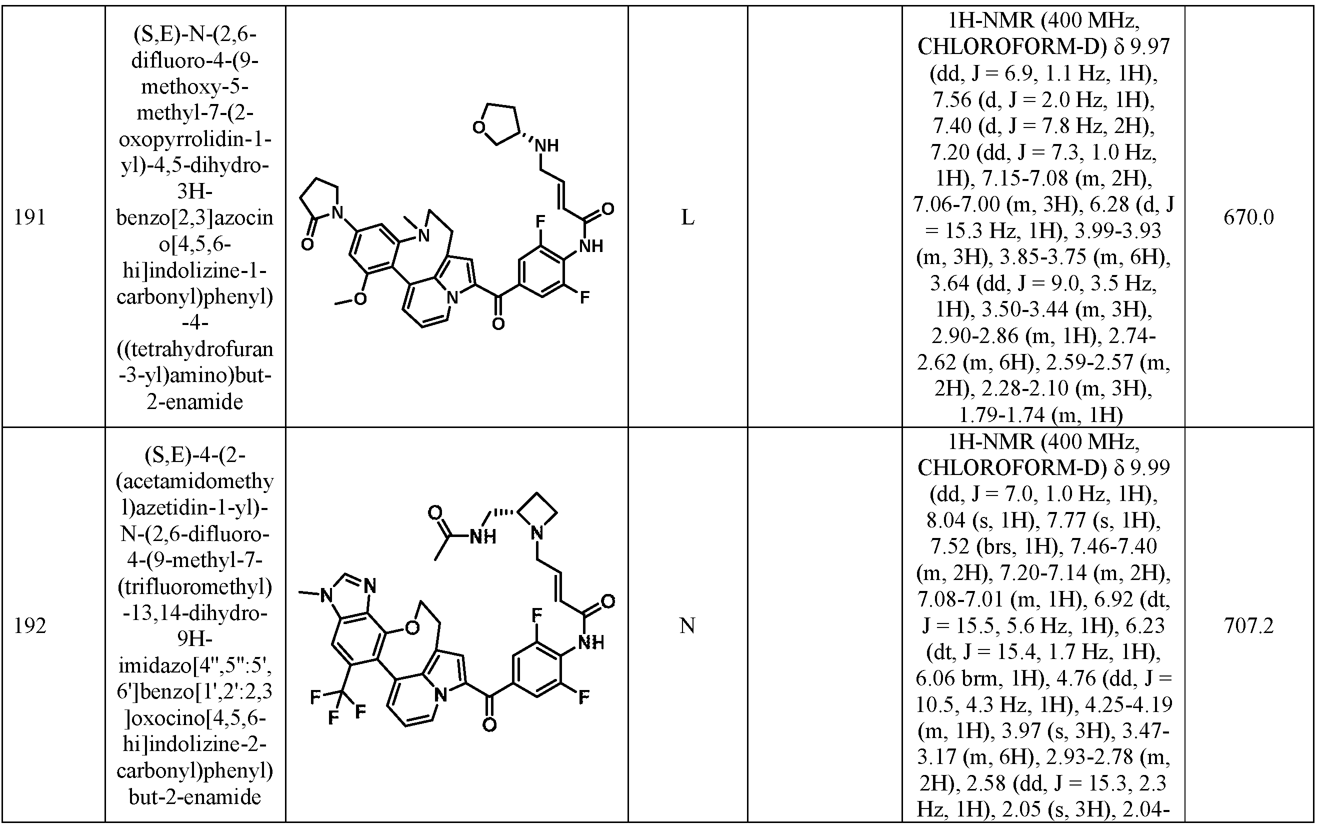

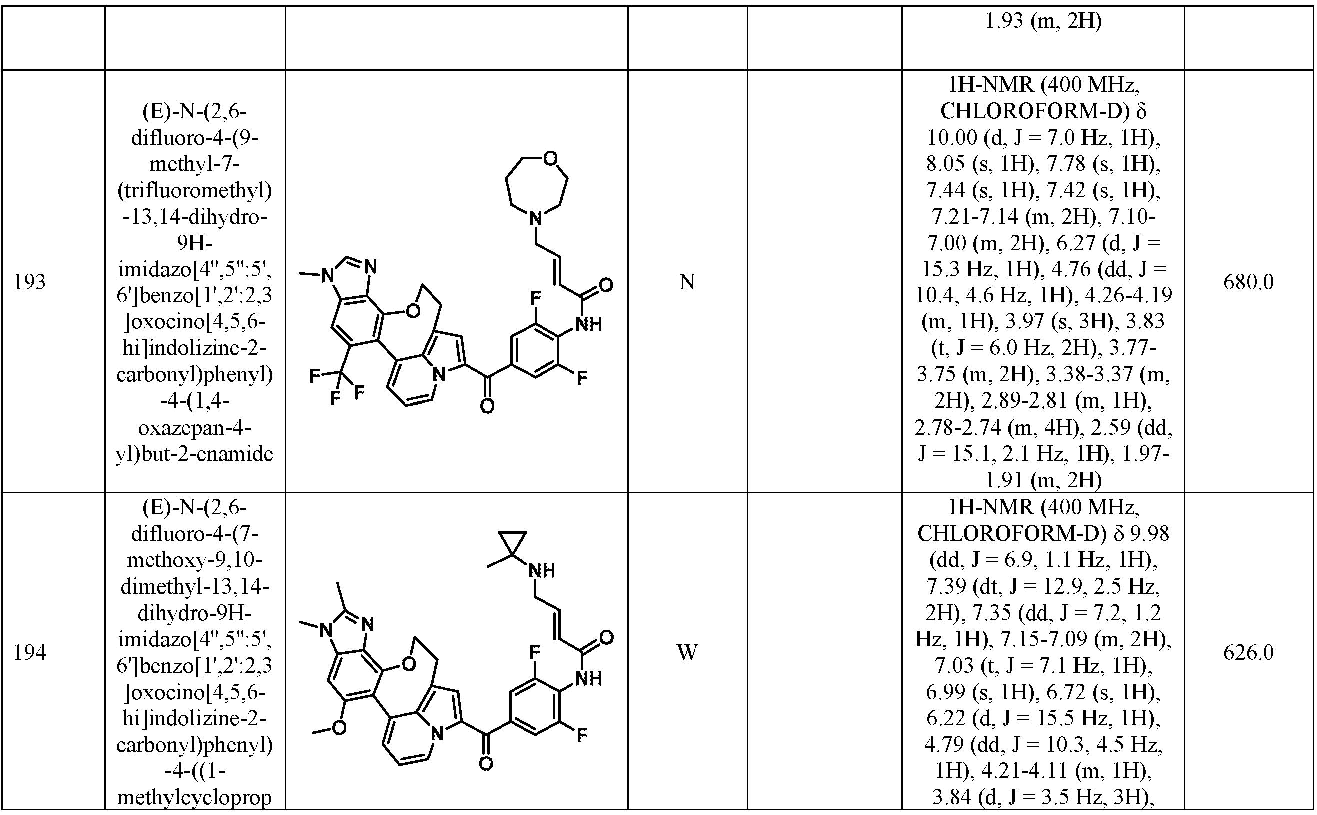

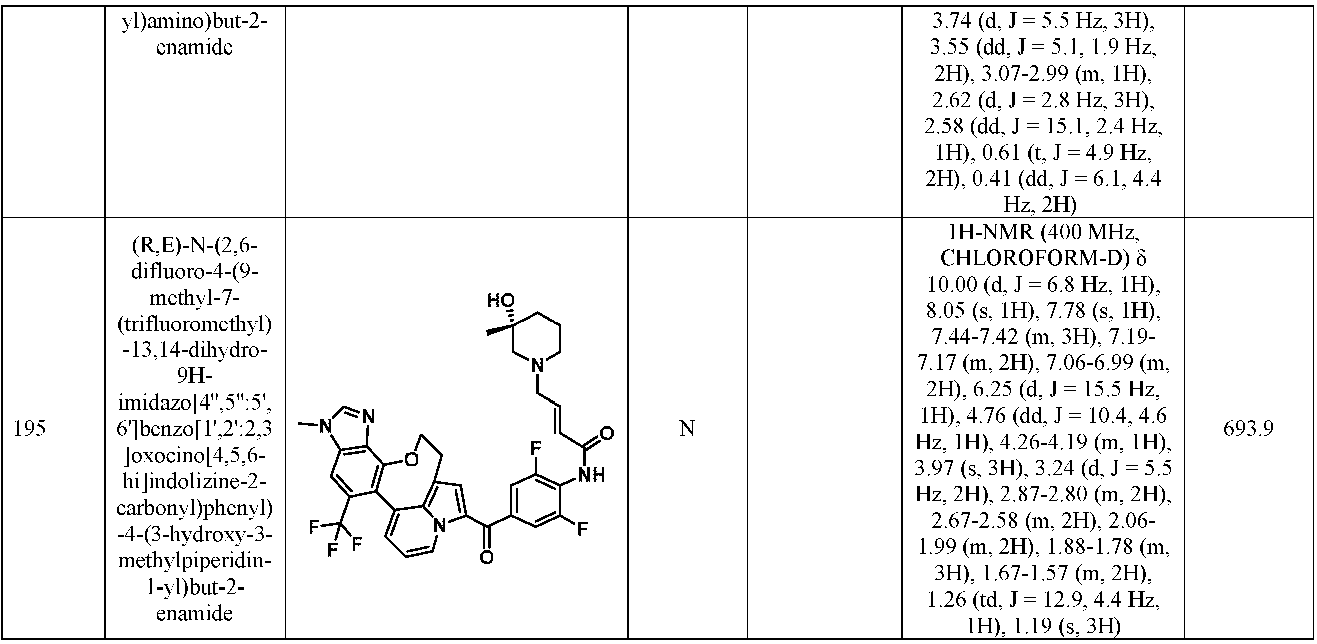

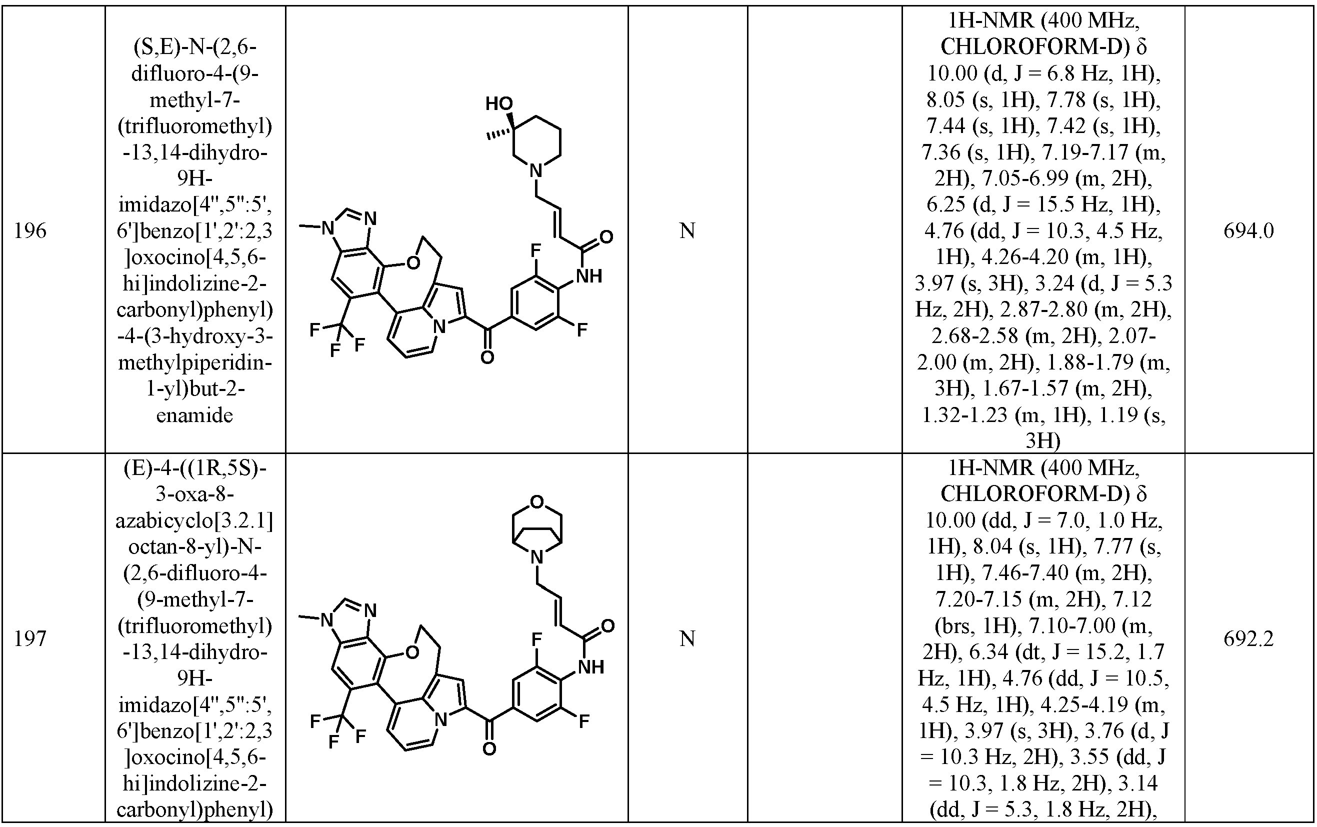

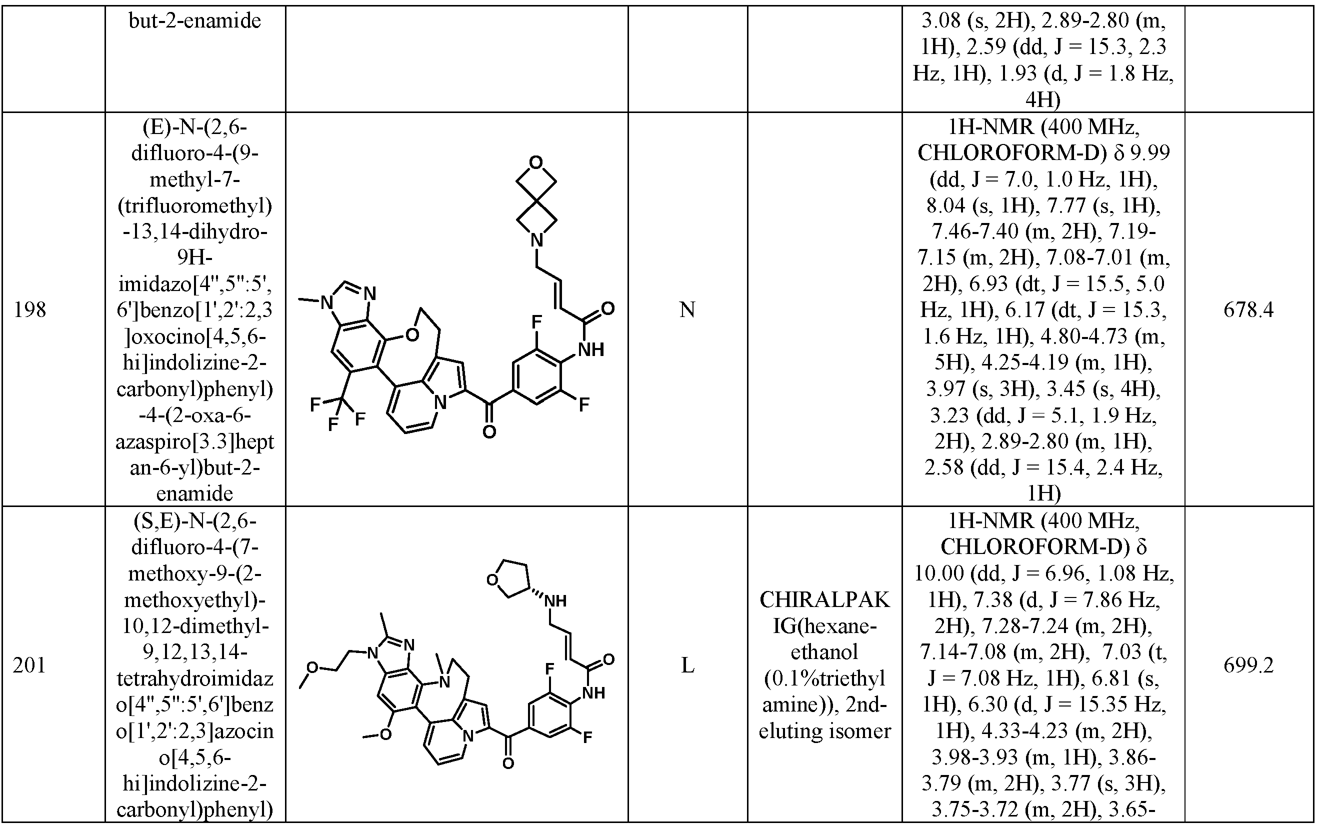

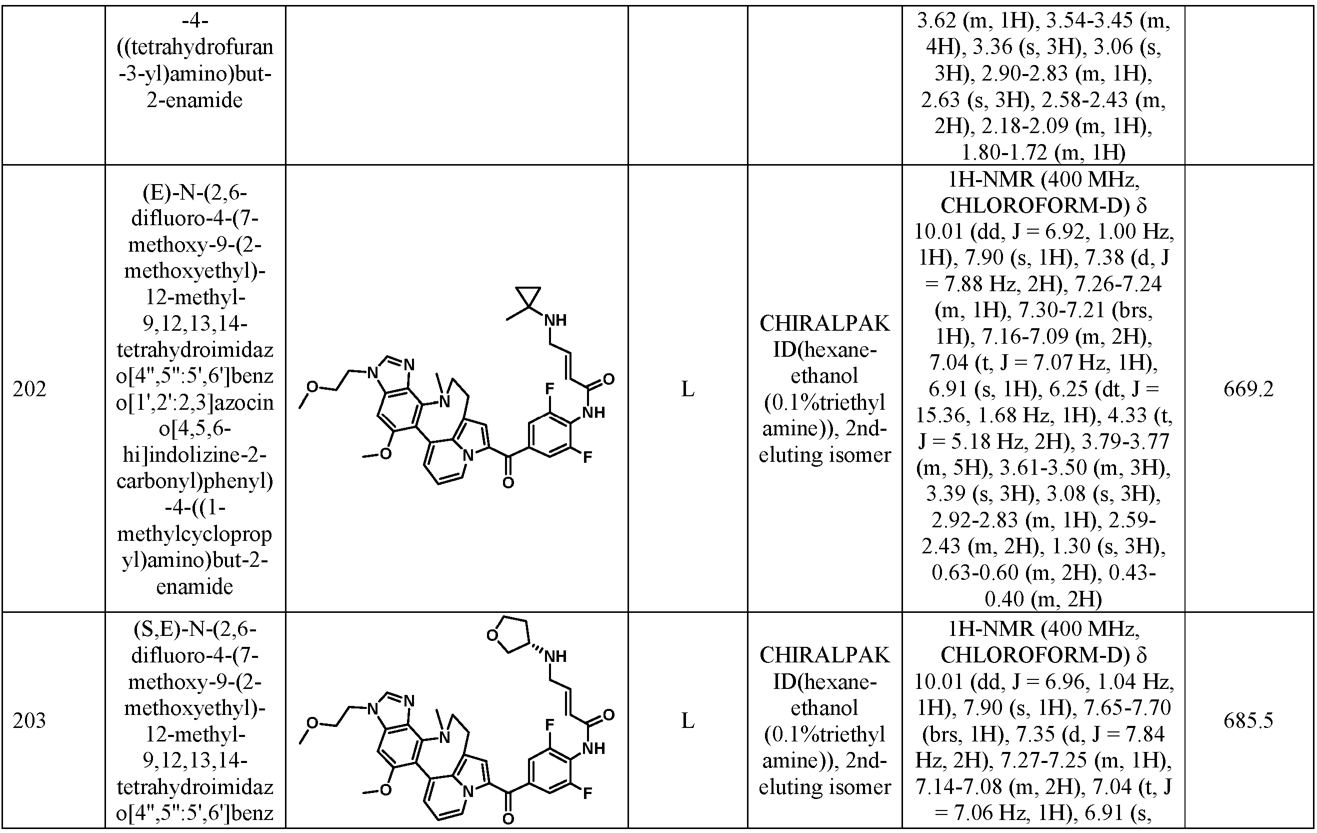

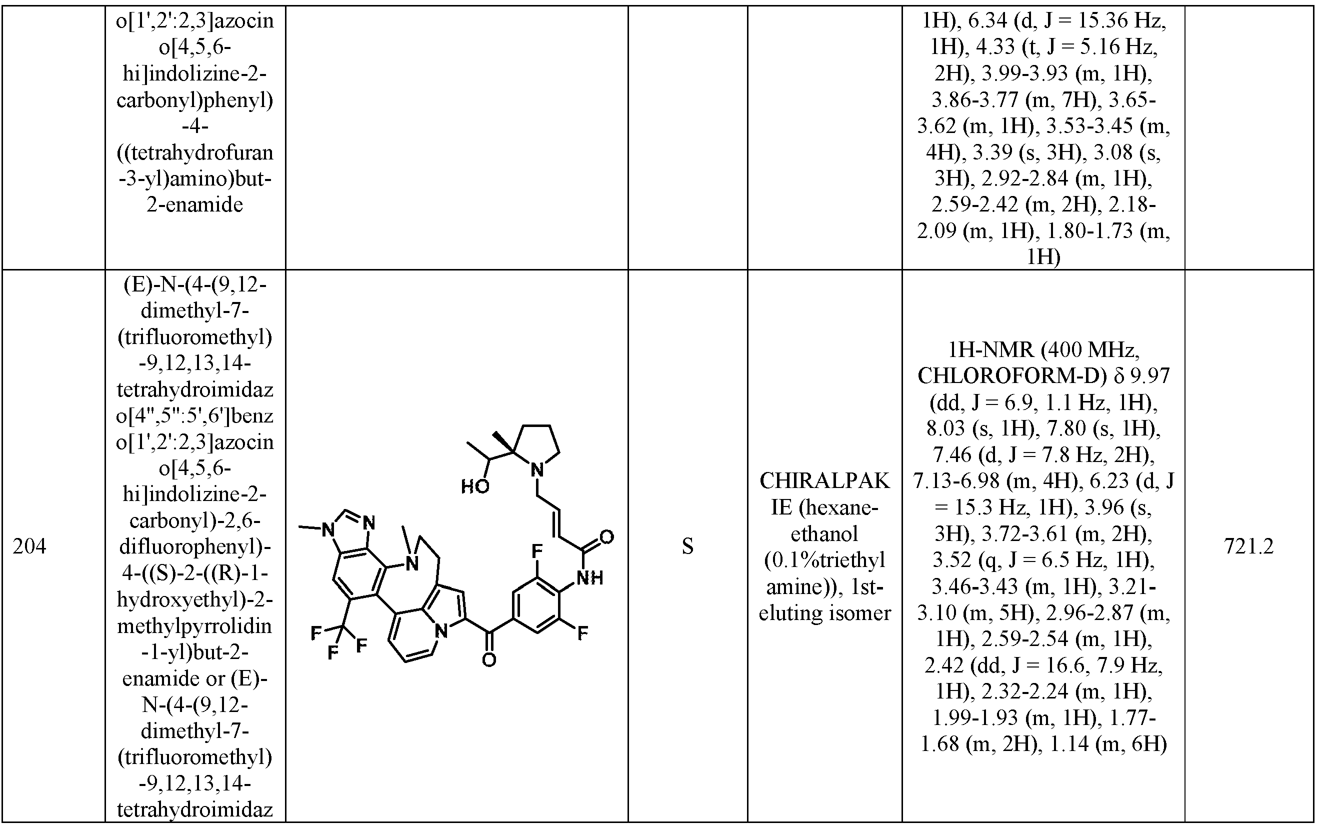

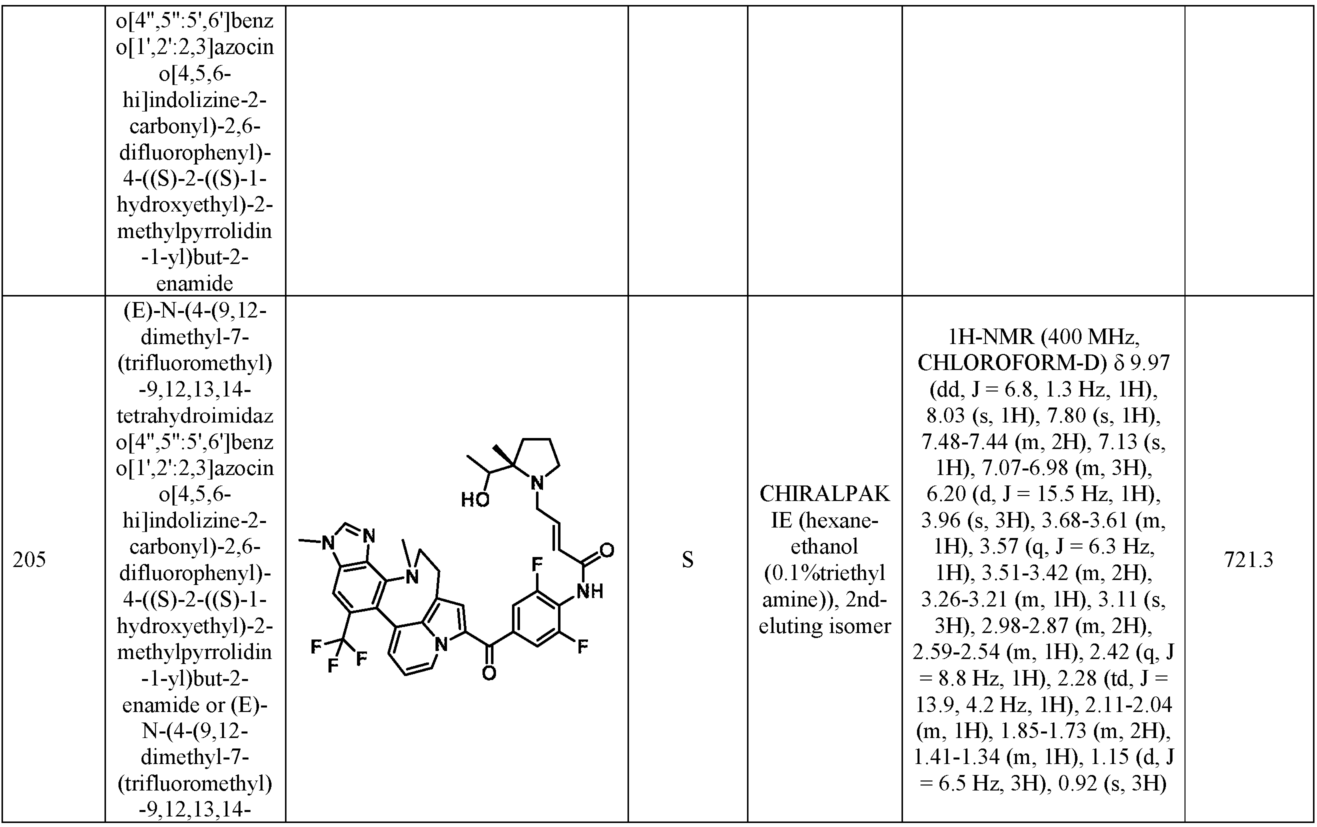

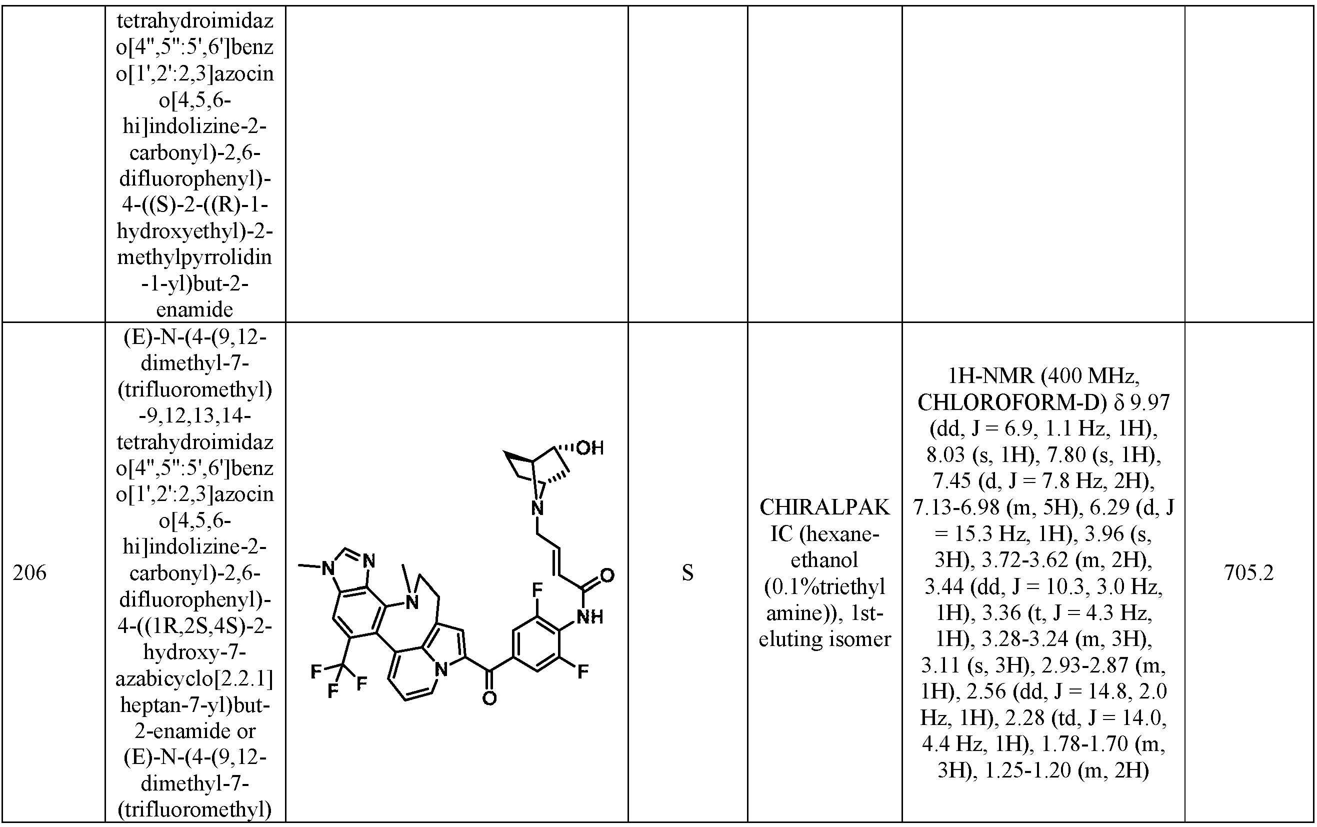

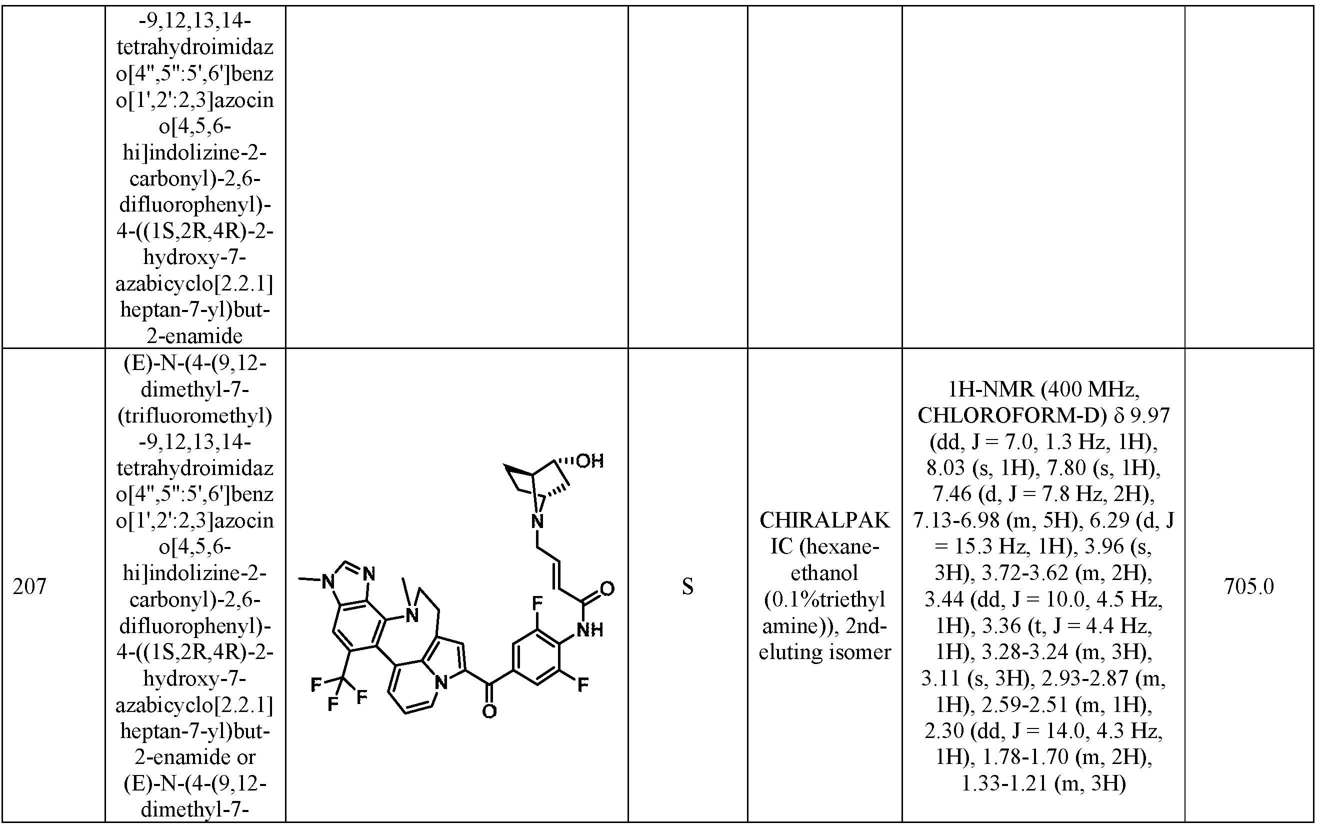

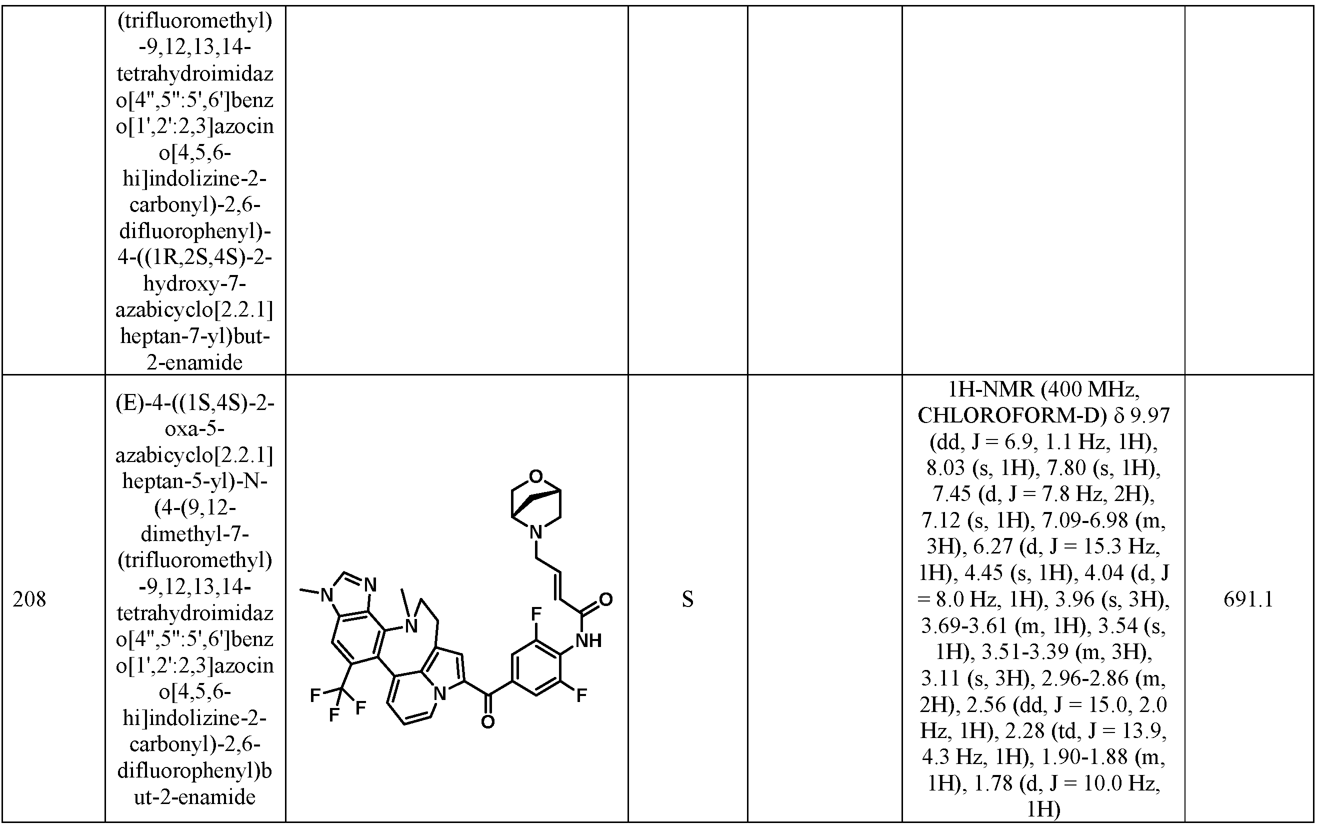

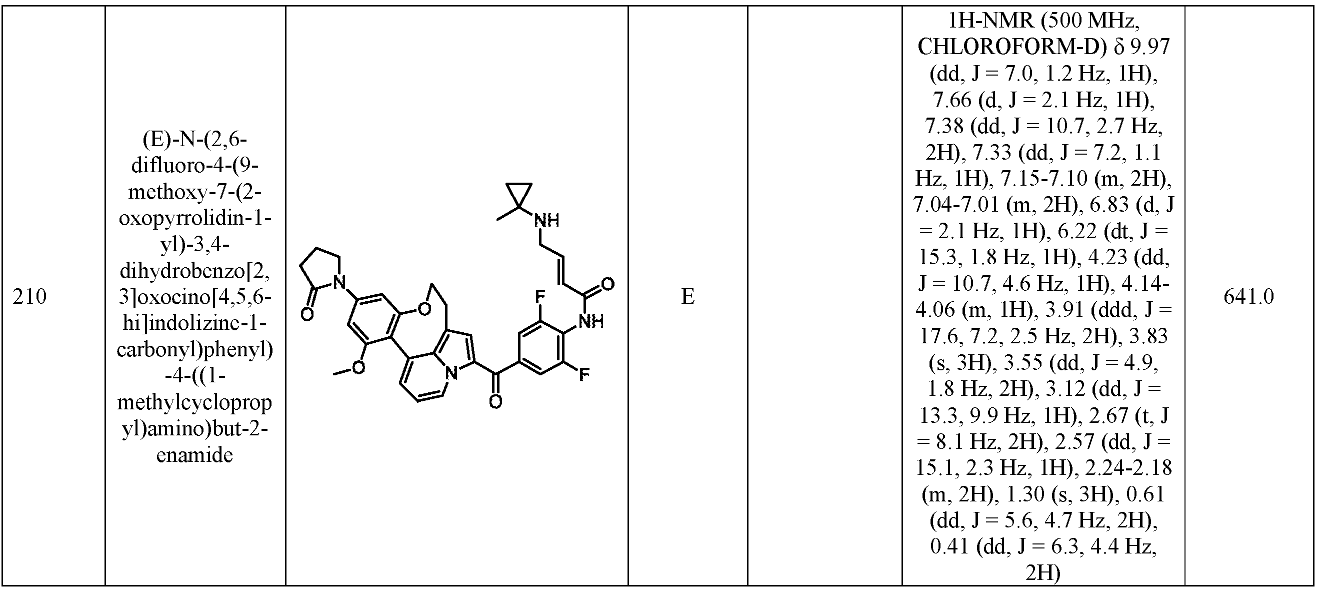

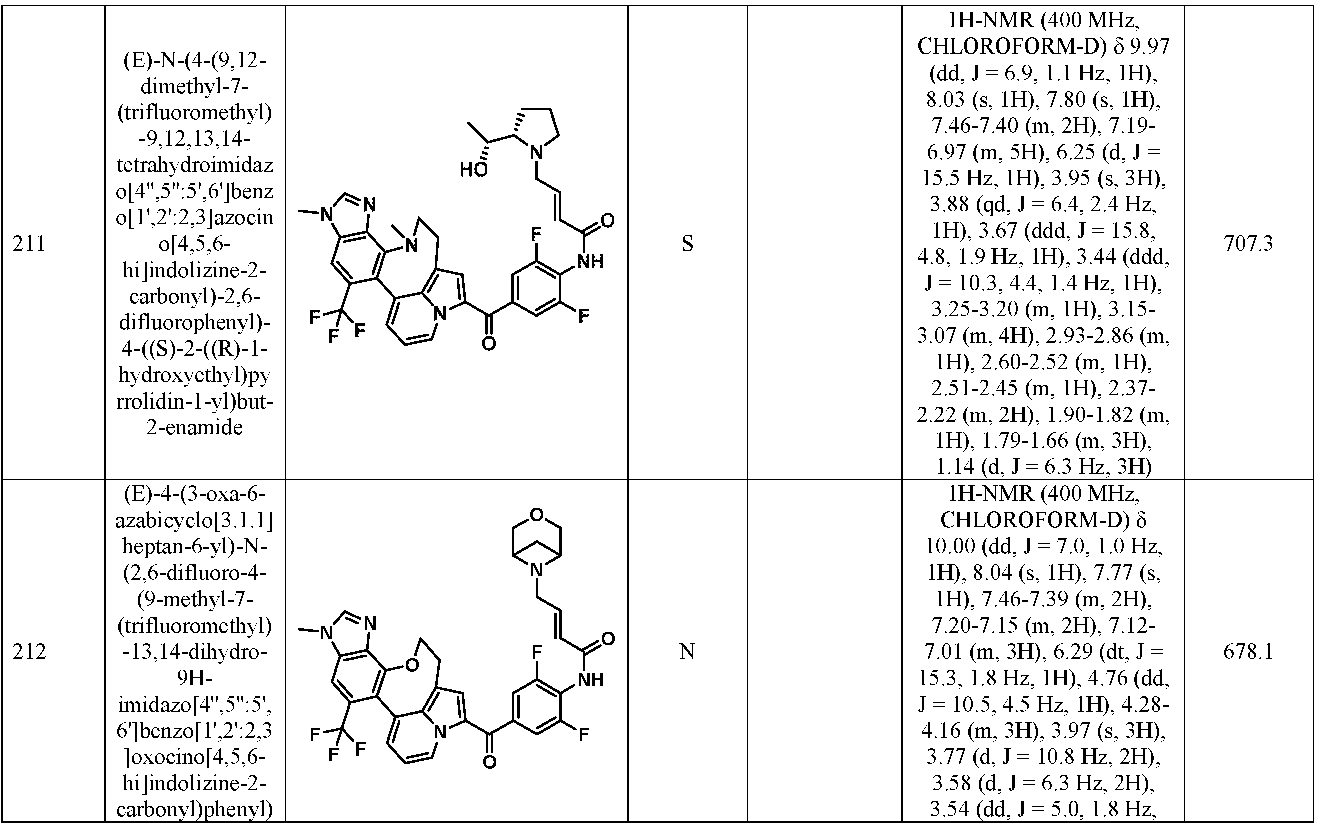

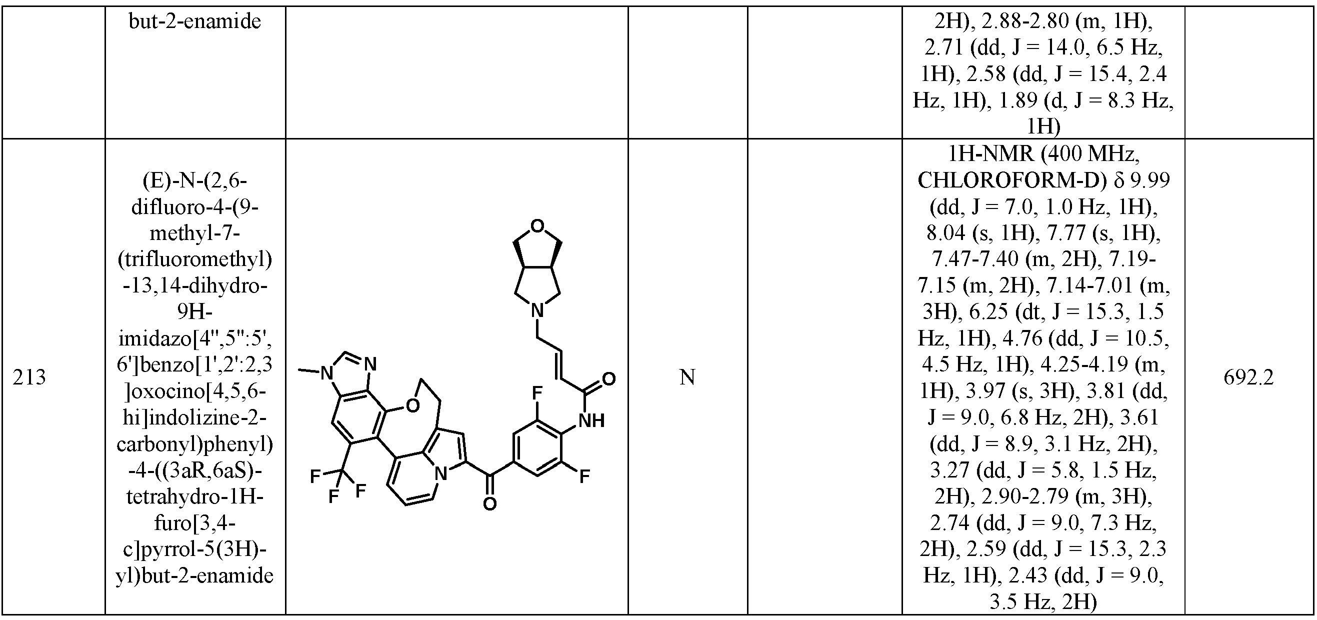

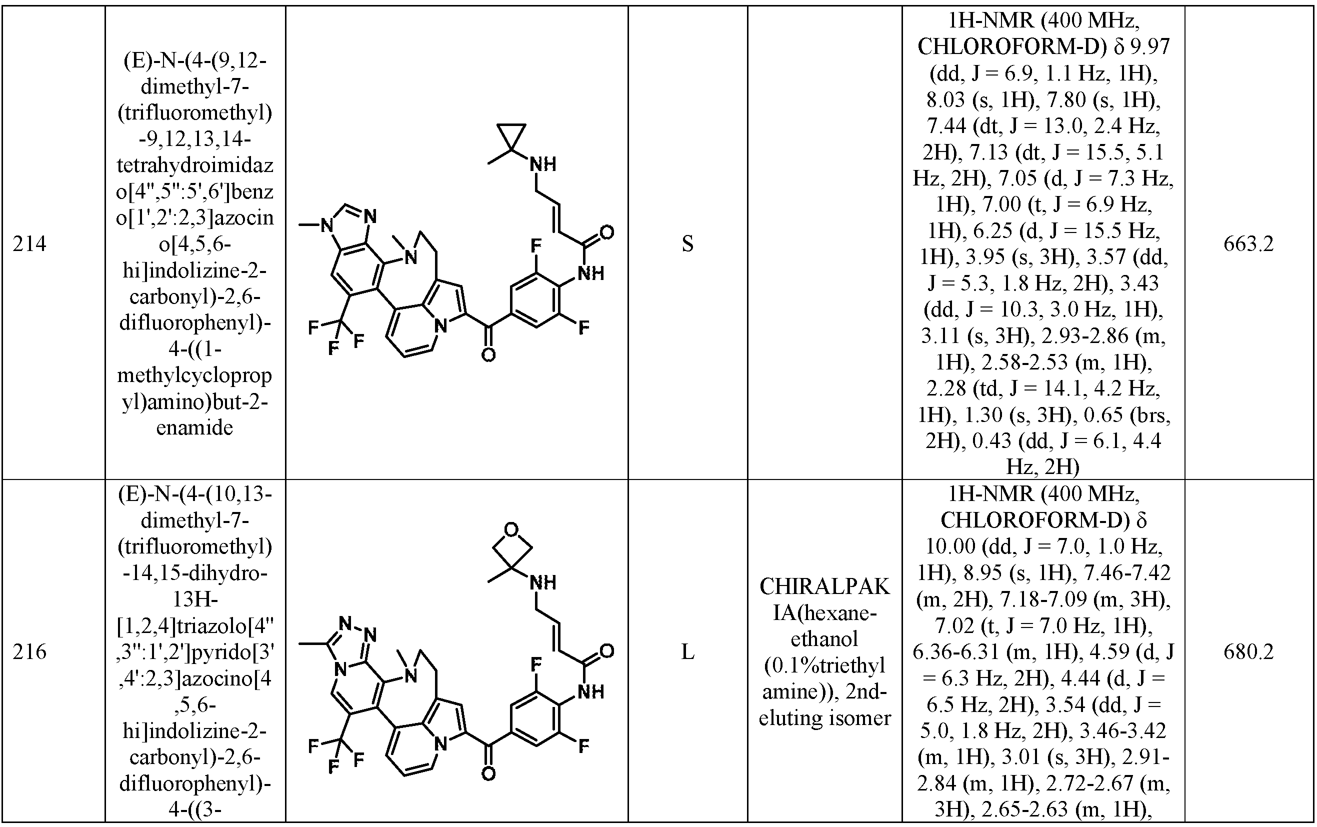

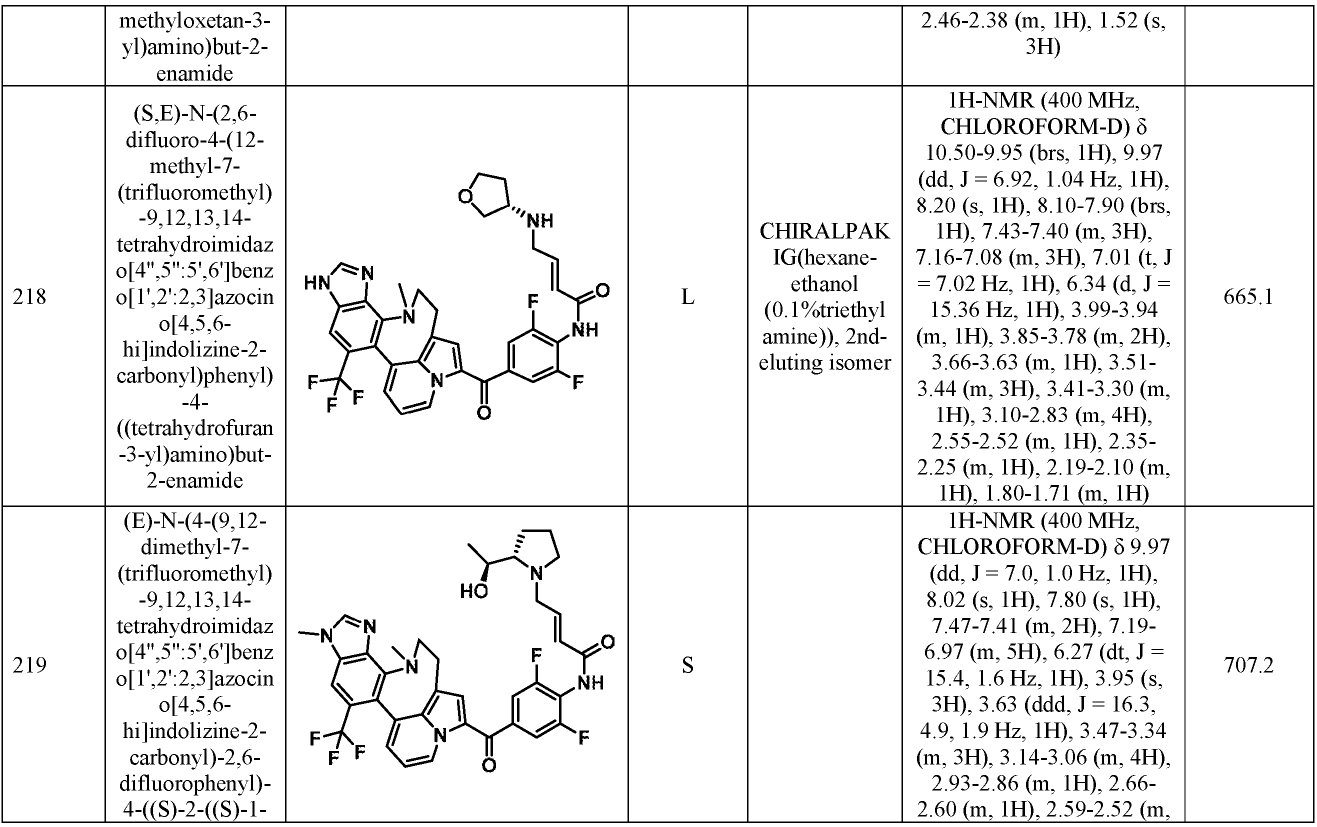

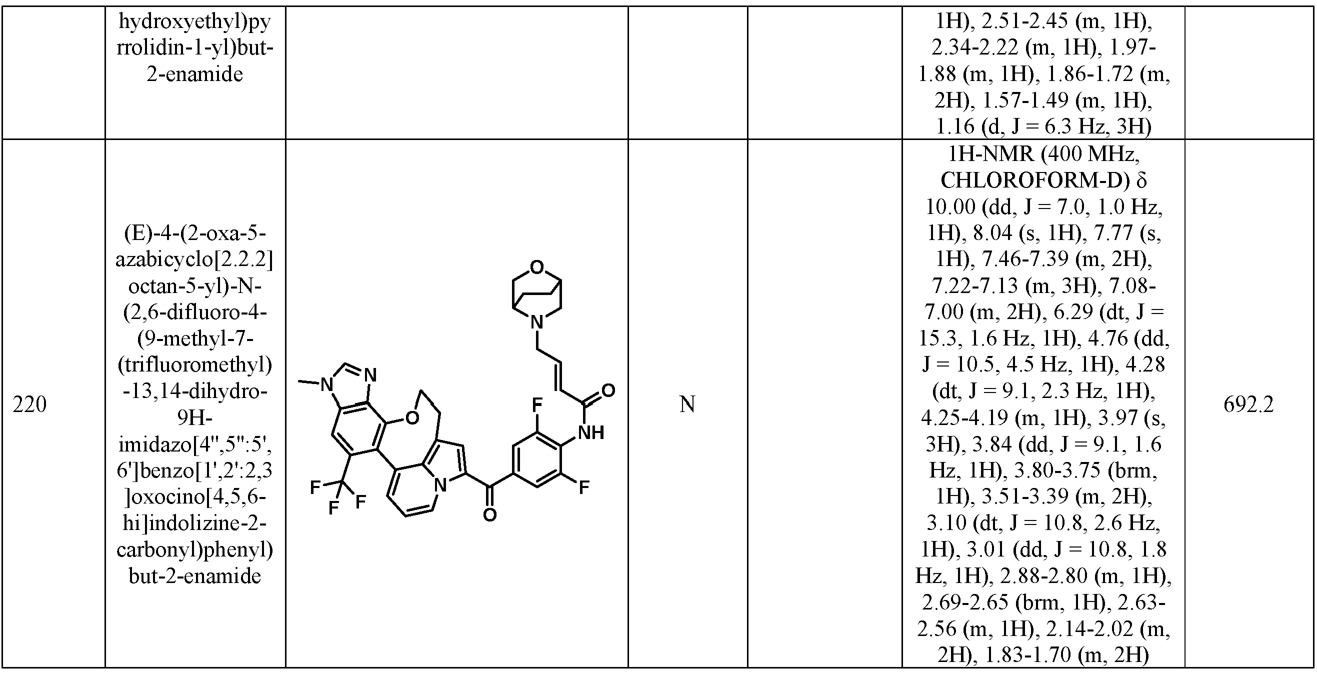

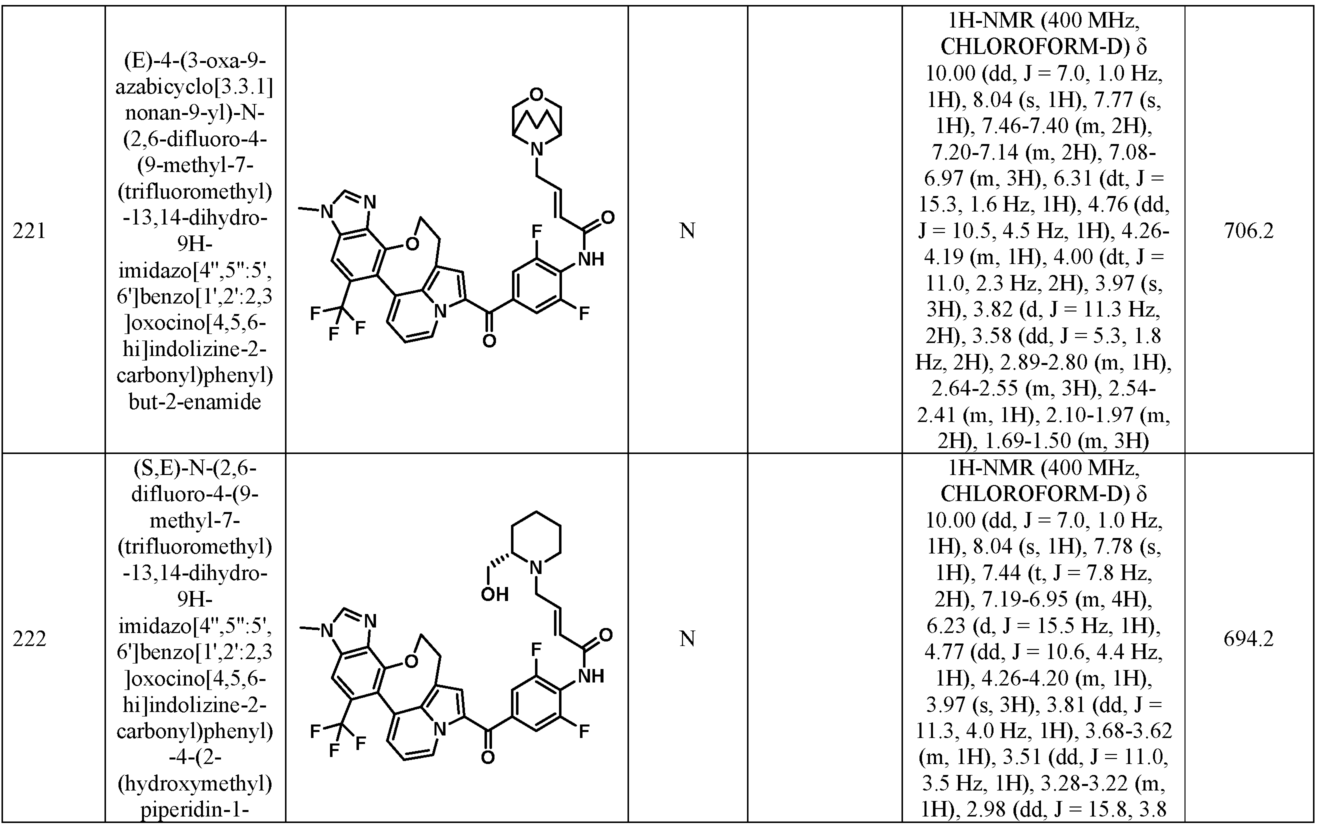

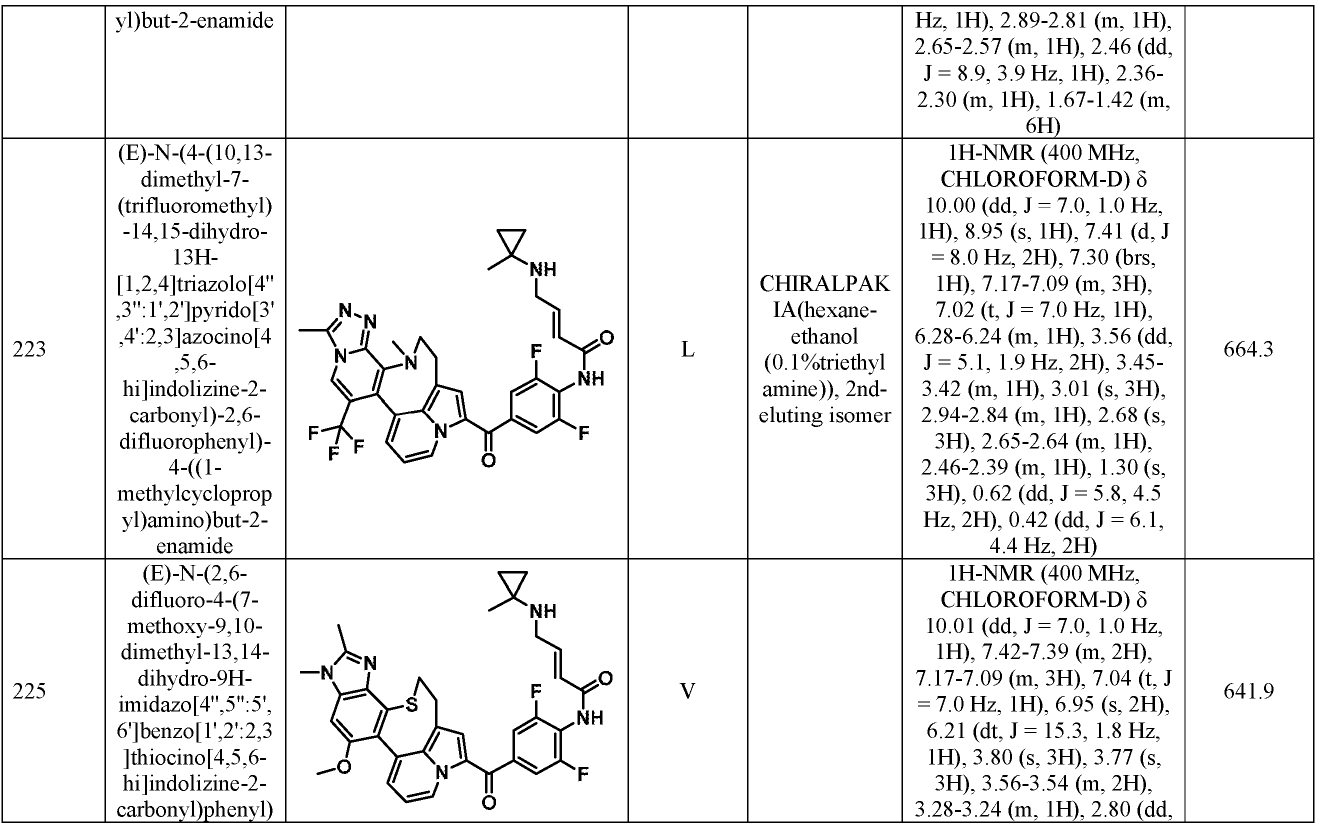

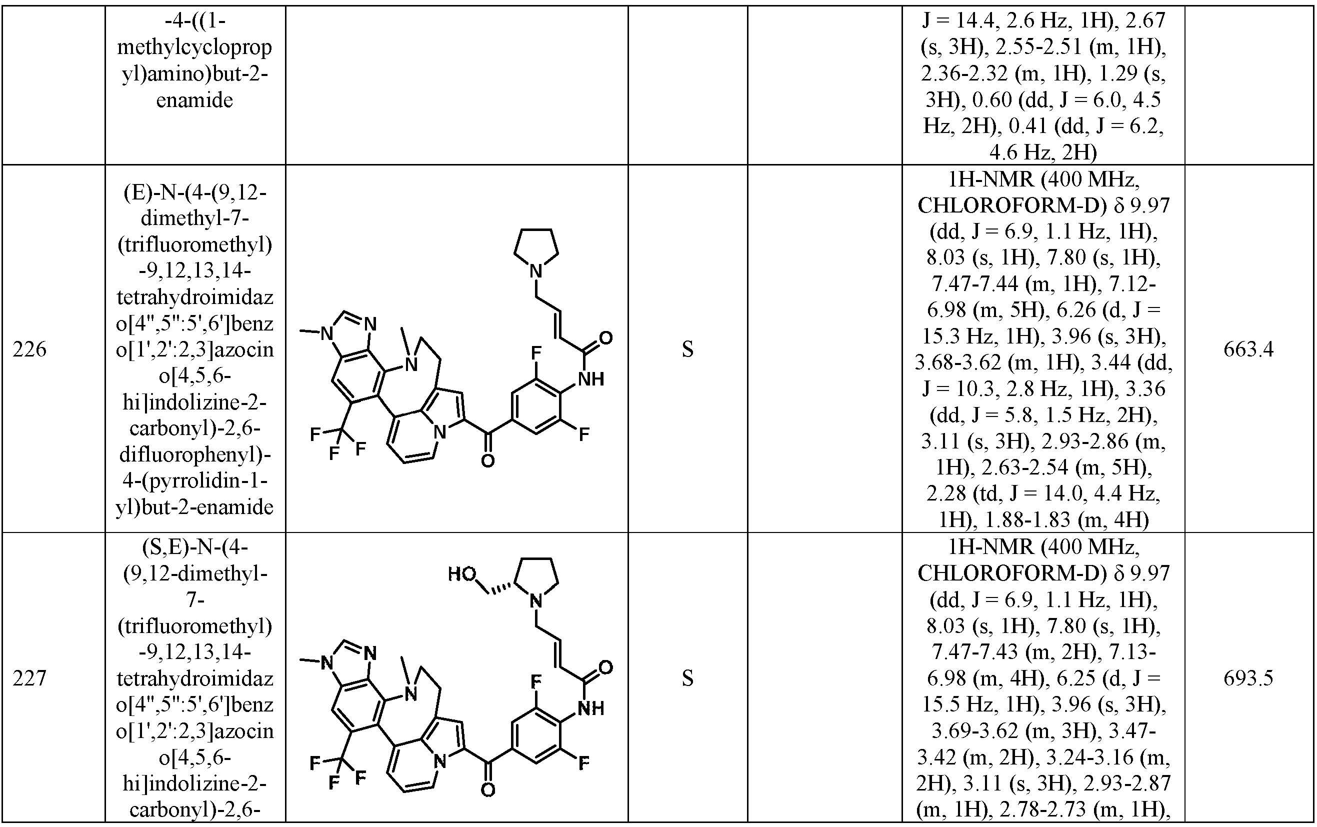

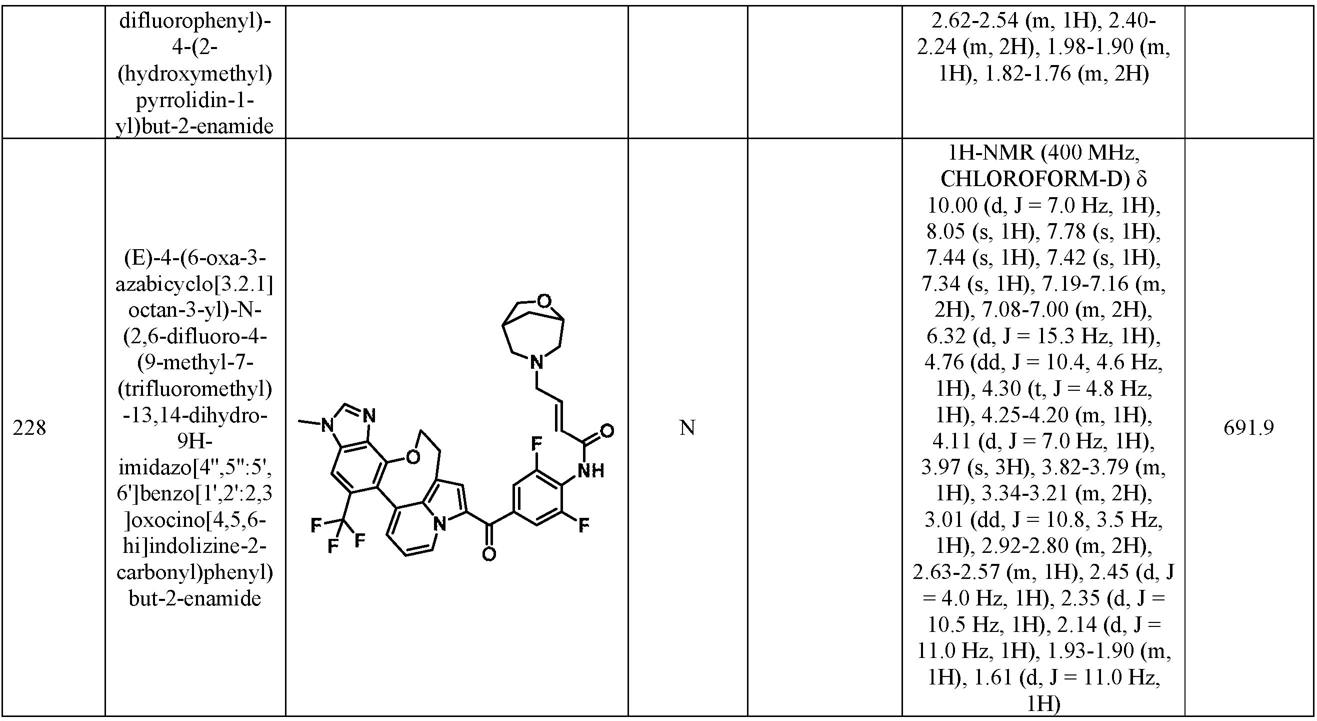

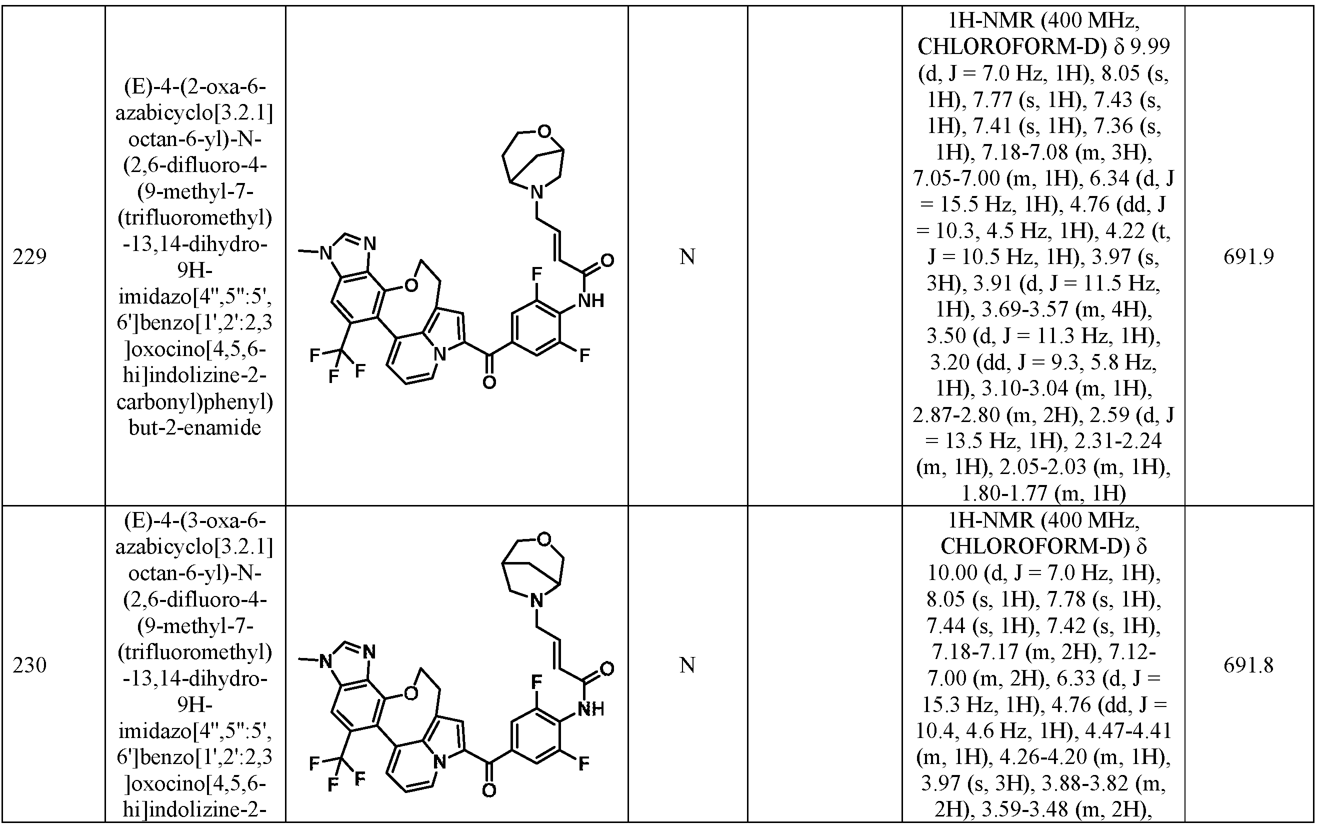

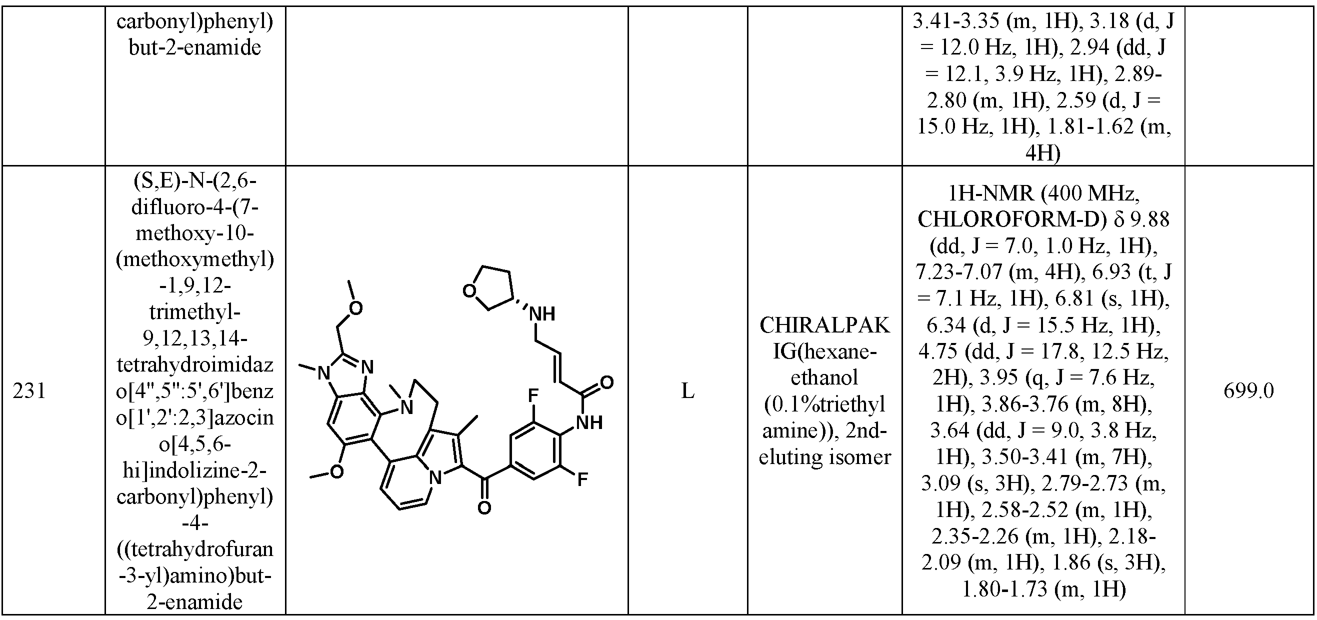

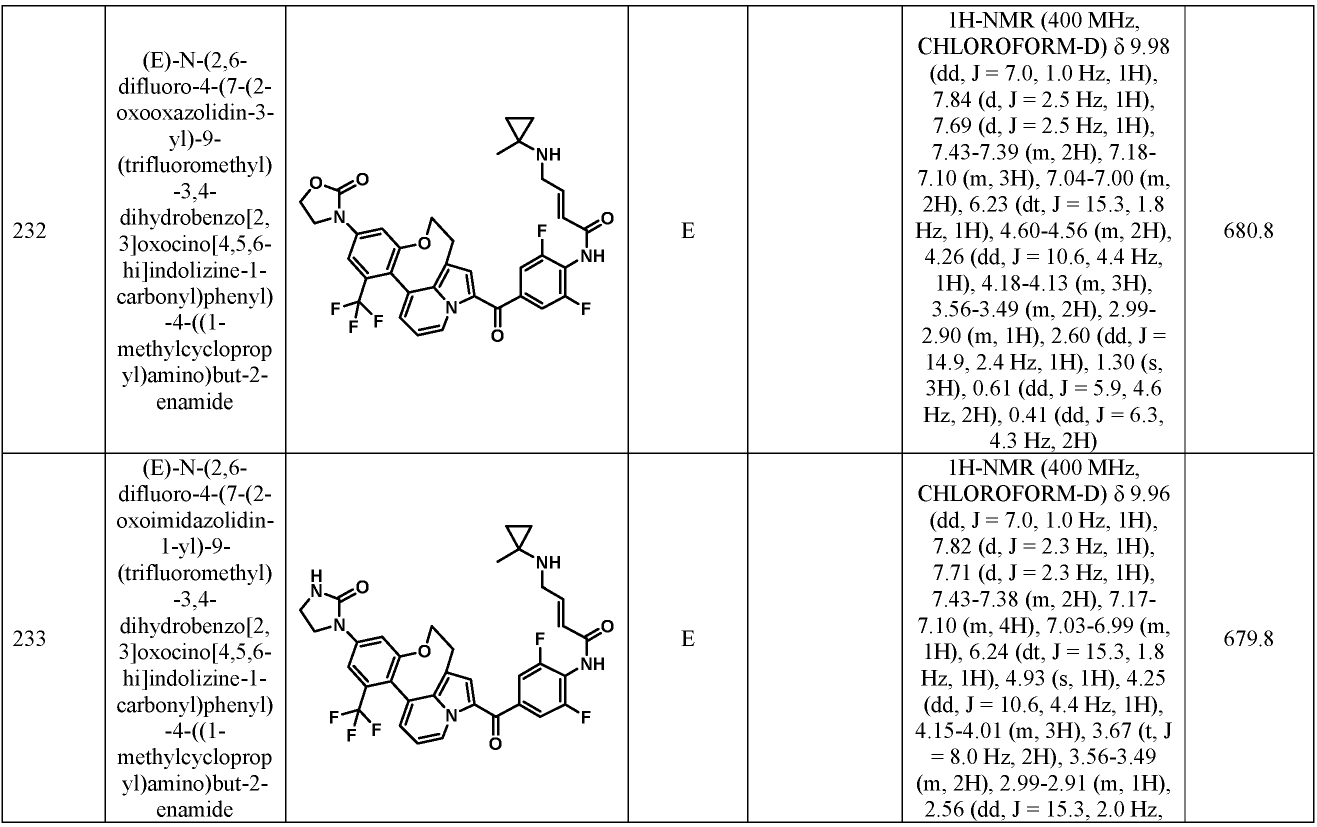

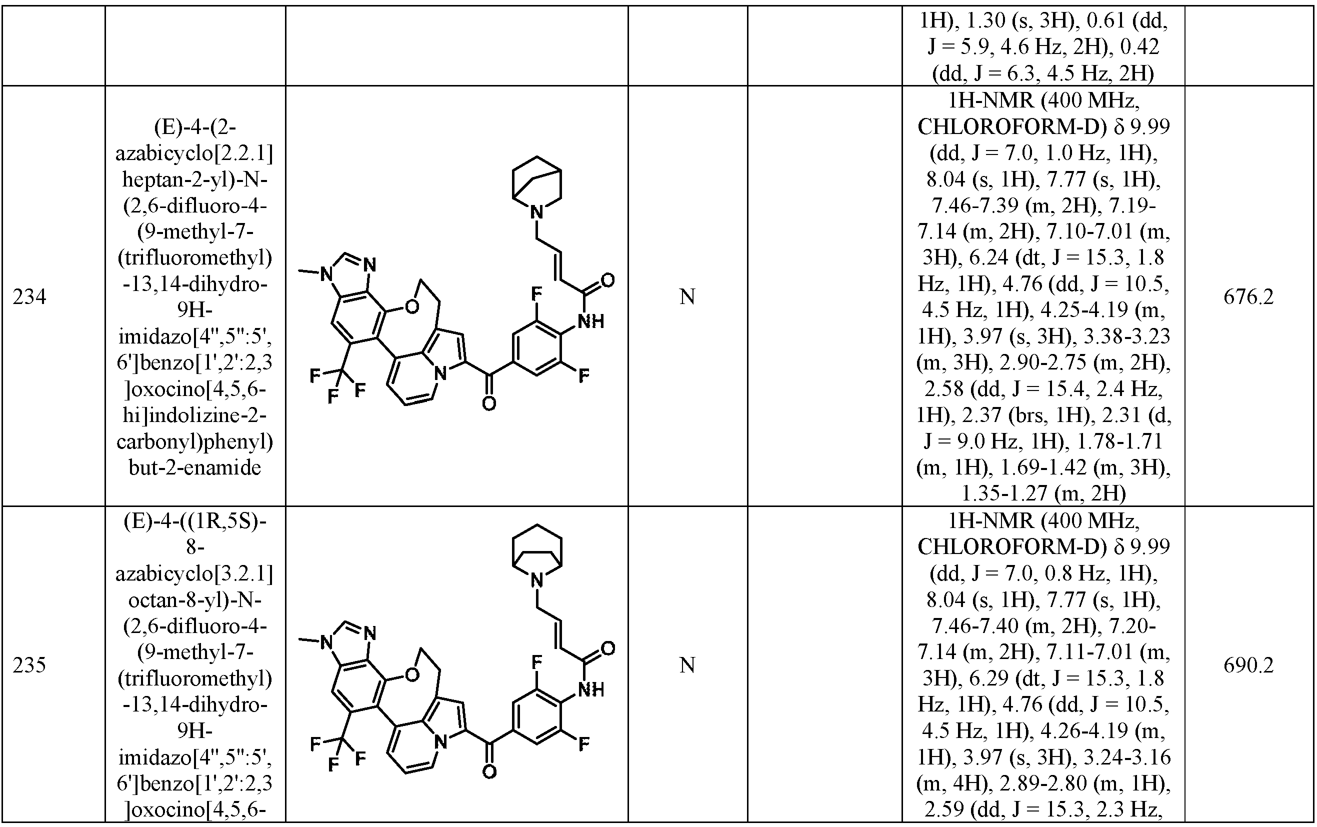

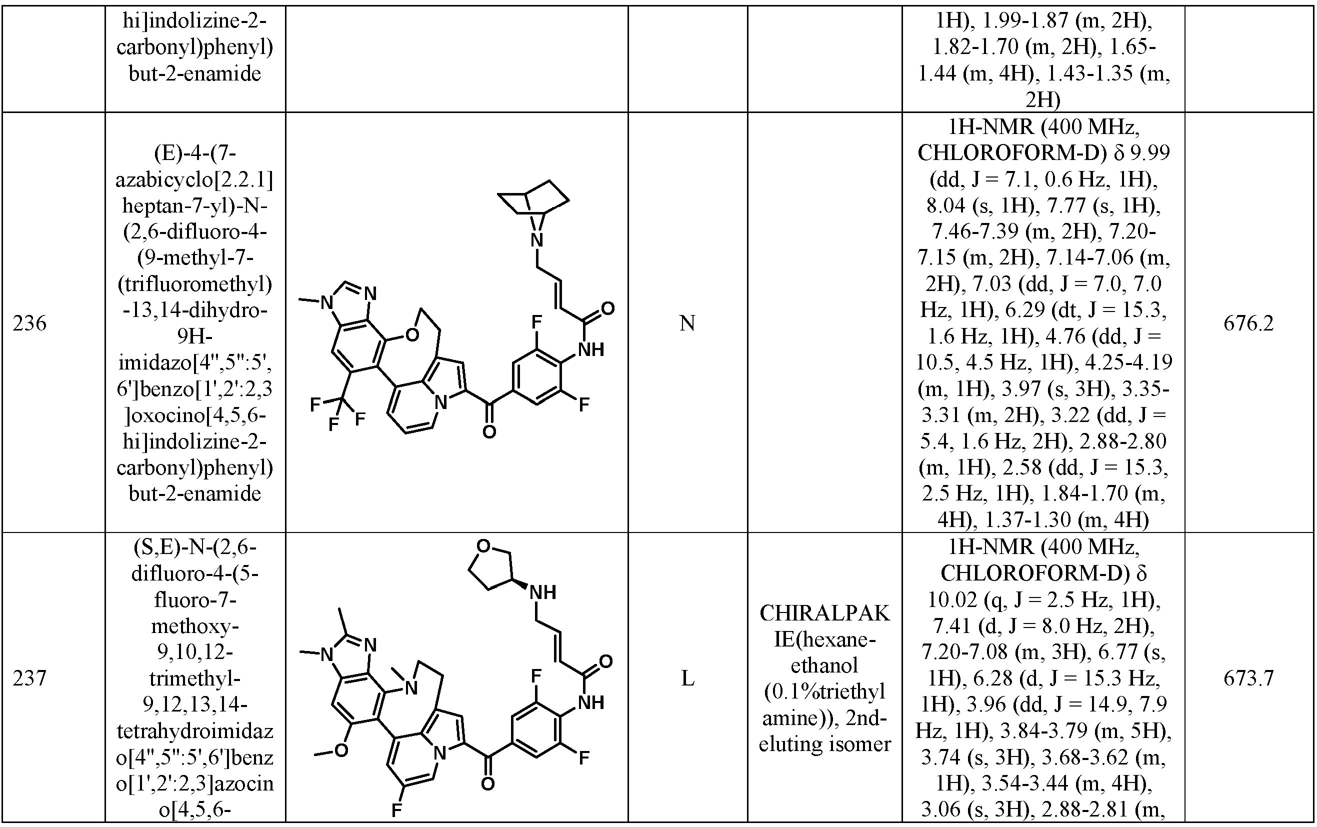

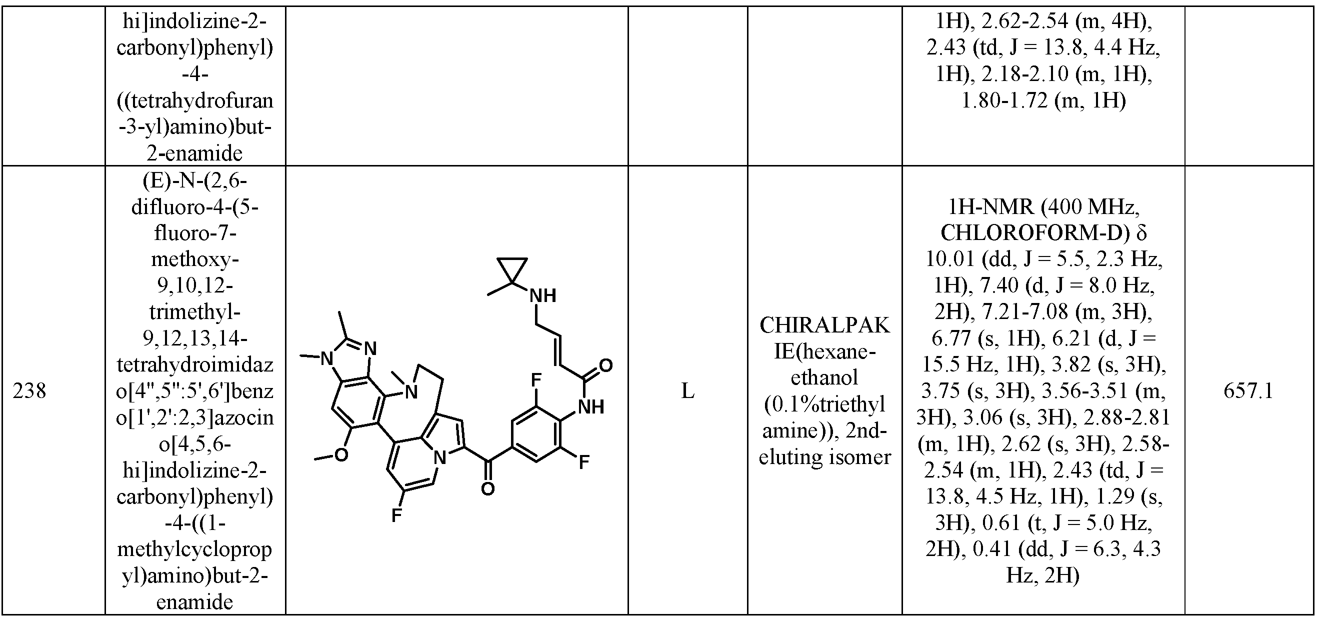

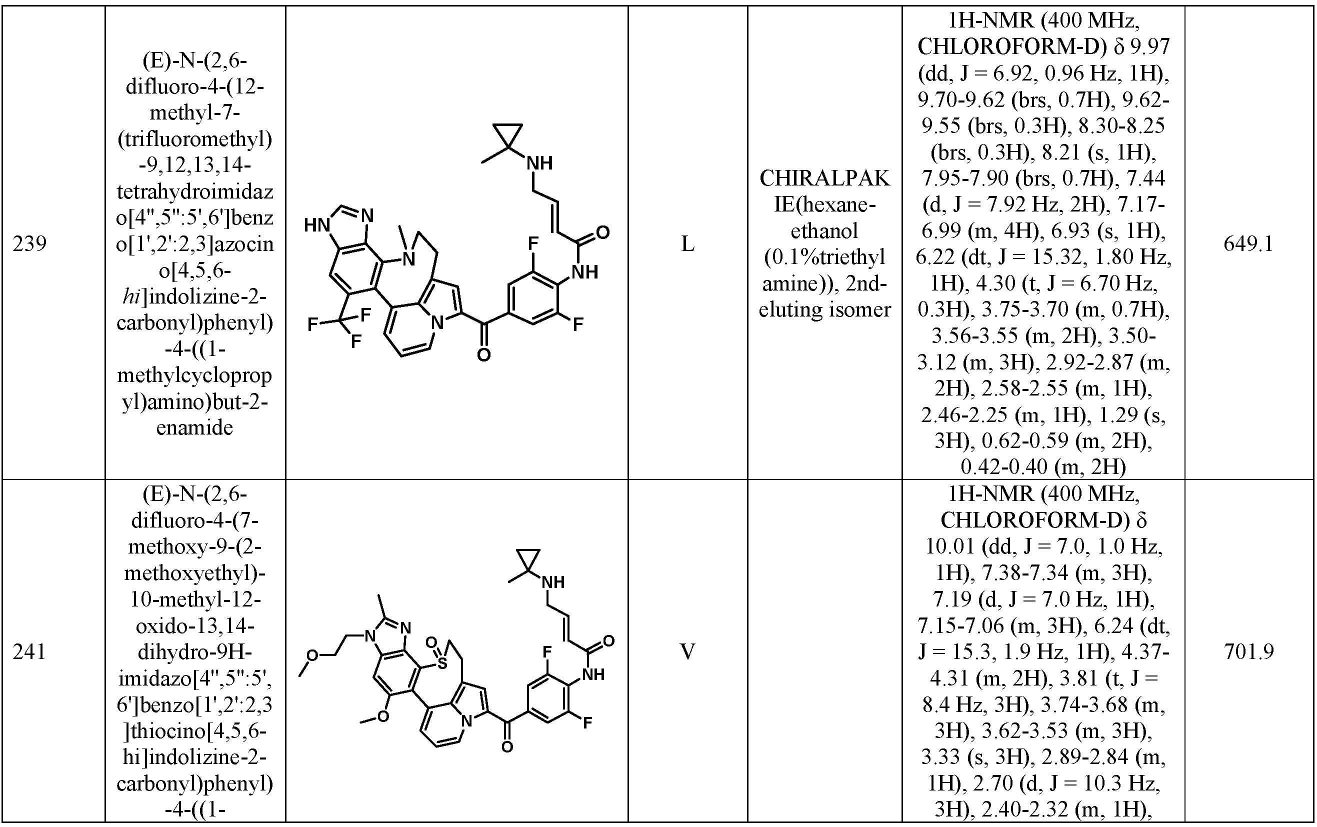

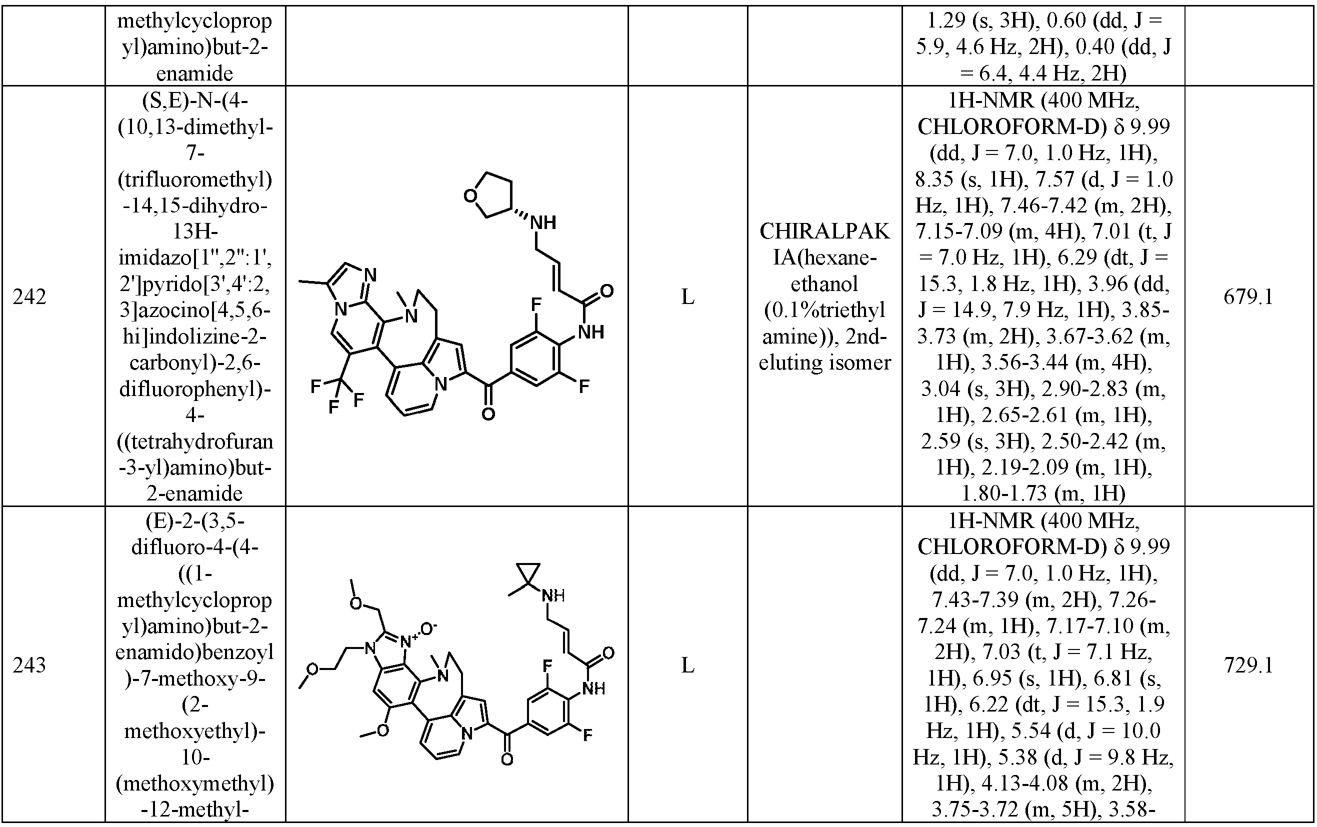

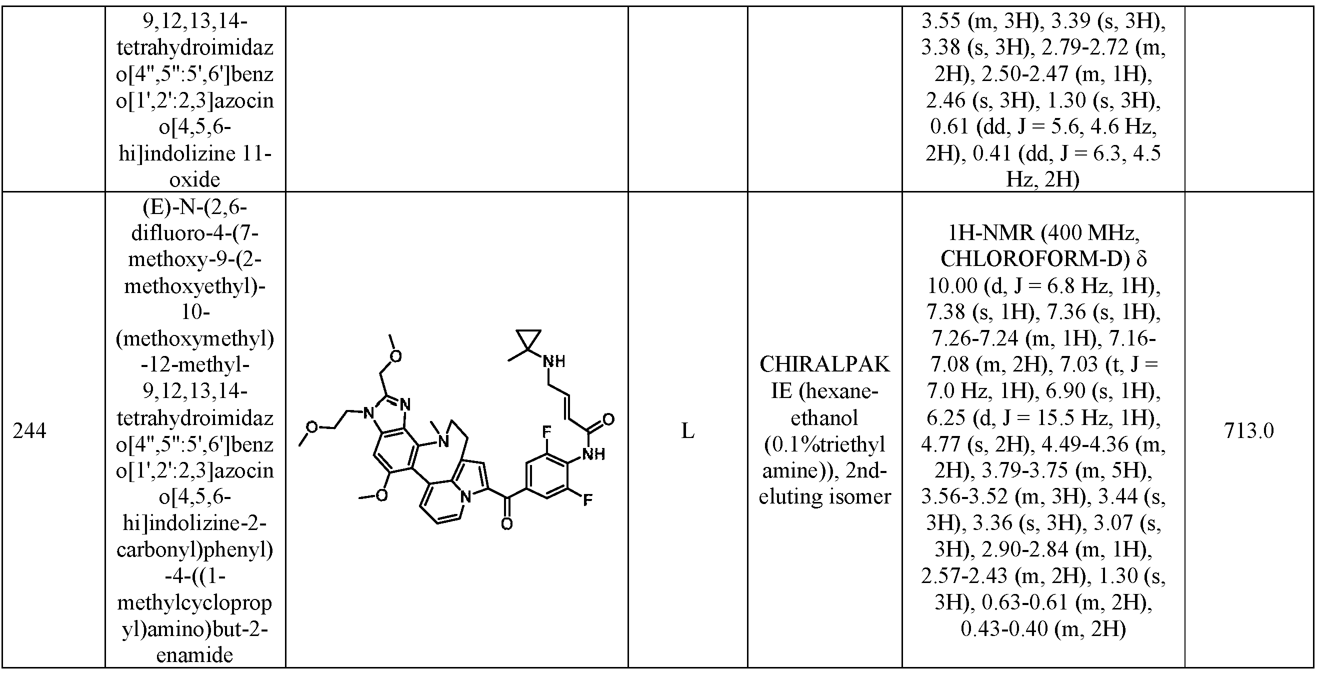

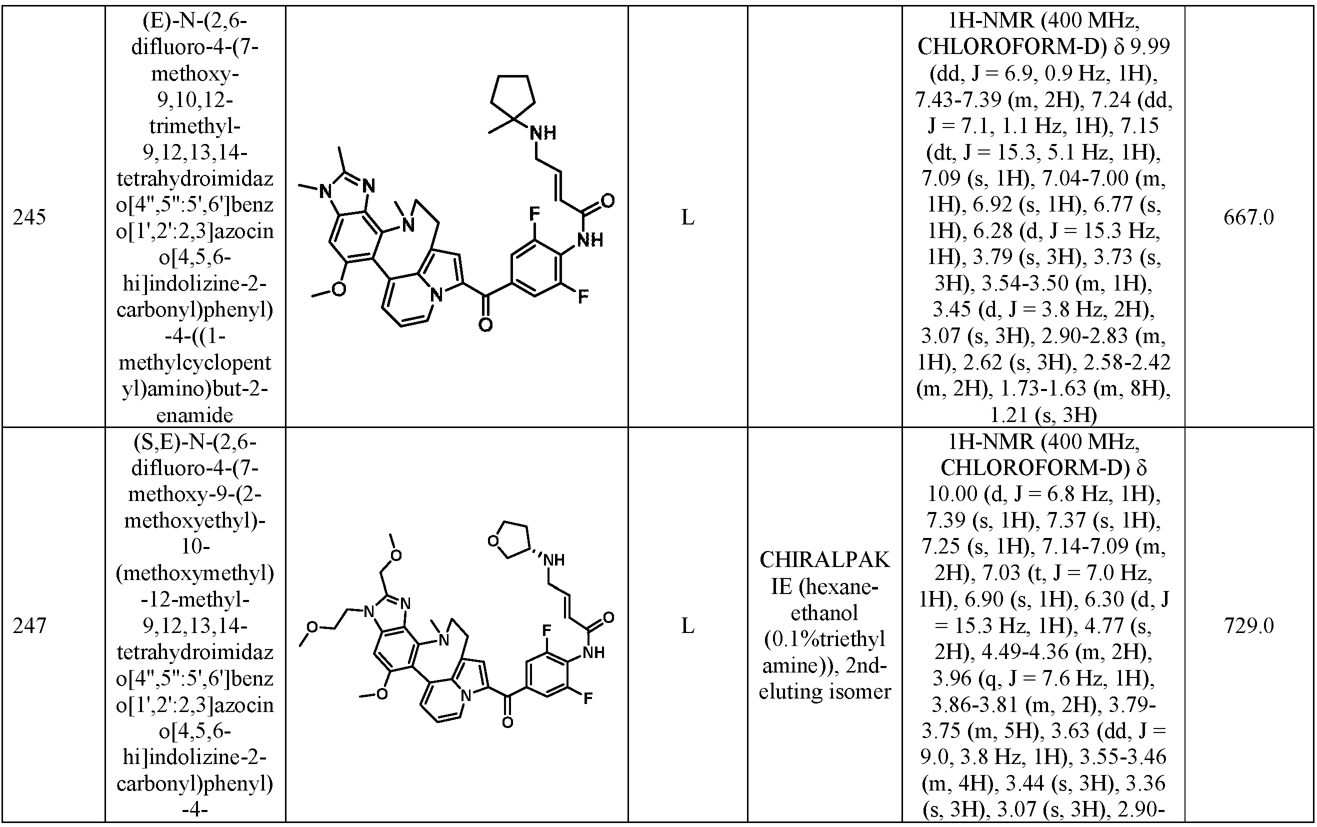

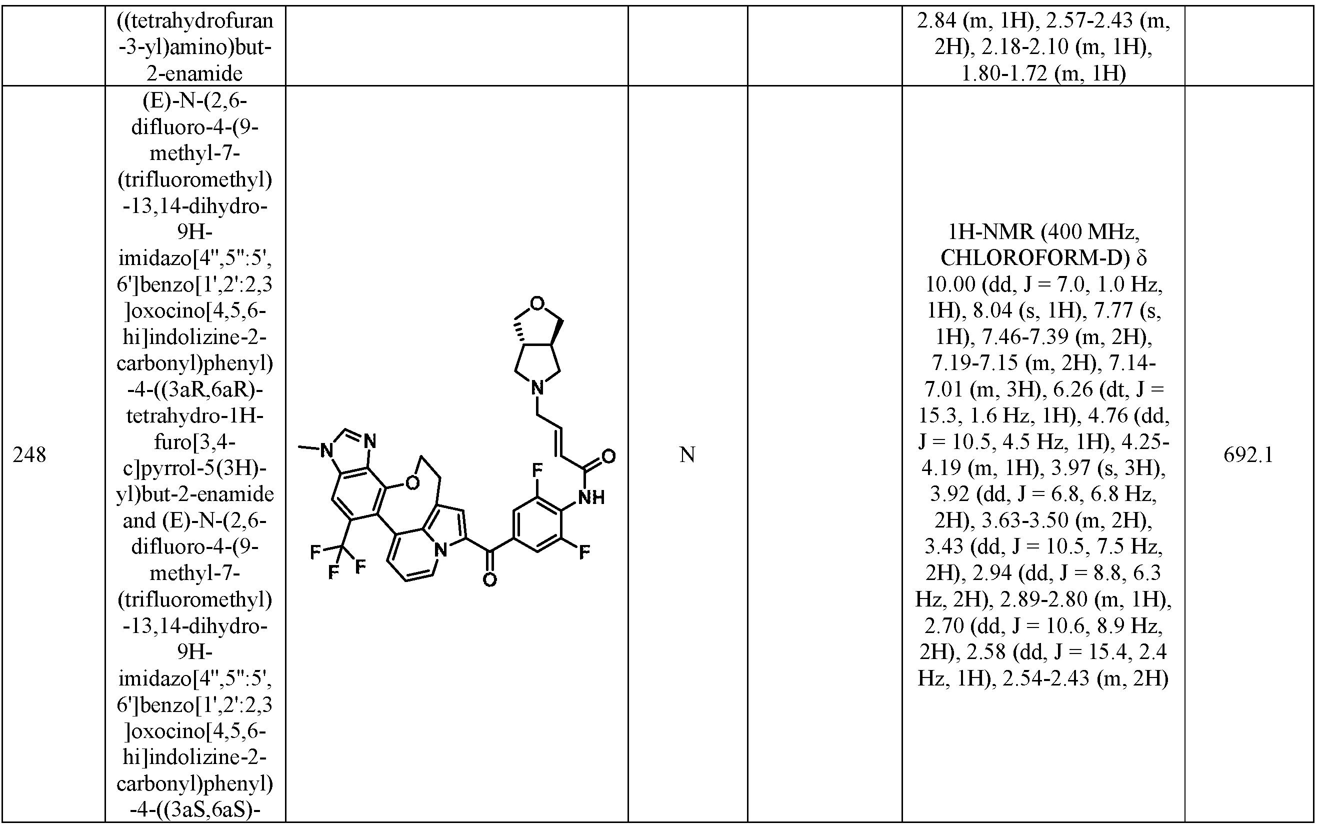

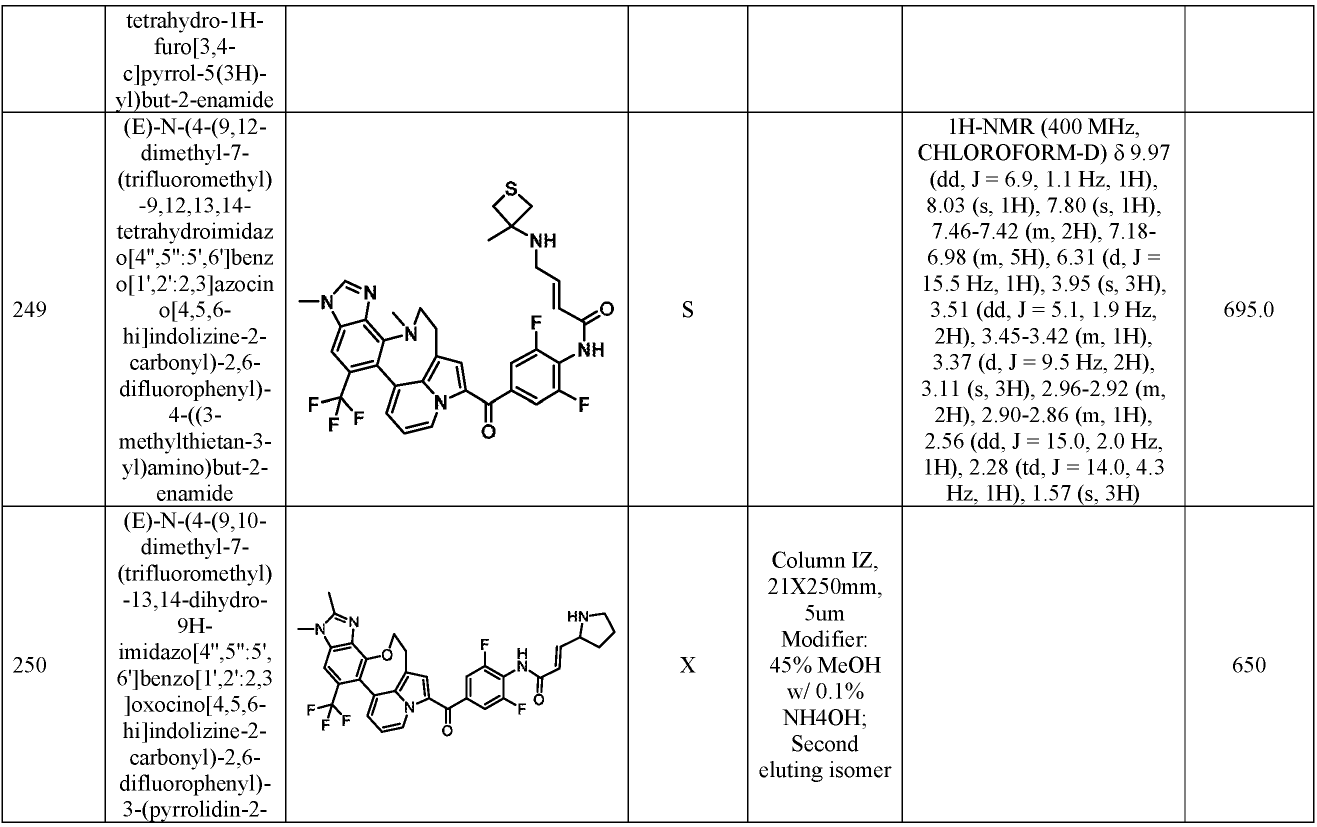

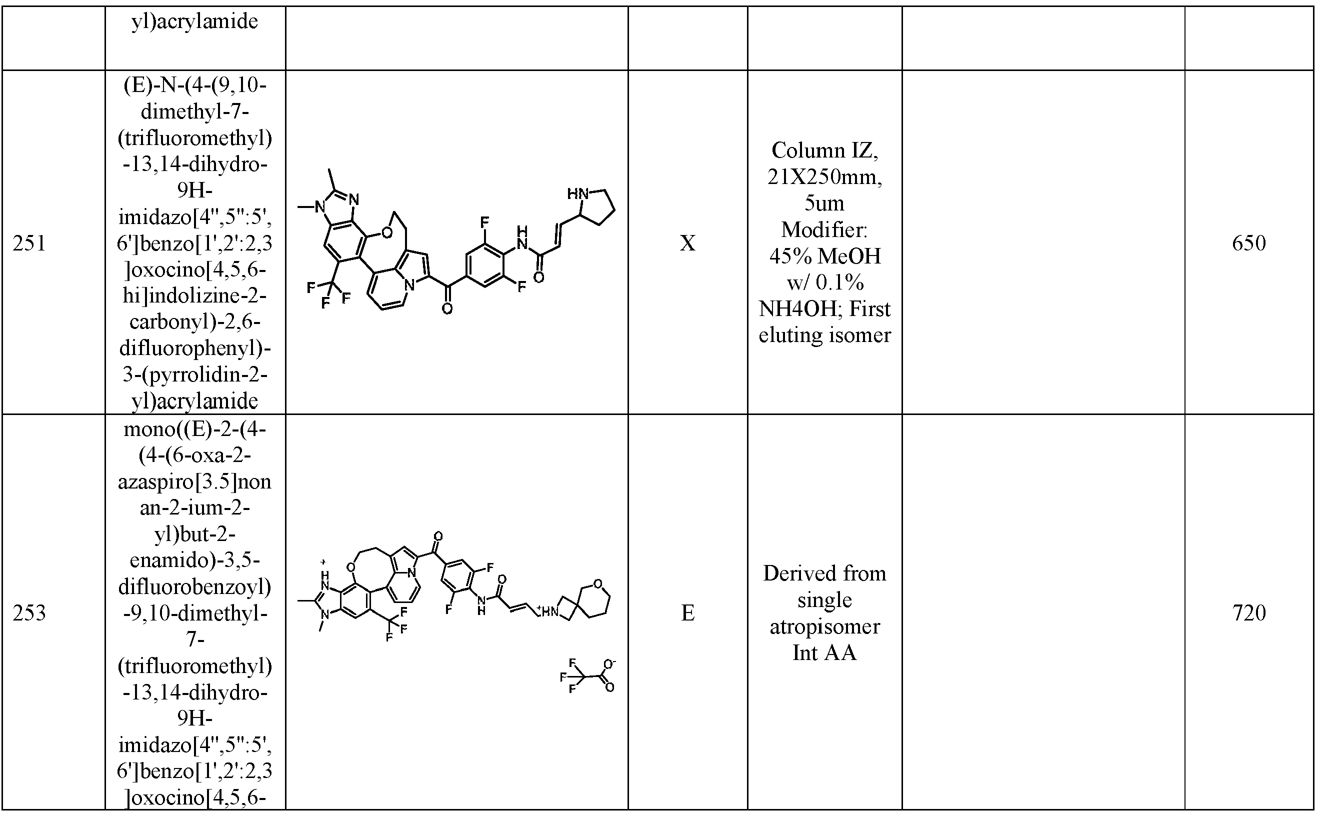

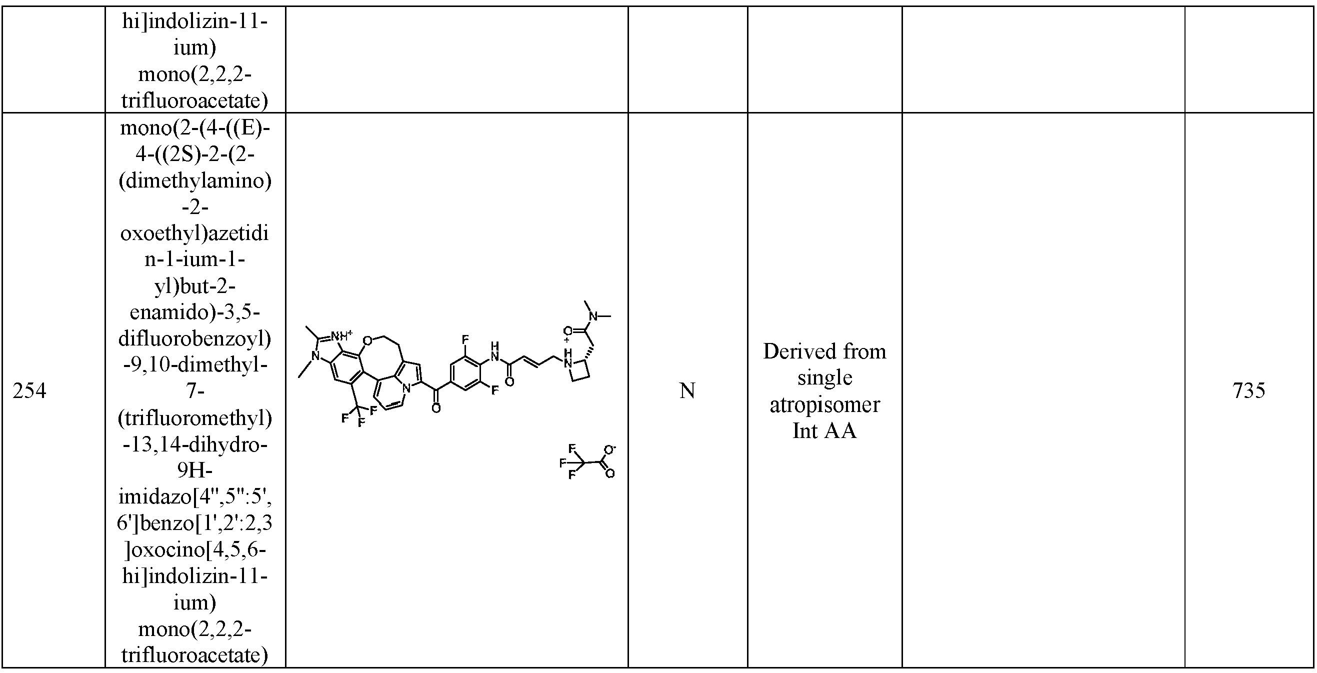

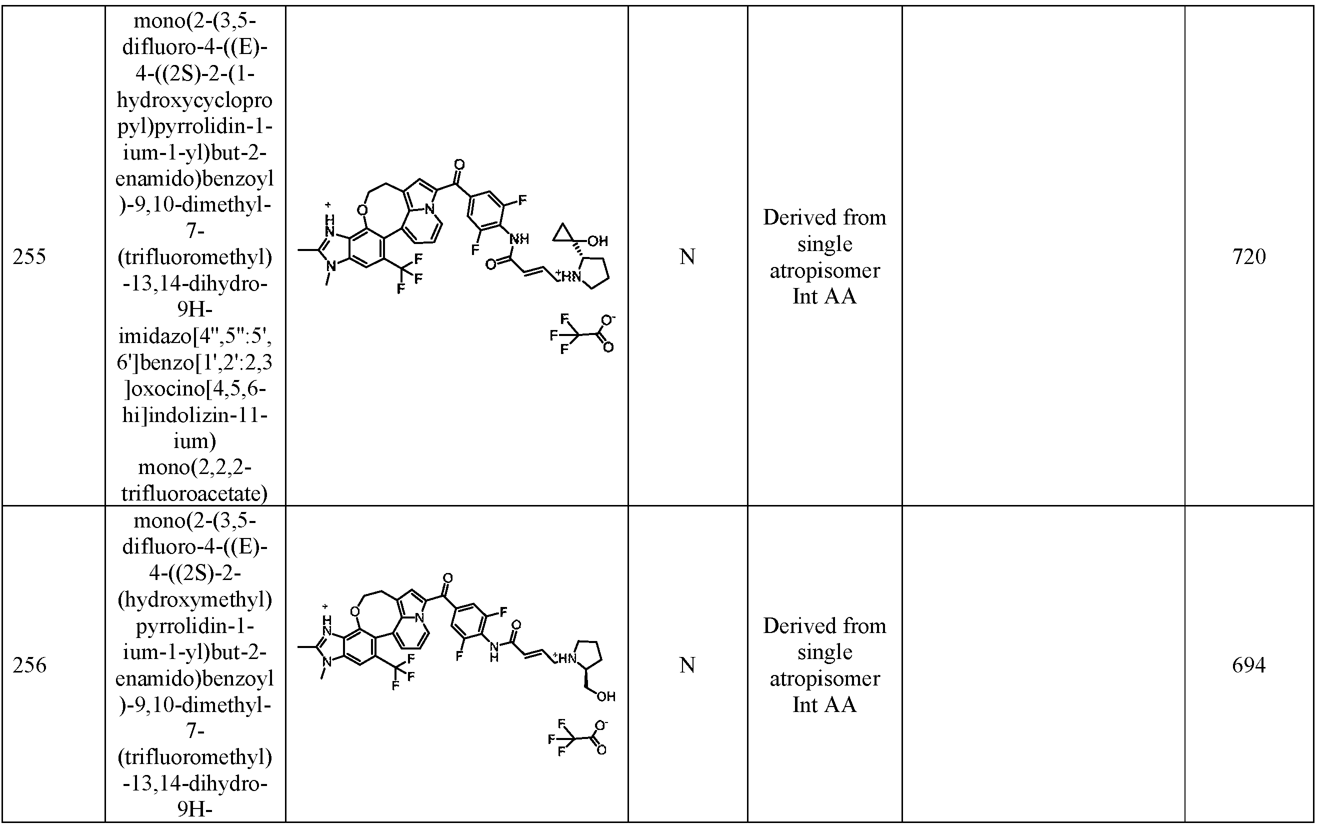

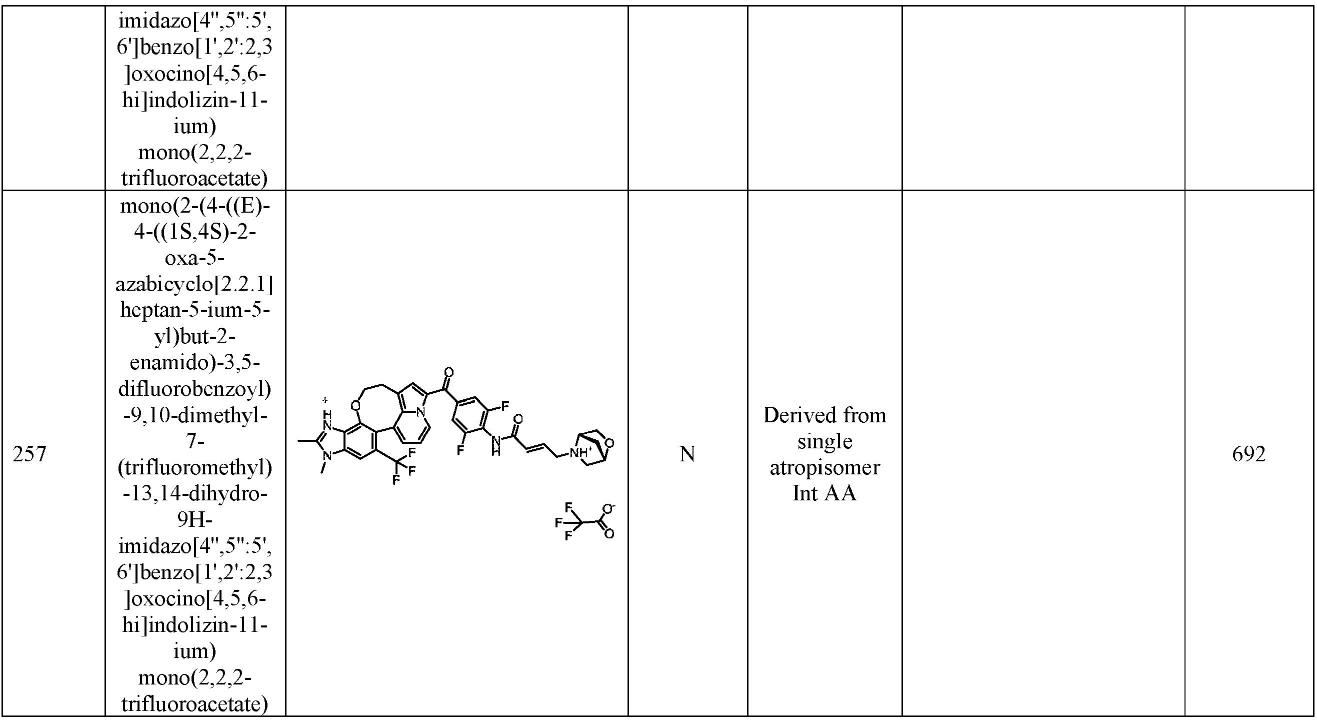

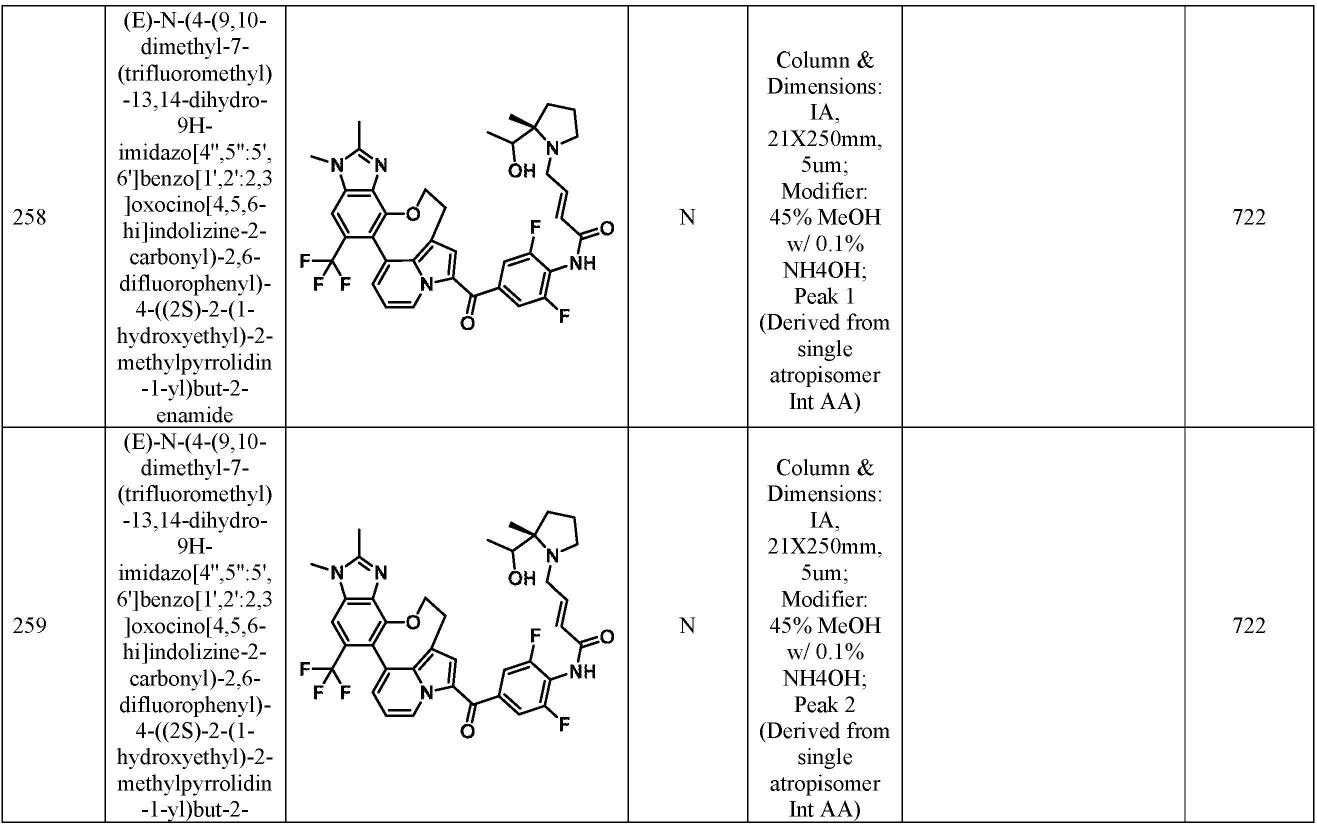

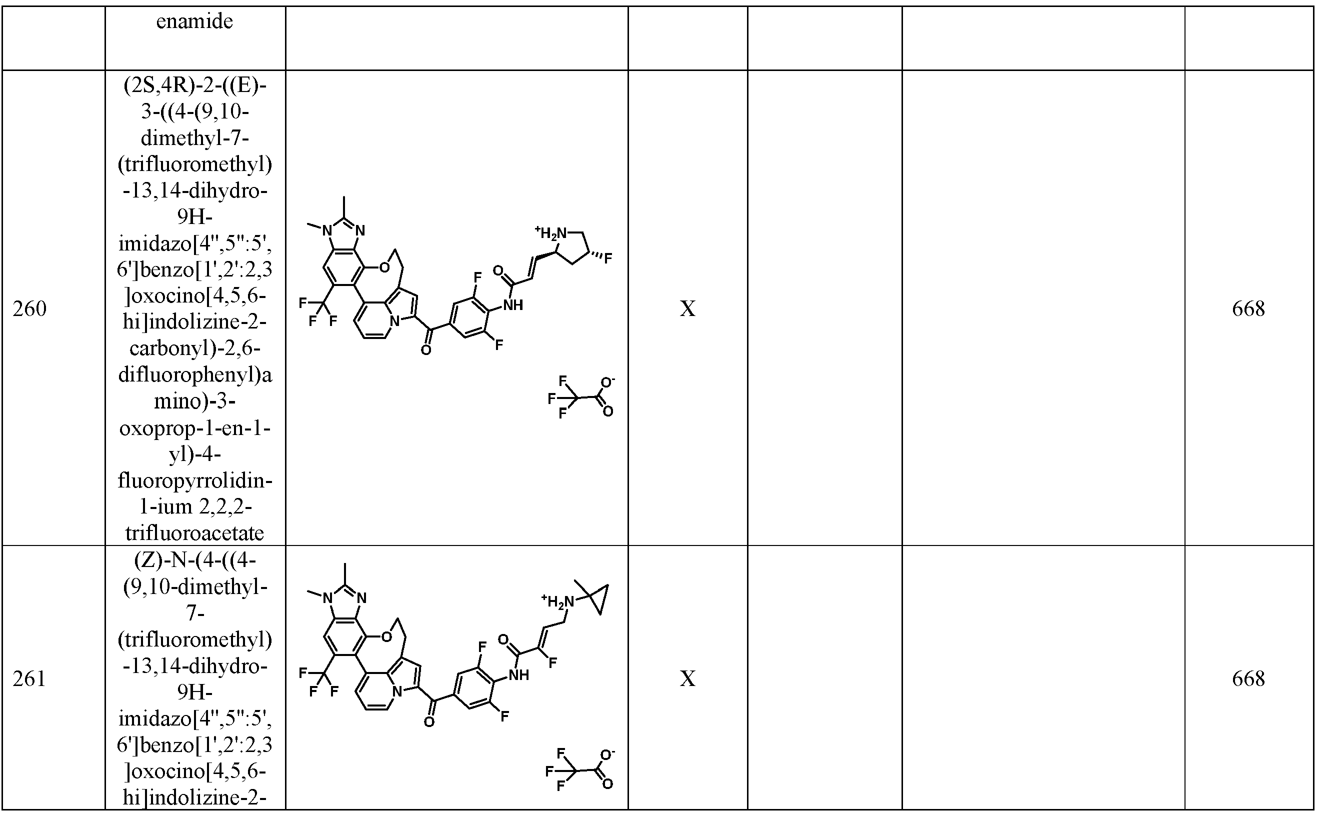

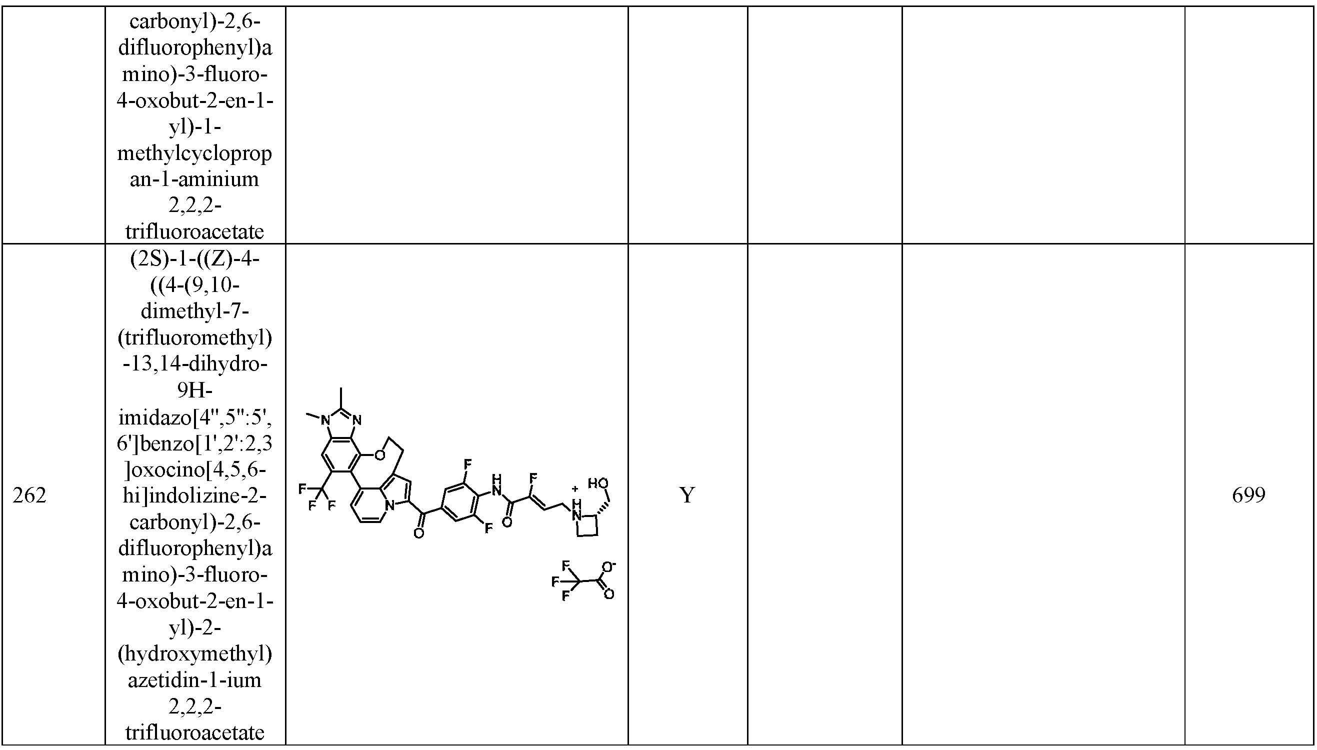

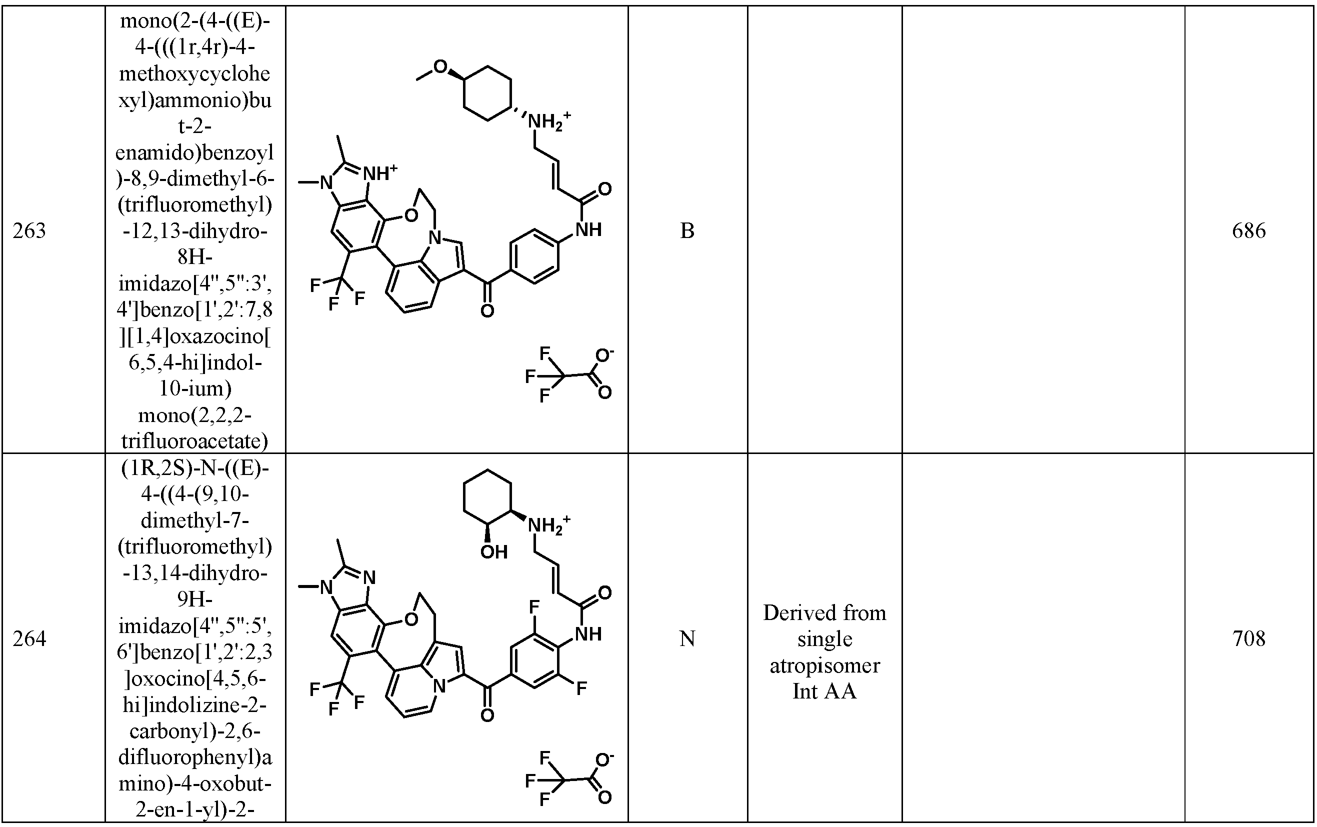

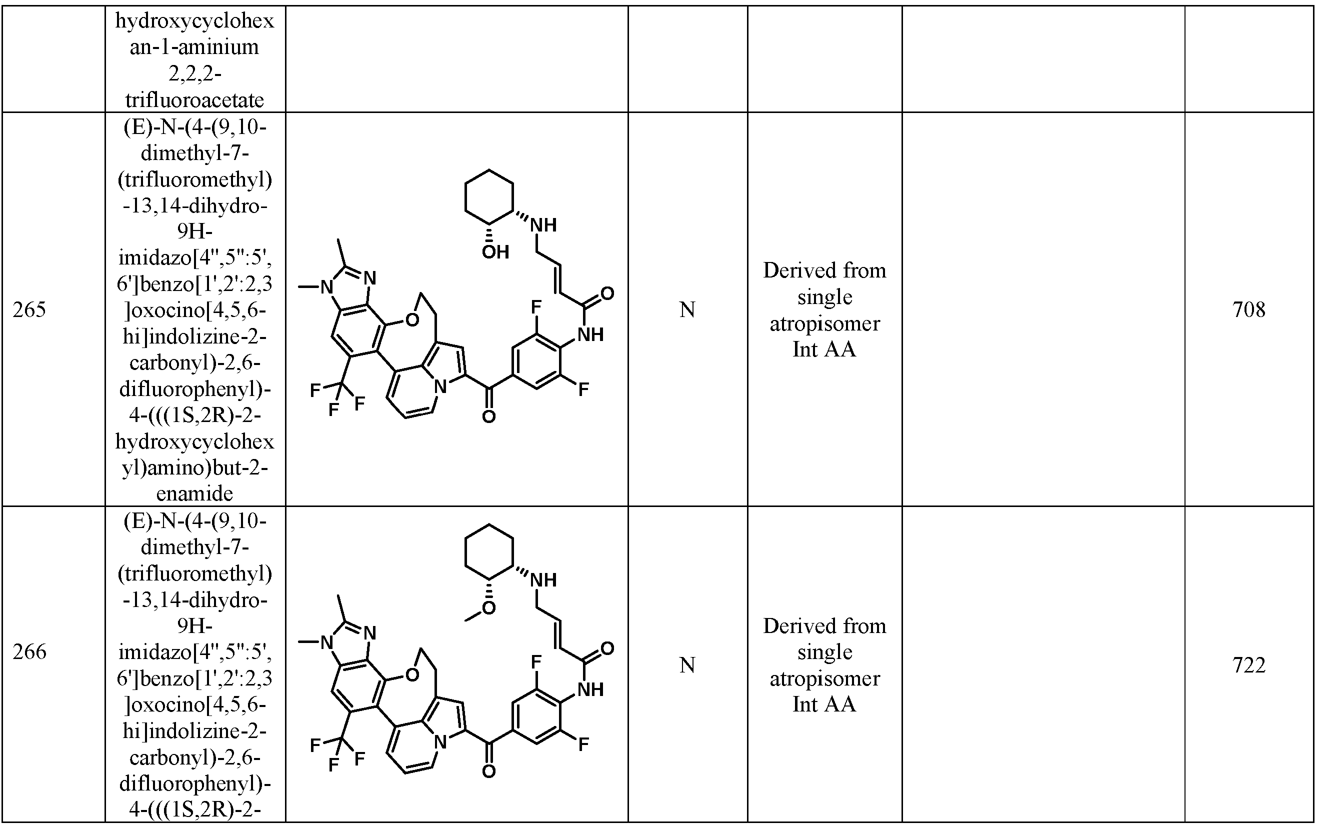

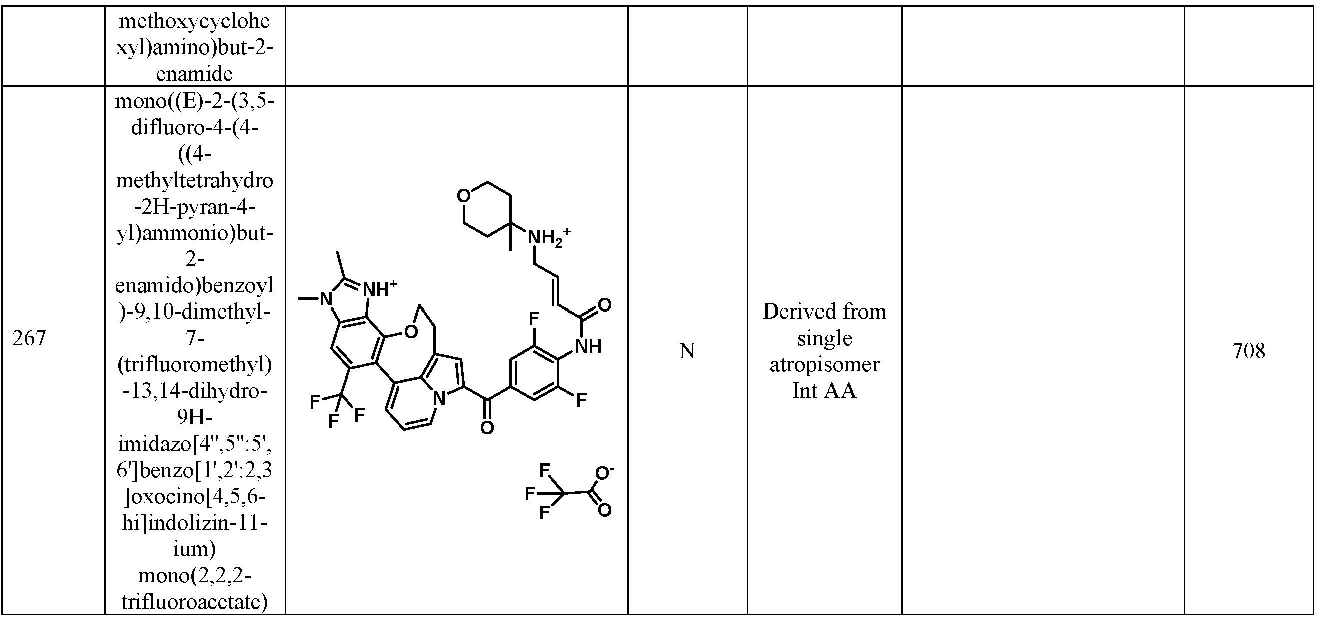

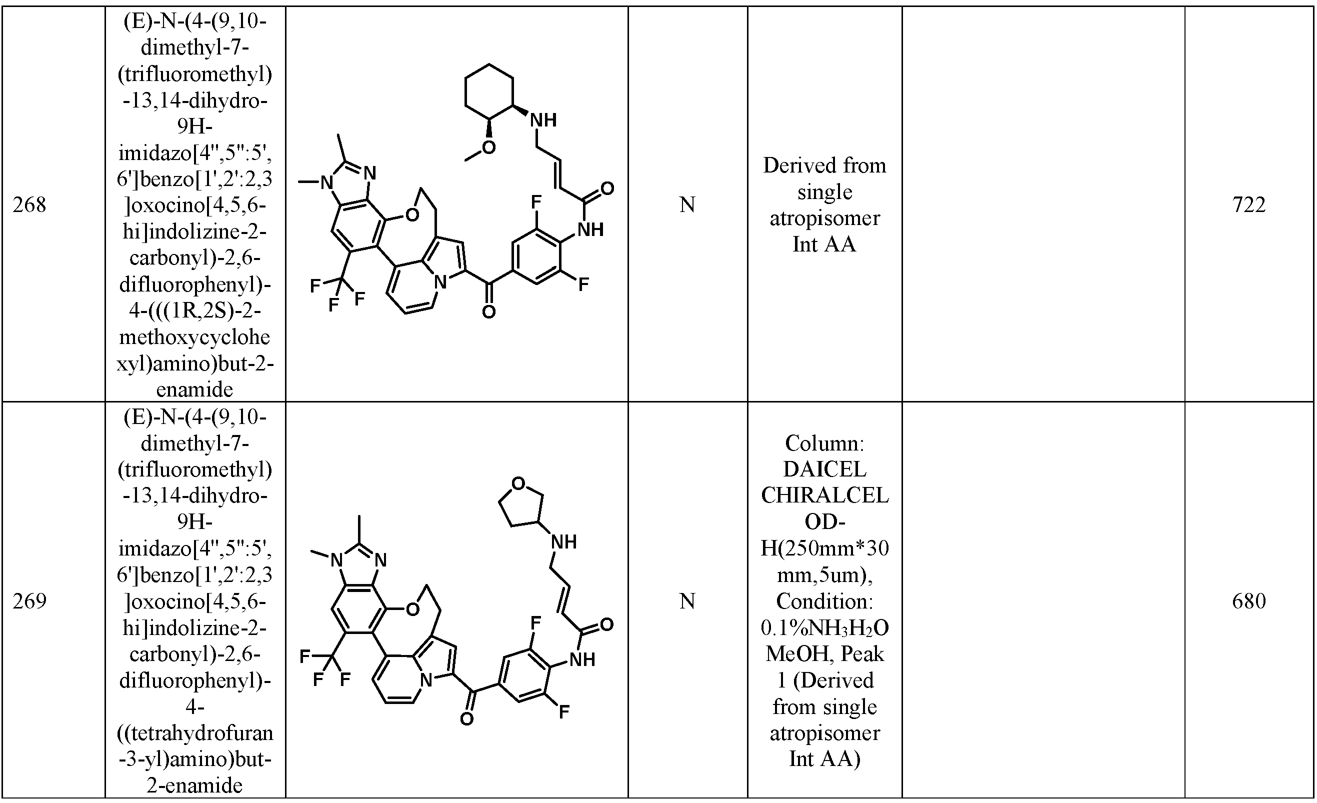

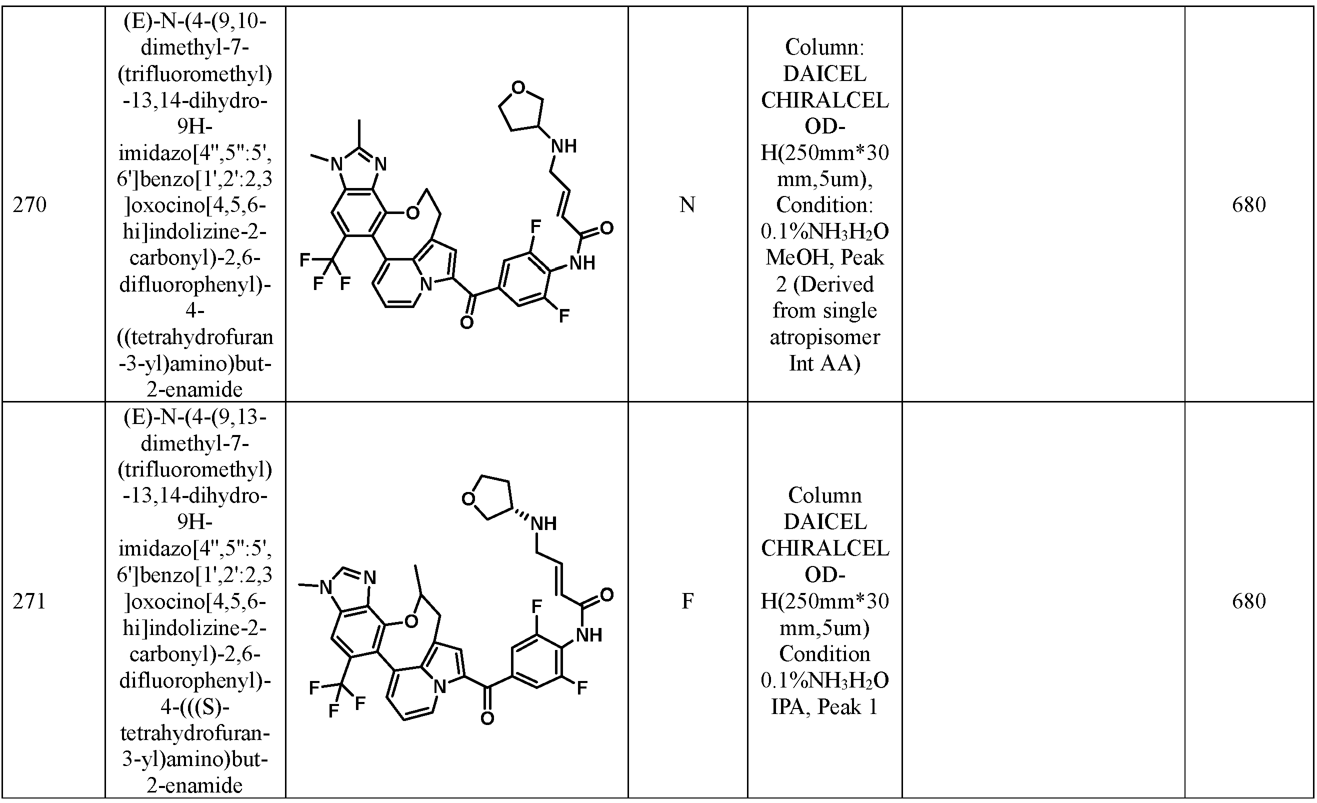

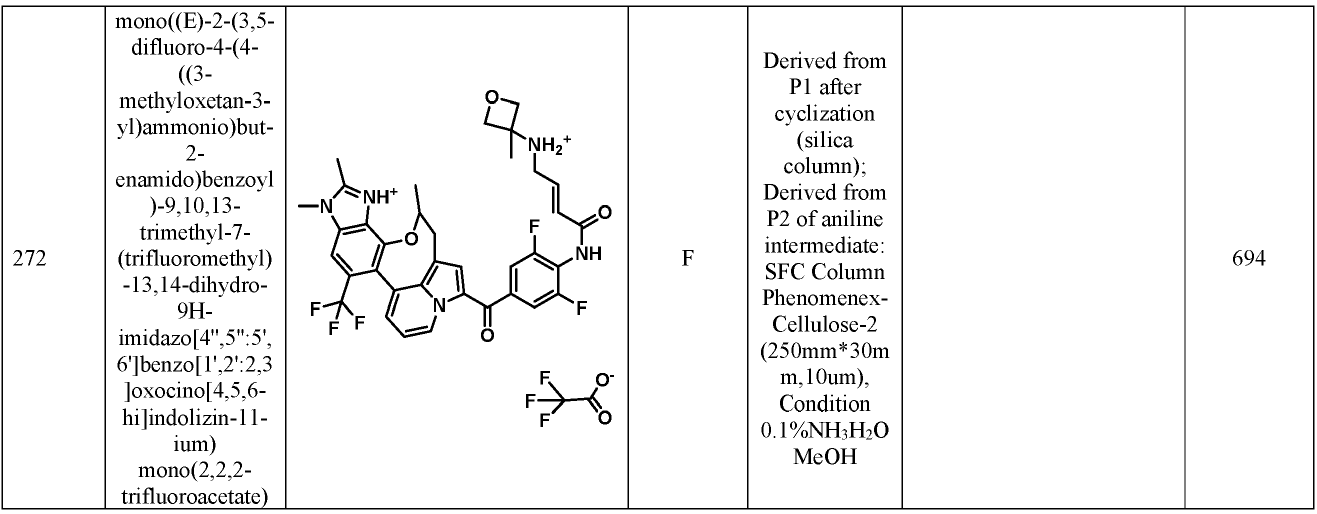

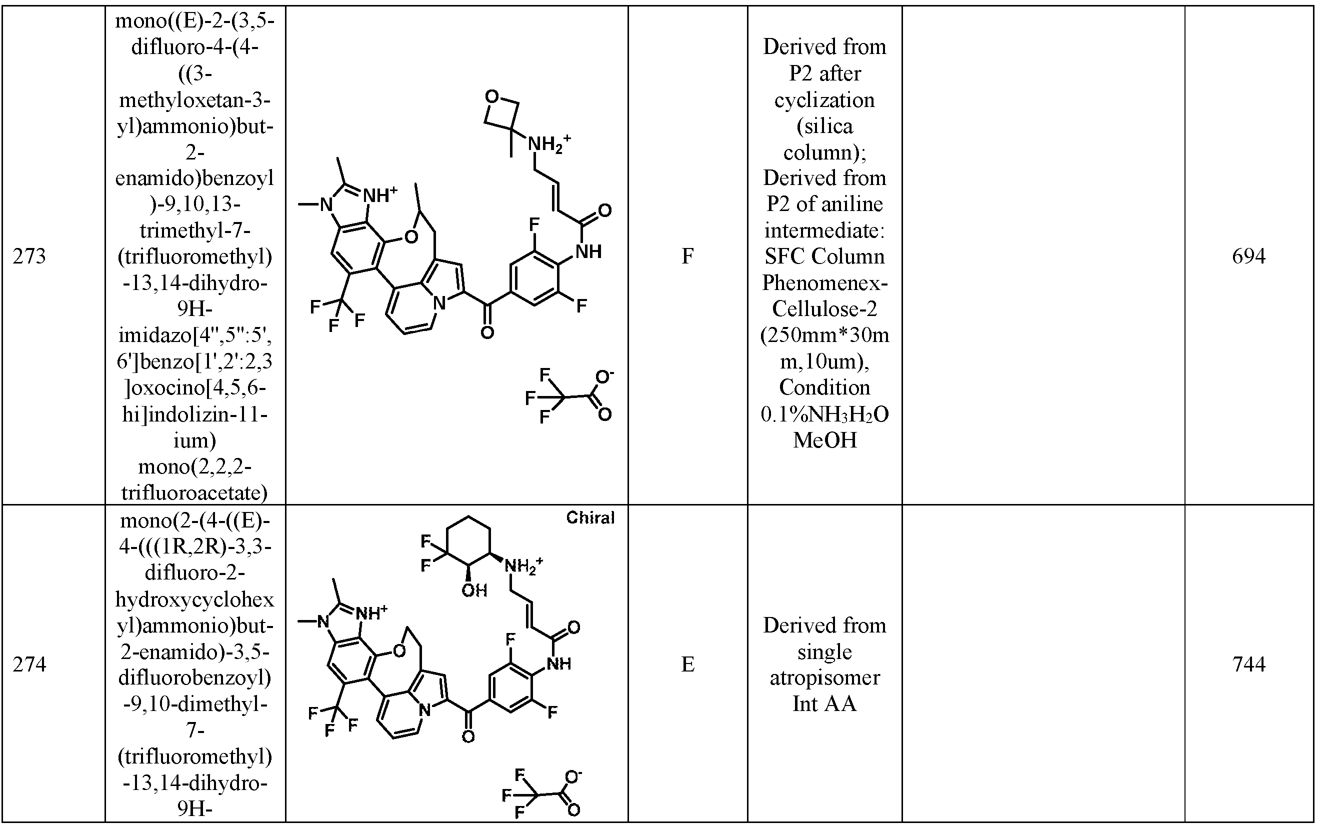

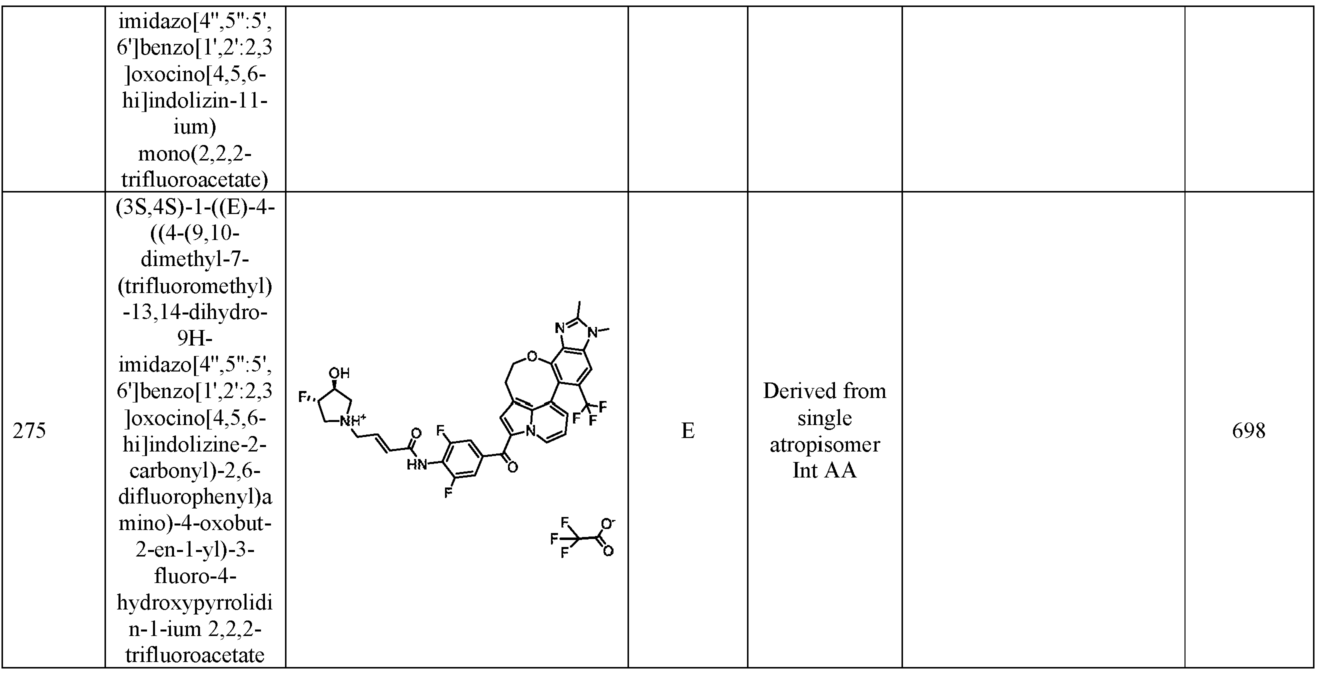

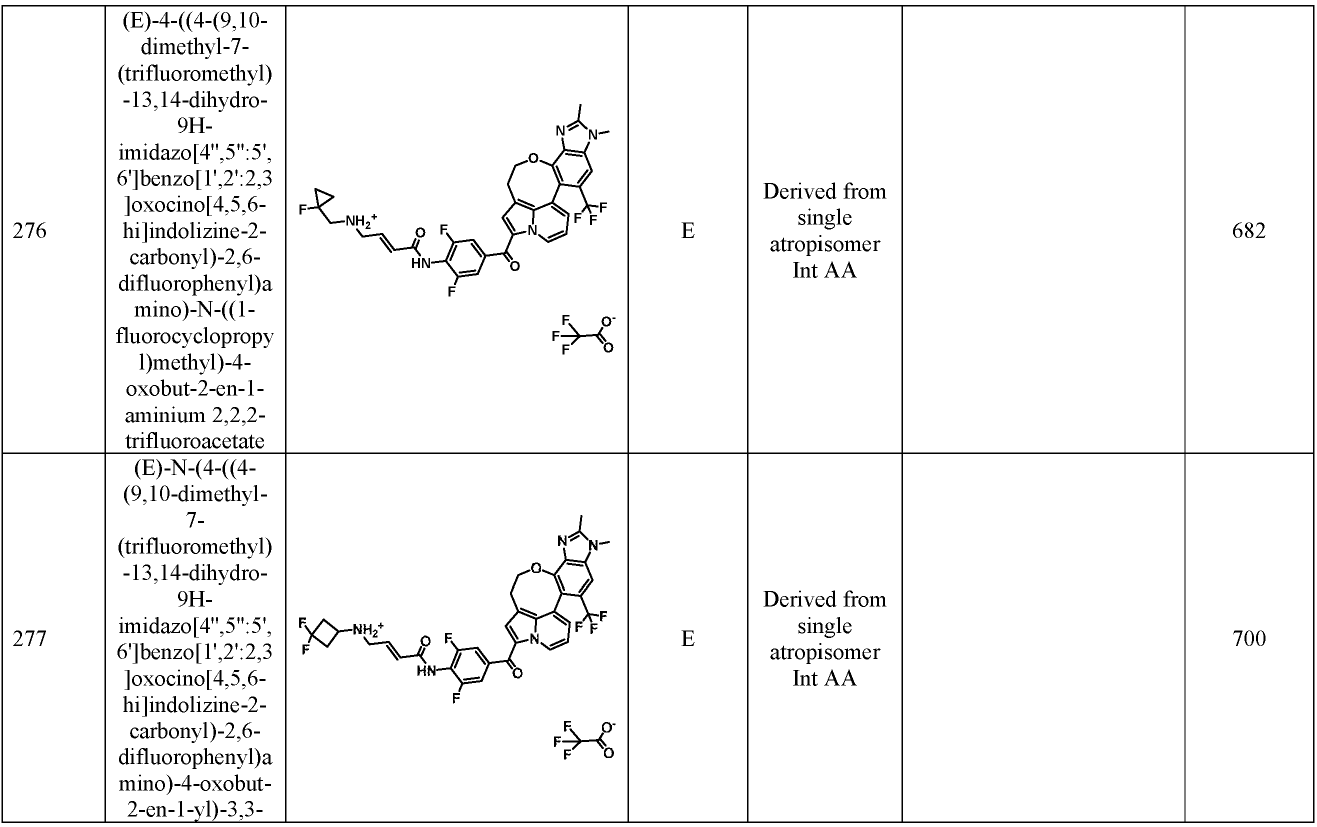

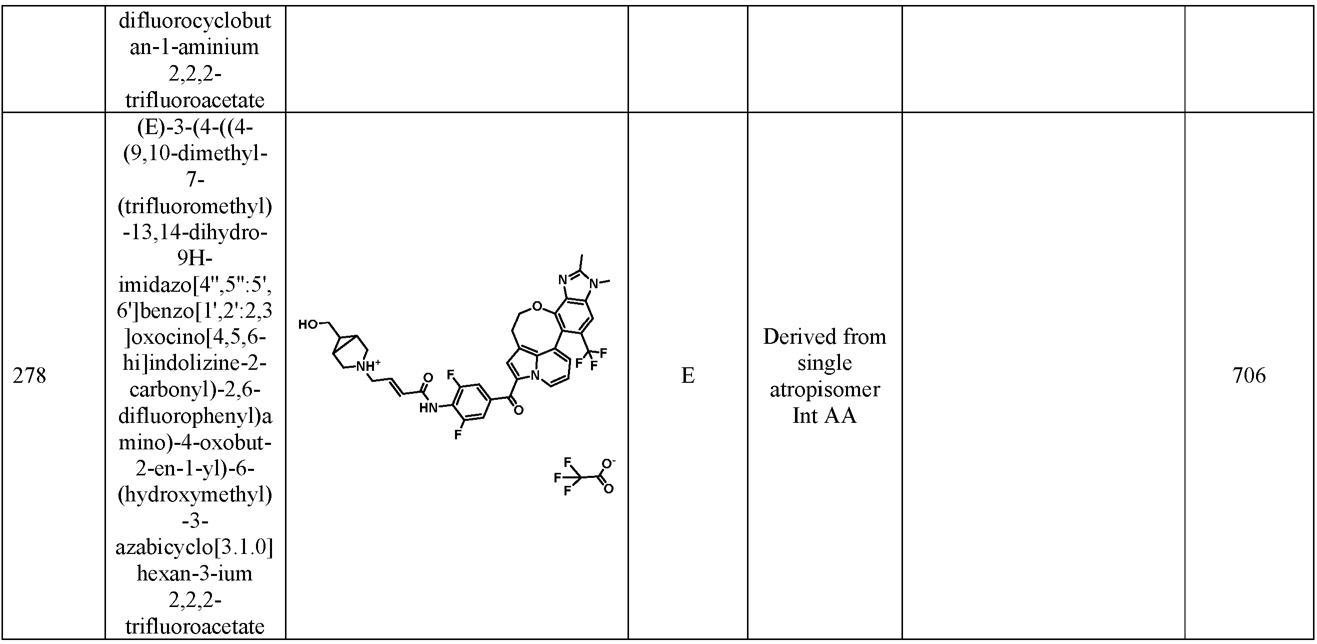

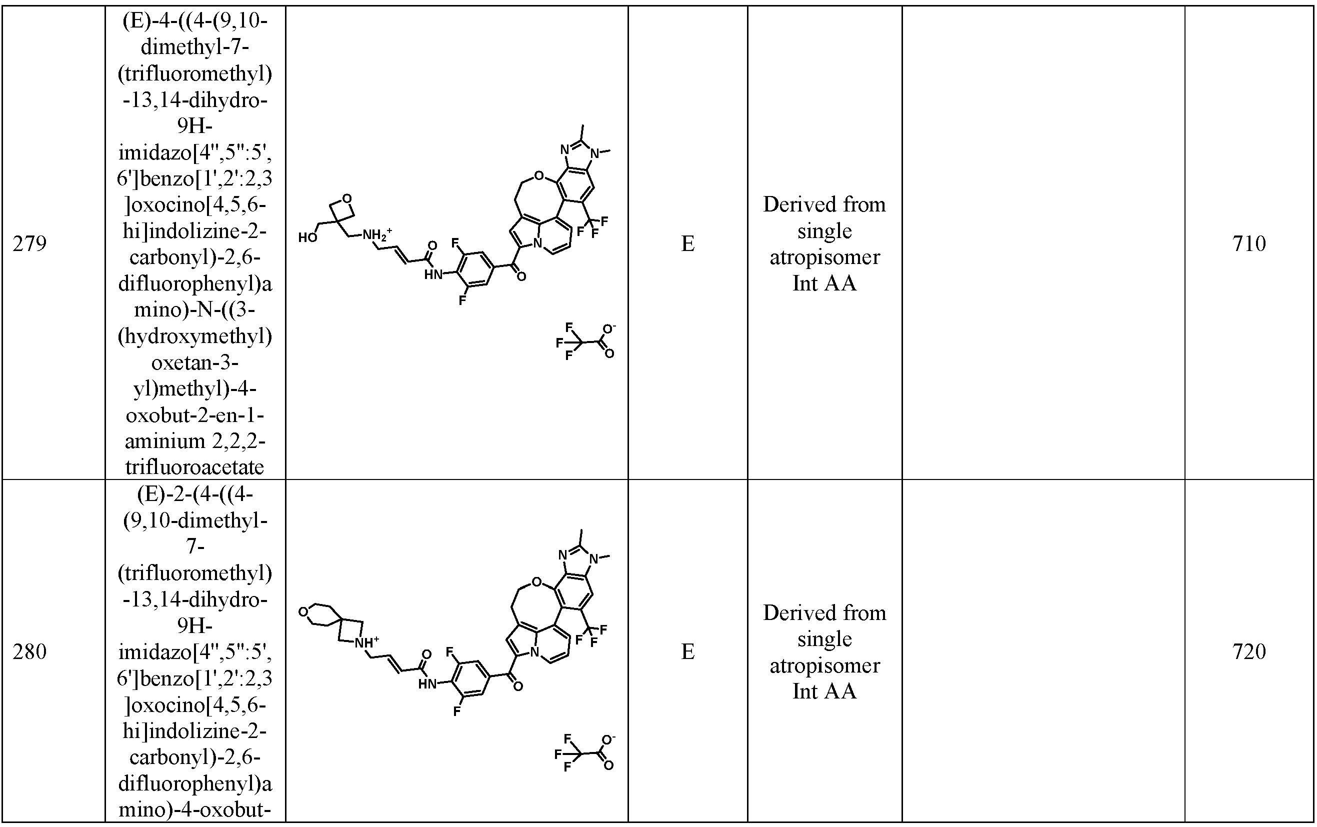

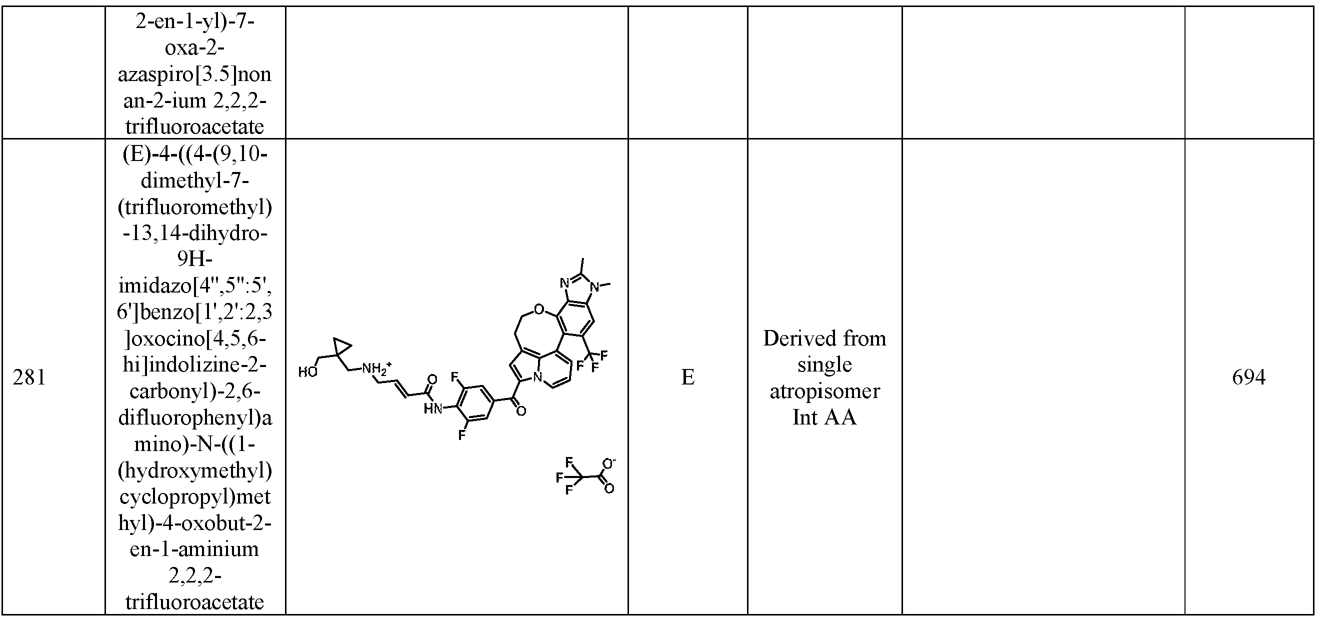

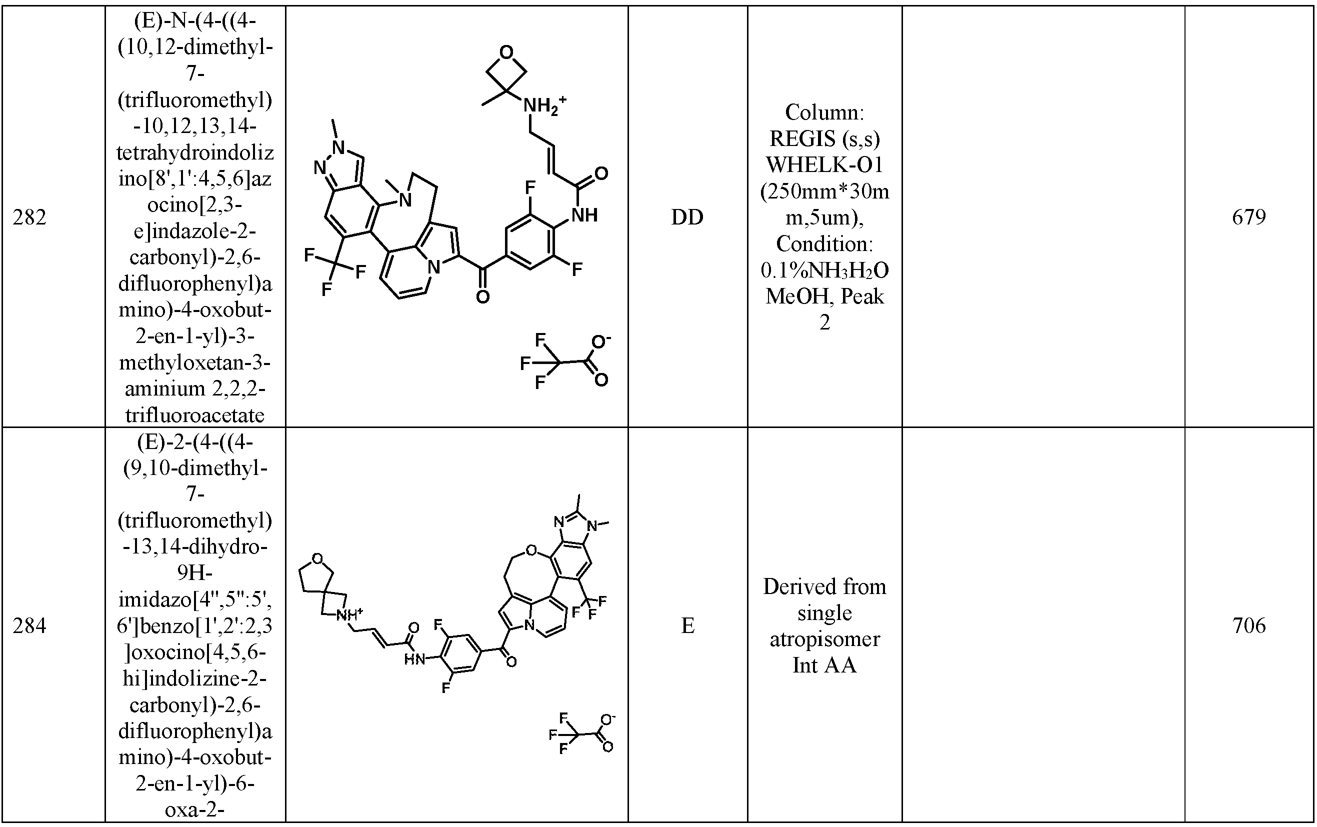

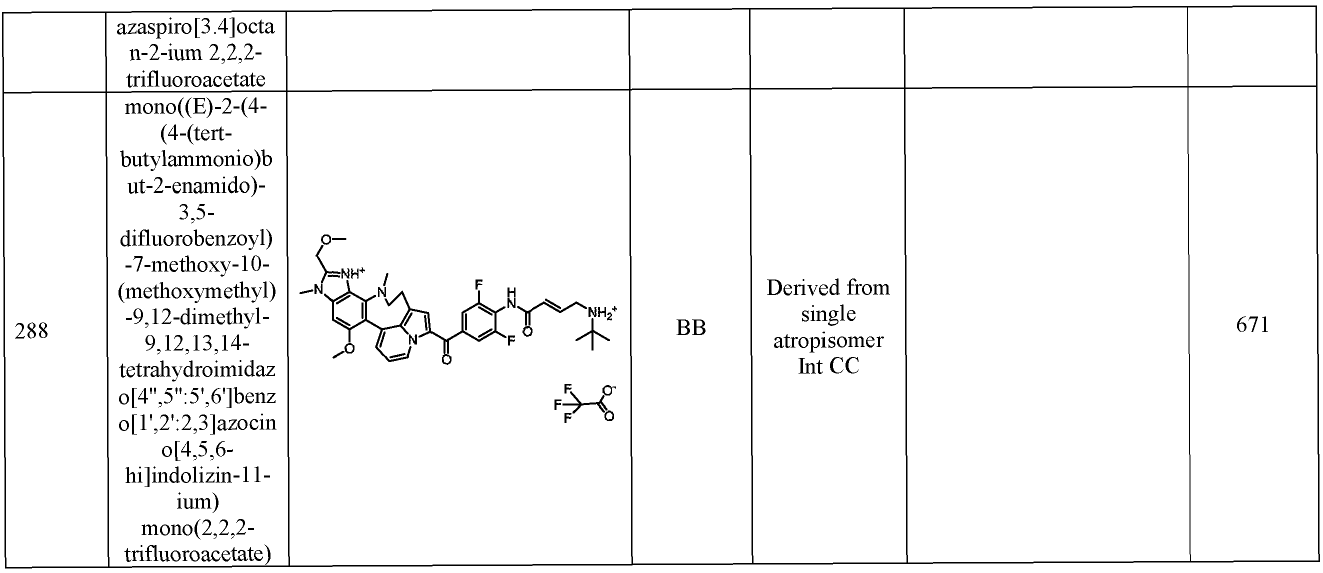

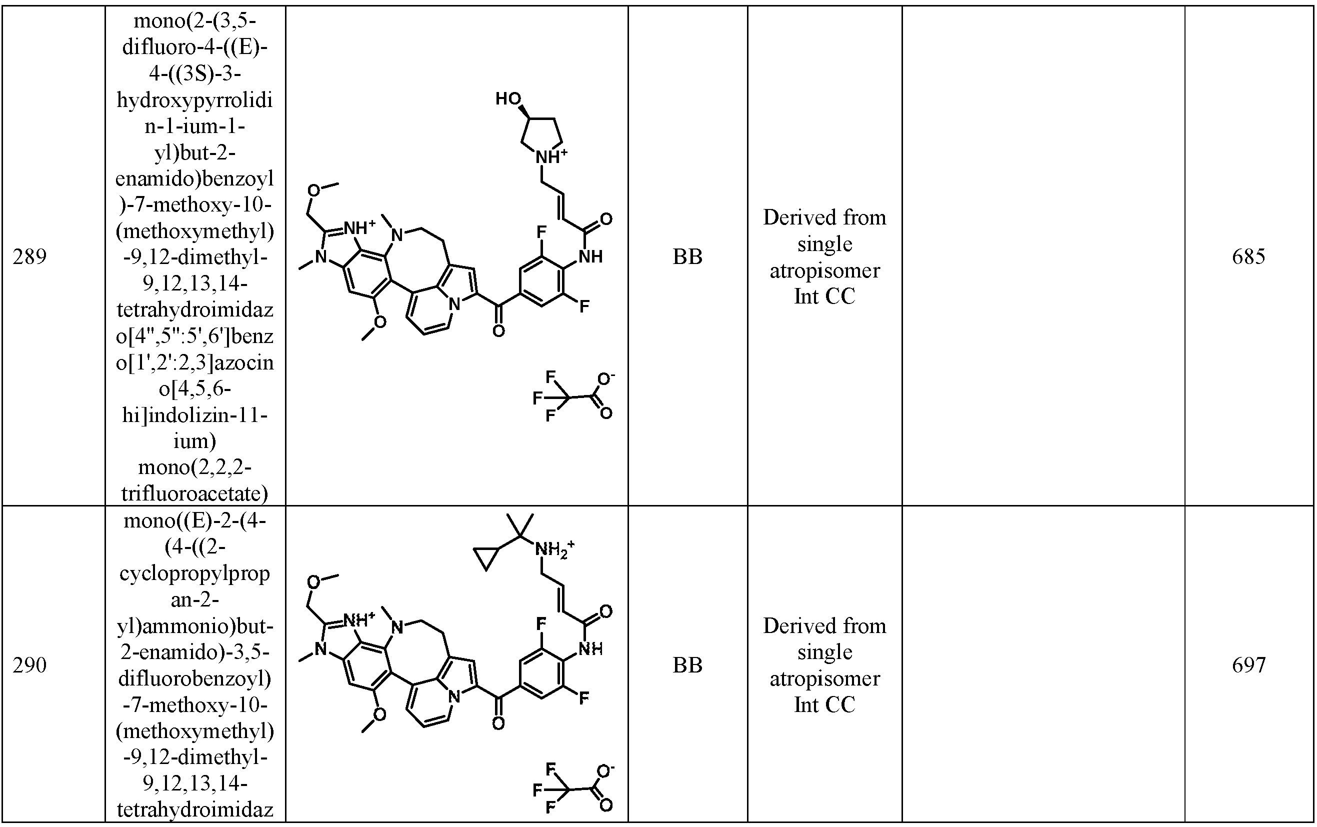

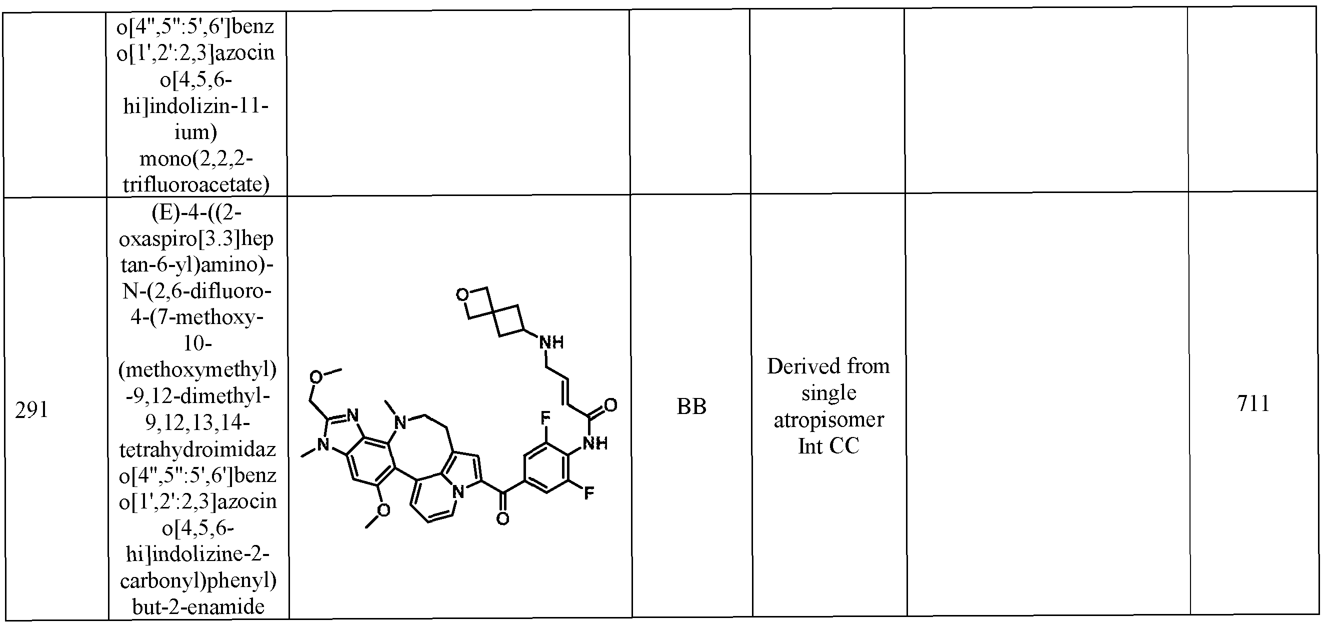

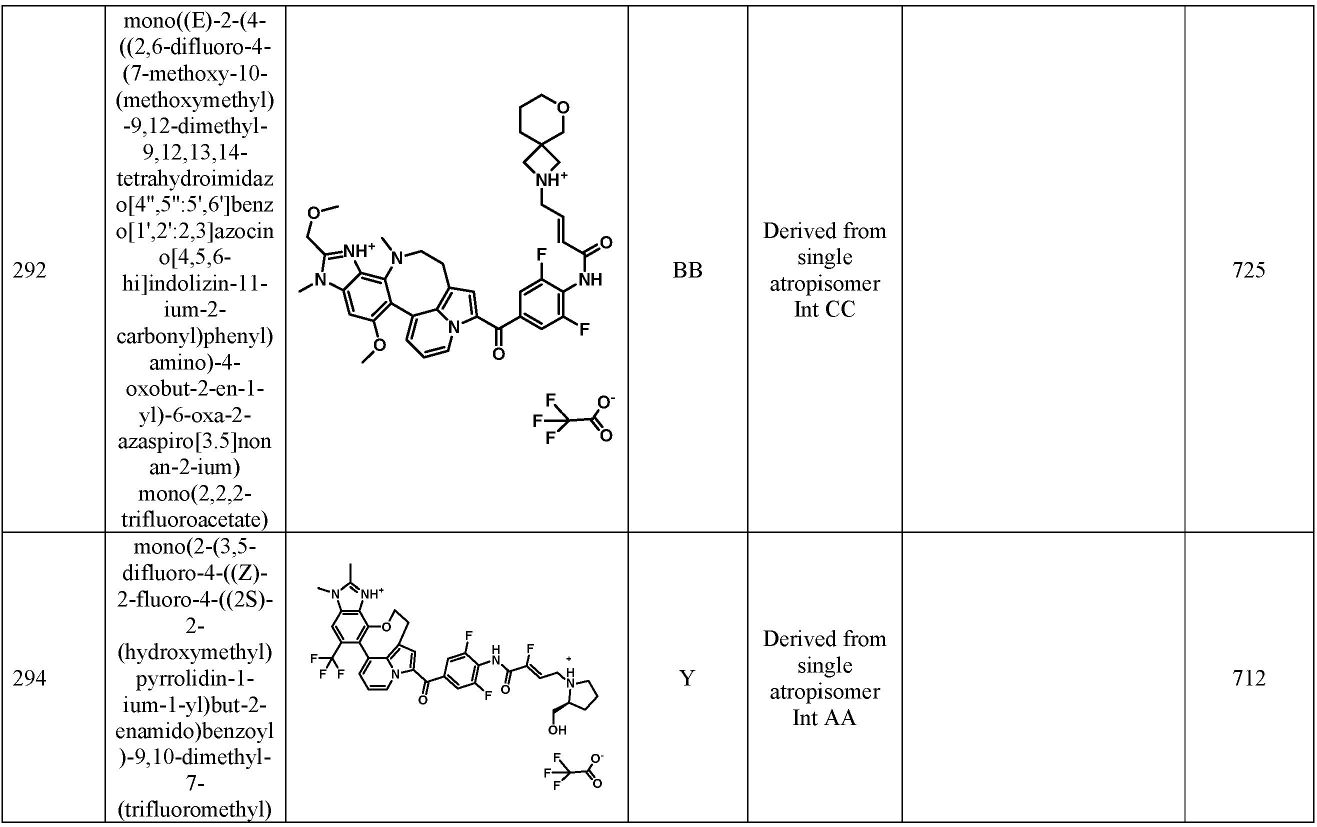

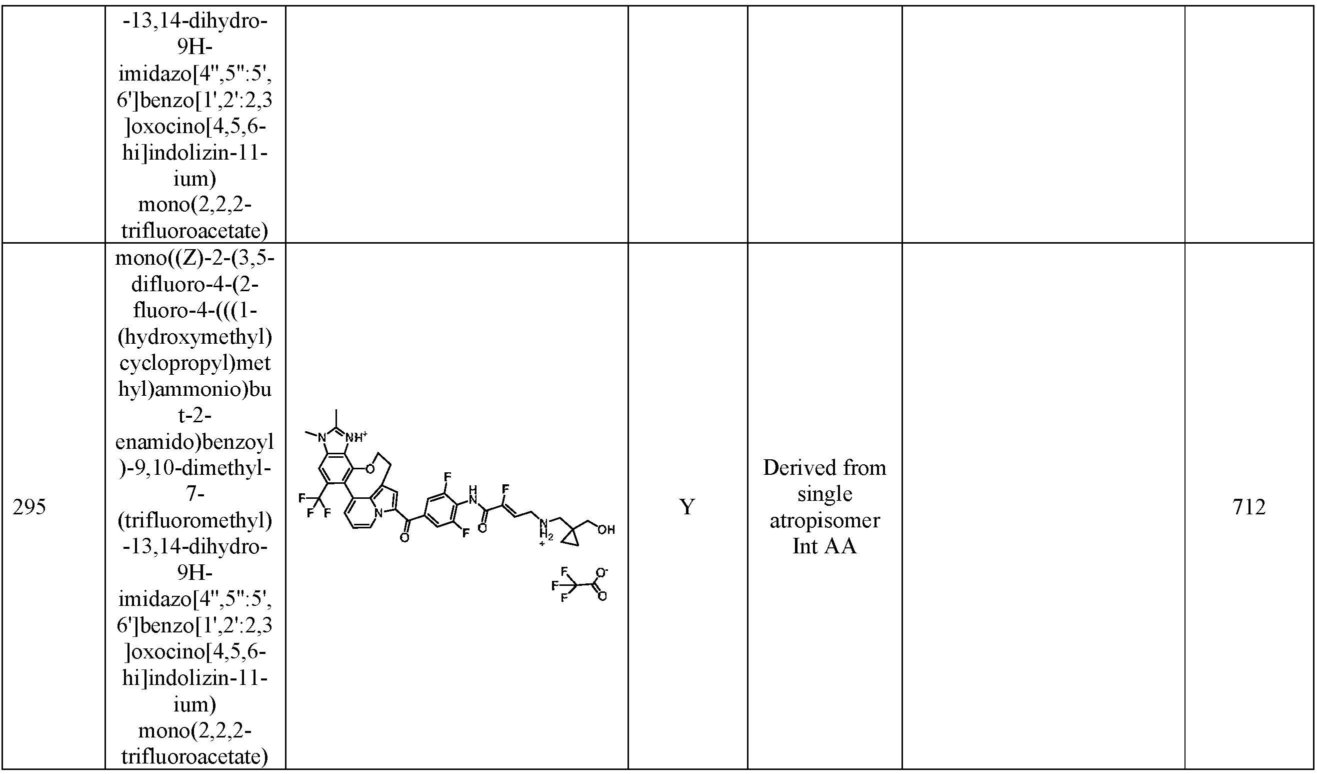

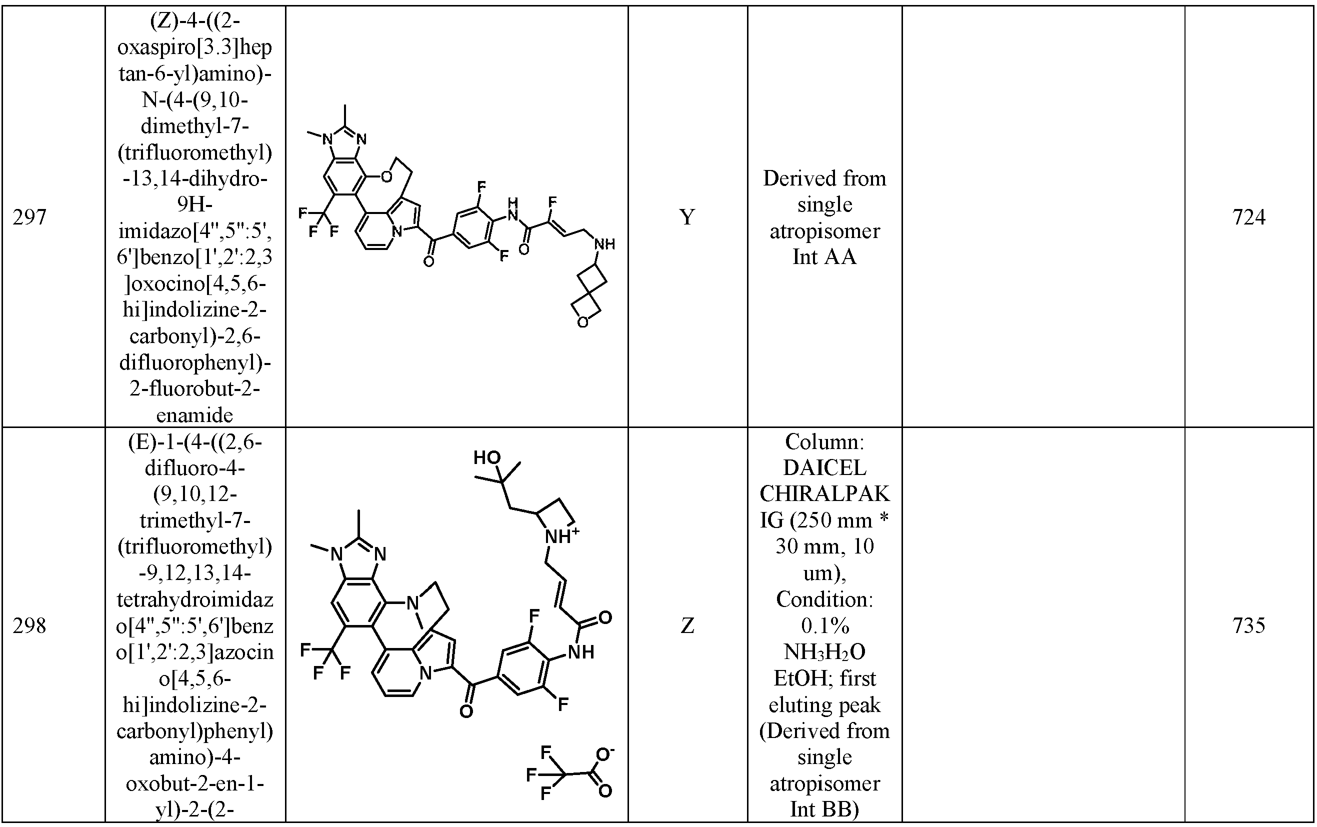

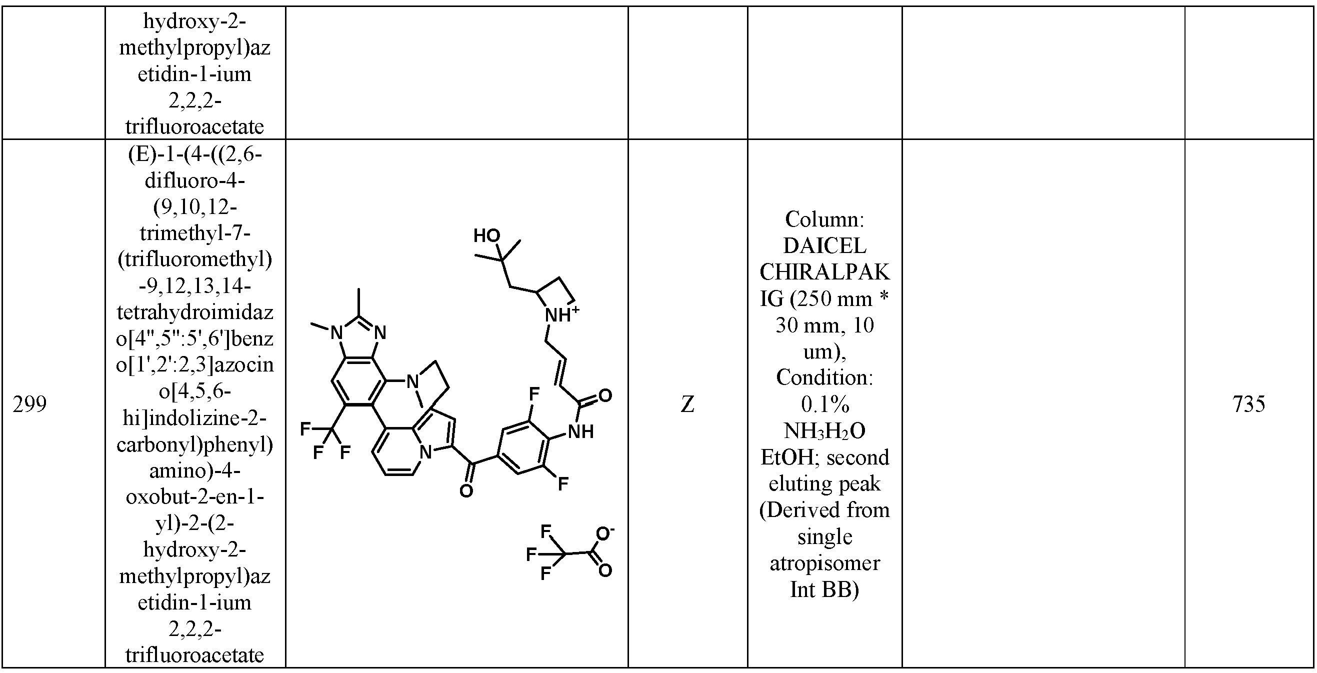

### Test Example 1: KRASG12C binding assay

As test compounds for the KRASG12C binding assay, the compounds obtained in the "Preparation of Compounds" section were used. Sample solutions each containing a test compound were prepared in the form of a 10 mM DMSO solution.

Gppcp-bound recombinant K-Ras4B G12C mutant protein (amino acids 1-169, C51S/C80L/C118S, SEQ ID NO:1) was diluted with a buffer (1×TBS, 0.1 mM TCEP, 0.25 mM MgCl₂, 50 µM Gppcp) to prepare a 50 nM protein solution. K-Ras4B is the major splicing variant of K-Ras. Amino acid sequence of residues 1 to 150 including the compounds' binding region of K-Ras4B is exactly same as that of K-Ras4A, the other splicing variant (Oncotarget. 2016 Jul 19; 7(29): 46717-46733). A sample solution containing 10 mM test compound was diluted ten-fold with DMSO to make a 1 mM sample solution, and then diluted twenty-fold with DMSO to make a 50 µM sample solution. 1 µL of a 50 µM sample solution was added to 100 µL of a 50 nM protein solution to adjust the final concentration of a test compound to be 0.5 µM in the mixture. The mixtures were stored in an incubator at 25°C for 1 hour, and 10 µL of a 1×TBS solution containing 2% formic acid was added to stop the reaction, followed by LC-MS measurement. LC-MS measurement was performed using Xevo G2-S Q-Tof manufactured by Waters, and reverse-phase chromatography was performed with a desalting column. A mass spectrum of positive ions was obtained by electrospray. For a mass spectrum, a spectrum of polyvalent ions was collectively converted to a molecular weight by using OpenLynx software by the MaxEnt technique, and a compound binding rate was calculated from the ratio of the signal intensity of a peak that corresponds to the molecular weight of the protein to the signal intensity of a peak that corresponds to the molecular weight of the protein conjugated with the compound.

The binding assay of the test compounds was conducted at a final concentration of 0.5 µM. A binding rate of 80% or more is rated "A", a binding rate of 60% or more and less than 80% is rated "B", a binding rate of 40% or more and less than 60% is rated "C", a binding rate of 20% or more and less than 40% is rated "D", and a binding rate of less than 20% is rated "E". N.D.=not determined. Binding rate % is the ratio of KRAS signal intensity at the molecular weight of one molecule adduct to the sum of all the KRAS signal intensity (unbound form and adducts) in mass spectrum. Thus, binding rate 100% means that only the one molecule adduct was observed without any unbound KRAS. The following table shows the results.

**Table 1**

| **Example** | | **Example** | |
|---|---|---|---|
| 1-1 | D | 24 | B |
| 1-2 | A | 25 | A |
| 2-1 | D | 26 | A |
| 2-2 | A | 27 | A |
| 3 | A | 28 | A |
| 4-1 | D | 29 | A |
| 4-2 | A | 30-1 | A |
| 5-1 | A | 30-2 | D |
| 5-2 | C | 31-1 | A |
| 6 | A | 31-2 | E |
| 7 | A | 32 | A |
| 8 | A | 33 | A |
| 9 | A | 34 | A |
| 10 | A | 35 | A |
| 11-1 | E | 36 | A |
| 11-2 | A | 37 | A |
| 12-1 | A | 38-1 | E |
| 12-2 | E | 38-2 | A |
| 13 | A | 39 | A |
| 14-1 | A | 40 | B |
| 14-2 | D | 41 | A |
| 15-1 | C | 42 | A |
| 15-2 | A | 42-1 | A |
| 16 | A | 42-2 | B |
| 17 | C | 43 | N.D. |
| 18 | A | 44 | A |
| 19 | D | 45 | A |
| 20 | A | 46 | A |
| 21-1 | N.D. | 47 | A |
| 21-2 | N.D. | 48 | A |
| 22 | A | 49 | A |
| 23 | A | 50 | N.D. |

**Table 1-1**

| **Example** | **IC50 (µM)** | **Example** | **IC50 (µM)** | **Example** | **IC50 (µM)** |
|---|---|---|---|---|---|
| 51 | A | 118 | A | 183 | A |
| 52 | A | 119 | A | 184 | A |
| 53 | A | 120 | A | 185 | A |
| 54 | A | 121 | A | 186 | A |
| 55 | A | 122 | A | 187 | A |
| 56 | A | 123 | A | 188 | B |
| 57 | A | 124 | A | 189 | A |
| 58 | A | 125 | A | 190 | A |
| 59 | A | 126 | A | 191 | B |
| 60 | A | 127 | A | 192 | A |
| 61 | A | 128 | A | 193 | A |
| 62 | A | 129 | A | 194 | A |
| 63 | A | 130 | A | 195 | B |
| 64 | A | 130-1 | A | 196 | B |
| 65 | A | 130-2 | C | 197 | A |
| 66 | A | 131 | A | 198 | A |
| 67 | A | 132 | A | 199 | A |
| 68 | A | 133 | A | 200 | A |
| 69 | A | 134 | A | 201 | A |
| 70 | A | 135 | A | 202 | A |
| 71 | A | 136 | A | 203 | A |
| 72 | A | 137 | A | 204 | A |
| 73 | A | 138 | A | 205 | A |
| 74 | A | 139 | A | 206 | A |
| 75 | A | 140 | A | 207 | A |
| 76 | A | 141 | A | 208 | A |
| 77 | A | 142 | A | 209 | A |
| 78 | A | 143 | A | 210 | A |
| 79 | A | 144 | A | 211 | A |
| 80 | A | 145 | A | 212 | A |
| 81 | A | 146 | A | 213 | A |
| 82 | A | 147 | A | 214 | A |
| 83 | B | 148 | A | 215 | A |
| 84 | A | 149 | A | 216 | A |
| 85 | A | 150 | A | 217 | A |
| 86 | A | 151 | A | 218 | A |
| 87 | A | 152 | A | 219 | A |
| 88 | A | 153 | A | 220 | A |
| 89 | A | 154 | A | 221 | A |
| 90 | A | 155 | A | 222 | A |
| 91 | A | 156 | A | 223 | A |
| 92 | A | 157 | A | 224 | A |
| 93 | A | 158 | B | 225 | A |
| 94 | A | 159 | A | 226 | A |
| 95 | A | 160 | A | 227 | A |
| 96 | A | 161 | A | 228 | A |
| 97 | A | 162 | A | 229 | A |
| 98 | A | 163 | A | 230 | A |
| 99 | N.D. | 164 | A | 231 | N.D. |
| 100 | A | 165 | A | 232 | A |
| 101 | A | 166 | A | 233 | A |
| 102 | A | 167 | A | 234 | A |
| 103 | A | 168 | A | 235 | A |
| 104 | A | 169 | A | 236 | A |
| 105 | A | 170 | A | 237 | A |
| 106 | A | 171 | A | 238 | A |
| 107 | A | 172 | A | 239 | A |
| 108 | A | 173 | A | 240 | A |
| 109 | A | 174 | A | 241 | A |
| 110 | A | 175 | A | 242 | A |
| 111 | A | 176 | A | 243 | A |
| 112 | A | 177 | A | 244 | A |
| 113 | A | 178 | A | 245 | A |
| 114 | A | 179 | A | 246 | A |
| 115 | A | 180 | A | 247 | A |
| 116 | A | 181 | A | 248 | A |
| 117 | A | 182 | A | 249 | A |

### Test Example 2: Evaluation of inhibitory activity of compounds on interaction between KRAS G12C and cRAF (in vitro)

Interaction between recombinant K-Ras4B G12C mutant protein (amino acids 1-169, SEQ ID NO:2) and cRAF RAS-binding domain (cRAF-RBD, amino acids 50-132, Jena Biosciences GmbH) was measured using Alpha technology (PerkinElmer Inc.).

Recombinant KRAS G12C protein with an N-terminal His-tag was expressed in E.coli and purified by affinity chromatography. To prepare GTP-bound form and GDP-bound form of KRAS G12C protein, 50 µM KRAS G12C protein was incubated with 1 mM GMPPNP (Guanosine-5'-[( β,γ )-imido]triphosphate, Tetralithium salt)(Jena Bioscience GmbH) and 1 mM GDP, respectively, in a loading buffer (20 mM Tris-HCl (pH 7.5), 50 mM NaCl, 1 mM DTT and 2.5 mM EDTA) for 1 hour on ice. After the incubation, MgCl₂ was added to a final concentration of 10 mM, followed by incubation at room temperature for 30 minutes. The mixture was allowed to pass through a NAP-5 column to remove free nucleotides and purified, and the resultant nucleotide-bound KRAS G12C protein was used for compound evaluation.

For the measurement of the inhibitory activity of compounds on interaction between KRAS G12C and cRAF-RBD, GMPPNP-bound KRAS G12C protein was incubated with various concentrations of compound in a reaction buffer (20 mM Tris-HCl (pH 7.5), 100 mM NaCl, 1 mM MgCl₂, 1 mM TCEP, 0.1% Tween 20) at 25°C for 1 hour. After the incubation, recombinant cRAF-RBD and Alpha detection reagents were added and incubated at room temperature for 1.5 hours for binding. Interaction of KRAS G12C and cRAF-RBD was monitored by measuring Alpha signal. Inhibition % was calculated using the signal from the reaction without test compound (DMSO control) as 0% inhibition and the signal from the reaction using GDP-bound KRAS G12C in place of GMPPNP-bound KRAS G12C as 100% inhibition. IC50 values were calculated from dose titration curve using curve fitting by XLfit software (IDBS).

**Table 2**

| **Example** | **IC50 (µ M)** | **Example** | **IC50 (µ M)** |
|---|---|---|---|
| 1-1 | >6.7 | 24 | 1.8 |
| 1-2 | 0.18 | 25 | 0.039 |
| 2-1 | >6.7 | 26 | 0.10 |
| 2-2 | 0.50 | 27 | 0.064 |
| 3 | 2.7 | 28 | 0.053 |
| 4-1 | >6.7 | 29 | 0.22 |
| 4-2 | 0.29 | 30-1 | 0.007 |
| 5-1 | 0.053 | 30-2 | 6.0 |
| 5-2 | >6.7 | 31-1 | 0.011 |
| 6 | 0.16 | 31-2 | >6.7 |
| 7 | 0.84 | 32 | 0.027 |
| 8 | 0.15 | 33 | 0.014 |
| 9 | 1.5 | 34 | 0.012 |
| 10 | 0.15 | 35 | 0.013 |
| 11-1 | >6.7 | 36 | 0.013 |
| 11-2 | 0.077 | 37 | 0.040 |
| 12-1 | 0.005 | 38-1 | >6.7 |
| 12-2 | >6.7 | 38-2 | 0.027 |
| 13 | 0.017 | 39 | 0.008 |
| 14-1 | 0.009 | 40 | 4.7 |
| 14-2 | >6.7 | 41 | 0.010 |
| 15-1 | 3.9 | 42 | 0.018 |
| 15-2 | 0.035 | 42-1 | 0.011 |
| 16 | 0.10 | 42-2 | 1.1 |
| 17 | 1.3 | 43 | 0.017 |
| 18 | 0.019 | 44 | 0.030 |
| 19 | >6.7 | 45 | 0.021 |
| 20 | 0.024 | 46 | 0.14 |
| 21-1 | 0.044 | 47 | 0.023 |
| 21-2 | 2.8 | 48 | 0.093 |
| 22 | 0.10 | 49 | 0.26 |
| 23 | 0.019 | 50 | 0.78 |

**Table 2-1**

| **Example** | **IC50 (µM)** | **Example** | **IC50 (µM)** | **Example** | **IC50 (µM)** |
|---|---|---|---|---|---|
| 51 | 0.04537 | 118 | 0.01257 | 183 | 0.03515 |
| 52 | 0.00455 | 119 | 0.01686 | 184 | 0.04828 |
| 53 | 0.02216 | 120 | 0.00559 | 185 | 0.04082 |
| 54 | 0.02209 | 121 | 0.02301 | 186 | 0.01672 |
| 55 | 0.01173 | 122 | 0.05409 | 187 | 0.03643 |
| 56 | 0.01239 | 123 | 0.04179 | 188 | 0.07248 |
| 57 | 0.07828 | 124 | 0.01301 | 189 | 0.01522 |
| 58 | 0.07789 | 125 | 0.00375 | 190 | 0.01026 |
| 59 | 0.02763 | 126 | 0.01798 | 191 | 0.09711 |
| 60 | 0.07317 | 127 | 0.0168 | 192 | 0.09149 |
| 61 | 0.07627 | 128 | 0.01877 | 193 | 0.03956 |
| 62 | 0.0174 | 129 | 0.0088 | 194 | 0.01028 |
| 63 | 0.01422 | 130 | 0.04144 | 195 | 0.03944 |
| 64 | 0.01416 | 130-1 | 0.02627 | 196 | 0.03508 |
| 65 | 0.06955 | 130-2 | 3.76266 | 197 | 0.01169 |
| 66 | 0.05343 | 131 | 0.02788 | 198 | 0.05709 |
| 67 | 0.02201 | 132 | 0.01082 | 199 | 0.01011 |
| 68 | 0.09116 | 133 | 0.01144 | 200 | 0.01372 |
| 69 | 0.01983 | 134 | 0.03099 | 201 | 0.01756 |
| 70 | 0.00985 | 135 | 0.01098 | 202 | 0.01301 |
| 71 | 0.02022 | 136 | 0.02635 | 203 | 0.0204 |
| 72 | 0.09775 | 137 | 0.03352 | 204 | 0.03636 |
| 73 | 0.04693 | 138 | 0.04717 | 205 | 0.02949 |
| 74 | 0.03333 | 139 | 0.05895 | 206 | 0.01536 |
| 75 | 0.01594 | 140 | 0.00413 | 207 | 0.02693 |
| 76 | 0.02218 | 141 | 0.00971 | 208 | 0.03543 |
| 77 | 0.08039 | 142 | 0.02813 | 209 | 0.04688 |
| 78 | 0.03369 | 143 | 0.01785 | 210 | 0.03813 |
| 79 | 0.03282 | 144 | 0.02243 | 211 | 0.05185 |
| 80 | 0.02834 | 145 | 0.07228 | 212 | 0.01626 |
| 81 | 0.02078 | 146 | 0.02035 | 213 | 0.06209 |
| 82 | 0.02495 | 147 | 0.00749 | 214 | 0.01154 |
| 83 | 0.03387 | 148 | 0.02323 | 215 | 0.01835 |
| 84 | 0.03988 | 149 | 0.02372 | 216 | 0.05762 |
| 85 | 0.06095 | 150 | 0.01714 | 217 | 0.01971 |
| 86 | 0.02233 | 151 | 0.01551 | 218 | 0.027 |
| 87 | 0.01823 | 152 | 0.08932 | 219 | 0.03853 |
| 88 | 0.03951 | 153 | 0.03154 | 220 | 0.02438 |
| 89 | 0.01596 | 154 | 0.01024 | 221 | 0.03925 |
| 90 | 0.01722 | 155 | 0.00895 | 222 | 0.07581 |
| 91 | 0.02722 | 156 | 0.00806 | 223 | 0.02006 |
| 92 | 0.01994 | 157 | 0.06762 | 224 | 0.02821 |
| 93 | 0.03363 | 158 | 0.07762 | 225 | 0.01008 |
| 94 | 0.07156 | 159 | 0.00722 | 226 | 0.06331 |
| 95 | 0.03117 | 160 | 0.00571 | 227 | 0.03405 |
| 96 | 0.03065 | 161 | 0.06324 | 228 | 0.03247 |
| 97 | 0.03043 | 162 | 0.02243 | 229 | 0.01271 |
| 98 | 0.01115 | 163 | 0.03354 | 230 | 0.02007 |
| 99 | 0.01223 | 164 | 0.0215 | 231 | 0.10113 |
| 100 | 0.03451 | 165 | 0.00375 | 232 | 0.04972 |
| 101 | 0.02735 | 166 | 0.03366 | 233 | 0.05263 |
| 102 | 0.01124 | 167 | 0.02786 | 234 | 0.02053 |
| 103 | 0.09924 | 168 | 0.0438 | 235 | 0.02187 |
| 104 | 0.04122 | 169 | 0.05964 | 236 | 0.01649 |
| 105 | 0.01915 | 170 | 0.01414 | 237 | 0.01138 |
| 106 | 0.01802 | 171 | 0.00634 | 238 | 0.00876 |
| 107 | 0.02367 | 172 | 0.01041 | 239 | 0.01817 |
| 108 | 0.05418 | 173 | 0.00702 | 240 | 0.00893 |
| 109 | 0.02475 | 174 | 0.00741 | 241 | 0.03097 |
| 110 | 0.03566 | 175 | 0.09043 | 242 | 0.01783 |
| 111 | 0.03135 | 176 | 0.06744 | 243 | 0.08315 |
| 112 | 0.02925 | 177 | 0.03954 | 244 | 0.0155 |
| 113 | 0.02956 | 178 | 0.03242 | 245 | 0.03309 |
| 114 | 0.06106 | 179 | 0.04159 | 246 | 0.01363 |
| 115 | 0.03894 | 180 | 0.04933 | 247 | 0.02343 |
| 116 | 0.02656 | 181 | 0.04805 | 248 | 0.018 |
| 117 | 0.02048 | 182 | 0.03518 | 249 | 0.02747 |

### Test Example 3: Assay of growth inhibition activity on KRAS-G12C mutant cell line (MIA PaCa-2) (in vitro)

MIA PaCa-2 cells (given by Sumitomo Dainippon Pharma Co., Ltd.), which are a KRAS-G12C mutant human pancreas cancer cell line, were suspended in a 10% fetal bovine serum-containing RPMI1640 medium (manufactured by Fujifilm Wako Pure Chemical Corporation.). The cell suspension was seeded into each well of a 384-well U bottom microplate and cultured in an incubator containing 5% CO₂ gas at 37°C for 1 day. The compounds obtained in the "Preparation of Compounds" section were used as test compounds and were dissolved in DMSO, respectively, and each test compound was diluted with DMSO to give a concentration 500 times the final concentration. The resultant solution of the test compound in DMSO was diluted with the medium used for suspending cells and added to each well of the cell-culture plate to give a DMSO final concentration of 0.2%, followed by culture in an incubator containing 5% CO₂ gas at 37°C for another 3 days. The cell count after 3-day culture in the presence of the test compound was measured using CellTiter-Glo 3D Reagent (manufactured by Promega Corporation). All wells were added with CellTiter-Glo 3D Reagent and mixed for 10 minutes. 30 minutes after mixing, luminescence was measured by a plate reader. The growth inhibition rate was calculated from the following equation, and the concentration of the test compound at which 50% inhibition was achieved (IC50 (nM)) was determined. $Growth Inhibition Rate \left(%\right) = \left(C-T\right) / \left(C\right) \times 100$
T: the emission intensity in a well into which a test compound was added.
C: the emission intensity in a well into which a test compound was not added.

**Table 3**

| **Example** | **IC50 (µ M)** | **Example** | **IC50 (µ M)** |
|---|---|---|---|
| 1-1 | 7.7 | 24 | 1.1 |
| 1-2 | 0.16 | 25 | 0.024 |
| 2-1 | 3.1 | 26 | 0.13 |
| 2-2 | 0.39 | 27 | 0.088 |
| 3 | 4.4 | 28 | 0.017 |
| 4-1 | 3.6 | 29 | 0.053 |
| 4-2 | 0.26 | 30-1 | 0.003 |
| 5-1 | 0.014 | 30-2 | 1.6 |
| 5-2 | 1.8 | 31-1 | 0.007 |
| 6 | 0.67 | 31-2 | 9.2 |
| 7 | 1.1 | 32 | 0.086 |
| 8 | 0.16 | 33 | 0.005 |
| 9 | 0.76 | 34 | 0.007 |
| 10 | 0.23 | 35 | 0.014 |
| 11-1 | 7.8 | 36 | 0.15 |
| 11-2 | 0.091 | 37 | 0.028 |
| 12-1 | 0.001 | 38-1 | 8.3 |
| 12-2 | 1.6 | 38-2 | 0.019 |
| 13 | 0.059 | 39 | 0.012 |
| 14-1 | 0.017 | 40 | 1.6 |
| 14-2 | 3.7 | 41 | 0.004 |
| 15-1 | 2.7 | 42 | 0.009 |
| 15-2 | 0.027 | 42-1 | 0.006 |
| 16 | 0.056 | 42-2 | 0.45 |
| 17 | 0.82 | 43 | 0.014 |
| 18 | 0.021 | 44 | 0.010 |
| 19 | 3.3 | 45 | 0.005 |
| 20 | 0.049 | 46 | 0.057 |
| 21-1 | 0.022 | 47 | 0.059 |
| 21-2 | 1.5 | 48 | N.D. |
| 22 | 0.10 | 49 | 0.15 |
| 23 | 0.013 | 50 | N.D. |

**Table 3-1**

| **Example** | **IC50 (µM)** | **Example** | **IC50 (µM)** | **Example** | **IC50 (µM)** |
|---|---|---|---|---|---|
| 51 | 0.0159 | 118 | 0.0042 | 183 | 0.11208 |
| 52 | 0.00144 | 119 | 0.00939 | 184 | 0.02811 |
| 53 | 0.0319 | 120 | 0.00652 | 185 | 0.07572 |
| 54 | 0.00539 | 121 | 0.02637 | 186 | 0.0103 |
| 55 | 0.00336 | 122 | 0.08817 | 187 | 0.03902 |
| 56 | 0.01264 | 123 | 0.03504 | 188 | 0.03967 |
| 57 | 0.06851 | 124 | 0.01371 | 189 | 0.0061 |
| 58 | 0.0702 | 125 | 0.00906 | 190 | 0.00908 |
| 59 | 0.01979 | 126 | 0.01429 | 191 | 0.16689 |
| 60 | 0.03215 | 127 | 0.02253 | 192 | 0.02426 |
| 61 | 0.01631 | 128 | 0.01178 | 193 | 0.06949 |
| 62 | 0.00691 | 129 | 0.00711 | 194 | 0.00638 |
| 63 | 0.00248 | 130 | 0.08282 | 195 | 0.01931 |
| 64 | 0.01097 | 130-1 | 0.01741 | 196 | 0.03148 |
| 65 | 0.0951 | 130-2 | 3.40762 | 197 | 0.0109 |
| 66 | 0.03011 | 131 | 0.04085 | 198 | 0.02139 |
| 67 | 0.00822 | 132 | 0.01665 | 199 | 0.00367 |
| 68 | 0.01639 | 133 | 0.00645 | 200 | 0.00462 |
| 69 | 0.01546 | 134 | 0.24428 | 201 | 0.00583 |
| 70 | 0.00249 | 135 | 0.02893 | 202 | 0.01023 |
| 71 | 0.0096 | 136 | 0.03614 | 203 | 0.01104 |
| 72 | 1.17161 | 137 | 0.01493 | 204 | 0.11821 |
| 73 | 0.02163 | 138 | 0.03084 | 205 | 0.00855 |
| 74 | 0.00822 | 139 | 0.05789 | 206 | 0.01032 |
| 75 | 0.00948 | 140 | 0.00759 | 207 | 0.01124 |
| 76 | 0.00714 | 141 | 0.03432 | 208 | 0.01136 |
| 77 | 0.05402 | 142 | 0.08552 | 209 | 0.01866 |
| 78 | 0.00692 | 143 | 0.0477 | 210 | 0.06028 |
| 79 | 0.0106 | 144 | 0.03025 | 211 | 0.01085 |
| 80 | 0.02225 | 145 | 0.1622 | 212 | 0.00416 |
| 81 | 0.03342 | 146 | 0.01963 | 213 | 0.02534 |
| 82 | 0.02989 | 147 | 0.01527 | 214 | 0.0011 |
| 83 | 0.01538 | 148 | 0.01906 | 215 | 0.0034 |
| 84 | 0.04956 | 149 | 0.02341 | 216 | 0.02327 |
| 85 | 0.0309 | 150 | 0.03121 | 217 | 0.00622 |
| 86 | 0.00611 | 151 | 0.03394 | 218 | 0.01421 |
| 87 | 0.00327 | 152 | 0.92746 | 219 | 0.01258 |
| 88 | 0.00692 | 153 | 0.11759 | 220 | 0.00652 |
| 89 | 0.00437 | 154 | 0.00793 | 221 | 0.02088 |
| 90 | 0.02161 | 155 | 0.01346 | 222 | 1.93832 |
| 91 | 0.00868 | 156 | 0.01035 | 223 | 0.02366 |
| 92 | 0.01208 | 157 | 0.06213 | 224 | 0.01783 |
| 93 | 0.05165 | 158 | 0.09544 | 225 | 0.00718 |
| 94 | 0.07301 | 159 | 0.00816 | 226 | 0.01352 |
| 95 | 0.01139 | 160 | 0.00925 | 227 | 0.01019 |
| 96 | 0.01446 | 161 | 0.02164 | 228 | 0.03581 |
| 97 | 0.00791 | 162 | 0.01524 | 229 | 0.01128 |
| 98 | 0.00394 | 163 | 0.04267 | 230 | 0.01445 |
| 99 | 0.00979 | 164 | 0.01373 | 231 | 0.07343 |
| 100 | 0.0234 | 165 | 0.00544 | 232 | 0.018 |
| 101 | 0.02651 | 166 | 0.05124 | 233 | 0.01402 |
| 102 | 0.01051 | 167 | 0.01637 | 234 | 0.00708 |
| 103 | 0.30287 | 168 | 0.01559 | 235 | 0.017 |
| 104 | 0.01553 | 169 | 0.01496 | 236 | 0.00403 |
| 105 | 0.01545 | 170 | 0.04773 | 237 | 0.00277 |
| 106 | 0.02195 | 171 | 0.01349 | 238 | 0.0027 |
| 107 | 0.02966 | 172 | 0.01621 | 239 | 0.00969 |
| 108 | 0.33229 | 173 | 0.01058 | 240 | 0.01447 |
| 109 | 0.01947 | 174 | 0.00745 | 241 | 0.87722 |
| 110 | 0.05583 | 175 | 0.02335 | 242 | 0.01671 |
| 111 | 0.00895 | 176 | 0.02506 | 243 | 0.09178 |
| 112 | 0.01005 | 177 | 0.03846 | 244 | 0.01004 |
| 113 | 0.01058 | 178 | 0.04135 | 245 | 0.02535 |
| 114 | 0.06452 | 179 | 0.13915 | 246 | 0.01723 |
| 115 | 0.06131 | 180 | 0.01268 | 247 | 0.02854 |
| 116 | 0.02987 | 181 | 0.02097 | 248 | 0.02175 |
| 117 | 0.05529 | 182 | 0.02782 | 249 | 0.02861 |

### Test Example 4: Procedure for RAS:RAF-RBD binding assay

The RAF-Ras binding domain (RBD) protein interaction assay utilizes recombinant biotinylated KRAS protein containing a G12C mutation (SEQ ID NO:3) and the GST-tagged Ras binding domain of c-RAF (residues 50-132) from Jena Biosciences (catalog #PR-366). It should be noted that KRAS-G12C protein is pre-loaded with a GTP analog, GMP-PNP to mimic KRAS in the activated state. Compounds are added to KRAS and then after a 30-minute incubation time the RAF-RBD and detection antibodies are added. Small molecule inhibitors that block the interaction of c-RAF-RBD prevent generation of a TR-FRET signal.

Biotinylated KRAS G12C protein is diluted to 20 nM in assay buffer (20 mM HEPES pH 7.5, 150 mM sodium chloride, 10 mM magnesium chloride, and 0.01% Tween20).

Each test compound (10 mM stock in DMSO) is diluted in DMSO to make a 10-point, 3-fold dilution series in a 384-well low dead volume microplate (Labcyte, catalog# LP-0200). Once titrations are made, 50 nL of the diluted compounds is acoustically dispensed into 384-well plates (Corning, catalog# 3820) using an Echo 655 (Labcyte).

Each well of the assay plate receives 5 µL of Biotinylated KRAS G12C assay solution and is incubated at room temperature for 30 minutes. Each well then receives 5 µL of 100 nM GST-c-RAF RBD protein and a 1:100 dilution of both anti-GST-d2 (Cisbio catalog # 61GSTDLA) and Strepavidin-Tb cryptate (Cisbio catalog #610SATLA) in assay buffer and the plate is mixed and briefly centrifuged followed by a 60 minute incubation at room temperature.

The time-resolved fluorescence resonance energy transfer signal of both plates is measured on an Envision (PerkinElmer) plate reader: dichroic mirror = LANCE/DELFIA DUAL/Bias; Emission1 = 615 nm; Emission2 = 665 nm; delay time = 60 ms. The signal of each well is determined as the ratio of the emission at 665 nm to that at 615 nm. Percent effect of each well is determined after normalization to control wells containing DMSO (no effect) or a saturating concentration of inhibitor (max effect). The apparent effect as a function of compound concentration is fit to a four-parameter logistic equation. Table 4 shows the results.

**Table 4**

| **Example** | **IC50 (µ M)** | **Example** | **IC50 (µ M)** |
|---|---|---|---|
| 250 | 0.05487 | 277 | 0.02636 |
| 251 | 0.01674 | 278 | 0.04432 |
| 252 | 0.0257 | 279 | 0.005733 |
| 253 | 0.02475 | 280 | 0.03768 |
| 254 | 0.02095 | 281 | 0.003805 |
| 255 | 0.01172 | 282 | 0.05759 |
| 256 | 0.0047 | 283 | 0.0647 |
| 257 | 0.009693 | 284 | 0.04041 |
| 258 | 0.006686 | 285 | 0.08501 |
| 259 | 0.01425 | 286 | 0.0928 |
| 260 | 0.01171 | 287 | 0.01662 |
| 261 | 0.02225 | 288 | 0.008149 |
| 262 | 0.01514 | 289 | 0.0688 |
| 263 | 0.009252 | 290 | 0.01111 |
| 264 | 0.007344 | 291 | 0.005545 |
| 265 | 0.01233 | 292 | 0.08488 |
| 266 | 0.01609 | 293 | 0.01604 |
| 267 | 0.003686 | 294 | 0.05957 |
| 268 | 0.01277 | 295 | 0.02528 |
| 269 | 0.009825 | 296 | 0.01484 |
| 270 | 0.01205 | 297 | 0.02668 |
| 271 | 0.0136 | 298 | 0.04158 |
| 272 | 0.01487 | 299 | 0.05247 |
| 273 | 0.03005 | 300 | 0.001219 |
| 274 | 0.02036 | | |
| 275 | 0.05751 | | |
| 276 | 0.008047 | | |

### Sequences

Gppcp-bound recombinant K-Ras4B G12C mutant protein (amino acids 1-169, C51S/C80L/C118S) (SEQ ID NO: 1): 
GMTEYKLVVVGACGVGKSALTIQLIQNHFVDEYDPTIEDSYRKQVVIDGE
TSLLDILDTAGQEEYSAMRDQYMRTGEGFLLVFAINNTKSFEDIHHYREQI
KRVKDSEDVPMVLVGNKSDLPSRTVDTKQAQDLARSYGIPFIETSAKTRQ
GVDDAFYTLVREIRKHKEK
Recombinant K-Ras4B G12C mutant protein (SEQ ID NO: 2): 
MASSHHHHHHSSENLYFQGMTEYKLVVVGACGVGKSALTIQLIQNHFVD
EYDPTIEDSYRKQVVIDGETCLLDILDTAGQEEYSAMRDQYMRTGEGFLC
VFAINNTKSFEDIHHYREQIKRVKDSEDVPMVLVGNKCDLPSRTVDTKQA
QDLARSYGIPFIETSAKTRQGVDDAFYTLVREIRKHKEK
Gppnp-bound recombinant K-Ras4B G12C mutant protein (SEQ ID NO: 3):

GLNDIFEAQKIEWHETEYKLVVVGACGVGKSALTIQLIQNHFVDEYDPTIE
DSYRKQVVIDGETSLLDILDTAGQEEYSAMRDQYMRTGEGFLLVFAINNT
KSFEDIHHYREQIKRVKDSEDVPMVLVGNKSDLPSRTVDTKQAQDLARSY
GIPFIETSAKTRQGVDDAFYTLVREIRKHKEK

## Claims

1. A compound of the Formula (I) 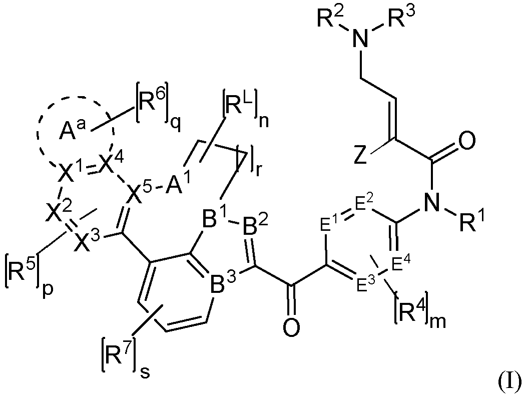 wherein:
R¹ is selected from the group consisting of H and C₁-C₆ alkyl;
R² and R³ are independently selected from the group consisting of:
(i) H;
(ii) C₁-C₆ alkyl, wherein the C₁-C₆ alkyl is unsubstituted or substituted by 1 to 2 R^{a} substituents independently selected from the group consisting of halo, hydroxy, C₁-C₃ alkoxy, C2-C6 alkenyl, C₂-C₆ alkynyl, C₁-C₃ alkylamino, C₁-C₃ dialkylamino, C₁-C₃ alkylsulfonyl, C2-C4 acyl, C₁-C₃ hydroxyalkyl, C₁-C₃ alkoxycarbonyl, C₁-C₃ hydroxycarbonyl, oxo (=O), C3-C8 cycloalkyl, and 4-to 10-membered mono- or bicyclic heterocycloalkyl;
wherein the C₃-C₈ cycloalkyl or the 4-to 10-membered mono- or bicyclic heterocycloalkyl of R^{a} is unsubstituted or substituted by 1 to 2 substituents selected from the group consisting of halo, hydroxy, amino, C₁-C₃ alkyl, C₁-C₃ fluoroalkyl, and C₁-C₃ alkoxy; and
(iii) a 3- to 8-membered monocyclic, bridged bicyclic, or spirocyclic saturated ring system containing 0 to 2 heteroatom groups selected from the group consisting of N, O, S, S(O), S(O)₂, P, P(O), and P(O)₂;
wherein the 3- to 8-membered monocyclic, bridged bicyclic, or spirocyclic saturated ring system is unsubstituted or substituted by 1 to 4 substituents independently selected from the group consisting of halo, hydroxy, C₁-C₃ alkyl, C₁-C₃ fluoroalkyl, C₁-C₃ alkoxy, C₂-C₆ alkenyl, C₂-C₆ alkynyl, C₁-C₃ alkylamino, C₁-C₃ dialkylamino, C₁-C₃ alkylsulfonyl, C₂-C₄ acyl, C₁-C₃ hydroxyalkyl, C₁-C₃ alkoxy(C₁-C₃)alkyl, carboxy, and C₁-C₃ alkoxycarbonyl;
or alternatively, R² and R³ together with the N atom to which they are attached form a 4-to 9-membered monocyclic, fused bicyclic, bridged bicyclic, or spirocyclic saturated ring system containing 0 to 2 additional heteroatoms selected from the group consisting of N, O, and S;
wherein the 4- to 9-membered monocyclic, fused bicyclic, bridged bicyclic, or spirocyclic saturated ring system is unsubstituted or substituted by 1 to 4 substituents independently selected from the group consisting of halo, hydroxy, C₁-C₃ alkyl, C₁-C₃ fluoroalkyl, C₁-C₃ alkoxy, C₁-C₄ hydroxyalkyl, C₃-C₅ hydroxycycloalkyl, - (CH₃)₂N(C=O)CH₂-, CH₃(C=O)NHCH₂-, and C₁-C₃ alkoxy(C₁-C₃)alkyl;
or alternatively, one of R² and R³ together with the N atom to which it is attached and an adjacent C atom form a 3- to 6-membered monocyclic saturated ring system containing 0 to 2 additional heteroatoms selected from the group consisting of N, O, and S;
wherein the 3- to 6- membered monocyclic saturated ring system is unsubstituted or substituted by 1 to 4 substituents independently selected from the group consisting of halo, hydroxy, C₁-C₃ alkyl, C₁-C₃ fluoroalkyl, C₁-C₃ alkoxy, C₁-C₃ hydroxyalkyl, and C₁-C₃ alkoxy(C₁-C₃)alkyl;
at each occurrence R⁴ is independently selected from the group consisting of halo, cyano, C₁-C₃ alkyl, C₁-C₃ fluoroalkyl, C₁-C₃ alkoxy, and C₁-C₄ cyanoalkyl;
E¹, E², E³, and E⁴ are independently selected from the group consisting of N and C, with the proviso that no more than three of E¹, E², E³, and E⁴ are N;
one of B¹ and B³ is C and the other is N;
B² is N or C(R^{B}), wherein R^{B} is selected from the group consisting of H, C₁-C₆ alkyl, C2-C6 alkenyl, C₂-C₆ alkynyl and C₃-C₆ cycloalkyl;
at each occurrence R^{L} is independently selected from the group consisting of fluoro, hydroxy, C₁-C₆ alkyl, C₁-C₃ alkoxy, C₁-C₃ fluoroalkyl, oxo (=O), C₂-C₄ acyl, and methenyl (=CH₂), or
alternatively, two geminally substituted R^{L} substituents together with the carbon atom to which they are attached form a 3- to 6-membered spirocyclic ring containing 0 to 1 heteroatoms selecting from the group consisting of N, O, and S;
ring A^{a} is present or absent, and if present, is a 5- to 6-memberered heteroaryl containing 1 to 3 heteroatoms selected from the group consisting of N, O, and S;
X¹, X², X³, X⁴, and X⁵ are independently selected from the group consisting of N and C, with the proviso that no more than two of X¹, X², X³, X⁴, and X⁵ are N;
at each occurrence R⁵ is independently selected from the group consisting of C₁-C₆ alkyl, C₁-C₆ fluoroalkyl, C₁-C₃ alkoxy, C₁-C₃ acyl, halo, hydroxy, amino, cyano, oxo (=O), C₁-C₄ cyanoalkyl, C₁-C₃ hydroxyalkyl, C₁-C₃ alkylamino, C₁-C₃ dialkylamino, C₁-C₃ alkoxy(C₁-C₃)alkyl, and a group of the formula 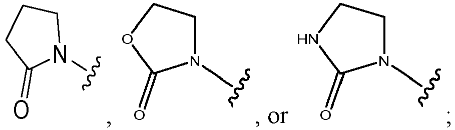
at each occurrence R⁶ is independently selected from the group consisting of C₁-C₃ alkyl, C₁-C₃ fluoroalkyl, C₁-C₃ alkoxy, halo, hydroxy, oxo (=O), cyano, carbamoyl, C₁-C₄ cyanoalkyl, C₁-C₃ hydroxyalkyl, C₁-C₃ alkoxy(C₁-C₃)alkyl, , C₃-C₆ cycloalkyl, and C₁-C₄ carbamoylalkyl;
each occurrence of R⁷ is independently selected from the group consisting of halo, C₁-C3 alkyl, C₁-C₃ fluoroalkyl, and C₁-C₃ alkoxy;
A¹ is selected from the group consisting of -CH₂-, -CH₂- N(R^{L})-, -C(=O)- N(R^{L})-, - CH₂-O-, -C(H)(R^{L})-, -N(H)-, -N(R^{L})-, -S-, -S(=O)-, and -O-;
Z is selected from the group consisting of H and halo;
subscript m is 0, 1, 2, or 3;
subscript n is 0, 1, 2, or 3;
subscript p is 0, 1, 2, 3, or 4;
subscript q is 0, 1, 2, or 3;
subscript r is 0, 1, 2, or 3; and
subscript s is 0, 1, 2, or 3,
or a pharmaceutically acceptable salt thereof.

2. The compound of claim 1 or the pharmaceutically acceptable salt thereof, wherein:
the C₁-C₆ alkyl is unsubstituted or substituted by 1 to 2 R^{a} substituents independently selected from the group consisting of halo, hydroxy, C₁-C₃ alkoxy, C₂-C₆ alkenyl, C₂-C₆ alkynyl, C₁-C₃ alkylamino, C₁-C₃ dialkylamino, C₁-C₃ alkylsulfonyl, C₂-C₄ acyl, C₁-C₃ hydroxyalkyl, C₁-C₃ alkoxycarbonyl, C₁-C₃ hydroxycarbonyl, C₃-C₈ cycloalkyl, and 4-to 10-membered mono- or bicyclic heterocycloalkyl;
R² and R³ together with the N atom to which they are attached form the 4- to 8-membered saturated heterocycloalkyl having 0 to 2 additional heteroatoms selected from the group consisting of N, O, and S;
wherein the 4- to 8-membered heterocycloalkyl is unsubstituted or substituted by 1 to 4 substituents independently selected from the group consisting of halo, hydroxy, C₁-C₃ alkyl, C₁-C₃ fluoroalkyl, C₁-C₃ alkoxy, C₁-C₃ hydroxyalkyl, and C₁-C₃ alkoxy(C₁-C₃)alkyl;
at each occurrence R^{L} is independently selected from the group consisting of fluoro, hydroxy, C₁-C₆ alkyl, C₁-C₃ alkoxy, C₁-C₃ fluoroalkyl, oxo (=O), and methenyl (=CH₂),
the ring A^{a} is present or absent, and if present, is the 5- to 6-memberered heteroaryl containing 1 to 2 heteroatoms selected from the group consisting of N, O, and S;
at each occurrence R⁵ is independently selected from the group consisting of C₁-C₆ alkyl, C₁-C₆ fluoroalkyl, C₁-C₃ alkoxy, C₁-C₃ acyl, halo, hydroxy, amino, cyano, oxo (=O), C₁-C₄ cyanoalkyl, C₁-C₃ hydroxyalkyl, C₁-C₃ alkylamino, C₁-C₃ dialkylamino, C₁-C₃ alkoxy(C1-C3)alkyl, and the group of the formula 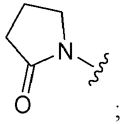
at each occurrence R⁶ is independently selected from the group consisting of C₁-C₃ alkyl, C₁-C₃ fluoroalkyl, C₁-C₃ alkoxy, halo, hydroxy, oxo (=O), cyano, carbamoyl, C₁-C₄ cyanoalkyl, C₁-C₃ hydroxyalkyl, C₁-C₃ alkoxy(C₁-C₃)alkyl, and C₁-C₄ carbamoylalkyl;
A¹ is selected from the group consisting of -CH₂-, -C(H)(R^{L})-, -N(H)-, -N(R^{L})-, and - O-;
Z is H; and
subscript q is 0, 1, or 2.

3. The compound of claim 1 or the pharmaceutically acceptable salt thereof, wherein
(a) the group 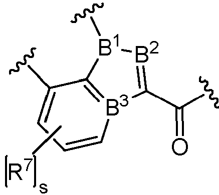 is 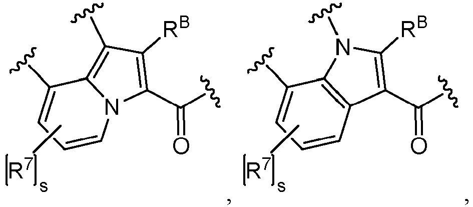 or 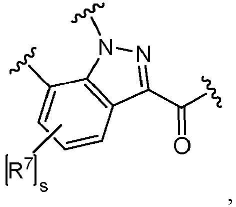 or
(b) the group 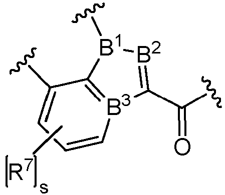 is 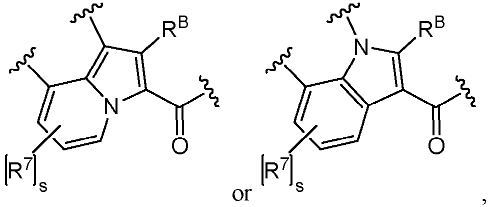 optionally wherein R^{B} is H or methyl, and the subscript s is 0.

4. The compound of claim 1 or the pharmaceutically acceptable salt thereof, wherein:
X¹ is N or C; and
X², X³, X⁴, and X⁵ are C.

5. The compound of claim 1 or the pharmaceutically acceptable salt thereof, wherein
(a) the group 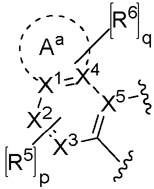 is 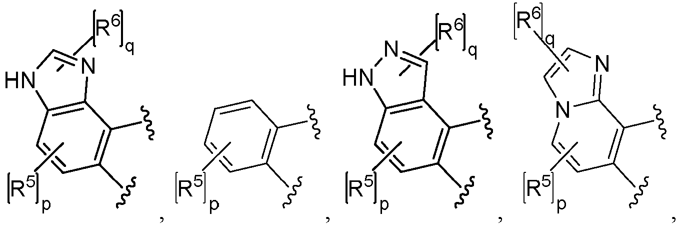 or 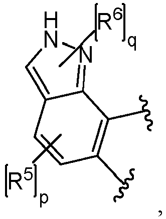 or
(b) the group 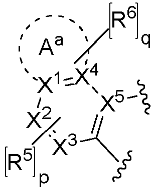 is 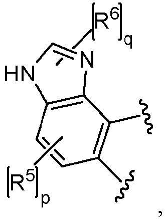 or
(c) the group 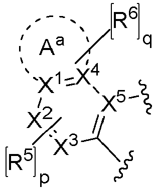 is 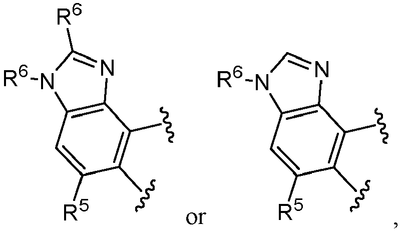 wherein
R⁵ is C₁-C₃ alkyl or C₁-C₃ fluoroalkyl; and
R⁶ is C₁-C₃ alkyl, C₁-C₃ fluoroalkyl, or C₁-C₃ alkoxy(C₁-C₃)alkyl.

6. The compound of claim 1 or the pharmaceutically acceptable salt thereof, wherein the group 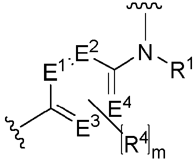 is

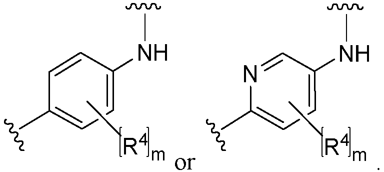

7. The compound of claim 1 or the pharmaceutically acceptable salt thereof, wherein
(a) the group 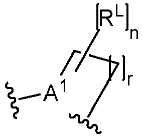 is 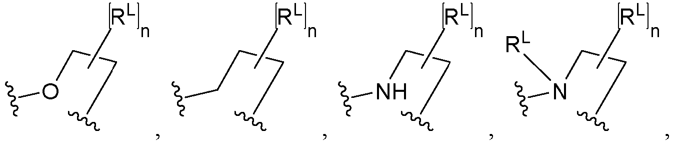 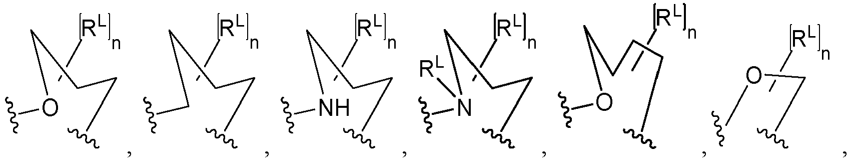 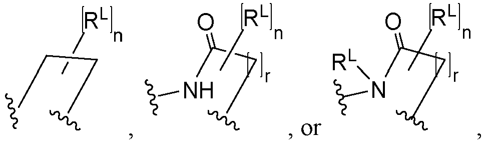 or
(b) the group 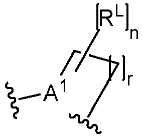 is 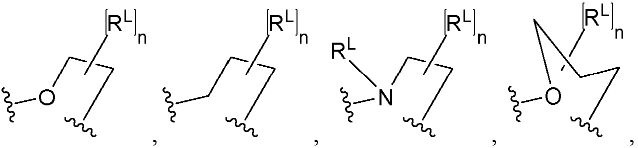 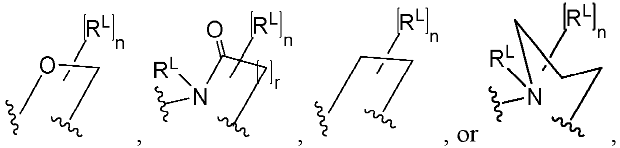 or
(c) the group 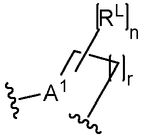 is

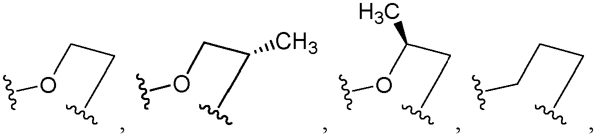

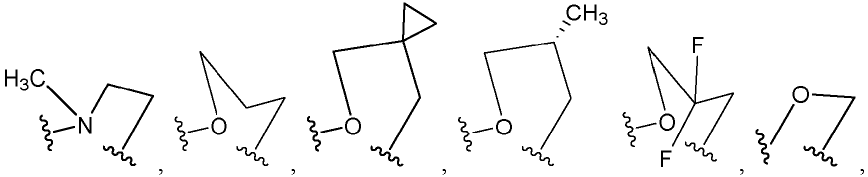

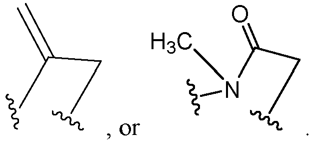

8. The compound of claim 1 or the pharmaceutically acceptable salt thereof, wherein
(a) the group 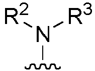 is 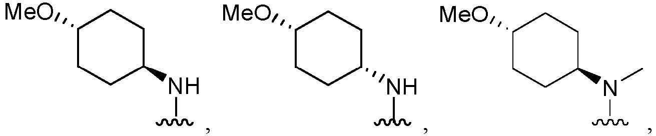 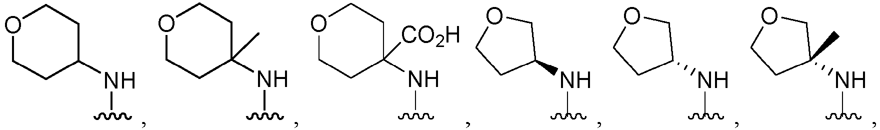 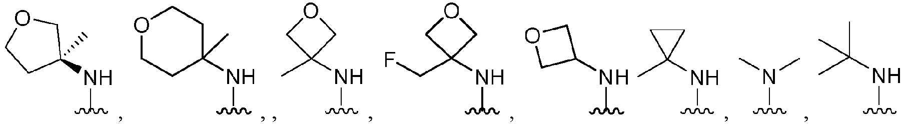 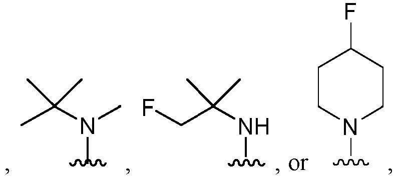 or
(b) the group 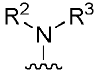 is

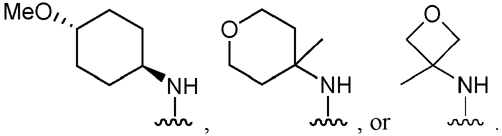

9. The compound of claim 1 or the pharmaceutically acceptable salt thereof, wherein R² and R³ are independently H, C₁-C₆ alkyl, or C₁-C₆ fluoroalkyl.

10. The compound of claim 1 or the pharmaceutically acceptable salt thereof, wherein:
(i) the group 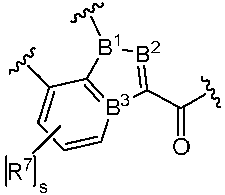 is 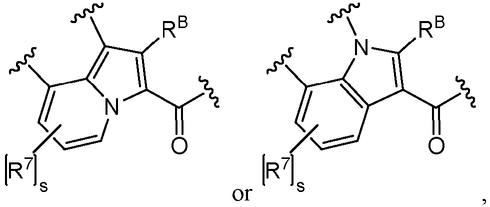 and R^{B} is H and the subscript s is 0;
(ii) the group 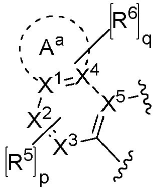 is 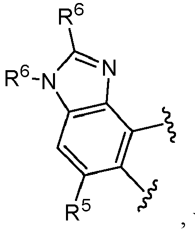 wherein R⁵ and R⁶ are independently C₁-C₃ alkyl or C₁-C₃ fluoroalkyl;
(iii) the group 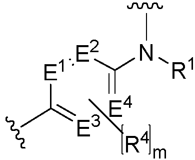 is 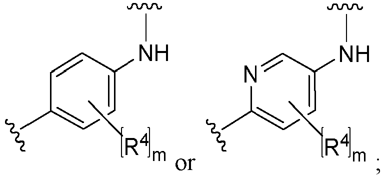
(iv) the group 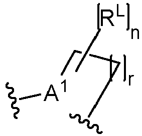 is 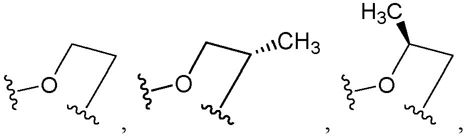 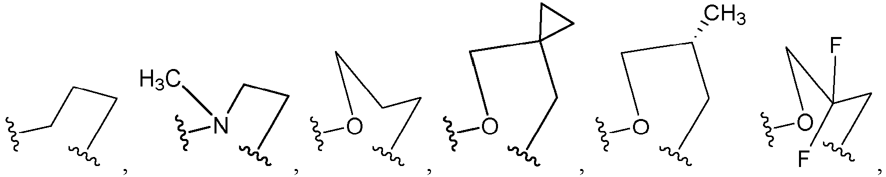 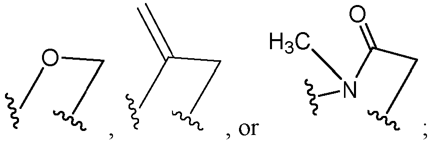 and
(v) the group 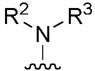 is

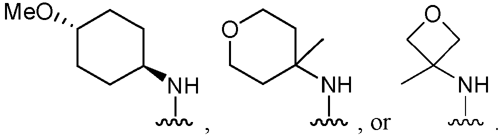

11. The compound of any one of claims 1-10 selected from: 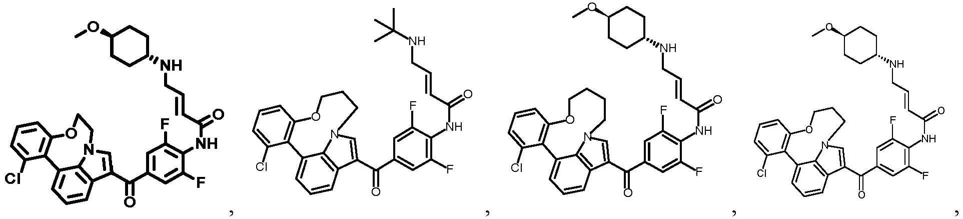 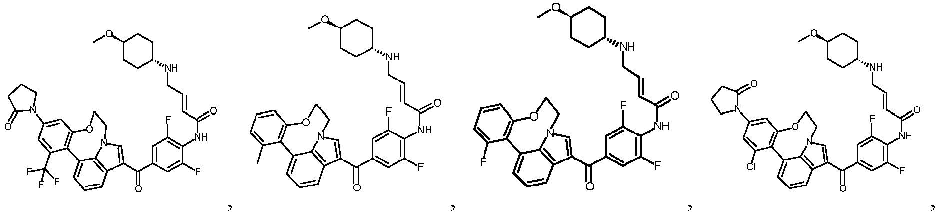 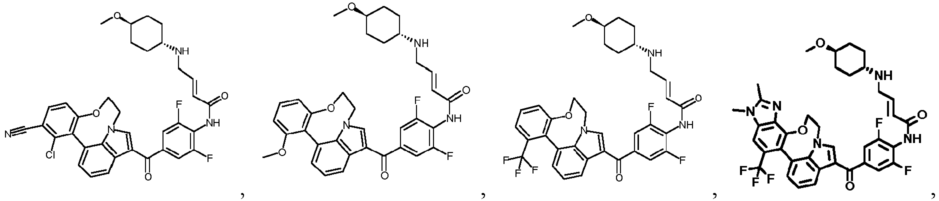 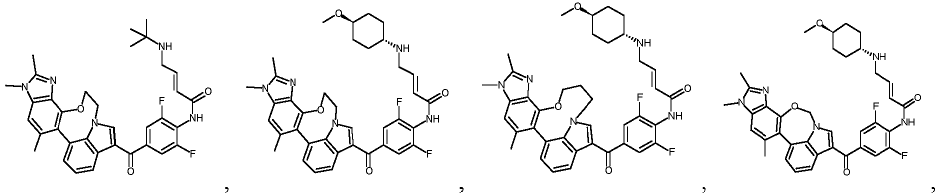 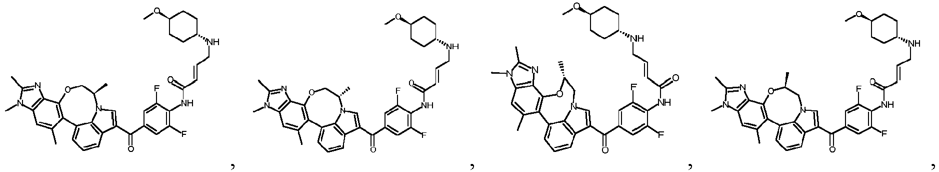 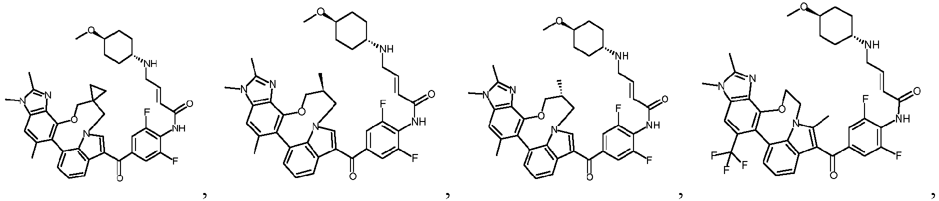 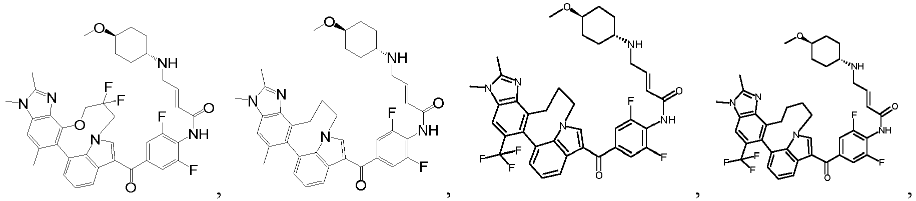 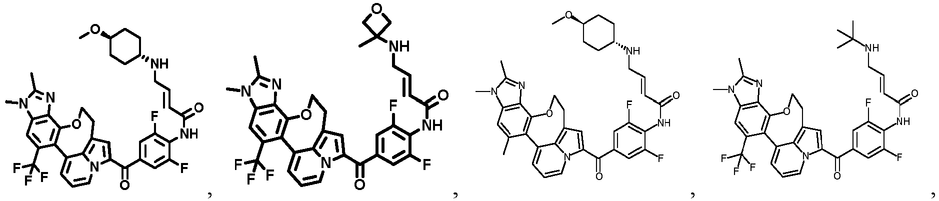 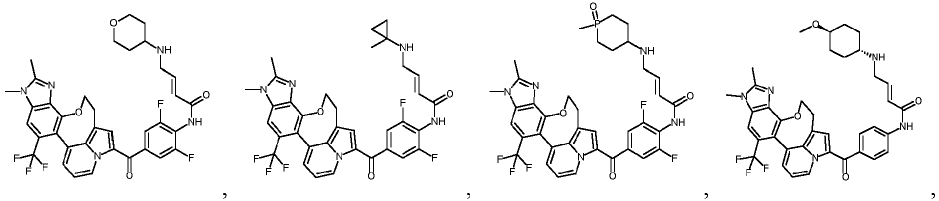 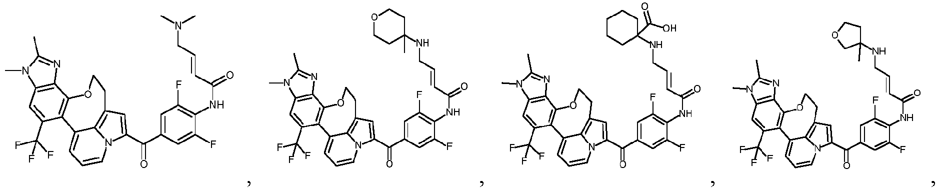 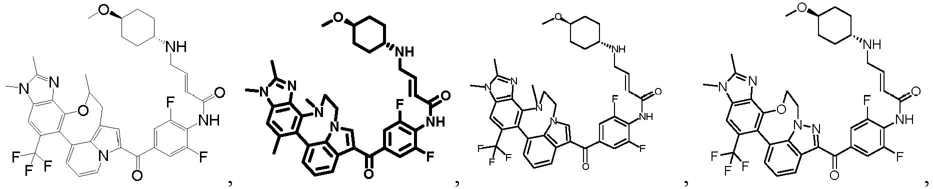 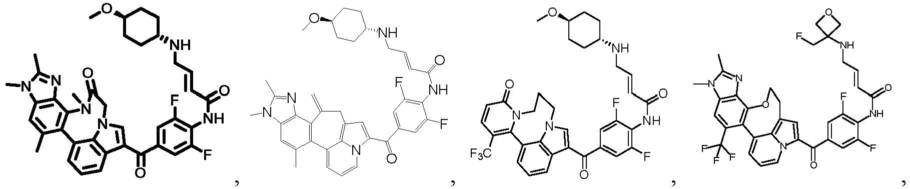 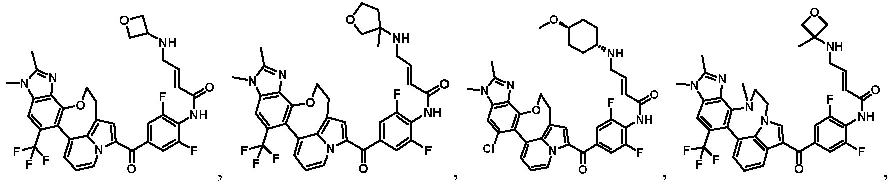 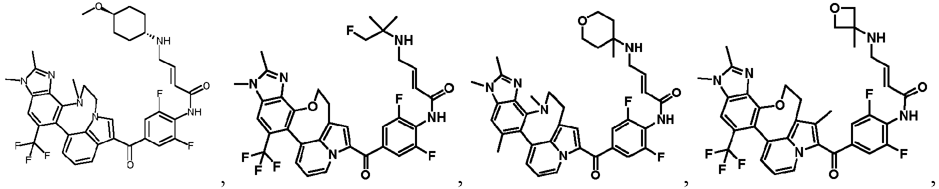 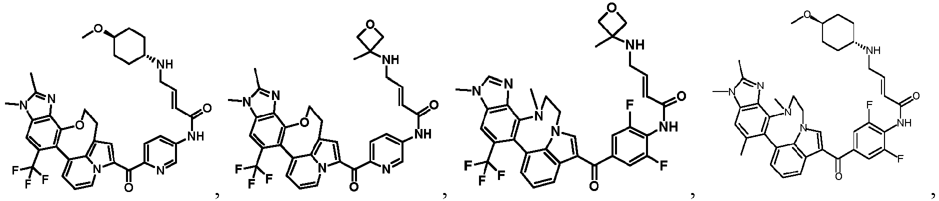 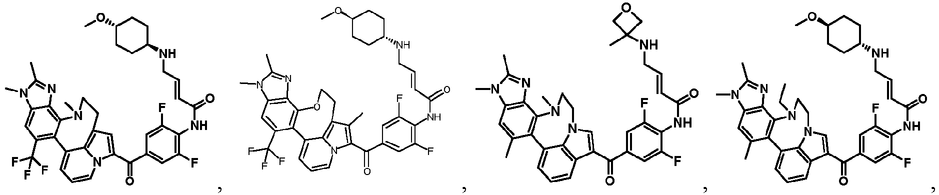 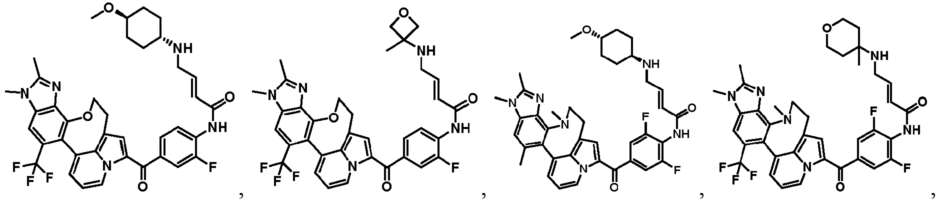 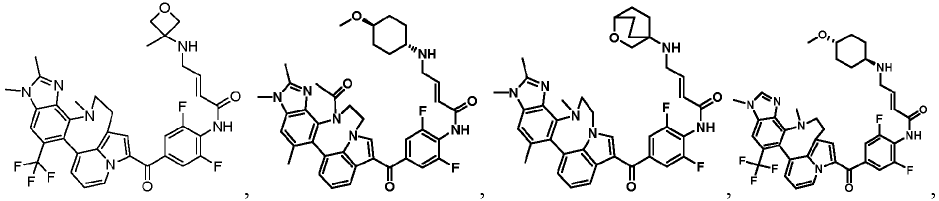 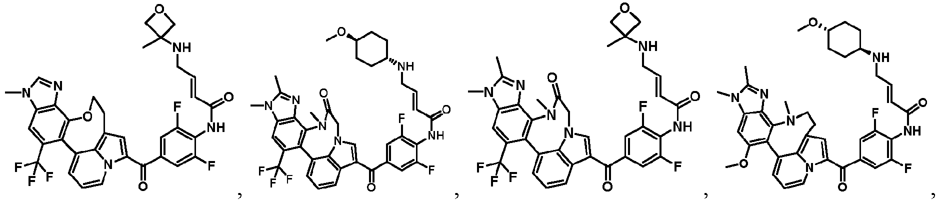 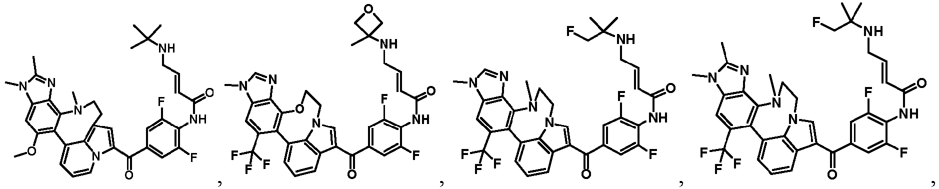 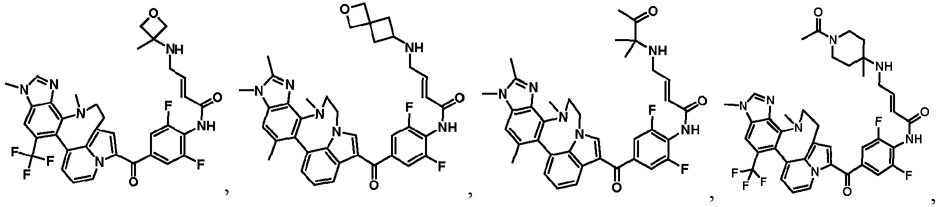 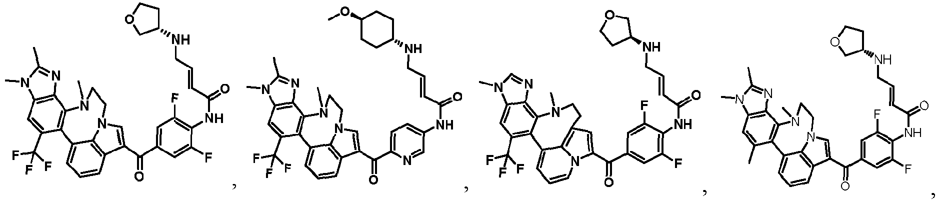 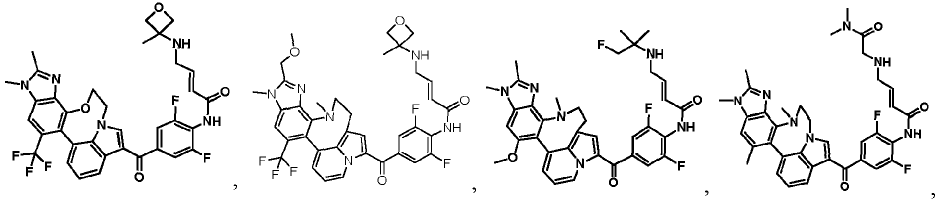 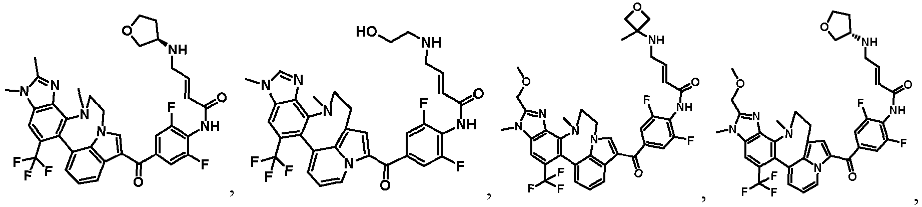 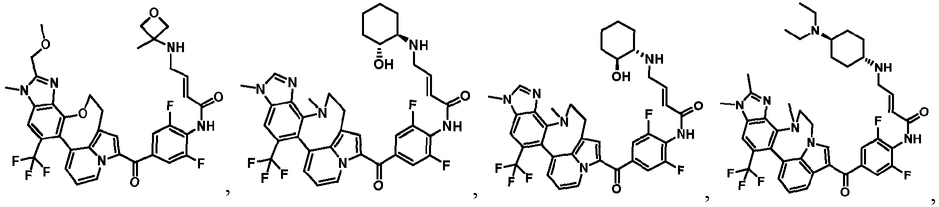 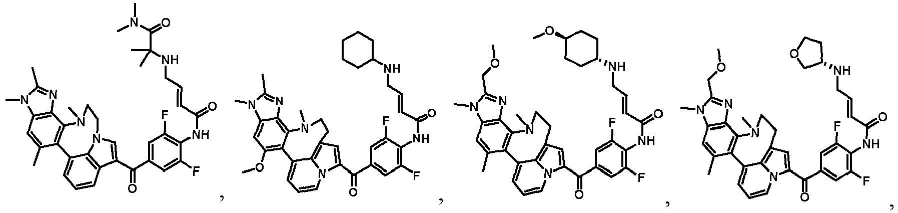 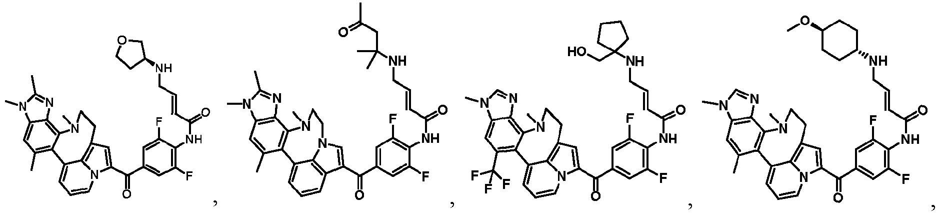 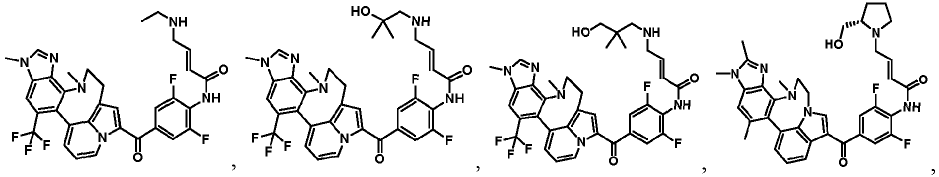 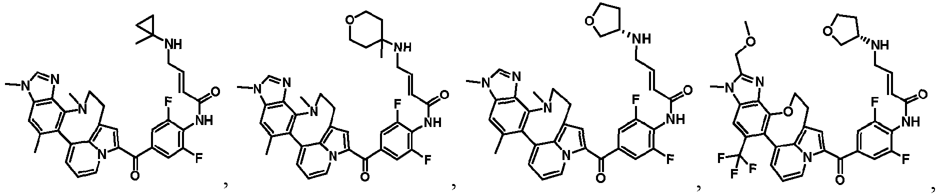 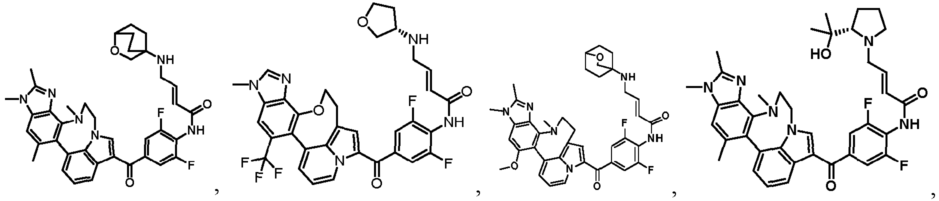 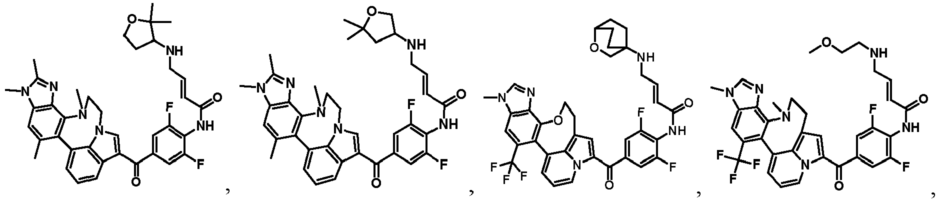 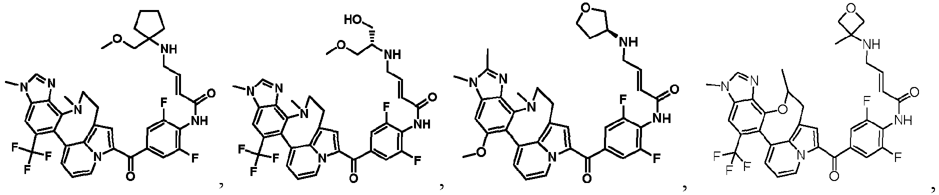 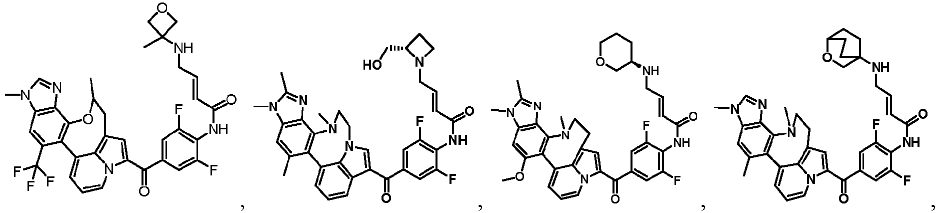 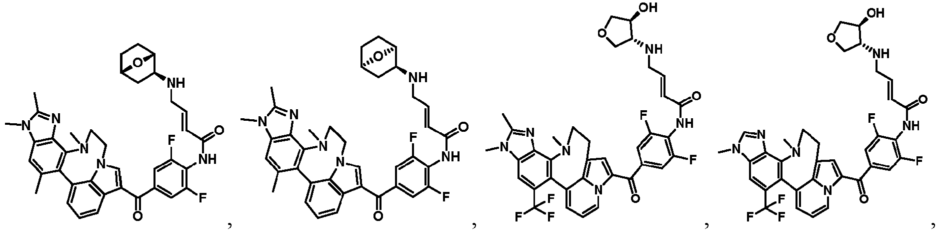 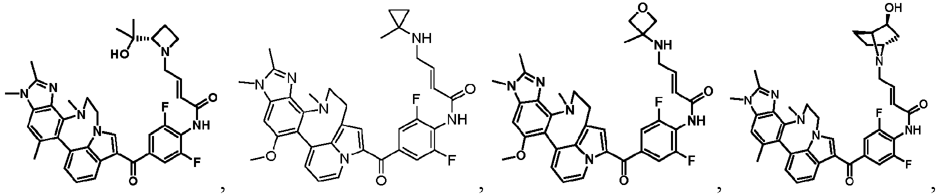 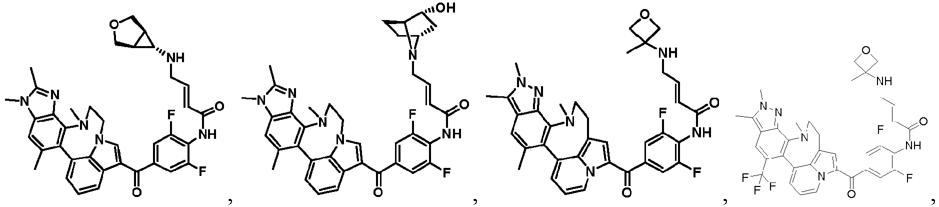 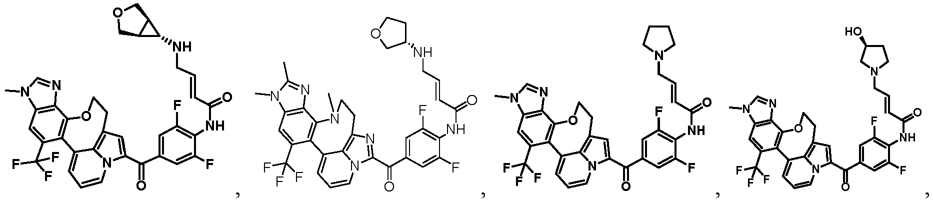 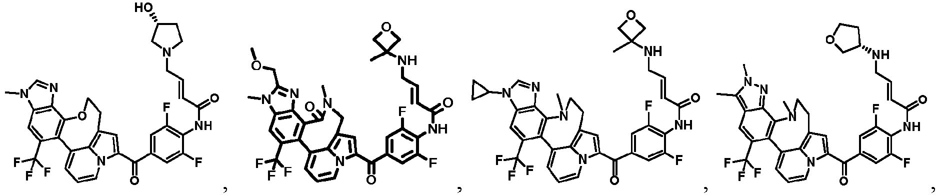 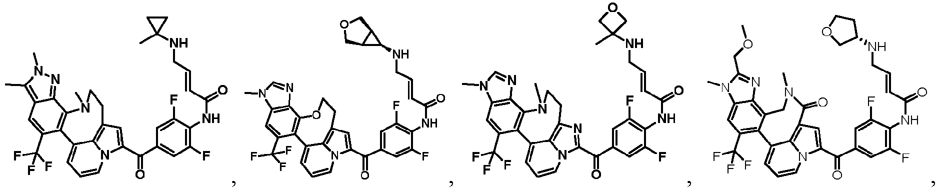 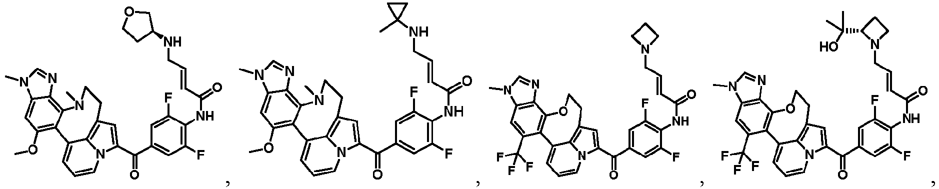 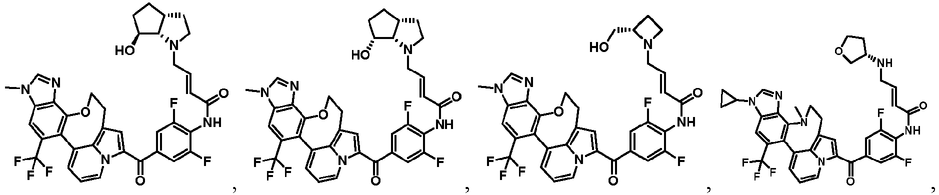 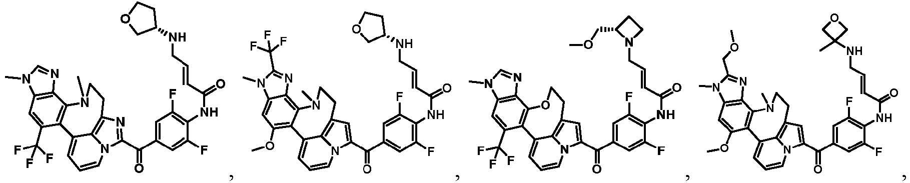 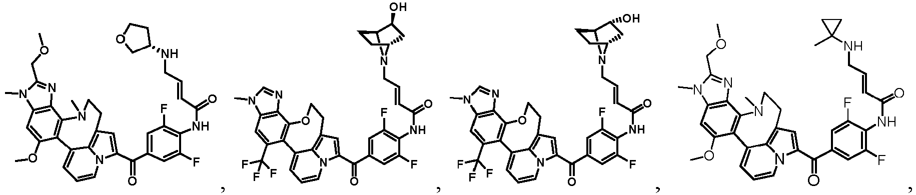 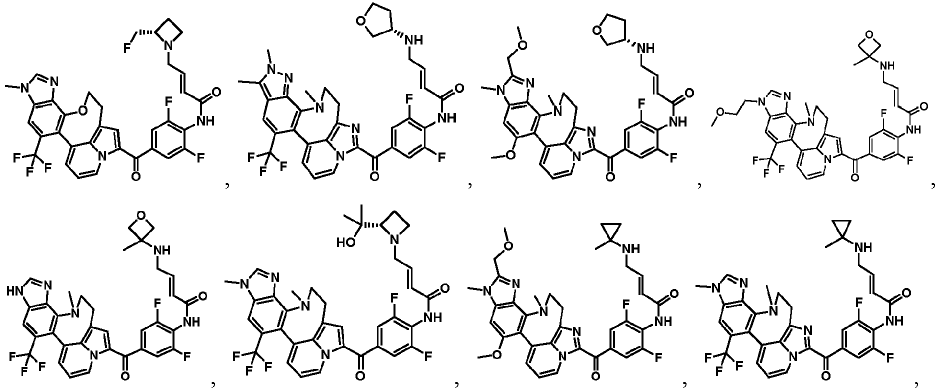 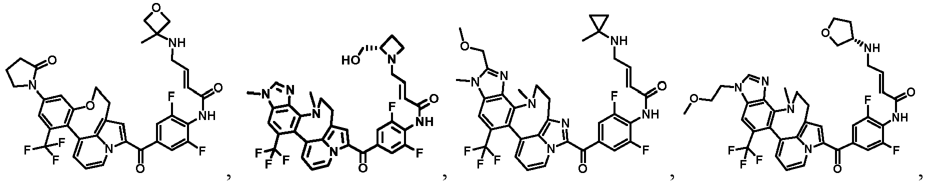 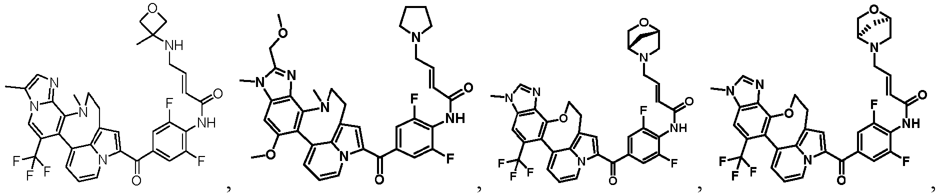 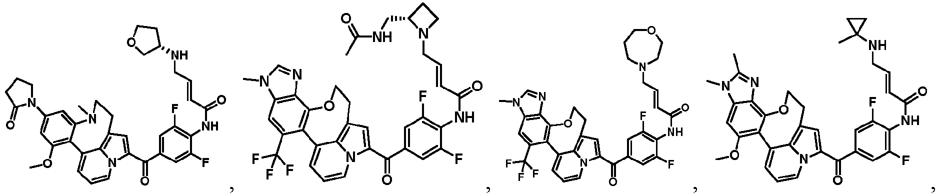 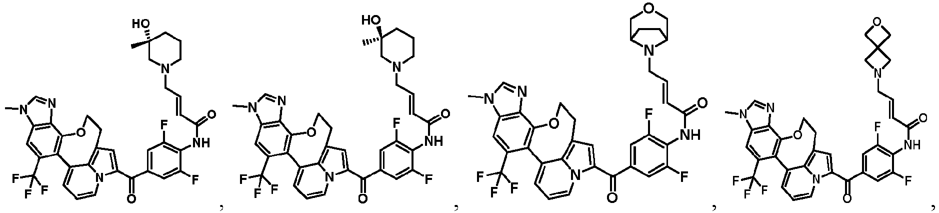 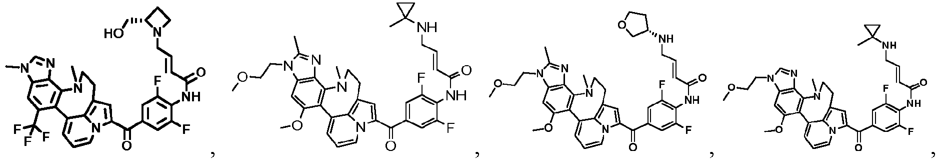 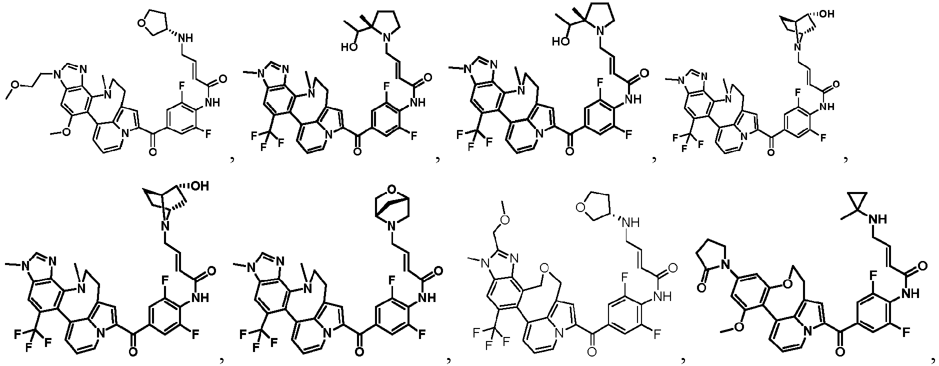 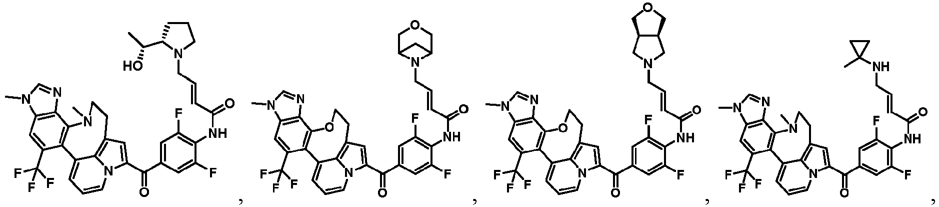 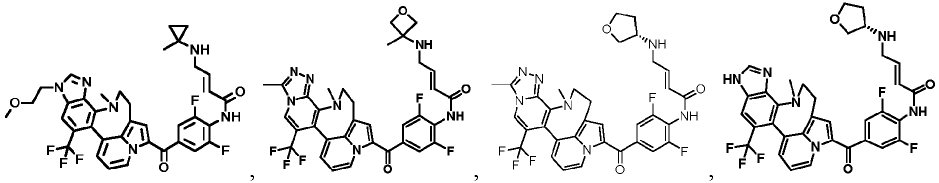 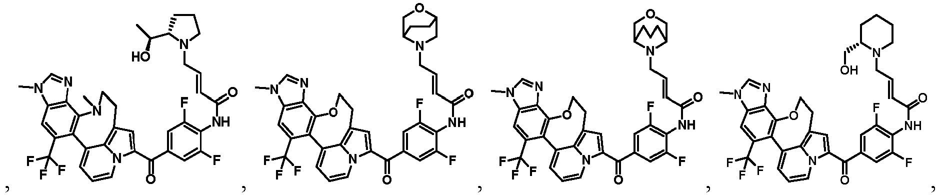 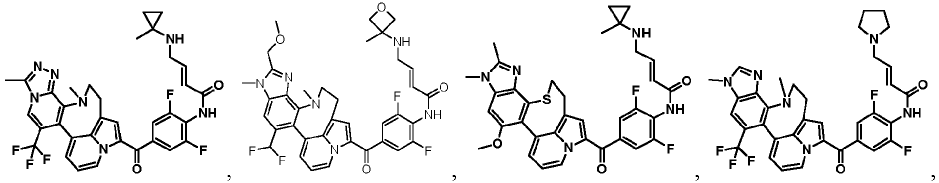 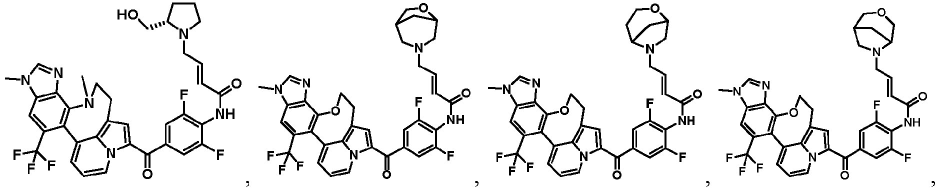 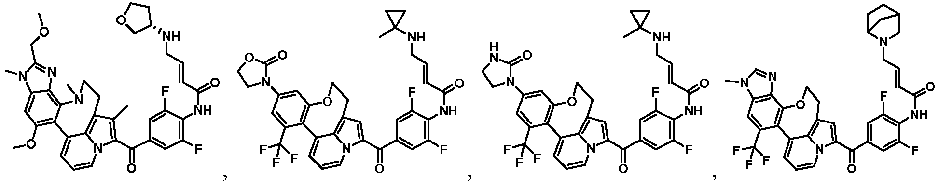 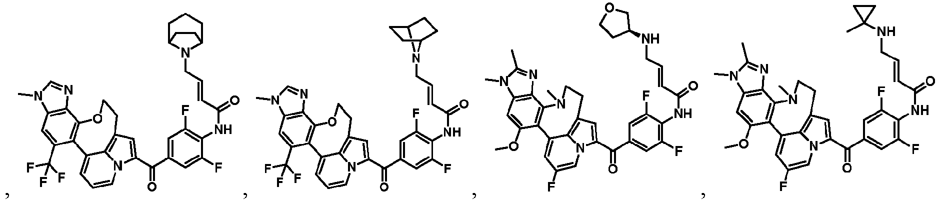 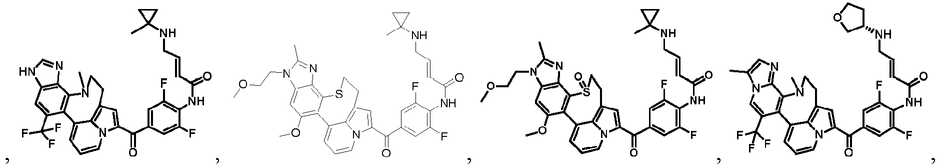 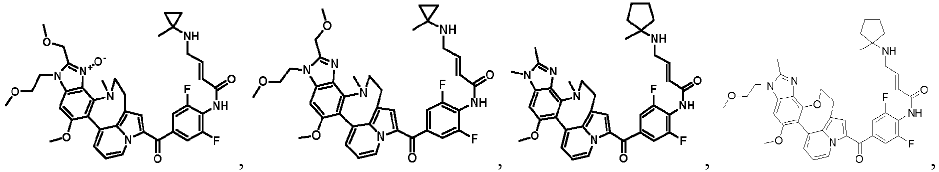 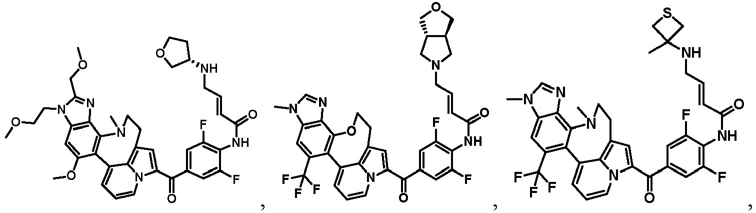 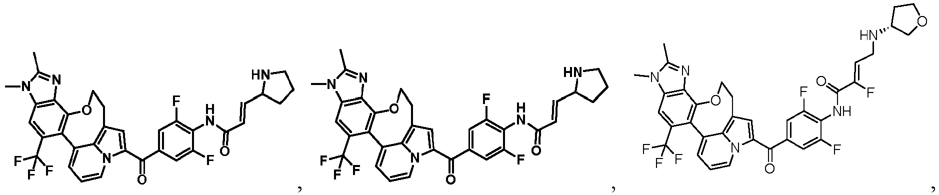 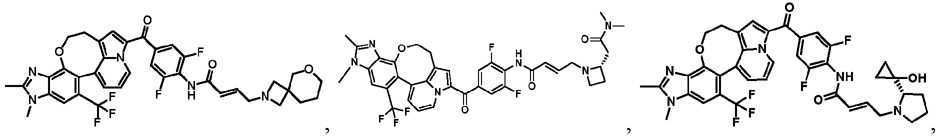 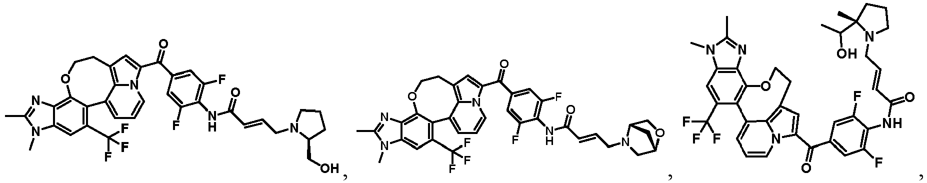 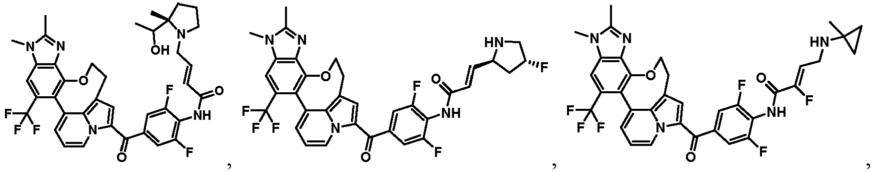 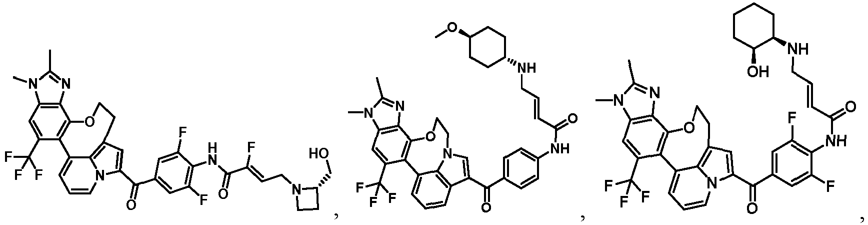 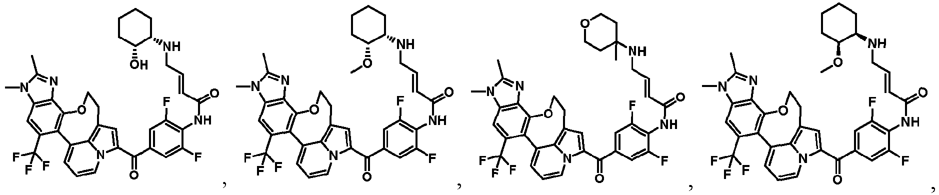 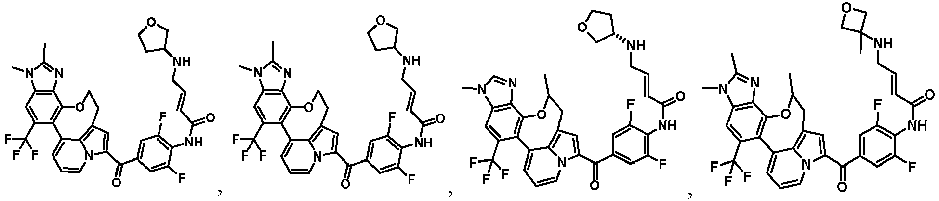 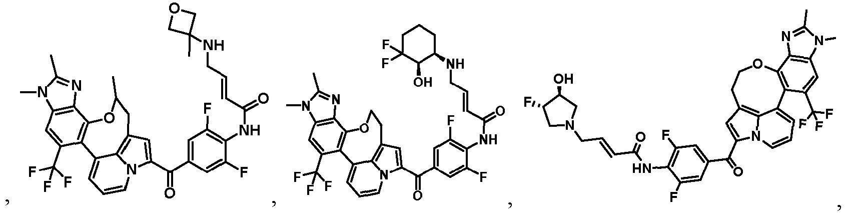 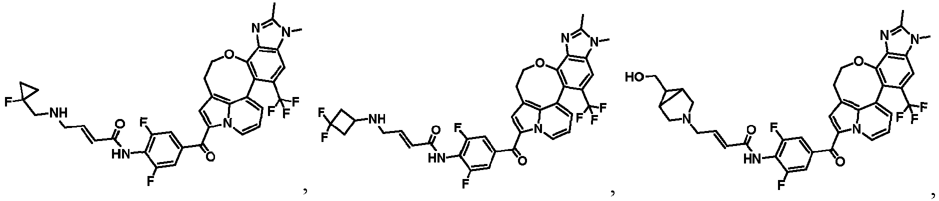 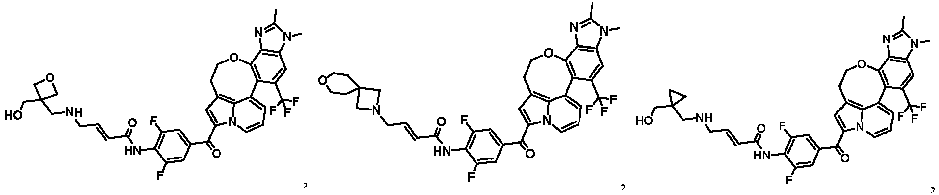 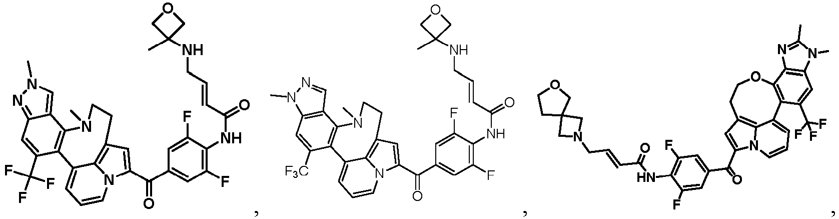 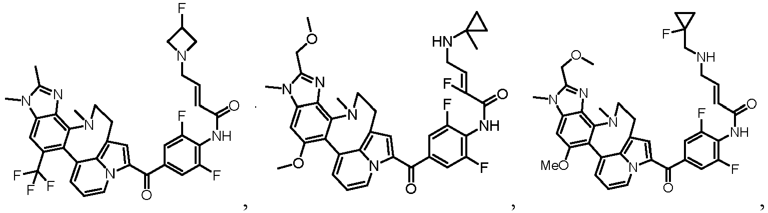 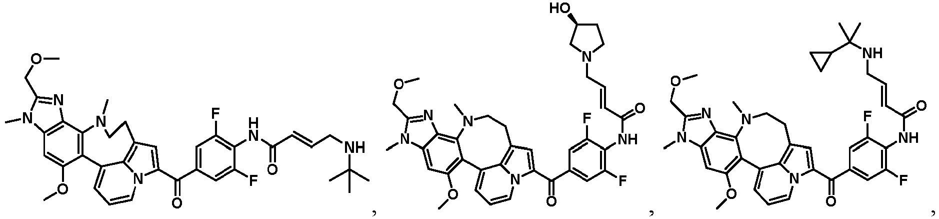 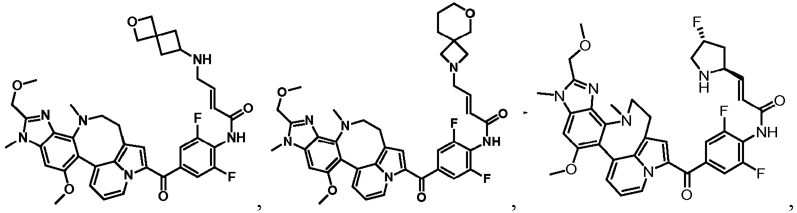 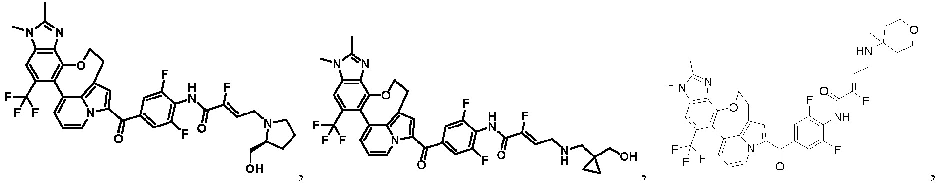 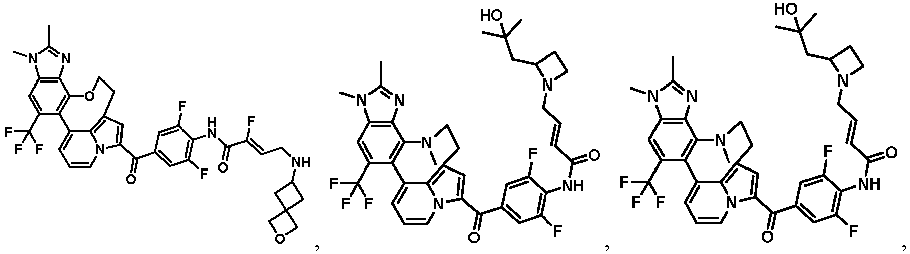 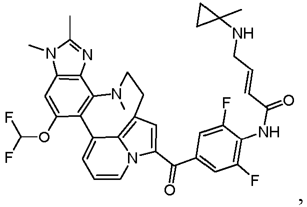 or the pharmauceitcally acceptable salt thereof.

12. A pharmaceutical composition comprising the compound of any one of claims 1-11 or the pharmaceutically acceptable salt thereof, and a pharmaceutically acceptable carrier, wherein the pharmaceutical composition optionally comprises an additional anti-cancer agent.

13. The compound of any one of claims 1-11, or the pharmaceutically acceptable salt thereof, for use in therapy.

14. The compound of any one of claims 1-11, or the pharmaceutically acceptable salt thereof, or a pharmaceutical composition comprising the compound of any one of claims 1-11, or the pharmaceutically acceptable salt thereof, for use in treating cancer.

15. The compound of any one of claims 1-11, or the pharmaceutically acceptable salt thereof, and an additional anti-cancer agent, or a pharmaceutical composition comprising the compound of any one of claims 1-11, or the pharmaceutically acceptable salt thereof, and an additional anti-cancer agent, for use in the treatment of cancer.

## Patentansprüche

1. Verbindung der Formel (I) 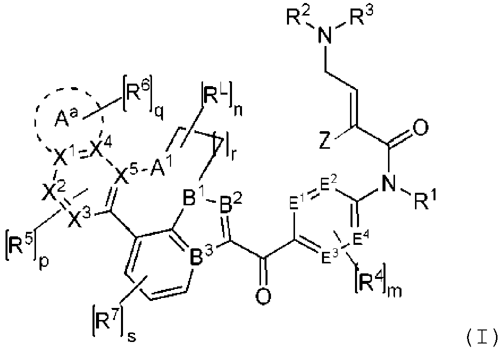 wobei:
R¹ ausgewählt ist aus der Gruppe bestehend aus H und C₁-C₆-Alkyl;
R² und R³ unabhängig ausgewählt sind aus der Gruppe bestehend aus:
(i) H;
(ii) C₁-C₆-Alkyl, wobei das C₁-C₆-Alkyl unsubstituiert oder durch 1 bis 2 R^{a}-Substituenten substituiert ist, die unabhängig ausgewählt sind aus der Gruppe bestehend aus Halogen, Hydroxy, C₁-C₃-Alkoxy, C₂-C₆-Alkenyl, C₂-C₆-Alkinyl, C₁-C₃-Alkylamino, C₁-C₃-Dialkylamino, C₁-C₃-Alkylsulfonyl, C₂-C₄-Acyl, C₁-C₃-Hydroxyalkyl, C₁-C₃-Alkoxycarbonyl, C₁-C₃-Hydroxycarbonyl, Oxo (=O), C₃-C₈-Cycloalkyl und 4- bis 10-gliedrigem mono- oder bicyclischem Heterocycloalkyl;
wobei das C₃-C₈-Cycloalkyl oder das 4- bis 10-gliedrige mono- oder bicyclische Heterocycloalkyl von R^{a} unsubstituiert oder durch 1 bis 2 Substituenten substituiert ist, die ausgewählt sind aus der Gruppe bestehend aus Halogen, Hydroxy, Amino, C₁-C₃-Alkyl, C₁-C₃-Fluoralkyl und C₁-C₃-Alkoxy; und
(iii) einem 3- bis 8-gliedrigen monocyclischen, verbrückten bicyclischen oder spirocyclischen gesättigten Ringsystem, das 0 bis 2 Heteroatomgruppen enthält, die ausgewählt sind aus der Gruppe bestehend aus N, O, S, S(O), S(O)₂, P, P(O) und P(O)₂;
wobei das 3- bis 8-gliedrige monocyclische, verbrückte bicyclische oder spirocyclische gesättigte Ringsystem unsubstituiert oder durch 1 bis 4 Substituenten substituiert ist, die unabhängig ausgewählt sind aus der Gruppe bestehend aus Halogen, Hydroxy, C₁-C₃-Alkyl, C₁-C₃-Fluoralkyl, C₁-C₃-Alkoxy, C₂-C₆-Alkenyl, C₂-C₆-Alkinyl, C₁-C₃-Alkylamino, C₁-C₃-Dialkylamino, C₁-C₃-Alkylsulfonyl, C₂-C₄-Acyl, C₁-C₃-Hydroxyalkyl, C₁-C₃-Alkoxy(C₁-C₃)alkyl, Carboxy und C₁-C₃-Alkoxycarbonyl;
oder alternativ R² und R³ zusammen mit dem N-Atom, an das sie gebunden sind, ein 4- bis 9-gliedriges monocyclisches, kondensiertes bicyclisches, verbrücktes bicyclisches oder spirocyclisches gesättigtes Ringsystem bilden, das 0 bis 2 zusätzliche Heteroatome enthält, die ausgewählt sind aus der Gruppe bestehend aus N, O und S;
wobei das 4- bis 9-gliedrige monocyclische, kondensierte bicyclische, verbrückte bicyclische oder spirocyclische gesättigte Ringsystem unsubstituiert oder durch 1 bis 4 Substituenten substituiert ist, die unabhängig ausgewählt sind aus der Gruppe bestehend aus Halogen, Hydroxy, C₁-C₃-Alkyl, C₁-C₃-Fluoralkyl, C₁-C₃-Alkoxy, C₁-C₄-Hydroxyalkyl, C₃-C₅-Hydroxycycloalkyl, - (CH₃)₂N(C=O)CH₂-, CH₃(C=O)NHCH₂- und C₁-C₃-Alkoxy(C₁-C₃)alkyl;
oder alternativ eines von R² und R³ zusammen mit dem N-Atom, an das es gebunden ist, und einem benachbarten C-Atom ein 3- bis 6-gliedriges monocyclisches gesättigtes Ringsystem bildet, das 0 bis 2 zusätzliche Heteroatome enthält, die ausgewählt sind aus der Gruppe bestehend aus N, O und S;
wobei das 3- bis 6-gliedrige monocyclische gesättigte Ringsystem unsubstituiert oder durch 1 bis 4 Substituenten substituiert ist, die unabhängig ausgewählt sind aus der Gruppe bestehend aus Halogen, Hydroxy, C₁-C₃-Alkyl, C₁-C₃-Fluoralkyl, C₁-C₃-Alkoxy, C₁-C₃-Hydroxyalkyl und C₁-C₃-Alkoxy(C₁-C₃)alkyl;
bei jedem Vorkommen R⁴ unabhängig ausgewählt ist aus der Gruppe bestehend aus Halogen, Cyano, C₁-C₃-Alkyl, C₁-C₃-Fluoralkyl, C₁-C₃-Alkoxy und C₁-C₄-Cyanoalkyl;
E¹, E², E³ und E⁴ unabhängig ausgewählt sind aus der Gruppe bestehend aus N und C, mit der Maßgabe, dass nicht mehr als drei von E¹, E², E³ und E⁴ N sind;
eines von B¹ und B³ C ist und das andere N ist;
B² N oder C(R^{B}) ist, wobei R^{B} ausgewählt ist aus der Gruppe bestehend aus H, C₁-C₆-Alkyl, C₂-C₆-Alkenyl, C₂-C₆-Alkinyl und C₃-C₆-Cycloalkyl;
bei jedem Vorkommen R^{L} unabhängig ausgewählt ist aus der Gruppe bestehend aus Fluor, Hydroxy, C₁-C₆-Alkyl, C₁-C₃-Alkoxy, C₁-C₃-Fluoralkyl, Oxo (=O), C₂-C₄-Acyl und Methenyl (=CH₂) oder
alternativ zwei geminal substituierte R^{L}-Substituenten zusammen mit dem Kohlenstoffatom, an das sie gebunden sind, einen 3- bis 6-gliedrigen spirocyclischen Ring bilden, der 0 bis 1 Heteroatome enthält, die ausgewählt sind aus der Gruppe bestehend aus N, O und S;
Ring A^{a} vorhanden oder nicht vorhanden ist und, falls er vorhanden ist, ein 5- bis 6-gliedriges Heteroaryl ist, das 1 bis 3 Heteroatome enthält, die ausgewählt sind aus der Gruppe bestehend aus N, O und S;
X¹, X², X³, X⁴ und X⁵ unabhängig ausgewählt sind aus der Gruppe bestehend aus N und C, mit der Maßgabe, dass nicht mehr als zwei von X¹, X², X³, X⁴ und X⁵ N sind;
bei jedem Vorkommen R⁵ unabhängig ausgewählt ist aus der Gruppe bestehend aus C₁-C₆-Alkyl, C₁-C₆-Fluoralkyl, C₁-C₃-Alkoxy, C₁-C₃-Acyl, Halogen, Hydroxy, Amino, Cyano, Oxo (=O), C₁-C₄-Cyanoalkyl, C₁-C₃-Hydroxyalkyl, C₁-C₃-Alkylamino, C₁-C₃-Dialkylamino, C₁-C₃-Alkoxy(C₁-C₃)alkyl und einer Gruppe der Formel 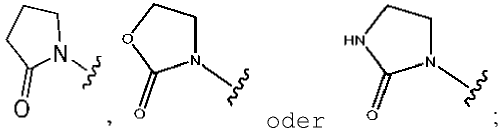
bei jedem Vorkommen R⁶ unabhängig ausgewählt ist aus der Gruppe bestehend aus C₁-C₃-Alkyl, C₁-C₃-Fluoralkyl, C₁-C₃-Alkoxy, Halogen, Hydroxy, Oxo (=O), Cyano, Carbamoyl, C₁-C₄-Cyanoalkyl, C₁-C₃-Hydroxyalkyl, C₁-C₃-Alkoxy (C₁-C₃)alkyl, C₃-C₆-Cycloalkyl und C₁-C₄-Carbamoylalkyl;
jedes Vorkommen von R⁷ unabhängig ausgewählt ist aus der Gruppe bestehend aus Halogen, C₁-C₃-Alkyl, C₁-C₃-Fluoralkyl und C₁-C₃-Alkoxy;
A¹ ausgewählt ist aus der Gruppe bestehend aus -CH₂-, - CH₂-N(R^{L})-, -C(=O)-N(R^{L})-, -CH₂-O-, -C(H)(R^{L})-, -N(H)-, - N(R^{L}) -, -S-, -S(=O)- und -O-;
Z ausgewählt ist aus der Gruppe bestehend aus H und Halogen;
Index m 0, 1, 2 oder 3 ist;
Index n 0, 1, 2 oder 3 ist;
Index p 0, 1, 2, 3 oder 4 ist;
Index q 0, 1, 2 oder 3 ist;
Index r 0, 1, 2 oder 3 ist; und
Index s 0, 1, 2 oder 3 ist;
oder ein pharmazeutisch annehmbares Salz davon.

2. Verbindung nach Anspruch 1 oder pharmazeutisch annehmbares Salz davon, wobei:
das C₁-C₆-Alkyl unsubstituiert oder durch 1 bis 2 R^{a}-Substituenten substituiert ist, die unabhängig ausgewählt sind aus der Gruppe bestehend aus Halogen, Hydroxy, C₁-C₃-Alkoxy, C₂-C₆-Alkenyl, C₂-C₆-Alkinyl, C₁-C₃-Alkylamino, C₁-C₃-Dialkylamino, C₁-C₃-Alkylsulfonyl, C₂-C₄-Acyl, C₁-C₃-Hydroxyalkyl, C₁-C₃-Alkoxycarbonyl, C₁-C₃-Hydroxycarbonyl, C₃-C₈-Cycloalkyl und 4- bis 10-gliedrigem mono- oder bicyclischem Heterocycloalkyl;
R² und R³ zusammen mit dem N-Atom, an das sie gebunden sind, ein 4- bis 8-gliedriges gesättigtes Heterocycloalkyl mit 0 bis 2 zusätzlichen Heteroatomen bilden, die ausgewählt sind aus der Gruppe bestehend aus N, O und S;
wobei das 4- bis 8-gliedrige Heterocycloalkyl unsubstituiert oder durch 1 bis 4 Substituenten substituiert ist, die unabhängig ausgewählt sind aus der Gruppe bestehend aus Halogen, Hydroxy, C₁-C₃-Alkyl, C₁-C₃-Fluoralkyl, C₁-C₃-Alkoxy, C₁-C₃-Hydroxyalkyl und C₁-C₃-Alkoxy (C₁-C₃) alkyl;
bei jedem Vorkommen R^{L} unabhängig ausgewählt ist aus der Gruppe bestehend aus Fluor, Hydroxy, C₁-C₆-Alkyl, C₁-C₃-Alkoxy, C₁-C₃-Fluoralkyl, Oxo (=O) und Methenyl (=CH₂),
der Ring A^{a} vorhanden oder nicht vorhanden ist und, falls er vorhanden ist, das 5- bis 6-gliedrige Heteroaryl ist, das 1 bis 2 Heteroatome enthält, die ausgewählt sind aus der Gruppe bestehend aus N, O und S;
bei jedem Vorkommen R⁵ unabhängig ausgewählt ist aus der Gruppe bestehend aus C₁-C₆-Alkyl, C₁-C₆-Fluoralkyl, C₁-C₃-Alkoxy, C₁-C₃-Acyl, Halogen, Hydroxy, Amino, Cyano, Oxo (=O), C₁-C₄-Cyanoalkyl, C₁-C₃-Hydroxyalkyl, C₁-C₃-Alkylamino, C₁-C₃-Dialkylamino, C₁-C₃-Alkoxy(C₁-C₃)alkyl und der Gruppe der Formel 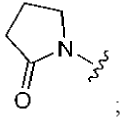
bei jedem Vorkommen R⁶ unabhängig ausgewählt ist aus der Gruppe bestehend aus C₁-C₃-Alkyl, C₁-C₃-Fluoralkyl, C₁-C₃-Alkoxy, Halogen, Hydroxy, Oxo (=O), Cyano, Carbamoyl, C₁-C₄-Cyanoalkyl, C₁-C₃-Hydroxyalkyl, C₁-C₃-Alkoxy (C₁-C₃)alkyl und C₁-C₄-Carbamoylalkyl;
A¹ ausgewählt ist aus der Gruppe bestehend aus -CH₂-, - C(H)(R^{L})-, -N(H)-, -N(R^{L})- und -O-;
Z H ist; und
Index q 0, 1 oder 2 ist;

3. Verbindung nach Anspruch 1 oder pharmazeutisch annehmbares Salz davon, wobei
(a) die Gruppe 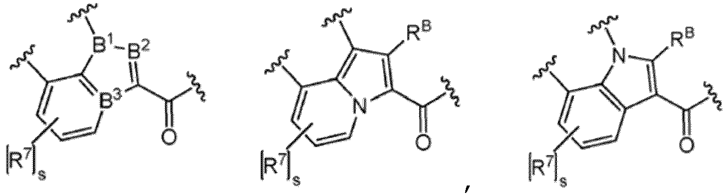 oder 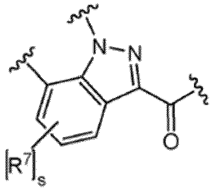 ist oder
(b) die Gruppe 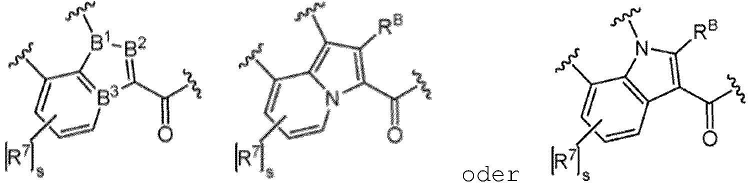 ist, wobei gegebenenfalls R^{B} H oder Methyl ist und der Index s 0 ist.

4. Verbindung nach Anspruch 1 oder pharmazeutisch annehmbares Salz davon, wobei:
X¹ N oder C ist; und
X², X³, X⁴ und X⁵ C sind.

5. Verbindung nach Anspruch 1 oder pharmazeutisch annehmbares Salz davon, wobei
(a) die Gruppe 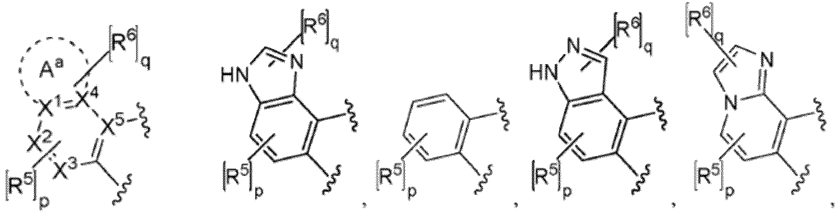 oder 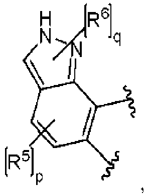 ist oder
(b) die Gruppe 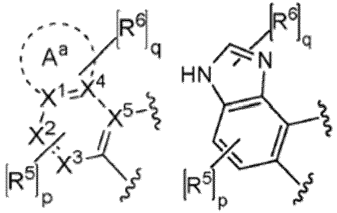 ist oder
(c) die Gruppe 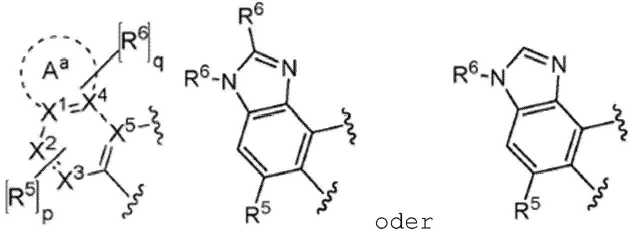 ist, wobei
R⁵ C₁-C₃-Alkyl oder C₁-C₃-Fluoralkyl ist; und
R⁶ C₁-C₃-Alkyl, C₁-C₃-Fluoralkyl oder C₁-C₃-Alkoxy (C₁-C₃)alkyl ist.

6. Verbindung nach Anspruch 1 oder pharmazeutisch annehmbares Salz davon, wobei die Gruppe 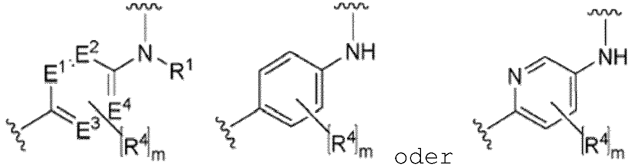 ist.

7. Verbindung nach Anspruch 1 oder pharmazeutisch annehmbares Salz davon, wobei
(a) die Gruppe 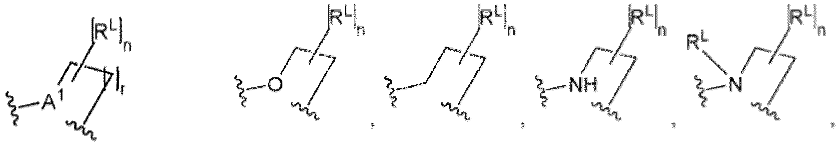 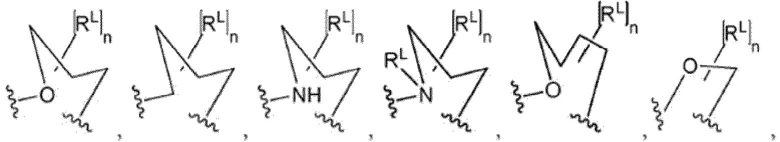 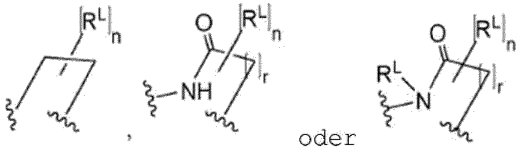 ist oder
(b) die Gruppe 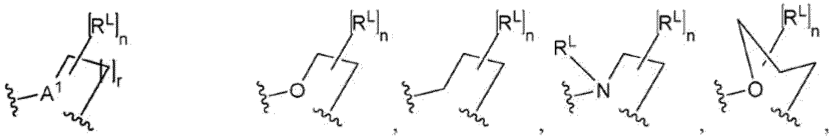 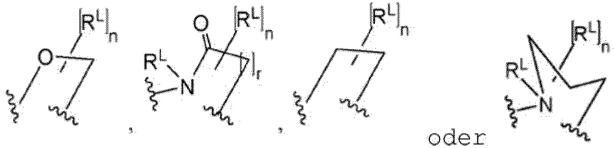 ist oder
(c) die Gruppe 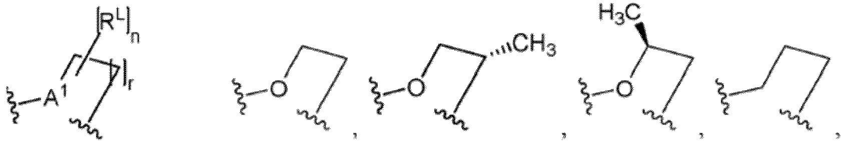 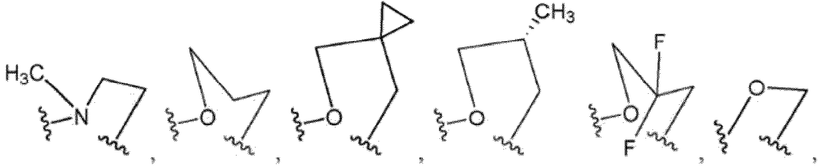 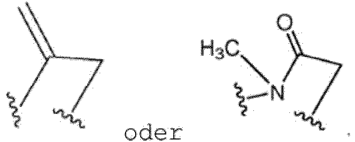 ist.

8. Verbindung nach Anspruch 1 oder pharmazeutisch annehmbares Salz davon, wobei
(a) die Gruppe 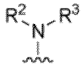 is 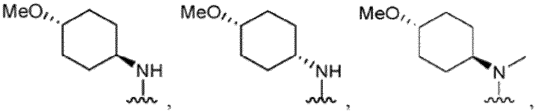 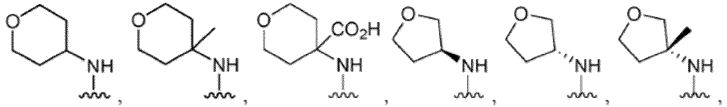 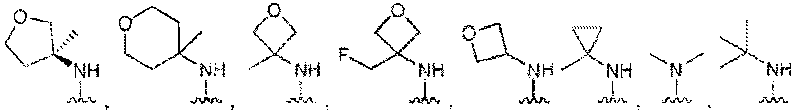 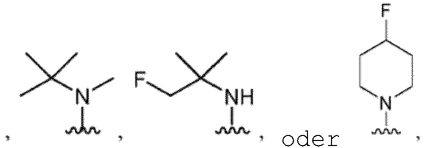 ist oder
(b) die Gruppe 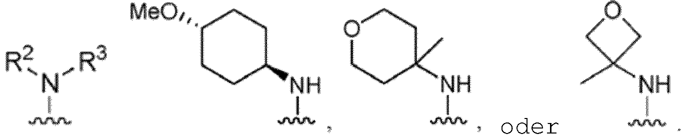 ist.

9. Verbindung nach Anspruch 1 oder pharmazeutisch annehmbares Salz davon, wobei R² und R³ unabhängig H, C₁-C₆-Alkyl oder C₁-C₆-Fluoralkyl sind.

10. Verbindung nach Anspruch 1 oder pharmazeutisch annehmbares Salz davon, wobei:
(i) die Gruppe 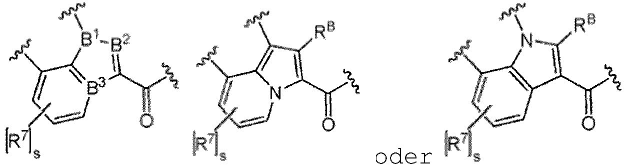 ist und R^{B} H ist und der Index s 0 ist;
(ii) die Gruppe 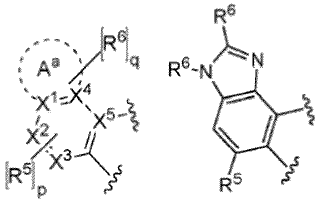 ist, wobei R⁵ und R⁶ unabhängig C₁-C₃-Alkyl oder C₁-C₃-Fluoralkyl sind;
(iii) die Gruppe 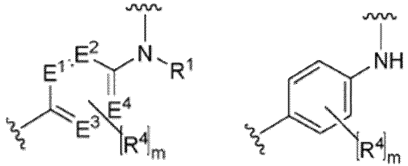 oder 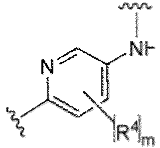 ist;
(iv) die Gruppe 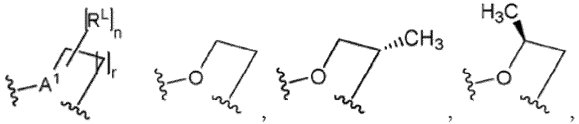 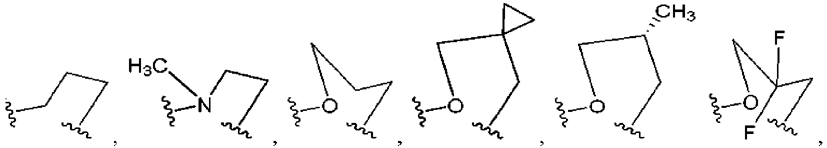 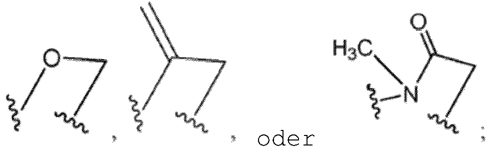 ist und
(v) die Gruppe 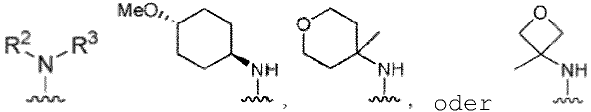 ist.

11. Verbindung nach einem der Ansprüche 1 bis 10, ausgewählt aus: 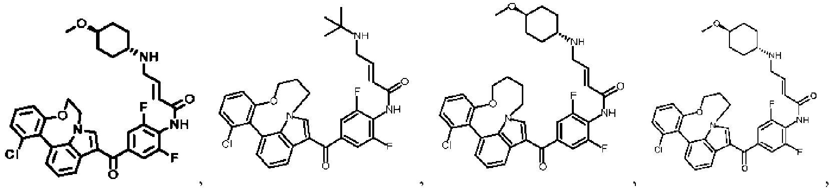 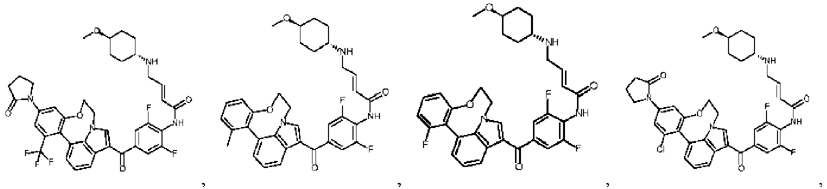 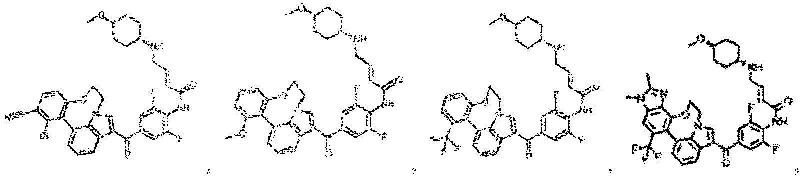 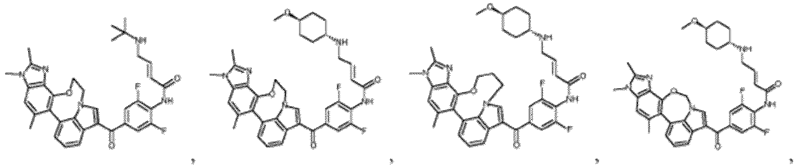 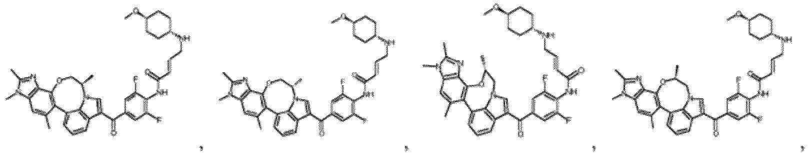 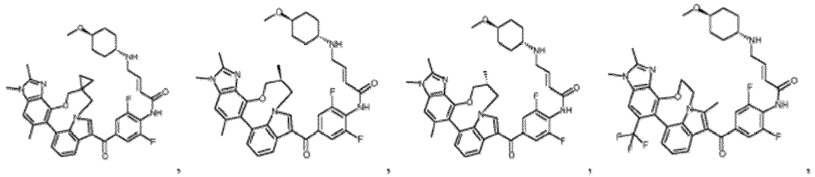 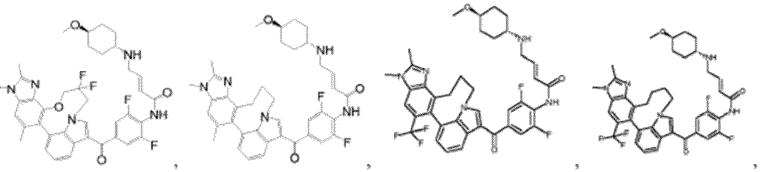 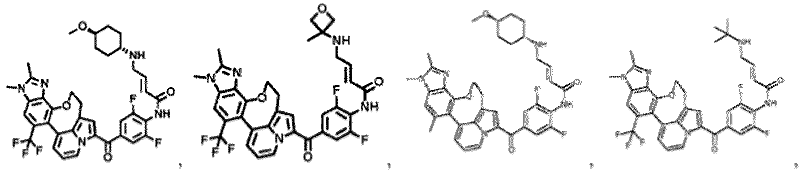 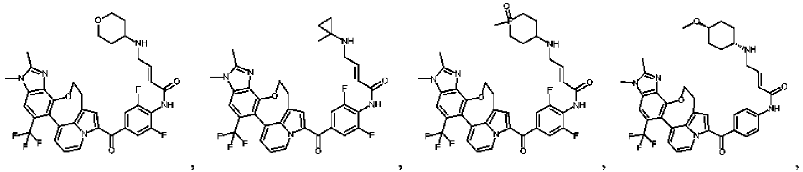 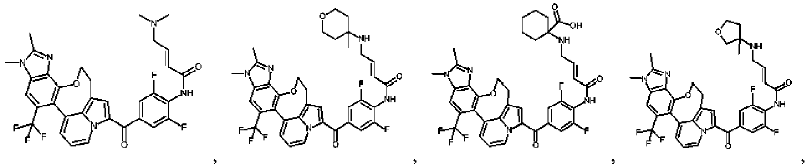 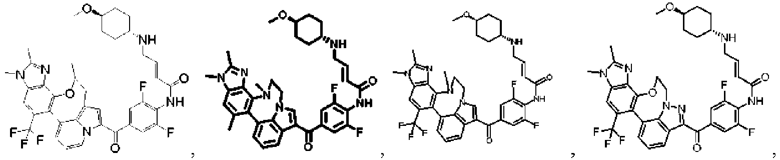 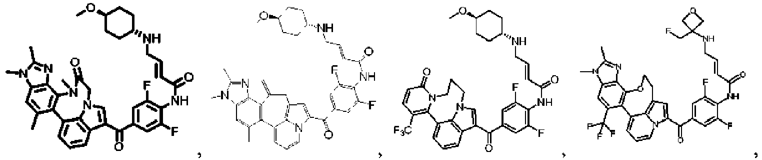 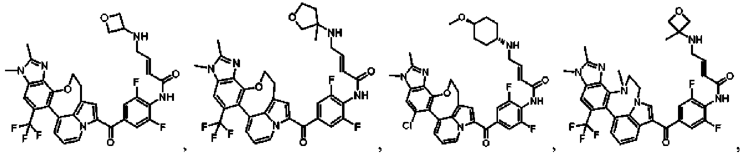 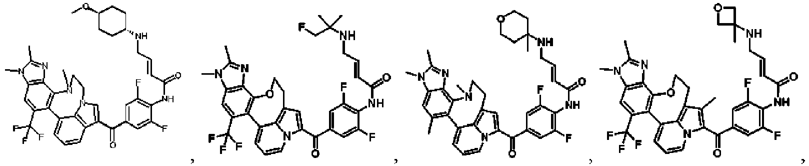 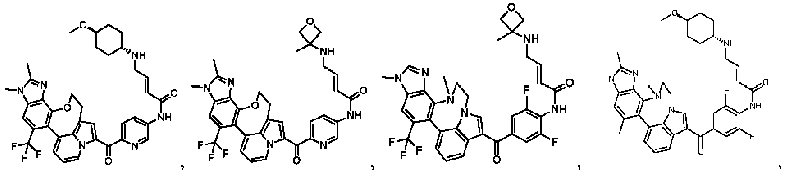 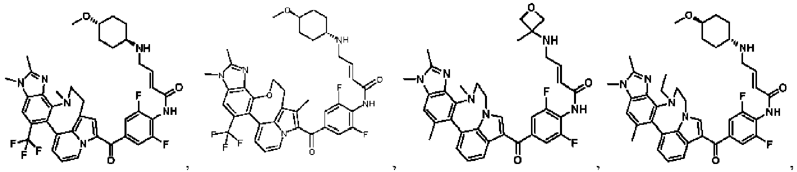 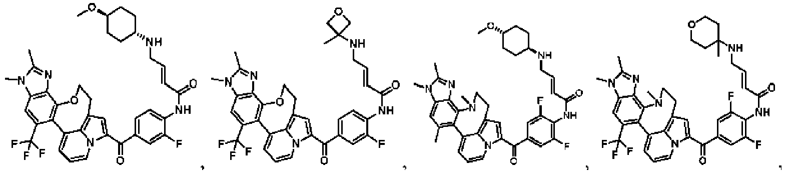 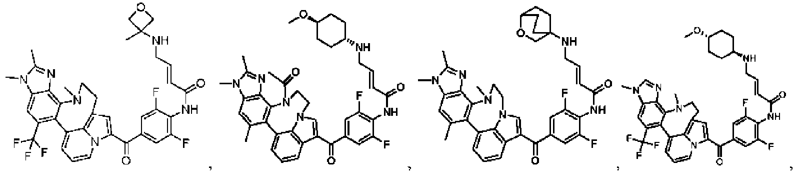 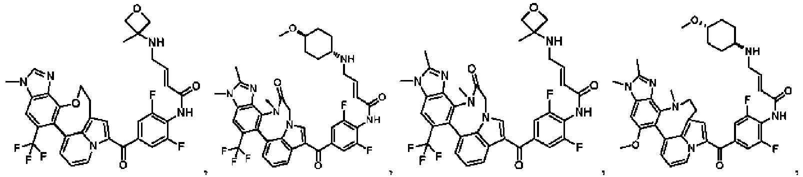 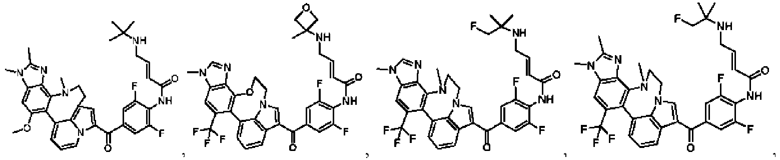 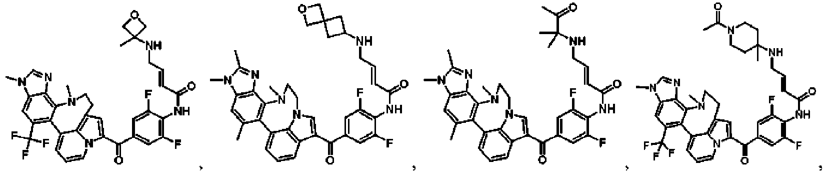 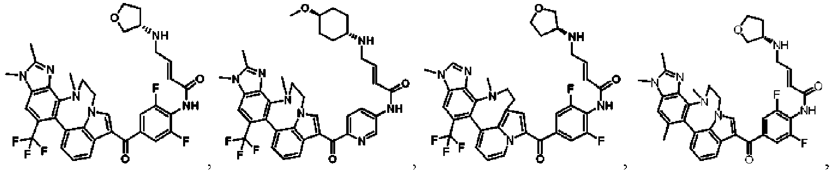 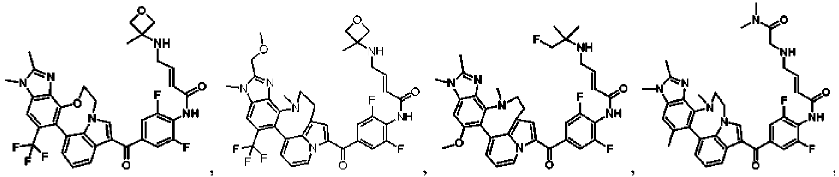 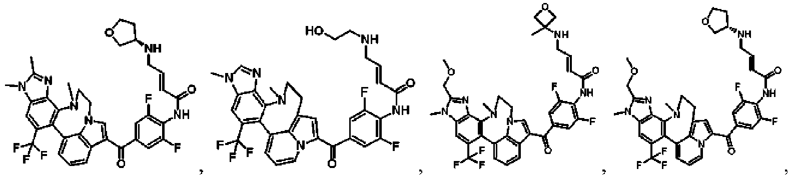 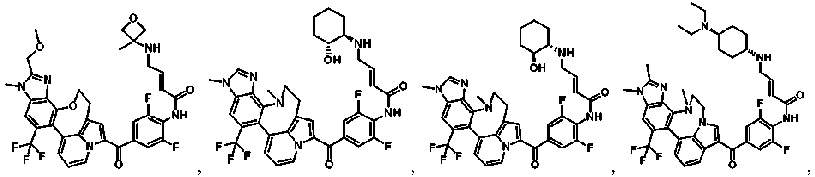 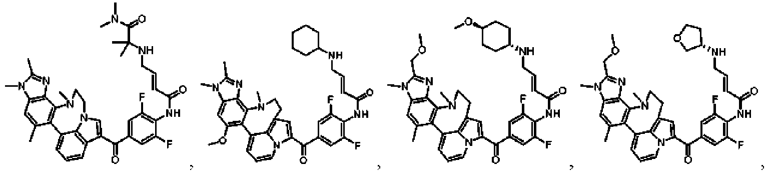 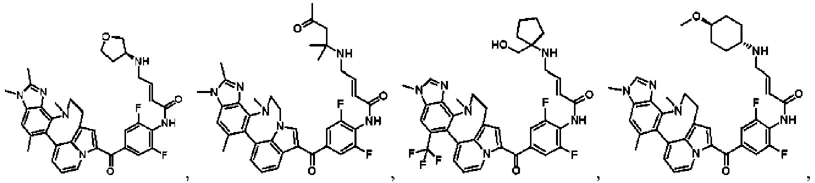 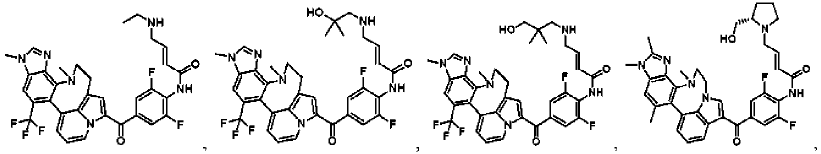 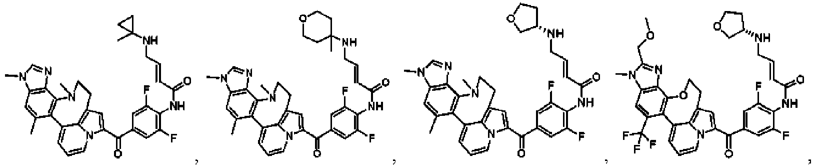 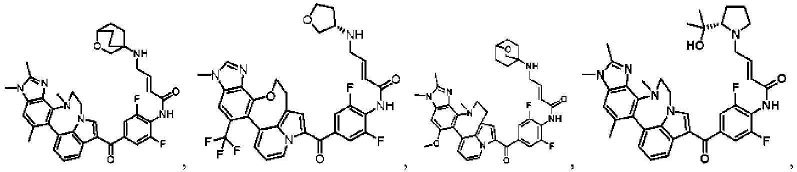 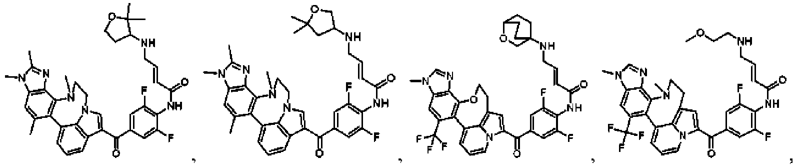 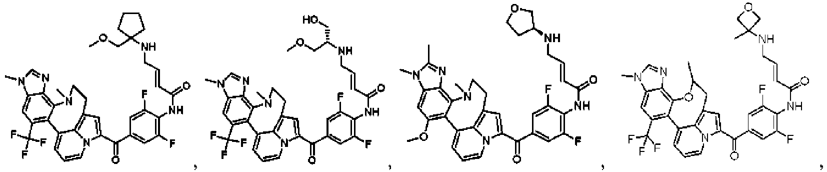 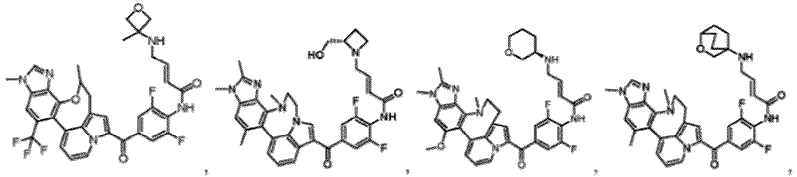 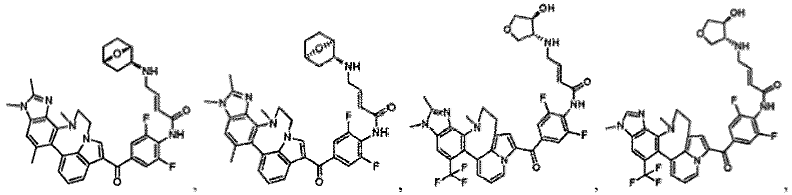 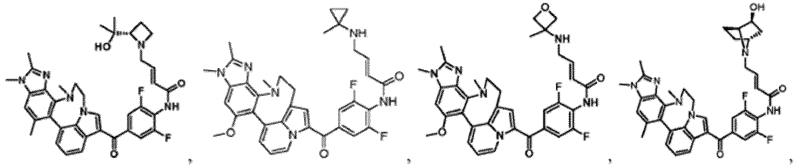 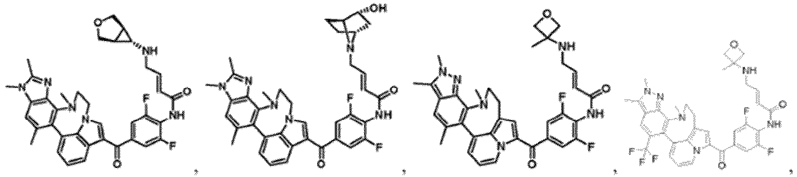 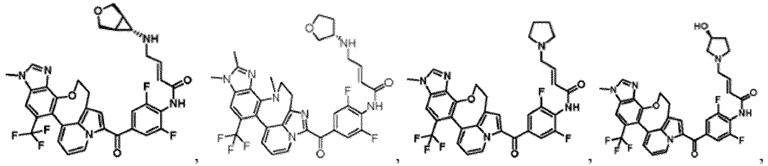 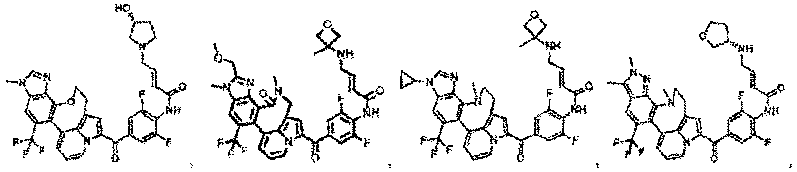 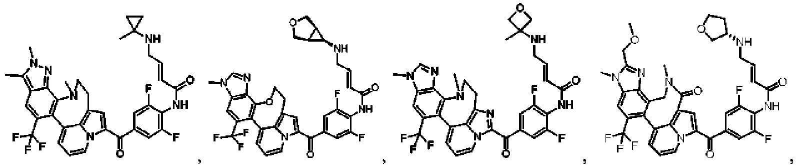 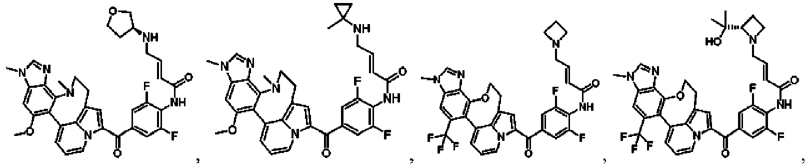 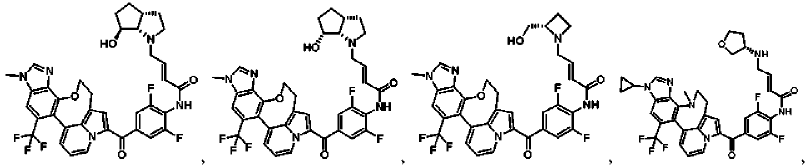 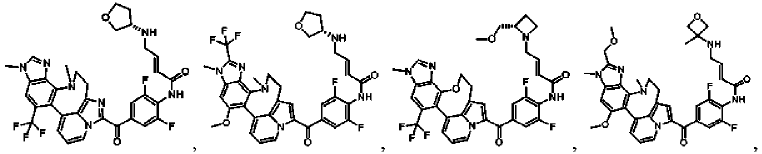 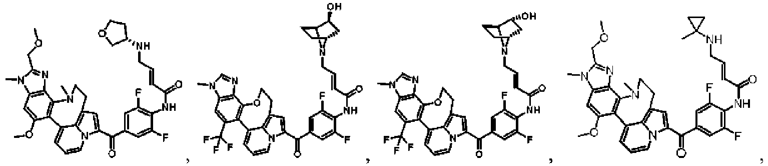 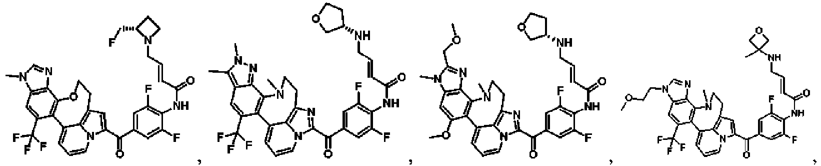 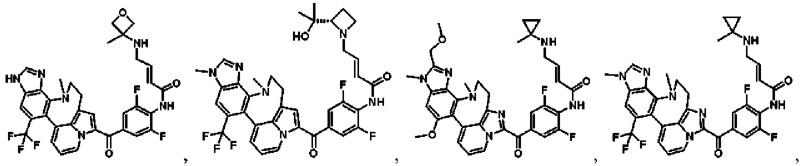 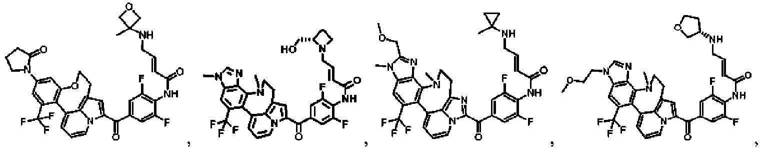 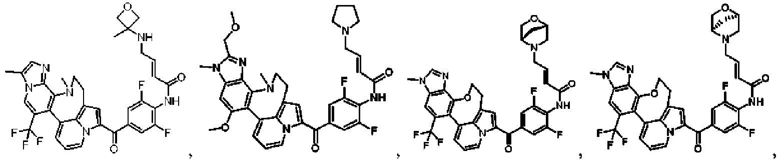 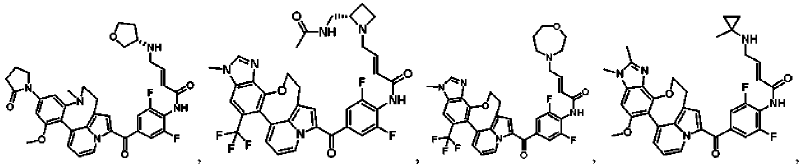 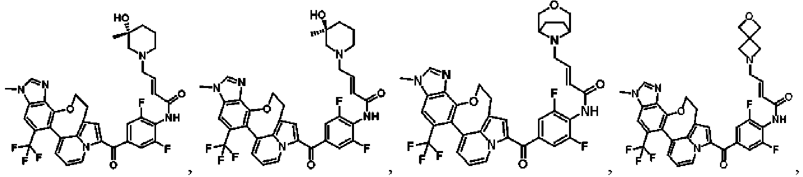 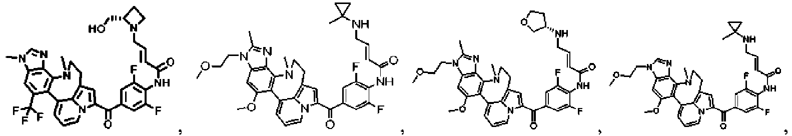 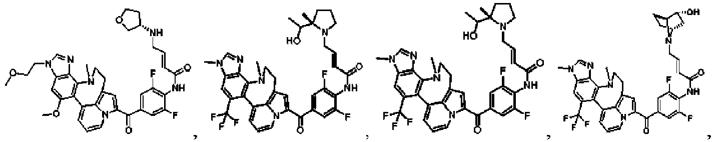 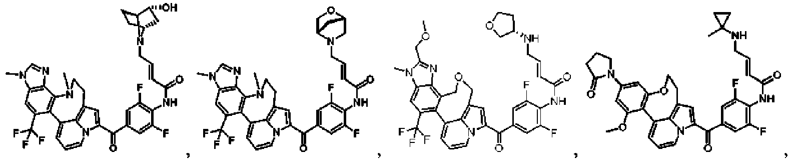 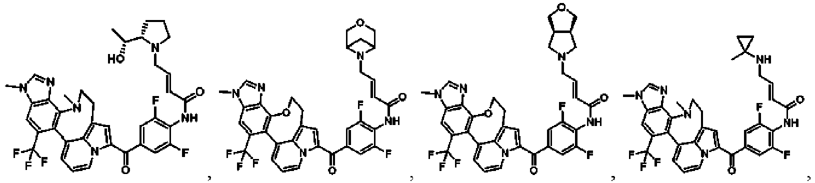 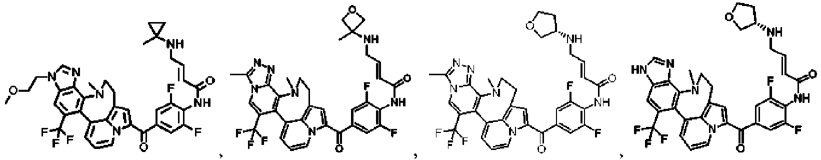 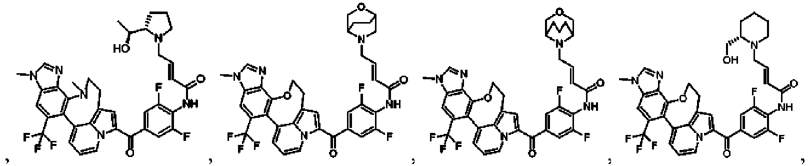 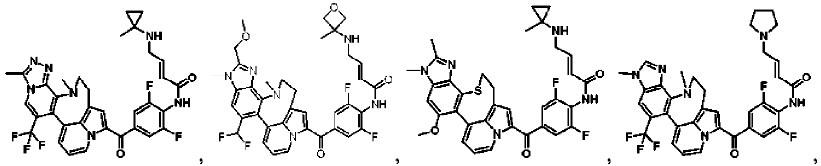 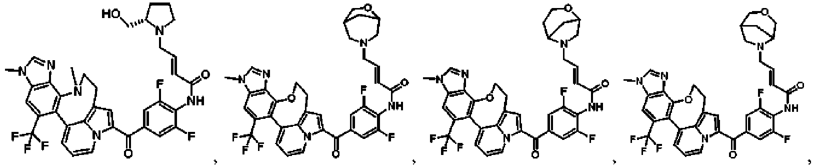 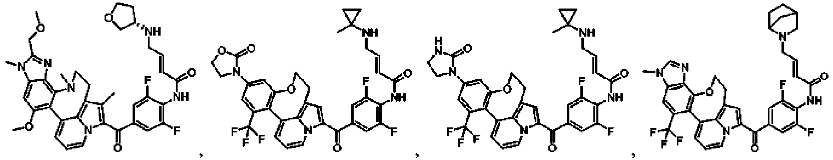 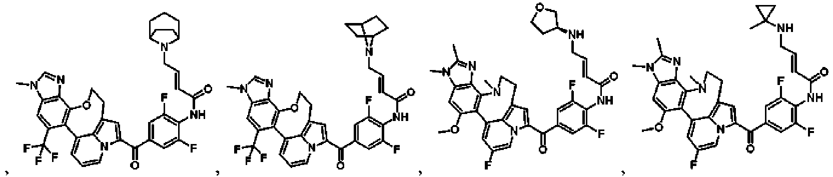 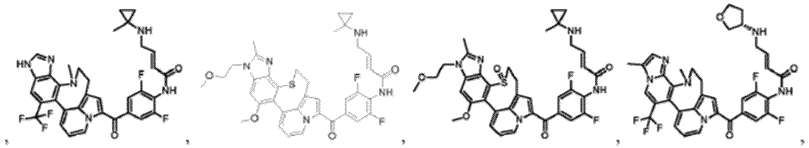 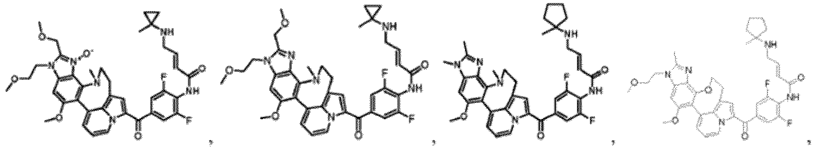 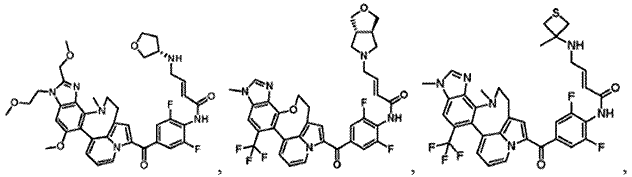 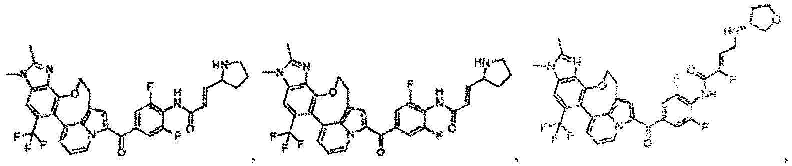 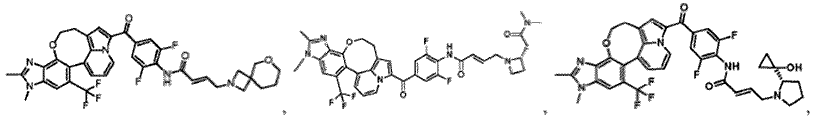 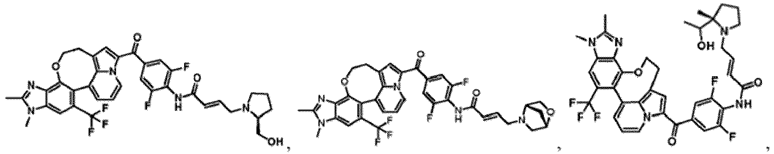 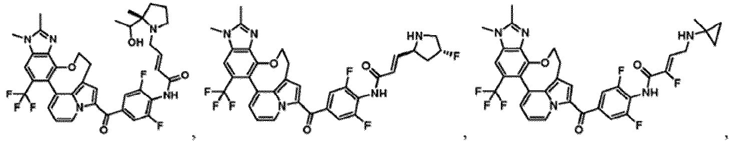 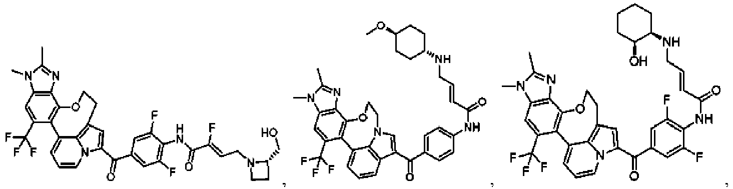 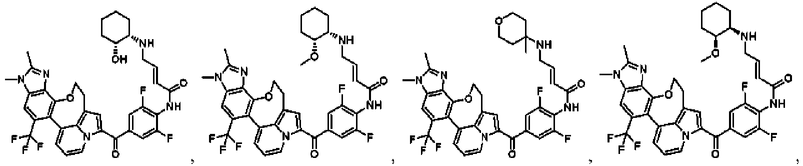 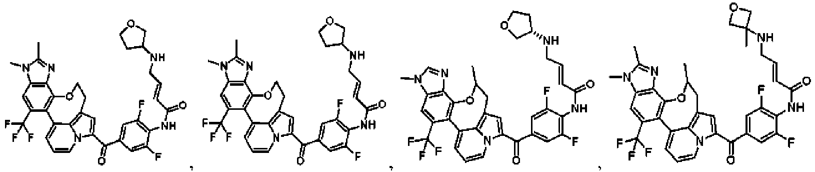 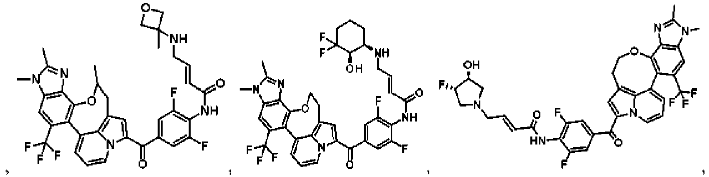 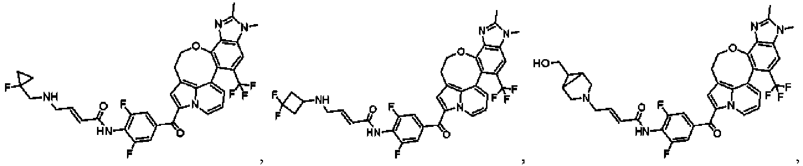 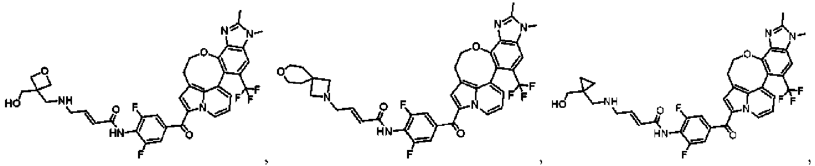 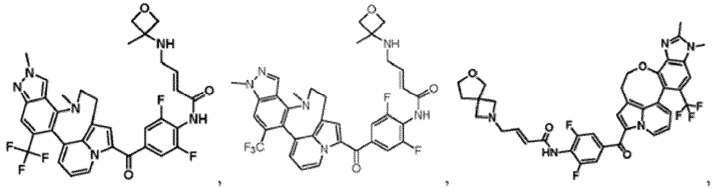 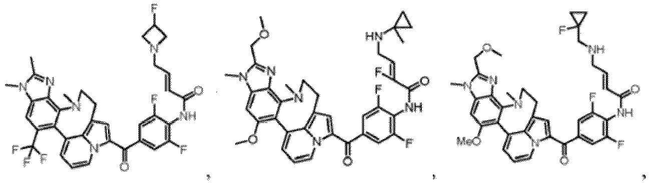 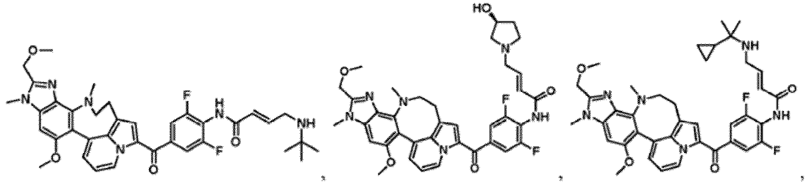 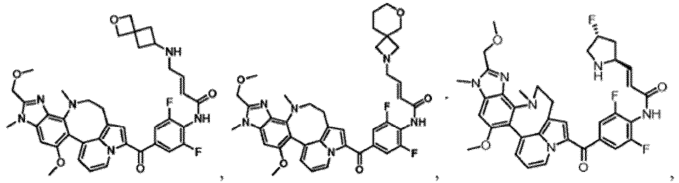 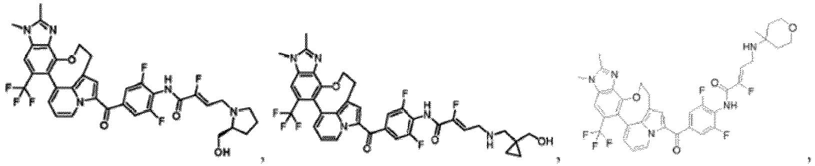 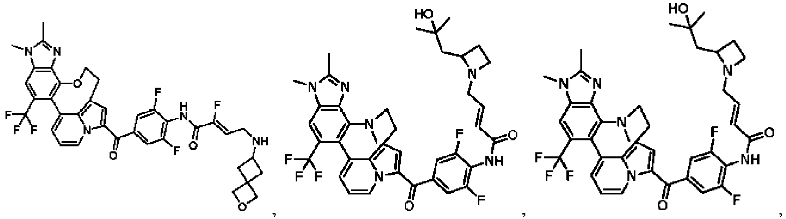 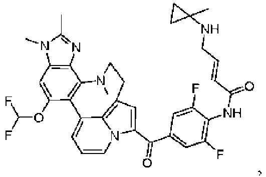 oder pharmazeutisch annehmbares Salz davon.

12. Pharmazeutische Zusammensetzung, umfassend die Verbindung nach einem der Ansprüche 1 bis 11 oder das pharmazeutisch annehmbare Salz davon und einen pharmazeutisch annehmbaren Träger, wobei die pharmazeutische Zusammensetzung gegebenenfalls ein zusätzliches Antikrebsmittel umfasst.

13. Verbindung nach einem der Ansprüche 1 bis 11 oder pharmazeutisch annehmbares Salz davon zur Verwendung bei einer Therapie.

14. Verbindung nach einem der Ansprüche 1 bis 11, oder pharmazeutisch annehmbares Salz davon, oder pharmazeutische Zusammensetzung, die die Verbindung nach einem der Ansprüche 1 bis 11, oder das pharmazeutisch annehmbare Salz davon, umfasst, zur Verwendung bei der Behandlung von Krebs.

15. Verbindung nach einem der Ansprüche 1 bis 11, oder pharmazeutisch annehmbares Salz davon, und ein zusätzliches Antikrebsmittel oder pharmazeutische Zusammensetzung, die die Verbindung nach einem der Ansprüche 1 bis 11, oder das pharmazeutisch annehmbare Salz davon, und ein zusätzliches Antikrebsmittel umfasst, zur Verwendung bei der Behandlung von Krebs.

## Revendications

1. Composé de formule (I) 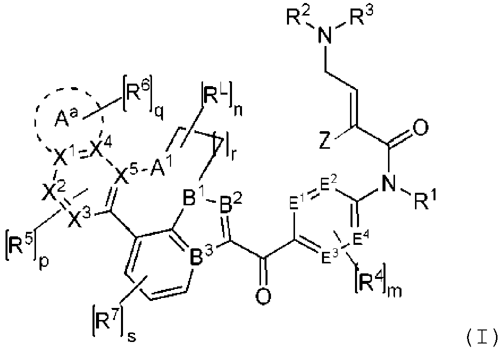
R¹ étant choisi dans le groupe constitué par H et C₁-C₆ alkyle ;
R² et R³ étant indépendamment choisis dans le groupe constitué par :
(i) H ;
(ii) C₁-C₆ alkyle, le C₁-C₆ alkyle étant non substitué ou substitué par 1 à 2 substituants R^{a} indépendamment choisis dans le groupe constitué par halogéno, hydroxy, C₁-C₃ alcoxy, C₂-C₆ alcényle, C₂-C₆ alcynyle, C₁-C₃ alkylamino, C₁-C₃ dialkylamino, C₁-C₃ alkylsulfonyle, C₂-C₄ acyle, C₁-C₃ hydroxyalkyle, C₁-C₃ alcoxy carbonyle, C₁-C₃ hydroxycarbonyle, oxo (=O), C₃-C₈ cycloalkyle, et hétérocycloalkyle monocyclique ou bicyclique à 4 à 10 chaînons ;
le C₃-C₈ cycloalkyle ou l'hétérocycloalkyle monocyclique ou bicyclique à 4 à 10 chaînons de R^{a} étant non substitué ou substitué par 1 à 2 substituants choisis dans le groupe constitué par halogéno, hydroxy, amino, C₁-C₃ alkyle, C₁-C₃ fluoroalkyle, et C₁-C₃ alcoxy ; et
(iii) un système cyclique saturé monocyclique, bicyclique ponté ou spirocyclique à 3 à 8 chaînons contenant 0 à 2 groupes hétéroatomiques choisis dans le groupe constitué par N, O, S, S(O), S(O)₂, P, P(O), et P(O)₂ ;
le système cyclique saturé monocyclique, bicyclique ponté ou spirocyclique à 3 à 8 chaînons étant non substitué ou substitué par 1 à 4 substituants indépendamment choisis dans le groupe constitué par halogéno, hydroxy, C₁-C₃ alkyle, C₁-C₃ fluoroalkyle, C₁-C₃ alcoxy, C₂-C₆ alcényle, C₂-C₆ alcynyle, C₁-C₃ alkylamino, C₁-C₃ dialkylamino, C₁-C₃ alkylsulfonyle, C₂-C₄ acyle, C₁-C₃ hydroxyalkyle, C₁-C₃ alcoxy(C₁-C₃) alkyle, carboxy, et C₁-C₃ alcoxycarbonyle ; ou en variante, R² et R³ conjointement avec l'atome de N auquel ils sont fixés formant un système cyclique saturé monocyclique, bicyclique condensé, bicyclique ponté ou spirocyclique à 4 à 9 chaînons contenant 0 à 2 hétéroatomes supplémentaires choisis dans le groupe constitué par N, O, et S ;
le système cyclique saturé monocyclique, bicyclique condensé, bicyclique ponté ou spirocyclique à 4 à 9 chaînons étant non substitué ou substitué par 1 à 4 substituants indépendamment choisis dans le groupe constitué par halogéno, hydroxy, C₁-C₃ alkyle, C₁-C₃ fluoroalkyle, C₁-C₃ alcoxy, C₁-C₄ hydroxyalkyle, C₃-C₅ hydroxycycloalkyle, -(CH₃)₂N(C=O)CH₂-, CH₃(C=O)NHCH₂-, et C₁-C₃ alcoxy(C₁-C₃)alkyle ;
ou en variante, l'un parmi R² et R³ conjointement avec l'atome de N auquel il est fixé et un atome de C adjacent formant un système cyclique saturé monocyclique à 3 à 6 chaînons contenant 0 à 2 hétéroatomes supplémentaires choisis dans le groupe constitué par N, O, et S ;
le système cyclique saturé monocyclique à 3 à 6 chaînons étant non substitué ou substitué par 1 à 4 substituants indépendamment choisis dans le groupe constitué par halogéno, hydroxy, C₁-C₃ alkyle, C₁-C₃ fluoroalkyle, C₁-C₃ alcoxy, C₁-C₃ hydroxyalkyle, et C₁-C₃ alcoxy(C₁-C₃)alkyle ;
en chaque occurrence R⁴ étant indépendamment choisi dans le groupe constitué par halogéno, cyano, C₁-C₃ alkyle, C₁-C₃ fluoroalkyle, C₁-C₃ alcoxy, et C₁-C₄ cyanoalkyle ;
E¹, E², E³, et E⁴ étant indépendamment choisis dans le groupe constitué par N et C, à condition que pas plus de trois parmi E¹, E², E³, et E⁴ ne soient N ;
l'un parmi B¹ et B³ étant C et l'autre étant N ;
B² étant N ou C(R^{B}), R^{B} étant choisi dans le groupe constitué par H, C₁-C₆ alkyle, C₂-C₆ alcényle, C₂-C₆ alcynyle et C₃-C₆ cycloalkyle ;
en chaque occurrence R^{L} étant indépendamment choisi dans le groupe constitué par fluoro, hydroxy, C₁-C₆ alkyle, C₁-C₃ alcoxy, C₁-C₃ fluoroalkyle, oxo (=O), C₂-C₄ acyle, et méthényle (=CH₂), ou
en variante, deux substituants R^{L} substitués de manière géminale conjointement avec l'atome de carbone auquel ils sont fixés formant un cycle spirocyclique à 3 à 6 chaînons contenant 0 à 1 hétéroatome choisi dans le groupe constitué par N, O, et S ;
le cycle A^{a} étant présent ou absent, et s'il est présent, étant un hétéroaryle à 5 à 6 chaînons contenant 1 à 3 hétéroatomes choisis dans le groupe constitué par N, O, et S ;
X¹, X², X³, X⁴, et X⁵ étant indépendamment choisis dans le groupe constitué par N et C, à condition que pas plus de deux parmi X¹, X², X³, X⁴, et X⁵ ne soient N ;
en chaque occurrence R⁵ étant indépendamment choisi dans le groupe constitué par C₁-C₆ alkyle, C₁-C₆ fluoroalkyle, C₁-C₃ alcoxy, C₁-C₃ acyle, halogéno, hydroxy, amino, cyano, oxo (=0), C₁-C₄ cyanoalkyle, C₁-C₃ hydroxyalkyle, C₁-C₃ alkylamino, C₁-C₃ dialkylamino, C₁-C₃ alcoxy (C₁-C₃)alkyle, et un groupe de la formule 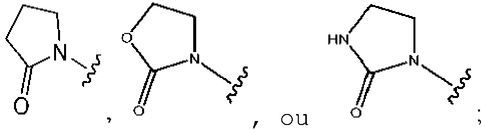
en chaque occurrence R⁶ étant indépendamment choisi dans le groupe constitué par C₁-C₃ alkyle, C₁-C₃ fluoroalkyle, C₁-C₃ alcoxy, halogéno, hydroxy, oxo (=O), cyano, carbamoyle, C₁-C₄ cyanoalkyle, C₁-C₃ hydroxyalkyle, C₁-C₃ alcoxy (C₁-C₃) alkyle, , C₃-C₆ cycloalkyle, et C₁-C₄ carbamoylalkyle ;
chaque occurrence de R⁷ étant indépendamment choisie dans le groupe constitué par halogéno, C₁-C₃ alkyle, C₁-C₃ fluoroalkyle, et C₁-C₃ alcoxy ;
A¹ étant choisi dans le groupe constitué par -CH₂-, -CH₂-N(R^{L})-, -C(=O)- N(R^{L})-, -CH₂-O-, -C(H)(R^{L})-, -N(H)-, - N(R^{L})-, -S-, -S(=O)-, et -O- ;
Z étant choisi dans le groupe constitué par H et halogéno ;
l'indice m étant 0, 1, 2, ou 3 ;
l'indice n étant 0, 1, 2, ou 3 ;
l'indice p étant 0, 1, 2, 3, ou 4 ;
l'indice q étant 0, 1, 2, ou 3 ;
l'indice r étant 0, 1, 2, ou 3 ; et
l'indice s étant 0, 1, 2, ou 3,
ou sel pharmaceutiquement acceptable correspondant.

2. Composé selon la revendication 1 ou sel pharmaceutiquement acceptable correspondant,
le C₁-C₆ alkyle étant non substitué ou substitué par 1 à 2 substituants R^{a} indépendamment choisis dans le groupe constitué par halogéno, hydroxy, C₁-C₃ alcoxy, C₂-C₆ alcényle, C₂-C₆ alcynyle, C₁-C₃ alkylamino, C₁-C₃ dialkylamino, C₁-C₃ alkylsulfonyle, C₂-C₄ acyle, C₁-C₃ hydroxyalkyle, C₁-C₃ alcoxycarbonyle, C₁-C₃ hydroxycarbonyle, C₃-C₈ cycloalkyle, et hétérocycloalkyle monocyclique ou bicyclique à 4 à 10 chaînons ;
R² et R³ conjointement avec l'atome de N auquel ils sont fixés formant l'hétérocycloalkyle saturé à 4 à 8 chaînons ayant 0 à 2 hétéroatomes supplémentaires choisis dans le groupe constitué par N, O, et S ;
l'hétérocycloalkyle à 4 à 8 chaînons étant non substitué ou substitué par 1 à 4 substituants indépendamment choisis dans le groupe constitué par halogéno, hydroxy, C₁-C₃ alkyle, C₁-C₃ fluoroalkyle, C₁-C₃ alcoxy, C₁-C₃ hydroxyalkyle, et C₁-C₃ alcoxy(C₁-C₃)alkyle ;
en chaque occurrence R^{L} étant indépendamment choisi dans le groupe constitué par fluoro, hydroxy, C₁-C₆ alkyle, C₁-C₃ alcoxy, C₁-C₃ fluoroalkyle, oxo (=O), et méthényle (=CH₂),
le cycle A^{a} étant présent ou absent, et s'il est présent, étant l'hétéroaryle à 5 à 6 chaînons contenant 1 à 2 hétéroatomes choisis dans le groupe constitué par N, O, et S ;
en chaque occurrence R⁵ étant indépendamment choisi dans le groupe constitué par C₁-C₆ alkyle, C₁-C₆ fluoroalkyle, C₁-C₃ alcoxy, C₁-C₃ acyle, halogéno, hydroxy, amino, cyano, oxo (=O), C₁-C₄ cyanoalkyle, C₁-C₃ hydroxyalkyle, C₁-C₃ alkylamino, C₁-C₃ dialkylamino, C₁-C₃ alcoxy (C₁-C₃)alkyle, et le groupe de la formule 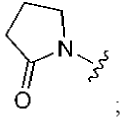
en chaque occurrence R⁶ étant indépendamment choisi dans le groupe constitué par C₁-C₃ alkyle, C₁-C₃ fluoroalkyle, C₁-C₃ alcoxy, halogéno, hydroxy, oxo (=O), cyano, carbamoyle, C₁-C₄ cyanoalkyle, C₁-C₃ hydroxyalkyle, C₁-C₃ alcoxy(C₁-C₃) alkyle, et C₁-C₄ carbamoylalkyle ;
A¹ étant choisi dans le groupe constitué par -CH₂-, - C(H)(R^{L})-, -N(H)-, -N(R^{L})-, et -O- ;
Z étant H ; et
l'indice q étant 0, 1, ou 2.

3. Composé selon la revendication 1 ou sel pharmaceutiquement acceptable correspondant,
(a) le groupe 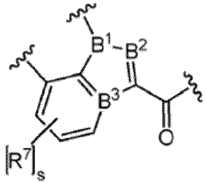 étant 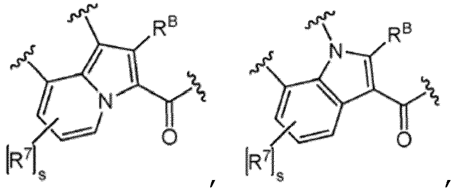 ou 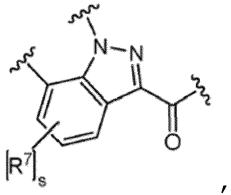 ou
(b) le groupe 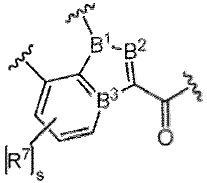 étant 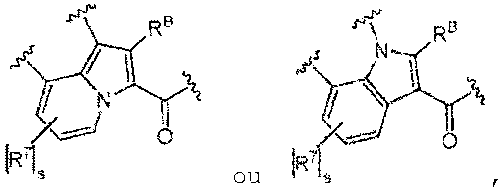 éventuellement R^{B} étant H ou méthyle, et l'indice s étant 0.

4. Composé selon la revendication 1 ou sel pharmaceutiquement acceptable correspondant,
X¹ étant N ou C ; et
X², X³, X⁴, et X⁵ étant C.

5. Composé selon la revendication 1 ou sel pharmaceutiquement acceptable correspondant,
(a) le groupe 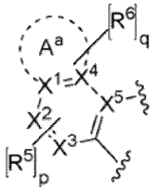 étant 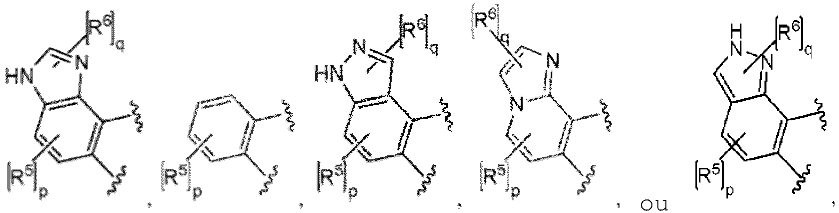 ou
(b) le groupe 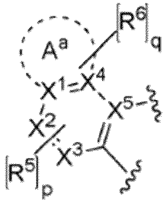 étant 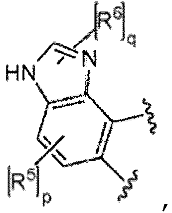 ou
(c) le groupe 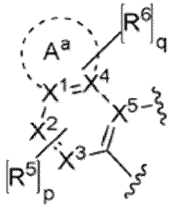 étant 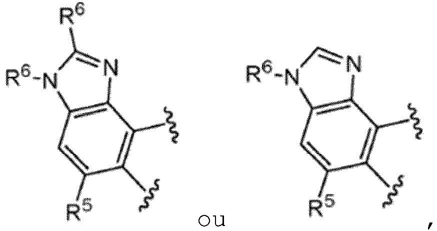
R⁵ étant C₁-C₃ alkyle ou C₁-C₃ fluoroalkyle ; et
R⁶ étant C₁-C₃ alkyle, C₁-C₃ fluoroalkyle, ou C₁-C₃ alcoxy(C₁-C₃)alkyle.

6. Composé selon la revendication 1 ou sel pharmaceutiquement acceptable correspondant, le groupe 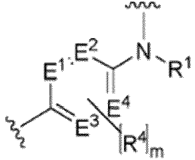 étant

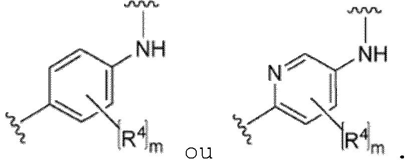

7. Composé selon la revendication 1 ou sel pharmaceutiquement acceptable correspondant,
(a) le groupe 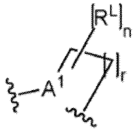 étant 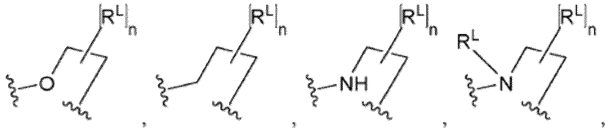 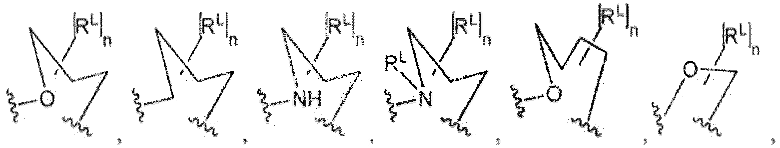 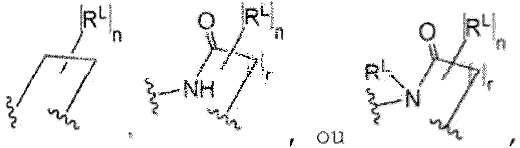 ou
(b) le groupe 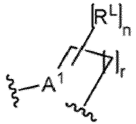 étant 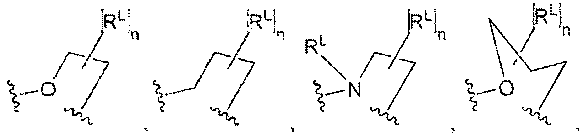 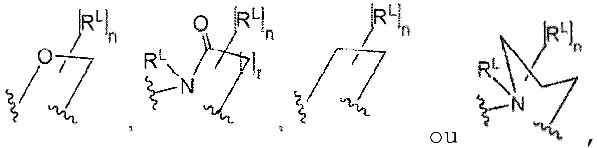 ou
(c) le groupe 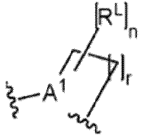 étant

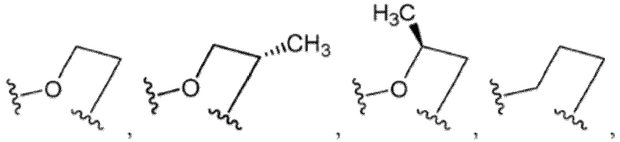

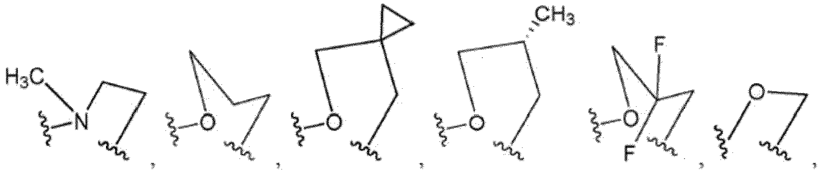

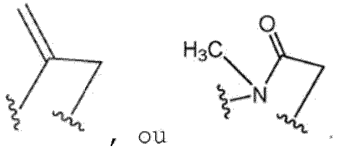

8. Composé selon la revendication 1 ou sel pharmaceutiquement acceptable correspondant,
(a) le groupe 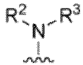 is 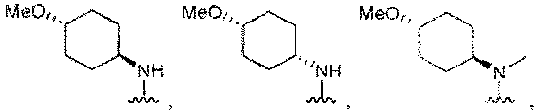 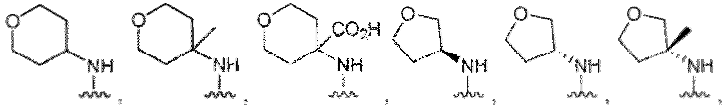 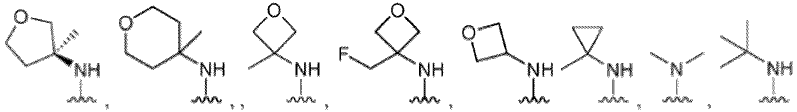 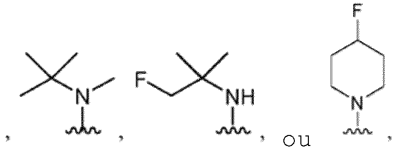 ou
(b) le groupe 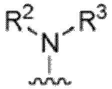 étant

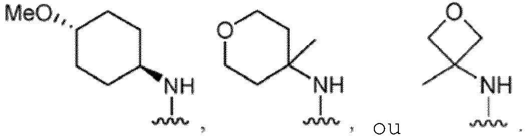

9. Composé selon la revendication 1 ou sel pharmaceutiquement acceptable correspondant, R² et R³ étant indépendamment H, C₁-C₆ alkyle, ou C₁-C₆ fluoroalkyle.

10. Composé selon la revendication 1 ou sel pharmaceutiquement acceptable correspondant,
(i) le groupe 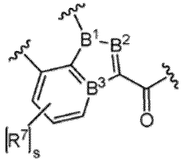 étant 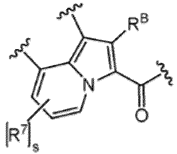 ou 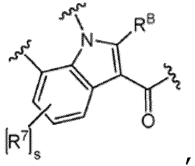 et R^{B} étant H et l'indice s étant 0 ;
(ii) le groupe 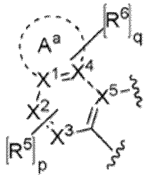 étant 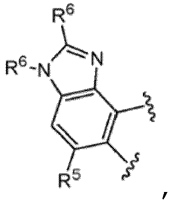 R⁵ et R⁶ étant indépendamment C₁-C₃ alkyle ou C₁-C₃ fluoroalkyle ;
(iii) le groupe 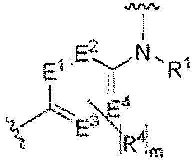 étant 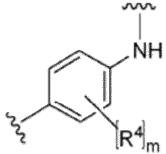 ou 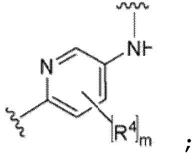
(iv) le groupe 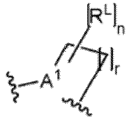 étant 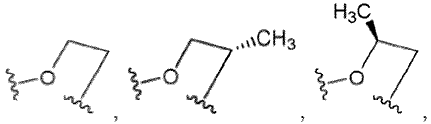 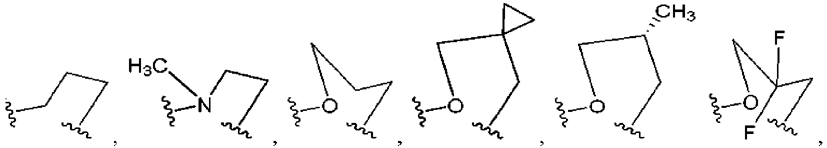 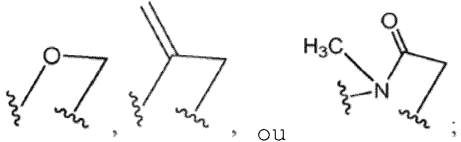 et
(v) le groupe 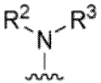 étant 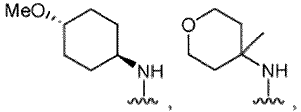 ou

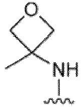

11. Composé selon l'une quelconque des revendications 1-10 choisi parmi : 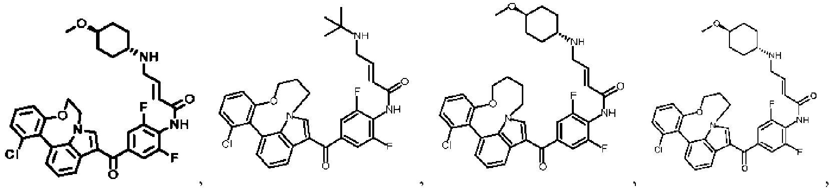 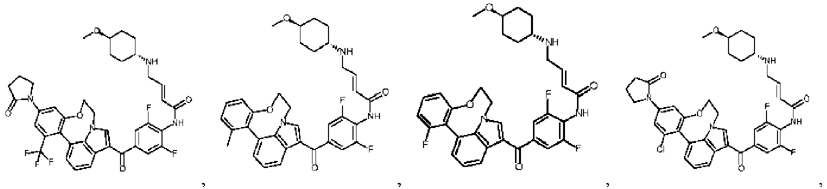 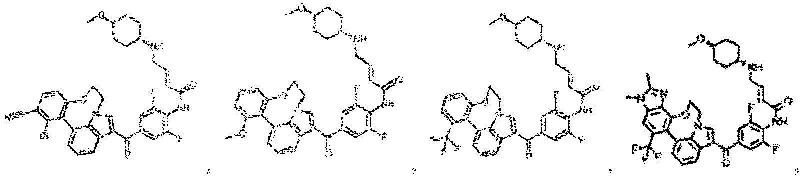 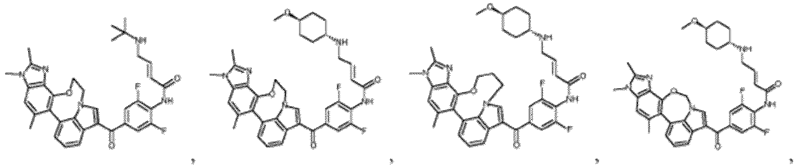 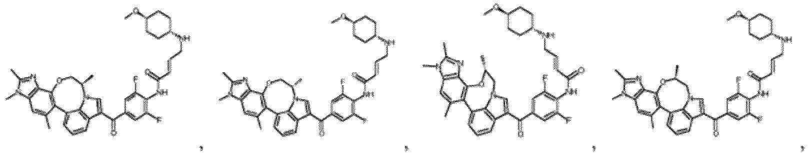 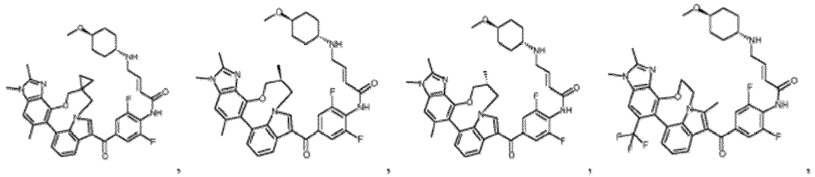 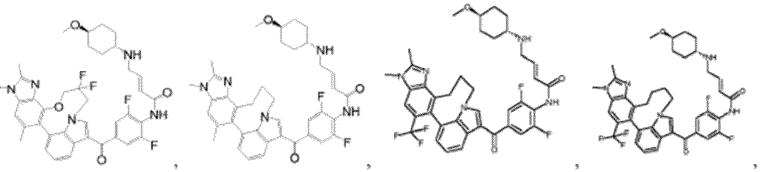 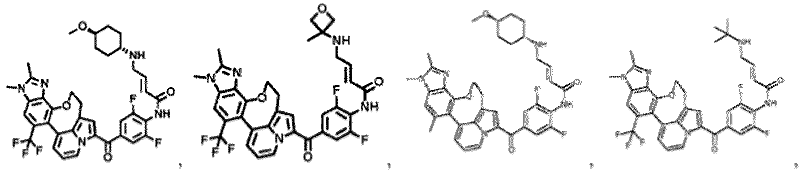 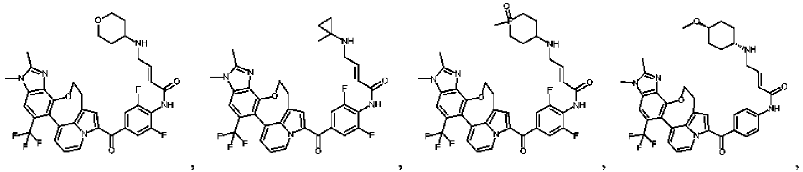 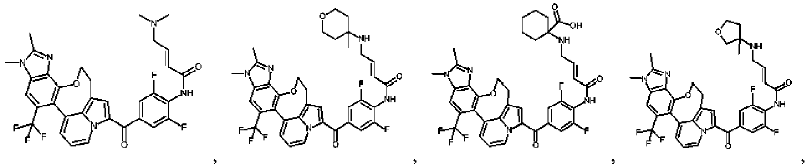 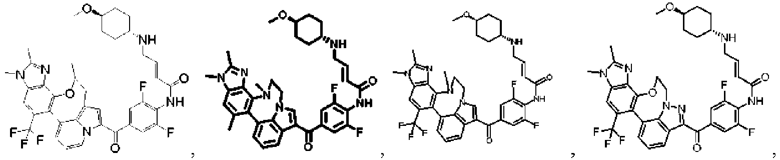 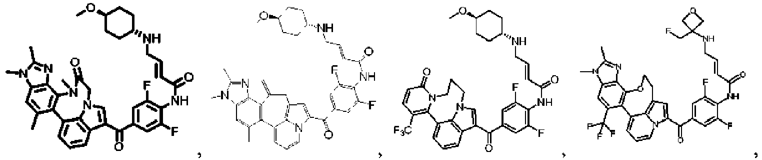 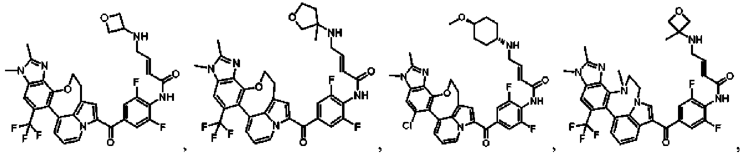 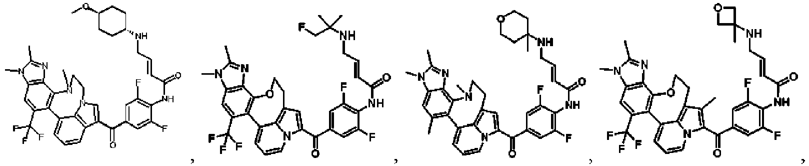 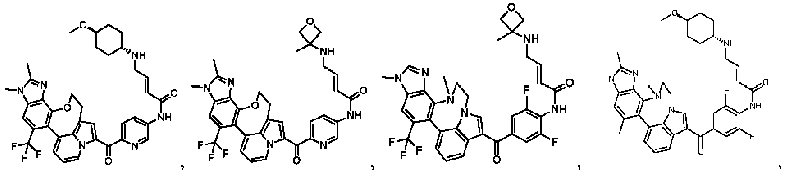 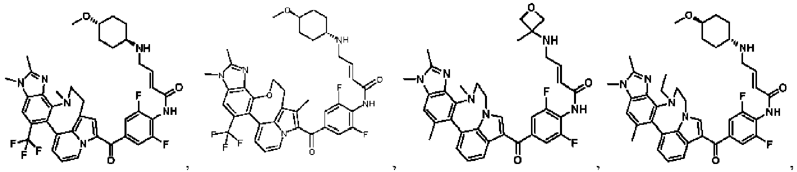 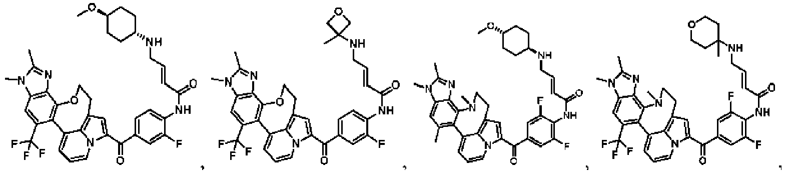 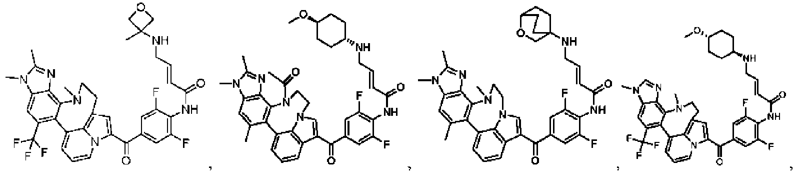 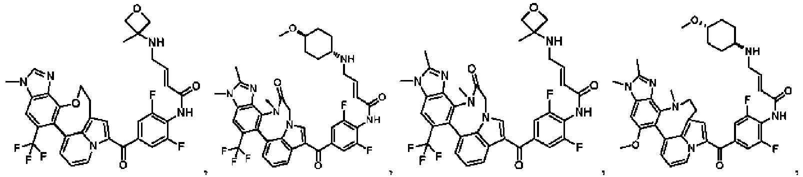 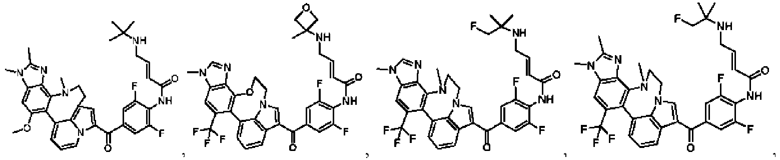 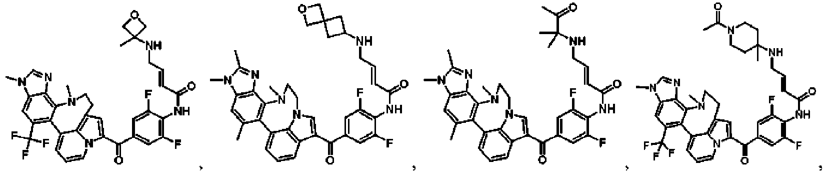 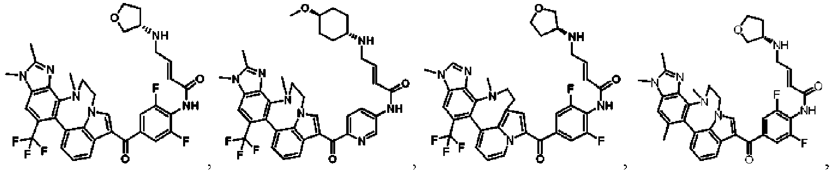 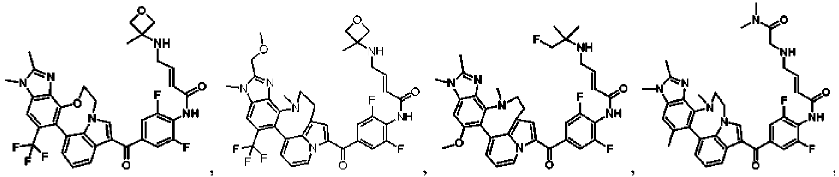 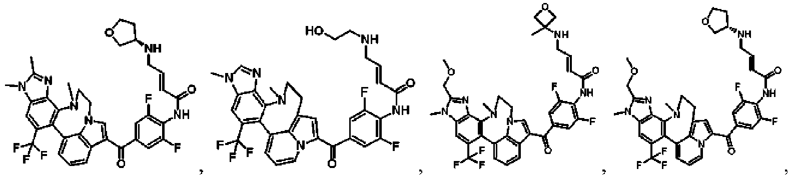 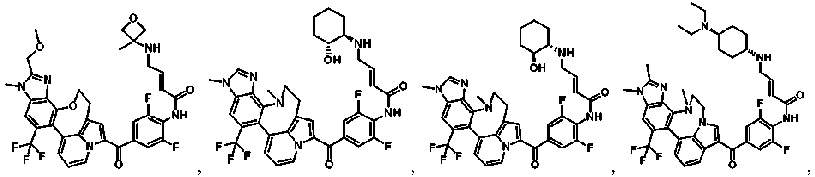 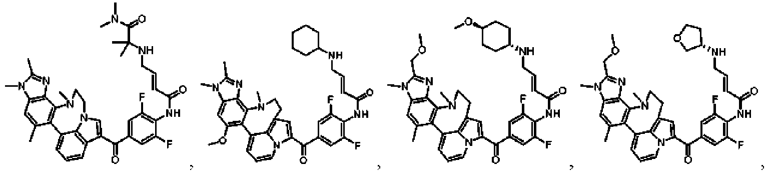 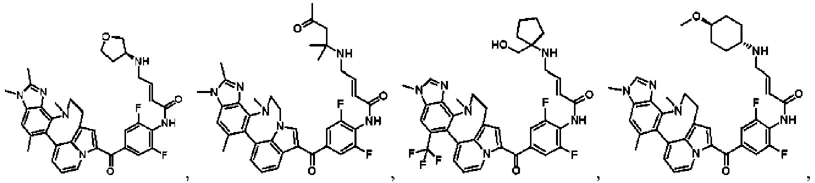 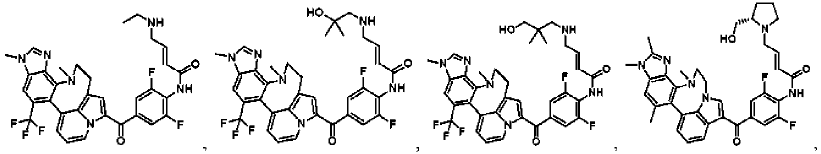 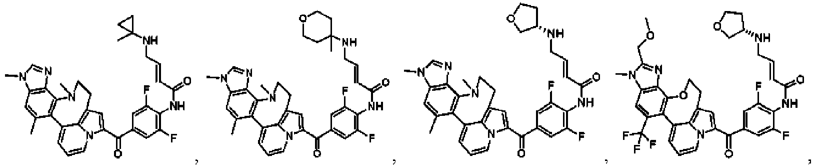 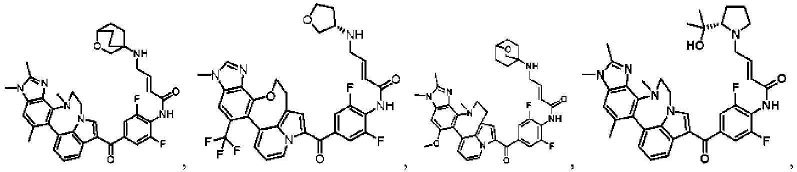 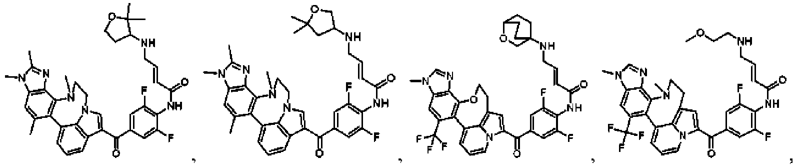 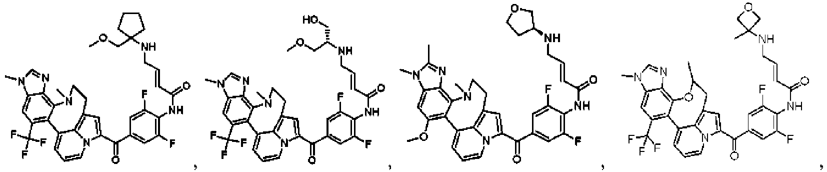 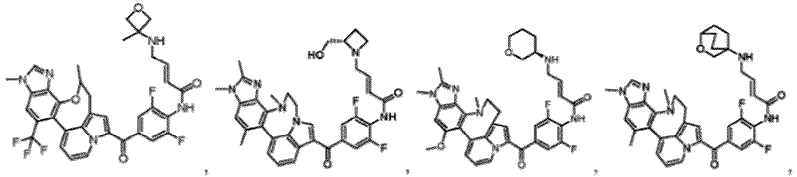 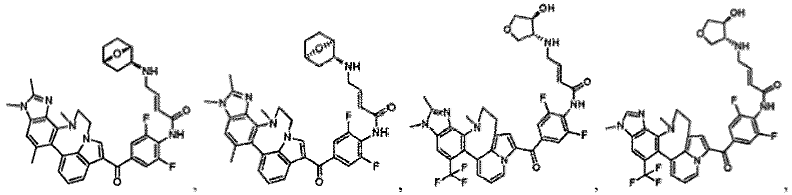 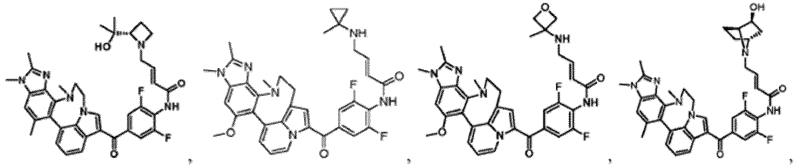 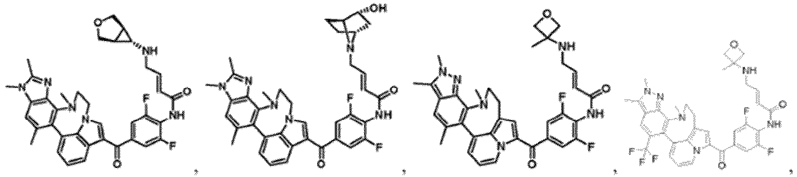 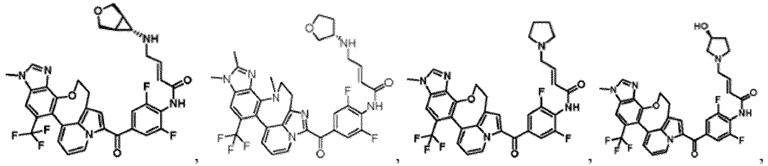 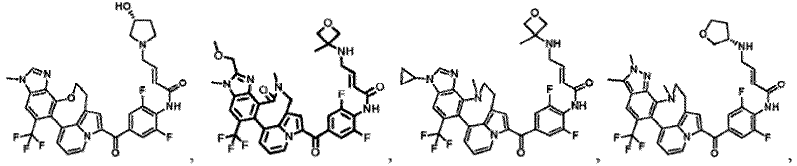 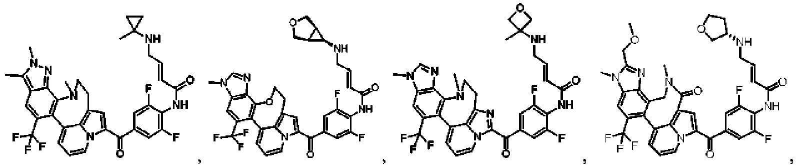 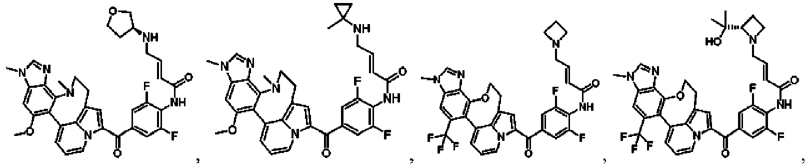 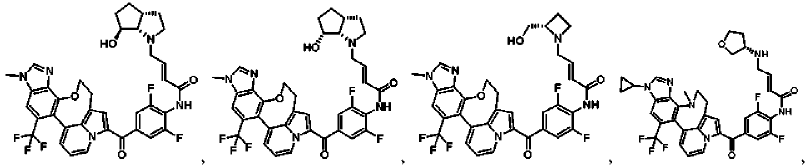 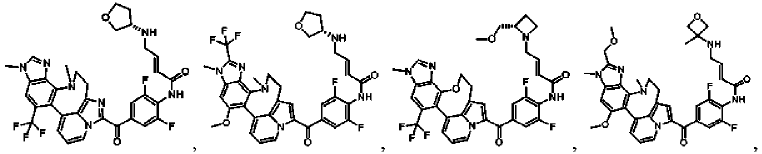 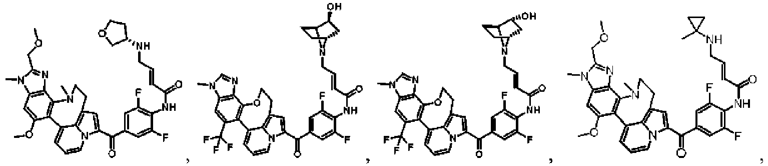 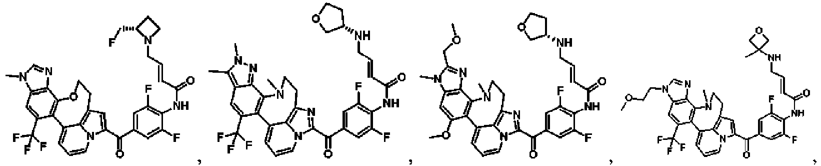 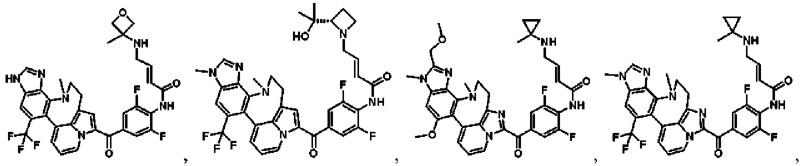 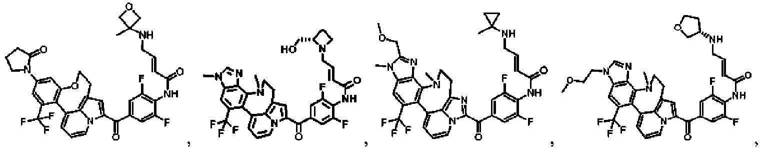 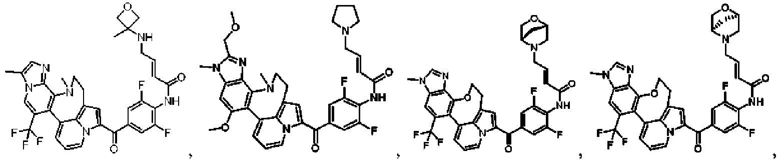 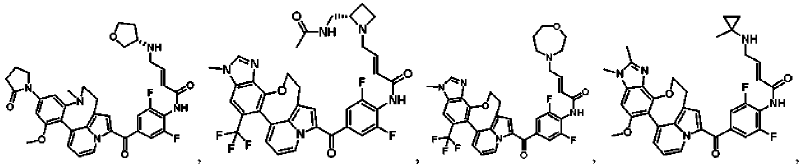 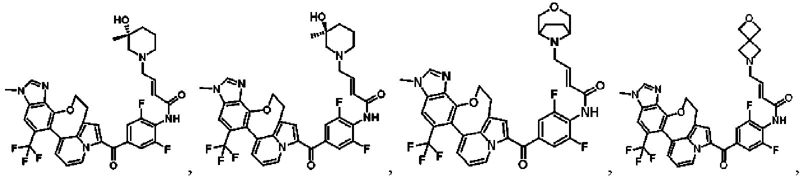 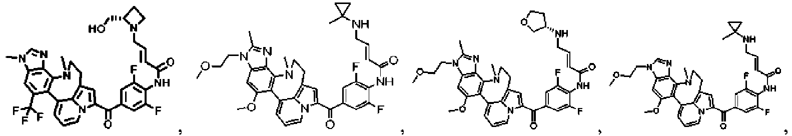 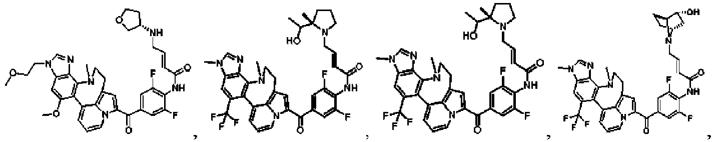 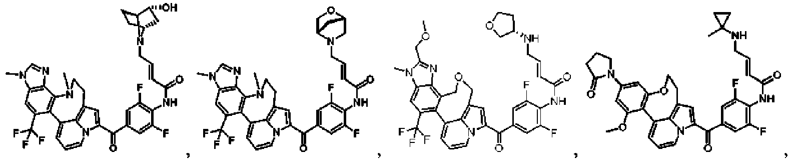 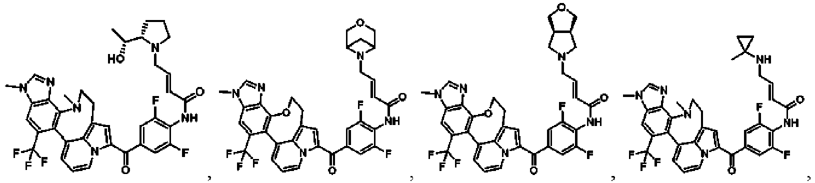 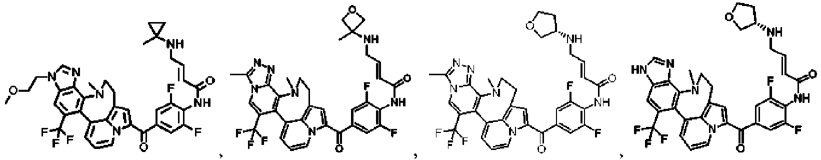 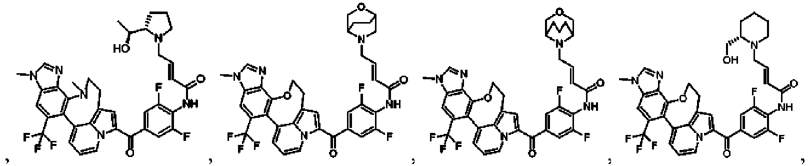 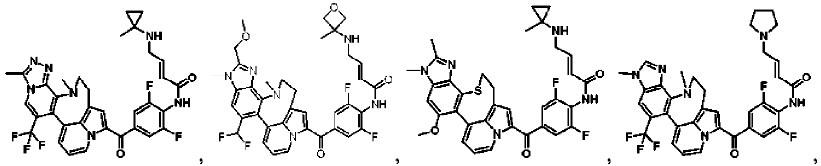 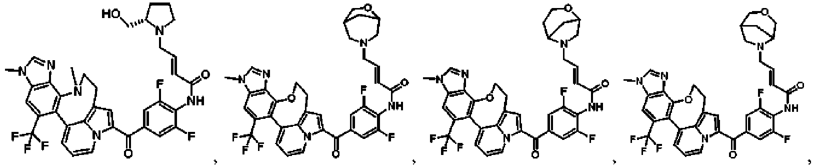 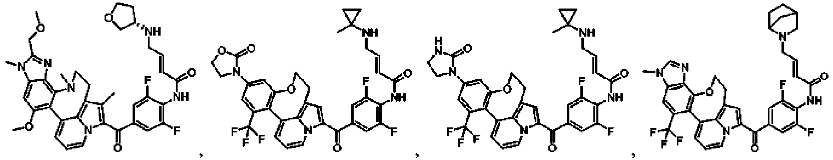 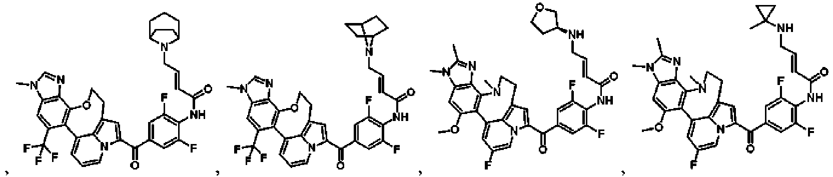 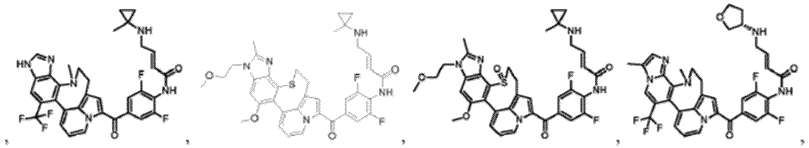 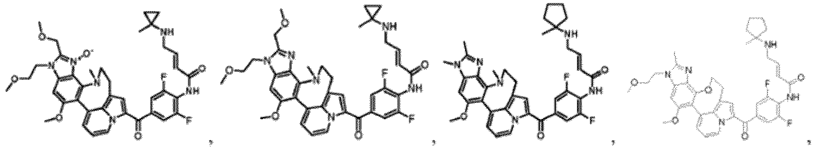 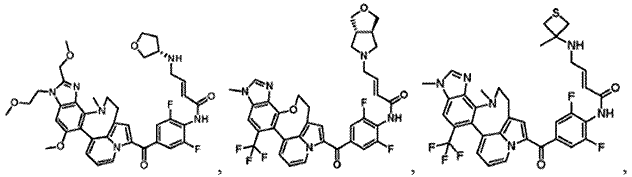 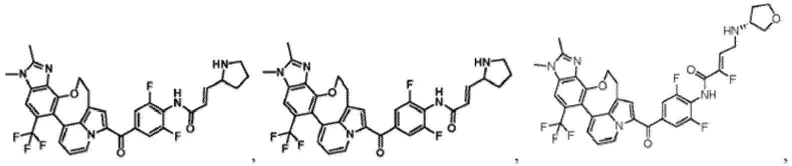 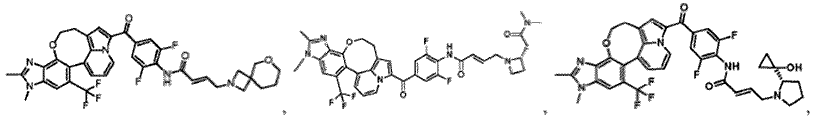 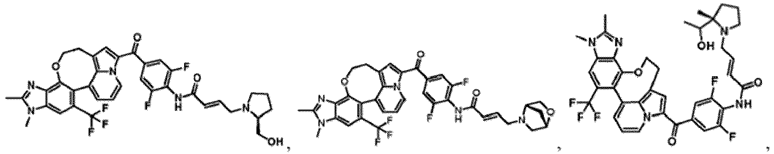 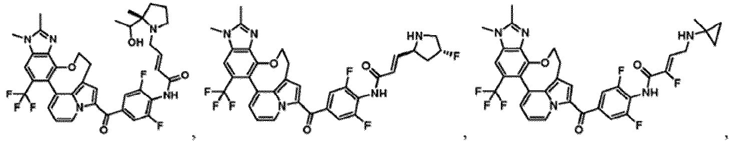 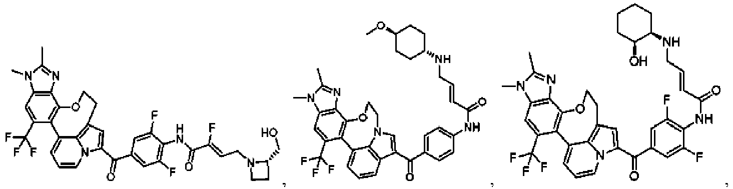 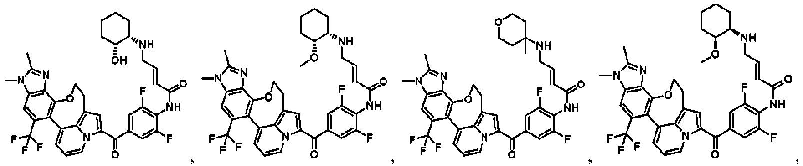 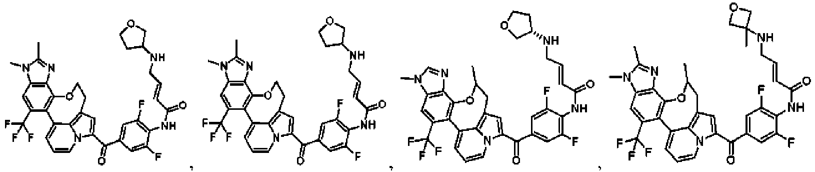 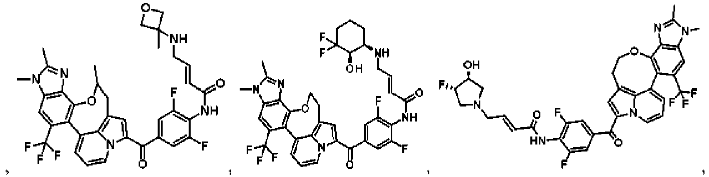 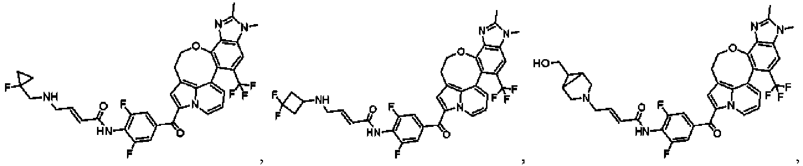 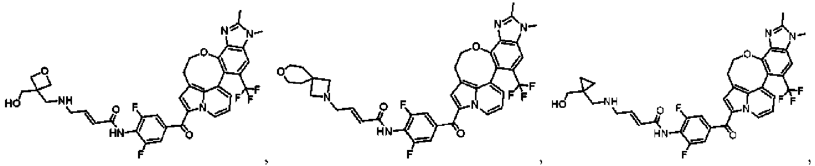 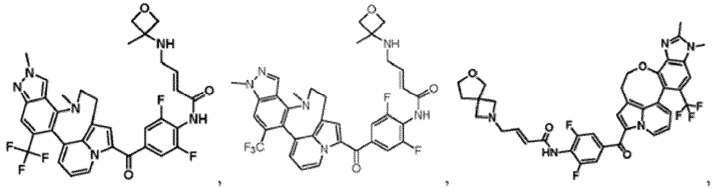 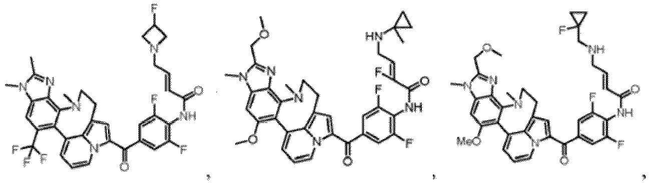 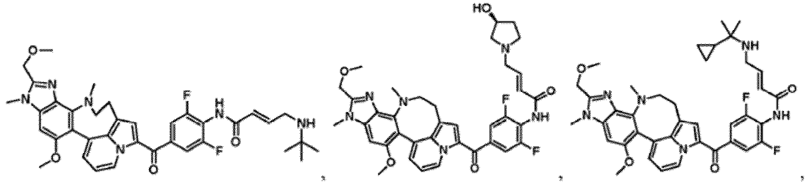 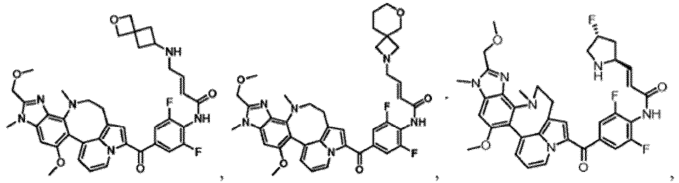 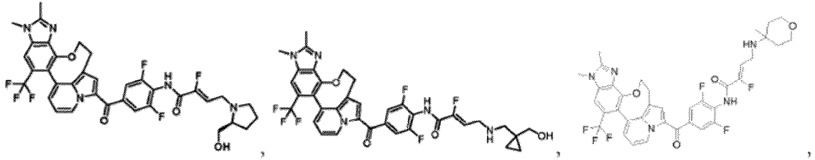 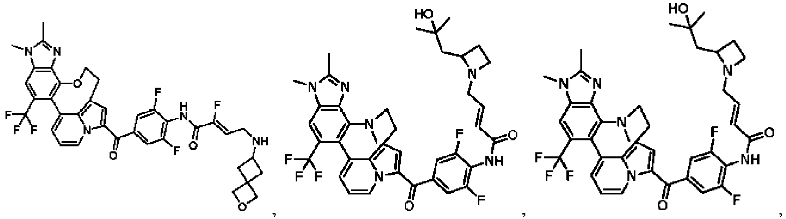 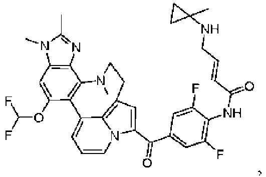 ou sel pharmaceutiquement acceptable correspondant.

12. Composition pharmaceutique comprenant le composé selon l'une quelconque des revendications 1 à 11 ou le sel pharmaceutiquement acceptable de celui-ci, et un véhicule pharmaceutiquement acceptable, où la composition pharmaceutique comprend facultativement un agent anticancéreux supplémentaire.

13. Composé selon l'une quelconque des revendications 1 à 11, ou sel pharmaceutiquement acceptable correspondant, pour utilisation en thérapie.

14. Composé selon l'une quelconque des revendications 1 à 11, ou sel pharmaceutiquement acceptable de celui-ci, ou composition pharmaceutique comprenant le composé selon l'une quelconque des revendications 1 à 11, ou le sel pharmaceutiquement acceptable de celui-ci, pour une utilisation dans le traitement du cancer.

15. Composé selon l'une quelconque des revendications 1 à 11, ou sel pharmaceutiquement acceptable correspondant, et agent anticancéreux supplémentaire, ou composition pharmaceutique comprenant le composé selon l'une quelconque des revendications 1 à 11, ou le sel pharmaceutiquement acceptable correspondant, et un agent anticancéreux supplémentaire, pour utilisation dans le traitement d'un cancer.
